# EUROPEAN PATENT APPLICATION

(11) **EP 2 108 642 A1**
(43) Date of publication of application: **14.10.2009**
(21) Application number: 07829979.9
(22) Date of filing: 17.10.2007
(51) Int. Cl.: C07D 209/46, A61K 31/404, A61K 31/4178, A61K 31/4184, A61K 31/423, A61K 31/4439, A61K 31/454, A61K 31/4545, A61K 31/496, A61K 31/506, A61K 31/536, A61K 31/5377, A61K 31/541, A61K 31/55, A61P 35/00, A61P 37/02, A61P 43/00, C07D 209/48, C07D 401/14, C07D 403/14, C07D 413/04

(54) **JAK INHIBITOR**

(30) Priority: 17.10.2006 JP 2006282088
(71) Applicant: Kyowa Hakko Kirin Co., Ltd., Tokyo 100-8185 (JP)
(72) Inventor: AMISHIRO, Nobuyoshi, 1-cho, Sakai-ku Sakai-shi, Osaka, 590-8554 (JP); ATSUMI,Toshiyuki, Sunto-gun, Shizuoka 411-8731 (JP); NAKAZATO, Tomoyuki, Sunto-Gun, Shinizuka 411-8731 (JP); UMEHARA, Hiroshi, Sunto-Gun, Shinizuka 411-8731 (JP); FUJIHARA, Hiroaki, Sunto-Gun, Shinizuka 411-8731 (JP); SHINOHARA, Fumikazu, Sunto-Gun, Shinizuka 411-8731 (JP); FUNAHASHI, Jun, Sunto-Gun, Shinizuka 411-0944 (JP); YAMAMOTO, Junichiro, Sunto-Gun, Shinizuka 411-8731 (JP); YAGI, Kaori, Sunto-Gun, Shinizuka 411-8731 (JP)
(74) Representative: Simcox, Michael Thomas
(86) International application number: PCT/JP2007/070245
(87) International publication number: WO 2008/047831

(57) **Abstract**

A JAK inhibitor comprising, as an active ingredient, a nitrogen-containing heterocyclic compound represented by formula (I)
{wherein W represents a nitrogen atom or -CH-;
X represents -C (=O) - or -CHR⁴- (wherein R⁴ represents a hydrogen atom, or the like);
R¹ represents the formula described below [wherein Q¹ represents-CR⁸-(wherein R⁸ represents a hydrogen atom, substituted or unsubstituted lower alkyl, or the like);
Q² represents -NR¹⁵- (wherein R¹⁵ represents a hydrogen atom, substituted or unsubstituted lower alkyl, or the like); and R⁵ and R⁶ may be the same or different and each represents a hydrogen atom, halogen, carboxy, substituted or unsubstituted lower alkyl, or the like], or the like; and
R² and R³ may be the same or different and each represents a hydrogen atom, halogen, substituted or unsubstituted lower alkyl, or the like} or a pharmaceutically acceptable salt thereof.

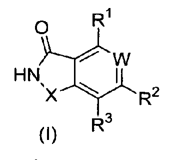

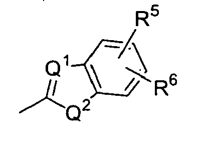

## Description

### Technical Field

The present invention relates to a JAK inhibitor comprising, as an active ingredient, a nitrogen-containing heterocyclic compound or a pharmaceutically acceptable salt thereof, and the like.

### Background Art

Janus kinases (hereinafter referred to as "JAK") are non-receptor tyrosine kinases that are activated by cytokine stimulation and have four subtypes: JAK1, JAK2, JAK3, and Tyk2 (hereinafter "JAK" or "JAK family" includes JAK1, JAK2, JAK3, and Tyk2). It is known that the JAK family phosphorylates signal transducers and activators of transcription (hereinafter referred to as "STAT") and thereby activates signals for cell growth, anti-apoptosis, etc.

Activation of JAK is reported in various tumors. In childhood T-cell acute lymphoblastic leukemia, a translocation, t(9;12)(p24;p13) is reported, and it is known that TEL-JAK2 fusion proteins arise as a result of the translocation to constitutively activate JAK2 [Science, vol. 278, p. 1309 (1997)]. In atypical chronic myelogenous leukemia, acute myeloid leukemia, and acute lymphoblastic leukemia, PCM1-JAK2 fusion proteins are reported [Cancer Research, Vol.65, p.2662 (2005)]. In addition, a point mutation of JAK3 is reported in acute myeloid leukemia resulting in constitutive activation of JAK3 [Cancer Cell, vol.10, p.65 (2006)].

Many cases of activation of JAK/STAT pathways such as increased secretion of cytokines and increased phosphorylation of STAT3 or STAT5 have been reported in other cancers as well. For example, increased phosphorylation of STAT3 is reported in colon cancer, head and neck cancer, etc., [World Journal of Gastroenterology, vol.10, p.1569 (2004); Cancer Research, vol.62, p.3351 (2002)]. In lung cancer, ovary cancer, etc., increased IL-6 secretion is observed, which is related to prognosis [Oncology Reports, vol.5, p.649 (1998); Gynecologic Oncology, vol.66, p.27 (1997)]. Growth inhibition and phosphorylation inhibition of downstream STAT3 by addition of a JAK inhibitor, AG490 are reported in gastric cancer cell lines and multiple myeloma lines [Oncogene, vol.23, p.4921 (2004); Clinical Cancer Research, vol.9, p.316 (2003)]. Based on these reports, JAK inhibitors are expected to serve as effective cancer therapeutic agents.

In myeloproliferative disorders such as polycythaemia vera, essential thrombocythemia, myelofibrosis, etc., many cases of mutation of JAK2 by substitution of valine at position 617 with phenylalanine (hereinafter referred to as V617F mutation) have been discovered, and it has been reported that a cell line having such a mutation shows susceptibility to a JAK inhibitor I [Lancet, vol.365, p.1054 (2005); Nature, vol.434, p.1144 (2005); Cancer Cell, vol.7, p.387 (2005)]. The V617F mutation in JAK2 has also been reported in acute myeloid leukemia, chronic myelomonocytic leukemia, atypical chronic myelogenous leukemia, and myelodysplastic syndrome [Blood, vol.106, p.3377 (2005)]. Based on these reports, JAK inhibitors are expected to be effective against myeloproliferative disorders and cancers with JAK2 mutations.

Inflammation and immunoreaction are controlled by cytokines such as IL-2 and IL-4. Activation of JAK/STAT by cytokine stimulation is known and it has been reported that various JAK inhibitors have shown efficacy in organ transplant rejection models [Nature Reviews Drug Discovery, vol.3, p.555 (2004)]. Accordingly, JAK inhibitors are considered to be effective also as therapeutic agents for disorders related to activation of the immune system, e.g., organ transplant rejection, rheumatism, and psoriasis.

As mentioned above, it is thought that JAK inhibitors are promising as therapeutic agents for diseases associated with abnormal cellular signaling such as cancers, myeloproliferative disorders and allergic diseases.

As JAK inhibitors, tetracyclic compounds (WO05/105814), CP-690550 [Science, Vo.302, p.875 (2003); WO04/0116449] and the like are known in addition to AG490 and JAK inhibitor I mentioned above.

Isoindolinone derivatives having an inhibitory activity against vascular endothelial growth factor receptor (VEGFR2) /kinase insert domain receptor (KDR) are known (Patent Documents 1 to 3 and Non-Patent Document 1).

Isoindolinone derivatives having an inhibitory activity against mitogen-activated protein kinase (MEK) are known (Patent Document 4).

Phthalimide derivatives having an inhibitory activity against AKT, 3-phosphoinositide-dependent protein kinase-1 (PDK-1), p70 ribosomal S6 kinase (p70S6K), and p160-Rho-associated coiled-coil-containing protein kinase (ROCK) are known (Patent Document 5).
Patent Document 1: WO 04/108672
Patent Document 2: U.S. Patent Application Publication 2005/0026976
Patent Document 3: WO 04/021532
Patent Document 4: WO 05/051300
Patent Document 5: WO 05/039564
Non-Patent Document 1: Bioorganic & Medicinal Chemistry Letters, Vol.14, p.4505 (2004)

### Disclosure of the Invention

### Problems to be Solved by the Invention

An object of the present invention is to provide a JAK inhibitor comprising, as an active ingredient, a nitrogen-containing heterocyclic compound or a pharmaceutically acceptable salt thereof, and the like.

### Means for Solving the Problems

The present invention relates to the following items (1) to (67).
(1) A JAK inhibitor comprising, as an active ingredient, a nitrogen-containing heterocyclic compound represented by formula (I): 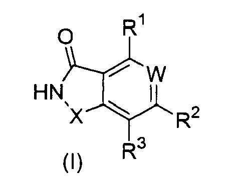
   <wherein W represents a nitrogen atom or -CH-;
   X represents -C(=O)- or -CHR⁴- (wherein R⁴ represents a hydrogen atom, hydroxy, substituted or unsubstituted lower alkyl, substituted or unsubstituted cycloalkyl, or substituted or unsubstituted lower alkoxy) ;
   R¹ represents substituted or unsubstituted aryl, 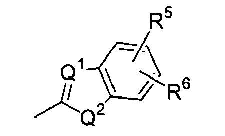
   [wherein Q¹ represents a nitrogen atom or -CR⁸- {wherein R⁸ represents a hydrogen atom, halogen, nitro, hydroxy, cyano, carboxy, substituted or unsubstituted lower alkyl, substituted or unsubstituted cycloalkyl, substituted or unsubstituted aralkyl, substituted or unsubstituted lower alkenyl, substituted or unsubstituted lower alkynyl, substituted or unsubstituted aryl, a substituted or unsubstituted heterocyclic group, substituted or unsubstituted lower alkanoyl, substituted or unsubstituted aroyl, substituted or unsubstituted heteroaroyl, substituted or unsubstituted lower alkoxy, substituted or unsubstituted lower alkoxycarbonyl, -CONR⁹R¹⁰ (wherein R⁹ and R¹⁰ may be the same or different and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, substituted or unsubstituted lower alkanoyl, substituted or unsubstituted aroyl, or substituted or unsubstituted heteroaroyl, or R⁹ and R¹⁰ are combined together with the adjacent nitrogen atom thereto to form a substituted or unsubstituted heterocyclic group) or -NR¹¹R¹² [wherein R¹¹ and R¹² may be the same or different and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, substituted or unsubstituted lower alkanoyl, substituted or unsubstituted aroyl, substituted or unsubstituted heteroaroyl, substituted or unsubstituted lower alkylsulfonyl, substituted or unsubstituted arylsulfonyl, or -CONR¹³R¹⁴ (wherein R¹³ and R¹⁴ are the same as R⁹ and R¹⁰ defined above, respectively)]};
   Q² represents an oxygen atom, a sulfur atom, or -NR¹⁵- [wherein R¹⁵ represents a hydrogen atom, substituted or unsubstituted lower alkyl, substituted or unsubstituted cycloalkyl, substituted or unsubstituted aralkyl, substituted or unsubstituted lower alkenyl, substituted or unsubstituted aryl, a substituted or unsubstituted heterocyclic group, substituted or unsubstituted lower alkanoyl, substituted or unsubstituted aroyl, substituted or unsubstituted heteroaroyl, substituted or unsubstituted lower alkoxycarbonyl, substituted or unsubstituted lower alkylsulfonyl, substituted or unsubstituted arylsulfonyl, or -CONR¹⁶R¹⁷ (wherein R¹⁶ and R¹⁷ are the same as R⁹ and R¹⁰ defined above, respectively)]; and
   R⁵ and R⁶ may be the same or different and each represents a hydrogen atom, halogen, nitro, hydroxy, cyano, carboxy, substituted or unsubstituted lower alkyl, substituted or unsubstituted cycloalkyl, substituted or unsubstituted aralkyl, substituted or unsubstituted lower alkenyl, substituted or unsubstituted lower alkynyl, substituted or unsubstituted aryl, a substituted or unsubstituted heterocyclic group, substituted or unsubstituted lower alkanoyl, substituted or unsubstituted lower alkoxycarbonyl, substituted or unsubstituted aroyl, substituted or unsubstituted heteroaroyl, -S(O)m¹R¹⁸ [wherein m¹ represents an integer of 0 to 2, R¹⁸ represents a hydrogen atom, hydroxy, substituted or unsubstituted lower alkoxy, substituted or unsubstituted lower alkyl, substituted or unsubstituted cycloalkyl, substituted or unsubstituted aralkyl, substituted or unsubstituted lower alkenyl, substituted or unsubstituted aryl, a substituted or unsubstituted heterocyclic group, or -NR¹⁹R²⁰ (wherein R¹⁹ and R²⁰ are the same as R⁹ and R¹⁰ defined above, respectively)], -OR²¹ [wherein R²¹ represents substituted or unsubstituted lower alkyl, substituted or unsubstituted cycloalkyl, substituted or unsubstituted aralkyl, substituted or unsubstituted lower alkenyl, substituted or unsubstituted aryl, a substituted or unsubstituted heterocyclic group, substituted or unsubstituted lower alkanoyl, substituted or unsubstituted aroyl, substituted or unsubstituted heteroaroyl, -CONR²²R²³ (wherein R²² and R²³ are the same as R⁹ and R¹⁰ defined above, respectively), or -S(O)m²R²⁴ (wherein m² is the same as m¹ defined above, and R²⁴ is the same as R¹⁸ defined above)], -CONR²⁵R²⁶ (wherein R²⁵ and R²⁶ are the same as R⁹ and R¹⁰ defined above, respectively), or -NR²⁷R²⁸ (wherein R²⁷ and R²⁸ are the same as R¹¹ and R¹² defined above, respectively)], or 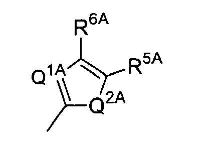
   (wherein Q^{1A}, Q^{2A}, R^{5A} and R^{6A} are the same as Q¹, Q², R⁵ and R⁶ defined above, respectively); and
   R² and R³ may be the same or different and each represents a hydrogen atom, halogen, nitro, hydroxy, cyano, carboxy, substituted or unsubstituted lower alkyl, substituted or unsubstituted cycloalkyl, substituted or unsubstituted aralkyl, substituted or unsubstituted lower alkenyl, substituted or unsubstituted lower alkynyl, substituted or unsubstituted aryl, a substituted or unsubstituted heterocyclic group, substituted or unsubstituted lower alkanoyl, substituted or unsubstituted lower alkoxycarbonyl, substituted or unsubstituted aroyl, substituted or unsubstituted heteroaroyl, -S(O)m³R²⁹ (wherein m³ is the same as m¹ defined above, and R²⁹ is the same as R¹⁸ defined above), -OR³⁰ (wherein R³⁰ is the same as R²¹ defined above), -CONR³¹R³² (wherein R³¹ and R³² are the same as R⁹ and R¹⁰ defined above, respectively), or -NR³³R³⁴ (wherein R³³ and R³⁴ are the same as R¹¹ and R¹² defined above, respectively)> or a pharmaceutically acceptable salt thereof.
(2) The JAK inhibitor according to above (1), wherein R¹ is 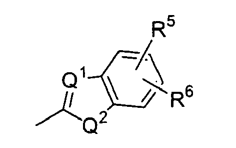
   (wherein Q¹, Q², R⁵ and R⁶ are the same as those defined above, respectively).
(3) The JAK inhibitor according to above (2), wherein Q¹ is -CH-.
(4) The JAK inhibitor according to above (2) or (3), wherein Q² is -NR¹⁵- (wherein R¹⁵ is the same as that defined above).
(5) The JAK inhibitor according to above (2) or (3), wherein Q² is -NH-.
(6) The JAK inhibitor according to any one of above (2) to (5), wherein R⁶ is a hydrogen atom, and R⁵ is substituted or unsubstituted lower alkyl or -CONR²⁵R²⁶ (wherein R²⁵ and R²⁶ are the same as those defined above, respectively).
(7) The JAK inhibitor according to any one of above (2) to (5), wherein R⁶ is a hydrogen atom, and R⁵ is -S(O)ₘ¹R¹⁸ (wherein m¹ and R¹⁸ are the same as those defined above, respectively), -OR²¹ (wherein R²¹ is the same as that defined above), or -NR²⁷R²⁸ (wherein R²⁷ and R²⁸ are the same as those defined above, respectively).
(8) The JAK inhibitor according to any one of above (2) to (5), wherein R⁶ is a hydrogen atom, and R⁵ is heteroalicyclic-lower alkyl or heteroalicyclic-lower alkyl substituted with hydroxy-lower alkyl.
(9) The JAK inhibitor according to any one of above (2) to (5), wherein R⁶ is a hydrogen atom, and R⁵ is [mono or di (lower alkyl)amino] lower alkyl or (hydroxy-lower alkylamino)lower alkyl.
(10) The JAK inhibitor according to any one of above (1) to (9), wherein R² is a hydrogen atom, and R³ is halogen, hydroxy, substituted or unsubstituted aryl, a substituted or unsubstituted heterocyclic group, or -OR³⁰ (wherein R³⁰ is the same as that defined above).
(11) The JAK inhibitor according to any one of above (1) to (9), wherein
   R² is halogen, hydroxy, unsubstituted lower alkyl, unsubstituted lower alkenyl, unsubstituted lower alkynyl, substituted or unsubstituted aryl, a substituted or unsubstituted aromatic heterocyclic group, or -OR^{30a} (wherein R^{30a} is the same as R³⁰ defined above), and
   R³ is halogen, hydroxy, substituted or unsubstituted aryl, a substituted or unsubstituted heterocyclic group, or -OR^{30b} (wherein R^{30b} is the same as R³⁰ defined above).
(12) The JAK inhibitor according to any one of above (1) to (9), wherein
   R² is substituted or unsubstituted lower alkoxy, and
   R³ is lower alkylsulfonyloxy.
(13) The JAK inhibitor according to any one of above (1) to (12), wherein X is -(C=O)-.
(14) The JAK inhibitor according to any one of above (1) to (12), wherein X is -CHR⁴- (wherein R⁴ is the same as that defined above).
(15) The JAK inhibitor according to any one of above (1) to (14), wherein W is a nitrogen atom.
(16) The Janus kinase (JAK) inhibitor according to any one of above (1) to (14), wherein W is -CH-.
(17) A therapeutic agent for a disease associated with JAK comprising, as an active ingredient, the nitrogen-containing heterocyclic compound or the pharmaceutically acceptable salt thereof described in any one of above (1) to (16).
(18) A therapeutic agent for a myeloproliferative disorder comprising, as an active ingredient, the nitrogen-containing heterocyclic compound or the pharmaceutically acceptable salt thereof described in any one of above (1) to (16).
(19) A therapeutic agent for a immunological disorder comprising, as an active ingredient, the nitrogen-containing heterocyclic compound or the pharmaceutically acceptable salt thereof described in any one of above (1) to (16).
(20) A nitrogen-containing heterocyclic compound represented by formula (II): 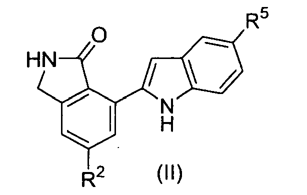 (wherein R² and R⁵ are the same as those defined above, respectively) or a pharmaceutically acceptable salt thereof.
(21) The nitrogen-containing heterocyclic compound or the pharmaceutically acceptable salt thereof according to above (20), wherein R² is halogen, hydroxy, substituted or unsubstituted lower alkoxy, substituted or unsubstituted lower alkylsulfonyl, or mono- or di-(substituted or unsubstituted lower alkyl)amino.
(22) The nitrogen-containing heterocyclic compound or the pharmaceutically acceptable salt thereof according to above (20), wherein R² is hydroxy-lower alkyl, lower alkoxy-lower alkyl, [mono- or di- (lower alkyl) amino] lower alkyl or substituted or unsubstituted lower alkanoyl.
(23) The nitrogen-containing heterocyclic compound or the pharmaceutically acceptable salt thereof according to above (20), wherein R² is substituted or unsubstituted aryl or a substituted or unsubstituted heterocyclic group.
(24) The nitrogen-containing heterocyclic compound or the pharmaceutically acceptable salt thereof according to above (24), wherein R² is substituted or unsubstituted lower alkenyl or substituted or unsubstituted lower alkynyl.
(25) The nitrogen-containing heterocyclic compound or the pharmaceutically acceptable salt thereof according to any one of above (20) to (23), wherein R⁵ is [mono- or di-(lower alkyl) amino] lower alkyl, heteroalicyclic-lower alkyl, or heteroalicyclic-lower alkyl substituted with hydroxy-lower alkyl.
(26) A nitrogen-containing heterocyclic compound represented by formula (III): 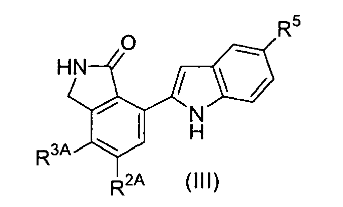
   [wherein R⁵ is the same as that defined above,
   R^{2A} represents substituted or unsubstituted lower alkyl, -OR³⁰ (wherein R³⁰ is the same as that defined above), or -NR³³R³⁴ (wherein R³³ and R³⁴ are the same as those defined above, respectively), and R^{3A} represents halogen, hydroxy, substituted or unsubstituted lower alkoxy, substituted or unsubstituted lower alkylsulfonyl, or -OS(O)₂R^{30A} (wherein R^{30A} represents amino, substituted or unsubstituted lower alkyl, substituted or unsubstituted aryl, or a substituted or unsubstituted aromatic heterocyclic group)] or a pharmaceutically acceptable salt thereof.
(27) The nitrogen-containing heterocyclic compound or the pharmaceutically acceptable salt thereof according to above (26), wherein R⁵ is substituted or unsubstituted heteroalicyclic-lower alkyl or [mono- or di-(substituted or unsubstituted lower alkyl)amino] lower alkyl.
(28) A nitrogen-containing heterocyclic compound represented by formula (IV): 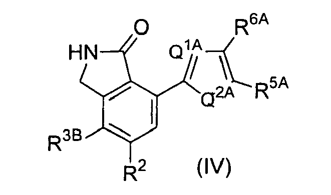 (wherein R², R^{5A}, R^{6A}, Q^{1A} and Q^{2A} are the same as those defined above, respectively, and R^{3B} represents a hydrogen atom, hydroxy, or halogen) or a pharmaceutically acceptable salt thereof.
(29) The nitrogen-containing heterocyclic compound or the pharmaceutically acceptable salt thereof according to above (28), wherein Q^{1A} is -CH-, and
   Q^{2A} is -NR^{15A}- (wherein R^{15A} represents a hydrogen atom or substituted or unsubstituted lower alkyl).
(30) The nitrogen-containing heterocyclic compound or the pharmaceutically acceptable salt thereof according to above (29), wherein R^{15A} is a hydrogen atom.
(31) The nitrogen-containing heterocyclic compound or the pharmaceutically acceptable salt thereof according to any one of above (28) to (30), wherein R^{5A} is a hydrogen atom, substituted or unsubstituted lower alkyl, or substituted or unsubstituted lower alkanoyl.
(32) The nitrogen-containing heterocyclic compound or the pharmaceutically acceptable salt thereof according to any one of above (28) to (31), wherein R^{6A} is a hydrogen atom.
(33) The nitrogen-containing heterocyclic compound or the pharmaceutically acceptable salt thereof according to any one of above (28) to (32), wherein R^{3B} is a hydrogen atom.
(34) A nitrogen-containing heterocyclic compound represented by formula (V): 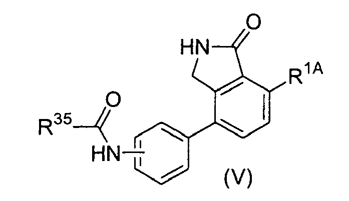
   [wherein R^{1A} represents substituted or unsubstituted aryl or substituted or unsubstituted thienyl, and
   R³⁵ represents aryl, aryl substituted with 1 to 3 subsituent(s) selected from Substituent Group A described below (Substituent Group A: halogen, lower alkyl, halogen-lower alkyl), or NHR^{35a} (wherein R^{35a} represents aryl or aryl substituted with 1 to 3 subsituent(s) selected from Substituent Group A described above)] or a pharmaceutically acceptable salt thereof.
(35) A nitrogen-containing heterocyclic compound represented by formula (VI): 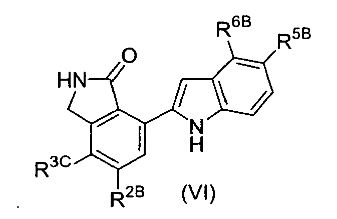
   (wherein R^{2B} represents substituted or unsubstituted lower alkoxy, substituted or unsubstituted heteroalicyclic-lower alkyl, or [mono- or di- (substituted or unsubstituted lower alkyl) amino] lower alkyl, R^{3C} represents a hydrogen atom or halogen,
   R^{5B} represents a hydrogen atom, substituted or unsubstituted heteroalicyclic-lower alkyl, or [mono- or di-(substituted or unsubstituted lower alkyl)amino]lower alkyl,
   R^{6B} represents hydroxy, substituted or unsubstituted lower alkoxy, or substituted or unsubstituted lower alkylsulfonyloxy} or a pharmaceutically acceptable salt thereof.
(36) The nitrogen-containing heterocyclic compound or the pharmaceutically acceptable salt thereof according to above (35), wherein R^{2B} is [di(lower alkyl)amino]lower alkyl, and R^{3C} and R^{5B} are hydrogen atoms.
(37) The nitrogen-containing heterocyclic compound or the pharmaceutically acceptable salt thereof according to above (35), wherein R^{2B} is lower alkoxy,
   R^{3C} is halogen, and
   R^{5B} is substituted or unsubstituted heteroalicyclic-lower alkyl.
(38) A pharmaceutical composition comprising, as an active ingredient, the nitrogen-containing heterocyclic compound or the pharmaceutically acceptable salt thereof described in any one of above (20) to (37).
(39) A protein kinase inhibitor comprising, as an active ingredient, the nitrogen-containing heterocyclic compound or the pharmaceutically acceptable salt thereof described in any one of above (20) to (37).
(40) A JAK inhibitor comprising, as an active ingredient, the nitrogen-containing heterocyclic compound or the pharmaceutically acceptable salt thereof described in any one of above (20) to (37).
(41) A therapeutic agent for a disease associated with JAK comprising, as an active ingredient, the nitrogen-containing heterocyclic compound or the pharmaceutically acceptable salt thereof described in any one of above (20) to (37).
(42) An antitumor agent comprising, as an active ingredient, the nitrogen-containing heterocyclic compound or the pharmaceutically acceptable salt thereof described in any one of above (20) to (37).
(43) A therapeutic agent for a hematopoietic tumor comprising, as an active ingredient, the nitrogen-containing heterocyclic compound or the pharmaceutically acceptable salt thereof described in any one of above (20) to (37).
(44) A therapeutic agent for a myeloproliferative disorder comprising, as an active ingredient, the nitrogen-containing heterocyclic compound or the pharmaceutically acceptable salt thereof described in any one of above (20) to (37).
(45) A therapeutic agent for an immunological disorder comprising, as an active ingredient, the nitrogen-containing heterocyclic compound or the pharmaceutically acceptable salt thereof described in any one of above (20) to (37).
(46) A method for inhibiting JAK comprising a step of administering an effective amount of the nitrogen-containing heterocyclic compound or the pharmaceutically acceptable salt thereof described in any one of above (1) to (16).
(47) A method for treating a disease associated with JAK comprising a step of administering an effective amount of the nitrogen-containing heterocyclic compound or the pharmaceutically acceptable salt thereof described in any one of above (1) to (16).
(48) A method for treating a myeloproliferative disorder comprising a step of administering an effective amount of the nitrogen-containing heterocyclic compound or the pharmaceutically acceptable salt thereof described in any one of above (1) to (16).
(49) A method for treating an immunological disorder comprising a step of administering an effective amount of the nitrogen-containing heterocyclic compound or the pharmaceutically acceptable salt thereof described in any one of above (1) to (16).
(50) A method for inhibiting a protein kinase comprising a step of administering an effective amount of the nitrogen-containing heterocyclic compound or the pharmaceutically acceptable salt thereof described in any one of above (20) to (37).
(51) A method for inhibiting JAK comprising a step of administering an effective amount of the nitrogen-containing heterocyclic compound or the pharmaceutically acceptable salt thereof described in any one of above (20) to (37).
(52) A method for treating a disease associated with JAK comprising a step of administering an effective amount of the nitrogen-containing heterocyclic compound or the pharmaceutically acceptable salt thereof described in any one of above (20) to (37).
(53) A method for treating a tumor comprising a step of administering an effective amount of the nitrogen-containing heterocyclic compound or the pharmaceutically acceptable salt thereof described in any one of above (20) to (37).
(54) A method for treating a hematopoietic tumor comprising a step of administering an effective amount of the nitrogen-containing heterocyclic compound or the pharmaceutically acceptable salt thereof described in any one of above (20) to (37).
(55) A method for treating a myeloproliferative disorder comprising a step of administering an effective amount of the nitrogen-containing heterocyclic compound or the pharmaceutically acceptable salt thereof described in any one of above (20) to (37).
(56) A method for treating an immunological disorder comprising a step of administering an effective amount of the nitrogen-containing heterocyclic compound or the pharmaceutically acceptable salt thereof described in any one of above (20) to (37).
(57) Use of the nitrogen-containing heterocyclic compound or the pharmaceutically acceptable salt thereof described in any one of above (1) to (16) for the manufacture of a JAK inhibitor.
(58) Use of the nitrogen-containing heterocyclic compound or the pharmaceutically acceptable salt thereof described in any one of above (1) to (16) for the manufacture of a therapeutic agent for a disease associated with JAK.
(59) Use of the nitrogen-containing heterocyclic compound or the pharmaceutically acceptable salt thereof described in any one of above (1) to (16) for the manufacture of a therapeutic agent for a myeloproliferative disorder.
(60) Use of the nitrogen-containing heterocyclic compound or the pharmaceutically acceptable salt thereof described in any one of above (1) to (16) for the manufacture of a therapeutic agent for an immunological disorder.
(61) Use of the nitrogen-containing heterocyclic compound or the pharmaceutically acceptable salt thereof described in any one of above (20) to (37) for the manufacture of a protein kinase inhibitor.
(62) Use of the nitrogen-containing heterocyclic compound or the pharmaceutically acceptable salt thereof described in anyone of above (20) to (37) for the manufacture of a JAK inhibitor.
(63) Use of the nitrogen-containing heterocyclic compound or the pharmaceutically acceptable salt thereof described in any one of above (20) to (37) for the manufacture of a therapeutic agent for a disease associated with JAK.
(64) Use of the nitrogen-containing heterocyclic compound or the pharmaceutically acceptable salt thereof described in any one of above (20) to (37) for the manufacture of an antitumor agent.
(65) Use of the nitrogen-containing heterocyclic compound or the pharmaceutically acceptable salt thereof described in any one of above (20) to (37) for the manufacture of a therapeutic agent for a hematopoietic tumor.
(66) Use of the nitrogen-containing heterocyclic compound or the pharmaceutically acceptable salt thereof described in any one of above (20) to (37) for the manufacture of a therapeutic agent for a myeloproliferative disorder.
(67) Use of the nitrogen-containing heterocyclic compound or the pharmaceutically acceptable salt thereof described in any one of above (20) to (37) for the manufacture of a therapeutic agent for an immunological disorder.

### Effect of the Invention

The present invention provides a JAK inhibitor which comprises, as an active ingredient, a nitrogen-containing heterocyclic compound or a pharmaceutically acceptable salt thereof, and the like.

### Best Mode for Carrying Out the Invention

Hereinafter, compounds represented by formula (I), (II), (III), (IV), (V) and (VI) are referred to as Compound (I), (II), (III), (IV), (V) and (VI). The same shall apply to the compounds of the other formula numbers.

Each of the groups of formula (I), (II), (III), (IV), (V) and (VI) are defined as follows.
(i) The halogen and the halogen moiety of halogen-lower alkyl include each atoms of fluorine, chlorine, bromine, and iodine.
(ii) Examples of the lower alkyl, and the lower alkyl moieties of the lower alkoxy, the lower alkoxycarbonyl, the lower alkylsulfonyl, the lower alkylsulfonyloxy, the lower alkoxy-lower alkyl, mono- or di-(lower alkyl)amino, and [mono- or di-(lower alkyl)amino]lower alkyl include linear or branched alkyl having 1 to 10 carbons. More specific examples thereof include methyl, ethyl, n-propyl, isopropyl, n-butyl, isobutyl, sec-butyl, tert-butyl, n-pentyl, neopentyl, n-hexyl, n-heptyl, n-octyl, n-nonyl, n-decyl and the like. The two lower alkyl moieties of di(lower alkyl)amino and [di(lower alkyl)amino]lower alkyl may be the same or different.
(iii) Examples of the cycloalkyl include cycloalkyl having 1 to 10 carbons. More specific examples thereof include cyclopropyl, cyclobutyl, cyclopentyl, cyclohexyl, cycloheptyl, cyclooctyl, cyclodecyl, noradamantyl, adamantyl, bicyclo[2.2.1]heptyl, bicyclo[2.2.2]octyl, bicyclo[3.3.0]octyl, bicyclo[3.3.1]nonyl and the like.
(iv) Examples of the lower alkenyl include linear or branched alkenyl having 2 to 10 carbons. More specific examples thereof include vinyl, allyl, 1-propenyl, 1-butenyl, 3-butenyl, 2-pentenyl, 4-pentenyl, 2-hexenyl, 5-hexenyl, 2-decenyl, 9-decenyl and the like.
(v) Examples of the lower alkynyl include linear or branched alkynyl having 2 to 10 carbons. More specific examples thereof include ethynyl, 2-propynyl, 3-butynyl, 4-pentynyl, 5-hexynyl, 9-decynyl and the like.
(vi) The alkylene moieties of the aralkyl, hydroxy-lower alkyl, halogen-lower alkyl, (hydroxy-lower alkylamino)lower alkyl, heteroalicyclic-lower alkyl, [mono- or di- (lower alkyl)amino]lower alkyl, lower alkoxy-lower alkyl, and heterocyclic-lower alkyl are the same as moieties produced by removing one hydrogen atom from the lower alkyl (ii) defined above.
(vii) Examples of the aryl, and the aryl moieties of the aroyl, the arylsulfonyl, and the aralkyl include monocyclic aryl and fused aryl in which two or more rings are fused. More specific examples thereof include aryl having 6 to 14 carbon atoms that constitute the ring, such as phenyl, naphthyl, indenyl and anthranyl.
(viii) Examples of the lower alkanoyl include linear or branched lower alkanoyl having 1 to 8 carbons. More specific examples thereof include formyl, acetyl, propionyl, butyryl, isobutyryl, valeryl, isovaleryl, pivaloyl, hexanoyl, heptanoyl, octanoyl and the like.
(ix) Examples of the heterocyclic group include an aromatic heterocyclic group, a heteroalicyclic group and the like.
   Examples of the aromatic heterocyclic group include monocyclic aromatic heterocyclic groups and fused aromatic heterocyclic groups in which two or more rings are fused. The type and number of heteroatoms contained in the aromatic heterocyclic group are not particularly limited. For example, the aromatic heterocyclic group may contain at least one heteroatom selected from the group consisting of a nitrogen atom, a sulfur atom, and an oxygen atom. More specific examples thereof include aromatic heterocyclic groups having 5 to 14 annular atoms such as furyl, thienyl, pyrrolyl, imidazolyl, pyrazolyl, triazolyl, tetrazolyl, oxazolyl, oxadiazolyl, thiazolyl, pyridyl, pyrazinyl, pyrimidinyl, pyridazinyl, triazinyl, indolyl, indazolyl, benzimidazolyl, benzofuryl, benzothienyl, benzoxazolyl, benzothiazolyl, quinolyl, isoquinolyl, phthalazinyl, naphthyridinyl, quinoxalinyl, quinazolinyl, cinnolinyl, purinyl, and coumarinyl.
   Examples of the heteroalicyclic group include monocyclic heteroalicyclic groups and fused heteroalicyclic groups in which two or more rings are fused. The type and number of heteroatoms contained in the heteroalicyclic group are not particularly limited. For example, the heteroalicyclic group may contain at least one heteroatom selected from the group consisting of a nitrogen atom, a sulfur atom, and an oxygen atom. More specific examples thereof include heteroalicyclic groups having 3 to 14 annular atoms such as pyrrolidinyl, thiazolidinyl, oxazolidinyl, piperidyl, azepanyl, 1,2-dihydropyridyl, piperazinyl, homopiperazinyl, morpholinyl, thiomorpholinyl, pyrazolinyl, oxazolinyl, dioxolanyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolyl, and soisoindolinyl.
   The heteroalicyclic moiety of the heteroalicyclic-lower alkyl is the same as the heteroalicyclic group defined above.
(x) Examples of the heterocyclic group formed together with the adjacent nitrogen atom thereto include five- or six-membered monocyclic heteroalicyclic groups containing at least one nitrogen atom (wherein the monocyclic heteroalicyclic group may further contain another nitrogen atom, an oxygen atom, or a sulfur atom), bicyclic or tricyclic fused heteroalicyclic group containing at least one nitrogen atom in which three- to eight-membered rings are fused (wherein the fused heteroalicyclic group may further contain another nitrogen atom, an oxygen atom, or a sulfur atom) and the like. More specific examples thereof include monocyclic heteroalicyclic groups and fused heteroalicyclic groups having 3 to 14 annular atoms such as pyrrolidinyl, piperidino, piperazinyl, morpholino, thiomorpholino, homopiperidino, homopiperazinyl, tetrahydropyridyl, tetrahydroquinolyl, and tetrahydroisoquinolyl.
(xi) Examples of the heteroaryl moiety of the heteroaroyl include furyl, thienyl, pyrrolyl, imidazolyl, pyrazolyl, triazolyl, tetrazolyl, oxazolyl, oxadiazolyl, thiazolyl, pyridyl, pyrazinyl, pyrimidinyl, pyridazinyl, triazinyl, indolyl, indazolyl, benzimidazolyl, benzoxazolyl, benzothiazolyl, quinolyl, isoquinolyl, phthalazinyl, naphthyridinyl, quinoxalinyl, quinazolinyl, cinnolinyl, purinyl, coumarinyl and the like.
(xii) Examples of the substituents in the substituted lower alkyl, the substituted lower alkoxy, the substituted lower alkylsulfonyl, the substituted lower alkylsulfonyloxy, the substituted cycloalkyl, the substituted lower alkenyl, the substituted lower alkynyl, the substituted lower alkoxycarbonyl, the substituted lower alkanoyl, mono- or di-(substituted lower alkyl)amino and [mono- or di- (substituted lower alkyl) amino] lower alkyl which may be the same or different and which are 1 to 3 in number, include
   (xii-a) halogen;
   (xii-b) hydroxy;
   (xii-c) oxo;
   (xii-d) cyano;
   (xii-e) carboxy;
   (xii-f) lower alkoxycarbonyl;
   (xii-g) arylsulfonyl;
   (xii-h) heteroaroyl;
   (xii-i) substituted or unsubstituted cycloalkyl [examples of the substituent in the substituted cycloalkyl, which is 1 to 3 in number, include halogen, hydroxy, lower alkoxy, substituted or unsubstituted lower alkyl (examples of the substituent in the substituted lower alkyl, which is 1 to 3 in number, include halogen, hydroxy, cyano, lower alkoxy and the like), and the like];
   (xii-j) substituted or unsubstituted lower alkoxy (examples of the substituent in the substituted lower alkoxy, which is 1 to 3 in number, include halogen, hydroxy, lower alkoxy and the like); (xii-k) substituted or unsubstituted aryl (examples of the substituent in the substituted aryl, which is 1 to 3 in number, include carboxy, lower alkoxycarbonyl and the like);
   (xii-l) substituted or unsubstituted heterocyclic groups {examples of the substituent in the substituted heterocyclic groups, which is 1 to 3 in number, include halogen, hydroxy, oxo, lower alkoxy, lower alkoxycarbonyl, lower alkylsulfonyl, lower alkanoyl, substituted or unsubstituted lower alkyl [examples of the substituent in the substituted lower alkyl, which is 1 to 3 in number, include halogen, hydroxy, cyano, substituted or unsubstituted lower alkoxy (examples of the substituent in the substituted lower alkoxy, which is 1 to 3 in number, include halogen, hydroxy and the like) and the like], substituted or unsubstituted aryl (examples of the substituent in the substituted aryl, which is 1 to 3 in number, include halogen, hydroxy, cyano, lower alkyl, lower alkoxy and the like), a substituted or unsubstituted heterocyclic group (examples of the substituent in the substituted heterocyclic group, which is 1 to 3 in number, include halogen, hydroxy, cyano, lower alkyl, lower alkoxy and the like) and the like};
   (xii-m) NR³⁶R³⁷ {wherein R³⁶ and R³⁷ may be the same or different and each represents a hydrogen atom, lower alkoxycarbonyl, lower alkenyl, lower alkynyl, lower alkanoyl, substituted or unsubstituted lower alkyl [examples of the substituent in the substituted lower alkyl, which is 1 to 3 in number, include halogen, amino, hydroxy, carboxy, carbamoyl, lower alkanoyl, substituted or unsubstituted lower alkoxy (examples of the substituent in the substituted lower alkoxy, which is 1 to 3 in number, include halogen, hydroxy and the like), lower alkoxycarbonyl, substituted or unsubstituted cycloalkyl [examples of the substituent in the substituted cycloalkyl, which is 1 to 3 in number, include halogen, hydroxy, substituted or unsubstituted lower alkyl (examples of the substituent in the substituted lower alkyl, which is 1 to 3 in number, include halogen, hydroxy and the like) and the like], mono- or di-lower alkylamino, a heterocyclic group and the like], substituted or unsubstituted cycloalkyl (examples of the substituent in the substituted cycloalkyl, which is 1 to 3 in number, include halogen, amino, hydroxy and the like), substituted or unsubstituted aralkyl (examples of the substituent in the substituted aralkyl, which is 1 to 3 in number, include halogen, hydroxy, cyano, lower alkoxy and the like), substituted or unsubstituted aryl [examples of the substituent in the substituted aryl, which is 1 to 3 in number, include halogen, amino, hydroxy, substituted or unsubstituted lower alkyl (examples of the substituent in the substituted lower alkyl, which is 1 to 3 in number, include halogen, hydroxy and the like), a substituted or unsubstituted heterocyclic group (examples of the substituent in the substituted heterocyclic group, which is 1 to 3 in number, include halogen, hydroxy, lower alkyl, lower alkoxy and the like) and the like], a substituted or unsubstituted heterocyclic group [examples of the substituent in the substituted heterocyclic group, which is 1 to 3 in number, include halogen, amino, hydroxy, substituted or unsubstituted lower alkyl (examples of the substituent in the substituted lower alkyl, which is 1 to 3 in number, include halogen, hydroxy and the like), or a substituted or unsubstituted heterocyclic group (examples of the substituent in the substituted heterocyclic group, which is 1 to 3 in number, include halogen, hydroxy, lower alkyl, lower alkoxy and the like) and the like];
   (xii-n) CONR³⁸R³⁹ {wherein R³⁸ and R³⁹ may be the same or different and each represents a hydrogen atom, substituted or unsubstituted lower alkyl [examples of the substituent in the substituted lower alkyl, which is 1 to 3 in number, include halogen, amino, hydroxy, carboxy, carbamoyl, substituted or unsubstituted lower alkoxy (examples of the substituent in the substituted lower alkoxy, which is 1 to 3 in number, include halogen, hydroxy and the like), lower alkoxycarbonyl, substituted or unsubstituted cycloalkyl (examples of the substituent in the substituted cycloalkyl, which is 1 to 3 in number, include halogen, hydroxy and the like), mono- or di-(lower alkyl)amino, a heterocyclic group and the like], substituted or unsubstituted cycloalkyl (examples of the substituent in the substituted cycloalkyl, which is 1 to 3 in number, include halogen, amino, hydroxy and the like), or lower alkanoyl, or R³⁸ and R³⁹ are combined together with the adjacent nitrogen atom thereto to form a heterocyclic group} and the like.

   In the above (xii), the halogen is the same as that defined in (i) above; the lower alkyl, and the lower alkyl moieties of the lower alkoxy, the lower alkoxycarbonyl, and the lower alkylsulfonyl are the same as those defined in (ii) above; the cycloalkyl is the same as that defined in (iii) above; the lower alkenyl is the same as that defined in (iv) above; the lower alkynyl is the same as that defined in (v) above; the alkylene moiety of the aralkyl is the same as that defined in (vi) above; the aryl, and the aryl moieties of the arylsulfonyl, and the aralkyl are the same as those defined in (vii) above; the lower alkanoyl is the same as that defined in (viii) above; the heterocyclic groups are the same as those defined in (ix) above; the heterocyclic groups formed together with the adjacent nitrogen atom thereto are the same as those defined in (x) above; and the heteroaryl moiety in the heteroaroyl is the same as that defined in (xi) above. The lower alkyl moiety of the mono- or di-(lower alkyl)amino is the same as that defined in (i) above, and the two lower alkyl moieties of the di-(lower alkyl)amino may be the same or different.
(xiii) Examples of the substituents in the substituted aryl, the substituted aroyl, the substituted aralkyl, the substituted arylsulfonyl, the substituted heteroaroyl, the substituted heterocyclic group, the substituted aromatic heterocyclic group, the substituted thienyl, the substituted heteroalicyclic-lower alkyl, and the substituted heterocyclic group formed together with the adjacent nitrogen atom thereto which may be the same or different and which are 1 to 3 in number, include
   (xiii-a) halogen;
   (xiii-b) hydroxy;
   (xiii-c) nitro;
   (xiii-d) cyano;
   (xiii-e) formyl;
   (xiii-f) carboxy;
   (xiii-g) lower alkoxycarbonyl;
   (xiii-h) aralkyloxy;
   (xiii-i) substituted or unsubstituted lower alkyl [the substituents in the substituted lower alkyl are the same as those defined in (xii) above];
   (xiii-j) substituted or unsubstituted lower alkoxy [the substituents in the substituted lower alkoxy are the same as those defined in (xii) above];
   (xiii-k) substituted or unsubstituted lower alkanoyl [the substituents in the substituted lower alkanoyl are the same as those defined in (xii) above];
   (xiii-1) substituted or unsubstituted lower alkylsulfonyl [the substituents in the substituted lower alkylsulfonyl are the same as those defined in (xii) above];
   (xiii-m) substituted or unsubstituted aroyl [examples of the substituent in the substituted aroyl, which is 1 to 3 in number, include halogen, nitro, hydroxy, cyano, carboxy, lower alkanoyl, lower alkoxycarbonyl, aralkyl, aroyl, substituted or unsubstituted lower alkyl (examples of the substituent in the substituted lower alkyl, which is 1 to 3 in number, include hydroxy and the like), and substituted or unsubstituted lower alkoxy (examples of the substituent in the substituted lower alkoxy, which is 1 to 3 in number, include hydroxy and the like) and the like];
   (xiii-n) substituted or unsubstituted heteroaroyl [the substituents in the substituted heteroaroyl are the same as the substituents defined in the substituted aroyl (xiii-m) above]; (xiii-o) substituted or unsubstituted heterocyclic groups [the substituents in the substituted heterocyclic groups are the same as the substituents defined in the substituted aroyl (xiii-m) above]; (xiii-p) NR⁴⁰R⁴¹ {wherein R⁴⁰ and R⁴¹ may be the same or different and each represents a hydrogen atom, substituted or unsubstituted lower alkyl [the substituents in the substituted lower alkyl are the same as those defined in (xii) above], substituted or unsubstituted lower alkanoyl [the substituents in the substituted lower alkanoyl are the same as those defined in (xii) above], substituted or unsubstituted aryl [the substituents in the substituted aryl are the same as the substituents defined in the substituted aroyl (xiii-m) above], or substituted or unsubstituted aroyl [the substituents in the substituted aroyl are the same as the substituents defined in the substituted aroyl (xiii-m) above]};
   (xiii-q) CONR⁴²R⁴³ {wherein R⁴² and R⁴³ may be the same or different and each represents a hydrogen atom, substituted or unsubstituted lower alkyl [the substituents in the substituted lower alkyl are the same as those defined (xii) above], substituted or unsubstituted aryl [the substituents in the substituted aryl are the same as the substituents defined in the substituted aroyl (xiii-m) above], or substituted or unsubstituted aroyl [the substituents in the substituted aroyl are the same as the substituents defined in the substituted aroyl (xiii-m) above], or R⁴² and R⁴³ are combined together with the adjacent nitrogen atom thereto to form a substituted or unsubstituted heterocyclic group [the substituents in the substituted heterocyclic group formed together with the adjacent nitrogen atom thereto are the same as the substituents defined in the substituted aroyl (xiii-m) above]} and the like.
      Examples of the substituents in the substituted heterocyclic group and the substituted heterocyclic group formed together with the adjacent nitrogen atom thereto may include substituents defined in (xiii-r) and (xiii-s) below in addition to the substituents defined in (xiii-a) to (xiii-q) above:
   (xiii-r) oxo; and
   (xiii-s) -O(CR⁴⁴R⁴⁵)ₙO- (wherein R⁴⁴ and R⁴⁵ may be the same or different and each represents a hydrogen atom, lower alkyl, or the like; n represents 2 or 3; and the two terminal oxygen atoms bond to a same carbon atom of a substituted heterocyclic group or a substituted heterocyclic group formed together with the adjacent nitrogen atom).

In the above (xiii), the halogen is the same as that defined in (i) above; the lower alkyl, and the lower alkyl moieties of the lower alkoxy, the lower alkoxycarbonyl, and the lower alkylsulfonyl are the same as those defined in (ii) above; the alkylene moieties of the aralkyl and the aralkyloxy are the same as those defined in (vi) above; the aryl, and the aryl moieties of the aralkyl, the aralkyloxy, and the aroyl are the same as those defined in (vii) above; the lower alkanoyl is the same as that defined in (viii) above; the heterocyclic groups are the same as those defined in (ix) above; the heterocyclic groups formed together with the adjacent nitrogen atom thereto are the same as those defined in (x) above; and the heteroaryl moiety in the heteroaroyl is the same as that defined in (xi) above.

Examples of pharmaceutically acceptable salts of Compounds (I), (II), (III), (IV), (V) and (VI) include pharmaceutically acceptable acid addition salts, metal salts, ammonium salts, organic amine addition salts, and amino acid addition salts and the like. Examples of the acid addition salts include inorganic acid salts such as hydrochlorides, sulfates, and phosphates; and organic acid salts such as acetates, trifluoroacetates, maleates, fumarates, tartrates, citrates, lactates, aspartates, and glutamates. Examples of the metal salts include alkali metal salts such as sodium salts and potassium salts; alkaline earth metal salts such as magnesium salts and calcium salts; aluminum salts; zinc salts; and the like. Examples of the ammonium salts include salts of ammonium, tetramethylammonium, or the like. Examples of the organic amine addition salts include addition salts of morpholine, piperidine, or the like. Examples of the amino acid addition salts include addition salts of lysine, glycine, phenylalanine, or the like.

Examples of protein kinases that are targeted by a protein kinase inhibitor comprising, as an active ingredient, the nitrogen-containing heterocyclic compound or the pharmaceutically acceptable salt thereof of the present invention include fibroblast growth factor receptors (FGFRs), Aurora kinases, Fms-like tyrosine kinase 3 (Flt-3), vascular endothelial growth factor receptors (VEGFRs), c-Kit, platelet-derived growth factors (PDGFRs), tropomyosin receptor kinases (Trks), Abl, lymphocyte-specific kinase (Lck) and the like.

Examples of diseases associated with JAK include cancers, myeloproliferative disorders, immunological disorders and the like.

Examples of cancers include cancer derived from hematopoietic tumor, multiple myeloma, breast cancer, ovary cancer, uterine body cancer, uterine cervix cancer, prostatic cancer, urinary bladder cancer, renal cancer, stomach cancer, esophageal cancer, liver cancer, biliary tract cancer, colon cancer, rectal cancer, pancreatic cancer, lung cancer, head and neck cancer, osteosarcoma, melanoma, and cancer derived from brain tumor, and the like.

Examples of the hematopoietic tumor include acute myelocytic leukemia, acute lymphomatic leukemia , chronic myelocytic leukemia, chronic lymphomatic leukemia, non-Hodgkin's lymphoma, Hodgkin's lymphoma, adult T-cell leukemia, chronic myelomonocytic leukemia, atypical myelocytic leukemia, myelo dysplasctic syndrome and the like.

Examples of the myeloproliferative disorders include polycythemia vera, essential thrombocythemia, myelofibrosis and the like.

Examples of the immunological disorders include transplant rejection, rheumatism, psoriasis, asthma, atopic dermatitis, allergic rhinitis, hay fever and the like.

The production methods of Compound (I) will now be described.

In the production methods described below, when a defined group is changed under the condition of the method to be employed or is not suitable for employing the method, a target compound can be obtained by employing methods of introducing and eliminating a protective group that are normally used in synthetic organic chemistry [for example, Protective Groups in Organic Synthesis third edition, written by T. W. Greene, John Wiley & Sons Inc. (1999)] and the like. In addition, the order of reaction steps, such as the introduction of a substituent, can be changed, if necessary.

Compound (I) can be produced by, for example, reaction steps descried below.

### Production method 1

Compound (IA) can be produced from Compound (AA-1) obtained by a method similar to a known method [Journal of the American Chemical Society, Vol.78, p.1631 (1956), or HETEROCYCLES, Vol.45, p.2217 (1997)] by a step described below. 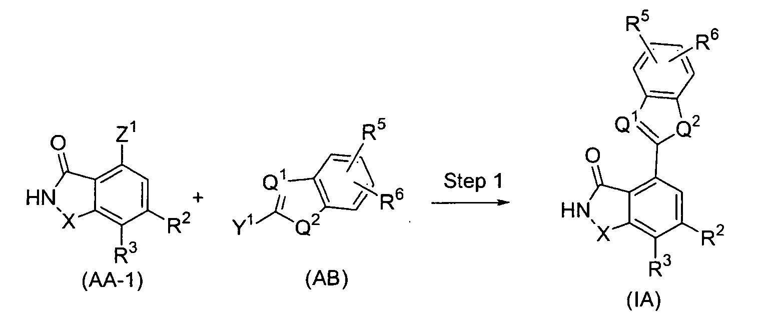 [wherein Y¹ represents M¹(R^{A})ₚ (wherein M¹ represents a tin atom, a boron atom, or a silicon atom; R^{A} represents halogen, hydroxy, lower alkyl, lower alkoxy, aryl, or aryloxy; and p represents an integer of 0 to 3); Z¹ represents a chlorine atom, a bromine atom, or an iodine atom; and X, R², R³, R⁵, R⁶, Q¹, and Q² are the same as those defined above, respectively]

### Step 1

Compound (IA) can be synthesized by reacting Compound (AA-1) with 1 to 30 equivalents of Compound (AB) in the presence of 0.001 to 1 equivalent of a transition metal catalyst, in a solvent at a temperature in the range of -50°C to 200°C for 5 minutes to 100 hours. In this step, 0.01 to 30 equivalents of an appropriate additive may be added so as to accelerate the reaction.

Examples of the solvent include methanol, ethanol, dichloromethane, acetonitrile, toluene, ethyl acetate, tetrahydrofuran (THF), 1,4-dioxane, N,N-dimethylformamide (DMF), N-methylpyrrolidone (NMP), water and the like. These may be used alone or in combinations.

Examples of the transition metal catalyst include palladium catalysts such as palladium acetate, tetrakis(triphenylphosphine)palladium, palladium chloride, palladium bromide, bis(triphenylphosphine)palladium chloride, and dichlorobis(acetonitrile)palladium; nickel catalysts such as nickel chloride, nickel acetylacetonate, bis (1,5-cyclooctadiene) nickel and nickel bromide; and the like.

Examples of the additive include triphenylphosphine, tri(o-tolyl)phosphine, 1,1'-bis(diphenylphosphino)ferrocene, 1,2-bis(diphenylphosphino)propane, 2,2'-bis(diphenylphosphino)-1,1'-binaphthyl, 1,2-bis(diphenylphosphino)ethane, silver oxide, copper iodide, lithium chloride, cesium fluoride, triethylamine, diethylamine, sodium hydroxide, potassium hydroxide, sodium carbonate and the like. These may be used alone or in combinations.

### Production method 2

Compound (IAa) can be produced by reacting Compound (AA-2) with Compound (AC). When T¹ in Compound (AA-2) is a protective group such as a 1-methyl-1-phenylethyl, Compound (IAa) can be produced by reacting Compound (AA-2) with Compound (AC) and then conducting deprotection. 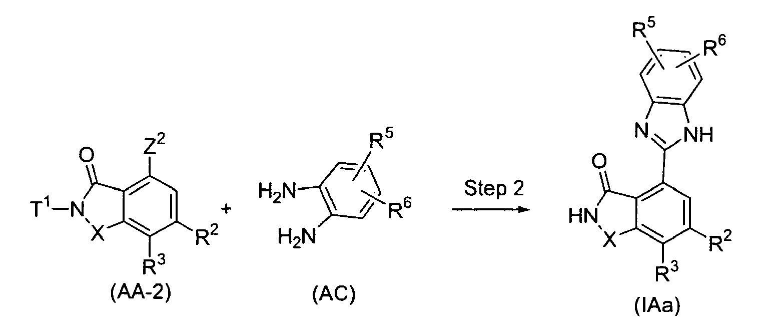 (wherein Z² represents formyl or carboxy, T¹ represents a hydrogen atom or a protective group such as 1-methyl-1-phenylethyl, and X, R², R³, R⁵, and R⁶ are the same as those defined above, respectively)

### Step 2

Compound (IAa) can be synthesized by reactng Compound (AA-2) with 1 to 30 equivalents of Compound (AC) in the presence of 0.001 to 100 equivalents of an oxidizing agent and 0.001 to 100 equivalents of a dehydrating agent, or in the presence of 0.001 to 100 equivalents of a dehydrating agent, in a solvent at a temperature in the range of -50°C to 200°C for 5 minutes to 100 hours. When T¹ of Compound (AA-2) is a protective group, Compound (IAa) is obtained by subsequent proper deprotection.

Examples of the solvent include methanol, ethanol, dichloromethane, acetonitrile, toluene, ethyl acetate, THF, 1,4-dioxane, DMF, NMP, acetic acid, water and the like. These may be used alone or in combinations.

Examples of the oxidizing agent include oxygen, sulfur dioxide, 2,3-dichloro-5,6-dicyano-1,4-benzoquinone (DDQ), nitrobenzene, Oxone (registered trademark), benzofuroxan and the like. These may be used alone or in combinations.

Examples of the dehydrating agent include iron trichloride, zinc dichloride, tin dichloride, titanium tetrabutoxide, sulfuric acid, p-toluenesulfonic acid, thionyl chloride and the like. These may be used alone or in combinations.

Compound (AA-2) can be produced by reacting Compound (AK) obtained by a method similar to a known method [Organic Letters, Vol. 1, p. 1183 (1999)] with 1 to 5 equivalents of a lithium reagent in a solvent at a temperature in the range of -90°C to room temperature for 5 minutes to 50 hours, followed by a reaction with 1 to 30 equivalents of DMF or carbon dioxide gas at a temperature in the range of -90°C to 100°C. In this step, 0.01 to 30 equivalents of an additive may be added so as to accelerate the reaction.

Examples of the solvent include toluene, diethyl ether, THF, 1,4-dioxane and the like. These may be used alone or in combinations.

Examples of the lithium reagent include n-butyllithium, sec-butyllithium, tert-butyllithium, lithium diisopropylamide (LDA) and the like.

Examples of the additive include N,N,N',N'-tetramethylethylenediamine (TMEDA) and the like.

Instead of carbon dioxide gas, dry ice can also be used.

### Production method 3

Among compounds belonging to Compound (I), Compound (Ia-2) having specific functional groups at R^{2b}, R^{3b}, R^{5b}, R^{6b}, and R^{8b}, or Compound (Ia-1) having specific functional groups at R^{2b}, R^{3b}, R^{5b}, and R^{6b} can also be produced by a step described below using Compound (AD-2) having other functional groups at R^{2a}, R^{3a}, R^{5a}, R^{6a}, and R^{8a}, or Compound (AD-1) having other functional groups at R^{2a}, R^{3a}, R^{5a}, and R^{6a} obtained by a method similar to Production method 1 or 2.

In Steps 3 to 9 described below, some of the compounds denotedas Compound (AD-1), or Compound (AD-2) are included in Compound (I). 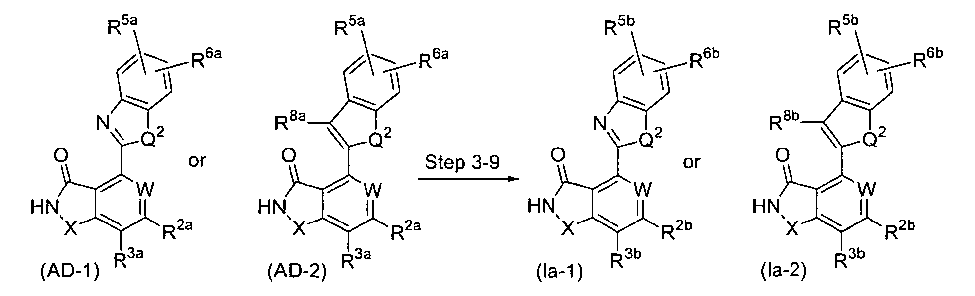 (wherein R^{2a}, R^{3a}, R^{5a}, R^{6a}, and R^{8a}, and R^{2b}, R^{3b}, R^{5b}, R^{6b}, and R^{8b} each represents a group defined in Steps 3 to 9 described below, and W, X, and Q² are the same as those defined above, respectively)

### Step 3

[In step 3, at least one of R^{3a}, R^{5a}, R^{6a}, and R^{8a} is carboxy and at least one of R^{3b}, R^{5b}, R^{6b}, and R^{8b} is -CONR⁹R¹⁰ (wherein R⁹ and R¹⁰ are the same as those defined above, respectively)]

Compound (Ia-1) or Compound (Ia-2) can be synthesized by reacting Compound (AD-1) or Compound (AD-2) with Compound (VIIa) represented by HNR⁹R¹⁰ (wherein R⁹ and R¹⁰ are the same as those defined above, respectively) in the presence of a condensing agent and an activating agent, in a solvent.

Examples of the solvent include dichloromethane, THF, 1,4-dioxane, acetonitrile, DMF, NMP and the like. These may be used alone or in combinations.

Examples of the condensing agent include di(cyclohexyl)carbodiimide, 1-ethyl-3-(3-dimethylaminopropyl) carbodiimide (EDCI) and a hydrochloride thereof, polymer bound-1-ethyl-3-(3-dimethylaminopropyl)carbodiimide, triphenylphosphine oxide-trifluoromethanesulfonic acid anhydride and the like.

Examples of the activating agent include 4-dimethylaminopyridine (DMAP), 1-hydroxybenzotriazole (HOBt), N-hydroxysuccinimide and the like.

Each of the condensing agent, the activating agent, and Compound (VIIa) is preferably used in an amount of 1 to 20 equivalents relative to the amount of Compound (AD-1) or Compound (AD-2). The reaction is normally performed at a temperature in the range of -20°C to 80°C and is finished within the range of 30 minutes to 48 hours.

### Step 4

[In step 4, at least one of R^{3a}, R^{5a}, R^{6a}, and R^{8a} is formyl and at least one of R^{3b}, R^{5b}, R^{6b}, and R^{8b} is -CH₂NR³⁶R³⁷ (wherein R³⁶ and R³⁷ are the same as those defined above, respectively)]

Compound (Ia-1) or Compound (Ia-2) can be synthesized by reacting Compound (AD-1) or Compound (AD-2) with Compound (VIIb) represented by HNR³⁶R³⁷ (wherein R³⁶ and R³⁷ are the same as those defined above, respectively) in the presence of a reducing agent, in a solvent. In this step, 0.01 to 30 equivalents of an appropriate additive may be added so as to accelerate the reaction.

Examples of the solvent include methanol, ethanol, acetonitrile, dichloromethane, THF, 1,4-dioxane, DMF, NMP, acetic acid and the like. These may be used alone or in combinations.

Examples of the reducing agent include sodium borohydride, sodium cyanotrihydroborate, sodium triacetoxyborohydride, pyridine-borane complex and the like.

Examples of the additive include acetic acid, molecular sieves, magnesium sulfate and the like.

Each of the reducing agent and Compound (VIIb) is preferably used in an amount of 1 to 20 equivalents relative to the amount of Compound (AD-1) or Compound (AD-2). The reaction is normally performed at a temperature in the range of -20°C to 80°C and is finished within the range of 30 minutes to 100 hours.

### Step 5

{In step 5, at least one of R^{2a}, R^{3a}, R^{5a}, R^{6a}, and R^{8a} is a chlorine atom, a bromine atom, an iodine atom, -OSO₂CF₃, or -OTs (p-toluenesulfonyl), and at least one of R^{2b}, R^{3b}, R^{5b}, R^{6b}, and R^{8b} is cyano, substituted or unsubstituted lower alkyl, substituted or unsubstituted cycloalkyl, Ar^{1b} [wherein Ar^{1b} represents substituted or unsubstituted aryl, or a substituted or unsubstituted aromatic heterocyclic group], HC=CHR^{B} [wherein R^{B} represents carboxy, substituted or unsubstituted lower alkyl, substituted or unsubstituted aryl, substituted or unsubstituted aralkyl, a substituted or unsubstituted heterocyclic group, substituted or unsubstituted lower alkanoyl, substituted or unsubstituted aroyl, substituted or unsubstituted heteroaroyl, substituted or unsubstituted lower alkoxycarbonyl, or -CONR⁹R¹⁰ (wherein R⁹ and R¹⁰ are the same as those defined above, respectively)] or C≡CR^{B} (wherein R^{B} is the same as defined above)}

Compound (Ia-I) or Compound (Ia-2) can be produced by reacting Compound (AD-1) or Compound (AD-2) with 1 to 30 equivalents of (Ar^{1b})_{q}M²ᵣ(R^{A})ₛ, (R^{B}HC=CH)_{q}M²ᵣ(R^{A})ₛ, (R^{B}C≡C)_{q}M²ᵣ(R^{A})ₛ, (R^{C})_{q}M²ᵣ(R^{A})ₛ, R^{B}HC=CH₂, or R^{B}C≡CH (wherein Ar^{1b}, R^{A}, and R^{B} are the same as those defined above, respectively; R^{C} represents cyano, substituted or unsubstituted lower alkyl, or substituted or unsubstituted cycloalkyl; M² represents a tin atom, a zinc atom, a boron atom, a silicon atom, an aluminum atom, a zirconium atom, a copper atom, or a mercury atom; q and r may be the same or different and each represents 1 or 2; and s represents an integer of 0 to 3) in the presence of 0.001 to 1 equivalent of a transition metal catalyst, in a solvent at a temperature in the range of -5°C to 200°C for 5 minutes to 80 hours. In this step, 0.01 to 30 equivalents of an additive may be added so as to accelerate the reaction.

Examples of the solvent include methanol, ethanol, dichloromethane, chloroform, acetonitrile, toluene, ethyl acetate, THF, 1,4-dioxane, DMF, NMP, water and the like. These may be used alone or in combinations.

Examples of the transition metal catalyst include palladium catalysts such as palladium acetate, tetrakis(triphenylphosphine)palladium, palladium chloride, palladium bromide, bis(triphenylphosphine)palladium chloride, and dichlorobis(acetonitrile)palladium; and nickel catalysts such as nickel chloride, nickel acetylacetonate, bis(1,5-cyclooctadiene)nickel and nickel bromide; and the like.

Examples of the additive include triphenylphosphine, tri(o-tolyl)phosphine, 1,1'-bis(diphenylphosphino)ferrocene, 1,2-bis(diphenylphosphino)propane, 2,2'-bis(diphenylphosphino)-1,1'-binaphthyl, 1,2-bis(diphenylphosphino)ethane, silver oxide, copper iodide, lithium chloride, cesium fluoride, triethylamine, diethylamine, sodium hydroxide, potassium hydroxide, sodium carbonate, cesium carbonate and the like. These may be used alone or in combinations.

### Step 6

{In step 6, at least one of R^{2a}, R^{3a}, R^{5a}, R^{6a}, and R^{8a} is hydroxy, and at least one of R^{2b}, R^{3b}, R^{5b}, R^{6b}, and R^{8b} is -O(C=O) R^{D} or -OSO₂R^{D} [wherein R^{D} represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or -NR^{D1}R^{D2} (wherein R^{D1} and R^{D2} are the same as R⁹ and R¹⁰, respectively)]}

Compound (Ia-1) or Compound (Ia-2) can be synthesized by reacting Compound (AD-1) or Compound (AD-2) with Compound (VIII) represented by R^{D}COX¹ or R^{D}SO₂X¹ (wherein X¹ represents a chlorine atom, a bromine atom, or an iodine atom; and R^{D} is the same as defined above) or (R^{D}CO)₂O or (R^{D}SO₂)₂O (wherein R^{D} is the same as defined above) in the presence of a base, in a solvent.

Examples of the solvent include dichloromethane, THF, 1,4-dioxane, acetonitrile, DMF, NMP, and the like. These may be used alone or in combinations.

Examples of the base include DMAP, triethylamine, diisopropylethylamine, pyridine, sodium carbonate, sodium hydrogencarbonate, potassium carbonate, sodium hydroxide, potassium hydroxide, sodium hydride and the like.

Each of the base and Compound (VIII) is preferably used in an amount of 1 to 20 equivalents relative to the amount of Compound (AD-1) or Compound (AD-2). The reaction is normally performed at a temperature in the range of -20°C to 80°C and is finished within the range of 30 minutes to 24 hours. Regarding some types of Compound (VIII), a salt of Compound (VIII) may be prepared in advance by mixing with an activating agent (for example, DMAP, HOBt, . N-hydroxysuccinimide and the like), and the salt may then be used for the reaction.

### Step 7

{In step 7, at least one of R^{3a}, R^{5a}, R^{6a}, and R^{8a} is -NHR¹¹ (wherein R¹¹ is the same as defined above), and at least one of R^{3b}, R^{5b}, R^{6b}, and R^{8b} is -NR¹¹(C=O)R^{D} or -NR¹¹SO₂R^{D} [wherein R¹¹ is the same as defined above; and R^{D} represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or -NR^{D1}R^{D2} (wherein R^{D1} and R^{D2} are the same as R⁹ and R¹⁰ defined above, respectively)]}

Compound (Ia-1) or Compound (Ia-2) can be synthesized by reacting Compound (AD-1) or Compound (AD-2) with Compound (VIII) represented by RD^{C}OX¹ or R^{D}SO₂X¹ (wherein X¹ and R^{D} are the same as those defined above, respectively) or (R^{D}CO)₂O or (R^{D}SO₂)₂O (wherein R^{D} is the same as defined above) in the presence of a base, in a solvent.

Examples of the solvent include dichloromethane, THF, 1,4-dioxane, acetonitrile, DMF, NMP and the like. These may be used alone or in combinations.

Examples of the base include DMAP, triethylamine, diisopropylethylamine, pyridine, sodium carbonate, sodium hydrogencarbonate, potassium carbonate, sodium hydroxide, potassium hydroxide, sodium hydride and the like.

Each of the base and Compound (VIII) is preferably used in an amount of 1 to 20 equivalents relative to the amount of Compound (AD-1) or Compound (AD-2). The reaction is normally performed at a temperature in the range of -20°C to 80°C and is finished within the range of 30 minutes to 24 hours. Regarding some types of Compound (VIII), a salt of Compound (VIII) may be prepared in advance by mixing with an activating agent (for example, DMAP, HOBt, N-hydroxysuccinimide and the like), and the salt may then be used for the reaction.

### Step 8

[In step 8, at least one of R^{2a}, R^{3a}, R^{5a}, R^{6a}, and R^{8a} is hydroxy, and at least one of R^{2b}, R^{3b}, R^{5b}, R^{6b}, and R^{8b} is -OR^{E} (wherein R^{E} represents substituted or unsubstituted lower alkyl, or substituted or unsubstituted aralkyl)]

Compound (Ia-1) or Compound (Ia-2) can be synthesized by reacting Compound (AD-1) or Compound (AD-2) with Compound (IX) represented by HOR^{E} (wherein R^{E} is the same as defined above) in the presence of a condensing agent, in a solvent.

Examples of the solvent include THF, ether, toluene and the like. These may be used alone or in combinations.

Examples of the condensing agent include mixtures of trivalent phosphorus compounds such as triphenylphosphine and tributylphosphine, and azo compounds such as diethyl azodicarboxylate (DEAD) and 1,1-(azodicarbinyl)dipiperidine and the like.

Each of Compound (IX) and the condensing agent is preferably used in an amount of 1 equivalent or more, preferably in the range of 1 to 5 equivalents relative to the amount of Compound (AD-1) or Compound (AD-2).

The reaction is normally performed at a temperature in the range of -20°C to 80°C, preferably 0°C to 30°C, and is finished within the range of 5 minutes to 48 hours.

### Step 9

[In step 9, at least one of R^{2a}, R^{3a}, R^{5a}, R^{6a}, and R^{8a} is hydroxy, and at least one of R^{2b}, R^{3b}, R^{5b}, R^{6b}, and R^{8b} is -OR^{F} (wherein R^{F} represents substituted or unsubstituted lower alkyl or substituted or unsubstituted aralkyl)]

Compound (Ia-1) or Compound (Ia-2) can be synthesized by reacting Compound (AD-1) or Compound (AD-2) with Compound (X) represented by X¹R^{F} (wherein X¹ and R^{F} are the same as those defined above, respectively) in the presence of a base, in a solvent.

Examples of the solvent include dichloromethane, THF, 1,4-dioxane, acetonitrile, DMF, NMP and the like. These may be used alone or in combinations.

Examples of the base include DMAP, triethylamine, diisopropylethylamine, pyridine, sodium carbonate, sodium hydrogencarbonate, potassium carbonate, sodium hydroxide, potassium hydroxide, sodium hydride and the like.

Each of the base and Compound (X) is preferably used in an amount of 1 to 20 equivalents relative to the amount of Compound (AD-1) or Compound (AD-2). The reaction is normally performed at a temperature in the range of -20°C to 80°C and is finished within the range of 30 minutes to 24 hours.

### Production method 4

Compound (I) can also be produced by a method similar to Production method 1 or Production method 2 using Compound (AF), Compound (AH), Compound (AI), Compound (AJ), Compound, (AL), Compound (AM), Compound (AN), Compound (AP), Compound (AQ), Compound (AR), Compound (AS), Compound (AT), and Compound (AV) obtained by Steps 10 to 14 described below. Substituents R^{2c}, R^{2d}, R^{2e}, R^{2f}, R^{3c}, R^{3d}, R^{3e}, and R^{3f} of Compound (AF), Compound (AH), Compound (AI), Compound (AJ), Compound, (AL), Compound (AM), Compound (AN), Compound (AP), Compound (AQ), Compound (AR), Compound (AS), Compound (AT), and Compound (AV) obtained by Steps 10 to 14 described below can be converted to desired substituents by a method similar to Steps 3 to 9 of Production method 3. 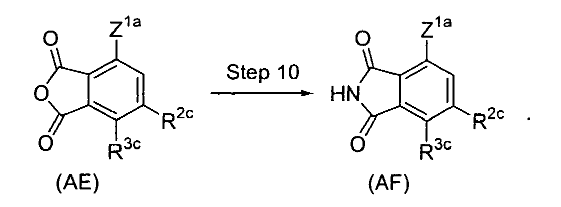 (wherein Z^{1a}, R^{2c} and R^{3c} are the same as Z¹, R² and R³ defined above, respectively)

### Step 10

Compound (AF) can be produced by reacting Compound (AE) obtained by a method similar to a known method [for example, Tetrahedron Letters, Vol.23, p.371 (1982); or Journal of Chemical Society Perkin Transaction I, Vol.19, p.2755 (1999)] with 1 to 30 equivalents of an imidizing agent in the presence or absence of a solvent at a temperature in the range of -50°C to 250°C for 5 minutes to 100 hours.

Examples of the solvent include methanol, ethanol, dichloromethane, acetonitrile, toluene, ethyl acetate, THF, 1,4-dioxane, DMF, NMP, water, acetic acid and the like. These may be used alone or in combinations.

Examples of the imidizing agent include ammonia; ammonium salts such as ammonium carbonate and ammonium acetate; urea; hexamethyldisilazane (HMDS) and the like. 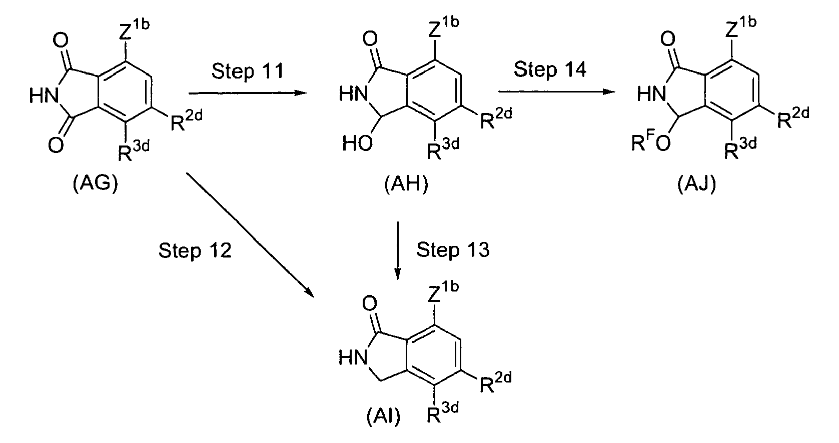 (wherein R^{F} represents substituted or unsubstituted lower alkyl, and Z^{1b}, R^{2d} and R^{3d} are the same as Z¹, R² and R³ defined above, respectively)

### Step 11

Compound (AH) can be produced by reacting Compound (AG) obtained by a method similar to a known method [Journal of the American Chemical Society, Vol.78, p.1631 (1956); or HETEROCYCLES, Vol.45, p.2217 (1997)] with 1 to 30 equivalents of a reducing reagent in a solvent at a temperature in the range of -90°C to 200°C for 5 minutes to 100 hours. In this step, 0.01 to 30 equivalents of an appropriate additive may be added so as to accelerate the reaction.

Examples of the solvent include methanol; ethanol; dichloromethane; acetonitrile; toluene; ethyl acetate; THF; 1,4-dioxane; DMF; NMP; buffer solutions such as sodium acetate-hydrochloric acid, acetic acid-sodium acetate, and citric acid-disodium hydrogenphosphate and the like. These may be used alone or in combinations.

Examples of the reducing agent include diisobutylaluminum hydride, sodium borohydride, lithium aluminum hydride, lithium borohydride, sodium trimethoxyborohydride, sodium borohydride cyanide, sodium triacetoxyborohydride and the like.

Examples of the additive include trifluoroborane-diethyl ether complex, titanium tetrachloride, methanesulfonic acid, cobalt dichloride and the like.

### Step 12

Compound (AI) can be produced by reducing Compound (AG) using 1 to 30 equivalents of borane or a borane compound in a solvent at a temperature in the range of -90°C to 200°C for 5 minutes to 100 hours.

Examples of the solvent include methanol, ethanol, dichloromethane, acetonitrile, toluene, ethyl acetate, THF, 1,4-dioxane, DMF, NMP, water and the like. These may be used alone or in combinations.

Examples of the borane compound include borane-THF complex, borane-dimethyl sulfide complex, diborane and the like.

### Step 13

Compound (AI) can also be produced by reducing Compound (AH) obtained in Step 11 using 1 to 30 equivalents of a hydrosilane compound in a solvent at a temperature in the range of -90°C to 200°C for 5 minutes to 100 hours. In this step, 0.01 to 30 equivalents of an additive may be added so as to accelerate the reaction.

Examples of the solvent include methanol, ethanol, dichloromethane, chloroform, acetonitrile, toluene, ethyl acetate, THF, 1,4-dioxane, acetic acid, trifluoroacetic acid and the like. These may be used alone or in combinations.

Examples of the hydrosilane compound include triethylsilane, trichlorosilane and the like.

Examples of the additive include trifluoroborane-diethyl ether complex, titanium tetrachloride and the like.

### Step 14

Compound (AJ) can be produced by reacting Compound (AH) obtained in Step 11 with 1 equivalent to the amount of solvent of R^{F}OH (wherein R^{F} is the same as defined above) in the presence of an acid and in the presence or absence of a solvent at a temperature in the range of -90°C to 200°C for 5 minutes to 100 hours.

Examples of the solvent include dichloromethane, chloroform, acetonitrile, toluene, ethyl acetate, THF, 1,4-dioxane, DMF, NMP and the like. These may be used alone or in combinations.

Examples of the acid include concentrated hydrochloric acid, concentrated sulfuric acid, DL-10-camphorsulfonic acid, p-toluenesulfonic acid, aluminum chloride, boron trifluoride and the like. 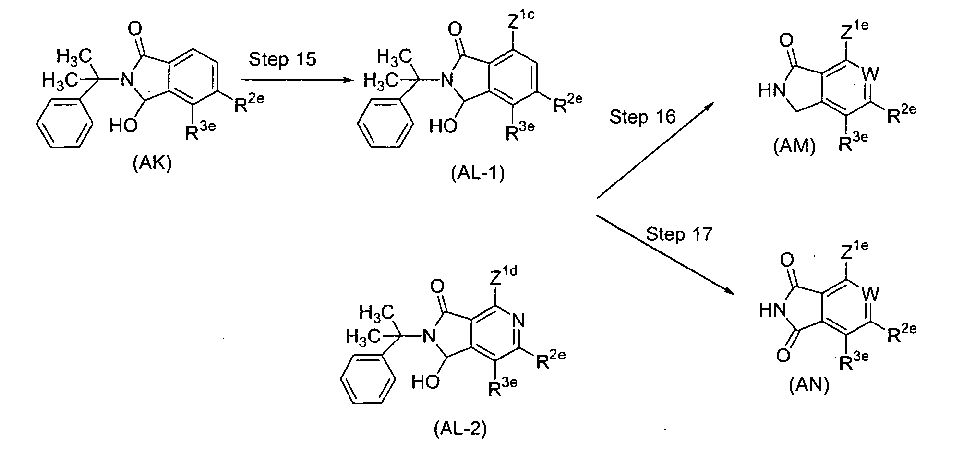 (wherein Z^{1c} represents formyl or a halogen atom selected from a chlorine atom, a bromine atom, and an iodine atom; Z^{1d} and Z^{1e} each represents a chlorine atom, a bromine atom, or an iodine atom; and W, R^{2e}, and R^{3e} are the same as W, R², and R³ defined above, respectively)

### Step 15

Compound (AL-1) can be produced by ortho-lithiation of Compound (AK), followed by halogenation or formylation of the resulting product. Compound (AL-2) can be obtained by a method similar to a known method [Tetrahedron Letters, Vol.32, p.4883 (1991) or Tetrahedron, Vol.49, p.2885 (1993)].

Compound (AL-1) can be produced by reacting Compound (AK) obtained by a method similar to a known method [Organic Letters, Vol. 1, p. 1183 (1999)] with 1 to 5 equivalents of a lithium reagent in a solvent at a temperature in the range of -90°C to room temperature for 5 minutes to 50 hours, followed by reacting with 1 to 30 equivalents of a halogenating agent or DMF at a temperature in the range of -90°C to 100°C. In this step, 0.01 to 30 equivalents of an additive may be added so as to accelerate the reaction.

Examples of the solvent include toluene, diethyl ether, THF, 1,4-dioxane and the like. These may be used alone or in combinations.

Examples of the lithium reagent include n-butyllithium, sec-butyllithium, tert-butyllithium, lithium diisopropylamide (LDA) and the like.

Examples of the additive include N,N,N',N'-tetramethylethylenediamine (TMEDA) and the like.

Examples of the halogenating agent include 2,2,2-trifluoroiodomethane, iodine monochloride, iodine, bromine, hexachloroethane, and the like.

### Step 16

Compound (AM) can be synthesized by reducing Compound (AL-1) or Compound (AL-2) using triethylsilane in the presence of trifluoroacetic acid, by a method similar to the method described in the literature [Organic Letters, Vol.1, p.1183 (1999)].

### Step 17

Compound (AN) can be synthesized by oxidizing Compound (AL-1) or Compound (AL-2) using pyridinium dichromate (PDC), and deprotecting the resulting product using trifluoroacetic acid by a method similar to the method described in the literature [Organic Letters, Vol.1, p.1183 (1999)]. 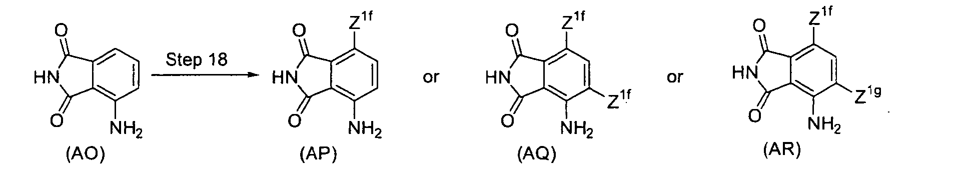 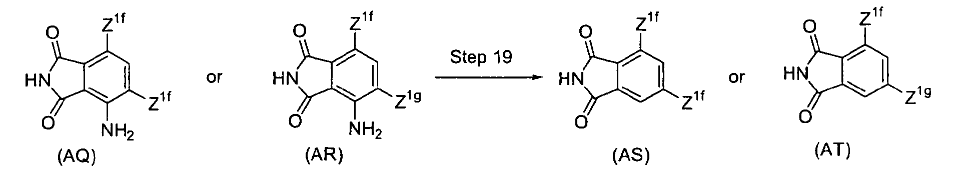 (wherein each of Z^{1f} and Z^{1g} is the same as Z¹ defined above)

### Step 18

Compound (AP), Compound (AQ), or Compound (AR) can be synthesized by halogenating Compound (AO).

Compound (AP) can be synthesized by reacting Compound (AO) with 1 equivalent of a halogenating agent in a solvent at a temperature in the range of -50°C to 200°C for 5 minutes to 100 hours. When 2 equivalents or more of the halogenating agent is used, Compound (AQ) can be synthesized. Alternatively, when different types of halogenating agents are used for Compound (AP), Compound (AR) can be synthesized. In this step, 0.01 to 30 equivalents of an additive may be added so as to accelerate the reaction.

Examples of the solvent include methanol, ethanol, dichloromethane, chloroform, carbon tetrachloride, acetonitrile, toluene, ethyl acetate, THF, 1,4-dioxane, acetic acid, trifluoroacetic acid and the like. These may be used alone or in combinations.

Examples of the halogenating agent include chlorine, gaseous hydrogen chloride, concentrated hydrochloric acid, hydrobromic acid, tetra-n-butylammonium tribromide, bromine, iodine, N-chlorosuccinimide (NCS), N-bromosuccinimide (NBS), N-iodosuccinimide (NIS), iodine monochloride and the like.

Examples of the additive include silver sulfate, copper acetate, calcium carbonate, zinc chloride and the like.

### Step 19

Compound (AS) can be produced using Compound (AQ) synthesized in Step 18, and Compound (AT) can be produced using Compound (AR) synthesized in Step 16, by a method similar to the method described in the literature [Journal of Chemical Society Perkin Transaction 1, p.873 (1986)]. More specifically, Compound (AS) or Compound (AT) can be synthesized by reacting Compound (AQ) or Compound (AR) with a nitrite compound in a solvent containing 1 to 30 equivalents of formamide at a temperature in the range of -50°C to 100°C for 5 minutes to 100 hours, and then adding triethylamine.

Examples of the solvent include methanol, ethanol, dichloromethane, chloroform, acetonitrile, toluene, ethyl acetate, THF, 1,4-dioxane, water, acetic acid, trifluoroacetic acid and the like. These may be used alone or in combinations.

Examples of the nitrite compound include sodium nitrite, tert-butyl nitrite and the like. 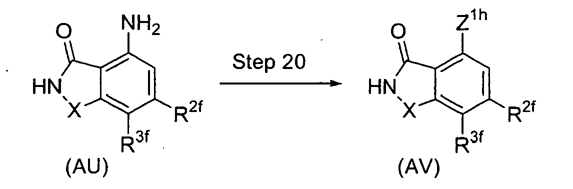 (wherein X is the same as defined above; and Z^{1h}, R^{2f}, and R^{3f} are the same as Z¹, R², and R³ defined above, respectively)

### Step 20

Compound (AV) can be produced by reacting a diazonium salt with a halogenating agent. The diazonium salt is capable of being prepared by reacting Compound (AU) obtained by a method similar to a known method [Bioorganic & Medicinal Chemistry Letters, Vol.14, p.4505 (2004)] with a nitrite compound.

Compound (AU) is reacted with 1 to 30 equivalents of a nitrite compound in the presence or absence of a solvent at a temperature in the range of -50°C to 100°C for 5 minutes to 48 hours to prepare its corresponding diazonium salt. The diazonium salt is then reacted with 1 to 30 equivalents of a halogenating agent in a solvent at a temperature in the range of -50°C to 200°C for 5 minutes to 48 hours to produce Compound (AV).

Examples of the solvent include methanol, ethanol, dichloromethane, acetonitrile, toluene, ethyl acetate, THF, 1,4-dioxane, DMF, NMP, water and the like. These may be used alone or in combinations.

Examples of the halogenating agent include iodine, copper chloride, copper bromide, copper iodide and the like. Among these, copper halides can be prepared by adding sodium chloride, sodium bromide, or the like to an aqueous copper sulfate solution, and then reducing the mixture with sodium nitrite. The copper halides thus prepared may be used for the above step without isolation.

By conducting the above-described methods in combinations if needed, Compound (I) having desired functional groups at desired positions can be obtained.

The conversion of functional groups contained in the substituents of Compound (I) and starting material can be performed not only by the above-described steps but also by other known methods [for example, Comprehensive Organic Transformations, written by R. C. Larock (1989)].

Compounds (II), (III), (IV), (V), and (VI) can be synthesized in a similar manner to the production methods described above.

Isolation and purification of the products obtained by the above-described production methods can be performed by appropriately combining methods that are generally used in organic synthesis, for example, filtration, extraction, washing, drying, concentration, crystallization, various types of chromatography and the like. Intermediates may be used in subsequent reactions without purification.

Isomers of Compounds (I), (II), (III), (IV), (V), and (VI) such as stereoisomers, regioisomers, geometrical isomers, and optical isomers can be present. The present invention also includes any possible isomers and mixtures containing these isomers in any ratio.

In order to produce salts of Compounds (I), (II), (III), (IV), (V), and (VI), when Compounds (I), (II), (III), (IV), (V), and (VI) are obtained in the form of salts, the salts can be purified without further treatment. On the other hand, when Compounds (I), (II), (III), (IV), (V), and (VI) are obtained in the free form, Compounds (I), (II), (III), (IV), (V), and (VI) are dissolved or suspended in an appropriate solvent, and an acid, a base, or the like is then added to the solution or the suspension to form salts.

Compounds (I), (II), (III), (IV), (V), and (VI) or pharmaceutically acceptable salts thereof can be present in the form of adducts of water or various solvents. The present invention also includes such adducts.

Hereinafter, tables 1-17 show specific examples of the compounds of the present invention, but these compounds do not limit the scope of the present invention.

In the tables below, Me, Et, and Ph represent methyl, ethyl, and phenyl, respectively.

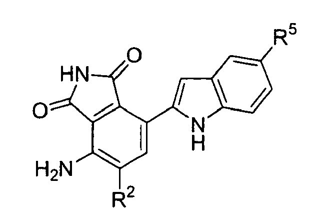

**Table 1**

| Compound Number | R⁵ | R² | Analytical data (MS m/z) |
|---|---|---|---|
| 1 | H | H | 276 [M-H]⁻ |
| 2 | 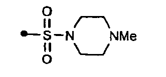 | Ph | 616 [M+H]⁺ |
| 3 | 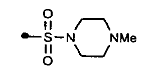 | 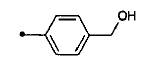 | 546 [M+H]⁺ |
| 4 | 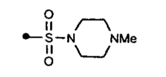 | H | 440 [M+H]⁺ |
| 5 | 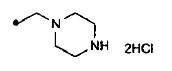 | H | 376 [M+H]⁺ |
| 6 | 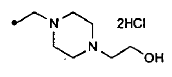 | H | 470 [M+H]⁺ |
| 7 | 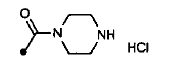 | H | 390 [M+H]⁺ |
| 8 | 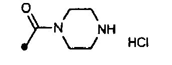 | Ph | 466 [M+H]⁺ |
| 9 | 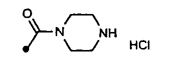 | 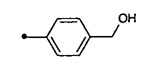 | 496 [M+H]⁺ |
| 10 | 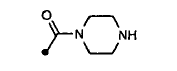 | 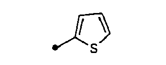 | 472 [M+H]⁺ |
| 11 | 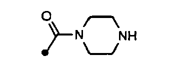 | 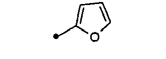 | 456 [M+H]⁺ |
| 12 | 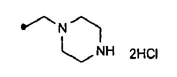 | 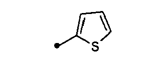 | 458 [M+H]⁺ |
| 13 | 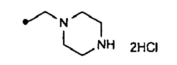 | 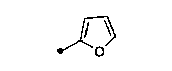 | 442 [M+H]⁺ |
| 14 | 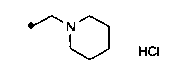 | 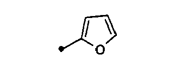 | 441 [M+H]⁺ |
| 15 | 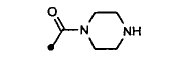 | 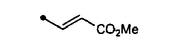 | 474 [M+H]⁺ |

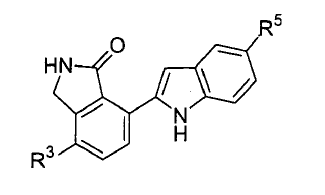

**Table 2-1**

| Compound Number | R⁵ | R³ | Analytical data (MS m/z) |
|---|---|---|---|
| 16 | H | Cl | 281 [M-H]⁻ |
| 17 | 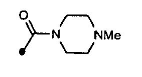 | Cl | 409 [M+H]⁺ |
| 18 | Br | Cl | 361 [M+H]⁺ |
| 19 | CO₂H | Cl | 327 [M+H]⁺ |
| 20 | 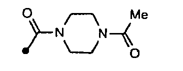 | Cl | 437 [M+H]⁺ |
| 21 | 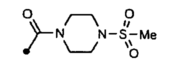 | Cl | 473 [M+H]⁺ |
| 22 | 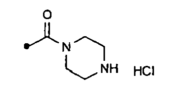 | Cl | 395 [M+H]⁺ |
| 23 | 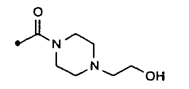 | Cl | 439 [M+H]⁺ |
| 24 | 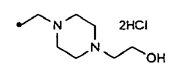 | Cl | 425 [M+H]⁺ |
| 25 | 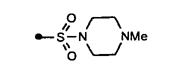 | Cl | 445 [M+H]⁺ |
| 26 | 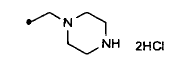 | Cl | 381 [M+H]⁺ |
| 27 | 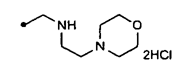 | Cl | 425 [M+H]⁺ |
| 28 | 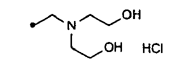 | Cl | 400 [M+H]⁺ |
| 29 | 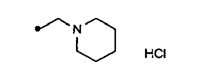 | Cl | 380 [M+H]⁺ |
| 30 | 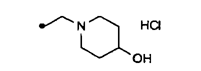 | Cl | 396 [M+H]⁺ |

**Table 2-2**

| Compound Number | R⁵ | R³ | Analytical data (MS m/z) |
|---|---|---|---|
| 31 | 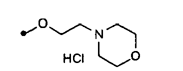 | Cl | 412 [M+H]⁺ |
| 32 | 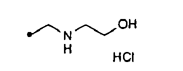 | Cl | 356 [M+H]⁺ |
| 33 | 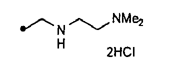 | Cl | 383 [M+H]⁺ |
| 34 | 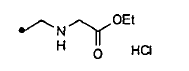 | Cl | 398 [M+H]⁺ |
| 35 | 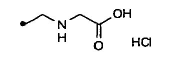 | Cl | 367 [M-H]⁻ |
| 36 | 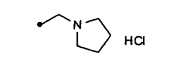 | Cl | 366 [M+H]⁺ |
| 37 | 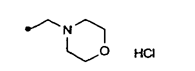 | Cl | 382 [M+H]⁺ |
| 38 | 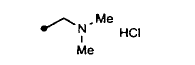 | Cl | 340 [M+H]⁺ |
| 39 | 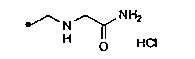 | Cl | 369 [M+H]⁺ |
| 40 | 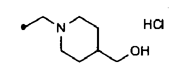 | Cl | 410 [M+H]⁺ |
| 41 | 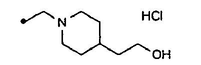 | Cl | 424 [M+H]⁺ |
| 42 | 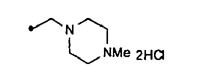 | Cl | 395 [M+H]⁺ |
| 43 | 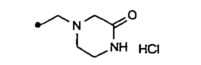 | Cl | 393 [M-H]⁻ |
| 44 | 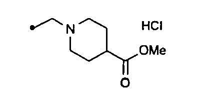 | Cl | 438 [M+H]⁺ |
| 45 | 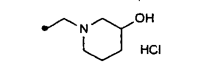 | Cl | 396 [M+H]⁺ |

**Table 2-3**

| Compound Number | R⁵ | R³ | Analytical data (MS m/z) |
|---|---|---|---|
| 46 | 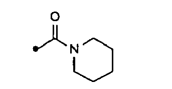 | Cl | 394 [M+H]⁺ |
| 47 | 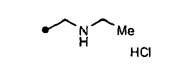 | Cl | 340 [M+H]⁺ |
| 48 | 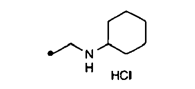 | Cl | 394 [M+H]⁺ |
| 49 | 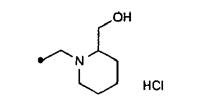 | Cl | 410 [M+H]⁺ |
| 50 | 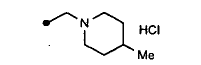 | Cl | 394 [M+H]⁺ |
| 51 | 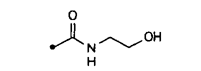 | Cl | 370 [M+H]⁺ |
| 52 | 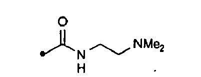 | Cl | 397 [M+H]⁺ |
| 53 | 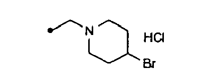 | Cl | 458 [M+H]⁺ |
| 54 | 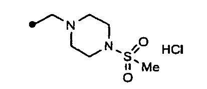 | Cl | 459 [M+H]⁺ |
| 55 | 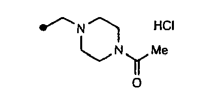 | Cl | 423 [M+H]⁺ |
| 56 | 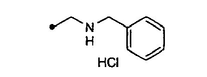 | Cl | 402 [M+H]⁺ |
| 57 | 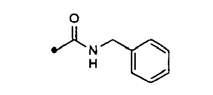 | Cl | 416 [M+H]⁺ |
| 58 | 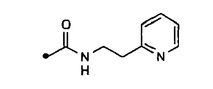 | Cl | 431 [M+H]⁺ |
| 59 | 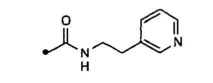 | Cl | 431 [M+H]⁺ |
| 60 | 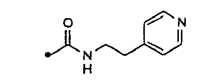 | Cl | 431 [M+H]⁺ |

**Table 2-4**

| Compound Number | R⁵ | R³ | Analytical data (MS m/z) |
|---|---|---|---|
| 61 | 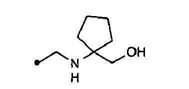 | Cl | 410 [M+H]⁺ |
| 62 | 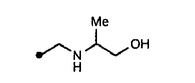 | Cl | 370 [M+H]⁺ |
| 63 | 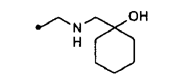 | Cl | 424 [M+H]⁺ |
| 64 | 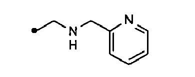 | Cl | 403 [M+H]⁺ |
| 65 | 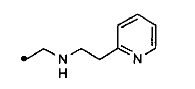 | Cl | 417 [M+H]⁺ |
| 66 | 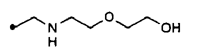 | Cl | 400 [M+H]⁺ |
| 67 | 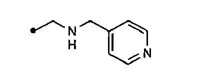 | Cl | 403 [M+H]⁺ |
| 68 | 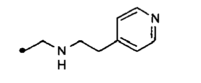 | Cl | 417 [M+H]⁺ |
| 69 | 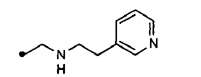 | Cl | 417 [M+H]⁺ |
| 70 | 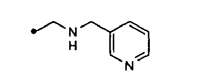 | Cl | 403 [M+H]⁺ |
| 71 | 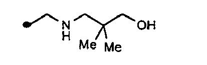 | Cl | 403 [M+H]⁺ |
| 72 | 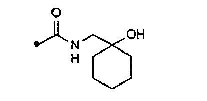 | Cl | 438 [M+H]⁺ |
| 73 | 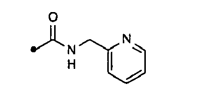 | Cl | 417 [M+H]⁺ |
| 74 | 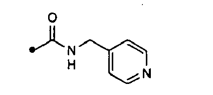 | Cl | 417 [M+H]⁺ |
| 75 | 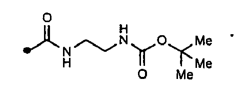 | Cl | 469 [M+H]⁺ |

**Table 2-5**

| Compound Number | R⁵ | R³ | Analytical data (MS m/z) |
|---|---|---|---|
| 76 | 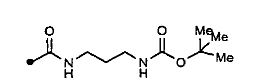 | Cl | 483 [M+H]⁺ |
| 77 | 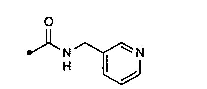 | Cl | 417 [M+H]⁺ |
| 78 | 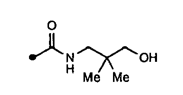 | Cl | 412 [M+H]⁺ |
| 79 | 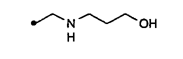 | Cl | 370 [M+H]⁺ |
| 80 | 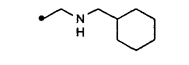 | Cl | 408 [M+H]⁺ |
| 81 | 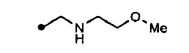 | Cl | 370 [M+H]⁺ |
| 82 | 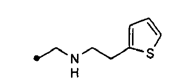 | Cl | 422 [M+H]⁺ |
| 83 | 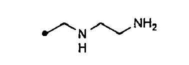 | Cl | 355 [M+H]⁺ |
| 84 | 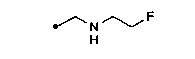 | Cl | 358 [M+H]⁺ |
| 85 | 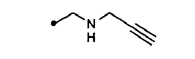 | Cl | 350 [M+H]⁺ |
| 86 | 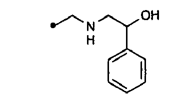 | Cl | 432 [M+H]⁺ |
| 87 | 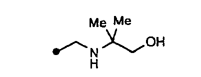 | Cl | 384 [M+H]⁺ |
| 88 | 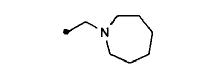 | Cl | 394 [M+H]⁺ |
| 89 | 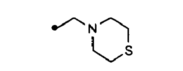 | Cl | 398 8 [M+H]⁺ |
| 90 | 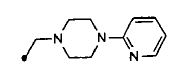 | Cl | 458 [M+H]⁺ |

**Table 2-6**

| Compound Number | R⁵ | R³ | Analytical data (MS m/z) |
|---|---|---|---|
| 91 | 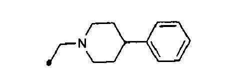 | Cl | 456 [M+H]⁺ |
| 92 | 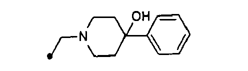 | Cl | 472 [M+H]⁺ |
| 93 | 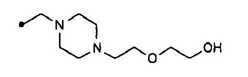 | Cl | 469 [M+H]⁺ |
| 94 | 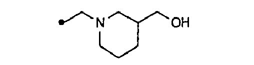 | Cl | 410 [M+H]⁺ |
| 95 | 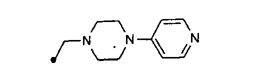 | Cl | 458 [M+H]⁺ |
| 96 | 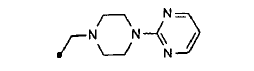 | Cl | 459 [M+H]⁺ |
| 97 | 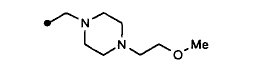 | Cl | 439 [M+H]⁺ |
| 98 | 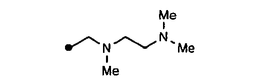 | Cl | 397 [M+H]⁺ |
| 99 | 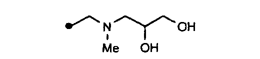 | Cl | 386 [M+H]⁺ |
| 100 | 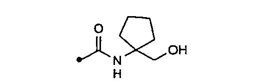 | Cl | 424 [M+H]⁺ |
| 101 | 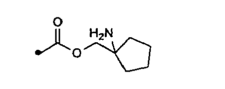 | Cl | 424 [M+H]⁺ |
| 102 | 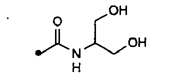 | Cl | 400 [M+H]⁺ |
| 103 | 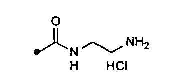 | Cl | 369 [M+H]⁺ |
| 104 | 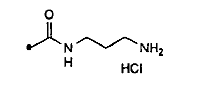 | Cl | 383 [M+H]⁺ |
| 105 | 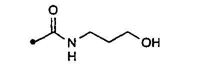 | cl | 384 [M+H]⁺ |

**Table 2-7**

| Compound Number | R⁵ | R³ | Analytical data (MS m/z) |
|---|---|---|---|
| 106 | 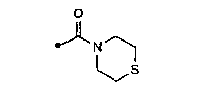 | Cl | 412 [M+H]⁺ |
| 107 | 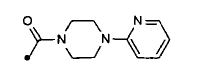 | Cl | 472 [M+H]⁺ |
| 108 | 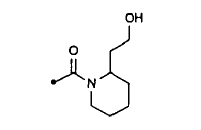 | Cl | 438 [M+H]⁺ |
| 109 | 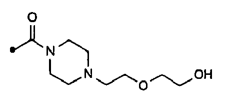 | Cl | 483 [M+H]⁺ |
| 110 | 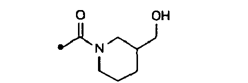 | Cl | 424 [M+H]⁺ |
| 111 | 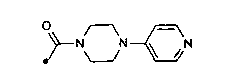 | Cl | 472 [M+H]⁺ |
| 112 | 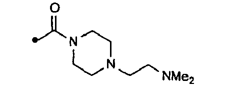 | Cl | 466 [M+H]⁺ |
| 113 | 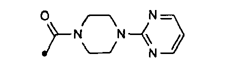 | Cl | 473 [M+H]⁺ |
| 114 | 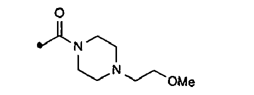 | Cl | 453 [M+H]⁺ |
| 115 | 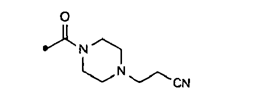 | Cl | 448 [M+H]⁺ |
| 116 | 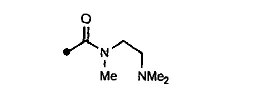 | Cl | 411 [M+H]⁺ |
| 117 | 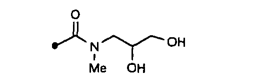 | Cl | 414 [M+H]⁺ |
| 118 | 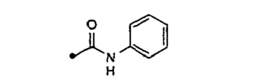 | Cl | 402 [M+H]⁺ |
| 119 | 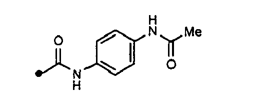 | Cl | 459 [M+H]⁺ |
| 120 | 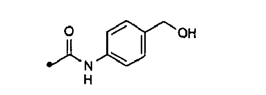 | Cl | 432 [M+H]⁺ |

**Table 2-8**

| Compound Number | R⁵ | R³ | Analytical data (MS m/z) |
|---|---|---|---|
| 121 | 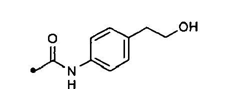 | Cl | 446 [M+H]⁺ |
| 122 | 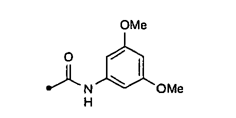 | Cl | 462 [M+H]⁺ |
| 123 | 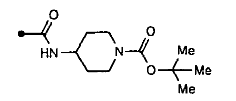 | Cl | 509 [M+H]⁺ |
| 124 | 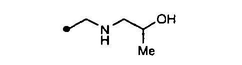 | Cl | 370 [M+H]⁺ |
| 125 | 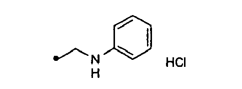 | Cl | 386 [M-H]⁻ |
| 126 | 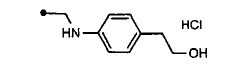 | Cl | 432 [M+H]⁺ |
| 127 | 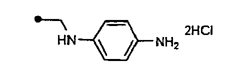 | Cl | 403 [M+H]⁺ |
| 128 | 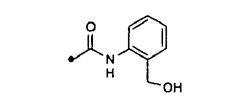 | Cl | 432 [M+H]⁺ |
| 129 | 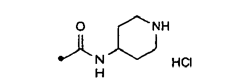 | Cl | 409 [M+H]⁺ |
| 130 | 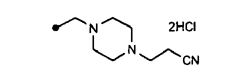 | Cl | 434 [M+H]⁺ |
| 131 | 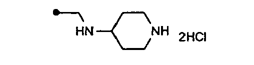 | Cl | 393 [M-H]⁻ |
| 132 | 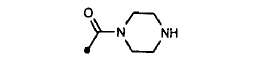 | F | 379 [M+H]⁺ |
| 133 | 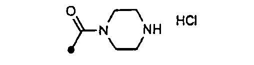 | OMe | 391 [M+H]⁺ |
| 134 | 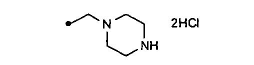 | F | 365 [M+H]⁺ |
| 135 | 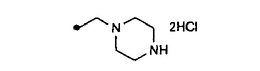 | OMe | 377 [M+H]⁺ |

**Table 2-9**

| Compound Number | R⁵ | R³ | Analytical data (MS m/z) |
|---|---|---|---|
| 136 | 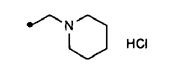 | OMe | 376 [M+H]⁺ |
| 137 | 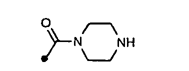 | OPh | 453 [M+H]⁺ |
| 138 | 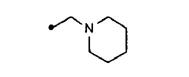 | OPh | 438 [M+H]⁺ |
| 139 | 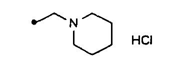 | OH | 362 [M+H]⁺ |
| 140 | 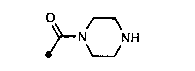 | Ph | 437 [M+H]⁺ |
| 141 | 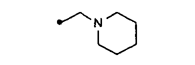 | Ph | 422 [M+H]⁺ |
| 142 | 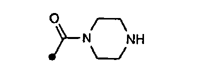 | 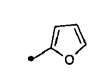 | 427 [M+H]⁺ |
| 143 | 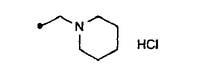 | 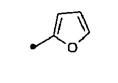 | 412 [M+H]⁺ |
| 144 | 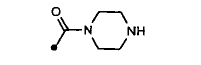 | OH | 377 [M+H]⁺ |
| 145 | 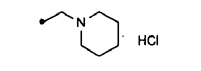 | 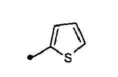 | 428 [M+H]⁺ |
| 146 | 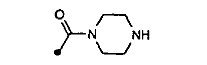 | 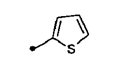 | 443 [M+H]⁺ |
| 147 | 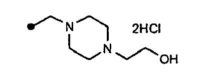 | OH | 407 [M+H]⁺ |
| 148 | 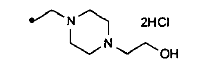 | 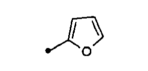 | 457 [M+H]⁺ |
| 149 | 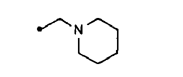 | 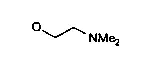 | 433 [M+H]⁺ |
| 150 | 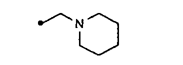 | 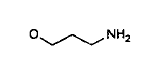 | 419 [M+H]⁺ |
| 151 | 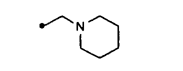 | 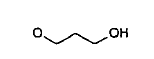 | 420 [M+H]⁺ |
| 152 | 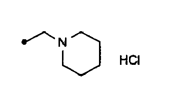 | 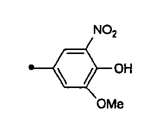 | 513 [M+H]⁺ |
| 153 | 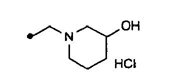 | OH | 378 [M+H]⁺ |

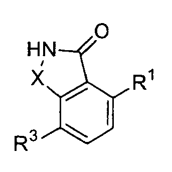

**Table 3-1**

| Compound Number | X | R¹ | R³ | Analytical data (MS m/z) |
|---|---|---|---|---|
| 154 | C=O | 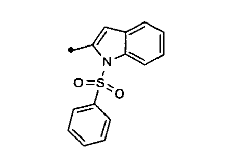 | NH₂ | 416 [M-H]⁻ |
| 155 | CH₂ | 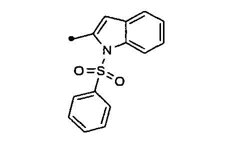 | Cl | 423 [M+H]⁺ |
| 156 | CH₂ | 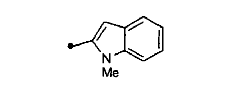 | Cl | 297 [M+H]⁺ |
| 157 | CH₂ | 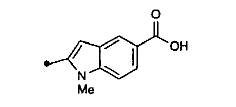 | Cl | 341 [M+H]⁺ |
| 158 | CH₂ | 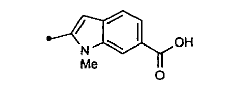 | Cl | 341 [M+H]⁺ |
| 159 | CH₂ | 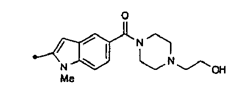 | Cl | 453 [M+H]⁺ |
| 160 | CH₂ | 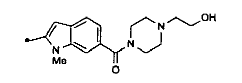 | Cl | 453 [M+H]⁺ |
| 161 | C=O | 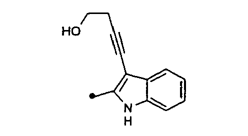 | NH₂ | 346 [M+H]⁺ |
| 162 | CH₂ | 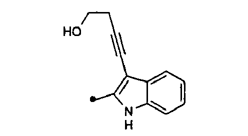 | Cl | 351 [M+H]⁺ |
| 163 | CH₂ | 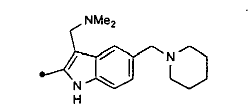 | Cl | 437 [M+H]⁺ |

**Table 3-2**

| Compound Number | X | R¹ | R³ | Analytical data (MS m/z) |
|---|---|---|---|---|
| 164 | CH₂ | 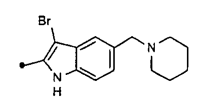 | Cl | 458 [M+H]⁺ |
| 165 | CH₂ | 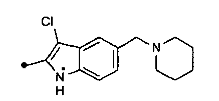 | Cl | 414 [M+H]⁺ |
| 166 | C=O | 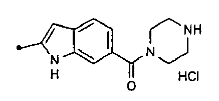 | NH₂ | 390 [M+H]⁺ |
| 167 | CH₂ | 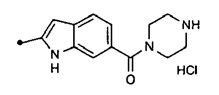 | Cl | 395 [M+H]⁺ |
| 168 | C=O | 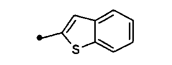 | H | 278 [M-H]⁻ |
| 169 | C=O | 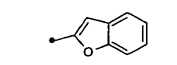 | H | 262 [M-H]⁻ |
| 170 | CH₂ | 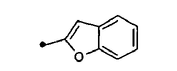 | Cl | 284 [M+H]⁺ |
| 171 | C=O | 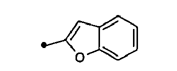 | NH₂ | 277 [M-H]⁻ |
| 172 | CH₂ | 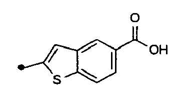 | Cl | 342 [M-H]⁻ |
| 173 | CH₂ | 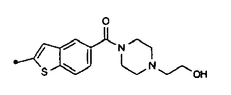 | Cl | 456 [M+H]⁺ |
| 174 | CH₂ | 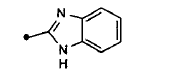 | Cl | 284 [M+H]⁺ |
| 175 | CH₂ | 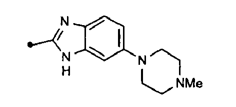 | Cl | 382 [M+H]⁺ |
| 176 | CH₂ | 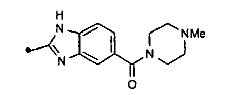 | Cl | 410 [M+H]⁺ |
| 177 | C=O | 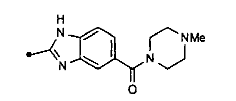 | Cl | 424 [M+H]⁺ |
| 178 | CH₂ | 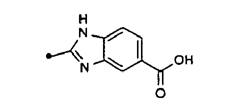 | Cl | 328 [M+H]⁺ |

**Table 3-3**

| Compound Number | X | R¹ | R³ | Analytical data (MS m/z) |
|---|---|---|---|---|
| 179 | CH₂ | 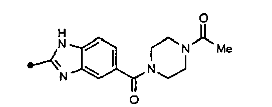 | Cl | 438 [M+H]⁺ |
| 180 | CH₂ | 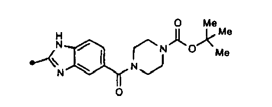 | Cl | 496 [M+H]⁺ |
| 181 | CH₂ | 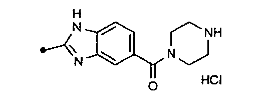 | Cl | 396 [M+H]⁺ |
| 182 | CH₂ | 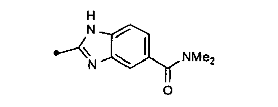 | Cl | 355 [M+H]⁺ |
| 18 | CH₂ | 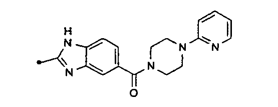 | Cl | 473 [M+H]⁺ |
| 184 | CH₂ | 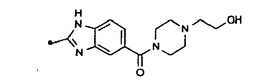 | Cl | 440 [M+H]⁺ |
| 185 | CH₂ | 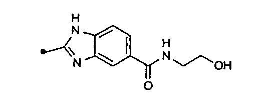 | Cl | 371 [M+H]⁺ |
| 186 | CH₂ | 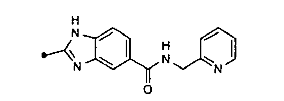 | Cl | 418 [M+H]⁺ |
| 187 | CH₂ | 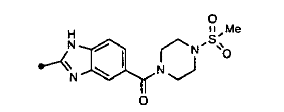 | Cl | 474 [M+H]⁺ |

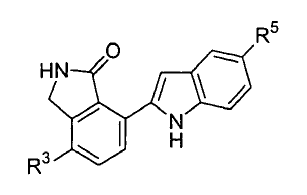

**Table 4-1**

| Compound Number | R⁵ | R³ | Analytical data (MS m/z) |
|---|---|---|---|
| 188 | 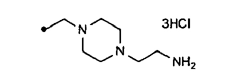 | Cl | 424 [M+H]⁺ |
| 189 | 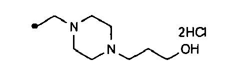 | Cl | 439 [M+H]⁺ |
| 190 | 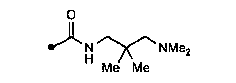 | Cl | 439 [M+H]⁺ |
| 191 | 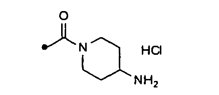 | Cl | 409 [M+H]⁺ |
| 192 | 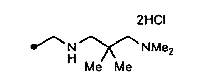 | Cl | 425 [M+H]⁺ |
| 193 | 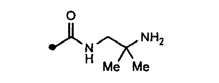 | Cl | 397 [M+H]⁺ |
| 194 | 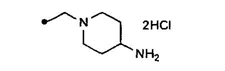 | Cl | 395 [M+H]⁺ |
| 195 | 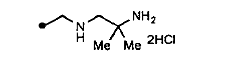 | Cl | 383 [M+H]⁺ |
| 196 | 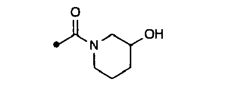 | Cl | 410 [M+H]⁺ |
| 197 | 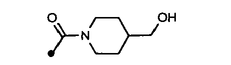 | Cl | 424 [M+H]⁺ |
| 198 | 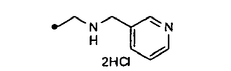 | Cl | 403 [M+H]⁺ |
| 199 | 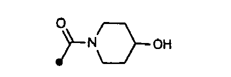 | Cl | 410 [M+H]⁺ |
| 200 | 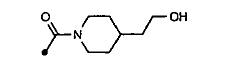 | Cl | 438 [M+H]⁺ |
| 201 | 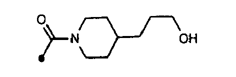 | Cl | 453 [M+H]⁺ |
| 202 | 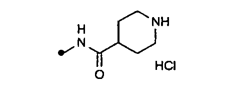 | Cl | 409 [M+H]⁺ |

**Table 4-2**

| Compound Number | R⁵ | R³ | Analytical data (MS m/z) |
|---|---|---|---|
| 203 | NH₂·HCl | Cl | 298 [M+H]⁺ |
| 204 | 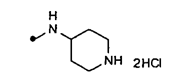 | Cl | 381 [M+H]⁺ |
| 205 | OH | Cl | 299 [M+H]⁺ |
| 206 | CN | Cl | 308 [M+H]⁺ |
| 207 | 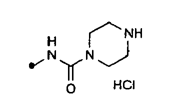 | Cl | 410 [M+H]⁺ |
| 208 | 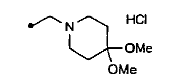 | Cl | 440 [M+H]⁺ |
| 209 | 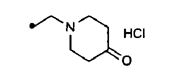 | Cl | 394 [M+H]⁺ |
| 210 | 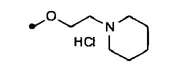 | Cl | 410 [M+H]⁺ |
| 211 | CO₂H | OH | 309 [M+H]⁺ |
| 212 | 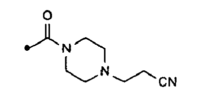 | OH | 430 [M+H]⁺ |
| 213 | 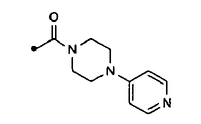 | OH | 454 [M+H]⁺ |
| 214 | 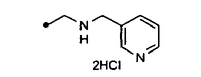 | OH | 385 [M+H]⁺ |
| 215 | 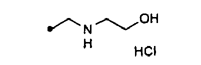 | OH | 336 [M-H]⁻ |
| 216 | 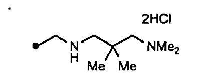 | OH | 407 [M+H]⁺ |
| 217 | 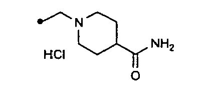 | OH | 405 [M+H]⁺ |

**Table 4-3**

| Compound Number | R⁵ | R³ | Analytical data (MS m/z) |
|---|---|---|---|
| 218 | 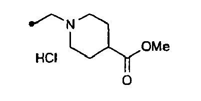 | OH | 420 [M+H]⁺ |
| 219 | 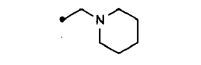 | 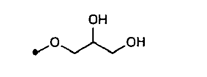 | 436 [M+H]⁺ |
| 220 | 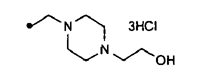 | 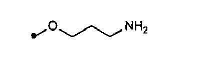 | 464 [M+H]⁺ |
| 221 | 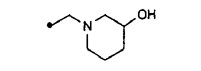 | 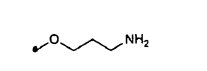 | 435 [M+H]⁺ |
| 222 | 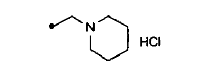 | 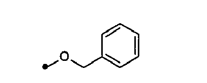 | 452 [M+H]⁺ |
| 223 | 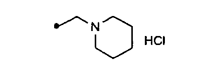 | 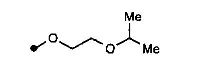 | 448 [M+H]⁺ |
| 224 | 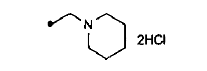 | 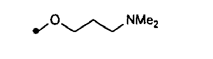 | 447 [M+H]⁺ |
| 225 | 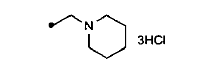 | 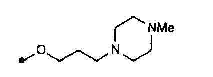 | 502 [M+H]⁺ |
| 226 | 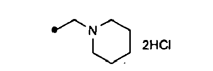 | 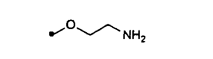 | 502 [M+H]⁺ |
| 227 | 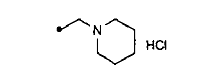 | 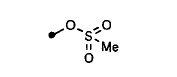 | 375 [M+H]⁺ |
| 228 | 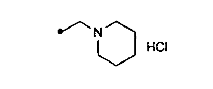 | 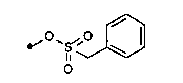 | 516 [M+H]⁺ |
| 229 | 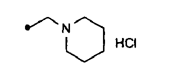 | 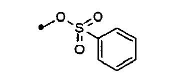 | 502 [M+H]⁺ |
| 230 | 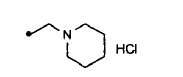 | 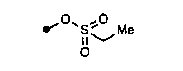 | 454 [M+H]⁺ |
| 231 | 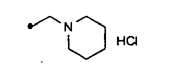 | 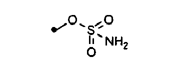 | 441 [M+H]⁺ |
| 232 | 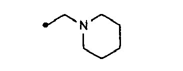 | 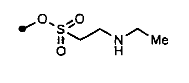 | 497 [M+H]⁺ |

**Table 4-4**

| Compound Number | R⁵ | R³ | Analytical data (MS m/z) |
|---|---|---|---|
| 233 | 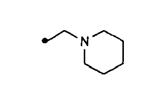 | 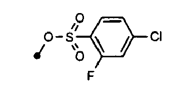 | 555 [M+H]⁺ |
| 234 | 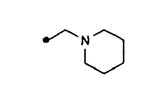 | 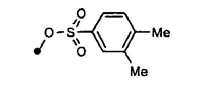 | 530 [M+H]⁺ |
| 235 | 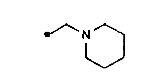 | 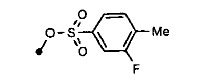 | 534 [M+H]⁺ |
| 236 | 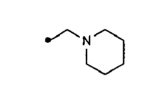 | 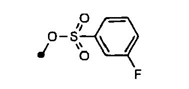 | 520 [M+H]⁺ |
| 237 | 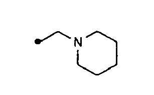 | 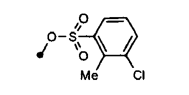 | 551 [M+H]⁺ |
| 238 | 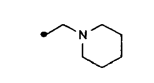 | 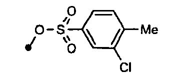 | 551 [M+H]⁺ |
| 239 | 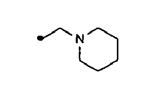 | 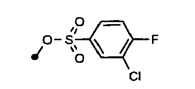 | 555 [M+H]⁺ |
| 240 | 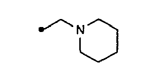 | 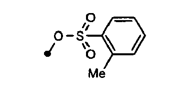 | 516 [M+H]⁺ |
| 241 | 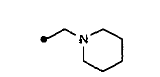 | 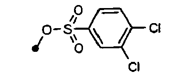 | 571 [M+H]⁺ |
| 242 | 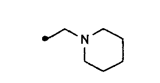 | 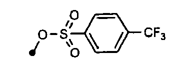 | 570 [M+H]⁺ |
| 243 | 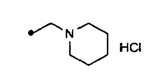 | 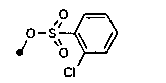 | 536 [M+H]⁺ |
| 244 | 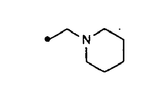 | 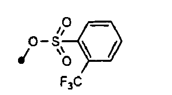 | 570 [M+H]⁺ |
| 245 | 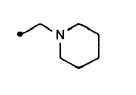 | 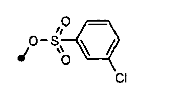 | 537 [M+H]⁺ |
| 246 | 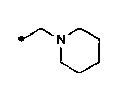 | 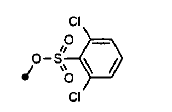 | 571 [M+H]⁺ |
| 247 | 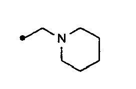 | 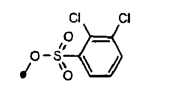 | 571 [M+H]⁺ |

**Table 4-5**

| Compound Number | R⁵ | R³ | Analytical data (MS m/z) |
|---|---|---|---|
| 248 | 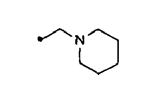 | 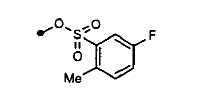 | 534 [M+H]⁺ |
| 249 | 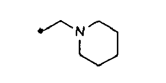 | 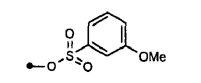 | 532 [M+H]⁺ |
| 250 | 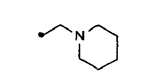 | 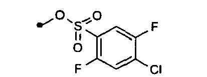 | 572 [M+H]⁺ |
| 251 | 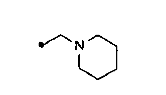 | 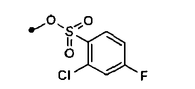 | 532 [M+H]⁺ |
| 252 | 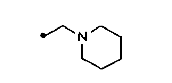 | 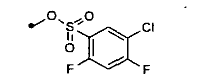 | 573 [M+H]⁺ |
| 253 | 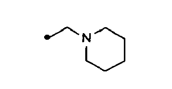 | 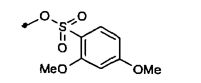 | 562 [M+H]⁺ |
| 254 | 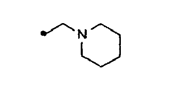 | 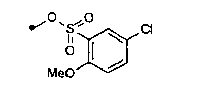 | 567 [M+H]⁺ |
| 255 | 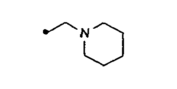 | 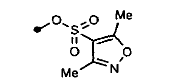 | 521 [M+H]⁺ |
| 256 | 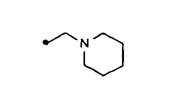 | 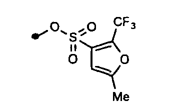 | 574 [M+H]⁺ |
| 257 | 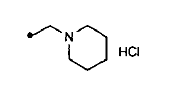 | 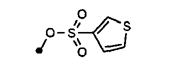 | 508 [M+H]⁺ |
| 258 | 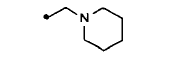 | 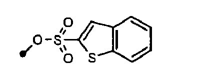 | 558 [M+H]⁺ |
| 259 | 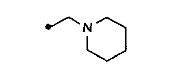 | 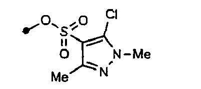 | 555 [M+H]⁺ |
| 260 | 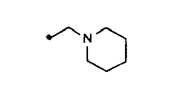 | 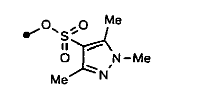 | 534 [M+H]⁺ |
| 261 | 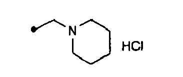 | 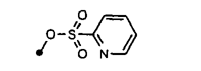 | 503 [M+H]⁺ |
| 262 | 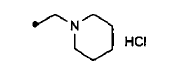 | 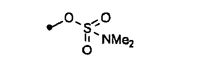 | 454 [M+H]⁺ |

**Table 4-6**

| Compound Number | R⁵ | R³ | Analytical data (MS m/z) |
|---|---|---|---|
| 263 | 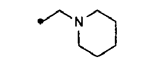 | 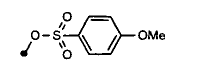 | 532 [M+H]⁺ |
| 264 | 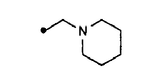 | 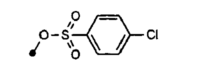 | 537 [M+H]⁺ |
| 265 | 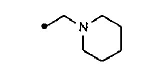 | 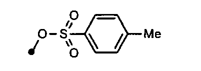 | 516 [M+H]⁺ |
| 266 | 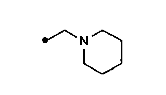 | 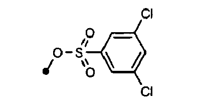 | 571 [M+H]⁺ |
| 267 | 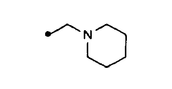 | 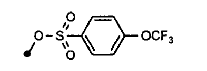 | 586 [M+H]⁺ |
| 268 | 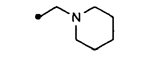 | 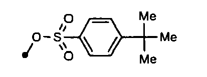 | 558 [M+H]⁺ |
| 269 | 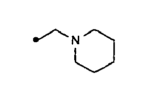 | 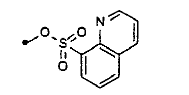 | 553 [M+H]⁺ |
| 270 | 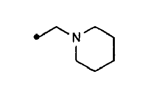 | 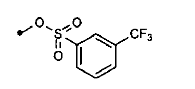 | 570 [M+H]⁺ |
| 271 | 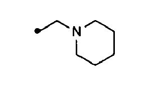 | 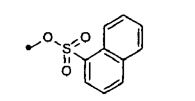 | 552 [M+H]⁺ |
| 272 | 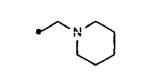 | 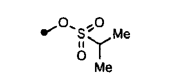 | 468 [M+H]⁺ |
| 273 | 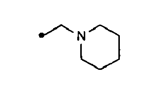 | 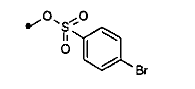 | 581 [M+H]⁺ |
| 274 | 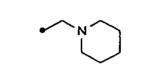 | 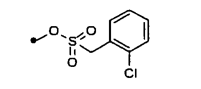 | 551 [M+H]⁺ |
| 275 | 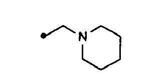 | 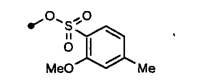 | 546 [M+H]⁺ |
| 276 | 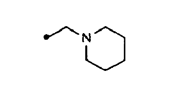 | 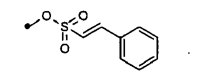 | 528 [M+H]⁺ |
| 277 | 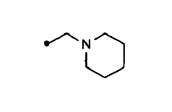 | 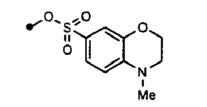 | 573 [M+H]⁺ |

**Table 4-7**

| Compound Number | R⁵ | R³ | Analytical data (MS m/z) |
|---|---|---|---|
| 278 | 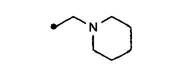 | 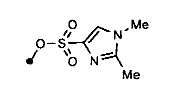 | 520 [M+H]⁺ |
| 279 | 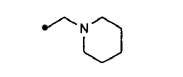 | 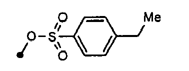 | 530 [M+H]⁺ |
| 280 | 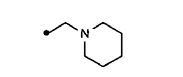 | 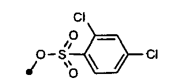 | 571 [M+H]⁺ |
| 281 | 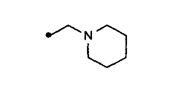 | 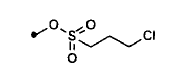 | 517 [M+H]⁺ |
| 282 | 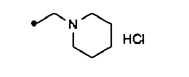 | 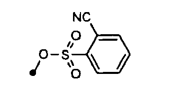 | 527 [M+H]⁺ |
| 283 | 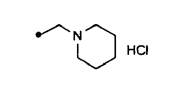 | 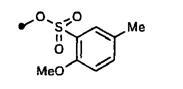 | 546 [M+H]⁺ |
| 284 | 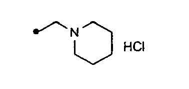 | 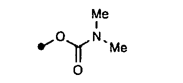 | 433 [M+H]⁺ |
| 285 | 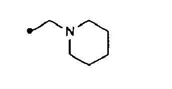 | 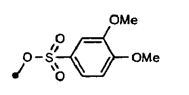 | 562 [M+H]⁺ |
| 286 | 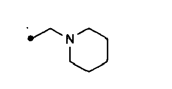 | 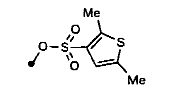 | 536 [M+H]⁺ |
| 287 | 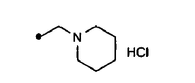 | 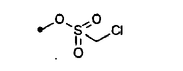 | 474 [M+H]⁺ |
| 288 | 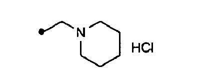 | 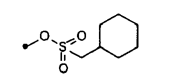 | 522 [M+H]⁺ |
| 289 | 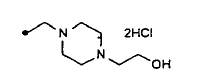 | 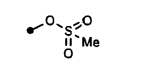 | 485 [M+H]⁺ |
| 290 | 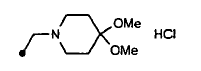 | 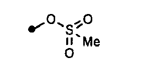 | 500 [M+H]⁺ |
| 291 | 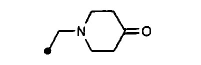 | 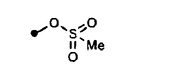 | 454 [M+H]⁺ |
| 292 | 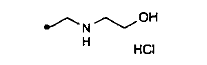 | 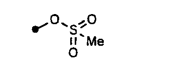 | 416 [M+H]⁺ |

**Table 4-8**

| Compound Number | R⁵ | R³ | Analytical data (MS m/z) |
|---|---|---|---|
| 293 | 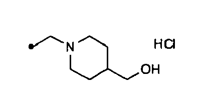 | 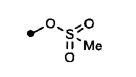 | 470 [M+H]⁺ |
| 294 | 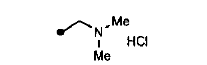 | 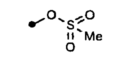 | 400 [M+H]⁺ |
| 295 | 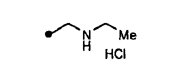 | 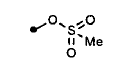 | 400 [M+H]⁺ |
| 296 | 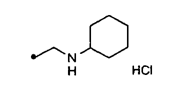 | 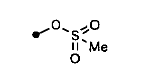 | 454 [M+H]⁺ |
| 297 | 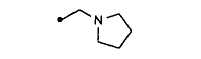 | 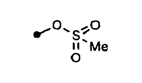 | 426 [M+H]⁺ |
| 298 | 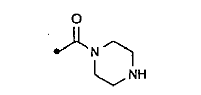 | 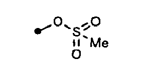 | 455 [M+H]⁺ |
| 299 | 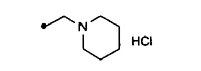 | 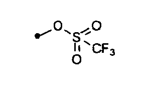 | 494 [M+H]⁺ |
| 300 | 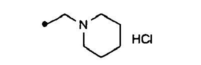 | Et | 374 [M+H]⁺ |
| 301 | 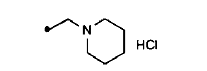 | Me | 360 [M+H]⁺ |
| 302 | 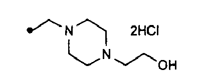 | Et | 419 [M+H]⁺ |
| 303 | 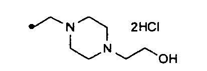 | Me | 405 [M+H]⁺ |
| 304 | 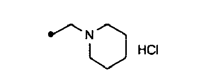 | 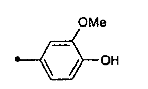 | 468 [M+H]⁺ |
| 305 | 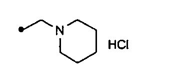 | 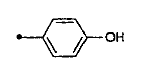 | 438 [M+H]⁺ |
| 306 | 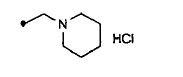 | 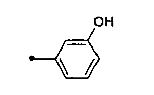 | 438 [M+H]⁺ |
| 307 | 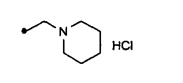 | 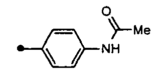 | 479 [M+H]⁺ |

**Table 4-9**

| Compound Number | R⁵ | R³ | Analytical data (MS m/z) |
|---|---|---|---|
| 308 | 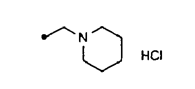 | 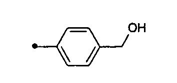 | 452 [M+H]⁺ |
| 309 | 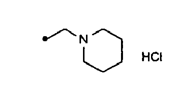 | 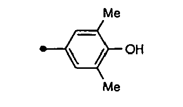 | 466 [M+H]⁺ |
| 310 | 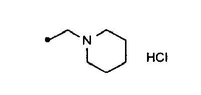 | 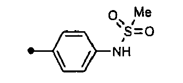 | 515 [M+H]⁺ |
| 311 | 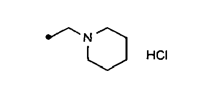 | 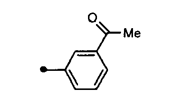 | 464 [M+H]⁺ |
| 312 | 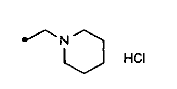 | 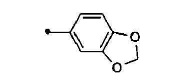 | 466 [M+H]⁺ |
| 313 | 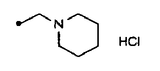 | 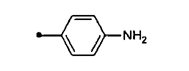 | 437 [M+H]⁺ |
| 314 | 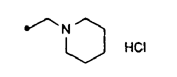 | 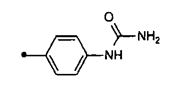 | 480 [M+H]⁺ |
| 315 | 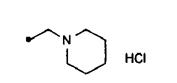 | 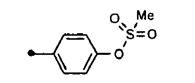 | 516 [M+H]⁺ |
| 316 | 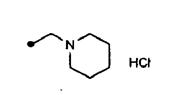 | 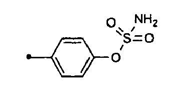 | 517 [M+H]⁺ |
| 317 | 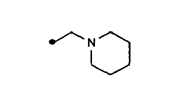 | 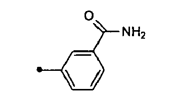 | 465 [M+H]⁺ |
| 318 | 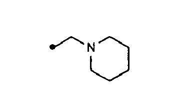 | 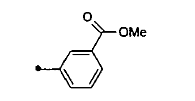 | 480 [M+H]⁺ |
| 319 | 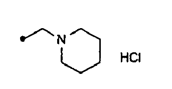 | 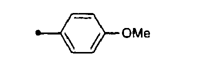 | 452 [M+H]⁺ |
| 320 | 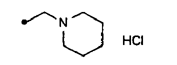 | 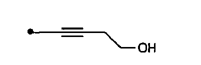 | 414 [M+H]⁺ |
| 321 | 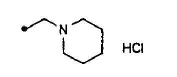 | 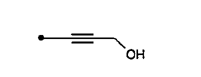 | 400 [M+H]⁺ |
| 322 | 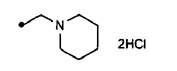 | 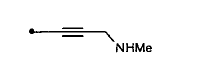 | 413 [M+H]⁺ |

**Table 4-10**

| Compound Number | R⁵ | R³ | Analytical data (MS m/z) |
|---|---|---|---|
| 323 | 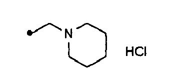 | 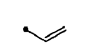 | 372 [M+H]⁺ |
| 324 | 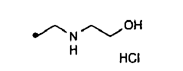 | Et | 350 [M+H]⁺ |
| 325 | 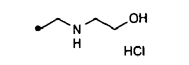 | Me | 336 [M+H]⁺ |
| 326 | 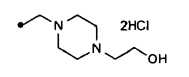 | 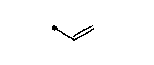 | 417 [M+H]⁺ |
| 327 | 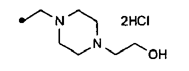 | 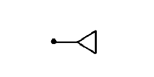 | 431 [M+H]⁺ |
| 328 | 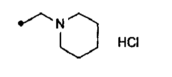 | 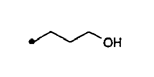 | 404 [M+H]⁺ |
| 329 | 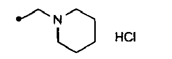 | 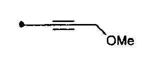 | 414 [M+H]⁺ |
| 330 | 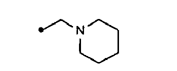 | 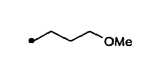 | 418 [M+H]⁺ |
| 331 | 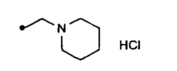 | 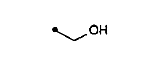 | 376 [M+H]⁺ |
| 332 | 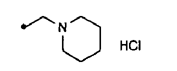 | CN | 371 [M+H]⁺ |
| 333 | 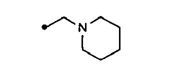 | NH₂ | 361 [M+H]⁺ |
| 334 | 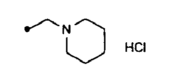 | 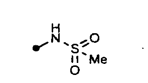 | 439 [M+H]⁺ |
| 335 | 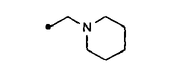 | 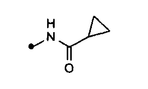 | 429 [M+H]⁺ |
| 336 | 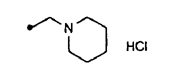 | H | 346 [M+H]⁺ |
| 337 | 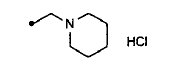 | F | 364 [M+H]⁺ |

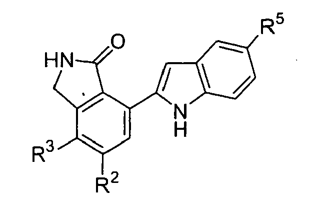

**Table 5-1**

| Compound Number | R² | R³ | R⁵ | Analytical data (MS m/z) |
|---|---|---|---|---|
| 338 | Cl | Cl | 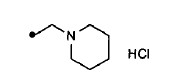 | 414 [M+H]⁺ |
| 339 | Cl | Cl | 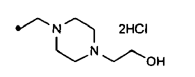 | 459 [M+H]⁺ |
| 340 | F | Cl | 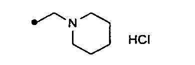 | 398 [M+H]⁺ |
| 341 | OMe | Cl | 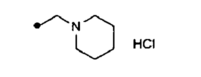 | 410 [M+H]⁺ |
| 342 | Me | Cl | 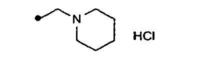 | 394 [M+H]⁺ |
| 343 | OMe | Cl | 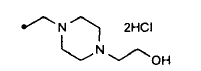 | 455 [M+H]⁺ |
| 344 | OMe | Cl | 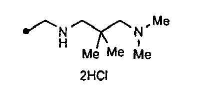 | 455 [M+H]⁺ |
| 345 | Me | Cl | 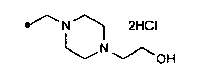 | 439 [M+H]⁺ |
| 346 | CH₂OMe | OH | 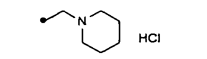 | 406 [M+H]⁺ |
| 347 | OH | Cl | 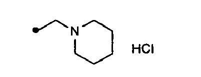 | 396 [M+H]⁺ |
| 348 | CH₂OMe | 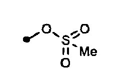 | 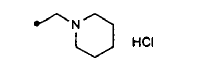 | 484 [M+H]⁺ |
| 349 | CH₂OMe | 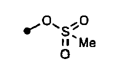 | 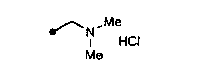 | 444 [M+H]⁺ |
| 350 | 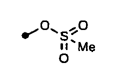 | Cl | 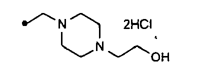 | 519 [M+H]⁺ |
| 351 | OMe | OH | 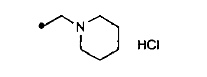 | 392 [M+H]⁺ |
| 352 | OMe | 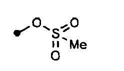 | 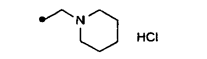 | 469 [M+H]⁺ |

**Table 5-2**

| Compound Number | R² | R³ | R⁵ | Analytical data (MS m/z) |
|---|---|---|---|---|
| 353 | Me | OH | 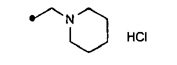 | 376 [M+H]⁺ |
| 354 | Me | 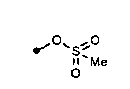 | 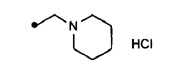 | 454 [M+H]⁺ |
| 355 | OMe | OMe | 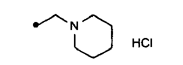 | 406 [M+H]⁺ |
| 356 | Me | 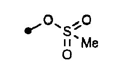 | 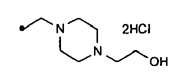 | 499 [M+H]⁺ |
| 357 | OMe | OH | 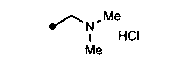 | 352 [M+H]⁺ |
| 358 | OMe | OH | 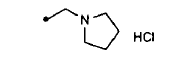 | 378 [M+H]⁺ |
| 359 | OMe | OH | 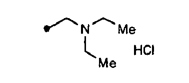 | 380 [M+H]⁺ |
| 360 | OMe | OH | 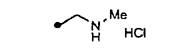 | 338 [M+H]⁺ |
| 361 | OMe | 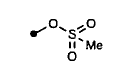 | 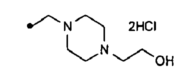 | 514 [M+H]⁺ |
| 362 | OMe | 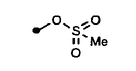 | 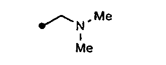 | 430 [M+H]⁺ |
| 363 | OMe | 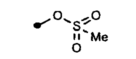 | 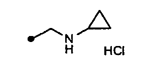 | 442 [M+H]⁺ |
| 364 | OMe | 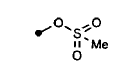 | 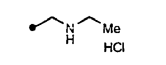 | 430 [M+H]⁺ |
| 365 | OMe | 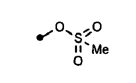 | 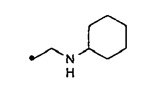 | 484 [M+H]⁺ |
| 366 | OMe | 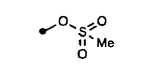 | 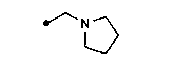 | 456 [M+H]⁺ |
| 367 | OMe | 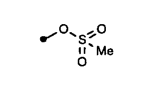 | 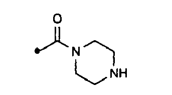 | 485 [M+H]⁺ |

**Table 5-3**

| Compound Number | R² | R³ | R⁵ | Analytical data (MS m/z) |
|---|---|---|---|---|
| 368 | OMe | 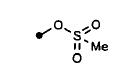 | 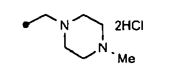 | 485 [M+H]⁺ |
| 369 | OMe | 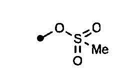 | 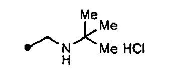 | 458 [M+H]⁺ |
| 370 | OMe | 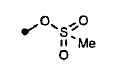 | NH₂·HCl | 388 [M+H]⁺ |
| 371 | OMe | 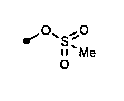 | 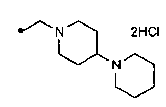 | 553 [M+H]⁺ |
| 372 | OMe | 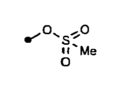 | 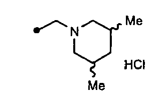 | 498 [M+H]⁺ |
| 373 | OMe | 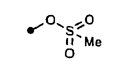 | 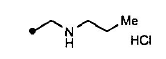 | 444 [M+H]⁺ |
| 374 | OMe | 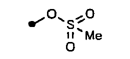 | 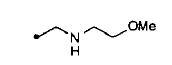 | 460 [M+H]⁺ |
| 375 | OMe | 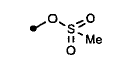 | 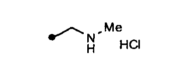 | 416 [M+H]⁺ |
| 376 | OMe | 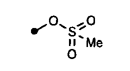 | 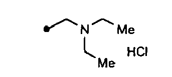 | 458 [M+H]⁺ |
| 377 | OMe | 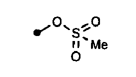 | 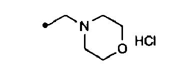 | 472 [M+H]⁺ |
| 378 | OMe | 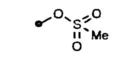 | 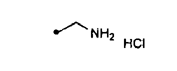 | 402 [M+H]⁺ |
| 379 | OMe | 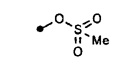 | Br | 452 [M+H]⁺ |
| 380 | OMe | 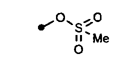 | OH | 389 [M+H]⁺ |
| 381 | OMe | 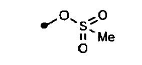 | H | 373 [M+H]⁺ |
| 382 | OMe | 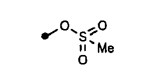 | 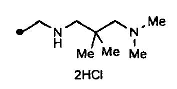 | 515 [M+H]⁺ |

**Table 5-4**

| Compound Number | R² | R³ | R⁵ | Analytical data (MS m/z) |
|---|---|---|---|---|
| 383 | OMe | 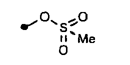 | 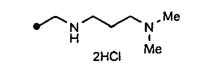 | 487 [M+H]⁺ |
| 384 | OMe | 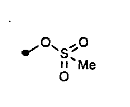 | 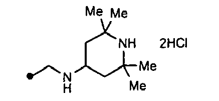 | 541 [M+H]⁺ |
| 385 | OMe | 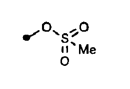 | 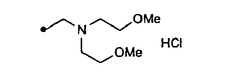 | 518 [M+H]⁺ |
| 386 | OMe | 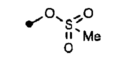 | 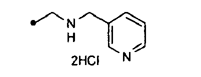 | 493 [M+H]⁺ |
| 387 | OMe | 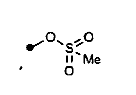 | 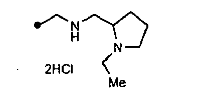 | 513 [M+H]⁺ |
| 388 | OMe | 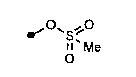 | 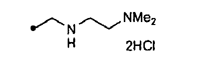 | 473 [M+H]⁺ |
| 389 | OMe | 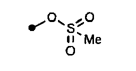 | 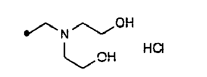 | 490 [M+H]⁺ |
| 390 | OMe | 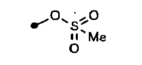 | 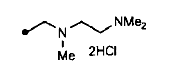 | 487 [M+H]⁺ |
| 391 | OMe | 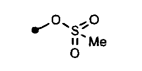 | 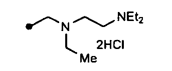 | 529 [M+H]⁺ |
| 392 | OMe | 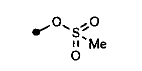 | 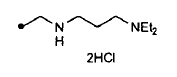 | 515 [M+H]⁺ |
| 393 | OMe | 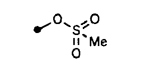 | 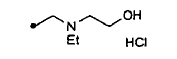 | 474 [M+H]⁺ |
| 394 | OMe | 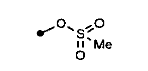 | 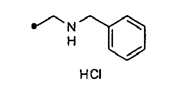 | 492 [M+H]⁺ |
| 395 | OMe | 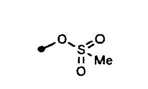 | 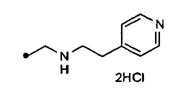 | 507 [M+H]⁺ |
| 396 | OMe | 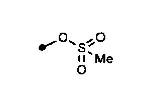 | 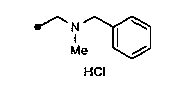 | 506 [M+H]⁺ |
| 397 | OMe | 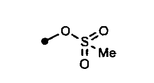 | 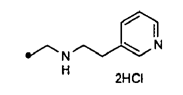 | 507 [M+H]⁺ |

**Table 5-5**

| Compound Number | R² | R³ | R⁵ | Analytical data (MS m/z) |
|---|---|---|---|---|
| 398 | OMe | 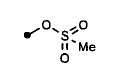 | 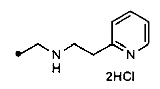 | 507 [M+H]⁺ |
| 399 | OMe | 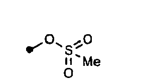 | 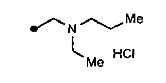 | 472 [M+H]⁺ |
| 400 | OMe | 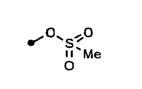 | 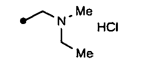 | 444 [M+H]⁺ |
| 401 | OMe | 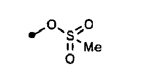 | 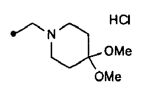 | 530 [M+H]⁺ |
| 402 | OMe | 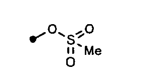 | 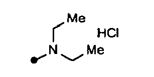 | 444 [M+H]⁺ |
| 403 | OMe | 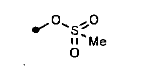 | 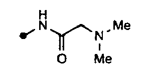 | 473 [M+H]⁺ |
| 404 | OMe | 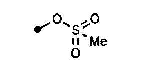 | 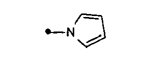 | 438 [M+H]⁺ |
| 405 | OMe | 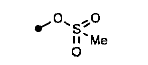 | 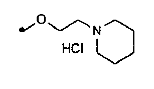 | 500 [M+H]⁺ |
| 406 | OMe | 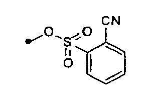 | 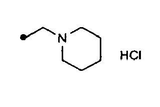 | 527 [M+H]⁺ |
| 407 | OMe | 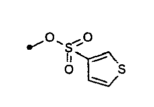 | 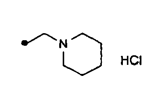 | 538 [M+H]⁺ |
| 408 | OMe | 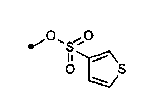 | 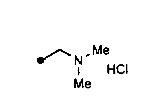 | 498 [M+H]⁺ |
| 409 | OMe | 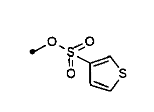 | 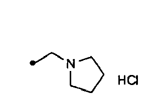 | 524 [M+H]⁺ |
| 410 | OMe | 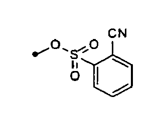 | 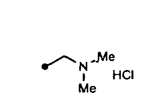 | 517 [M+H]⁺ |
| 411 | OMe | 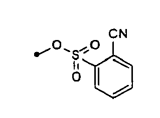 | 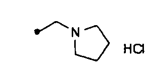 | 543 [M+H]⁺ |
| 412 | OMe | 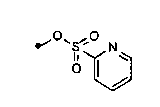 | 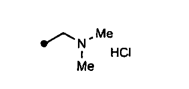 | 493 [M+H]⁺ |

**Table 5-6**

| | | | | |
|---|---|---|---|---|
| Compound Number | R² | R³ | R⁵ | Analytical data (MS m/z) |
| 413 | OMe | 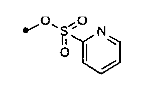 | 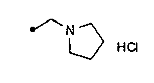 | 519 [M+H]⁺ |
| 414 | OMe | 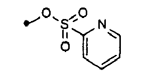 | 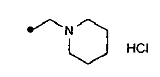 | 543 [M+H]⁺ |
| 415 | OMe | 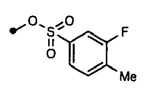 | 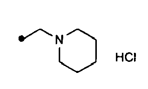 | 543 [M+H]⁺ |
| 416 | OMe | 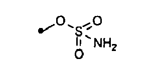 | 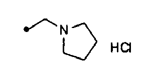 | 457 [M+H]⁺ |
| 417 | OMe | 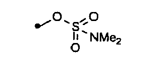 | 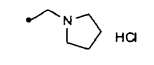 | 569 [M+H]⁺ |
| 418 | OMe | 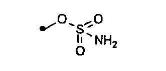 | 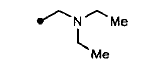 | 459 [M+H]⁺ |

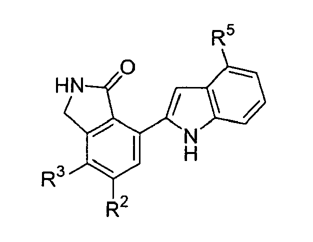

**Table 6**

| Compound Number | R² | R³ | R⁵ | Analytical data (MS m/z) |
|---|---|---|---|---|
| 419 | OMe | 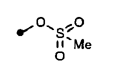 | 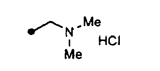 | 430 [M+H]⁺ |
| 420 | OMe | 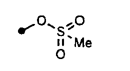 | 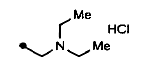 | 458 [M+H]⁺ |
| 421 | OMe | 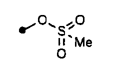 | 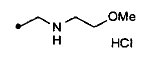 | 460 [M+H]⁺ |
| 422 | OMe | 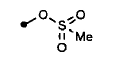 | 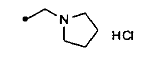 | 456 [M+H]⁺ |

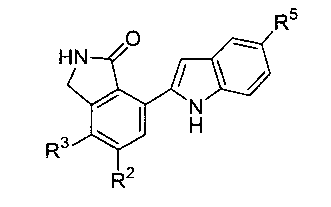

**Table 7-1**

| Compound Number | R² | R³ | R⁵ | Analytical data (MS m/z) |
|---|---|---|---|---|
| 423 | 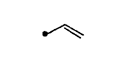 | Cl | 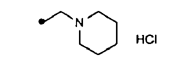 | 406 [M+H]⁺ |
| 424 | 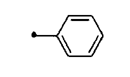 | Cl | 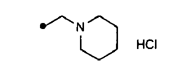 | 456 [M+H]⁺ |
| 425 | 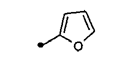 | Cl | 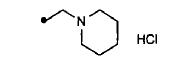 | 446 [M+H]⁺ |
| 426 | Et | Cl | 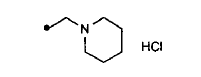 | 408 [M+H]⁺ |
| 427 | 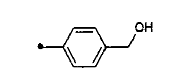 | Cl | 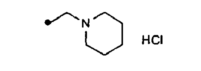 | 486 [M+H]⁺ |
| 428 | 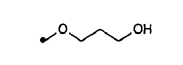 | Cl | 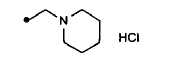 | 454 [M+H]⁺ |
| 429 | 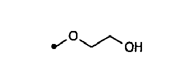 | Cl | 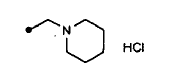 | 440 [M+H]⁺ |
| 430 | 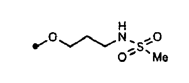 | Cl | 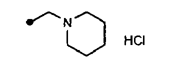 | 531 [M+H]⁺ |
| 431 | 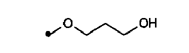 | Cl | 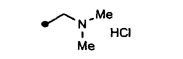 | 414 [M+H]⁺ |
| 432 | 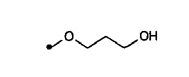 | Cl | 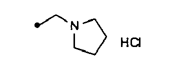 | 440 [M+H]⁺ |
| 433 | 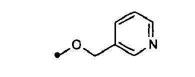 | Cl | 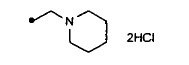 | 487 [M+H]⁺ |
| 434 | 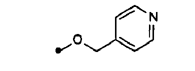 | Cl | 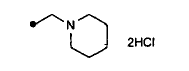 | 487 [M+H]⁺ |
| 435 | 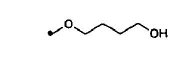 | Cl | 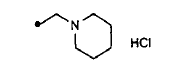 | 468 [M+H]⁺ |
| 436 | 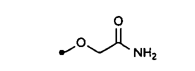 | Cl | 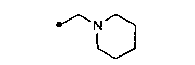 | 454 [M+H]⁺ |
| 437 | 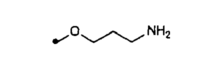 | Cl | 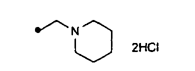 | 453 [M+H]⁺ |

**Table 7-2**

| Compound Number | R² | R³ | R⁵ | Analytical data (MS m/z) |
|---|---|---|---|---|
| 438 | 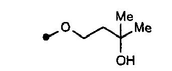 | Cl | 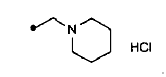 | 482 [M]⁺ |
| 439 | 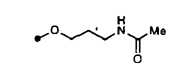 | Cl | 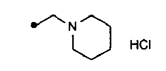 | 495 [M+H]⁺ |
| 440 | 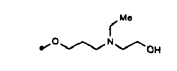 | Cl | 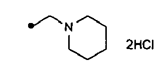 | 525 [M+H]⁺ |
| 441 | 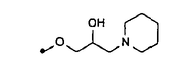 | Cl | 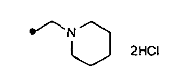 | 537 [M+H]⁺ |
| 442 | 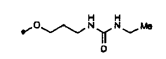 | Cl | 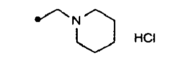 | 524 [M+H]⁺ |
| 443 | 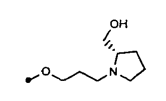 | Cl | 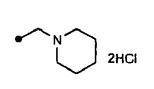 | 537 [M+H]⁺ |
| 444 | 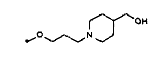 | Cl | 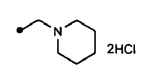 | 551 [M+H]⁺ |
| 445 | 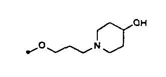 | Cl | 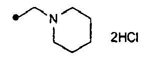 | 537 [M+H]⁺ |
| 446 | 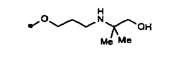 | Cl | 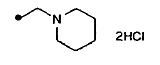 | 525 [M+H]⁺ |
| 447 | 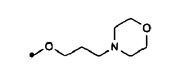 | Cl | 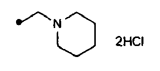 | 523 [M+H]⁺ |
| 448 | 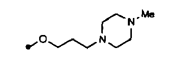 | Cl | 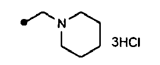 | 536 [M+H]⁺ |
| 449 | 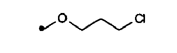 | Cl | 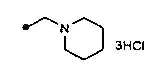 | 472 [M+H]⁺ |
| 450 | OMe | 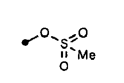 | 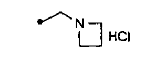 | 442 [M+H]⁺ |
| 451 | OMe | 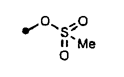 | 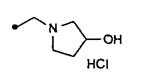 | 472 [M+H]⁺ |
| 452 | OMe | 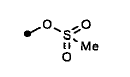 | 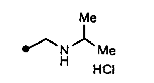 | 444 [M+H]⁺ |

**Table 7-3**

| Compound Number | R² | R³ | R⁵ | Analytical data (MS m/z) |
|---|---|---|---|---|
| 453 | OMe | 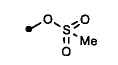 | 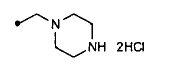 | 444 [M+H]⁺ |
| 454 | OMe | 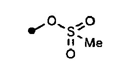 | 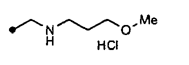 | 474 [M+H]⁺ |
| 455 | OMe | 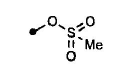 | 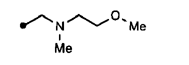 | 474 [M+H]⁺ |
| 456 | OMe | 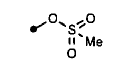 | CO₂H | 417 [M+H]⁺ |
| 457 | OMe | 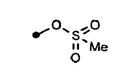 | 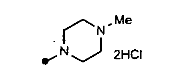 | 471 [M+H]⁺ |
| 458 | F | Cl | 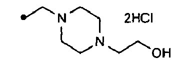 | 443 [M+H]⁺ |
| 459 | OMe | 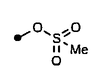 | 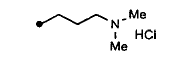 | 458 [M+H]⁺ |
| 460 | OMe | 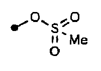 | 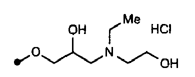 | 534 [M+H]⁺ |

**Table 8**

| Compound Number | Structure | Analytical data (MS m/z) |
|---|---|---|
| 461 | 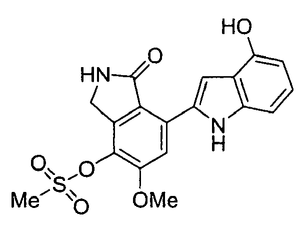 | 603 [M+H]⁺ |

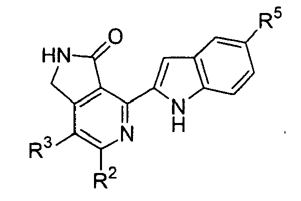

**Table 9**

| Compound Number | R² | R³ | R⁵ | Analytical data (MS m/z) |
|---|---|---|---|---|
| 462 | H | H | 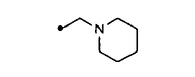 | 347 [M+H]⁺ |
| 463 | H | H | 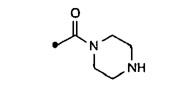 | 362 [M+H]⁺ |
| 464 | H | Cl | 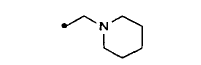 | 381 [M+H]⁺ |
| 465 | H | Cl | 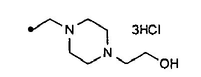 | 426 [M+H]⁺ |
| 466 | Me | H | 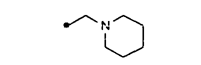 | 361 [M+H]⁺ |
| 467 | Me | H | 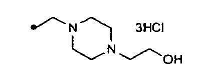 | 406 [M+H]⁺ |

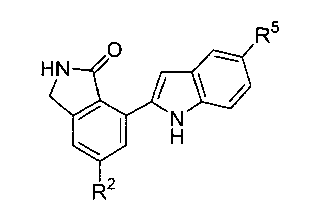

**Table 10-1**

| Example Number | Compound Number | R² | R⁵ |
|---|---|---|---|
| 1 | 468 | OH | 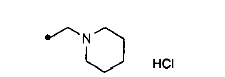 |
| 2 | 469 | 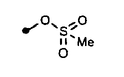 | 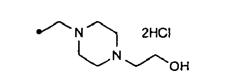 |
| 3 | 470 | OMe | 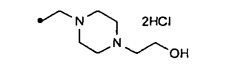 |
| 4 | 471 | NMe₂ HCl | 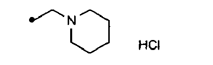 |
| 5 | 472 | NMe₂ HCl | 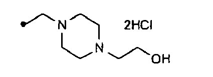 |
| 6 | 473 | 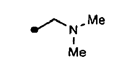 | 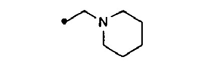 |
| 7 | 474 | CH₂OMe | 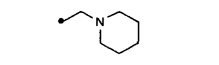 |
| 8 | 475 | 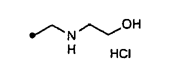 | 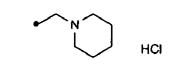 |
| 9 | 476 | 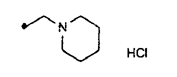 | 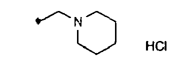 |
| 10 | 477 | CH₂OH | 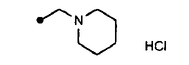 |
| 11 | 478 | 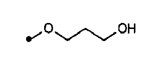 | 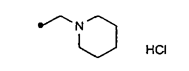 |
| 12 | 479 | CH₂NH₂ HCl | 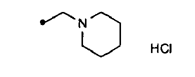 |
| 13 | 480 | 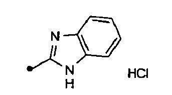 | H |
| 14 | 481 | 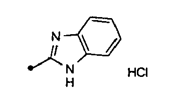 | 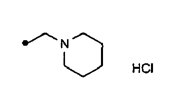 |
| 15 | 482 | 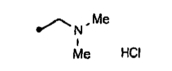 | H |

**Table 10-2**

| Example Number | Compound Number | R² | R⁵ |
|---|---|---|---|
| 16 | 483 | 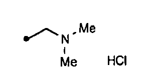 | 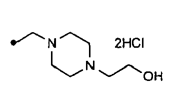 |
| 17 | 484 | 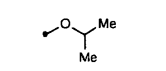 | 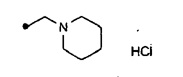 |
| 18 | 485 | 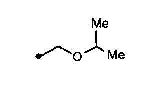 | 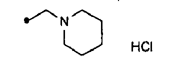 |
| 19 | 486 | 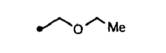 | 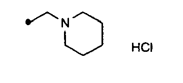 |
| 20 | 487 | 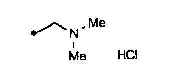 | NH₂ HCl |
| 21 | 488 | 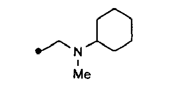 | 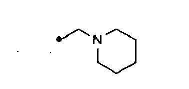 |
| 22 | 489 | 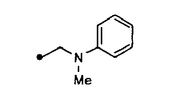 | 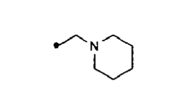 |
| 23 | 490 | 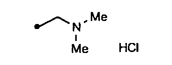 | 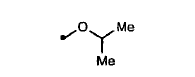 |
| 24 | 491 | 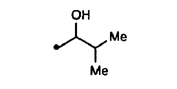 | 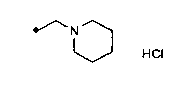 |
| 25 | 492 | 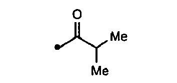 | 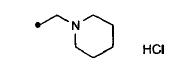 |
| 26 | 493 | 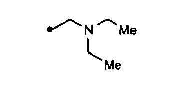 | 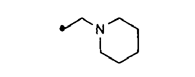 |
| 27 | 494 | 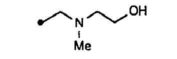 | 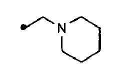 |
| 28 | 495 | 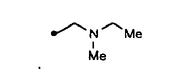 | 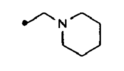 |
| 29 | 496 | 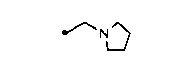 | 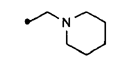 |
| 30 | 497 | 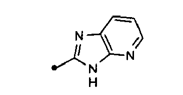 | 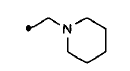 |

**Table 10-3**

| Example Number | Compound Number | R² | R⁵ |
|---|---|---|---|
| 31 | 498 | 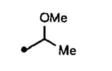 | 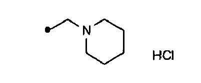 |
| 32 | 499 | 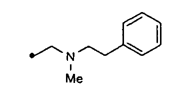 | 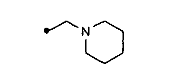 |
| 33 | 500 | 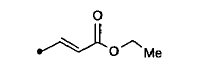 | 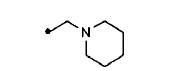 |
| 34 | 501 | 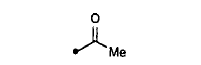 | 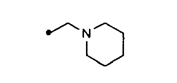 |
| 35 | 502 | 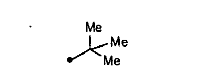 | 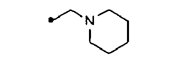 |
| 36 | 503 | 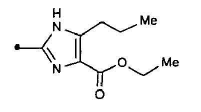 | 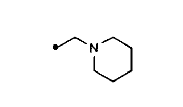 |
| 37 | 504 | 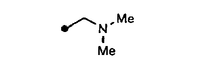 | 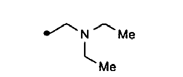 |
| 38 | 505 | F | 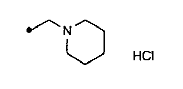 |
| 39 | 506 | 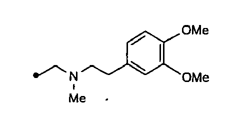 | 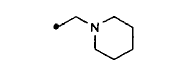 |
| 40 | 507 | Cl | 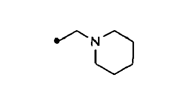 |
| 41 | 508 | 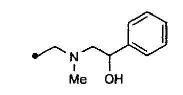 | 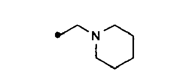 |
| 42 | 509 | 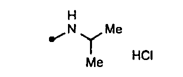 | 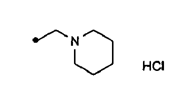 |
| 43 | 510 | 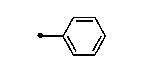 | 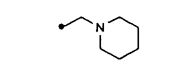 |
| 44 | 511 | 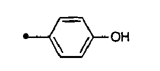 | 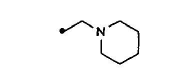 |
| 45 | 512 | 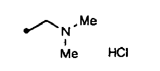 | 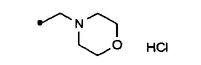 |

**Table 10-4**

| Example Number | Compound Number | R⁵ | R² |
|---|---|---|---|
| 46 | 513 | 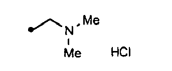 | 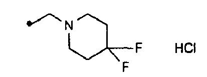 |
| 47 | 514 | 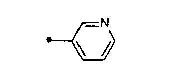 | 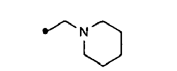 |
| 48 | 515 | 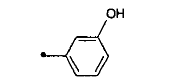 | 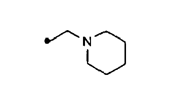 |
| 49 | 516 | 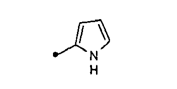 | 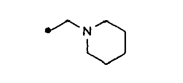 |
| 50 | 517 | 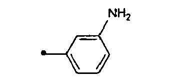 | 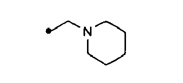 |
| 51 | 518 | 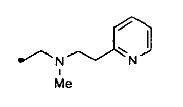 | 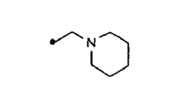 |
| 52 | 519 | 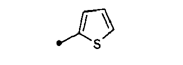 | 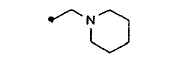 |
| 53 | 520 | 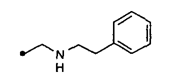 | 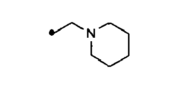 |
| 54 | 521 | 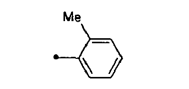 | 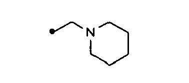 |
| 55 | 522 | 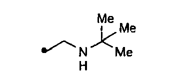 | 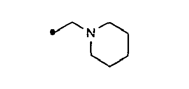 |
| 56 | 523 | 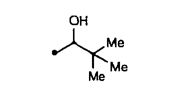 | 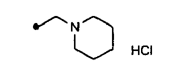 |
| 57 | 524 | 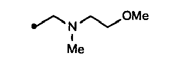 | 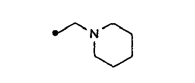 |
| 58 | 525 | 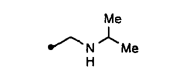 | 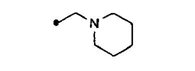 |
| 59 | 526 | 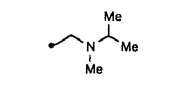 | 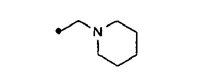 |
| 60 | 527 | 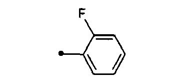 | 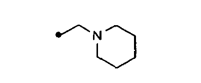 |

**Table 10-5**

| Example Number | Compound Number | R² | R⁵ |
|---|---|---|---|
| 61 | 528 | 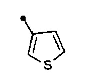 | 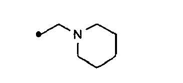 |
| 62 | 529 | CONMe₂ | 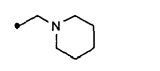 |
| 63 | 530 | 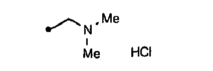 | 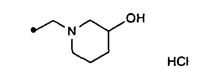 |
| 64 | 531 | 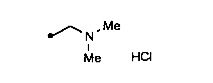 | 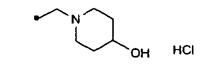 |
| 65 | 532 | 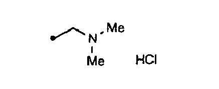 | 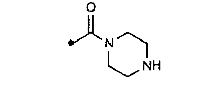 |
| 66 | 533 | 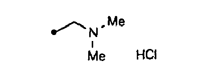 | CO₂Me |
| 67 | 534 | 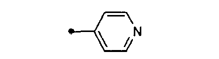 | 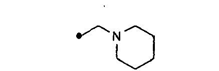 |
| 68 | 535 | 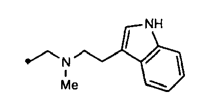 | 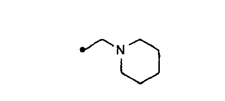 |
| 69 | 536 | 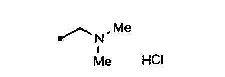 | 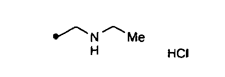 |
| 70 | 537 | 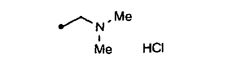 | 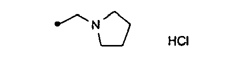 |
| 71 | 538 | 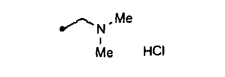 | 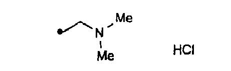 |
| 72 | 539 | 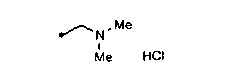 | 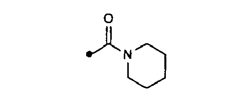 |
| 73 | 540 | 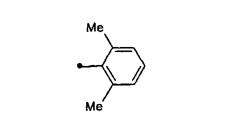 | 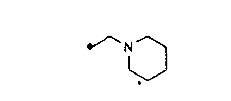 |
| 74 | 541 | 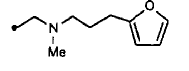 | 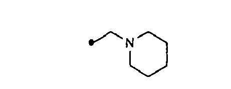 |
| 75 | 542 | 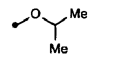 | H |

**Table 10-6**

| Example Number | Compound Number | R² | R⁵ |
|---|---|---|---|
| 76 | 543 | 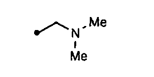 | 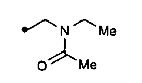 |
| 77 | 544 | 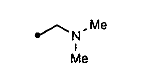 | 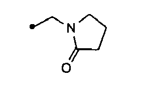 |
| 78 | 545 | 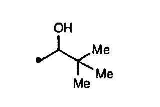 | H |
| 79 | 546 | Br | 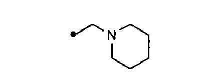 |
| 80 | 547 | 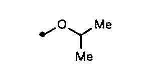 | 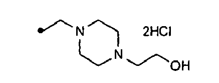 |
| 81 | 548 | 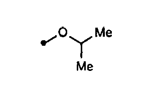 | 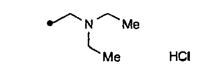 |
| 82 | 549 | 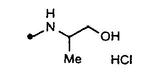 | 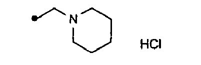 |
| 83 | 550 | 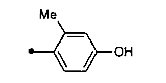 | 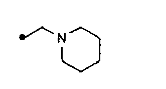 |
| 84 | 551 | 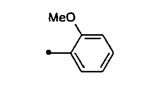 | 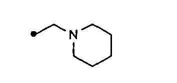 |
| 85 | 552 | 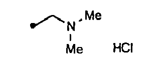 | 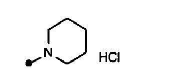 |
| 86 | 553 | 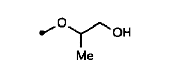 | 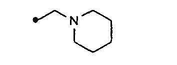 |
| 87 | 554 | 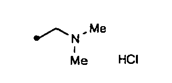 | 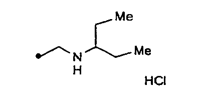 |
| 88 | 555 | 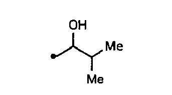 | 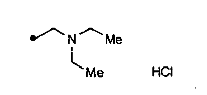 |
| 89 | 556 | 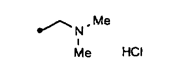 | 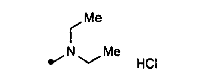 |
| 90 | 557 | 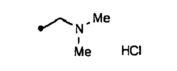 | 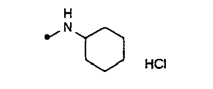 |

**Table 10-7**

| Example Number | Compound Number | R² | R⁵ |
|---|---|---|---|
| 91 | 558 | 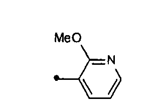 | 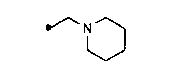 |
| 92 | 559 | 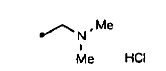 | 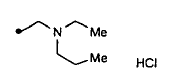 |
| 93 | 560 | 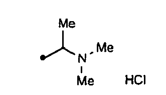 | 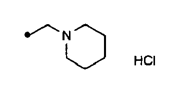 |
| 94 | 561 | 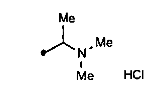 | 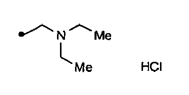 |

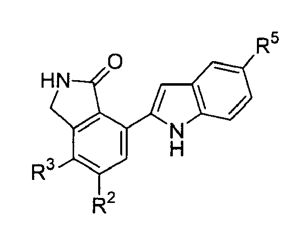

**Table 11**

| Example Number | Compound Number | R² | R³ | R⁵ |
|---|---|---|---|---|
| 95 | 562 | 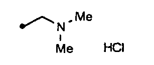 | OH | 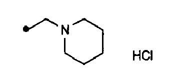 |
| 96 | 563 | 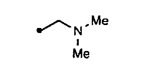 | 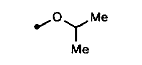 | 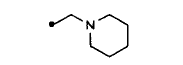 |
| 97 | 564 | 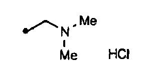 | 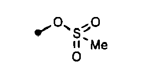 | 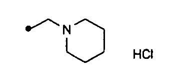 |
| 98 | 565 | 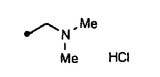 | 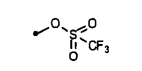 | 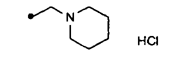 |

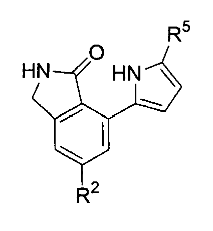

**Table 12**

| Example Number | Compound Number | R² | R⁵ |
|---|---|---|---|
| 99 | 566 | 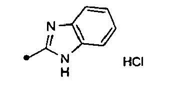 | H |
| 100 | 567 | CH₂OMe | CHO |
| 101 | 568 | 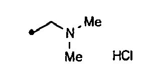 | H |
| 102 | 569 | 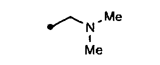 | CHO |
| 103 | 570 | 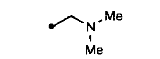 | 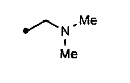 |
| 104 | 571 | 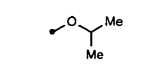 | H |

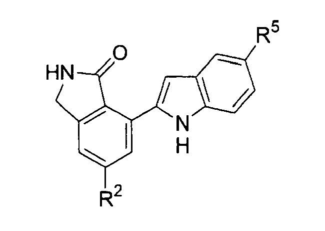

**Table 13-1**

| Example Number | Compound Number | R² | R⁵ |
|---|---|---|---|
| 105 | 572 | -NH₃⁺Cl⁻ | 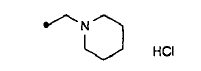 |
| 106 | 573 | 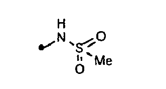 | 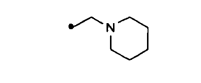 |
| 107 | 574 | 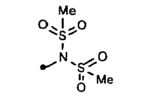 | 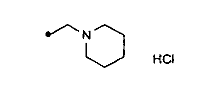 |
| 108 | 575 | 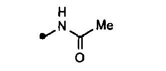 | 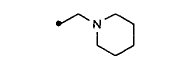 |
| 109 | 576 | 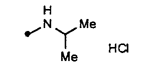 | 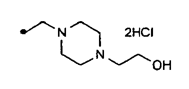 |
| 110 | 577 | 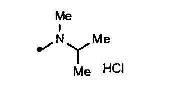 | 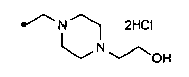 |
| 111 | 578 | 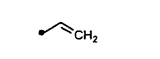 | 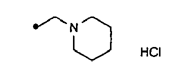 |
| 112 | 579 | 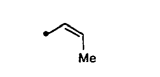 | 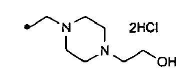 |
| 113 | 580 | 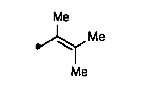 | 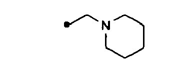 |
| 114 | 581 | 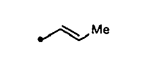 | 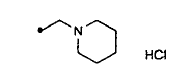 |
| 115 | 582 | 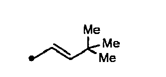 | 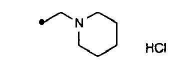 |
| 116 | 583 | 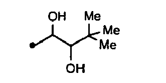 | 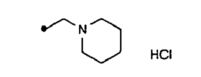 |
| 117 | 584 | 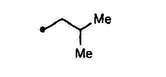 | 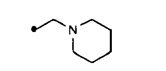 |
| 118 | 585 | 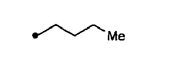 | 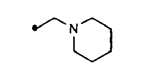 |
| 119 | 586 | 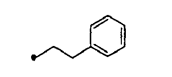 | 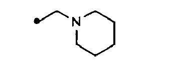 |

**Table 13-2**

| Example Number | Compound Number | R² | R⁵ |
|---|---|---|---|
| 120 | 587 | 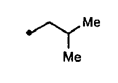 | 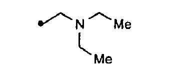 |
| 121 | 588 | Me | 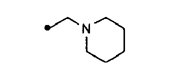 |
| 122 | 589 | 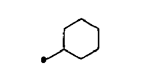 | 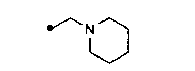 |
| 123 | 590 | 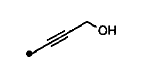 | 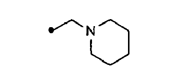 |
| 124 | 591 | 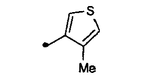 | 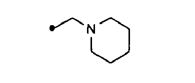 |
| 125 | 592 | 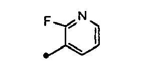 | 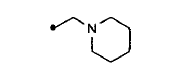 |
| 126 | 593 | 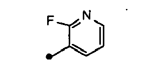 | 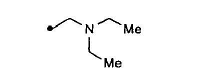 |
| 127 | 594 | 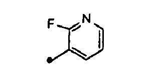 | 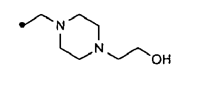 |
| 128 | 595 | 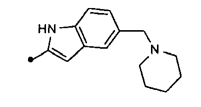 | 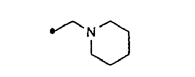 |
| 129 | 596 | 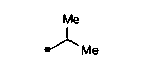 | 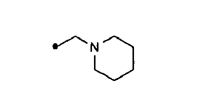 |
| 130 | 597 | 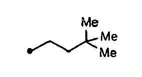 | 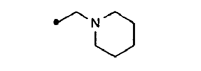 |
| 131 | 598 | 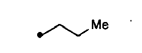 | 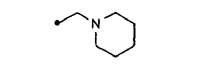 |
| 132 | 599 | 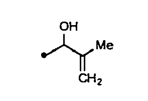 | 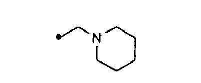 |
| 133 | 600 | 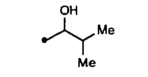 | 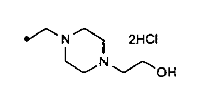 |
| 134 | 601 | 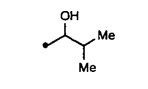 | 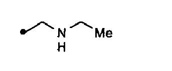 |
| 135 | 602 | 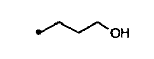 | 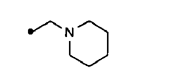 |

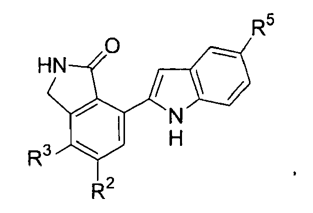

**Table 14**

| Example Number | Compound Number | R² | R³ | R⁵ |
|---|---|---|---|---|
| 136 | 603 | 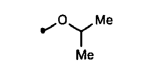 | Cl | 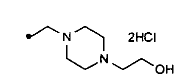 |
| 137 | 604 | 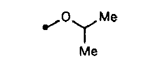 | Cl | 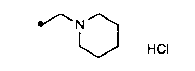 |
| 138 | 605 | 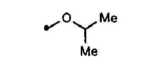 | Cl | 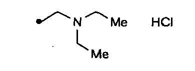 |
| 139 | 606 | NH₂ | Cl | 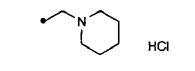 |
| 140 | 607 | 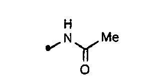 | Cl | 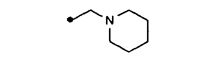 |
| 141 | 608 | 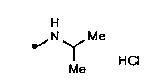 | Cl | 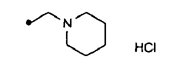 |
| 142 | 609 | 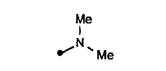 | Cl | 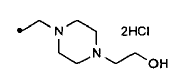 |
| 143 | 610 | 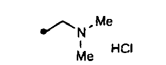 | Cl | 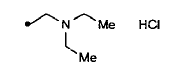 |
| 144 | 611 | 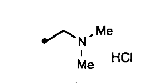 | Cl | 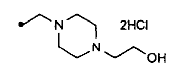 |
| 145 | 612 | 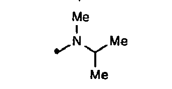 | Cl | 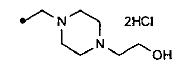 |
| 146 | 613 | 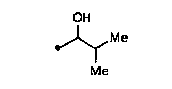 | Cl | 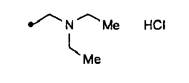 |
| 147 | 614 | 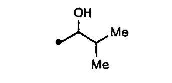 | Cl | 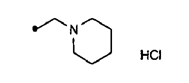 |
| 148 | 615 | 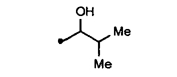 | Cl | 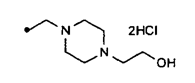 |

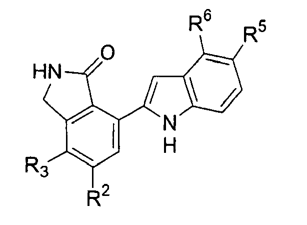

**Table 15**

| Example Number | Compound Number | R² | R³ | R⁵ | R⁶ |
|---|---|---|---|---|---|
| 149 | 616 | 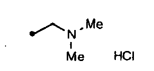 | H | H | OH |
| 150 | 617 | 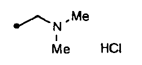 | H | H | 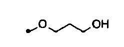 |
| 151 | 618 | 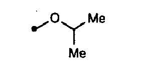 | Cl | 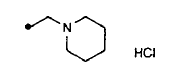 | OH |
| 152 | 619 | 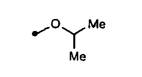 | Cl | 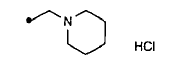 | 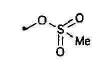 |
| 153 | 620 | 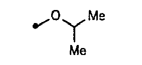 | Cl | 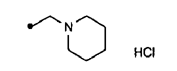 | 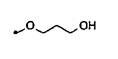 |
| 154 | 621 | 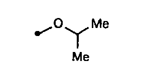 | Cl | 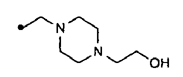 | 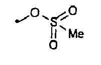 |

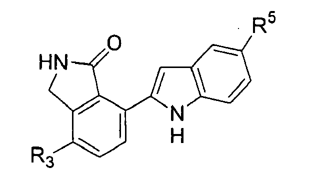

**Table 16**

| Example Number | Compound Number | R³ | R⁵ |
|---|---|---|---|
| 155 | 622 | 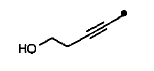 | 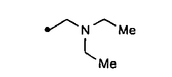 |
| 156 | 623 | 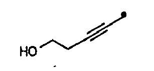 | 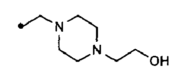 |
| 157 | 624 | 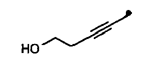 | 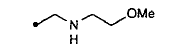 |
| 158 | 625 | 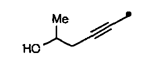 | 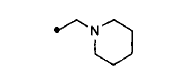 |
| 159 | 626 | 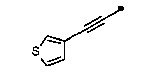 | 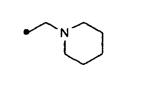 |
| 160 | 627 | 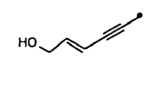 | 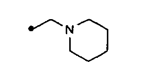 |
| 161 | 628 | 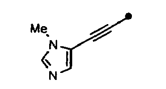 | 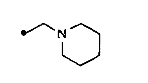 |

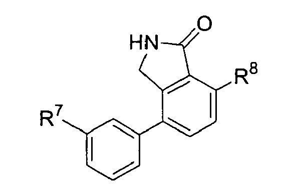

**Table 17**

| Example Number | Compound Number | R⁷ | R⁸ |
|---|---|---|---|
| 162 | 629 | 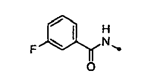 | Ph |
| 163 | 630 | 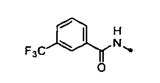 | 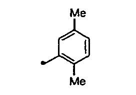 |
| 164 | 631 | 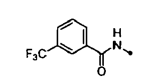 | 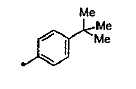 |
| 165 | 632 | 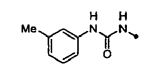 | Ph |
| 166 | 633 | 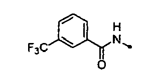 | 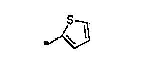 |
| 167 | 634 | 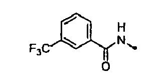 | 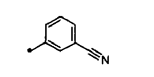 |
| 168 | 635 | 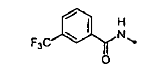 | 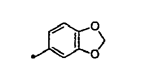 |
| 169 | 636 | 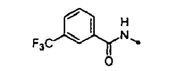 | 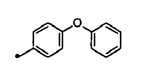 |

The pharmacologic actions of compounds (I), (II), (III), (IV), (V), and (VI) will now be described based on test examples.

### Test Example 1: JAK2 enzyme inhibitory activity

In order to measure the JAK2 enzyme inhibitory activity, the following process was employed. A protein with a His (histidine) tag fused at the N terminal of a functional domain (826-1132 amino acids) of human JAK2 was used as JAK2. After a biotinylated Lyn-substrate peptide serving as a substrate was immobilized on a 96-well plate coated with NeutrAvidin (Pierce, catalog No. 31000), the peptide was blocked with a 0.25% gelatin (Bio-Rad Laboratories, Inc., catalog No. 170-6537) so the plate could be used as a plate for measuring kinase reactions. A sample containing, in terms of final concentrations in a volume of 60 µL, 0.3 pg/L His-tagged JAK2 protein, 20 mmol/L Tris·Cl (pH 7.5), 5 mmol/L β-glycerophosphate, 2 mmol/L dithiothreitol, 0.1 mmol/L Na₃VO₄, 5 mmol/L MgCl₂, 5 mmol/L MnCl₂, 10 µmol/L ATP, 0.1% albumin bovine serum (BSA, Sigma Chemical Co., catalog No. A4503), 0.01% Briji 35 (Merck Chemicals, catalog No. 1.01894.0100), 0.1% dimethylsulfoxide (DMSO), 10 µmol/L test compound was prepared and added to wells of the plate for measuring kinase reactions, and enzymatic reactions were conducted at 30°C for 60 minutes. The plate was washed three times with TBS-T (10 mmol/L Tris·Cl (pH 7.5), 150 mmol/L NaCl, 0.05% Tween 20 (Bio-Rad Laboratories, Inc., catalog No. 170-6531)), then reacted with an europium-labeled anti-phosphotyrosine antibody (ParkinElmer Co., Ltd., catalog No. AD0160) for 60 minutes at room temperature, and washed with TBS-T three times. DELFIA Enhancement Solution (ParkinElmer Co., Ltd., catalog No. 1244-105) was added thereto and the time-resolved fluorescence (excitation wavelength: 340 nm, measurement wavelength: 615 nm) was measured. The relative activity (%) of the well containing the test compound was calculated by assuming the activity of the well of the enzyme to which 0.1% DMSO was added as 100% and the activity of the well to which no enzyme was added as 0%, and the difference between 100 and the obtained value was assumed to be the JAK2 inhibition ratio (%) of the test compound.

Compounds 305, 320, 483, 484, 504, 509, 550, 555, 562, and 571 showed a JAK2 inhibition ratio of 50% or more at a concentration of 1 µmol/L. These results indicate that Compound (I) used in the present invention shows effective JAK2 inhibitory activity.

### Test Example 2: Cell growth inhibitory activity against human acute myeloid leukemia cell line

The cell growth inhibitory activity of test compound against a human acute myeloid leukemia cell line (HEL92.1.7) (ATCC No. TIB-180) having a JAK2 V617F mutation was measured according to the following process.

A Roswell Park Memorial Institute's Medium (RPMI) 1640 medium (GIBCO, catalog No. 11875-093) containing 10% fetal bovine serum (GIBCO, catalog No. 10437-028) was used to culture each cells. HEL92.1.7 cells controlled at 2. 5 × 10⁴ cells/mL were inoculated into a TC MICROWELL 96U plate (Nalge Nunc, catalog No. 163320) at 80 µL per well, and were cultured at 37°C for 4 hours in a 5% carbon dioxide gas incubator. Then, 20 µL per well of DMSO solutions of test compounds adjusted to a final concentration of 10 µmol/L were added to the HEL92.1.7 cells, and the resulting mixtures were cultured again at 37°C for 72 hours in a 5% carbon dioxide gas incubator. Finally, 25 µL of WST-1 reagent {4-[3-(4-indophenyl)-2-(4-nitrophenyl)-2H-5-tetrazolio]-1,3-benz ene disulfonate sodium salt} (Roche Diagnostics K.K., catalog No. 1644807) diluted to 40% with the medium described above was added, and incubation was conducted at 37°C for 2 hours, followed by measuring absorbance at 450 nm (reference wavelength: 690 nm) with a microplate spectrophotometer SPECTRA max 340PC (Molecular Devices). As for the cell growth inhibition ratio, the relative activity (%) of the well containing a test compound was calculated by assuming the activity of the well in which cells were similarly cultured with addition of DMSO as 100% and the activity of the well in which cells were similarly cultured in a medium to which only DMSO was added as 0%, and then the difference between 100 and the obtained value was assumed as the growth inhibition ratio (%) of the test compound. The higher the value, the stronger the growth inhibition activity against the cells.

Compounds 305, 320, 483, 484, 504, 509, 550, 555, 562, and 571 showed a cell growth inhibition ratio of 50% or more at a concentration of 10 µmol/L against HEL92.1.7. These results show that Compound (I) used in the present invention exhibits a cell growth inhibitory activity against a human acute myeloid leukemia cell line (HEL92.1.7) having a JAK2 V617F mutation.

Compounds (I), (II), (III), (IV), (V), and (VI), or pharmaceutically acceptable salts thereof can be used as they are or in various pharmaceutical forms in accordance with the pharmacological action, the purpose of administration, or the like. A pharmaceutical composition of the present invention can be produced by homogeneously mixing Compounds (I), (II), (III), (IV), (V), or (VI), or pharmaceutically acceptable salts thereof in an amount that is effective as an active ingredient with a pharmaceutically acceptable carrier. The forms of this carrier can be in a wide range in accordance with the drug formulation desirable for the administration. The pharmaceutical composition is preferably in a unit dosage form suitable for oral administration or parenteral administration such as injection.

In the preparation of tablets, for example, excipients such as lactose and mannitol, disintegrators such as starch, lubricants such as magnesium stearate, binders such as polyvinyl alcohol and hydroxypropyl cellulose, surfactants such as sucrose fatty acid esters and sorbitol fatty acid esters and the like may be used in accordance with a known procedure. Tablets containing 1 to 200 mg of an active ingredient per tablet are preferred.

In the preparation of injections, water; physiological saline; vegetable oil such as olive oil and peanut oil; solvents such as ethyl oleate and propylene glycol; solubilizing agents such as sodium benzoate, sodium salicylate, and urethane; isotonizing agents such as sodium chloride and glucose; preservatives such as phenol, cresol, p-hydroxybenzoates, and chlorobutanol; and antioxidants such as ascorbic acid and sodium pyrosulfite and the like may be used by a known procedure.

Compounds (I), (II), (III), (IV), (V), and (VI), or pharmaceutically acceptable salts thereof can be administered either orally or parenterally in the form of an injection or the like. The effective dose and frequency of administration are different depending on, for example, the dosage form, the age, the weight, and the symptom of a patient and the like. In general, Compounds (I), (II), (III), (IV), (V), and (VI), or pharmaceutically acceptable salts thereof are preferably administered in an amount in the range of 0.01 to 100 mg/kg per day.

The present invention will now be described in further detail by examples and reference examples shown below, but the present invention is not limited thereto.

In proton nuclear magnetic resonance spectra (¹H-NMR), exchangeable hydrogen may not be clearly observed in some compounds and under some measuring conditions. With regard to indication of multiplicity of signals, commonly used notation is used here, and br denotes a broad signal when visually observed.

Analytical data of each compound in the reference examples and examples below were measured using the following analytical instruments.

¹H-NMR: JEOL JNM-EX270 (270 MHz) or JEOL JNM-AL300 (300 MHz) MS: JEOL SX-102AQQ (FAB method), JEOL JMS-DX303 (FAB method), Micromass Quattro (APCI method) or Micromass LCT (ESI, APCI method)

### Reference Example 1-1 (Compound BA)

### Step 1

5-Formylindole (200 mg, 1.38 mmol) was dissolved in acetonitrile (2 mL), and the solution was added with di-tert-butyldicarbonate (0.349 mL, 1.52 mmol) and DMAP (1.7 mg, 0. 014 mmol), followed by stirring at room temperature for 1 hour. The solvent was evaporated under reduced pressure, and the residue was dissolved in methanol (2 mL). Trimethyl orthoformate (0.300 mL, 2.75 mmol) and p-toluenesulfonic acid monohydrate (5.2 mg, 0.027 mmol) were added thereto, and the mixture was stirred at room temperature for one hour. An aqueous saturated sodium hydrogencarbonate solution and water were added to the reaction mixture, and the mixture was then extracted with ethyl acetate. The organic layer was washed with saturated brine and then dried over anhydrous sodium sulfate. The solvent was evaporated under reduced pressure to obtain 1-(tert-butoxycarbonyl)-5-(dimethoxymethyl)indole (396 mg, yield 99%).
ESI-MS m/z: 260 [M-CH₃O]⁺; ¹H-NMR (CDCl₃)δ(ppm): 1.67 (s, 9H), 3.34 (s, 6H), 5.50 (s, 1H), 6.57 (d, J = 3.7 Hz, 1H), 7.40 (dd, J = 1.5, 8.4 Hz, 1H), 7.60 (d, J = 3.7 Hz, 1H), 7.66 (d, J = 1.5 Hz, 1H), 8.13 (d, J = 8.4 Hz, 1H).

### Step 2

1-(tert-Butoxycarbonyl)-5-(dimethoxymethyl)indole (396 mg, 1.36 mmol) was dissolved in THF (2 mL), and the solution was added with triisopropyl borate (0.478 mL, 2.07 mmol). In an argon atmosphere, an LDA-heptane/THF/ethylbenzene solution (2.0 mol/L, 1.73 mL, and 3.5 mmol) was added dropwise at 0°C over a period of 10 minutes, followed by stirring at the same temperature for 30 minutes. An aqueous saturated ammonium chloride solution (4 mL) and a 10% aqueous potassium hydrogensulfate solution (10 mL) were added to the reaction mixture. The pH of the reaction mixture was adjusted to 2, and the mixture was then stirred at room temperature for 2 hours. The reaction mixture was extracted with ethyl acetate. The organic layer was washed with saturated brine and then dried over anhydrous sodium sulfate. The solvent was then evaporated under reduced pressure. The residue was suspended in diisopropyl ether/hexane (1/1), and the suspension was stirred under ice cooling for 30 minutes. The solid was collected by filtration and washed with diisopropyl ether/hexane (1/1). The product was then dried under reduced pressure to obtain Compound BA (303 mg, yield 77%). ESI-MS *m*/*z*: 288 [M-H]⁻; ¹H-NMR (CDCl₃)δ(ppm): 1.77 (s, 9H), 6.69 (s, 2H), 7.57 (s, 1H), 7.90 (dd, *J* = 1.8, 8.8 Hz, 1H), 8.13 (d, *J* = 1.8 Hz, 1H), 8.14 (d, *J* = 8.8 Hz, 1H), 10.08 (s, 1H).

### Reference Example 1-2 (Compound BB)

### Step 1

Indole-5-carboxylic acid (1.00 g, 6.21 mmol) was dissolved in DMF (10 mL) and the solution was added with EDCI (2.38 g, 12.4 mmol), HOBT monohydrate (839 mg, 6.21 mmol) and 1-(*tert*-butoxycarbonyl)piperazine (1.73 g, 9.29 mmol), followed by stirring at room temperature for 3.5 hours. The reaction mixture was added with water and extracted with ethyl acetate. The organic layer was washed with saturated aqueous sodium hydrogencarbonate solution, 1 mol/L hydrochloric acid, and saturated brine, and was dried over anhydrous sodium sulfate. The solvent was evaporated under reduced pressure to obtain 5-[4-(*tert*-butoxycarbonyl)piperazin-1-ylcarbonyl]indole (2.26 g).

### Step 2

5-[4-(*tert*-Butoxycarbonyl)piperazin-1-ylcarbonyl]indole (2.26 g) was dissolved in acetonitrile (20 mL) and the solution was added with di-*tert*-butyldicarbonate (3.15 mL, 13.7 mmol) and DMAP (84 mg, 0.69 mmol), followed by stirring at room temperature for 1 hour. The solvent was evaporated under reduced pressure, and the residue was purified by flash column chromatography (chloroform/methanol=19/1, 4/1). The obtained solid was dissolved in chloroform, and the solution was added with diisopropyl ether and then stirred under ice-cooling for 1 hour. The obtained solid was collected by filtration, washed with hexane and then dried under reduced pressure to obtain 5-[4-(*tert*-butoxycarbonyl)piperazin-1-ylcarbonyl]-1-(tert-butoxy carbonyl)indole (1.63 g, yield 61%, 2 steps).
APCI-MS *m*/*z*: 430 [M+H]⁺; ¹H-NMR (DMSO-*d*₆)δ(ppm): 1.41 (s, 9H), 1.64 (s, 9H), 3.32-3.60 (m, 8H), 6.77 (d, *J* = 3.7 Hz, 1H), 7.37 (dd, *J* = 1.3, 8.4 Hz, 1H), 7.69 (d, *J* = 1.3 Hz, 1H), 7.75 (d, *J* = 3.7 Hz, 1H), 8.09 (d, *J* = 8.4 Hz, 1H).

### Step 3

In a similar manner to Step 2 of Reference Example 1-1, 5-[4-(*tert*-butoxycarbonyl)piperazin-1-ylcarbonyl]-1-(*tert*-butoxy carbonyl)indole (200 mg, 0.466 mmol) was dissolved in THF (2 mL), and the solution was treated with triisopropyl borate (0.161 mL, 0.698 mmol) and LDA-heptane/THF/ethylbenzene solution (2.0 mol/L, 0.58 mL, 1.2 mmol). Then, the reaction mixture was added with saturated aqueous ammonium chloride solution (2 mL) and 10% aqueous potassium hydrogensulfate solution (10 mL) to adjust the pH to 2, followed by stirring at room temperature for 20 minutes. The reaction mixture was extracted with ethyl acetate. The organic layer was washed with saturated brine and dried over anhydrous sodium sulfate. The solvent was evaporated under reduced pressure, and the residue was suspended in chloroform/diisopropyl ether/hexane (1/10/10), followed by stirring under ice-cooling for 30 minutes. The solid was collected by filtration, washed with diisopropyl ether and then dried under reduced pressure to obtain Compound BB (201 mg, yield 91%). ESI-MS *m*/*z*: 474 [M+H]⁺; ¹H-NMR (CDCl₃)δ(ppm): 1.47 (s, 9H), 1.57 (s, 9H), 3.30-3.85 (m, 8H), 6.78 (s, 2H), 7.40 (dd, *J* = 1.7, 8.6 Hz, 1H), 7.48 (s, 1H), 7.67 (d, *J* = 1.7 Hz, 1H), 8.04 (d, *J* = 8.6 Hz, 1H).

### Reference Example 1-3 (Compound BC)

### Step 1

5-Formylindole (500 mg, 3.44 mmol) was dissolved in acetonitrile (10 mL), and the solution was added with 1-(*tert*-butoxycarbonyl)piperazine (961 mg, 5.16 mmol), acetic acid (3.90 mL, 68.1 mmol) and sodium triacetoxyborohydride (3.65 g, 17.2 mmol) little by little, followed by stirring at room temperature for 2 hours. The reaction mixture was added with water and sodium carbonate to adjust the pH to 9, followed by extracting with ethyl acetate. The organic layer was washed with saturated brine and dried over anhydrous sodium sulfate. The solvent was evaporated under reduced pressure and the residue was purified by flash column chromatography (hexane/ethyl acetate = 19/1, 7/3, 1/1) to obtain 5-[4-(*tert*-butoxycarbonyl)piperazin-1-ylmethyl]indole (505 mg, yield 47%).
APCI-MS *m*/*z*: 316 [M+H]⁺; ¹H-NMR (CDCl₃)δ(ppm): 1.44 (s, 9H), 2.42-2.51 (m, 4H), 3.40-3.49 (m, 4H), 3.66 (s, 2H), 6.52 (m, 1H), 7.16 (dd, *J* = 1.6, 8.4 Hz, 1H), 7.21 (m, 1H), 7.35 (d, *J* = 8.4 Hz, 1H), 7.54 (br s, 1H), 8.25 (br s, 1H).

### Step 2

In a similar manner to Step 2 of Reference Example 1-2, 5-[4-(*tert*-butoxycarbonyl)piperazin-1-ylmethyl]indole (495 mg, 1.57 mmol) was dissolved in acetonitrile (5.0 mL), and the solution was treated with di-*tert*-butyldicarbonate (1.19 mL, 5.19 mmol) and DMAP (19.2 mg, 0.157 mmol). The reaction mixture was purified by flash column chromatography (hexane/ethyl acetate=19/1, 9/1, 17/3, 7/3, 1/1) to obtain. 5-[4-(*tert*-butoxycarbonyl)piperazin-1-ylmethyl]-1-(*tert*-butoxyca rbonyl)indole (346 mg, yield 53%).
ESI-MS *m*/*z*: 416 [M+H]⁺; ¹H-NMR (CDCl₃)δ(ppm): 1.45 (s, 9H), 1.67 (s, 9H), 2.35-2.46 (m, 4H), 3.37-3.48 (m, 4H), 4.11 (s, 2H), 6.53 (d, *J* = 3.7 Hz, 1H), 7.27 (dd, *J* = 1.8, 8.5 Hz, 1H), 7.48 (br s, 1H), 7.58 (d, *J* = 3.7 Hz, 1H), 8.07 (br d, *J* = 8.5 Hz, 1H).

### Step 3

In a similar manner to Step 2 of Reference Example 1-1, 5-[4-(*tert*-butoxycarbonyl)piperazin-1-ylmethyl]-1-(*tert*-butoxyca rbonyl) indole (336 mg, 0.809 mmol) was dissolved in THF (2 mL), and the solution was treated with triisopropyl borate (0.280 mL, 1.21 mmol) and LDA-heptane/THF/ethylbenzene solution (2.0 mol/L, 1.01 mL, 2.0 mmol). Then, the reaction mixture was added with saturated aqueous ammonium chloride solution (2 mL) and 10% aqueous potassium hydrogensulfate solution (10 mL) to adjust the pH to 2, followed by stirring at room temperature for 30 minutes. The reaction mixture was added with saturated aqueous sodium hydrogencarbonate solution to adjust the pH to 9, followed by extracting with ethyl acetate. After washing with saturated brine, the organic layer was dried over anhydrous sodium sulfate. The solvent was evaporated under reduced pressure and the residue was suspended in diisopropyl ether/hexane (2/5), followed by stirring at room temperature for 30 minutes. The solid was collected by filtration, washed with hexane and dried under reduced pressure to obtain Compound BC (209 mg, yield 56%). APCI-MS *m*/*z*: 460 [M+H]⁺; ¹H-NMR (CDCl₃)δ(ppm): 1.45 (s, 9H), 1. 74 (s, 9H), 2.37-2.44 (m, 4H), 3.40-3.48 (m, 4H), 3.59 (s, 2H), 6.80 (br s, 2H), 7.33 (dd, *J* = 1.6, 8.6 Hz, 1H), 7.45 (br s, 1H), 7.52 (d, *J* = 1.6 Hz, 1H), 7.96 (d, *J* = 8.6 Hz, 1H).

### Reference Example 1-4 (Compound BD)

### Step 1

5-Formylindole (5.50 g, 37.9 mmol) was dissolved in acetonitrile (100 mL), and the solution was added with di-*tert*-butyldicarbonate (9.60 mL, 41.8 mmol) andDMAP (46.0 mg, 0.377 mmol), followed by stirring at room temperature for 2 hours. The reaction mixture was added with piperidine (13.6 mL, 137 mmol) and acetic acid (39.4 mL, 688 mmol), and then sodium triacetoxyborohydride (36.5 g, 172 mmol) was added thereto little by little, followed by stirring at room temperature for 3 hours. The reaction mixture was added with water and sodium carbonate to adjust the pH to 9, followed by extracting with ethyl acetate. The organic layer was washed with saturated brine and dried over anhydrous sodium sulfate. The solvent was evaporated under reduced pressure and the residue was purified by flash column chromatography (hexane/ethyl acetate=19/1 to 4/1 to 7/3 to 3/2 to 1/1 to 2/3) to obtain 5-(piperidinomethyl)-1-(tert-butoxycarbonyl)indole (7.91 g, yield 66%).
ESI-MS *m*/*z*: 315 [M+H]⁺; ¹H-NMR (CDCl₃)δ(ppm): 1.37-1.48 (m, 2H), 1.54-1.62 (m, 4H), 1.66 (s, 9H), 2.33-2.45 (m, 4H), 3.56 (s, 2H), 6.53 (d, *J* = 3.7 Hz, 1H), 7.27 (dd, *J* = 1.5, 8.4 Hz, 1H), 7.49 (d, *J* = 1.5 Hz, 1H), 7.58 (d, *J* = 3.7 Hz, 1H), 8.05 (d, *J* = 8.4 Hz, 1H).

### Step 2

In a similar manner to Step 2 of Reference Example 1-1, 5-(piperidinomethyl)-1-(*tert*-butoxycarbonyl)indole (7.91 g, 25.2 mmol) was dissolved in THF (80 mL), and the solution was treated with triisopropyl borate (8.72 mL, 37.8 mmol) and LDA-heptane/THF/ethylbenzene solution (2.0 mol/L, 31.5 mL, 63 mmol). The reaction mixture was added with saturated aqueous ammonium chloride solution (80 mL) and 10% aqueous potassium hydrogensulfate solution (300 mL) to adjust the pH to 2, followed by stirring at room temperature for 2 hours. The reaction mixture was added with saturated aqueous sodium hydrogencarbonate solution to adjust the pH to 9, followed by extracting with ethyl acetate. The organic layer was washed with saturated brine and dried over anhydrous sodium sulfate. The solvent was evaporated under reduced pressure and the residue was suspended in hexane, followed by stirring at room temperature for 30 minutes. The solid was collected by filtration, washed with hexane and dried under reduced pressure to obtain Compound BD (6.59 g, yield 73%).
ESI-MS *m*/*z*: 359 [M+H]⁺; ¹H-NMR (CDCl₃)δ(ppm): 1.32-1.48 (m, 2H), 1.52-1.76 (m, 4H), 1.73 (s, 9H), 2.33-2.45 (m, 4H), 3.56 (s, 2H), 6.91 (br s, 2H), 7.34 (dd, *J* = 1.7, 8.6 Hz, 1H), 744 (s, 1H), 7.52 (d, *J* = 1.7 Hz, 1H), 7.94 (d, *J* = 8.6 Hz, 1H).

### Reference Example 1-5 (Compound BE)

### Step 1

Indole-5-carboxylic acid (4.09 g, 25.4 mmol) was dissolved in DMF (200 mL), and the solution was added with EDCI (9.74 g, 50.8 mmol) and HOBT monohydrate (1.95 g, 12.7 mmol) under ice-cooling, followed by stirring at the same temperature for 10 minutes. Then, the reaction mixture was added with N-methylpiperazine (8.45 mL, 76.2 mmol), followed by stirring at room temperature for 3.7 hours. The reaction mixture was added with water and extracted with ethyl acetate and chloroform. The organic layer was washed with saturated brine and was dried over anhydrous sodium sulfate. The solvent was evaporated under reduced pressure and the residue was purified by flash column chromatography (chloroform/methanol =100/0, 90/10) to obtain 5-(4-methylpiperazin-l-ylcarbonyl)indole (5.81 g, yield 94%). APCI-MS *m*/*z*: 244 [M+H]⁺; ¹H-NMR (CDCl₃)δ(ppm): 2.33 (s, 3H), 2.43 (m, 4H), 3.69 (m, 4H), 6.58 (m, 1H), 7.24-7.28 (m, 2H), 7.38 (d, *J* = 8.4 Hz, 1H), 7.73 (s, 1H), 8.48 (s, 1H).

### Step 2

5-(4-Methylpiperazin-1-ylcarbonyl)indol (5.80 g, 23.8 mmol) was dissolved in acetonitrile (170 mL), and the solution was added with di-*tert*-butyldicarbonate (15.6 g, 71.4 mmol) and DMAP (2.91 g, 23.8 mmol), followed by stirring at room temperature for 230 minutes. The reaction mixture was was added with water and extracted with ethyl acetate. The organic layer was washed with saturated brine and was dried over anhydrous sodium sulfate. The solvent was evaporated under reduced pressure and the residue was purified by silica gel column chromatography (chloroform/methanol =95/5, 90/10) to obtain 5-(4-methylpiperazin-1-ylcarbonyl)-1-(*tert*-butoxycarbonyl)indole (7.83 g, yield 96%).
APCI-MS *m*/*z*: 344 [M+H]⁺; ¹H-NMR (CDCl₃)δ(ppm): 1.68 (s, 9H), 2.32 (s, 3H), 2.42 (m, 4H), 3.68 (m, 4H), 6.59 (dd, *J* = 0.7, 3.7 Hz, 1H), 7.35 (dd, *J* = 1.7, 8.5 Hz, 1H), 7.64 (m, 2H), 8.16 (d, *J* = 8.6 Hz, 1H).

### Step 3

5-(4-Methylpiperazin-1-ylcarbonyl)-1-(*tert*-butoxycarbonyl)i ndole (545 mg, 1.59 mmol) was dissolved in THF (27 mL), and the solution was added with tert-butyllithium-pentane solution (1.44 mol/L, 2.43 mL, 3.50 mmol) by drops for 5 minutes at -78 °C under argon atmosphere, followed by stirring at the same temperature for 2 hours. Then, the solution was added with trimethyl borate (0.281 mL, 2.39 mmol), and the mixture was warmed from -78 °C to room temperature for 4.7 hours. The reaction mixture was added with 4 mol/L hydrochloric acid (3.98 mL), stirred for 10 minutes and then added with saturated aqueous sodium hydrogencarbonate solution, followed by extracting with ethyl acetate. The organic layer was washed with saturated brine and dried over anhydrous sodium sulfate. The solvent was evaporated under reduced pressure. The residue was purified by slurry using diisopropyl ether, then purified by preparative thin-layer chromatography (chloroform/methanol =12/5) to obtain Compound BE (107 mg, yield 17%). APCI-MS *m*/*z*: 388 [M+H]⁺; ¹H-NMR (CDCl₃)δ(ppm): 1.75 (s, 9H), 2.33 (s, 3H), 2.43 (m, 4H), 3.49-3.76 (m, 4H), 7.41 (dd, *J* = 1.7, 8.7 Hz, 1H), 7.48 (s, 1H), 7.67 (d, *J* = 1.1 Hz, 1H), 8.03 (d, *J* = 8.8 Hz, 1H).

### Reference Example 1-6 (Compound BF)

In a similar manner to Step 2 of Reference Example 1-1, 5-bromo-1-(*tert*-butoxycarbonyl)indole (1.00 g, 3.38 mmol) was dissolved in THF (5 mL), and the solution was treated with triisopropyl borate (1.17 mL, 5.07 mmol) and LDA-heptane/THF/ethylbenzene solution (2.0 mol/L, 2.54 mL, 5.1 mmol). The reaction mixture was added with 2 mol/L hydrochloric acid (10 mL), followed by stirring under ice-cooling for 2 hours. The reaction mixture was extracted with ethyl acetate. The organic layer was washed with saturated brine and dried over anhydrous sodium sulfate. The solvent was evaporated under reduced pressure. The residue was suspended in hexane/diisopropyl ether (5/1), then the solid was collected by filtration and washed with hexane, followed by drying under reduced pressure to obtain Compound BF (1.02 g, yield 89%). APCI-MS *m*/*z*: 340 [M+H]⁺; ¹H-NMR (CDCl₃)δ(ppm): 1.74 (s, 9H), 6.92 (s, 2H), 7.40 (s, 1H), 7.43(dd, *J* = 1.8, 9.2 Hz, 1H), 7.73 (d, *J* = 1.8 Hz, 1H), 7.87 (d, *J* = 9.2 Hz, 1H).

### Reference Example 1-7 (Compound BG)

5-Bromo-1-(*tert*-butoxycarbonyl) indole (10.0 g, 33.8 mmol) was dissolved in THF (100 mL), and the residue was added with n-butyllithium-hexane solution (2.71 mol/L, 13.7 mL, 37.1 mmol) by drops to the solution for 10 minutes at -78 °C under argon atmosphere, followed by stirring at the same temperature for 20 minutes. The mixture was added with triisopropyl borate (8.58 mL, 37.2 mmol) at -78 °C, followed by stirring at the same temperature for 45 minutes. The mixture was added with n-butyllithium-hexane solution (2.71 mol/L, 13.7 mL, 37.1 mmol) by drops for 10 minutes at -78 °C, followed by stirring at the same temperature for 10 minutes. The atmosphere in the reaction vessel was substituted with carbon dioxide, followed by stirring at 78 °C for 1 hour, then the mixture was warmed to room temperature, followed by stirring at the same temperature for 1 hour. The reaction mixture was added with saturated aqueous ammonium chloride solution (20 mL) and 10% aqueous potassium hydrogensulfate solution (120 mL) to adjust the pH to 2, followed by stirring at room temperature for 1 hour. The mixture was extracted with ethyl acetate. The organic layer was washed with saturated brine and dried over anhydrous sodium sulfate. The solvent was evaporated under reduced pressure. The residue was suspended in hexane/chloroform (2/1), and the suspenton was stirred under ice-cooling for 1 hour. The solid was collected by filtration and washed with hexane, followed by drying under reduced pressure to obtain Compound BG (4.45 g, yield 43%). ESI-MS *m*/*z*: 304 [M-H]⁻; ¹H-NMR (DMSO-*d*₆)δ(ppm) : 1.60 (s, 9H), 6.74 (s, 1H), 7.87 (dd, *J* = 1.8, 8.8 Hz, 1H), 8.13 (d, *J* = 8.8 Hz, 1H), 8.18 (d, *J* = 1.8 Hz, 1H), 8.27 (s, 2H).

### Reference Example 1-8 (Compound BH)

### Step 1

In a similar manner to Step 1 of Reference Example 1-2, indole-6-carboxylic acid (200 mg, 1.24 mmol) was dissolved in DMF (2 mL), and the solution was treated with EDCI (475 mg, 2.48 mmol), HOBT monohydrate (168 mg, 1.24 mmol) and 1-(*tert*-butoxycarbonyl)piperazine (461 mg, 2.48 mmol) to obtain 6-[4-(*tert*-butoxycarbonyl)piperazin-1-ylcarbonyl]indole (529 mg).

### Step 2

In a similar manner to Step 2 of Reference Example 1-2, 6-[4-(*tert*-butoxycarbonyl)piperazin-1-ylcarbonyl]indole (529 mg) was dissolved in acetonitrile (5 mL), and the solution was treated with di-*tert*-butyldicarbonate (0.740 mL, 3.22 mmol) and DMAP (20 mg, 0. 16 mmol), followed by purification by flash column chromatography (hexane/ethyl acetate=19/1, 9/1, 4/1) to obtain 6-[4-(*tert-*butoxycarbonyl)piperazin-1-ylcarbonyl]-1-(*tert*-butoxy carbonyl)indole (518 mg, yield 97%, 2 Steps).
APCI-MS *m*/*z*: 430 [M+H]⁺; ¹H-NMR (CDCl₃)δ(ppm): 1.47 (s, 9H), 1.67 (s, 9H), 3.24-3.93 (m, 8H), 6.59 (d, *J* = 3.5 Hz, 1H), 7.29 (dd, *J* = 1.5, 8.1 Hz, 1H), 7.59 (d, *J* = 8.1 Hz, 1H), 7.66 (d, *J* = 3.5 Hz, 1H), 8.26 (br s, 1H).

### Step 3

In a similar manner to Step 2 of Reference Example 1-1, 6-[4-(*tert*-butoxycarbonyl)piperazin-1-ylcarbonyl]-1-(*tert*-butoxy carbonyl)indole (224 mg, 0.522 mmol) was dissolved in THF (2 mL), and the solution was treated with triisopropyl borate (0.181 mL, 0.784 mmol) and LDA-heptane/THF/ethylbenzene solution (2.0 mol/L, 0.652 mL, 1.3 mmol). Then, the reaction mixture was added with saturated aqueous ammonium chloride solution (2 mL) and 10% aqueous potassium hydrogensulfate solution (5 mL) to adjust the pH to 2, followed by stirring at room temperature for 30 minutes. The reaction mixture was extracted with ethyl acetate. The organic layer was washed with saturated brine and dried over anhydrous sodium sulfate. The solvent was evaporated under reduced pressure. The residue was dissolved in chloroform, and the solution was added with diisopropyl ether and stirred under ice-cooling for 30 minutes. The solid was collected by filtration, washed with diisopropyl ether and then dried under reduced pressure to obtain Compound BH (95.7 mg, yield 39%). ESI-MS *m*/*z*: 474 [M+H]⁺; ¹H-NMR (DMSO-*d*₆)δ(ppm): 1.40 (s, 9H), 1.59 (s, 9H), 3.33-3.57 (m, 8H), 6.67 (s, 1H), 7.24 (dd, *J* = 1.1, 7.9 Hz, 1H), 7.61 (d, *J* = 7.9 Hz, 1H), 8.12 (s, 1H), 8.26 (s, 2H).

### Reference Example 1-9 (Compound BI)

### Step 1

5-Hydroxyindole (200 mg, 1.50 mmol) was dissolved in DMF (2.0 mL), and the solution was added with potassium carbonate (622 mg, 4.50 mmol) and 4-(2-chloroethyl)morpholine hydrochloride (335 mg, 1.80 mmol), followed by stirring at 60 °C for 3 hours. Then, the mixture was added with potassium carbonate (622 mg, 4.50 mmol) and 4-(2-chloroethyl)morpholine hydrochloride (223 mg, 1.20 mmol), followed by stirring at 60 °C for 5 hours. The reaction mixture was added with water and extracted with ethyl acetate. The organic layer was washed with saturated brine and dried over anhydrous sodium sulfate. The solvent was evaporated under reduced pressure. The residue was dissolved in acetonitrile (2.0 mL), and the solution was added with di-*tert*-butyldicarbonate (0.494 mL, 2.15 mmol) and DMAP (1.8 mg, 0.015 mmol), followed by stirring at room temperature for 20 hours. The solvent was evaporated under reduced pressure and the residue was purified by flash column chromatography (hexane/ethyl acetate=19/1, 4/1, 7/3, 3/2) to obtain 5-[2-(morpholin-4-yl)ethoxy]-1-(*tert*-butoxycarbonyl)indole (369 mg, yield 71%).
ESI-MS *m*/*z*: 347 [M+H]⁺; ¹H-NMR (CDCl₃)δ(ppm): 1.66 (s, 9H), 2.47-2.55 (m, 4H), 2.83 (t, *J* = 5.5 Hz, 2H), 3.79-3.80 (m, 4H), 4.16 (t, *J* = 5.5 Hz, 2H), 6.48 (d, *J* = 3.7 Hz, 1H), 6.83 (dd, *J* = 2.6, 8.8 Hz, 1H), 7.03 (d, *J* = 2.6 Hz, 1H), 7.56 (d, *J* = 3.7 Hz, 1H), 8.01 (d, *J* = 8.8 Hz, 1H).

### Step 2

In a similar manner to Step 2 of Reference Example 1-1, 5-[2-(morpholin-4-yl)ethoxy]-1-(*tert*-butoxycarbonyl)indole (359mg, 1.04 mmol) was dissolved in THF (10 mL), and the solution was treated with triisopropyl borate (0.360 mL, 1.56 mmol) and LDA-heptane/THF/ethylbenzene solution (2.0 mol/L, 1.30 mL, 2.6 mmol). The reaction mixture was added with saturated aqueous ammonium chloride solution (3 mL) and 10% aqueous potassium hydrogensulfate solution (15mL) to adjust the pH to 2, followed by stirring at room temperature for 30 minutes. The reaction mixture was added with saturated aqueous sodium hydrogencarbonate solution to adjust the pH to 9, followed by extracting with ethyl acetate. The organic layer was washed with saturated brine and dried over anhydrous sodium sulfate. The solvent was evaporated under reduced pressure. The residue was dissolved in chloroform, and the solution was added with diisopropyl ether under ice-cooling and stirred for 30 minutes. The solid was collected by filtration, washed with diisopropyl ether and then dried under reduced pressure to obtain Compound BI (347 mg, yield 86%) .
APCI-MS *m*/*z*: 391 [M+H]⁺; ¹H-NMR (CDCl₃)δ(ppm): 1.73 (s, 9H), 2.56-2.61 (m, 4H), 2.84 (t, *J* = 5.8 Hz, 2H), 3.70-3.80 (m, 4H), 4.17 (t, *J* = 5.8 Hz, 2H), 6.84 (br s, 2H), 6.97 (dd, *J* = 2.6, 9.1 Hz, 1H), 7.04 (d, *J* = 2.6 Hz, 1H), 7.39 (s, 1H), 7.88 (d, *J* = 9.1 Hz, 1H).

### Reference Example 1-10 (Compound BJ)

### Step 1

5-Formylindole (2.00 g, 13.8 mmol) was dissolved in acetonitrile (40 mL), and the solution was added with di-*tert*-butyldicarbonate (3.31 g, 15.2 mmol) and DMAP (16.8 mg, 0.138 mmol), followed by stirring at room temperature for 2.7 hours. The solution was added with a solution of N-(2-hydroxyethyl)piperazine (6.46 g, 49.6 mmol) in acetonitrile (15 mL), and acetic acid (14.2 mL, 248 mmol), then sodium triacetoxyborohydride (13.1 g, 62.0 mmol) was added thereto little by little, followed by stirring at room temperature for 2.5 hours. The reaction mixture was added with water and sodium carbonate to adjust the pH to 9, and extracted with ethyl acetate. The organic layer was washed with saturated brine and dried over anhydrous sodium sulfate. The solvent was evaporated under reduced pressure. The residue was purified by flash column chromatography (chloroform/methanol =9/1, 4/1) to obtain 5-[4-(2-hydroxyethyl)piperazin-1-ylmethyl]indole (4.73 g, yield 95%).
APCI-MS *m*/*z*: 360 [M+H]⁺; ¹H-NMR (CDCl₃)δ(ppm): 1.67 (s, 9H), 2.06-2.63 (m, 10H), 3.63 (m, 4H), 6.54 (d, *J* = 3.8 Hz, 1H), 7.28 (m, 1H), 7.50 (s, 1H), 7.58 (d, *J* = 3.6 Hz, 1H), 8.08 (d, *J* = 8.6 Hz, 1H).

### Step 2

In a similar manner to Step 2 of Reference Example 1-1, 5-[4-(2-hydroxyethyl)piperazin-1-ylmethyl]indole (956 mg, 2.66 mmol) was dissolved in THF (29 mL), and the solution was treated with triisopropyl borate (1.23 mL, 5.32 mmol) and LDA (2 mol/L, 6.65 mL, 13.3 mmol), followed by purification by slurry using hexane to obtain Compound BJ (663 mg, yield 62%).
ESI-MS *m*/*z*: 404 [M+H]⁺; ¹H-NMR (CDCl₃)δ(ppm): 1. 72 (s, 9H), 2.54 (m, 10H), 3.60 (m, 4H), 7.15-7.32 (m, 2H), 7.51 (m, 1H), 7.95 (d, *J* = 8.6 Hz, 1H).

### Reference Example 1-11 (Compound BK)

1-Methylindole (200 mg, 1.52 mmol) was dissolved in THF (5 mL), and the solution was added with tert-butyllithium-pentane solution (1.48 mol/L, 1.23 mL, 1.82 mmol) by drops at -78 °C for 5 minutes under argon atmosphere. The mixture was warmed to room temperature, followed by stirring for 30 minutes. The mixture was cooled to -78 °C again, added with triisopropyl borate (0. 526 mL, 2.28 mmol) and warmed from -78 °C to room temperature for 1 hour. The reaction mixture was added with saturated aqueous ammonium chloride solution (5 mL) and 10% aqueous potassium hydrogensulfate solution (5 mL) to adjust the pH to 2, followed by stirring at room temperature for 30 minutes. The mixture was extracted with ethyl acetate. The organic layer was washed with saturated brine and dried over anhydrous sodium sulfate. The solvent was evaporated under reduced pressure. The residue was suspended in diisopropyl ether, then the solid was collected by filtration, washed with diisopropyl ether and then dried under reduced pressure to obtain Compound BK (144 mg, yield 54%). ESI-MS *m*/*z*: 174 [M-H]⁻; ¹H-NMR (CDCl₃)δ(ppm): 4.01 (s, 3H), 4.73 (br s, 2H), 6.97 (d, *J* = 0.7 Hz, 1H), 7.17 (m, 1H), 7.27-7.42 (m, 3H).

### Reference Example 1-12 (Compound BL)

### Step 1

Indole-5-carboxylic acid (200 mg, 1.24 mmol) was dissolved in DMF (2 mL), and the solution was added with 60% sodium hydride-mineral oil dispersant (164 mg, 4.1 mmol), followed by stirring at room temperature for 10 minutes. The mixture was added with methyl iodide (0.258 mL, 0.410 mmol), stirred at room temperature for 3 hours and then added with 4 mol/L aqueous potassium hydroxide solution (2 mL), followed by stirring at 80 °C for 5 hours. The reaction mixture was added with water, and the organic layer was separated from aqueous layer. The aqueous layer was added with 1 mol/L hydrochloric acid to adjust the pH to 1, followed by stirring at room temperature for 1 hour. The obtained solid was collected by filtration and dried under reduced pressure to obtain 1-methylindole-5-carboxylic acid (142 mg, yield 65%).
APCI-MS *m*/*z*: 174 [M-H]⁻; ¹H-NMR (DMSO-*d*₆)δ(ppm): 3.83 (s, 3H), 6.57 (d, *J* = 3.0 Hz, 1H), 7.43 (d, *J* = 3.0 Hz, 1H), 7.50 (d, *J* = 8.6 Hz, 1H), 7.76 (dd, *J* = 1.6, 8.6 Hz, 1H), 8.22 (d, *J* = 1.6 Hz, 1H), 12.49 (br s, 1H).

### Step 2

In a similar manner to Reference Example 1-11, 1-methylindole-5-carboxylic acid (100 mg, 0.571 mmol) was dissolved in THF (5 mL), and the solution was treated with tert-butyllithium-pentane solution (1.48 mol/L, 0.970 mL, 1.44 mmol) and triisopropyl borate (0.198 mL, 0.858 mmol). The reaction mixture was added with saturated aqueous ammonium chloride solution (5 mL) and 10% aqueous potassium hydrogensulfate solution (5mL) to adjust the pH to 2, and the mixture was stirred at room temperature for 30 minutes, followed by extracting with ethyl acetate. The organic layer was washed with saturated brine and dried over anhydrous sodium sulfate. The solvent was evaporated under reduced pressure. The residue was suspended in diisopropyl ether, and the solid was collected by filtration, washed with diisopropyl ether and then dried under reduced pressure to obtain Compound BL (75.0 mg, yield 60%). APCI-MS *m*/*z*: 220 [M+H]⁺; ¹H-NMR (DMSO-*d*₆)δ(ppm): 3.96 (s, 3H), 7.19 (s, 1H), 7.49 (d, *J* = 8.9 Hz, 1H), 7.77 (dd, *J* = 1.7, 8.9 Hz, 1H), 8.23 (d, *J* = 1.7 Hz, 1H), 8.40 (br s, 2H), 12.42 (br s, 1H).

### Reference Example 1-13 (Compound BM)

### Step 1

In a similar manner to Step 1 of Reference Example 1-12, indole-6-carboxylic acid (200 mg, 1.24 mmol) was dissolved in DMF (2 mL), and the solution was treated with 60% sodium hydride-mineral oil dispersant (109 mg, 2.71 mmol), methyl iodide (0.258 mL, 0.410 mmol) and 4 mol/L aqueous potassium hydroxide solution (2 mL). The reaction mixture was added with water and separated into aqueous layer and organic layer, and the organic layer was removed. The aqueous layer was added with 1 mol/L hydrochloric acid to adjust the pH to 1, followed by stirring at room temperature for 1 hour. The obtained solid was collected by filtration and dried under reduced pressure to obtain 1-methylindole-6-carboxylic acid (154 mg, yield 71%).
APCI-MS *m*/*z*: 174 [M-H]⁻; ¹H-NMR (DMSO-*d*₆)δ(ppm): 3.86 (s, 3H), 6.50 (dd, *J* = 0.7, 3.1 Hz, 1H), 7.54 (d, *J* = 3.1 Hz, 1H), 7.60 (dd, *J* = 0.7, 8.4 Hz, 1H), 7.64 (dd, *J* = 1.3, 8.4 Hz, 1H), 8.07 (d, *J* = 1.3 Hz, 1H), 12.59 (br s, 1H).

### Step 2

In a similar manner to Reference Example 1-11, 1-methylindole-6-carboxylic acid (100 mg, 0.571 mmol) was dissolved in THF (5 mL), and the solution was treated with tert-butyllithium-pentane solution (1.48 mol/L, 0.970 mL, 1.44 mmol) and triisopropyl borate (0.198 mL, 0. 858 mmol). The reaction mixture was added with saturated aqueous ammonium chloride solution (5 mL) and 10% aqueous potassium hydrogensulfate solution (5 mL) to adjust the pH to 2, followed by stirring at room temperature for 30 minutes. The reaction mixture was extracted with ethyl acetate. The organic layer was washed with saturated brine and dried over anhydrous sodium sulfate. The solvent was evaporated under reduced pressure. The residue was suspended in diisopropyl ether, and the solid was collected by filtration, washed with diisopropyl ether and then dried under reduced pressure to obtain Compound BM (23.4 mg, yield 19%).
APCI-MS *m*/*z:* 220 [M+H]⁺; ¹H-NMR (DMSO-*d*₆)δ(ppm) : 3.99 (s, 3H), 7.10 (s, 1H), 7.61 (s, 2H), 8.06 (s, 1H), 8.46 (s, 2H), 12.57 (br s, 1H).

### Reference Example 1-14 (Compound BN)

In a similar manner to Reference Example 1-11, benzothiophene-5-carboxylic acid (200 mg, 1.12 mmol) was dissolved in THF (5 mL), and the solution was treated with tert-butyllithium-pentane solution (1.48 mol/L, 1.89 mL, 2.80 mmol) and triisopropyl borate (0.388 mL, 1.68 mmol). The reaction mixture was added with saturated aqueous ammonium chloride solution (5 mL) and 10% aqueous potassium hydrogensulfate solution (5mL) to adjust the pH to 2, followed by stirring at room temperature for 30 minutes. The mixture was extracted with ethyl acetate. The organic layer was washed with saturated brine and dried over anhydrous sodium sulfate. The solvent was evaporated under reduced pressure. The residue was suspended in hexane/chloroform/methanol (40/4/1), and the solid was collected by filtration, washed with hexane and dried under reduced pressure to obtain Compound BN (179 mg, yield 72%).
ESI-MS *m*/*z*: 221 [M-H]⁻; ¹H-NMR (DMSO-*d*₆)δ(ppm): 7.90 (dd, *J* = 1.5, 8.8 Hz, 1H), 8.06 (s, 1H), 8.08 (d, *J* = 8.8 Hz, 1H), 8.49 (d, *J* = 1.5 Hz, 1H), 8.59 (s, 2H), 12.93 (s, 1H).

### Reference Example 1-15 (Compound BO)

### Step 1

5-Aminoindole (100 mg, 0.757 mmol) was dissolved in DMF (2 mL), and the solution was added with EDCI (290 mg, 1.51 mmol), HOBT monohydrate (102 mg, 0.755 mmol) and 1-(tert-butoxycarbonyl)piperidine-4-carboxylic acid (208 mg, 0.907 mmol), followed by stirring at room temperature for 2 hours. The reaction mixture was added with water and extracted with ethyl acetate. The organic layer was washed with 1 mol/L hydrochloric acid, saturated aqueous sodium hydrogencarbonate solution and saturated brine, and dried over anhydrous sodium sulfate. The solvent was evaporated under reduced pressure and the residue was purified by slurry using acetonitrile to obtain 5-[1-(*tert*-butoxycarbonyl)piperidine-4-carbonylamino]indole (142 mg, yield 55%).
ESI-MS *m*/*z*: 344 [M+H]⁺.

### Step 2

In a similar manner to Step 2 of Reference Example 1-2, 5-[1-(*tert*-butoxycarbonyl)piperidine-4-carbonylamino]indole (140 mg, 0.408 mmol) was dissolved in acetonitrile (6 mL), and the solution was treated with di-*tert*-butyldicarbonate (0.103 mL, 0.448 mmol) and DMAP (1.0 mg, 0.0082 mmol), followed by purification by flash column chromatography (hexane/ethyl acetate=95/5 to 90/10 to 80/20 to 70/30 to 60/40 to 50/50) to obtain 5-[1-(*tert*-butoxycarbonyl)piperidine-4-carbonylamino]-1-(*tert*-bu toxycarbonyl)indole (117 mg, yield 65%).
ESI-MS *m*/*z*: 444 [M+H]⁺; ¹H-NMR (CDCl₃)δ(ppm): 1.46 (s, 9H), 1.65 (s, 9H), 1.68-1.83 (m, 2H), 1.84-1.96 (m, 2H), 2.39 (m, 1H), 2.70-2.87 (m, 2H), 4.10-4.26 (m, 2H), 6.50 (s, 1H), 7.22 (d, *J* = 8.7 Hz, 1H), 7.45-7.60 (m, 2H), 7.92 (s, 1H), 8.04 (d, *J* = 8.7 Hz, 1H).

### Step 3

In a similar manner to Step 2 of Reference Example 1-1, 5-[1-(*tert*-butoxycarbonyl)piperidine-4-carbonylamino]-1-(*tert*-bu toxycarbonyl) indole (115 mg, 0.259 mmol) was dissolved in THF (3 mL), and the solution was treated with triisopropyl borate (0.090 mL, 0.39 mmol) and LDA-heptane/THF/ethylbenzene solution (2.0 mol/L, 0.45 mL, 0.90 mmol), followed by purification by slurry using diisopropyl ether and hexane to obtain Compound BO (116 mg, yield 92%). ESI-MS *m*/*z*: 488 [M+H]⁺; ¹H-NMR (CDCl₃)δ(ppm): 1.47 (s, 9H), 1.71-1.85 (m, 2H), 1.73 (s, 9H), 1.88-1.99 (m, 2H), 2.27 (m, 1H), 2.74-2.88 (m, 2H), 4.10-4.30 (m, 2H), 6.83 (s, 2H), 7.40 (dd, *J* = 2.2, 9.0 Hz, 1H), 7.41 (s, 1H), 7.83 (d, J = 2.2 Hz, 1H), 7.94 (d, *J* = 9.0 Hz, 1H).

### Reference Example 1-16 (Compound BP)

### Step 1

5-Aminoindole (100 mg, 0.757 mmol) was dissolved in acetonitrile (2 mL), and the solution was added with di-*tert*-butyldicarbonate (0.350 mL, 1.52 mmol), followed by stirring at room temperature for 1 hour. The reaction mixture was added with DMAP (1.0 mg, 0.0082 mmol), stirred at room temperature for 20 hours and then, the solvent was evaporated under reduced pressure. The residue was purified by flash column chromatography (hexane/ethyl acetate=95/5 to 90/10 to 80/20 to 70/30) to obtain 5-(*tert*-butoxycarbonylamino)-1-(*tert*-butoxycarbonyl)indole (236 mg, yield 94%).
ESI-MS *m*/*z:* 333 [M+H]⁺; ¹H-NMR (CDCl₃)δ(ppm): 1.53 (s, 9H), 1. 66 (s, 9H), 6.49 (s, 1H), 6.50 (s, 1H), 7. 12 (dd, *J* = 2.9, 8.8 Hz, 1H), 7.55 (d, *J* = 2.9 Hz, 1H), 7.73(s, 1H), 8.02 (d, *J* = 8.8 Hz, 1H).

### Step 2

In a similar manner to Step 2 of Reference Example 1-1, 5-(*tert*-butoxycarbonylamino)-1-(*tert*-butoxycarbonyl)indole (230 mg, 0.692 mmol) was dissolved in THF (5 mL), and the solution was treated with triisopropyl borate (0.240 mL, 1.04 mmol) and LDA-heptane/THF/ethylbenzene solution (2.0 mol/L, 1.21mL, 2.42 mmol), followed by purification by slurry using hexane to obtain Compound BP (210 mg, yield 81%).
ESI-MS *m*/*z*: 377 [M+H]⁺; ¹H-NMR (CDCl₃)δ(ppm): 1.54 (s, 9H), 1.72 (s, 9H), 6.55 (br s, 1H), 7.10 (s, 2H), 7.26 (dd, *J* = 2.1, 8.9 Hz, 1H), 7.40 (s, 1H), 7.68 (d, *J* = 2.1 Hz, 1H), 7.90 (d, *J* = 8.9 Hz, 1H).

### Reference Example 1-17 (Compound BQ)

### Step 1

5-Aminoindole (100 mg, 0.757 mmol) was dissolved in acetonitrile (2 mL), and the solution was added with 1-(*tert*-butoxycarbonyl)-4-piperidinone (181 mg, 0.908 mmol), acetic acid (0.870 mL, 15.2 mmol) and sodium triacetoxyborohydride (160 mg, 0.755 mmol) little by little, followed by stirring at room temperature for 1 hour. The reaction mixture was added with water and sodium carbonate to adjust the pH to 9. The mixture was extracted with ethyl acetate. The organic layer was washed with saturated brine and dried over anhydrous sodium sulfate. The solvent was evaporated to obtain 5-[1-(*tert*-butoxycarbonyl)-4-piperidylamino]indole (300 mg). 5-[1-(*tert*-butoxycarbonyl)-4-piperidylamino]indole
APCI-MS *m*/*z*: 316 [M+H]⁺; ¹H-NMR (CDCl₃)δ(ppm): 1.21-1.42 (m, 2H), 1.47 (s, 9H), 2.03-2.14 (m, 2H), 2.86-2.98 (m, 2H), 3.43 (m, 1H), 3.97-4.13 (m, 2H), 6.39 (m, 1H), 6.62 (dd, *J* = 2.2, 8.6 Hz, 1H), 6.88 (d, *J* = 2.2 Hz, 1H), 7.13 (dd, *J* = 2.7, 2.8 Hz, 1H), 7.21 (d, *J* = 8.6 Hz, 1H), 7.96 (br s, 1H).

### Step 2

In a similar manner to Step 2 of Reference Example 1-2, 5-[1-(*tert*-butoxycarbonyl)-4-piperidylamino]indole (300 mg) was dissolved in acetonitrile (6 mL), and the solution was treated with di-*tert*-butyldicarbonate (0.348 mL, 1.51 mmol), triethylamine (0.106 mL, 0.760 mmol) and DMAP (3.0 mg, 0.025 mmol), followed by purification by flash column chromatography (hexane/ethyl acetate=95/5 to 90/10 to 80/20 to 70/30) to obtain 5-[1-(*tert*-butoxycarbonyl)-4-piperidyl-N-(*tert*-butoxycarbonyl)am ino]-1-(*tert*-butoxycarbonyl)indole (326 mg, yield 84%, 2 steps).
ESI-MS m/z: 516 [M+H]⁺; ¹H-NMR (CDCl₃)δ(ppm): 1.30-1.40 (m, 2H), 1.37 (s, 18H), 1.68 (s, 9H), 1.87-1.98 (m, 2H), 2.70-2.84 (m, 2H), 4.00-4.22 (m, 2H), 4.39 (m, 1H), 6.56 (d, *J* = 3.7 Hz, 1H), 7.05 (dd, *J* = 1.9,8.7 Hz, 1H), 7.31 (d, *J* = 1.9 Hz, 1H), 7.65 (d, *J* = 3.7 Hz, 1H), 8.14 (d, *J* = 8.7 Hz, 1H).

### Step 3

In a similar manner to Step 2 of Reference Example 1-1, 5-[1-(*tert*-butoxycarbonyl)-4-piperidyl-N-(*tert*-butoxycarbonyl)am ino]-1-(*tert*-butoxycarbonyl)indole (320 mg, 0.621 mmol) was dissolved in THF (5 mL), and the solution was treated with triisopropyl borate (0.215 mL, 0.932 mmol) and LDA-heptane/THF/ethylbenzene solution (2.0 mol/L, 0.776 mL, 1.55 mmol), followed by purification by slurry using hexane to obtain Compound BQ (177 mg, yield 62%). ESI-MS *m*/*z*: 460 [M+H]⁺; ¹H-NMR (CDCl₃)δ(ppm): 1.20-1.42 (m, 2H), 1.47 (s, 9H), 1.72 (s, 9H), 2.02-2.13 (m, 2H), 2.86-3.00 (m, 2H), 3.46 (m, 1H), 4.00-4.17 (m, 2H), 6.68 (dd, *J* = 2.3, 8.9 Hz, 1H), 6.76 (d, *J* = 2.3 Hz, 1H), 6.87 (br s, 2H), 7.33 (s, 1H), 7.79 (d, *J* = 8.9 Hz, 1H).

### Reference Example 1-18 (Compound BR)

### Step 1

5-Hydroxyindole (209 mg, 1.57 mmol) was dissolved in DMF (6 mL), and the solution was added with imidazole (132 mg, 1.93 mmol) and *tert*-butyldimethylsilyl chloride (333 mg, 2.21 mmol) at 0°C, followed by stirring at room temperature for 3.5 hours. The reaction mixture was added with saturated aqueous sodium hydrogencarbonate solution at 0°C and extracted with ethyl acetate. The organic layer was washed with saturated aqueous sodium chloride solution and dried over anhydrous sodium sulfate. The solvent was evaporated under reduces pressure to obtain 5-(*tert*-butyldimethylsilyloxy)indole (517 mg).

### Step 2

In a similar manner to Step 2 of Reference Example 1-2, 5-(*tert*-butyldimethylsilyloxy)indole (510 mg) was dissolved in acetonitrile (2 mL), and the solution was treated with di-*tert*-butyldicarbonate (0.510 mL, 2.22 mmol) and DMAP (10.1 mg, 0. 0827 mmol), followed by purification by flash column chromatography (hexane/ethyl acetate=100/0 to 95/5) to obtain 5-(*tert*-butyldimethylsilyloxy)-1-(*tert*-butoxycarbonyl)indole (549 mg).

### Step 3

In a similar manner to Step 2 of Reference Example 1-1, 5-(*tert*-butyldimethylsilyloxy)-1-(*tert*-butoxycarbonyl)indole (549 mg) was dissolved in THF (10 mL), and the solution was treated with triisopropyl borate (0.547 mL, 2.37 mmol) and
LDA-heptane/THF/ethylbenzene solution (2.0 mol/L, 2.00 mL, 4.00 mmol), followed by purification by slurry using hexane to obtain Compound BR (431 mg, yield 70%, 3 steps).
ESI-MS *m*/*z*: 392 [M+H]⁺; ¹H-NMR (CDCl₃)δ(ppm): 0.20 (s, 6H), 1.00 (s, 9H), 1.72 (s, 9H), 6.88 (dd, *J* = 2.6, 9.0 Hz, 1H), 6.97 (br s, 2H), 7.00 (d, *J* = 2.6 Hz, 1H), 7.37 (s, 1H), 7.84 (d, *J* = 9.0 Hz, 1H).

### Reference Example 1-19 (Compound BS)

### Step 1

In a similar manner to Step 1 of Reference Example 1-9, 5-hydroxyindole (200 mg, 1.50 mmol) was dissolved in DMF (2 mL), and the solution was treated with 1-(2-chloroethyl)piperidine (553 mg, 3.00 mmol) and potassium carbonate (1.25 g, 9.00 mmol). The reaction mixture was added with water and extracted with ethyl acetate. The organic layer was washed with saturated brine and dried over anhydrous sodium sulfate. The solvent was evaporated under reduced pressure. The residue was dissolved in acetonitrile (2.0 mL), and the solution was treated with di-*tert*-butyldicarbonate (0.483 mL, 2.10 mmol) and DMAP(1.8 mg, 0.015 mmol), followed by purification by flash column chromatography (hexane/ethyl acetate=7/3 to 1/1) to obtain 5-[2-(1-piperidino)ethoxy]-1-(*tert*-butoxycarbonyl)indole (348 mg, yield 67%).
ESI-MS *m*/*z:* 345 [M+H]⁺; ¹H-NMR (CDCl₃)δ(ppm): 1.50-1.62 (m, 2H), 1.62-1.73 (m, 4H), 1.66 (s, 9H), 2.45-2.60 (m, 4H), 2.79 (t, *J* = 5.1 Hz, 2H), 4.15 (t, *J* = 6.1 Hz, 2H), 6.48 (d, *J* = 3.6 Hz, 1H), 6.93 (dd, *J* = 2.5, 9.0 Hz, 1H), 7.03 (d, *J* = 2.5 Hz, 1H), 7.55 (d, *J* = 3.6 Hz, 1H), 7.99 (d, *J* = 9.0 Hz, 1H).

### Step 2

In a similar manner to Step 2 of Reference Example 1-1, 5-[2-(1-piperidino)ethoxy]-1-(*tert*-butoxycarbonyl)indole (346 mg, 1. 00 mmol) was dissolved in THF (1.7 mL), and the solution was treated with triisopropyl borate (0.346 mL, 1.50 mmol) and LDA-heptane/THF/ethylbenzene solution (2.0 mol/L, 1.25 mL, 2.50 mmol), followed by purification by slurry using diisopropyl ether and hexane to obtain Compound BS (200 mg, yield 52%). ESI-MS *m*/*z*: 389 [M+H]⁺; ¹H-NMR (CDCl₃)δ(ppm): 1.43-1.50 (m, 2H), 1.51-1.72 (m, 4H), 1.73 (s, 9H), 2.45-2.64 (m, 4H), 2.81 (t, *J* = 6.0 Hz, 2H), 4.16 (t, *J* = 6.0 Hz, 2H), 6.97 (dd, *J* = 2.5, 9.2 Hz, 1H), 7.04 (d, *J* = 2.5 Hz, 1H), 7.39 (s, 1H), 7.88 (d, *J* = 9.2 Hz, 1H).

### Reference Example 1-20 (Compound BT)

### Step 1

1-(*tert*-Butoxycarbonyl)-4-piperidinone (400 mg, 2.01 mmol) was dissolved in methanol (4 mL), and the solution was added with 10% hydrogen chloride-methanol solution (4 mL), followed by stirring at 50°C for 2 hours. The reaction mixture was added with diisopropyl ether and the precipitated solid was collected by filtration, washed with diisopropyl ether and then dried under reduced pressure to obtain 4,4-dimethoxypiperidine hydrochloride (238 mg, yield 65%).
ESI-MS *m*/*z:* 182 [M+H]⁺; ¹H-NMR (DMSO-*d*₆)δ(ppm): 1.83-1.90 (m, 4H), 2.96-3.05 (m, 4H), 3.11 (s, 6H), 8.71 (brs, 2H).

### Step 2

In a similar manner to Step 1 of Reference Example 1-4, 5-formylindole (1.20 g, 8.27 mmol) was dissolved in acetonitrile (24 mL), and the solution was treated with di- tert-butyldicarbonate (2.09 mL, 9.10 mmol) and DMAP (10.1 mg, 0.0827 mmol), and then the mixture was treated with 4,4-dimethoxypiperidine hydrochloride (3.00 g, 16.5 mmol), triethylamine (2.53 mL, 18.2 mmol), acetic acid (4.73 mL, 82.6 mmol) and sodium triacetoxyborohydride (5.25 g, 24.8 mmol), followed by purification by flash column chromatography (hexane/ethyl acetate=95/5 to 90/10 to 85/15) to obtain 5-(4,4-dimethoxypiperidinomethyl)-1-(*tert*-butoxycarbonyl)indole (1.20 g, yield 39%).
APCI-MS *m*/*z:* 375 [M+H]⁺; ¹H-NMR (CDCl₃)δ(ppm): 1.67 (s, 9H), 1.74-1.82 (m, 4H), 2.41-2.49 (m, 4H), 3.18 (s, 6H), 3.59 (s, 2H), 6.53 (d, *J* = 3.5 Hz, 1H), 7.28 (dd, *J* = 1.5, 8.4 Hz, 1H), 7.49 (d, *J* = 1.5 Hz, 1H), 7.57 (d, *J* = 3.5 Hz, 1H), 8.06 (d, *J* = 8.4 Hz, 1H).

### Step 3

In a similar manner to Step 2 of Reference Example 1-1, 5-(4,4-dimethoxypiperidinomethyl)-1-(*tert*-butoxycarbonyl)indole (1.20 g, 3.21 mmol) was dissolved in THF (24 mL), and the solution was treated with triisopropyl borate (1.11 mL, 4.81 mmol) and LDA-heptane/THF/ethylbenzene solution (2.0 mol/L, 4.01 mL, 8.02 mmol), followed by purification by slurry using diisopropyl ether and hexane to obtain Compound BT (753 mg, yield 56%).
APCI-MS *m*/*z*: 419 [M+H]⁺; ¹H-NMR (CDCl₃)δ(ppm): 1.73 (s, 9H), 1.75-1.81 (m, 4H), 2.40-2.48 (m, 4H), 3.18 (s, 6H), 3.59 (s, 2H), 6.80 (br s, 2H), 7.34 (dd, *J* = 1.3, 8.8 Hz, 1H), 7.44 (s, 1H), 7.52 (d, *J* = 1.3 Hz, 1H), 7.95 (d, *J* = 8.8 Hz, 1H).

### Reference Example 1-21 (Compound BU)

### Step 1

In a similar manner to Step 2 of Reference Example 1-18, 5-[4-(2-hydroxyethyl)-1-piperazinylmethyl]-1-(*tert*-butoxycarbony l)indole (5.02 g, 14.0 mmol) was dissolved in DMF (50 mL), and the solution was treated with imidazole (1.43 g, 21.0 mmol) and *tert*-butyldimethylsilyl chloride (3.16 g, 21.0 mmol) to obtain 5-{4-[2-(*tert*-butyldimethylsilyloxy)ethyl]-1-piperazinylmethyl}-1-(*tert*-butoxycarbonyl)indole (5.97 g, yield 90%). APCI-MS *m*/*z*: 474 [M+H]⁺; ¹H-NMR (CDCl₃)δ(ppm): 0.06 (s, 6H), 0.87 (s, 9H), 1.67 (s, 9H), 2.80-3.15 (m, 10H), 2.80-3.15 (m, 2H), 3.70-3.85 (m, 2H), 6.56 (d, *J* = 3.6 Hz, 1H), 7.32 (m, 1H), 7.58 (m, 1H), 7.61 (d, *J* = 3.6 Hz, 1H), 8.12 (d, *J* = 8.6 Hz, 1H).

### Step 2

In a similar manner to Step 2 of Reference Example 1-1, 5-{4-[2-(*tert*-butyldimethylsilyloxy)ethyl]-1-piperazinylmethyl}-1-(*tert*-butoxycarbonyl)indole (5.00 g, 10.6 mmol) was dissolved in THF (50 mL), and the solution was treated with triisopropyl borate (3.67 mL, 15.9 mmol) and LDA-heptane/THF/ethylbenzene solution (2.0 mol/L, 13.3 mL, 26.6 mmol), followed by purification by flash column chromatography (hexane/ethyl acetate=50/50 to 20/80 to 0/100) to obtain Compound BU (3.16 g, yield 58%).
ESI-MS *m*/*z*: 518 [M+H]⁺; ¹H-NMR (CDCl₃)δ(ppm): 0. 04 (s, 6H), 0.88 (s, 9H), 1.73 (s, 9H), 2.44-2.61 (m, 8H), 2.52 (t, *J* = 6.5 Hz, 2H), 3.58 (s, 2H), 3.75 (t, *J* = 6.5 Hz, 2H), 6.85 (br s, 2H), 7.34 (d, *J* = 8.7 Hz, 1H), 7.44 (s, 1H), 7.53 (s, 1H), 7.95 (d, *J* = 8.7 Hz, 1H).

### Reference Example 1-22 (Compound BV)

### Step 1

In a similar manner to Step 1 of Reference Example 1-4, 5-formylindole (2.00 g, 13.8 mmol) was dissolved in acetonitrile (20 mL), and the solution was treated with di-*tert*-butyldicarbonate (3.49 mL, 15.2 mmol) and DMAP (17.0 mg, 0.139 mmol), then the mixture was treated with 4-piperidinemethanol (6.36 g, 55.2 mmol), acetic acid (15.8 mL, 276 mmol) and sodium triacetoxyborohydride (8.77 g, 41.4 mmol) to obtain 5-(4-hydroxymethylpiperidinomethyl)-1-(*tert*-butoxycarbonyl)indol e (2.85 g, yield 60%).
ESI-MS *m*/*z*: 345 [M+H]⁺; ¹H-NMR (CDCl₃)δ(ppm): 1.24-1.36 (m, 2H), 1.44-1.74 (m, 3H), 1.67 (s, 9H), 1.91-2.02 (m, 2H), 2.89-2.97 (m, 2H), 3.49 (d, *J* = 6.4 Hz, 2H), 3.58 (s, 2H), 6.53 (dd, *J* = 0.7, 3.7 Hz, 1H), 7.27 (dd, *J* = 1.5, 8.6 Hz, 1H), 7.49 (dd, *J* = 0.7, 1.5 Hz, 1H), 7.57 (d, *J* = 3.7 Hz, 1H), 8.06 (d, *J* = 8.6 Hz, 1H).

### Step 2

In a similar manner to Step 1 of Reference Example 1-18, 5-(4-hydroxymethylpiperidinomethyl)-1-(*tert*-butoxycarbonyl)indol e (2.85 g, 8.27 mmol) was dissolved in DMF (50 mL), and the solution was treated with imidazole (619 mg, 9.09 mmol and *tert*-butyldimethylsilyl chloride (1.37 g, 9.09 mmol), followed by purification by flash column chromatography (hexane/ethyl acetate=95/5 to 80/20) to obtain 5-[4-(*tert*-butyldimethylsilyloxy)methylpiperidinomethyl]-1-(*tert* -butoxycarbonyl)indole (2.90 g, yield 76%).
ESI-MS *m*/*z*: 459 [M+H]⁺; ¹H-NMR (CDCl₃)δ(ppm): 0.02 (s, 6H), 0.88 (s, 9H), 1.14-1.40 (m, 2H), 1.59-1.74 (m, 3H), 1.66 (s, 9H), 1.88-2.00 (m, 2H), 2.86-2.95 (m, 2H), 3.41 (d, *J* = 6.4 Hz, 2H), 3.56 (s, 2H), 6.50 (dd, *J* = 0.6, 3.7 Hz, 1H), 7.25 (dd, *J* = 1.7, 8.3 Hz, 1H), 7.46 (dd, *J* = 0.6, 1.7 Hz, 1H), 7.55 (d, *J* = 3.7 Hz, 1H), 8.03 (d, *J* = 8.3 Hz, 1H).

### Step 3

In a similar manner to Step 2 of Reference Example 1-1, 5-[4-(*tert*-butyldimethylsilyloxy)methylpiperidinomethyl]-1-(*tert* -butoxycarbonyl) indole (2.90 g, 6.32 mmol) was dissolved in THF (30 mL), and the solution was treated with triisopropyl borate (1.51 mL, 6.54 mmol) and LDA-heptane/THF/ethylbenzene solution (2.0 mol/L, 5.45 mL, 10.9 mmol), followed by purification by flash column chromatography (hexane/ethyl acetate=50/50 to 20/80) to obtain Compound BV (1.91 g, yield 60%).
ESI-MS *m*/*z*: 503 [M+H]⁺; ¹H-NMR (CDCl₃)δ(ppm): 0.02 (s, 6H), 0.88 (s, 9H), 1.15-1.31 (m, 2H), 1.47 (m, 1H), 1.62-1.77 (m, 2H), 1.73 (s, 9H), 1.89-2.01 (m, 2H), 2.90 (d, *J* = 11.5 Hz, 2H), 3.43 (d, *J* = 6.4 Hz, 2H), 3.58 (s, 2H), 7.02 (br s, 2H), 7.33 (dd, *J* = 1.4, 8.7 Hz, 1H), 7.44 (s, 1H), 7.52 (d, *J* = 1.4 Hz, 1H), 7.94 (d, *J* = 8.7 Hz, 1H).

### Reference Example 1-23 (Compound BW)

### Step 1

In a similar manner to Step 1 of Reference Example 1-4, 5-formylindole (2.00 g, 13.8 mmol) was dissolved in acetonitrile (20 mL), and the solution was treated with di-*tert*-butyldicarbonate (3.49 mL, 15.2 mmol) and DMAP (17.0 mg, 0.139 mmol), then the mixture was treated with *N,N*,2,2-tetramethyl-1,3-propanediamine (8.79 mL, 55.2 mmol), acetic acid (15.8 mL, 276 mmol) and sodium triacetoxyborohydride (8.76 g, 41.3 mmol) to obtain 5-(*N,N*,2,2-tetramethyl-1,3-propanediaminomethyl)-1-(*tert*-butoxyc arbonyl)indole (10.2 g).

### Step 2

In a similar manner to Step 2 of Reference Example 1-1, 5-(*N,N,*2,2-tetramethyl-1,3-propanediaminomethyl)-1-(*tert*-butoxyc arbonyl)indole (10.2 g)was dissolved in THF (50 mL), and the solution was treated with triisopropyl borate (6.37 mL, 27.6 mmol) and LDA-heptane/THF/ethylbenzene solution (2.0 mol/L, 17.3 mL, 34.6 mmol), followed by purification by slurry using ethyl acetate and hexane to obtain Compound BW (2.52 g, yield 45%, 2 steps). ESI-MS *m*/*z*: 404 [M+H]⁺; ¹H-NMR (CDCl₃)δ(ppm): 0.90 (s, 6H), 1.73 (s, 9H), 2.16 (s, 2H), 2.25 (s, 6H), 2.47 (s, 2H), 3.88 (s, 2H), 7.33 (d, *J* = 8.5 Hz, 1H), 7.42 (br s, 1H), 7.52 (s, 1H), 7.95 (d, J = 8.5 Hz, 1H).

### Reference Example 1-24 (Compound BX)

### Step 1

In a similar manner to Step 1 of Reference Example 1-4, 5-formylindole (2.00 g, 13.8 mmol) was dissolved in acetonitrile (20 mL), and the solution was treated with di-*tert*-butyldicarbonate (3.49 mL, 15.2 mmol) and DMAP (17.0 mg, 0.139 mmol), then the mixture was treated with ethanolamine (8.33 mL, 138 mmol), acetic acid (15.8 mL, 276 mmol) and sodium triacetoxyborohydride (4.80 g, 22.6 mmol) to obtain 5-[(2-hydroxyethyl)aminomethyl]-1-(*tert*-butoxycarbonyl)indole (5.40 g).

### Step 2

In a similar manner to Step 1 of Reference Example 1-18, 5-[(2-hydroxyethyl)aminomethyl]-1-(*tert*-butoxycarbonyl)indole (5.40 g) was dissolved in DMF (50 mL) and the solution was treated with imidazole (940 mg, 13.8 mmol) and *tert*-butyl dimethylsilylchloride (2.08 g, 13.8 mmol), followed by purification by slurry using diisopropyl ether and hexane to obtain 5-{[2-(*tert*-butyldimethylsilyloxy)ethyl]aminomethyl}-1-(*tert*-but oxycarbonyl)indole (4.80 g, yield 86%, 2 steps).
ESI-MS *m*/*z*: 291 [M+H]⁺; ¹H-NMR (CDCl₃)δ(ppm): 0.07 (s, 6H), 0.86 (s, 9H), 1.65 (s, 9H), 3.00 (t, *J* = 5.2 Hz, 2H), 3.95 (t, *J* = 5.2 Hz, 2H), 4.24 (s, 2H), 6.57 (d, *J* = 3.6 Hz, 1H), 7.43 (d, *J* = 8.4 Hz, 1H), 7.60 (d, *J* = 3.6 Hz, 1H), 7.75 (s, 1H), 8.13 (d, *J* = 8.1 Hz, 1H).

### Step 3

In a similar manner to Step 2 of Reference Example 1-1, 5-{[2-(*tert*-butyldimethylsilyloxy)ethyl]aminomethyl}-1-(*tert*-but oxycarbonyl) indole (4. 70 g, 11.6 mmol) was dissolved in THF (50 mL), and the solution was treated with triisopropyl borate (4.02 mL, 17.4 mmol) and LDA-heptane/THF/ethylbenzene solution (2.0 mol/L, 14.5 mL, 29.0 mmol), followed by purification by flash column chromatography (chloroform/methanol =95/5 to 20/80) to obtain Compound BX (1.09 g, yield 21%).
ESI-MS *m*/*z*: 449 [M+H]⁺; ¹H-NMR (CDCl₃)δ(ppm): 0.06 (s, 6H), 0.89 (s, 9H), 1.73 (s, 9H), 2.77 (t, *J* = 5.2 Hz, 2H), 3.76 (t, *J* = 5.2 Hz, 2H), 3.92 (s, 2H), 7.34 (d, *J* = 8.8 Hz, 1H), 7.42 (s, 1H), 7.54 (s, 1H), 7.95 (d, *J* = 8.8 Hz, 1H).

### Reference Example 1-25 (Compound BY)

### Step 1

In a similar manner to Step 1 of Reference Example 1-1, 5-formylindole (1.00 g, 6.89 mmol) was treated with di-*tert*-butyldicarbonate (2.40 mL, 10.3 mmol) and DMAP (0.00840 g, 0.0688 mmol). Then, the mixture was treated with trimethyl orthoformate (1.50 mL, 13.8 mmol) and p-toluenesulfonic acid monohydrate (0.00260 g, 0.138 mmol) to obtain 1-(*tert*-butoxycarbonyl)-4-(dimethoxymethyl)indole (2.00 g, yield 99%).
ESI-MS *m*/*z*: 292 [M-CH₃O]⁺; ¹H-NMR (CDCl₃)δ(ppm): 1.53 (s, 9H), 3.35 (s, 6H), 5.68 (s, 1H), 6.82 (d, *J* = 3.8 Hz, 1H), 7.26-7.39 (m, 2H), 7.61 (d, *J* = 3.8 Hz, 1H), 8.15 (d, *J* = 7.9 Hz, 1H).

### Step 2

In a similar manner to Step 2 of Reference Example 1-1, Compound BY (1.40 g, yield 70%) was obtained by treating 1-(*tert*-butoxycarbonyl)-5-(dimethoxymethyl)indole (2.00 g, 6.89 mmol) with triisopropyl borate (2.40 mL, 10.3 mmol) and LDA-heptane/THF/ethylbenzene solution (2.0 mol/L, 10.3 mL, 20.7 mmol).
ESI-MS *m*/*z*: 288 [M-H]⁻; ¹H-NMR (CDCl₃)δ(ppm): 1.62 (s, 9H), 7.29 (d, *J* = 0.8 Hz, 1H), 7.52 (dd, *J* = 7.4, 8.2 Hz, 1H), 7.84 (dd, *J* = 0.8, 7.4 Hz, 1H), 8.43 (d, *J* = 8.2 Hz, 1H), 10.3 (s, 1H).

### Reference Example 1-26 (Compound BZ)

### Step 1

In a similar manner to Step 1 of Reference Example 1-4, 5-formylindole (5.27 g, 36.3 mmol) was dissolved in acetonitrile (100 mL), and the solution was treated with di-*tert*-butyldicarbonate (9.17 mL, 39.9 mmol) and DMAP (44.0 mg, 0.360 mmol), then the mixture was treated with 51% aqueous dimethylamine solution (72.0 mL, 724 mmol), acetic acid (41.6mL, 727 mmol) and sodium triacetoxyborohydride (46.2 g, 218 mmol), followed by purification by slurry using diisopropyl ether to obtain 5-(dimethylaminomethyl)-1-(*tert*-butoxycarbonyl)indole (3.61 g, yield 36%).
ESI-MS *m*/*z*: 275 [M+H]⁺; ¹H-NMR (CDCl₃)δ(ppm): 1.68 (s, 9H), 2.72 (s, 6H), 4.23 (s, 2H), 6.62 (d, *J* = 3.7 Hz, 1H), 7.46 (dd, *J* = 1.4, 8.5 Hz, 1H), 7.67 (d, *J* = 3.7 Hz, 1H), 7.80 (d, *J* = 1.4 Hz, 1H), 8.21 (d, J = 8.5 Hz, 1H).

### Step 2

In a similar manner to Step 2 of Reference Example 1-1, 5-(dimethylaminomethyl)-1-(*tert*-butoxycarbonyl)indole (7.30 g, 26.6 mmol) was dissolved in THF (80 mL), and the solution was treated with triisopropyl borate (9.21 mL, 39.9 mmol) and LDA-heptane/THF/ethylbenzene solution (2.0 mol/L, 33.3 mL, 66.6 mmol), followed by purification by slurry using hexane to obtain Compound BZ (4.92 g, yield 58%).
ESI-MS *m*/*z*: 319 [M+H]⁺; ¹H-NMR (CDCl₃)δ(ppm): 1.73 (s, 9H), 2.26 (s, 6H), 3.52 (s, 2H), 7.32 (dd, *J* = 1.4, 8.8 Hz, 1H), 7.43 (s, 1H), 7.52 (d, *J* = 1.4 Hz, 1H), 7.95 (d, *J* = 8.8 Hz, 1H).

### Reference Example 1-27 (Compound CA)

### Step 1

In a similar manner to Step 2 of Reference Example 1-2, 5-iodoindole (1.16 g, 4.78 mmol) was dissolved in acetonitrile (20 mL), and the solution was treated with di-*tert*-butyldicarbonate (1.32 mL, 5.73 mmol) and DMAP (5.8 mg, 0.0477 mmol), followed by purification by flash column chromatography (hexane/ethyl acetate=1/0 to 9/1) to obtain 1-(*tert*-butoxycarbonyl)-5-iodoindole (1.64 g, yield 99%). ESI-MS *m*/*z*: 344 [M+H]⁺; ¹H-NMR (CDCl₃)δ(ppm): 1.66 (s, 9H), 6.48 (d, J = 3.8 Hz, 1H), 7.52-7.62 (m, 2H), 7.87-7.94 (d, 1H).

### Step 2

1-(*tert*-Butoxycarbonyl)-5-iodoindole (3.46 g, 10.1 mmol) was dissolved in toluene (45.0 mL), and the solution was added with 4-methylpiperazine (2.24 mL, 20.2 mmol), 2,2'-(diphenylphosphino)-1,1'-binaphthyl (1.26 g, 2.02 mmol), cesium carbonate (4.94 mg, 15.2 mmol) and palladium acetate (226 mg, 1.01 mmol), followed by stirring under reflux for 24 hours. The mixture was added with water and extracted with ethyl acetate. The organic layer was washed with saturated brine and dried over anhydrous sodium sulfate. The solvent was evaporated under reduced pressure. The residue was purified by flash column chromatography (chloroform/methanol =9/1) to obtain 1-(*tert*-butoxycarbonyl)-5-(4-methylpiperazino)indole (887 mg, 28%).
ESI-MS *m*/*z*: 316 [M+H]⁺; ¹H-NMR (CDCl₃)δ(ppm): 1.46 (s, 9H), 2.29 (s, 3H), 2.33-2.38 (m, 4H), 2.55-2.61 (m, 4H), 6.50 (d, *J* = 3.6 Hz, 1H), 6.82-6.88 (m, 1H), 6.99-7.05 (m, 1H), 7.53 (d, *J* = 3.6 Hz, 1H), 7.88 (d, *J* = 9.2 Hz, 1H).

### Step 3

In a similar manner to Step 2 of Reference Example 1-1, 1-(*tert*-butoxycarbonyl)-5-(4-methylpiperazino)indole (880 mg, 2.79 mmol) was dissolved in THF (2.0 mL), and the solution was treated with triisopropyl borate (0.966 mL, 4.19 mmol) and LDA-heptane/THF/ethylbenzene solution (2.0 mol/L, 4.19mL, 8.37 mmol), then the mixture was purified by slurry using diisopropyl ether and hexane to obtain Compound CA (738 mg, yield 74%).
ESI-MS *m*/*z*: 360 [M+H]⁺; ¹H-NMR (CDCl₃)δ(ppm): 1.48 (s, 9H), 2.31 (s, 3H), 2.36-2.46 (m, 4H), 2.58-2.65 (m, 4H), 6.55 (s, 1H), 6.82-6.88 (m, 1H), 6.99-7.05 (m, 1H), 7.88 (d, *J* = 9.2 Hz, 1H).

### Reference Example 1-28 (Compound CB)

### Step 1

1-(*tert*-Butoxycarbonyl)-5-iodoindole (1.00 g, 2.91 mmol) was dissolved in diethylamine (20 mL), and the solution was added with bis(triphenylphosphine)dichloropalladium (163 mg, 0.233 mmol), cuprous iodide (44.4 mg, 0.233 mmol) and 1-dimethylamino-2-propyne (0.628 mL, 5.82 mmol), followed by stirring at 80°C for 7.0 hours. The reaction mixture was added with water and extracted with ethyl acetate. The organic layer was washed with saturated brine and dried over anhydrous sodium sulfate. The solvent was evaporated under reduced pressure. The residue was purified by preparative thin-layer chromatography (chloroform/methanol =19/1) to obtain 1-(*tert*-butoxycarbonyl)-5-(3-dimethylamino-2-propyn-1-yl)indole (0.78 g, yield 90%).
ESI-MS *m*/*z*: 299 [M+H]⁺; ¹H-NMR (CDCl₃)δ(ppm): 1.67 (s, 9H), 2.39 (s, 6H), 3.48 (s, 2H), 6.52 (d, *J* = 3.8 Hz, 1H), 7.38 (dd, *J* = 1.6, 8.4 Hz, 1H), 7.59 (d, *J* = 3.8 Hz, 1H), 7.65 (d, *J* = 1.6 Hz, 1H), 8.06 (d, *J* = 8.4 Hz, 1H).

### Step 2

1-(*tert*-Butoxycarbonyl)-5-(3-dimethylamino-2-propynl-yl)ind ole (0.78 g, 2.61 mmol) was dissolved in ethanol (78 mL), and the solution was added with 10% Pd/C (39.0 mg) under hydrogen atmosphere, followed by stirring at room temperature for 2 days. The reaction mixture was filterd using Celite, then the filtrate was concentrated. The residue was purified by preparative thin-layer chromatography (chloroform/methanol=19/1, 4/1.) to obtain 1-(*tert*-butoxycarbonyl)-5-(3-dimethylamino)propylindole (645 mg, yield 82%).
ESI-MS *m*/*z*: 303 [M+H]⁺; ¹H-NMR (CDCl₃)δ(ppm): 1.67 (s, 9H), 1.80-1.90 (m, 2H), 2.25 (s, 6H), 2.34 (t, *J* = 7.8 Hz, 2H), 2.73 (t, J = 7.8 Hz, 2H), 6.51 (dd, *J* = 0.6, 3.9 Hz, 1H), 7.19 (dd, *J* = 1.8, 8.7 Hz, 1H), 7.36 (d, *J* = 1.8 Hz, 1H), 7.56 (d, *J* = 3.9 Hz, 1H), 8.03 (d, *J* = 8.7 Hz, 1H).

### Step 3

1-(*tert*-Butoxycarbonyl)-5-(3-dimethylamino)propylindole (645 mg, 2.13 mmol) was dissolved in THF (6.5 mL), and the solution was added with triisopropyl borate (0.729 mL, 0.825 mmol). The mixture was cooled to 0°C and added with LDA-heptane/THF/ethylbenzene solution (2.98 mL, 2.0 mol/L, 5.96 mmol), followed by stirring at 0°C for 3.0 hours. The reaction mixture was added with 1 mol/L hydrochloric acid (25 mL) and stirred at room temperature for 1.0 hour. The mixture was added with a saturated aqueous sodium carbonate solution by drops until the pH of the mixture is adjusted to 8, then extracted with ethyl acetate. The organic layer was washed with saturated brine and dried over anhydrous sodium sulfate. The solvent was evaporated under reduced pressure. The residue was purified by slurry using diisopropyl ether and hexane to obtain Compound CB (486 mg, yield 66%).
¹H-NMR (DMSO-d₆)δ(ppm): 1.59 (s, 9H), 1.69-1.79 (m, 2H), 2.18 (s, 6H), 2.27 (t, *J* = 6.9 Hz, 2H), 2.65 (t, *J* = 7.5 Hz, 2H), 6.56 (s, 1H), 7.12 (dd, *J* = 1.5, 8.4 Hz, 1H), 7.36 (d, *J* = 1.5 Hz, 1H), 7.96 (d, *J* = 8.4 Hz, 1H), 8.17 (br s, 2H).

### Reference Example 1-29 (Compound CC)

### Step 1

5-Hydroxyindole (3.49 g, 26.2 mmol) was dissolved in acetonitrile (35.0 mL), and the solution was added with di-*tert*-butyldicarbonate (18.1 mL, 78.6 mmol) and DMAP (294 mg, 2.62 mmol), followed by stirring at room temperature for 4 hours. The reaction mixture was concentrated under reduced pressure, and the residue was dissolved in methanol (130 mL) and added with potassium carbonate (18.1 g, 131 mmol), followed by stirring at room temperature for 4 hours. The mixture was added with acetic acid (7.50 mL) to neutralize, and then added with water, followed by extracting with ethyl acetate. The organic layer was washed with saturated brine and dried over anhydrous sodium sulfate. The solvent was evaporated under reduced pressure. The residue was purified by flash column chromatography (hexane/ethyl acetate=2/1) to obtain 1-(*tert*-butoxycarbonyl)-5-hydroxyindole (5.74 g, 94%).
ESI-MS *m*/*z*: 234 [M+H]⁺; ¹H-NMR (CDCl₃)δ(ppm): 1.66 (s, 9H), 4.68 (s, 1H), 6.45 (d, *J* = 3.8 Hz, 1H), 6.83 (dd, *J* = 2.6, 8.9 Hz, 1H), 6.97 (d, *J* = 2.6 Hz, 1H), 7.56 (d, *J* = 3.8 Hz, 1H ), 7.98 (d, *J* = 8.9 Hz, 1H).

### Step 2

1-(*tert*-Butoxycarbonyl)-5-hydroxyindole (2.33 g, 10.0 mmol) and triphenylphosphine (5.25 g, 20.0 mmol) were dissolved in toluene (46.0 mL), and the solution was added with glycidol (1.32 mL, 20.0 mmol) and 40% DEAD-toluene solution (9.10 mL, 20 mmol) at room temperature, followed by stirring at 80°C for 4 hours. The solvent was evaporated under reduced pressure and the residue was purified by flash column chromatography (hexane/ethyl acetate=4/1). A crude product (1.28 g) of 1-(*tert*-butoxycarbonyl)-5-hydroxyindole (1.28 g) was obtained. The obtained crude product was dissolved in N,N-dimethylacetoamide (20.0 mL), and the solution was added with 2- (ethylamino) ethanol (8.60 mL, 87.8 mmol), followed by stirring at 80°C for 4 hours. The reaction mixture was added with water and extracted with ethyl acetate. The organic layer was washed with saturated brine and dried over anhydrous sodium sulfate. The solvent was evaporated under reduced pressure and the residue was purified by flash column chromatography (chloroform/methanol =9/1) to obtain 1-(*tert*-butoxycarbonyl)-5-{3-[N-ethyl(2-hydroxyethyl)amino]-2-hy droxypropoxy}indole (1.53 g, 40%).
ESI-MS *m*/*z*: 379 [M+H]⁺; ¹H-NMR (CDCl₃)δ(ppm): 1.07 (t, *J* = 7.2 Hz, 3H), 1.66 (s, 9H), 2.61-2.80 (m, 6H), 3.61-3.68 (m, 2H), 3.99-4.14 (m, 3H), 6.48 (d, *J* = 3.9 Hz, 1H), 6.94 (dd, *J* = 2.6, 8.7 Hz, 1H), 7.04 (d, *J* = 2.6 Hz, 1H), 7.56 (d, *J* = 3.9 Hz, 1H), 8.01 (d, *J* = 8.7 Hz, 1H).

### Step 3

1-(*tert*-Butoxycarbonyl)-5-{3-[N-ethyl(2-hydroxyethyl)amino] -2-hydroxypropoxy}indole (1.53 g, 4.04 mmol) was dissolved in acetonitrile (20.0 mL), and the solution was added with imidazole (1.10 g, 16.2 mmol) and *tert*-butyldimethylsilyl chloride (1.83 g, 12.1 mmol), followed by stirring at room temperature for 2 hours. The reaction mixture was added with water and extracted with ethyl acetate. The organic layer was washed with saturated brine and dried over anhydrous sodium sulfate. The solvent was evaporated under reduced pressure and the residue was purified by flash column chromatography (hexane/ethyl acetate=85/15) to obtain 1-(*tert*-butoxycarbonyl)-5-{3-[N-ethyl(2-(tert-butyldimethylsilyl oxy)ethyl)amino]-2-(tert-butyldimethylsilyloxy)propoxy}indole (2.34 g, 96%).
ESI-MS *m*/*z:* 607 [M+H]⁺.

### Step 4

In a similar manner to Step 2 of Reference Example 1-1, 1-(*tert*-butoxycarbonyl)-5-{3-[N-ethyl(2-(tert-butyldimethylsilyl oxy)ethyl)amino]-2-(tert-butyldimethylsilyloxy)propoxy}indole (2.34 g, 3.86 mmol) was dissolved in THF (46.0 mL), and the solution was treated with triisopropyl borate (1.33 mL, 5.78 mmol) and LDA-heptane/THF/ethylbenzene solution (2.0 mol/L, 5.78 mL, 11.6 mmol) to obtain Compound CC (1.89 g, yield 75%). ESI-MS *m*/*z*: 651 [M+H]⁺; ¹H-NMR (CDCl₃)δ(ppm): 0.04 (s, 6H), 0.10 (s, 3H), 0.12 (s, 3H), 0.88 (s, 9H), 0.90 (s, 9H), 1.02 (t, *J* = 6.7 Hz, 3H), 1.73 (s, 9H), 2.51-2.70 (m, 6H), 3.66 (t, *J* = 6.7 Hz, 2H), 3.82-3.92 (m, 1H), 4.01-4.22 (m, 2H), 6.90-6.74 (m, 3H), 7.87 (dd, *J* = 3.1, 9.4 Hz, 1H).

### Reference Example 1-30 (Compound CD)

### Step 1

4-Hydroxyindole (831 mg, 6.24 mmol) was dissolved in DMF (8.0 mL), and the solution was added with imidazole (510 mg, 7.49 mmol) and *tert*-butyldimethylsilyl chloride (1.03 g, 6.86 mmol), followed by stirring at room temperature for 5 hours. The reaction mixture was added with water and extracted with ethyl acetate. The organic layer was washed with saturated brine and dried over anhydrous sodium sulfate. The solvent was evaporated under reduced pressure and the residue was purified by flash column chromatography (hexane/ethyl acetate=85/15) to obtain 4-(*tert*-butyldimethylsilyloxy)indole (1.53 g, 99%).
ESI-MS *m*/*z:* 248 [M+H]⁺; ¹H-NMR (CDCl₃)δ(ppm): 0.24 (s, 6H), 1.06 (s, 9H), 6.52 (dd, *J* = 2.8, 5.4 Hz, 1H), 6.59 (dd, *J* = 2.3, 2.8 Hz, 1H), 7.01-7.08 (m, 1H), 7.09 (dd, *J* = 2.3, 3.3 Hz, 1H), 7.25 (s, 1H), 8.09 (br s, 1H).

### Step 2

In a similar manner to Step 2 of Reference Example 1-1, 4-(*tert*-butyldimethylsilyloxy) indole (1.53 g, 6.23 mmol) was treated with di-*tert*-butyldicarbonate (2.15 mL, 9.36 mmol) and DMAP (7.60 mg, 0.0624 mmol). Then, the residue was purified by flash column chromatography (hexane/ethyl acetate=10/1) to obtain 1-(*tert*-butoxycarbonyl)-4-(*tert*-butyldimethylsilyloxy)indole (2.17 g, 99%).
ESI-MS *m*/*z*: 348 [M+H]⁺; ¹H-NMR (CDCl₃)δ(ppm): 0.22 (s, 6H), 1.04 (s, 9H), 1.67 (s, 9H), 6.62 (dd, *J* = 0.8, 3.6 Hz, 1H), 6.65 (dd, *J* = 0.8, 8.2 Hz, 1H), 7.15 (dd, *J* = 8.2, 8.2 Hz, 1H), 7.49 (d, *J* = 3.6 Hz, 1H), 7.75 (d, *J* = 8.2 Hz, 1H).

### Step 3

In a similar manner to Step 2 of Reference Example 1-1, Compound CD (645 mg, yield 46%) was obtained by treating 1-(*tert*-butoxycarbonyl)-4-(*tert*-butyldimethylsilyloxy)indole (1.25 g, 3.60 mmol) with triisopropyl borate (1.25 mL, 5.40 mmol) and LDA-heptane/THF/ethylbenzene solution (2.0 mol/L, 5.40 mL, 10.8 mmol).
ESI-MS *m*/*z:* 392 [M+H]⁺; ¹H-NMR (DMSO-*d*₆)δ(ppm): 0.01 (s, 6H), 0.82 (s, 9H), 1.40 (s, 9H), 6.34 (d, *J* = 7.7 Hz, 1H). 6.46 (d, *J* = 7.7 Hz, 1H), 6.96 (dd, *J* = 7.7, 7.7 Hz, 1H), 7.52 (d, *J* = 7.7 Hz, 1H), 7.99 (s, 2H).

### Reference Example 1-31 (Compound CE)

### Step 1

In a similar manner to Step 1 of Reference Example 1-4, 5-formylindole (5.09 g, 35.1 mmol) was disolved in acetonitrile (50 mL), and the solution was treated with di-*tert*-butyldicarbonate (8.90 mL, 38.7 mmol) and DMAP (43.0 mg, 0.352 mmol). Then, the mixture was treated with diethylamine (36.3 mL, 351 mmol), acetic acid (40.1 mL, 700 mmol) and sodium triacetoxyborohydride (22.3 g, 105 mmol), followed by reduced concentration to obtain 5-(diethylaminomethyl)-1-(*tert*-butoxycarbonyl)indole (11.5 g). ESI-MS *m*/*z:* 303 [M+H]⁺

### Step 2

In a similar manner to Step 2 of Reference Example 1-1, 5-(diethylaminomethyl)-1-(*tert*-butoxycarbonyl)indole (11.5 g) was dissolved in THF (100 mL), and the solution was treated with triisopropyl borate (12.1 mL, 52.4 mmol) and LDA-heptane/THF/ethylbenzene solution (2.0 mol/L, 44.0 mL, 87.7 mmol). Then, the mixture was purified by slurry using hexane to obtain Compound CE (9.34 mg, yield 77%, 2 steps).
ESI-MS *m*/*z*: 347 [M+H]⁺; ¹H-NMR (CDCl₃)δ(ppm): 1.06 (t, *J* = 7.1 Hz, 6H), 1.73 (s, 9H), 2.54 (q, *J* = 7.1 Hz, 4H), 3.66 (s, 2H), 6.97 (br s, 2H), 7.36 (dd, *J* = 8.6 Hz, 1H), 7.44 (s, 1H), 7.54 (s, 1H), 7.95 (d, *J* = 8.6 Hz, 1H).

### Reference Example 1-32 (Compound CF)

### Step 1

5-Fluoro-2-nitrotoluene (500 mg, 3.22 mmol) was dissolved in acetonitrile (5 mL), and the solution was added with sodium carbonate (645 mg, 6.44 mmol) and piperidine (0.638 mL, 6.44 mmol) followed by stirring at room temperature for 12 hours, and then at 70°C for 3 hours. The precipitated solid was removed by filtration, and the reaction mixture was concentrated under reduced pressure. The residue was purified by flash column chromatography (hexane/ethyl acetate=100/0, 95/5) to obtain 2-nitro-5-piperidinotoluene (670 mg, 94%).
ESI-MS *m*/*z*: 221 [M+H]⁺; ¹H-NMR (CDCl₃)δ(ppm): 1.67-1.73 (m, 6H), 2.63 (s, 3H), 3.36-3.45 (m, 4H), 6.60 (d, *J* = 2.9 Hz, 1H), 6.67 (dd, *J* = 2.9, 9.2 Hz, 1H), 8.07 (d, *J* = 9.2 Hz, 1H).

### Step 2

2-Nitro-5-piperidinotoluene (670 mg, 3.04 mmol) was dissolved in DMF (15 mL), and the solution was added with pyrrolidine (0.760 mL, 9.10 mmol) and N,N-dimethylformamide dimethylacetal (1.22 mL, 9.15 mmol) followed by stirring at 120°C for 9 hours. Then, N,N-dimethylformamide dimethylacetal (0.41 mL, 3.1 mmol) was added and stirred at 120°C for 6 hours. The reaction mixture was added with saturated brine and extracted with ethyl acetate. The organic layer was washed with saturated brine and dried over anhydrous sodium sulfate. The solvent was evaporated under reduced pressure to obtain N,N-dimethyl[2-(2-nitro-5-piperidinophenyl)vinyl]amine (1.49 g).

### Step 3

N,N-Dimethyl[2-(2-nitro-5-piperidinophenyl)vinyl]amine (1.49 g) was dissolved in THF (30 mL), and the solution was added with 10%Pd-C (720 mg) follwed by stirring at room temperature for 15 hours under hydrogen atmosphere at normal pressure. The reaction mixture was filtered with Celite, and the solevnt was evaporated under reduced pressure. The residue was purified by flash column chromatography (hexane/ethyl acetate=90/10, 80/20, 75/25) to obtain 5-piperidinoindole (180 mg, 29%, 2 steps).
ESI-MS *m*/*z:* 201 [M+H]⁺; ¹H-NMR (CDCl₃)δ(ppm): 1.53-1.64 (m, 2H), 1.73-1.84 (m, 4H), 3.06-3.15 (m, 4H), 6.47 (m, 1H), 7.01 (dd, *J* = 2.1, 8.8 Hz, 1H), 7.16 (dd, *J* = 2.6, 2.9 Hz, 1H), 7.20 (d, *J* = 2.1 Hz, 1H), 7.29 (d, *J* = 8.8 Hz, 1H), 8.02 (br s, 1H).

### Step 4

In a similar manner to Step 2 of Reference Example 1-2, 5-piperidinoindole (180 mg, 0.899 mmol) was dissolved in acetonitrile (10 mL), and the solution was treated withdi-*tert*-butyldicarbonate (0.227 mL, 0.980 mmol) and DMAP (1.0 mg, 0.0089 mmol), followed by purification by flash column chromatography (hexane/ethyl acetate=95/5, 90/10) to obtain 5-piperidino-1-(*tert*-butoxycarbonyl)indole (194 mg, 72%).
ESI-MS *m*/*z*: 301 [M+H]⁺; ¹H-NMR (CDCl₃)δ(ppm): 1.53-1.62 (m, 2H), 1.65 (s, 9H), 1.70-1.80 (m, 4H), 2.99-3.16 (m, 4H), 6.47 (d, *J* = 3.5 Hz, 1H), 7.03 (dd, *J* = 2.3, 8.6 Hz, 1H), 7.08 (d, *J* = 2.3 Hz, 1H), 7.52 (d, *J* = 3.5 Hz, 1H), 7.98 (d, *J* = 8.6 Hz, 1H).

### Step 5

In a similar manner to Step 2 of Reference Example 1-1, 5-piperidino-1-(*tert*-butoxycarbonyl)indole (190 mg, 0.632 mmol) was dissolved in THF (5 mL), and the solution was treated with triisopropyl borate (0.220 mL, 0.953 mmol) and LDA-heptane/THF/ethylbenzene solution (2.0 mol/L, 0.80 mL, 1.6 mmol). Then, the mixture was purified by slurry using hexane to obtain Compound CF (127 mg, yield 58%).
ESI-MS *m*/*z:* 345 [M+H]⁺; ¹H-NMR (CDCl₃)δ(ppm): 1.52-1.64 (m, 2H), 1. 70-1. 80 (m, 4H), 1. 72 (s, 9H), 3.11-3.28 (m, 4H), 6.93 (br s, 2H), 7.05-7.10 (m, 2H), 7.38 (s, 1H), 7.86 (d, *J* = 8.9 Hz, 1H).

### Reference Example 1-33 (Compound CG)

### Step 1

In a similar manner to Step 1 of Reference Example 1-32, 5-fluoro-2-nitrotoluene (500 mg, 3.22 mmol) was disolved in acetonitrile (5 mL), and the solution was treated with calcium carbonate (645 mg, 6.44 mmol) and cyclohexylamine (0.737 mL, 6.44 mmol) to obtain 2-nitro-5-(cyclohexylamino)toluene (831 mg).

### Step 2

In a similar manner to Step 2 of Reference Example 1-32, 2-nitro-5-(cyclohexylamino)toluene (831 mg) was dissolved in DMF (15 mL), and the solution was treated with pyrrolidine (0.806 mL, 9.66 mmol) and *N,N*-dimethylformamide dimethylacetal (1.29 mL, 9.68 mmol) to obtain N,N-dimethyl{2-[2-nitro-5-(cyclohexylamino)phenyl]vinyl}amine (1.60 g).

### Step 3

In a similar manner to Step 3 of Reference Example 1-32, N,N-dimethyl{2-[2-nitro-5-(cyclohexylamino)phenyl]vinyl}amine (1.60 g) was dissolved in THF (30 mL), and the solution was treated with 10%Pd-C (720 mg) under hydrogen atmosphere at normal pressure, followed by purification by flash column chromatography (hexane/ethyl acetate=90/10, 85/15) to obtain 5-(cyclohexylamino)indole (429 mg, 62%, 3 steps).
ESI-MS *m*/*z*: 215 [M+H]⁺; ¹H-NMR (CDCl₃)δ(ppm): 1.07-1.46 (m, 5H), 1.65 (m, 1H), 1.70-1.82 (m, 2H), 2.04-2.16 (m, 2H), 3.26 (m, 1H), 6.38 (m, 1H), 6.61 (dd, *J* = 2.2, 8.6 Hz, 1H), 6.86 (d, *J* = 2.2 Hz, 1H), 7.10 (t, *J* = 2.7 Hz, 1H), 7.18 (d, *J* = 8.6 Hz, 1H), 7.94 (br s, 1H).

### Step 4

In a similar manner to Step 2 of Reference Example 1-2, 5-(cyclohexylamino)indole (420 mg, 1.96 mmol) was dissolved in acetonitrile (10 mL), and the solution was treated with di-*tert*-butyldicarbonate (0.946 mL, 4.12 mmol) and DMAP (2.4 mg, 0.020 mmol), followed by purification by flash column chromatography (hexane/ethyl acetate=100/0, 95/5) to obtain 5-[N-(*tert*-butoxycarbonyl)cyclohexylamino]-1-(*tert*-butoxycarbony l)indole (420 mg, 52%).
ESI-MS *m*/*z*: 415 [M+H]⁺; ¹H-NMR (CDCl₃)δ(ppm): 1.02-1.43 (m, 4H), 1.35 (s, 9H), 1.49-1.78 (m, 4H), 1.67 (s, 9H), 1.85-1.95 (m, 2H), 4.13 (m, 1H), 6.54 (d, *J* = 3.7 Hz, 1H), 6.99 (dd, *J* = 1.8, 8.6 Hz, 1H), 7.25 (m, 1H), 7.60 (d, *J* = 3.7 Hz, 1H), 8.06 (d, *J* = 8.6 Hz, 1H).

### Step 5

In a similar manner to Step 2 of Reference Example 1-1, 5-[N-(*tert*-butoxycarbonyl)cyclohexylamino]-1-(*tert*-butoxycarbony 1)indole (415 mg, 1.00 mmol) was dissolved in THF (5 mL), and the solution was treated with triisopropyl borate (0.346 mL, 1.50 mmol) and LDA-heptane/THF/ethylbenzene solution (2.0 mol/L, 1.25 mL, 2.5 mmol). Then, the mixture was purified by slurry using hexane and diisopropylether to obtain Compound CG (65.0 mg, yield 14%).
ESI-MS *m*/*z*: 459 [M+H]⁺; ¹H-NMR (CDCl₃)δ(ppm): 1.03-1.20 (m, 2H), 1.24-1.44 (m, 2H), 1.35 (s, 9H), 1.45-1.67 (m, 2H), 1.68-1.82 (m, 2H), 1.75 (s, 9H), 1.85-1.96 (m, 2H), 4.13 (m, 1H), 6.79 (s, 1H), 7.04 (dd, *J* = 2.0, 9.0 Hz, 1H), 7.46 (s, 1H), 7.95 (d, *J* = 9.0 Hz, 1H).

### Reference Example 1-34 (Compound CH)

### Step 1

In a similar manner to Step 1 of Reference Example 1-32, 5-fluoro-2-nitrotoluene (500 mg, 3.22 mmol) was dissolved in acetonitrile (5 mL), and the solution was treated with calcium carbonate (645 mg, 6.44 mmol) and diethylamine (0.670 mL, 6.48 mmol) to obtain 2-nitro-5-(diethylamino)toluene (702 mg).

### Step 2

In a similar manner to Step 2 of Reference Example 1-32, 2-nitro-5-(diethylamino)toluene (702 mg) was dissolved in DMF (15 mL), and the solution was treated with pyrrolidine (0.806 mL, 9.68 mmol) and *N,N*-dimethylformamide dimethylacetal (1.29 mL, 9.68 mmol) to obtain N,N-dimethyl{2-[2-nitro-5-(diethylamino)phenyl]vinyl}amine (1.20 g).

### Step 3

In a similar manner to Step 3 of Reference Example 1-32, N,N-dimethyl{2-[2-nitro-5-(diethylamino)phenyl]vinyl}amine (1.20 g) was dissolved in THF (30 mL), and the solution was treated with 10%Pd-C (720 mg) under hydrogen atmosphere at normal pressure, followed by purification by flash column chromatography (hexane/ethyl acetate=90/10, 85/15, 75/25) to obtain 5-(diethylamino)indole (407 mg, yield 67%, 3 steps).
ESI-MS *m*/*z*: 189 [M+H]⁺; ¹H-NMR (CDCl₃)δ(ppm): 1.11 (t, *J* = 7.1 Hz, 6H), 3.29 (q, *J* = 7.1 Hz, 4H), 6.43 (m, 1H), 6.87 (dd, *J* = 2.4, 8.7 Hz, 1H), 7.06 (d, *J* = 2.4 Hz, 1H), 7.13 (t, *J* = 2.8 Hz, 1H), 7.27 (d, *J* = 8.7 Hz, 1H), 7.98 (s, 1H).

### Step 4

In a similar manner to Step 2 of Reference Example 1-2, 5-(diethylamino)indole (400 mg, 2.12 mmol) was dissolved in acetonitrile (10 mL), and the solution was treated with di-*tert*-butyldicarbonate (0.536 mL, 2.33 mmol) andDMAP (2.6 mg, 0.021 mmol), followed by purification by flash column chromatography (hexane/ethyl acetate=100/0, 95/5) to obtain 5-diethylamino-1-(*tert*-butoxycarbonyl)indole (519 mg, yield 85%). ESI-MS *m*/*z*: 289 [M+H]⁺; ¹H-NMR (CDCl₃)δ(ppm): 1.15 (t, *J* = 7.0 Hz, 6H), 1.65 (s, 9H), 3.35 (q, *J* = 7.0 Hz, 4H), 6.44 (d, *J* = 3.6 Hz, 1H), 6.82 (dd, *J* = 2.3, 9.0 Hz, 1H), 6.85 (d, *J* = 2.3 Hz, 1H), 7.50 (d, *J* = 3.6 Hz, 1H), 7.93 (d, *J* = 9.0 Hz, 1H).

### Step 5

In a similar manner to Step 2 of Reference Example 1-1, 5-diethylamino-1-(*tert*-butoxycarbonyl)indole (515 mg, 1.79 mmol) was dissolved in THF (10 mL), and the solution was treated with triisopropyl borate (0.620 mL, 2.69 mmol) and LDA-heptane/THF/ethylbenzene solution (2.0 mol/L, 2.24 mL, 4.5 mmol). Then, the mixture was purified by slurry using hexane and diisopropylether to obtain Compound CH (129 mg, yield 22%). ESI-MS *m*/*z*: 333 [M+H]⁺; ¹H-NMR (CDCl₃)δ(ppm): 1.16 (t, *J* = 7.0 Hz, 6H), 1.72 (s, 9H), 3.37 (q, *J* = 7.0 Hz, 4H), 6.81-6.89 (m, 2H), 6.95 (br s, 2H), 7.36 (s, 1H), 7.83 (d, *J* = 8.9 Hz, 1H).

### Reference Example 1-35 (Compound CI)

### Step 1

In a similar manner to Step 1 of Reference Example 1-2, indole-5-carboxylic acid (1.00 g, 6.21 mmol) was dissolved in DMF (10 mL), and the solution was treated with EDCI (1.73 g, 12.4 mmol), HOBt monohydrate (839 mg, 6.21 mmol), and piperidine (0.922 mL, 9.31 mmol). The reaction mixture was added with water, and the precipitated solid was collected by filtration. The obtained solid was washed with water and dried under reduced pressure to obtain 5-(piperidinocarbonyl)indole (1.25 g, yield 88%). ESI-MS *m*/*z*: 229 [M+H]⁺

### Step 2

In a similar manner to Step 2 of Reference Example 1-2, 5-(piperidinocarbonyl)indole (1.23 g, 5.34 mmol) was dissolved in acetonitrile (40 mL), and the solution was treated with di-*tert*-butyldicarbonate (1.35 mL, 5.88 mmol) and DMAP (6.0 mg, 0.054 mmol), followed by purification by flash column chromatography (hexane/ethyl acetate=95/5, 90/10, 80/20) to obtain 5-(piperidinocarbonyl)-1-(tert-butoxycarbonyl)indole (1.75 mg, yield 100%).
ESI-MS *m*/*z:* 329 [M+H]⁺; ¹H-NMR (CDCl₃)δ(ppm): 1.50-1.80 (m, 6H), 1.68 (s, 9H), 3.24-3.84 (m, 4H), 6.59 (d, *J* = 3.6 Hz, 1H), 7.34 (dd, *J* = 1.7, 8.6 Hz, 1H), 7.61-7.77 (m, 2H), 8.15 (d, *J* = 8.6 Hz, 1H).

### Step 3

In a similar manner to Step 2 of Reference Example 1-1, 5-(piperidinocarbonyl)-1-(tert-butoxycarbonyl)indole (1.75 g, 5.33 mmol) was dissolved in THF (40 mL), and the solution was treated with triisopropyl borate (1.84 mL, 7.97 mmol) and LDA-heptane/THF/ethylbenzene solution (2.0 mol/L, 6.7 mL, 13 mmol). Then, the mixture was purified by slurry using diisopropylether to obtain Compound CI (1.85 g, yield 93%).
ESI-MS *m*/*z*: 373 [M+H]⁺; ¹H-NMR (CDCl₃)δ(ppm): 1.60-1.75 (m, 6H), 1.75 (s, 9H), 3.28-3.87 (m, 4H), 6.87 (br s, 2H), 7.39 (dd, *J* = 1.5, 8.8 Hz, 1H), 7.47 (s, 1H), 7.64 (d, *J* = 1.5 Hz, 1H), 8.02 (d, *J* = 8.8 Hz, 1H).

### Reference Example 1-36 (Compound CJ)

### Step 1

5-Hydroxyindole (1.00 g, 7.51 mmol) was dissolved in DMF (10 mL), and the solution was added with potassium carbonate (2.08 g, 15.0 mmol) and isopropyl bromide (0.846 mL, 9.01 mmol) followed by stirring at 60°C for 6 hours. Then, the reaction mixtutre was added with potassium carbonate (2.08 g, 15.0 mmol) and isopropyl bromide (1.41 mL, 15.0 mmol), followed by stirring at 60°C for 3 hours. The reaction mixture was added with water and then extracted with ethyl acetate. The organic layer was washed with saturated brine and dried over anhydrous sodium sulfate, and then evaporated under reduced pressure to obtain 5-(2-propyloxy)indole (1.69 g).

### Step 2

In a similar manner to Step 2 of Reference Example 1-2, 5- (2-propyloxy) indole (1.69 g) was dissolved in acetonitrile (10 mL), and the solution was treated with di-*tert*-butyldicarbonate (1.90 mL, 8.27 mmol) and DMAP (9.2 mg, 0.075 mmol) to obtain 5-(2-propyloxy)-1-(tert-butoxycarbonyl)indole (2.26 g).
ESI-MS *m*/*z*: 373 [M+H]⁺; ¹H-NMR (CDCl₃)δ(ppm): 1.60-1.75 (m, 6H), 1.75 (s, 9H), 3.28-3.87 (m, 4H), 6.87 (br s, 2H), 7.39 (dd, *J* = 1.5, 8.8 Hz, 1H), 7.47 (s, 1H), 7.64 (d, *J* = 1.5 Hz, 1H), 8.02 (d, *J* = 8.8 Hz, 1H).

### Step 3

In a similar manner to Step 2 of Reference Example 1-1, 5-(2-propyloxy)-1-(tert-butoxycarbonyl)indole was dissolved in THF (20 mL), and the solution was treated with triisopropyl borate (2.60 mL, 11.3 mmol) and LDA-heptane/THF/ethylbenzene solution (2.0 mol/L, 9.4 mL, 19 mmol). Then, the mixture was purified by slurry using hexane to obtain Compound CJ (1.72 g, yield 72%, 3 steps). ESI-MS *m*/*z*: 392 [M+H]⁺; ¹H-NMR (CDCl₃)δ(ppm): 0.20 (s, 6H), 1.00 (s, 9H), 1.72 (s, 9H), 6.88 (dd, *J* = 2.6, 9.0 Hz, 1H), 6.97 (br s, 2H), 7.00 (d, J = 2.6 Hz, 1H), 7.37 (s, 1H), 7.84 (d, *J* = 9.0 Hz, 1H).

### Reference Example 1-37 (Compound CK)

### Step 1

In a similar manner to Step 1 of Reference Example 1-1, 2-formylpyrrole (2.00 g, 21.0 mmol) was treated with di-*tert*-butyldicarbonate (5.30 mL, 21. 9 mmol) andDMAP (26.0 mg, 0.210 mmol), and then with trimethyl orthoformate (4.60 mL, 42.0 mmol) and p-toluenesulfonic acid monohydrate (80.0 mg, 0.421 mmol) to obtain 1-(tert-butoxycarbonyl)-2-(dimethoxymethyl)pyrrole (3.90 g).

### Step 2

In a similar manner to Step 2 of Reference Example 1-1, 1-(tert-butoxycarbonyl)-2-(dimethoxymethyl)pyrrole (3.90 g) was treated with triisopropyl borate (7.30 mL, 31.6 mmol) and LDA-heptane/THF/ethylbenzene solution (2.0 mol/L, 26.3 mL, 53 mmol) to obtain Compound CK (2.04 g, yield 70%, 2 steps). ESI-MS *m*/*z*: 140 [M+H]⁺; ¹H-NMR (DMSO-*d*₆)δ(ppm): 6.74 (m, 1H), 6.95 (m, 1H), 8.17 (s, 2H), 9.55 (s, 1H), 11.66 (br s, 1H).

Hereinafter, structures of Compounds BA to BZ obtained in Reference Examples 1-1 to 1-37 will be described in Tables 13-1 to 13-3. In addition, Me, TBS and Boc respectively represents methyl, tert-butyldimethylsilyl and tert-butoxycarbonyl in the following tables.

(HO)₂B-R^{1B}

**Table 18-1**

| Compound Number | R^{1B} |
|---|---|
| BA | 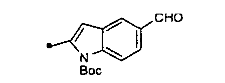 |
| BB | 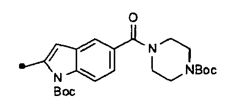 |
| BC | 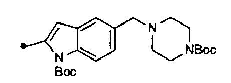 |
| BD | 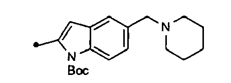 |
| BE | 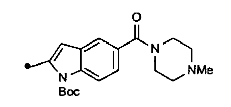 |
| BF | 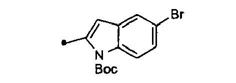 |
| BG | 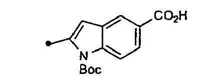 |
| BH | 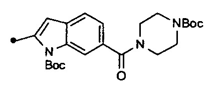 |
| BI | 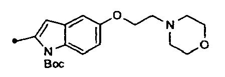 |
| BJ | 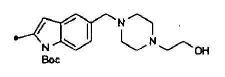 |
| BK | 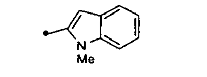 |
| BL | 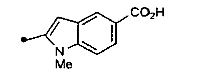 |
| BM | 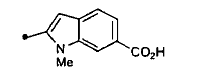 |
| BN | 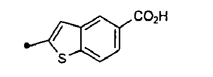 |

**Table 18-2**

| Compound Number | R^{1B} |
|---|---|
| BO | 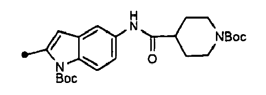 |
| BP | 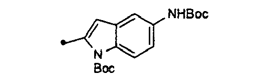 |
| BQ | 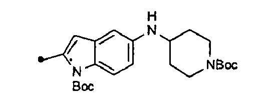 |
| BR | 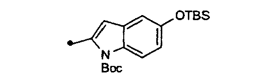 |
| BS | 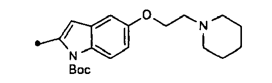 |
| BT | 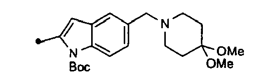 |
| Bu | 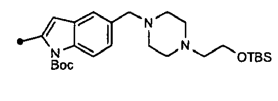 |
| BV | 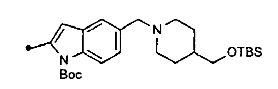 |
| BW | 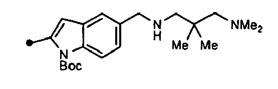 |
| BX | 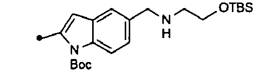 |
| BY | 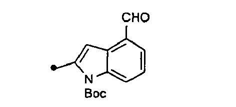 |
| BZ | 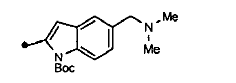 |

**Table 18-3**

| Compound Number | R^{1B} |
|---|---|
| CA | 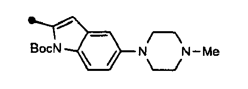 |
| CB | 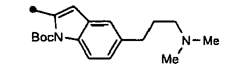 |
| CC | 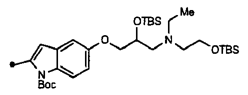 |
| CD | 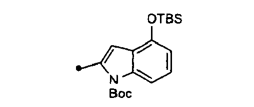 |
| CE | 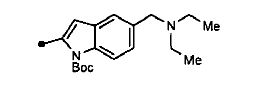 |
| CF | 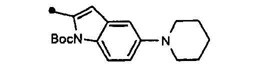 |
| CG | 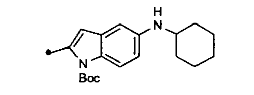 |
| CH | 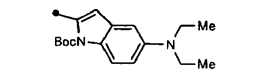 |
| CI | 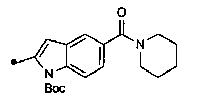 |
| CJ | 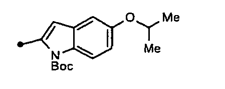 |
| CK | 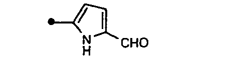 |

### Reference Example 2-1:

### 3-Amino-6-(1H-indol-2-yl)phthalimide (Compound 1)

### Step 1

3-Aminophthalimide (2.00 g, 12.3 mmol) was dissolved in methanol (200 mL), and the solution was added with N-bromosuccinimide (2.19 g, 12.3 mmol), followed by stirring at room temperature for 50 minutes. The obtained solid was collected by filtration and washed with methanol to obtain 3-amino-6-bromophthalimide (2.21 g, yield 74%).
APCI-MS *m*/*z*: 241 [M-H]⁻; ¹H-NMR (DMSO-*d*₆)δ(ppm): 6.53 (br s, 2H), 6.88 (d, *J* = 8.9 Hz, 1H), 7.49 (d, *J* = 8.9 Hz, 1H), 11.07 (br s, 1H).

### Step 2

3-Amino-6-bromophthalimide (100 mg, 0.415 mmol) was dissolved in acetonitrile (7 mL), and the solution was added with 1-(*tert*-butoxycarbonyl)indole-2-boronic acid (217 mg, 0.830 mmol), palladium acetate (7.5 mg, 0.033 mmol), tri(o-tolyl)phosphine (20 mg, 0.066 mmol) and triethylamine (0.578 mL, 4.15 mmol), followed by stirring under refulx for 7.5 hours under argon atmosphere. The reaction mixture was added with water and extracted with ethyl acetate. The organic layer was washed with saturated brine and dried over anhydrous sodium sulfate. The solvent was evaporated under reduced pressure and the residue was purified by preparative thin-layer chromatography (chloroform/methanol =20/1, chloroform/acetonitrile=15/1) to obtain 3-amino-6-[1-(tert-butoxycarbonyl)indol-2-yl]phthalimide (106 mg, yield 67%).
APCI-MS *m*/*z*: 376 [M-H]-; ¹H-NMR (DMSO-*d*₆)δ(ppm): 1.27 (s, 9H), 6.55 (s, 2H), 6.65 (s, 1H), 7.02 (d, *J* = 8.4 Hz, 1H), 7.24 (dd, *J* = 6.8, 7.5 Hz, 1H), 7.33 (dd, *J* = 7.2, 7.2 Hz, 1H), 7.43 (d, *J* = 8.4 Hz, 1H), 7.59 (d, *J* = 7.7 Hz, 1H), 8.13 (d, *J* = 8.1 Hz, 1H), 10.96 (s, 1H).

### Step 3

3-Amino-6-[1-(tert-butoxycarbonyl)indol-2-yl]phthalimide (21.8 mg, 0.0578 mmol) was dissolved in methanol (2.2 mL), and the solution was added with 10% hydrogen chloride-methanol solution (2.2 mL), followed by stirring at 70°C for 1.3 hours. The reaction mixture was added with saturated aqueous sodium hydrogencarbonate solution, and extracted with ethyl acetate. The organic layer was washed with saturated brine and dried over anhydrous sodium sulfate. The solvent was evaporated under reduced pressure and the residue was purified by preparative thin-layer chromatography (chloroform/acetone=6/1, chloroform/methanol =12/1) to obtain Compound 1 (14 mg, yield 87%).
APCI-MS *m*/*z*: 276 [M-H]⁻; ¹H-NMR (DMSO-*d*₆)δ(ppm): 6.71 (s, 2H), 7.00-7.09 (m, 4H), 7.46 (d, *J* = 7.9 Hz, 1H), 7.54 (d, *J* = 7.6 Hz, 1H), 8.03 (d, *J* = 8.8 Hz, 1H), 11.31 (s, 1H), 12.09 (s, 1H).

### Reference Example 2-2:

### 3-Amino-4-phenyl-6-[1H-5-(4-methylpiperazin-1-ylsulfonyl)indol-2 -yl]phthalimide (Compound 2)

### Step 1

Iodine (6.29 g, 24.8 mmol) was dissolved in ethanol (225 mL), and the solution was added with silver sulfate (3.87 g, 12.4 mmol) and 3-amino-6-bromophthalimide (3.00 g, 12.4 mmol), followed by stirring at room temperature for 23 hours. The reaction mixture was added with 10% aqueous sodium thiosulfate solution and extracted with ethyl acetate. The organic layer was washed with 0.5 mol/L hydrochloric acid and saturated brine and dried over anhydrous sodium sulfate. The solvent was evaporated under reduced pressure. The residue was purified by slurry using hexane and methanol to obtain 3-amino-4-iodo-6-bromophthalimide (4.3 g, yield 96%). APCI-MS *m*/*z*: 365 [M-H]⁻; ¹H-NMR (DMSO-*d*₆)δ(ppm): 6.34 (s, 2H), 8.10 (s, 1H), 11.23 (s, 1H).

### Step 2

3-Amino-4-iodo-6-bromophthalimide (80.0 mg, 0.218 mmol) was dissolved in THF (5.6 mL), and the solution was added with phenylboronic acid (80 mg, 0.65 mmol), tetrakis(triphenylphosphine)palladium (20 mg, 0.017 mmol) and copper(I) thiophene-2-carboxylate (125 mg, 0.65 mmol), followed by stirring at room temperature for 14 hours under argon atmosphere. The reaction mixture was added with saturated aqueous ammonium chloride solution and extracted with ethyl acetate. The organic layer was washed with saturated aqueous sodium hydrogencarbonate solution and saturated brine and dried over anhydrous sodium sulfate. The solvent was evaporated under reduced pressure. The residue was purified by slurry using isopropylether, then by preparative thin-layer chromatography (chloroform/methanol =60/1) to obtain 3-amino-4-phenyl-6-bromophthalimide (46 mg, yield 66%) and 3-amino-4,6-diphenylphthalimide (7.6 mg, yield 11%). 3-amino-4-phenyl-6-bromophthalimide APCI-MS *m*/*z*: 315 [M-H]⁻; ¹H-NMR (DMSO-*d*₆)δ(ppm): 6.10 (s, 2H), 7.45-7.53 (m, 6H), 11.24 (s, 1H).
3-amino-4,6-diphenylphthalimide
APCI-MS *m*/*z*: 313 [M-H]⁻; ¹H-NMR (DMSO-*d*₆)δ(ppm): 5.58 (s, 2H), 7.27-7.58 (m, 11H), 7.63 (br s, 1H).

### Step 3

In a similar manner to Step 2 of Reference Example 2-1, 3-amino-4-phenyl-6-bromophthalimide (70.0 mg, 0.221 mmol) was dissolved in acetonitrile (5.6 mL), and the solution was treated with a mixture (281 mg) of 1-(*tert*-butoxycarbonyl)-5-(4-methylpiperazin-1-ylsulfonyl)indol-2-boronic acid synthesized in a similar manner to the literature [Bioorganic & Medicinal Chemistry Letters, 2004, vol.14, p.351] and 5-(4-methylpiperazin-1-ylsulfonyl)-1*H*-indol-2-boronic acid, palladium acetate (4.0 mg, 0.018 mmol), tri(o-tolyl)phosphine (11 mg, 0.035 mmol) and triethylamine (0.308 mL, 2.21 mmol), followed by purification by preparative thin-layer chromatography (ethyl acetate/methanol=20/1) to obtain Compound 2 (21 mg, yield 19%) and 3-amino-4-phenyl-6-[1-(tert-butoxycarbonyl)-5-(4-methylpiperazin -1-ylsulfonyl)indol-2-yl]phthalimide(28 mg, yield 20%).

### Compound 2

ESI-MS *m*/*z*: 516 [M+H]⁺; ¹H-NMR (DMSO-*d*₆)δ(ppm): 2.18 (br s, 3H), 2.45 (br s, 4H), 2.88 (br s, 4H), 6.32 (s, 2H), 7.40-7.57 (m, 7H), 7.68 (d, *J* = 8.6 Hz, 1H), 7.88 (s, 1H), 7.95 (s, 1H), 11.43 (s, 1H), 12.35 (s, 1H).

### 3-Amino-4-phenyl-6-[1-(tert-butoxycarbonyl)-5-(4-methylpiperazin -1-ylsulfonyl)indol-2-yl]phthalimide

ESI-MS *m*/*z*: 616 [M+H]⁺; ¹H-NMR (DMSO-*d*₆)δ(ppm): 1.32 (s, 9H), 2.12 (s, 3H), 2.35 (br s, 4H), 2.89 (br s, 4H), 6.97 (s, 2H), 7.41 (s, 1H), 7.45-7.53 (m, 5H), 7.68 (dd, *J* = 1.9, 8.8 Hz, 1H), 8.01 (d, *J* = 1.6 Hz, 1H), 8.32 (d, *J* = 8.9 Hz, 1H), 11.18 (s, 1H).

### Reference Example 2-3:

### 3-Amino-6-[1H-5-(piperazin-1-ylmethyl)indol-2-yl]phthalimide dihydrochloride (Compound 5)

### Step 1

In a similar manner to Step 2 of Reference Example 2-1, 3-amino-6-bromophthalimide (30.0 mg, 0.124 mmol) was dissolved in acetonitrile (2 mL), and the solution was treated with Compound BC (85.0 mg, 0.185 mmol), palladium acetate (2.2 mg, 0.0098 mmol), tri(o-tolyl)phosphine (6.0 mg, 0.020 mmol) and triethylamine (0.169 mL, 1.24 mmol), followed by purification by flash column chromatography (hexane/ethyl acetate=4/1, 3/2, 1/1, 2/3) to obtain 3-amino-6-[1-(*tert*-butoxycarbonyl)-5-(piperazin-1-ylmethyl)indol -2-yl]phthalimide (26.7 mg, yield 37%).
ESI-MS *m*/*z*: 576 [M+H]⁺; ¹H-NMR (CDCl₃)δ(ppm): 1.43 (s, 9H), 1.45 (s, 9H), 2.36-2.46 (m, 4H), 3.48-3.58 (m, 4H), 3.60 (s, 2H), 5.37 (s, 2H), 6.54 (s, 1H), 6.90 (d, *J* = 8.4 Hz, 1H), 7.29 (dd, *J* = 3.8, 8.6 Hz, 1H), 7.44 (d, *J* = 8.4 Hz, 1H), 7.47 (br s, 1H), 7.50 (m, 1H), 8.11 (d, *J* = 8.6 Hz, 1H).

### Step 2

3-Amino-6-[1-(*tert*-butoxycarbonyl)-5-(piperazin-1-ylmethyl) indol-2-yl]phthalimide (26.0 mg, 0.045 mmol) was dissolved in 4 mol/L hydrogen chloride-ethyl acetate solution (2 mL), followed by stirring at 50°C for 9 hours The reaction mixture was cooled to room temperature, then the precipitated solid was collected by filtration, washed with ethyl acetate and then dried under reduced pressure to obtain Compound 5 (21.0 mg, yield 100%).
¹H-NMR (DMSO-*d*₆)δ(ppm): 3.15-3.70 (m, 8H), 4.44 (s, 2H), 6.76 (s, 2H), 7.09 (d, *J* = 8.8 Hz, 1H), 7.11 (s, 1H), 7.31 (d, *J* = 8.4 Hz, 1H), 7.55 (d, *J* = 8.4 Hz, 1H), 7.76 (br s, 1H), 8.03 (d, *J* = 8.8 Hz, 1H), 9.30 (br s, 1H), 9.40 (br s, 1H), 11.33 (s, 1H), 11.45 (br s, 1H), 12.20 (br s, 1H).

### Reference Example 2-4:

### 3-Amino-6-{1H-5-[4-(2-hydroxyethyl)piperazin-1-ylmethyl]indol-2-yl}phthalimide dihydrochloride (Compound 6)

### Step 1

In a similar manner to Step 2 of Reference Example 2-1, 3-amino-6-bromophthalimide (83.0 mg, 0.344 mmol) was dissolved in acetonitrile (5 mL), and the solution was treated with Compound BA (150 mg, 0.519 mmol), palladium acetate (6.2 mg, 0.028 mmol), tri(o-tolyl)phosphine (17 mg, 0.055 mmol) and triethylamine (0.480 mL, 3.44 mmol), followed by purification by flash column chromatography (hexane/ethyl acetate=4/1, 3/2, 1/1, 2/3) to obtain 3-amino-6-[1-(*tert*-butoxycarbonyl)-5-formylindol-2-yl]phthalimid e (74.1 mg, yield 53%).
ESI-MS *m*/*z*: 404 [M-H]⁻; ¹H-NMR (CDCl₃)δ(ppm): 1.44 (s, 9H), 5.41 (br s, 2H), 6.69 (s, 1H), 6.93 (d, *J* = 8.4 Hz, 1H), 7.41 (br s, 1H), 7.47 (d, *J* = 8.4 Hz, 1H), 7.87 (dd, *J* = 1.7, 8.8 Hz, 1H), 8.09 (d, *J* = 1.7 Hz, 1H), 8.32 (d, *J* = 8.8 Hz, 1H), 10.07 (s, 1H).

### Step 2

3-Amino-6-[1-(*tert*-butoxycarbonyl)-5-formylindol-2-yl]phtha limide (45.0 mg, 0.111 mmol) was dissolved in acetonitrile (2 mL), and the solution was added with 1- (2-hydroxyethyl)piperazine (57 mg, 0.440 mmol), acetic acid (0.508 mL, 8.87 mmol) and sodium triacetoxyborohydride (118 mg, 0.557 mmol), followed by stirring at room temperature for 2.5 hours. The reaction mixture was added with water and sodium carbonate, extracted with ethyl acetate. The organic layer was washed with saturated brine and dried over anhydrous sodium sulfate. The solvent was evaporated under reduced pressure and the residue was purified by flash column chromatography (hexane/ethyl acetate=4/1, 1/1) to obtain 3-amino-6-{1-(*tert*-butoxycarbonyl)-5-[4-(2-hydroxyethyl)piperazi n-1-ylmethyl]indol-2-yl}phthalimide (22.2 mg, yield 38%).
ESI-MS m/z: 520 [M+H]⁺; ¹H-NMR (CDCl₃)δ(ppm): 1.43 (s, 9H), 2.48-2.65 (m, 10H), 3.59-3.68 (m, 4H), 5.38 (s, 2H), 6.54 (s, 2H), 6.89 (d, *J* = 8.4 Hz, 1H), 7.29 (dd, *J* = 1.5, 8.4 Hz, 1H), 7.43 (d, *J* = 8.4 Hz, 1H), 7.48 (br s, 1H), 8.11 (d, *J* = 8.4 Hz, 1H).

### Step 3

In a similar manner to Step 2 of Reference Example 2-3, 3-amino-6-{1-(*tert*-butoxycarbonyl)-5-[4-(2-hydroxyethyl)piperazi n-1-ylmethyl]indol-2-yl}phthalimide (22.0 mg, 0.043 mmol) was treated with 4 mol/L hydrogen chloride-ethyl acetate solution (2 mL). Then, the obtained solid was collected by filtration, washed with ethyl acetate and dried under reduced pressure to obtain Compound 6 (21.0 mg, yield 100%).
¹H-NMR (DMSO-*d*₆)δ(ppm): 3.15-3.60 (m, 10H), 3.64-3.85 (m, 4H), 4.45 (br s, 2H), 7.09 (d, *J* = 9.0 Hz, 1H), 7.12 (m, 1H), 7.34 (d, *J* = 8.4 Hz, 1H), 7.55 (d, *J* = 8.4 Hz, 1H), 7.79 (s, 1H), 8.03 (d, *J* = 9.0 Hz, 1H), 11.02 (br s, 1H), 11.34 (s, 1H), 11.72 (br s, 1H), 12.22 (s, 1H).

### Reference Example 2-5:

### 3-Amino-4-phenyl-6-[1H-5-(piperazin-1-ylcarbonyl)indol-2-yl]phth alimide hydrochloride (Compound 8)

### Step 1

In a similar manner to Step 2 of Reference Example 2-1, 3-amino-4-phenyl-6-bromophthalimide (100 mg, 0.315 mmol) was dissolved in acetonitrile (7 mL), and the solution was treated with Compound BB (298 mg, 0.630 mmol), palladium acetate (5.7 mg, 0.025 mmol), tri(o-tolyl)phosphine (15 mg, 0.050 mmol) and triethylamine (0.439 mL, 3.15 mmol), followed by purification by preparative thin-layer chromatography (chloroform/acetonitrile=7/1, hexane/ethyl acetate=1/1) to obtain 3-amino-4-phenyl-6-{1-(*tert*-butoxycarbonyl)-5-[4-(*tert*-butoxycar bonyl)piperazin-1-ylcarbonyl]indol-2-yl}phthalimide (29 mg).

### Step 2

3-Amino-4-phenyl-6-{1-(*tert*-butoxycarbonyl)-5-[4-(*tert*-buto xycarbonyl)piperazin-1-ylcarbonyl]indol-2-yl}phthalimide (29.0mg) was dissolved in methanol (2.3 mL), and the solution was added with 10% hydrogen chloride-methanol solution (2.3 mL), followed by stirring at 70°C for 1.3 hours. The reaction mixture was cooled to room temperature. Then, the obtained solid was collected by filtration, washed with methanol and then dried under reduced pressure to obtain Compound 8 (12 mg, yield 7.4%, 2 Steps).
¹H-NMR (DMSO-*d*₆)δ(ppm): 3.16 (br s, 4H), 3.74 (br s, 4H), 6.28 (br s, 2H), 7.22 (dd, *J* = 1.6, 8.3 Hz, 1H), 7.30 (s, 1H), 7.47-7.57 (m, 6H), 7.68 (s, 1H), 7.90 (s, 1H), 9.04 (br s, 2H), 11.42 (s, 1H), 12.13 (s, 1H).

### Reference Example 2-6:

### 3-Amino-4-(thiophen-2-yl)-6-[1H-5-(piperazin-1-ylcarbonyl)indol-2-yl]phthalimide (Compound 10)

### Step 1

3-Amino-4-iodo-6-bromophthalimide (100 mg, 0.273 mmol) was dissolved in THF (5 mL), and the solution was added with 2-(tributylstannyl)thiophene (0.173 mL, 0.546 mmol) and bis(triphenylphosphine)dichloropalladium (15 mg, 0.022 mmol), followed by stirring under reflux for 12.5 hours under argon atmosphere. The reaction mixture was added with 10% aqueous ammonium fluoride solution, and extracted with ethyl acetate. The organic layer was washed with saturated brine and dried over sodium sulfate. The solvent was evaporated under reduced pressure. The residue was purified by slurry using hexane, and then purified by preparative thin-layer chromatography (chloroform/acetonitrile=30/1) to obtain 3-amino-4-(thiophen-2-yl)-6-bromophthalimide (77 mg, yield 87%).
APCI-MS *m*/*z*: 321 [M-H]⁻; ¹H-NMR (DMSO-*d*₆)δ(ppm): 6.31 (s, 2H), 7.23 (dd, *J* = 3.6, 5.3 Hz, 1H), 7.43 (dd, *J* = 1.2, 3.6 Hz, 1H), 7.59 (s, 1H), 7.75 (dd, *J* = 1.2, 5.1 Hz, 1H), 11.26 (s, 1H).

### Step 2

In a similar manner to Step 2 of Reference Example 2-1, 3-amino-4-(thiophen-2-yl)-6-bromophthalimide (100 mg, 0.309 mmol) was dissolved in acetonitrile (7 mL), and the solution was treated with Compound BB (293 mg, 0.618 mmol), palladium acetate (5.5 mg, 0.025 mmol), tri(o-tolyl)phosphine (15 mg, 0.049 mmol) and triethylamine (0.431 mL, 3.09 mmol), followed by purification by preparative thin-layer chromatography (chloroform/acetonitrile=7/1) to obtain 3-amino-4-(thiophen-2-yl)-6-{1-(*tert*-butoxycarbonyl)-5-[4-(*tert-*butoxycarbonyl)piperazin-1-ylcarbonyl]indol-2-yl}phthalimide (152 mg, yield 73%).
ESI-MS *m*/*z*: 672 [M+H]⁺; ¹H-NMR (CDCl₃)δ(ppm): 1.48 (s, 18H), 3.47 (br s, 4H), 3.60 (br s, 4H), 5.86 (s, 1H), 7.19 (dd, *J* = 3.6, 5.1 Hz, 1H), 7.33 (d, *J* = 3.6 Hz, 1H), 7.37 (dd, *J* = 1.6, 8.7 Hz, 1H), 7.47 (m, 2H), 7.58 (s, 1H), 7.69 (s, 1H), 8.20 (d, *J* = 8.7 Hz, 1H).

### Step 3

In a similar manner to Step 3 of Reference Example 2-1, 3-amino-4-(thiophen-2-yl)-6-{1-(*tert*-butoxycarbonyl)-5-[4-(*tert-*butoxycarbonyl)piperazin-1-ylcarbonyl]indol-2-yl}phthalimide (146 mg, 0.218 mmol) was dissolved in methanol (4.4 mL), and the solution was treated with 10% hydrogen chloride-methanol solution (4.4 mL), followed by purification by preparative thin-layer chromatography (chloroform/methanol=5/1) to obtain Compound 10 (46 mg, yield 45%). APCI-MS *m*/*z*: 472 [M+H]⁺; ¹H-NMR (DMSO-*d*₆)δ(ppm): 2.69 (br s, 4H), 3.44 (br s, 4H), 6.48 (s, 2H), 7.14 (dd, *J* = 1.6, 8.5 Hz, 1H), 7.23 (s, 1H), 7.28 (dd, *J* = 3.5, 5.2 Hz, 1H), 7.48-7.51 (m, 2H), 7.58 (s, 1H), 7.78 (dd, *J* = 1.1, 5.2 Hz, 1H), 8.01 (s, 1H), 12.10 (s, 1H).

### Reference Example 2-7:

### (E)-3-Amino-4-(2-methoxycarbonylvinyl)-6-[1H-5-(piperazin-1-ylca rbonyl)indol-2-yl]phthalimide (Compound 15)

### Step 1

3-Amino-4-iodo-6-bromophthalimide (500 mg, 1.36 mmol) was dissolved in acetonitrile (25 mL), and the solution was added with methyl acrylate (0.245 mL, 2.72 mmol), palladium acetate (24 mg, 0.11 mmol) and triethylamine (1.90 mL, 13.6 mmol), followed by stirring under reflux for 5 hours under argon atmosphere. The mixture was added with methyl acrylate (0.122 mL, 1.36 mmol), palladium acetate (24 mg, 0.11 mmol) and triethylamine (1.90 mL, 13.6 mmol) and further stirred for 142 hours. The mixture was added with methyl acrylate (0.245 mL, 2.72 mmol), palladium acetate (24 mg, 0.11 mmol) and triethylamine (0.95 mL, 6.8 mmol) and further stirred for 20 hours. The reaction mixture was added with water, extracted with ethyl acetate. The organic layer was washed with saturated brine and dried over sodium sulfate. The solvent was evaporated under reduced pressure and the residue was purified by slurry using chloroform to obtain (E)-3-amino-4-(2-methoxycarbonylvinyl)-6-bromophthalimide (313 mg, yield 71%).
APCI-MS *m*/*z*: 323 [M-H]⁻; ¹H-NMR (DMSO-*d*₆)δ(ppm): 3.73 (s, 3H), 6.71 (d, *J* = 15.8 Hz, 1H), 6.84 (s, 2H), 7.86 (d, *J* = 15.8 Hz, 1H), 8.01 (s, 1H), 11.24 (s, 1H).

### Step 2

In a similar manner to Step 2 of Reference Example 2-1, (E)-3-amino-4-(2-methoxycarbonylvinyl)-6-bromophthalimide (100 mg, 0.308 mmol) was dissolved in acetonitrile (7 mL), and the solution was treated with Compound BB (292 mg, 0. 616 mmol), palladium acetate (5.5 mg, 0.025 mmol), tri(o-tolyl)phosphine (15 mg, 0.049 mmol) and triethylamine (0.429 mL, 3.08 mmol), followed by purification by preparative thin-layer chromatography (chloroform/acetonitrile=6/1) to obtain (E)-3-amino-4-(2-methoxycarbonylvinyl)-6-{1-(*tert*-butoxycarbonyl )-5-[4-(*tert*-butoxycarbonyl)piperazin-1-ylcarbonyl]indol-2-yl}ph thalimide (102 mg, yield 49%).
ESI-MS *m*/*z*: 674 [M+H]⁺; ¹H-NMR (CDCl₃)δ(ppm): 1.47 (s, 9H), 1.48 (s, 9H), 3.16-3.49 (m, 8H), 3.84 (s, 3H), 5.78 (s, 2H), 6.50 (d, *J* = 15.5 Hz, 1H), 6.64 (s, 1H), 7.44 (m, 1H), 7.55 (s, 1H), 7.67 (d, *J* = 9.2 Hz, 1H), 7.69 (s, 1H), 7.79 (d, *J* = 15.9 Hz, 1H), 8.19 (d, *J* = 8.6 Hz, 1H).

### Step 3

In a similar manner to Step 3 of Reference Example 2-1, (E)-3-amino-4-(2-methoxycarbonylvinyl)-6-{1-(*tert*-butoxycarbonyl )-5-[4-(*tert*-butoxycarbonyl)piperazin-1-ylcarbonyl]indol-2-yl}ph thalimide (97.7 mg, 0.145 mmol) was dissolved in methanol (2.9 mL), and the solution was treated with 10% hydrogen chloride-methanol solution (2.9mL), followed by purification by preparative thin-layer chromatography (chloroform/methanol/7 mol/L ammonia-methanol solution=10/0.8/0.2) to obtain Compound 15 (18 mg, yield 26%).
¹H-NMR (DMSO-*d*₆)δ(ppm): 2.70 (br s, 4H), 3.45 (br s, 4H), 3.77 (s, 3H), 6.92 (d, *J* = 15.5 Hz, 1H), 7.01 (s, 2H), 7.14 (d, *J* = 8.4 Hz, 1H), 7.40 (s, 1H), 7.50 (d, *J* = 8.4 Hz, 1H), 7.58 (s, 1H), 7.97 (d, *J* = 15.5 Hz, 1H), 8.36 (s, 1H), 12.06 (s, 1H).

### Reference Example 2-8:

### 4-Chloro-7-(1H-indol-2-yl)isoindolinone (Compound 16)

### Step 1

3-Chlorobenzoylchloride (10.0 g, 57.1 mmol) was dissolved in dichloromethane (200 mL), and the solution was added with cumylamine (9.04 mL, 62.9 mmol), triethylamine (12.0 mL, 85.7 mmol) and DMAP (698 mg, 5.71 mmol), followed by stirring at room temperature for 3 hours. The reaction mixture was added with water, and extracted with ethyl acetate. The organic layer was washed with saturated brine and dried over anhydrous sodium sulfate. The solvent was evaporated under reduced pressure and the residue was purified by slurry using diisopropyl ether to obtain 3-chloro-N-(1-methyl-1-phenylethyl)benzamide (15.0 g, yield 96%). APCI-MS *m*/*z*: 274 [M+H]⁺; ¹H-NMR (CDCl₃)δ(ppm): 1.83 (s, 6H), 6.35 (br s, 1H), 7.24 (m, 1H), 7.28-7.38 (m, 3H), 7.44-7.47 (m, 3H), 7.62 (d, *J* = 7.6 Hz, 1H), 7.73 (br s, 1H).

### Step 2

3-Chloro-N-(1-methyl-1-phenylethyl)benzamide(7.00 g, 25.6 mmol) was dissolved in THF (280 mL), and the solution was added with TMEDA (12.4 mL, 81.9 mmol), then added with sec-butyllithium-hexane solution (0.99 mol/L, 82.7 mL, 81.9 mmol) by drops at -78 °C for 40 minutes under argon atmosphere, followed by stirring at the same temperature for 2.5 hours. Then, the mixture was added with DMF (4.36 mL, 56.3 mmol) and warmed from -78 °C to room temperature over 2 hours. The reaction mixture was added with water, and extracted with ethyl acetate. The organic layer was washed with saturated brine and dried over anhydrous sodium sulfate. The solvent was evaporated under reduced pressure and the residue was purified by slurry using diisopropyl ether to obtain 4-chloro-3-hydroxy-2-(1-methyl-1-phenylethyl)isoindolinone (6.86 g, yield 89%).
APCI-MS *m*/*z*: 302 [M+H]⁺; ¹H-NMR (CDCl₃)δ(ppm): 1.94 (s, 3H), 1.98 (s, 3H), 6.26 (br s, 1H), 7.21-7.35 (m, 4H), 7.39-7.51 (m, 4H), 7.58 (d, *J* = 7.3 Hz, 1H).

### Step 3

4-Chloro-3-hydroxy-2-(1-methyl-1-phenylethyl)isoindolinone (6.86 g, 22.7 mmol) was dissolved in THF (274 mL), and the solution was added with TMEDA (7.55 mL, 50.0 mmol), then added with sec-butyllithium-hexane solution (0.99 mol/L, 50.5 mL, 50.0 mmol) by drops at -78 °C over 20 minutes under argon atmosphere, followed by stirring at the same temperature for 2 hours. Then, the mixture was added with iodine (6.92 g, 27.3 mmol) and warmed from -78 °C to room temperature for 2.5 hours. The reaction mixture was added with 10% aqueous sodium thiosulfate solution and extracted with ethyl acetate. The organic layer was washed with saturated brine and dried over anhydrous sodium sulfate. The solvent was evaporated under reduced pressure and the residue was purified by flash column chromatography (chloroform/methanol =100/0, 85/15) to obtain 4-chloro-3-hydroxy-7-iodo-2-(1-methyl-1-phenylethyl)isoindolinon e (8.22 g, yield 85%).
APCI-MS *m*/*z*: 428 [M+H]⁺; ¹H-NMR (CDCl₃)δ(ppm) : 1.96 (s, 3H), 2.00 (s, 3H), 2.44 (d, *J* = 7.9 Hz, 1H), 6.69 (d, *J* = 8.1 Hz, 1H), 7.20 (d, *J* = 8.4 Hz, 1H), 7.23-7.28 (m, 1H), 7.32-7.37 (m, 2H), 7.44-7.47 (m, 2H), 7.85 (d, *J* = 8.3 Hz, 1H).

### Step 4

4-Chloro-3-hydroxy-7-iodo-2-(1-methyl-1-phenylethyl)isoindo linone (6.94 g, 16.2 mmol) was dissolved in nitromethane (280 mL), and the solution was added with trifluoroacetic acid (17.7 mL, 230 mmol) and triethylsilane (7.35 mL, 46.1 mmol), followed by stirring at room temperature for 23 hours. The reaction mixture was added with water and extracted with ethyl acetate. The organic layer was washed with saturated brine and dried over anhydrous sodium sulfate. The solvent was evaporated under reduced pressure and the residue was purified by slurry using diisopropyl ether to obtain 4-chloro-7-iodoisoindolinone (4.73 g, yield 99%).
APCI-MS *m*/*z*: 294 [M+H]⁺; ¹H-NMR (DMSO-*d*₆)δ(ppm): 4.28 (s, 2H), 7.41 (d, *J* = 8.2 Hz, 1H), 7.91 (d, *J* = 8.1 Hz, 1H), 8.96 (br s, 1H).

### Step 5

In a similar manner to Step 2 of Reference Example 2-1, 4-chloro-7-iodoisoindolinone (100 mg, 0.341 mmol) was dissolved in acetonitrile (7 mL), and the solution was treated with 1-(*tert*-butoxycarbonyl)indol-2-boronic acid (178 mg, 0.682 mmol), palladium acetate (6.1 mg, 0.027 mmol), tri(o-tolyl)phosphine (17 mg, 0.055 mmol) and triethylamine (0.475 mL, 3.41 mmol), followed by purification by preparative thin-layer chromatography (chloroform/methanol =30/1) to obtain 4-chloro-7-(1-(*tert*-butoxycarbonyl)indol-2-yl)isoindolinone (57 mg, yield 44%).
APCI-MS *m*/*z*: 383 [M+H]⁺; ¹H-NMR (CDCl₃)δ(ppm) : 1.38 (s, 9H), 4.42 (s, 2H), 6.59 (br s, 2H), 7.23 (m, 1H), 7.34 (dd, *J* = 7.1, 8.1 Hz, 1H), 7.44 (d, *J* = 8.1 Hz, 1H), 7.56 (d, *J* = 7.9 Hz, 4H), 8.22 (d, *J* = 8.4 Hz, 1H).

### Step 6

In a similar manner to Step 3 of Reference Example 2-1, 4-chloro-7-(1-(*tert*-butoxycarbonyl)indol-2-yl)isoindolinone (39.3 mg, 0.103 mmol) was dissolved in methanol (3.9 mL), and the solution was treated with 10% hydrogen chloride-methanol solution (3.9 mL), followed by purification by preparative thin-layer chromatography (chloroform/acetone=12/1) to obtain Compound 16 (24 mg, yield 83%). APCI-MS *m*/*z*: 281 [M-H]⁻; ¹H-NMR (DMSO-*d*₆)δ(ppm): 4.50 (s, 2H), 7.03 (dd, *J* = 7.1, 7.9 Hz, 1H), 7.14 (dd, *J* = 7.4, 7.8 Hz, 1H), 7.27 (s, 1H), 7.47 (d, *J* = 8.1 Hz, 1H), 7.58 (d, *J* = 7.8 Hz, 1H), 7.73 (d, *J* = 8.3 Hz, 1H), 8.21 (d, *J* = 8.6 Hz, 1H), 9.52 (s, 1H), 13.72 (s, 1H).

### Reference Example 2-9:

### 7-(1H-5-Carboxyindol-2-yl)-4-chloroisoindolinone (Compound 19)

### Step 1

4-Chloro-7-iodoisoindolinone (1.61 g, 5.49 mmol) was dissolved in DMF (50 mL), and the solution was added with Compound BG (2.51 g, 8.23 mmol), palladium acetate (99 mg, 0.441 mmol) and triethylamine (7.65 mL, 54.9 mmol), followed by stirring at 60 °C for 4 hours under argon atmosphere. The reaction mixture was added with water, and extracted with ethyl acetate. The organic layer was washed with saturated brine and dried over anhydrous sodium sulfate. The solvent was evaporated under reduced pressure and the residue was dissolved in ethyl acetate. The solution was added with diisopropyl ether and stirred under ice-cooling for 1 hour. The precipitated solid was collected by filtration, washed with diisopropyl ether and then dried under reduced pressure to obtain 7-[1-(*tert*-butoxycarbonyl)-5-carboxyindol-2-yl]-4-chloroisoindol inone (1.72 g, yield 74%).
ESI-MS *m*/*z*: 425 [M-H]⁻; ¹H-NMR (DMSO-*d*₆)δ(ppm): 1.18 (s, 9H), 4.41 (s, 2H), 6.81 (s, 1H), 7.52 (d, *J* = 8.0 Hz, 1H), 7.74 (d, *J* = 8.0 Hz, 1H), 7.93 (dd, *J* = 1.7, 8.8 Hz, 1H), 8.21 (d, *J* = 8.8 Hz, 1H), 8.24 (d, *J* = 1.7 Hz, 1H), 8.88 (s, 1H).

### Step 2

In a similar manner to Step 2 of Reference Example 2-3, 7-[1-(*tert*-butoxycarbonyl)-5-carboxyindol-2-yl]-4-chloroisoindol inone (1.71 g, 4.01 mmol) was treated with 4 mol/L hydrogen chloride-ethyl acetate(70 mL). Then, the obtained solid was collected by filtration, washed with hexane and then dried under reduced pressure to obtain Compound 19 (1.13 g, yield 86%). APCI-MS m/z: 327 [M+H]⁺; ¹H-NMR (DMSO-*d*₆)δ(ppm) : 4.52 (s, 2H), 7.43 (s, 1H), 7.55 (d, *J* = 8.8 Hz, 1H), 7.75 (dd, *J* = 1.5, 8.8 Hz, 1H), 7.77 (d, *J* = 8.4 Hz, 1H), 8.24 (d, *J* = 8.4 Hz, 1H), 8.27 (br s, 1H), 9.59 (s, 1H), 12.49 (br s, 1H), 14.00 (s, 1H).

### Reference Example 2-10:

### 4-Chloro-7-[1H-5-(4-acetylpiperazin-1-ylcarbonyl)indol-2-yl]isoi ndolinone (Compound 20)

Compound 19 (30.0 mg, 0.0918 mmol) was dissolved in DMF (1 mL), and the solution was added with EDCI (35 mg, 0.18 mmol), HOBT monohydrate (12 mg, 0.089 mmol) and 1-acetylpiperazine (47 mg, 0.37 mmol), followed by stirring at 60 °C for 2 hours. The reaction mixture was added with water and the precipitated solid was collected by filtration and washed with water, followed by drying under reduced pressure to obtain Compound 20 (23.2 mg, yield 58%).
¹H-NMR (DMSO-*d*₆)δ(ppm): 2.02 (s, 3H), 3.45-3.60 (m, 8H), 4.51 (s, 2H), 7.20 (d, *J* = 8.4 Hz, 1H), 7.34 (s, 1H), 7.54 (d, *J* = 8.4 Hz, 1H), 7.68 (br s, 1H), 7.74 (d, *J* = 8.4 Hz, 1H), 8.22 (d, *J* = 8.4 Hz, 1H), 9.55 (br s, 1H), 13.91 (br s, 1H).

### Reference Example 2-11:

### 4-Chloro-7-[1H-5-(piperidinomethyl)indol-2-yl]isoindolinone hydrochloride (Compound 29)

### Step 1

In a similar manner to Step 2 of Reference Example 2-4, 4-chloro-7-[1-(*tert*-butoxycarbonyl)-5-formylindol-2-yl]isoindoli none (36.2 mg, 0.0881 mmol) was dissolved in acetonitrile (2 mL), and the solution was treated with piperidine (0.035 mL, 0.35 mmol), acetic acid (0.101 mL, 1.76 mmol) and sodium triacetoxyborohydride (93 mg, 0.44 mmol), followed by purification by preparative thin-layer chromatography (hexane/ethyl acetate=1/2) to obtain 4-chloro-7-[1-(*tert*-butoxycarbonyl)-5-(piperidinomethyl)indol-2-yl]isoindolinone (31.2 mg, yield 74%).
ESI-MS *m*/*z*: 480 [M+H]⁺; ¹H-NMR (CDCl₃)δ(ppm): 1.34 (s, 9H), 1.40-1.52 (m, 2H), 1.52-1.62 (m, 4H), 2.34-2.50 (m, 4H), 3.59 (s, 2H), 4.38 (s, 2H), 6.54 (s, 1H), 7.30 (dd, *J* = 1.5, 8.8 Hz, 1H), 7.42 (d, *J* = 8.1 Hz, 1H), 7.49 (d, *J* = 1.5 Hz, 1H), 7.54 (d, *J* = 8.1 Hz, 1H), 7.64 (s, 1H), 8.15 (d, *J* = 8.8 Hz, 1H).

### Step 2

In a similar manner to Step 2 of Reference Example 2-3, 4-chloro-7-[1-(*tert*-butoxycarbonyl)-5-(piperidinomethyl)indol-2-yl]isoindolinone (30.0 mg, 0.0629 mmol) was treated with 4 mol/L hydrogen chloride-ethyl acetate solution (2 mL). Then, the mixture was added with diisopropyl ether. The precipitated solid was collected by filtration, washed with diisopropyl ether and then dried under reduced pressure to obtain Compound 29 (11.2 mg, yield 43%). mp >295 °C; ¹H-NMR (DMSO-*d*₆)δ(ppm): 1.45 (m, 1H), 1.59-1.74 (m, 3H), 1.75-1.89 (m, 2H), 2.40-2.58 (m, 2H), 2.80-2.95 (m, 2H), 4.33 (d, *J* = 4.6 Hz, 2H), 4.52 (s, 2H), 7.28 (dd, *J* = 1.0, 5.4 Hz, 1H), 7.36 (d, *J* = 1.0 Hz, 1H), 7.58 (d, *J* = 8.4 Hz, 1H), 7.75 (s, 1H), 7.76 (d, *J* = 8.6 Hz, 1H), 8.25 (d, *J* = 8.6 Hz, 1H), 9.44 (br s, 1H), 9.57 (s, 1H), 13.90 (s, 1H).

### Reference Example 2-12:

### 4-Chloro-7-[1H-5-(ethoxycarbonylmethylaminomethyl)indol-2-yl]iso indolinone hydrochloride (Compound 34)

### Step 1

In a similar manner to Step 2 of Reference Example 2-4, 4-chloro-7-[1-(*tert*-butoxycarbonyl)-5-formylindol-2-yl]isoindoli none (30.0 mg, 0.0730 mmol) was dissolved in acetonitrile (2 mL), and the solution was treated with glycine ethylester hydrochloride (41.0 mg, 0.294 mmol), triethylamine (0.081 mL, 0.58 mmol), acetic acid (0.084 mL, 1. 5 mmol) and sodium triacetoxyborohydride (77.0 mg, 0.360 mmol). The reaction mixture was added with water and sodium hydrogen carbonate, followed by extracting with ethyl acetate. The organic layer was washed with saturated brine and dried over anhydrous sodium sulfate. The solvent was evaporated under reduced pressure to obtain 4-chloro-7-[1-(*tert*-butoxycarbonyl)-5-(ethoxycarbonylmethylamino methyl)indol-2-yl]isoindolinone (39.2 mg).

### Step 2

In a similar manner to Step 2 of Reference Example 2-3, 4-chloro-7-[1-(*tert*-butoxycarbonyl)-5-(ethoxycarbonylmethylamino methyl)indol-2-yl]isoindolinone (39.2 mg) was treated with 4 mol/L hydrogen chloride-ethyl acetate solution (2 mL). The precipitated solid was collected by filtration, washed with ethyl acetate and then dried under reduced pressure to obtain Compound 34 (27.9 mg, yield 88%, 2 Steps).
¹H-NMR (DMSO-*d*₆)δ(ppm) : 1.22 (t, *J* = 7.1 Hz, 3H), 3.95 (d, *J* = 5.8 Hz, 2H), 4.20 (q, *J* = 7.1 Hz, 2H), 4.24 (s, 2H), 4.52 (s, 2H), 7.26 (dd, *J* = 1.4, 8.3 Hz, 1H), 7.35 (d, *J* = 1.4 Hz, 1H), 7.56 (d, *J* = 8.3 Hz, 1H), 7.72 (s, 1H), 7.76 (d, *J* = 8.6 Hz, 1H), 8.25 (d, *J* = 8.6 Hz, 1H), 9.38 (br s, 2H), 9.57 (s, 1H), 13.89 (s, 1H).

### Reference Example 2-13:

### 4-Chloro-7-[1H-5-(carboxymethylaminomethyl)indol-2-yl]isoindolin one hydrochloride (Compound 35)

### Step 1

Compound 34 (20.0 mg, 46.0 mmol) was dissolved in acetonitrile (1 mL), and the solution was treated with di-*tert*-butyldicarbonate (0.012 mL, 0.052 mmol) and triethylamine (0.026 mL, 0.19 mmol), followed by stirring at room temperature for 1 hour. The reaction mixture was added with water, and extracted with ethyl acetate. The organic layer was washed with saturated brine and dried over anhydrous sodium sulfate. The solvent was evaporated under reduced pressure to obtain 4-chloro-7-[1-(*tert*-butoxycarbonyl)-5-(ethoxycarbonylmethylamino methyl)indol-2-yl]isoindolinone (43.4 mg).

### Step 2

4-Chloro-7-[1-(*tert*-butoxycarbonyl)-5-(ethoxycarbonylmethyl aminomethyl) indol-2-yl] isoindolinone (43.4 mg) was dissolved in DMF (2 mL), and the solution was added with 4 mol/L aqueous lithium hydroxide solution (1 mL), followed by stirring at room temperature for 1 hour. The reaction mixture was added with 1 mol/L hydrochloric acid, and extracted with ethyl acetate. The organic layer was washed with saturated brine and dried over anhydrous sodium sulfate. The solvent was evaporated under reduced pressure to obtain 4-chloro-7-[1-(*tert*-butoxycarbonyl)-5-(carboxymethylaminomethyl) indol-2-yl]isoindolinone (25.0 mg).

### Step 3

In a similar manner to Step 2 of Reference Example 2-3, 4-chloro-7-[1-(*tert*-butoxycarbonyl)-5-(carboxymethylaminomethyl) indol-2-yl] isoindolinone (25.0 mg) was treated with 4 mol/L hydrogen chloride-ethyl acetate solution (1 mL). The precipitated solid was collected by filtration, washed with ethyl acetate and then dried under reduced pressure to obtain Compound 35 (16.8 mg, yield 90%, 3 Steps).
¹H-NMR (DMSO-*d*₆)δ(ppm): 3.84 (br s, 2H), 4.23 (br s, 2H), 4.52 (s, 2H), 7.27 (dd, *J* = 1.3, 8.4 Hz, 1H), 7.35 (br s, 1H), 7.56 (d, *J* = 8.4 Hz, 1H), 7.73 (s, 1H), 7.76 (d, *J* = 8.4 Hz, 1H), 8.25 (d, *J* = 8.4 Hz, 1H), 9.31 (br s, 2H), 9.58 (s, 1H), 13.70 (br s, 1H), 13.89 (s, 1H).

### Reference Example 2-14:

### 4-Chloro-7-[1H-5-(benzylaminomethyl)indol-2-yl]isoindolinone hydrochloride (Compound 56)

### Step 1

4-Chloro-7-[1-(*tert*-butoxycarbonyl)-5-formylindol-2-yl]isoi ndolinone (20.0 mg, 0.0487 mmol) was dissolved in dichloromethane (0.5 mL), and the solution was added with benzylamine (0.11 mL, 0.10 mmol) and sodium triacetoxyborohydride (32 mg, 0.15 mmol) followed by stirring at room temperature for 16 hours. The reaction mixture was added with 3 mol/L aqueous sodium hydroxide solution, and extracted with dichloromethane. The organic layer was dried over anhydrous sodium sulfate. The mixture was added with aldehyde resin and stirred at room temperature for 16 hours. The resin was filtered off and the solvent of the filtrate was evaporated under reduced pressure. The residue was dissolved in chloroform, and the solution was filtered through a column filled with SCX (positive-ion exchange resin). The resulting SCX was washed with 7 mol/L ammonia-methanol solution and the solvent of the filtrate was evaporated under reduced pressure to obtain 4-chloro-7-[1-(tert-butoxycarbonyl)-5-(benzylaminomethyl)indol-2 -yl]isoindolinone.

### Step 2

4-Chloro-7-[1-(tert-butoxycarbonyl)-5-(benzylaminomethyl)in dol-2-yl]isoindolinone was dissolved in 10% hydrogen chloride-methanol solution (0.5 mL) followed by stirring at 55 °C for 12 hours. The solvent of the reaction mixture was evaporated under reduced pressure. The residue was added with 3 mol/L aqueous sodium hydroxide solution followed by stirring for 30 minutes. The obtained solid was collected by filtration and washed with water, followed by drying under reduced pressure to obtain Compound 56 (9.90 mg, yield 49%, 2 steps).
¹H-NMR (DMSO-*d*₆)δ(ppm): 4.17 (br s, 2H), 4.23 (br s, 2H), 4.52 (s, 2H), 7.29 (dd, J = 1.2, 8.4 Hz, 1H), 7.35 (d, J = 1.2 Hz, 1H), 7.40-7.62 (m, 6H), 7.56 (d, J = 8.6 Hz, 1H), 7.74 (s, 1H), 7.75 (d, J = 8.6 Hz, 1H), 8.25 (d, J = 8.4 Hz, 1H), 9.35 (br s, 1H), 9.56 (s, 1H), 13.89 (s, 1H).

### Reference Example 2-15:

### 4-Phenoxy-7-[1H-5-(piperazin-1-ylcarbonyl)indol-2-yl]isoindolino ne (Compound 137)

### Step 1

3-Phenoxybenzoic acid (3.00 g, 14.0 mmol) was dissolved in DMF (45 mL), and the solution was added with EDCI (4.03 g, 21.0 mmol) and HOBT monohydrate (1.07 g, 7.00 mmol) under ice-cooling, followed by stirring at the same temperature for 5 minutes. Then, the mixture was added with cumylamine (4.03 mL, 28.0 mmol) and stirred at room temperature for 2 hours. The reaction mixture was added with water and extracted with ethyl acetate. The organic layer was washed with saturated brine and dried over anhydrous sodium sulfate. The solvent was evaporated under reduced pressure. The residue was purified by slurry using hexane to obtain 3-phenoxy-N-(1-methyl-1-phenylethyl)benzamide (4.51 g, yield 97%). APCI-MSm/z: 332 [M+H]⁺; ¹H-NMR (CDCl₃)δ(ppm): 1.81 (m, 6H), 6.36 (s, 1H), 7.01 (m, 2H), 7.12 (m, 2H), 7.21-7.46 (m, 10H).

### Step 2

In a similar manner to Step 2 of Reference Example 2-8, 3-phenoxy-N-(1-methyl-1-phenylethyl)benzamide (4.00 g, 12.1 mmol) was dissolved in THF (160 mL), and the solution was treated with TMEDA (5.80 mL, 38.7 mmol), sec-butyllithium-hexane solution (0.99 mol/L, 39.0 mL, 38.7 mmol) and DMF (2.10 mL, 26.6 mmol), followed by purification by slurry using diisopropylether to obtain 4-phenoxy-3-hydroxy-2-(1-methyl-1-phenylethyl)isoindolinone (3.92 g, yield 90%).
APCI-MS m/z: 360 [M+H]⁺; ¹H-NMR (CDCl₃)δ(ppm): 1.93 (s, 3H), 1.97 (s, 3H), 2.55 (d, J = 7.6 Hz, 1H), 6.31 (d, J = 7.6 Hz, 1H), 6.94-7.47 (m, 13H).

### Step 3

In a similar manner to Step 3 of Reference Example, 2-8, 4-phenoxy-3-hydroxy-2-(1-methyl-1-phenylethyl)isoindolinone (700 mg, 1.95 mmol) was dissolved in THF (28 mL), and the solution was -treated with TMEDA (0.94 mL, 6.2 mmol), sec-butyllithium-hexane solution (1.01 mol/L, 6.20 mL, 6.24 mmol) and iodine (594 mg, 2.34 mmol), followed by purification by flash column chromatography (chloroform/methanol=100/0, 99/1) to obtain 4-phenoxy-3-hydroxy-7-iodo-2-(1-methyl-1-phenylethyl)isoindolino ne (708 mg, yield 75%).
ESI-MS m/z: 486 [M+H]⁺; ¹H-NMR (CDCl₃)δ(ppm): 1.95 (s, 3H), 1.98 (s, 3H), 2.46 (d, J = 6.9 Hz, 1H), 6.16 (d, J = 6.9 Hz, 1H), 6.69 (d, J = 8.6 Hz, 1H), 7.06 (m, 2H), 7.17-7.48 (m, 8H), 7.77 (d, J = 8.6 Hz, 1H).

### Step 4

In a similar manner to Step 4 of Reference Example 2-8, 4-phenoxy-3-hydroxy-7-iodo-2-(1-methyl-1-phenylethyl)isoindolino ne (700 mg, 1.44 mmol) was dissolved in nitromethane (28 mL), and the solution was treated with trifluoroacetic acid (1.10 mL, 14.4 mmol) and triethylsilane (0.460 mL, 2.88 mmol), followed by purification by slurry using diisopropylether to obtain 4-phenoxy-7-iodoisoindolinone (369 mg, yield 73%). APCI-MS m/z: 352 [M+H]⁺; ¹H-NMR (CDCl₃)δ(ppm): 4.31 (s, 2H), 6.78 (d, J = 8.4 Hz, 1H), 7.01-7.05 (m, 3H), 7.18 (m, 1H), 7.35-7.41 (m, 2H), 7.81 (d, J = 8.6 Hz, 1H).

### Step 5

In a similar manner to Step 2 of Reference Example 2-1, 4-phenoxy-7-iodoisoindolinone (80.0 mg, 0.228 mmol) was dissolved in acetonitrile (5.6 mL), and the solution was treated with Compound BB (216 mg, 0.456 mmol), palladium acetate (4.1 mg, 0.018 mmol), tri(o-tolyl)phosphine (11 mg, 0.036 mmol) and triethylamine (0.318 mL, 2.28 mmol), followed by purification by preparative thin-layer chromatography (chloroform/acetonitrile=4/1) to obtain 4-phenoxy-7-[1-(tert-butoxycarbonyl)-5-(piperazin-1-ylcarbonyl)i ndol-2-yl]isoindolinone (81.1 mg, yield 54%).
ESI-MS m/z: 653 [M+H]⁺; ¹H-NMR (CDCl₃)δ(ppm): 1.41 (s, 9H), 1.51 (s, 9H), 3.50 (m, 8H), 4.44 (s, 2H), 6.37 (s, 1H), 6.61 (s, 1H), 7.08-7.47 (m, 8H), 7.65 (s, 1H), 8.30 (d, J = 8.6 Hz, 1H).

### Step 6

In a similar manner to Step 3 of Reference Example 2-1, 4-phenoxy-7-[1-(tert-butoxycarbonyl)-5-(piperazin-1-ylcarbonyl)i ndol-2-yl]isoindolinone (77.5 mg, 0.119 mmol) was dissolved in methanol (2.3 mL), and the solution was treated with 10% hydrogen chloride-methanol solution (2.3 mL), followed by purification by preparative thin-layer chromatography (chloroform/methanol=8/1) to obtain Compound 137 (30.7 mg, yield 57%).
¹H-NMR (DMSO-d₆)δ(ppm): 2.69 (m, 4H), 3.44 (m, 4H), 4.45 (s, 2H), 7.11-7.25 (m, 6H), 7.42-7.51 (m, 3H), 7.59 (s, 1H), 8.16 (d, J = 8.7 Hz, 1H), 9.41 (s, 1H), 13.86 (s, 1H).

### Reference Example 2-16:

### 4-Hydroxy-7-[1H-5-(piperidinomethyl)indol-2-yl]isoindolinone hydrochloride (Compound 139)

### Step 1

In a similar manner to Step 1 of Reference Example 2-15, 3-acetoxybenzoic acid (7.92 g, 44.0 mmol) was dissolved in DMF (45 mL), and the solution was treated with EDCI (12.7 g, 66.0 mmol), HOBT monohydrate (3.37 g, 22.0 mmol) and cumylamine (14.2 mL, 96.8 mmol), followed by purification by slurry using diisopropylether to obtain 3-acetoxy-N-(1-methyl-1-phenylethyl)benzamide (9.57 g, yield 73%).

### Step 2

3-Acetoxy-N-(1-methyl-1-phenylethyl)benzamide (9.57 g, 32.2 mmol) was dissolved in methanol (190 mL'), and the solution was added with 4 mol/L aqueous sodium hydroxide solution (32.2 mL), followed by stirring at room temperature for 20 minutes. The reaction mixture was added with 1 mol/L hydrochloric acid to adjust the pH to 7, and then extracted with ethyl acetate. The organic layer was washed with saturated brine and dried over anhydrous sodium sulfate. The solvent was evaporated under reduced pressure. The residue was dissolved in dichloromethane (190 mL), and the solution was added with diisopropylethylamine (16.8 mL, 96.6 mmol) and chloromethyl methyl ether (3.70 mL, 48.3 mmol) under ice-cooling. Then, the reaction mixture was warmed to room temperature and stirred for 2 hours. The mixture was added with diisopropylethylamine (5.60 mL, 32.2 mmol) and chloromethyl methyl ether (1.20 mL, 15.8 mmol), followed by further stirring for 2 hours. The reaction mixture was added with water and extracted with chloroform. The organic layer was washed with saturated brine and dried over anhydrous sodium sulfate. The solvent was evaporated under reduced pressure. The residue was purified by flash column chromatography (chloroform/methanol=100/0, 99/1) to obtain 3-methoxymethoxy-N-(1-methyl-1-phenylethyl)benzamide (8.09 g, yield 84%).
APCI-MS m/z: 298 [M+H]⁺; ¹H-NMR (CDCl₃)δ(ppm): 1.81 (s, 6H), 2.31 (s, 3H), 6.39 (s, 1H), 7.20-7.27 (m, 2H), 7.32-7.48 (m, 6H), 7.61 (ddd, J = 1.2, 1.5, 7.9 Hz, 1H).

### Step 3

In a similar manner to Step 2 of Reference Example 2-8, 3-methoxymethoxy-N-(1-methyl-1-phenylethyl)benzamide (8.07 g, 27.0 mmol) was dissolved in THF (320 mL), and the solution was treated with TMEDA (13.0 mL, 85.4 mmol), sec-butyllithium-hexane solution (0.99 mol/L, 87.1 mL, 86.3 mmol) and DMF (4.60 mL, 59.3 mmol), followed by purification by slurry using hexane to obtain 4-methoxymethoxy-3-hydroxy-2-(1-methyl-1-phenylethyl)isoindolino ne (8.50 g, yield 96%).
APCI-MS m/z: 300 [M+H]⁺; ¹H-NMR (CDCl₃)δ(ppm): 1.82 (s, 6H), 3.47 (s, 3H), 5.19 (s, 2H), 6.40 (s, 1H), 7.14-7.27 (m, 2H), 7.30-7.37 (m, 4H), 7.44-7.47 (m, 3H).

### Step 4

In a similar manner to Step 3 of Reference Example 2-8, 4-methoxymethoxy-3-hydroxy-2-(1-methyl-1-phenylethyl)isoindolino ne (8.50 mg, 26. 0 mmol) was dissolved in THF (340 mL), and the solution was treated with TMEDA (12.5 mL, 83.1 mmol), sec-butyllithium-hexane solution (0.99 mol/L, 84.0 mL, 83.1 mmol) and iodine (7.91 g, 31.2 mmol), followed by purification by flash column chromatography (hexane/ethyl acetate=90/10, 70/30) to obtain 4-methoxymethoxy-3-hydroxy-7-iodo-2-(1-methyl-1-phenylethyl)isoi ndolinone (7.65 g, yield 65%).
APCI-MSm/z: 328 [M+H]⁺; ¹H-NMR (CDCl₃)δ(ppm): 1.93 (s, 3H), 1.98 (s, 3H), 2.66 (d, J = 7.9 Hz, 1H), 3.52 (s, 3H), 5.30 (s, 2H), 6.31 (d, J = 7.9 Hz, 1H), 7.19-7.45 (m, 8H).

### Step 5

In a similar manner to Step 4 of Reference Example 2-8, 4-methoxymethoxy-3-hydroxy-7-iodo-2-(1-methyl-1-phenylethyl)isoi ndolinone (1.15 g, 2.54 mmol) was dissolved in nitromethane (58 mL), and the solution was added with trifluoroacetic acid (1.20 mL, 7.62 mmol) and triethylsilane (0.587 mL, 7.62 mmol), followed by stirring at room temperature for 30 minutes. The reaction mixture was added with water and extracted with ethyl acetate. The organic layer was washed with saturated aqueous sodium hydrogencarbonate solution and saturated brine, and dried over anhydrous sodium sulfate. The solvent was evaporated under reduced pressure. The residue was purified by flash column chromatography (chloroform/methanol=100/0, 99/1, hexane/ethyl acetate=80/20, 60/40) to obtain 4-methoxymethoxy-7-iodo-2-(1-methyl-1-phenylethyl)isoindolinone (935 mg, yield 84%). ESI-MS m/z: 454 [M+H]⁺; ¹H-NMR (CDCl₃)δ(ppm): 1.94 (s, 3H), 1.98 (s, 3H), 2.50 (d, J = 7.4 Hz, 1H), 3.50 (s, 3H), 5.27 (s, 2H), 6.13 (d, J = 7.3 Hz, 1H), 7.02 (d, J = 8.8 Hz, 1H), 7.23-7.35 (m, 4H), 7.43-7.46 (m, 2H), 7.81 (d, J = 8.6 Hz, 1H).

### Step 6

4-Methoxymethoxy-7-iodo-2-(1-methyl-1-phenylethyl)isoindoli none (463 mg, 1.06 mmol) was dissolved in 10% hydrogen chloride-methanol solution (14 mL), and the solution was stirred at 70 °C for 2.5 hours. Then, the solution was added with 10% hydrogen chloride-methanol solution (4.3 mL) and further stirred at 2.5 hours. The reaction mixture was added with chloroform and purified by slurry. The solid was collected by filtration and washed with chloroform/methanol(9/1). The obtained solid was dried under reduced pressure to obtain 4-hydroxy-7-iodoisoindolinone (248 mg, yield 85%).
APCI-MS m/z: 276 [M+H]⁺; ¹H-NMR (DMSO-d₆)δ(ppm): 4.12 (s, 2H), 6.77 (d, J = 8.2 Hz, 1H), 7.64 (d, J = 8.2 Hz, 1H), 8.65 (s, 1H), 10.21 (s, 1H).

### Step 7

In a similar manner to Step 2 of Reference Example 2-1, 4-hydroxy-7-iodoisoindolinone (220 mg, 0.800 mmol) was dissolved in acetonitrile (13.2 mL), and the solution was treated with Compound BD (573 mg, 1.60 mmol), palladium acetate (14.4 mg, 0.064 mmol), tri(o-tolyl)phosphine (39.0 mg, 0.128 mmol) and triethylamine (1.12 mL, 8.00 mmol), followed by purification by preparative thin-layer chromatography (chloroform/methanol=7/1) to obtain 4-hydroxy-7-[1-(tert-butoxycarbonyl)-5-(piperidinomethyl)indol-2 -yl]isoindolinone (294 mg, yield 80%).
ESI-MS m/z: 462 [M+H]⁺; ¹H-NMR (CDCl₃)δ(ppm): 1.26 (s, 9H), 1.49 (m, 2H), 1.67 (m, 4H), 2.55 (m, 4H), 3.62 (s, 2H), 4.11 (m, 2H), 6.03 (s, 1H), 6.11 (s, 1H), 6.59 (d, J = 8.3 Hz, 1H), 7.04 (d, J = 8.1 Hz, 1H), 7.15 (d, J = 8.4 Hz, 1H), 7.26 (m, 1H), 8.14 (d, J = 8.4 Hz, 1H).

### Step 8

In a similar manner to Step 2 of Reference Example 2-5, 4-hydroxy-7-[1-(tert-butoxycarbonyl)-5-(piperidinomethyl)indol-2 -yl]isoindolinone (284 mg, 0.616 mmol) was dissolved in methanol (7. 1 mL), and the solution was treated with 10% hydrogen chloride-methanol solution (7.1 mL). The obtained solid was collected by filtration and washed with methanol, followed by drying under reduced pressure to obtain Compound 139 (189 mg, yield 77%).
mp 253-256 °C; ¹H-NMR (DMSO-d₆)δ(ppm): 1.65-1.76 (m, 6H), 2.82-2.86 (m, 2H), 3.32 (m, 2H), 4.29 (m, 2H), 4.37 (s, 2H), 7.05 (s, 1H), 7.11 (d, J = 8.4 Hz, 1H), 7.21 (d, J = 8.3 Hz, 1H), 7.49 (d, J = 8.3 Hz, 1H), 7.69 (s, 1H), 8.03 (d, J = 8.4 Hz, 1H), 9.27 (s, 1H), 9.71 (br s, 1H), 10.40 (s, 1H), 13.85 (s, 1H).

### Reference Example 2-17:

### 4-Phenyl-7-[1H-5-(piperazin-1-ylcarbonyl)indol-2-yl]isoindolinon e (Compound 140)

### Step 1

3-Amino-6-bromophthalimide (3.10 g, 12.9 mmol) was dissolved in THF (240 mL), and the solution was added with diisobutylaluminiumhydride (0.94 mol/L, 68.6 mL, 64.5 mmol) by drops at -78 °C for 25 minutes. Then, the reaction mixture was warmed to room temperature, followed by stirring for 1.5 hours. Then, the reaction mixture was ice-cooled, added with water and filtered using Celite. The filtrate was extracted with ethyl acetate. The organic layer was dried over anhydrous sodium sulfate. The solvent was evaporated under reduced pressure. The residue was purified by slurry using chloroform to obtain 7-amino-4-bromo-3-hydroxyisoindolinone (2.26 g, yield 72%). APCI-MS m/z: 243 [M+H]⁺; ¹H-NMR (DMSO-d₆)δ(ppm): 5.64 (d, J = 9.5 Hz, 1H), 6.16 (s, 2H), 6.21 (d, J = 9.5 Hz, 1H), 6.58 (d, J = 8.6 Hz, 1H), 7.30 (d, J = 8.6 Hz, 1H), 8.65 (s, 1H).

### Step 2

7-Amino-4-bromo-3-hydroxyisoindolinone (3.11 g, 12.8 mmol) was dissolved in nitromethane (125 mL), and the solution was added with trifluoroacetic acid (9.90 mL, 128 mmol) and triethylsilane (4.10 mL, 25.6 mmol), followed by stirring at room temperature for 2 hours. The reaction mixture was added with water, extracted with ethyl acetate. The organic layer was washed with saturated aqueous sodium hydrogencarbonate solution and saturated brine, and dried over anhydrous sodium sulfate. The solvent was evaporated under reduced pressure and the residue was purified by slurry using chloroform to obtain 7-amino-4-bromoisoindolinone (2.16 g, yield 74%). APCI-MSm/z: 227 [M+H]⁺; ¹H-NMR (CDCl₃+CD₃OD)δ(ppm): 4.25 (s, 2H), 6.56 (d, J = 8.6 Hz, 1H), 7.33 (d, J = 8.6 Hz, 1H).

### Step 3

In a similar manner to Step 2 of Reference Example 2-1, 7-amino-4-bromoisoindolinone (250 mg, 1.10 mmol) was dissolved in acetonitrile (15 mL), and the solution was treated with phenylboronic acid (402 mg, 3.30 mmol), palladium acetate (20 mg, 0.088 mmol), tri(o-tolyl)phosphine (53.6 mg, 0.176 mmol) and triethylamine (1.53 mL, 11.0 mmol), followed by purification by preparative thin-layer chromatography (chloroform/methanol=15/1, chloroform/acetonitrile=6/1) to obtain 7-amino-4-phenylisoindolinone (219 mg, yield 89%). APCI-MS m/z: 225 [M+H]⁺; ¹H-NMR (CDCl₃)δ(ppm): 4.47 (s, 2H), 5.32 (s, 2H), 6.01 (s, 1H), 6.68 (d, J = 8.3 Hz, 1H), 7.29-7.45 (m, 6H).

### Step 4

7-Amino-4-phenylisoindolinone (80.0 mg, 0.357 mmol) was dissolved in acetonitrile (4.8 mL), and the solution was added with potassium iodide (71.0 mg, 0.428 mmol), copper iodide (82.0 mg, 0.428 mmol), iodine (109 mg, 0.428 mmol) and tert-butyl nitrite (0.068 mL, 0.57 mmol), followed by stirring under ice-cooling for 1.5 hours. Then, the reaction mixture was warmed to room temperature and further added with tert-butyl nitrite (0.068 mL, 0.57 mmol), followed by stirring at 50 °C for 1.5 hours. The reaction mixture was added with 10% aqueous sodium thiosulfate solution and extracted with ethyl acetate. The organic layer was washed with saturated brine and dried over anhydrous sodium sulfate. The solvent was evaporated under reduced pressure and the residue was purified by preparative thin-layer chromatography (chloroform/acetonitrile=6/1) to obtain 7-iodo-4-phenylisoindolinone (72.6 mg, yield 61%). ESI-MS m/z: 336 [M+H]⁺; ¹H-NMR (CDCl₃)δ(ppm): 4.40 (s, 2H), 6.58 (s, 1H), 7.36-7.54 (m, 5H), 8.01 (d, J = 7.9 Hz, 1H), 8.25 (d, J = 7.9 Hz, 1H).

### Step 5

In a similar manner to Step 2 of Reference Example 2-1, 7-iodo-4-phenylisoindolinone (69.0 mg, 0.206 mmol) was dissolved in acetonitrile (5.5 mL), and the solution was treated with Compound BB (195 mg, 0.412 mmol), palladium acetate (3.7 mg, 0.016 mmol), tri(o-tolyl)phosphine (10 mg, 0.032 mmol) and triethylamine (0.287 mL, 2.06 mmol), followed by purification by preparative thin-layer chromatography (chloroform/methanol=12/1, chloroform/acetonitrile=6/1) to obtain 4-phenyl-7-{1-(tert-butoxycarbonyl)-5-[4-(tert-butoxycarbonyl)pi perazin-1-ylcarbonyl]indol-2-yl}isoindolinone (49.5 mg, yield 38%). ESI-MS m/z: 637 [M+H]⁺; ¹H-NMR (DMSO-d₆)δ(ppm): 1.35 (s, 9H), 1.48 (s, 9H), 3.47 (m, 8H), 4.51 (s, 2H), 6.63 (s, 1H), 6.64 (s, 1H), 7.35-7.73 (m, 9H), 8.28 (d, J = 8.4 Hz, 1H).

### Step 6

In a similar manner to Step 3 of Reference Example 2-1, 4-phenyl-7-{1-(tert-butoxycarbonyl)-5-[4-(tert-butoxycarbonyl)pi perazin-1-ylcarbonyl]indol-2-yl}isoindolinone (49.0 mg, 0.0770 mmol) was dissolved in methanol (1.5 mL), and the solution was treated with 10% hydrogen chloride-methanol solution (1.5 mL), followed by purification by preparative thin-layer chromatography (chloroform/methanol=4/1) to obtain Compound 140 (15.4 mg, yield 46%).
¹H-NMR (DMSO-d₆)δ(ppm): 2.70 (m, 4H), 3.46 (m, 4H), 4.61 (s, 2H), 7.15 (d, J = 8.6 Hz, 1H), 7.33 (s, 1H), 7.44-7.65 (m, 7H), 7.69 (d, J = 8.3 Hz, 1H), 8.29 (d, J = 7.9 Hz, 1H), 9.42 (s, 1H), 14.16 (s, 1H).

### Reference Example 2-18:

### 4-[2-(Dimethylamino)ethoxy]-7-[1H-5-(piperidinomethyl)indol-2-yl ]isoindolinone (Compound 149)

### Step 1

4-Hydroxy-7-[1-(tert-butoxycarbonyl)-5-(piperidinomethyl)in dol-2-yl]isoindolinone (60.0 mg, 0.130 mmol) was dissolved in THF (3.6 mL), and the solution was added with triphenylphosphine (68 mg, 0.26 mmol), 2-dimethylaminoethanol (0.026 mL, 0.26 mmol) and 40% DEAD-toluene solution (0.118 mL) under ice-cooling, followed by stirring for 20 minutes. The reaction mixture was warmed to room temperature and stirred for 14 hours. The mixture was added with triphenylphosphine (68 mg, 0.26 mmol), 2-dimethylaminoethanol (0.026 mL, 0.26 mmol) and 40% DEAD-toluene solution (0.118 mL) and stirred for 1.7 hours. Then, the mixture was further added with triphenylphosphine (34 mg, 0.13 mmol), 2-dimethylaminoethanol (0.026 mL, 0.26 mmol) and 40% DEAD-toluene solution (0.059 mL), followed by stirring for 1.3 hours. The reaction mixture was added with water and extracted with ethyl acetate. The organic layer was washed with saturated brine and dried over anhydrous sodium sulfate. The solvent was evaporated under reduced pressure and the residue was purified by preparative thin-layer chromatography (chloroform/methanol/7 mol/L ammonia-methanol solution=12/0.5/0.5, chloroform/methanol=3/1) to obtain 4-[2-(dimethylamino)ethoxy]-7-[1-(tert-butoxycarbonyl)-5-(piperi dinomethyl)indol-2-yl]isoindolinone (36.9 mg, yield 53%).
ESI-MS m/z: 533 [M+H]⁺; ¹H-NMR (CDCl₃)δ(ppm): 1.32 (s, 9H), 1.42 (m, 2H), 1.59 (m, 4H), 2.37 (s, 6H), 2.42 (m, 4H), 2.79 (t, J = 5.6 Hz, 2H), 3.59 (s, 2H), 4.21 (t, J = 5.6 Hz, 2H), 4.36 (s, 2H), 6.49 (s, 1H), 7.01 (d, J = 8.2 Hz, 1H), 7.27 (m, 1H), 7.33 (s, 1H), 7.38 (d, J = 8.1 Hz, 1H), 7.47 (s, 1H), 8.16 (d, J = 8.6 Hz, 1H).

### Step 2

In a similar manner to Step 2 of Reference Example 2-5, 4-[2-(dimethylamino)ethoxy]-7-[1-(tert-butoxycarbonyl)-5-(piperi dinomethyl)indol-2-yl]isoindolinone (58.9 mg, 0.111 mmol) was dissolved in methanol (1.8 mL), and the solution was treated with 10% hydrogen chloride-methanol solution (1.8 mL). The solvent was evaporated under reduced pressure and the residue was purified by preparative thin-layer chromatography (chloroform/methanol/7 mol/L ammonia-methanol solution=12/0.5/0.5) to obtain Compound 149 (35.7 mg, yield 74%).
¹H-NMR (DMSO-d₆)δ(ppm): 1.39 (m, 2H), 1.48 (m, 4H), 2.23 (s, 6H), 2.33 (m, 4H), 2.67 (t, J = 5.6 Hz, 2H), 3.45 (s, 2H), 4.23 (t, J = 5.8 Hz, 2H), 4.36 (s, 2H), 7.02 (m, 2H), 7.33 (d, J = 8.6 Hz, 1H), 7.34 (d, J = 8.4 Hz, 1H), 7.39 (s, 1H), 8.10 (d, J = 8.7 Hz, 1H), 9.25 (s, 1H), 13.56 (s, 1H).

### Reference Example 2-19:

### 4-(4-Hydroxy-3-methoxy-5-nitrophenyl)-7-[1H-5-(piperidinomethyl) indol-2-yl]isoindolinone hydrochloride (Compound 152)

### Step 1

7-Amino-4-bromoisoindolinone (150 mg, 0.661 mmol) was dissolved in dimethoxythane (10.5 mL), and the solution was added with 2-methoxy4-(4,4,5,5-tetramethyl-1,3,2-dioxaboran-2-yl)phenol (331 mg, 1.32 mmol), [bis(diphenylphosphino)ferrocene]dichloropalladium (43.2 mg, 0.0529 mmol) and potassium carbonate (456 mg, 3.31 mmol), and stirred at 90 °C for 4.3 hours under argon atmosphere. The reaction mixture was added with water and extracted with ethyl acetate. The organic layer was washed with saturated brine and dried over anhydrous sodium sulfate. The solvent was evaporated under reduced pressure and the residue was purified by preparative thin-layer chromatography (chloroform/acetonitrile=4/1, chloroform/methanol=10/1) to obtain 7-amino-4-(4-hydroxy-3-methoxyphenyl)isoindolinone (169 mg, yield 94%).
APCI-MS m/z: 271 [M+H]⁺; ¹H-NMR (DMSO-d₆)δ(ppm): 3.79 (s, 3H), 4.37 (s, 2H), 6.10 (s, 2H), 6.63 (d, J = 8.4 Hz, 1H), 6.78 (d, J = 8.1 Hz, 1H), 6.85 (d, J = 8.2 Hz, 1H), 6.97 (s, 1H), 7.27 (d, J = 8.2 Hz, 1H), 8.21 (s, 1H), 8.93 (s, 1H).

### Step 2

In a similar manner to Step 4 of Reference Example 2-17, 7-amino-4-(4-hydroxy-3-methoxyphenyl)isoindolinone (202 mg, 0.747 mmol) was dissolved in acetonitrile (10.1 mL), and the solution was treated with potassium iodide (149 mg, 0.896 mmol), copper iodide (171 mg, 0.896 mmol), iodine (228 mg, 0.896 mmol) and tert-butyl nitrite (0.266 mL, 0.896 mmol), followed by purification by preparative thin-layer chromatography (chloroform/methanol=15/1) to obtain 7-iodo-4-(4-hydroxy-3-methoxy-5-nitrophenyl)isoindolinone (100 mg, yield 31%). APCI-MS m/z: 427 [M+H]⁺; ¹H-NMR (DMSO-d₆)δ(ppm): 3.93 (s, 3H), 4.42 (s, 2H), 7.40 (d, J = 8.1 Hz, 1H), 7.44 (d, J = 1.6 Hz, 1H), 7.60 (d, J = 1.5 Hz, 1H), 7.99 (d, J = 8.1 Hz, 1H), 8.30 (s, 1H), 8.85 (s, 1H).

### Step 3

In a similar manner to Step 2 of Reference Example 2-1, 7-iodo-4-(4-hydroxy-3-methoxy-5-nitrophenyl)isoindolinone (40.0mg, 0. 0939 mmol) was dissolved in acetonitrile (2.8 mL), and the solution was treated with Compound BD (75.0 mg, 0.207 mmol), palladium acetate (1.9 mg, 0.0085 mmol), tri(o-tolyl)phosphine (5.1 mg, 0.017 mmol) and triethylamine (0.146 mL, 1.05 mmol), followed by purification by preparative thin-layer chromatography (chloroform/methanol=4/1) to obtain 4-(4-hydroxy-3-methoxy-5-nitrophenyl)-7-[1-(tert-butoxycarbonyl) -5- (piperidinomethyl) indol-2-yl] isoindolinone (29.8 mg, yield 52%). ESI-MS m/z: 613 [M+H]⁺; ¹H-NMR (DMSO-d₆)δ(ppm): 1.20 (s, 9H), 1.35 (m, 2H), 1:41 (m, 4H), 2.55 (m, 4H), 3.73 (s, 2H), 3.85 (s, 3H), 4.54 (s, 2H), 6.58 (s, 1H), 7.25 (s, 1H), 7.30 (d, J = 8.4 Hz, 1H), 7.48 (d, J = 7.9 Hz, 1H), 7.52 (s, 1H), 7.62 (s, 1H), 7.70 (d, J = 7.6 Hz, 1H), 8.10 (d, J = 8.6 Hz, 1H), 8.30 (s, 1H), 8.67 (s, 1H).

### Step 4

In a similar manner to Step 2 of Reference Example 2-5, 4-(4-hydroxy-3-methoxy-5-nitrophenyl)-7-[1-(tert-butoxycarbonyl) -5-(piperidinomethyl)indol-2-yl]isoindolinone (26.5 mg, 0.0432 mmol) was dissolved in methanol (1.1 mL), and the solution was treated with 10% hydrogen chloride-methanol solution (1.1 mL). The obtained solid was collected by filtration and washed with methanol, followed by drying under reduced pressure to obtain Compound 152 (13.1 mg, yield 55%).
ESI-MS m/z: 513 [M+H]⁺; ¹H-NMR (DMSO-d₆)δ(ppm): 1.53-1.74 (m, 6H), 3.07 (m, 4H), 3.95 (s, 3H), 4.31 (s, 2H), 4.66 (s, 2H), 7.27 (d, J = 8.7 Hz, 1H), 7.36 (s, 1H), 7.49 (d, J = 1.8 Hz, 1H), 7.57 (d, J = 8.2 Hz, 1H), 7.65 (d, J = 2.0 Hz, 1H), 7.57 (m, 2H), 8.31 (d, J = 8.4 Hz, 1H), 9.45 (s, 1H), 10.20 (br s, 1H), 14.15 (s, 1H).

### Reference Example 2-20:

### 3-Amino-6-[1H-3-(4-hydroxy-1-butynyl)indol-2-yl]phtalimide (Compound 161)

### Step 1

3-Amino-6-[1-(tert-butoxycarbonyl)indol-2-yl]phtalimide (300 mg, 0. 795 mmol) was dissolved in ethanol (21 mL), and the solution was added with iodine (242 mg, 0.954 mmol) and silver sulfate (248 mg, 0.795 mmol), followed by stirring at room temperature for 1.3 hours. The reaction mixture was added with water and extracted with ethyl acetate. The organic layer was washed with saturated brine and dried over anhydrous sodium sulfate. The solvent was evaporated under reduced pressure and the residue was purified by slurry using chloroform to obtain 3-amino-6-[1-(tert-butoxycarbonyl)-3-iodoindol-2-yl]phtalimide (326 mg, yield 82%).
APCI-MS m/z: 502 [M-H]⁻; ¹H-NMR (DMSO-d₆)δ(ppm): 1.21 (s, 9H), 6.64 (s, 2H), 7.05 (d, J = 8.4 Hz, 1H), 7.32-7.45 (m, 4H), 8.13 (d, J = 7.9 Hz, 1H), 10.98 (s, 1H).

### Step 2

3-Amino-6-[1-(tert-butoxycarbonyl)-3-iodoindol-2-yl]phtalim ide(150 mg, 0.298 mmol) was dissolved in diethylamine (7.5 mL), and the solution was added with bis(triphenylphosphine)dichloropalladium (16.7 mg, 0.0234 mmol), copper iodide (11.4 mg, 0.0596 mmol) and 3-butyn-1-ol (0.226 mL, 2.98 mmol), followed by stirring at 50 °C for 3.7 hours under argon atmosphere. The reaction mixture was added with water and extracted with ethyl acetate. The organic layer was washed with saturated brine and dried over anhydrous sodium sulfate. The solvent was evaporated under reduced pressure and the residue was purified by preparative thin-layer chromatography (chloroform/methanol=10/1) to obtain 3-amino-6-[1-(tert-butoxycarbonyl)-3-(4-hydroxy-1-butynyl)indol-2-yl]phtalimide (115 mg, yield 87%).
APCI-MS m/z: 444 [M-H]⁻; ¹H-NMR (CDCl₃+CD₃OD)δ(ppm): 2.62 (t, J = 6.8 Hz, 2H), 3.70 (t, J = 6.8 Hz, 2H), 6.96 (d, J = 8.6 Hz, 1H), 7.26-7.39 (m, 2H), 7.57 (d, J = 8.4 Hz, 1H), 7.65 (d, J = 7.3 Hz, 1H), 8.17 (d, J = 8.3 Hz, 1H).

### Step 3

3-Amino-6-[1-(tert-butoxycarbonyl)-3-(4-hydroxy-1-butynyl)i ndol-2-yl]phtalimide (40.0 mg, 0.0898 mmol) was dissolved in dichloromethane (2.0 mL). The solution was treated with trifluoroacetic acid (1 mL) and stirred at room temperature for 1 hour. The reaction mixture was added with saturated aqueous sodium hydrogencarbonate solution and extracted with ethyl acetate. The organic layer was washed with saturated brine and dried over anhydrous sodium sulfate. The solvent was evaporated under reduced pressure and the residue was purified by preparative thin-layer chromatography (chloroform/methanol=6/1) to obtain Compound 161 (19 mg, yield 61%).
¹H-NMR (DMSO-d₆)δ(ppm): 2.46 (m, 2H), 3.21 (m, 2H), 4.31 (t, J = 4.8 Hz, 1H), 6.70 (s, 2H), 7.04 (d, J = 8.6 Hz, 1H), 7.19 (m, 2H), 7.38 (m, 1H), 7.47 (d, J = 8.6 Hz, 1H), 8.17 (m, 1H), 10.99 (s, 1H), 11.90 (s, 1H).

### Reference Example 2-21:

### 4-Chloro-7-[1H-3-dimethylaminomethyl-5-(piperidinomethyl)indol-2 -yl]isoindolinone (Compound 163)

Compound 29 (25.0 mg, 0.0600 mmol) was suspended in acetic acid (0.5 mL), and the suspension was added with 50% aqueous dimethylamine solution (0.024 mL, 0.24 mmol) and 37% aqueous formamide solution (23 mg, 0. 12 mmol), followed by stirring at room temperature for 10 hours. The reaction mixture was added with water and sodium carbonate to adjust the pH to 9, and extracted with ethyl acetate. The organic layer was washed with water and saturated brine and then dried over anhydrous sodium sulfate. The solvent was evaporated under reduced pressure. The residue was dissolved in ethyl acetate, and the solution was added with hexane and stirred for 30 minutes. The solid was collected by filtration and washed with hexane, followed by drying under reduced pressure to obtain Compound 163 (12.0 mg, yield 46%). ¹H-NMR (DMSO-d₆)δ(ppm): 1.32-1.56 (m, 6H), 2.14 (s, 6H), 2.29-2.40 0 (m, 4H), 3.47 (s, 2H), 3.49 (s, 2H), 4.45 (s, 2H), 7.08 (d, J = 8.4 Hz, 1H), 7.33 (d, J = 8.4 Hz, 1H), 7.54 (s, 1H), 7.78 (d, J = 8.1 Hz, 1H), 8.13 (d, J = 8.1 Hz, 1H), 9.10 (s, 1H), 12.16 (s, 1H).

### Reference Example 2-22:

### 4-Chloro-7-[1H-3-bromo-5-(piperidinomethyl)indol-2-yl]isoindolin one (Compound 164)

Compound 29 (30.0 mg, 0.0721 mmol) was suspended in THF (2 mL), and the suspension was added with triethylamine (0. 100 mL, 0.715 mmol), water (1 mL) and N-bromosuccineimide (12.8 mg, 0.719 mmol), followed by stirring at room temperature for 20 hours. The reaction mixture was added with water and extracted with ethyl acetate. The organic layer was washed with saturated brine, followed by drying over anhydrous sodium sulfate. The solvent was evaporated under reduced pressure. The residue was suspended in hexane, and the solid was collected by filtration and washed with hexane, followed by drying under reduced pressure to obtain Compound 164 (29.1 mg, yield 88%). ¹H-NMR (DMSO-d₆)δ(ppm): 1.33-1.56 (m, 6H), 2.29-2.40 (m, 4H), 3.52 (s, 2H), 4.46 (s, 2H), 7.17 (d, J = 8.2 Hz, 1H), 7.34 (s, 1H), 7.39 (d, J = 8.2 Hz, 1H), 7.82 (d, J = 8.4 Hz, 1H), 7.90 (d, J = 8.4 Hz, 1H), 9.07 (br s, 1H), 12.24 (s, 1H).

### Reference Example 2-23:

### 3-(Benzothiophen-2-yl)phtalimide (Compound 168)

### Step 1

3-Aminophthalic acid (5.00 g, 27.6 mmol) was dissolved in 8.4 mol/L hydrochloric acid (60 mL), and the solution was added with an aqueous solution (10 mL) of sodium nitrate (2.0 g, 29 mmol) by drops under ice-cooling for 20 minutes, followed by stirring at the same temperature for 3 hours. Then, an aqueous solution (10 mL), in which potassium iodide (6.9 g, 41 mmol) and urea (291 mg) were dissolved, is added by drops to the mixture, followed by stirring at room temperature for 20 hours. The reaction mixture was added with 10% aqueous sodium thiosulfate solution and extracted with ethyl acetate. The organic layer was washed with saturated brine and dried over anhydrous sodium sulfate. The solvent was evaporated under reduced pressure and the residue was washed with chloroform to obtain 3-iodophthalic acid (5.0 g, yield 62%).

### Step 2

3-Iodophthalic acid (4.00 g, 13.7 mmol) was dissolved in anhydrous acetic acid, and the solution was stirred at 145 °C for 1 hour. The solvent of the reaction mixture was evaporated under reduced pressure and the residue was purified by slurry using diisopropylether to obtain 3-iodophthalic acid anhydride (3.6 g, yield 96%).
FAB-MS m/z: 275 [M+H]⁺; ¹H-NMR (CDCl₃)δ(ppm): 7.56 (dd, J = 7.6, 7.6 Hz, 1H), 8.01 (dd, J = 0.8, 7.4 Hz, 1H), 8.30 (dd, J = 0.7, 7.9 Hz, 1H).

### Step 3

3-Iodophthalic anhydride (598 mg, 2.18 mmol) was dissolved in DMF (14 mL), and the solution was added with an aqueous solution (10 mL) of hexamethyldisilazane (HMDS) (4.6 mL, 22 mmol) and methanol (0.44 mL, 11 mmol), followed by stirring at room temperature for 18.5 hours. The reaction mixture was added with water and extracted with ethyl acetate. The organic layer was washed with saturated brine and dried over anhydrous sodium sulfate. The solvent was evaporated under reduced pressurea and the residue was purified by slurry using chloroform to obtain 3-iodophtalimide (403 mg, yield 68%). APCI-MS m/z: 272 [M-H]⁻; ¹H-NMR (DMSO-d₆)δ(ppm): 7.53 (dd, J = 7.4, 7.8 Hz, 1H), 7.84 (dd, J = 0.6, 7.3 Hz, 1H), 8.22 (dd, J = 0.6, 7.8 Hz, 1H), 11.52 (br s, 1H).

### Step 4

In a similar manner to Step 2 of Reference Example 2-1, 3-iodophtalimide (50.0 mg, 0.183 mmol) was dissolved in acetonitrile (2.5 mL), and the solution was treated with 2-benzothiopheneboronic acid (65.0 mg, 0.366 mmol), palladium acetate (2.0 mg, 0.0092 mmol), tri(o-tolyl)phosphine (5.6 mg, 0.018 mmol) and triethylamine (0.128 mL, 0.915 mmol), followed by purification by preparative thin-layer chromatography (chloroform/acetonitrile=30/1) to obtain Compound 168 (43.3 mg, yield 85%).
¹H-NMR (DMSO-d₆)δ(ppm): 7.43-7.48 (m, 2H), 7.84-8.07 (m, 5H), 8.15 (s, 1H), 11.48 (br s, 1H).

### Reference Example 2-24:

### 4-Chloro-7-(1H-benzimidazol-2-yl)isoindolinone (Compound 174)

### Step 1

4-Chloro-3-hydroxy-2-(1-methyl-1-phenylethyl)isoindolinone (1.00 g, 3.31 mmol) was dissolved in THF(40 mL), and the solution was added with TMEDA (1.10 mL, 7.28 mmol), and added with sec-butyllithium-hexane solution (0.99 mol/L, 7.36 mL, 7.28 mmol) by drops at -78 °C for 5 minutes under argon atmosphere, and the mixture was stirred for 2 hours at the same temperature. Then, the mixture was added with DMF (0.384 mL, 4.97 mmol) and warmed from -78 °C to room temperature for 3.5 hours. The reaction mixture was added with water and extracted with ethyl acetate. The organic layer was washed with saturated brine and dried over anhydrous sodium sulfate. The solvent was evaporated under reduced pressure and the residue was purified by flash column chromatography (hexane/ethyl acetate=95/5, 60/40) and preparative thin-layer chromatography (hexane/ethyl acetate=1/1) to obtain 4-chloro-3-hydroxy-7-formyl-2-(1-methyl-1-phenylethyl)isoindolin one (993 mg, yield 91%).
APCI-MS m/z: 330 [M+H]⁺; ¹H-NMR (CDCl₃)δ(ppm): 1.96 (s, 3H), 2.01 (s, 3H), 6.32 (d, J = 8.4 Hz, 1H), 7.28 (m, 2H), 7.36 (m, 2H), 7.46 (m, 2H), 7.57 (dd, J = 0.7, 8.3 Hz, 1H), 7.94 (d, J = 8.3 Hz, 1H), 10.92 (s, 1H).

### Step 2

4-Chloro-3-hydroxy-7-formyl-2-(1-methyl-1-phenylethyl)isoin dolinone (100 mg, 0.303 mmol) was dissolved in nitrobenzene (2 mL), and the solution was added with o-phenylenediamine (49 mg, 0.45 mmol), followed by stirring at 130 °C for 5.2 hours. The reaction mixture was added with hexane and the obtained solid was collected by filtration and purified by preparative thin-layer chromatography (hexane/ethyl acetate=2/1, 1/1) to obtain 4-chloro-3-hydroxy-7-(1H-benzimidazol-2-yl)-2-(1-methyl-1-phenyl ethyl)isoindolinone (42.8 mg, yield 34%).
APCI-MS m/z: 418 [M+H]⁺; ¹H-NMR (DMSO-d₆)δ(ppm): 1.90 (s, 3H), 1.92 (s, 3H), 6.52 (d, J = 10_{.}4 Hz, 1H), 6.99 (d, J = 10.2 Hz, 1H), 7.16-7.22 (m, 3H), 7.30 (dd, J = 7.1, 7.7 Hz, 2H), 7.48 (d, J = 7.1 Hz, 2H), 7.66 (m, 2H), 7.88 (d, J = 8.6 Hz, 1H), 8.72 (d, J = 8.6 Hz, 1H), 14.03 (s, 1H).

### Step 3

4-Chloro-3-hydroxy-7-(1H-benzimidazol-2-yl)-2-(1-methyl-1-p henylethyl)isoindolinone (39.4 mg, 0.0943 mmol) was dissolved in nitromethane (2.8 mL), and the solution was added with trifluoroacetic acid (0.073 mL, 0.94 mmol) and triethylsilane (0.030 mL, 0.19 mmol), followed by stirring at room temperature for 23.5 hours. Then, the mixure was added with trifluoroacetic acid (0.146 mL, 1.89 mmol) and triethylsilane (0.045 mL, 0.28 mmol) and warmed to 70 °C, followed by stirring for 7.5 hours. Further, the mixure was added with trifluoroacetic acid (1.00 mL) and triethylsilane (0.15 mL, 0.94 mmol), followed by stirring for 4 hours. The reaction mixture was added with saturated aqueous sodium hydrogencarbonate solution and extracted with ethyl acetate. The organic layer was washed with saturated brine and dried over anhydrous sodium sulfate. The solvent was evaporated under reduced pressure. The residue was purified by slurry using chloroform/methanol (9/1) and by preparative thin-layer chromatography (hexane/ethyl acetate=2/1, 1/1) to obtain Compound 174 (13.7 mg, yield 51%).
¹H-NMR (DMSO-d₆)δ(ppm): 4.57 (s, 2H), 7.25 (m, 2H), 7.70 (m, 2H), 7.88 (d, J = 8.4 Hz, 1H), 8.31 (s, 0.4H), 8.70 (d, J = 8.4 Hz, 1H), 9.76 (s, 1H), 14.82 (s, 0.6H).

### Reference Example 2-25:

### 4-Chloro-7-(1H-5-carboxybenzimidazol-2-yl)isoindolinone (Compound 178)

### Step 1

4-Chloro-3-hydroxy-7-formyl-2-(1-methyl-1-phenylethyl)isoin dolinone (200 mg, 0.606 mmol) was suspended in acetonitrile (8 mL), and the suspension was added with 2,3-dichloro-5,6-dicyano-1,4-benzoquinone (151 mg, 0.665 mmol), followed by stirring at room temperature for 4 hours. The reaction mixture was added with water and stirred under ice-cooling for 1 hour. The obtained solid was collected by filtration, washed with water and dried under reduced pressure. The solid was suspended in methanol, and the suspetion was added with water and stirred at room temperature for 1 hour. The solid was collected by filtration, washed with water and dried under reduced pressure. The solid was suspended in methanol and added with diisopropylether, followed by stirring under ice-cooling for 30 minutes. The solid was collected by filtration and washed with diisopropylether/methanol (10/1), followed by drying under reduced pressure to obtain 4-chloro-3-hydroxy-7-(1H-5-carboxybenzimidazol-2-yl)-2-(1-methyl -1-phenylethyl)isoindolinone (181 mg, yield 65%).
ESI-MS m/z: 462 [M+H]⁺; ¹H-NMR (DMSO-d₆)δ(ppm): 1.91 (s, 6H), 6.53 (d, J = 10.3 Hz, 1H), 7.03 (d, J = 10.3 Hz, 1H), 7.18 (m, 1H), 7.26-7.32 (m, 2H), 7.45-7.56 (m, 2H), 7.70-7.85 (m, 2H), 7.89 (d, J = 8.4Hz, 1H), 8.24 (s, 1H), 8.71 (d, J = 8.4 Hz, 1H), 12.70 (br s, 1H), 14.24 (s, 1H).

### Step 2

In a similar manner to Step 3 of Reference Example 2-24, 4-chloro-3-hydroxy-7-(1H-5-carboxybenzimidazol-2-yl)-2-(1-methyl -1-phenylethyl)isoindolinone (180 mg, 0.390 mmol) was dissolved in trifluoroacetic acid (3.6 mL), and the solution was treated with triethylsilane (0.187 mL, 1.17 mmol). The solvent was evaporated under reduced pressure. The residue was suspended in ethyl acetate and the solid was collected by filtration, washed with ethyl acetate and then dried under reduced pressure. The solid was suspended in methanol and stirred at room temperature for 30 minutes. Then, the solid was collected by filtration and washed with methanol, followed by drying under reduced pressure to obtain Compound 178 (62.2 mg, yield 49%).
¹H-NMR (DMSO-d₆)δ(ppm): 4.58 (s, 2H), 7.78 (d, J = 8.6 Hz, 1H), 7.87 (dd, J = 1.5, 8.6 Hz, 1H), 7.91 (d, J = 8.4 Hz, 1H), 8.29 (d, J = 1.5 Hz, 1H), 8.67 (d, J = 8.4 Hz, 1H), 9.84 (s, 1H).

### Reference Example 2-26:

### 4-Chloro-7-[1H-5-(4-acetylpiperazin-1-ylcarbonyl)benzimidazol-2-yl]isoindolinone (Compound 179)

Compound 178 (15.0 mg, 0.0458 mmol) was dissolved in DMF (0.5 mL), and the solution was added with EDCI (9.7 mg, 0.051 mmol), HOBT monohydrate (3.1 mg, 0. 023 mmol) and 1-acetylpiperazin (7.0 mg, 0. 055 mmol), followed by stirring at room temperature for 30 hours. The reaction mixture was added with water and extracted with ethyl acetate. The organic layer was washed with saturated brine and dried over anhydrous sodium sulfate. The solvent was evaporated under reduced pressure and the residue was purified by flash column chromatography (chloroform/methanol=19/1, 4/1). The obtained solid was suspended in chloroform, added with diisopropylether and stirred under ice-cooling for 30 minutes. The solid was collected by filtration, washed with diisopropylether and then dried under reduced pressure to obtain Compound 179 (11.5 mg, yield 58%).
¹H-NMR (DMSO-d₆)δ(ppm): 2.03 (s, 3H), 3.45-3.60 (m, 8H), 4.58 (s, 2H), 7.30 (dd, J = 1.5, 8.4 Hz, 1H), 7.78 (d, J = 8.4 Hz, 1H), 7.82 (s, 1H), 7.90 (d, J = 8.4 Hz, 1H), 8.70 (d, J = 8.4 Hz, 1H), 9.81 (s, 1H), 15.00 (s, 1H).

### Reference Example 2-27:

### 4-Chloro-7-{1H-5-[4-(1,1-dimethylethoxycarbonyl)piperazin-1-ylca rbonyl]benzimidazol-2-yl}isoindolinone (Compound 180)

In a similar manner to Example 179, Compound 178 (50.7 mg, 0.155 mmol) was dissolved in DMF (1 mL), and the solution was treated with EDCI (32.7 mg, 0.171 mmol), HOBT monohydrate (10.5 mg, 0.0777 mmol) and 1-(tert-butoxycarbonyl)piperazine (34.6 mg, 0.186 mmol). The reaction mixture was added with water and extracted with chloroform. The organic layer was washed with 1 mol/L hydrochloric acid, saturated aqueous sodium hydrogencarbonate solution and saturated brine, and dried over anhydrous sodium sulfate. The solvent was evaporated under reduced pressure and the residue was purified by flash column chromatography (chloroform/methanol=19/1, 4/1) to obtain Compound 180 (18.8 mg, yield 25%).
¹H-NMR (CDCl₃)δ(ppm): 1.48 (s, 9H), 3.40-3.80 (m, 8H), 4.57 (s, 2H), 6.70 (br s, 1H), 7.39 (dd, J = 1.5, 8.4 Hz, 1H), 7.62 (d, J = 8.4 Hz, 1H), 7.72 (d, J = 8.4 Hz, 1H), 7.83 (br s, 1H), 8.86 (d, J = 8.4 Hz, 1H), 14.52 (br s, 1H).

### Reference Example 2-28:

### 4-Chloro-7-{1H-5-[4-(2-hydroxyethyl)piperazin-1-ylcarbonyl]benzi midazol-2-yl}isoindolinone (Compound 184)

Compound 178 (30.0 mg, 0.0915 mmol) was dissolved in DMF (1.0 mL), and the solution was added with thionyl chloride (0.020 mL, 0.27 mmol) under ice-cooling and stirred for 40 minutes. The mixture was added with 1-(2-hydroxyethyl)piperazine (0.112 mL, 0.913 mmol) and stirred for 30 minutes under ice-cooling. The reaction mixture was added with water. The obtained solid was collected by filtration and washed with water, followed by drying under reduced pressure to obtain Compound 184 (20.8 mg, yield 52%).
¹H-NMR (DMSO-d₆)δ(ppm): 2.38-2.50 (m, 4H), 3.44-3.68 (m, 8H), 4.44 (br s, 1H), 4.56 (s, 2H), 7.27 (dd, J = 1.5, 8.3 Hz, 1H), 7.69-7.78 (m, 2H), 7.88 (d, J = 8.6 Hz, 1H), 8.68 (d, J = 8.6 Hz, 1H), 9.79 (br s, 1H), 14.94 (s, 1H).

### Reference Example 2-29:

### 4-Methanesulfonyloxy-7-[1H-5-(piperidinomethyl)indol-2-yl]isoind olinone hydrochloride (Compound 227)

### Step 1

4-Hydroxy-7-[1-(*tert*-butoxycarbonyl)-5-(piperidinomethyl)in dol-2-yl]isoindolinone (0.230 g, 0.498 mmol) was dissolved in dichloromethane (4.6 mL), and the solution was added with triethylamine (0.139 mL, 0.997 mmol) and methanesulfonyl chloride (0.0460 mL, 0.598 mmol), followed by stirring under ice-cooling for 1.5 hours. The reaction mixture was added with water and extracted with chloroform. The organic layer was washed with saturated brine and dried over anhydrous sodium sulfate. The solvent was evaporated under reduced pressure and the residue was purified by flash column chromatography (chloroform/methanol=85/15) to obtain 4-methanesulfonyloxy-7-[1-(*tert*-butoxycarbonyl)-5-(piperidinomet hyl)indol-2-yl]isoindolinone (0.104 g, yield 61%). ESI-MS *m*/*z*: 540 [M+H]⁺; ¹H-NMR (CDCl₃)δ(ppm): 1.31 (s, 9H), 1.38-1.48 (m, 2H), 1.53-1.62 (m, 4H), 2.35-2.54 (m, 4H), 3.29 (s, 1H), 3.58 (s, 2H), 4.54 (s, 2H), 6.55 (s, 1H), 7.31 (dd, *J* = 1.3, 8.8 Hz, 1H), 7.45-7. 53 (m, 3H), 8.18 (d, *J* = 8.4 Hz, 1H).

### Step 2

In a similar manner to Step 2 of Reference Example 2-5, 4-methanesulfonyloxy-7-[1-(*tert*-butoxycarbonyl)-5-(piperidinomet hyl)indol-2-yl]isoindolinone (0.373 g, 0.691 mmol) was treated with 10% hydrogen chloride-methanol solution (7.5 mL), followed by purification by slurry using diisopropylether and methanol to obtain Compound 227 (0.237 g, yield 72%).
mp 222-225 °C; ¹H-NMR (DMSO-*d*₆)δ(ppm): 1.30-1.44 (m, 1H), 1.59-1.87 (m, 5H), 2.78-2.95 (m, 2H), 3.32-3.68 (m, 2H), 3.58 (s, 3H), 4.29-4.36 (m, 2H), 4.59 (s, 2H), 7.31 (d, *J* = 8.4 Hz, 1H), 7.34 (d, *J* = 0.8 Hz, 1H), 7.58 (d, *J* = 8.4 Hz, 1H), 7.68 (d, *J* = 8.8 Hz, 1H), 7.78 (d, *J* = 0.8 Hz, 1H), 8.31 (d, *J* = 8.8 Hz, 1H), 9.55 (s, 1H), 9.76 (br s, 1H), 13.9 (s, 1H).

### Reference Example 2-30:

### 4-(2-Fluoro-4-chlorobenzenesulfonyloxy)-7-[1H-5-(piperidinomethy 1)indol-2-yl]isoindolinone (Compound 233)

### Step 1

4-Hydroxy-7-[1-(*tert*-butoxycarbonyl)-5-(piperidinomethyl)in dol-2-yl]isoindolinone (0.0300 g, 0.0650 mmol) was dissolved in acetonitrile (0.650 mL), and the solution was added with triethylamine (0.0540 mL, 0.390 mmol) and 4-chloro-2-fluorobenzenesulfonyl chloride (0.0450 g, 0.195 mmol), followed by stirring at room temperature for 12 hours. The reaction mixture was added with water and the solvent was evaporated. The residue was added with water and extracted with chloroform. The organic layer was washed with saturated brine and dried over anhydrous magnesium sulfate, and then filtered through a column filled with SCX (positive-ion exchange resin). The SCX was washed with 2 mol/L ammonia-methanol solution and the filtrate was evaporated to obtain 4-(2-fluoro-4-chlorobenzenesulfonyloxy)-7-[1-(*tert*-butoxycarbony 1)-5-(piperidinomethyl)indol-2-yl]isoindolinone (0.0116 g, yield 27%).
ESI-MS *m*/*z*: 655 [M+H]⁺

### Step 2

4-(2-Fluoro-4-chlorobenzenesulfonyloxy)-7-[1-(*tert*-butoxyca rbonyl)-5-(piperidinomethyl)indol-2-yl]isoindolinone (0.0116 g, 0.0177 mmol) was dissolved in 10% hydrogen chloride-methanol solution (0.650 mL) and the solution was stirred at 55 °C for 12 hours. The reaction mixture was concentrated and added with 1,1,1,3,3,3-hexafluoro-2-propanol (0.500 mL) and AG 1-X8 resin, followed by stirring at room temperature for 2 hours. The mixture was filtered, and the filtrate was concentrated and filtered through a column filled with SCX (positive-ion exchange resin). The SCX was washed with 2 mol/L ammonia-methanol solution and the filtrate was evaporated to obtain Compound 233 (0.00740 g, yield 75%).

### Reference Example 2-31:

### 4-(4-Hydroxy-3-methoxyphenyl)-7-[1H-5-(piperidinomethyl)indol-2-yl]isoindolinone hydrochloride (Compound 304)

### Step 1

7-Amino-4-(4-hydroxy-3-methoxyphenyl)isoindolinone (50.0 mg, 0.185 mmol) was dissolved in acetonitrile (2.5 mL), and the solution was added with *tert*-butyldimethylsilyl chloride (33.5 mg, 0. 222 mmol), 1,8-diazabicyclo[5.4.0]undec-7-ene (DBU) (0.033 mL, 0.22 mmol), followed by stirring at room temperature for 1.3 hours. The mixture was added with *tert*-butyldimethylsilyl chloride (33.5 mg, 0. 222 mmol) and DBU (0.033 mL, 0.22 mmol), followed by further stirring for 1 hour. The reaction mixture was added with water and extracted with ethyl acetate. The organic layer was washed with saturated brine and dried over anhydrous sodium sulfate. The solvent was evaporated under reduced pressure and the residue was purified by preparative thin-layer chromatography (chloroform/acetonitrile=2/1) to obtain 7-amino-4-(4-*tert*-butyldimethylsilyloxy-3-methoxyphenyl)isoindol inone (51.1 mg, yield 72%).
APCI-MS *m*/*z*: 385 [M+H]⁺; ¹H-NMR (CDCl₃)δ(ppm): 0.19 (s, 6H), 1.01 (s, 9H), 3.83 (s, 3H), 4.45 (s, 2H), 5.28 (s, 1H), 5.99 (s, 1H), 6.66 (d, *J* = 8.2 Hz, 1H), 6.82-6.89 (m, 3H), 7.32 (d, *J* = 8.2 Hz, 1H).

### Step 2

In a similar manner to Step 4 of Reference Example 2-17, 7-amino-4-(4-*tert*-butyldimethylsilyloxy-3-methoxyphenyl)isoindol inone (43.0 mg, 0.110 mmol) was dissolved in acetonitrile (6 mL), and the solution was treated with potassium iodide (29.2 mg, 0.176 mmol), copper iodide (33.5 mg, 0.896 mmol), iodine (44.7 mg, 0.896 mmol) and tert-butyl nitrate (0.059 mL, 0.50 mmol), followed by purification by preparative thin-layer chromatography (chloroform/acetonitrile=5/1) to obtain 7-iodo-4-(4-*tert*-butyldimethylsilyloxy-3-methoxyphenyl)isoindoli none (33.8 mg, yield 62%).
APCI-MS *m*/*z*: 496 [M+H]⁺; ¹H-NMR (CDCl₃)δ(ppm): 0.20 (s, 6H), 1.02 (s, 9H), 3.84 (s, 3H), 4.39 (s, 2H), 6.56 (br s, 1H), 6.86-6.94 (m, 3H), 7.23 (d, *J* = 7.9 Hz, 1H), 7.97 (d, *J* = 7.9 Hz, 1H).

### Step 3

In a similar manner to Step 2 of Reference Example 2-1, 7-iodo-4-(4-*tert*-butyldimethylsilyloxy-3-methoxyphenyl)isoindoli none (32.8 mg, 0.0662 mmol) was dissolved in acetonitrile (3.3 mL), and the solution was treated with Compound BD (47 mg, 0.13 mmol), palladium acetate (1.5 mg, 0.0066 mmol), tri(o-tolyl)phosphine (4.0 mg, 0.013 mmol) and triethylamine (0.092 mL, 0.66 mmol), followed by purification by preparative thin-layer chromatography (chloroform/methanol=8/1) to obtain 4-(4-*tert*-butyldimethylsilyloxy-3-methoxyphenyl)-7-[1-(*tert*-buto xycarbonyl)-5-(piperidinomethyl)indol-2-yl]isoindolinone (34.7 mg, yield 77%).
APCI-MS *m*/*z*: 682 [M+H]⁺; ¹H-NMR (CDCl₃)δ(ppm): 0.22 (s, 6H), 1. 04 (s, 9H), 1.33 (s, 9H), 1.43 (br s, 2H), 1.63 (br s, 4H), 2.48 (br s, 4H), 3.66 (s, 2H), 3.86 (s, 3H), 4.48 (s, 2H), 6.58 (s, 1H), 6.95 (m, 3H), 7.30 (d, *J* = 8.7 Hz, 1H), 7.51 (d, *J* = 8.1 Hz, 1H), 7.52 (br s, 1H), 7.58 (d, *J* = 7.7 Hz, 1H), 7.62 (br s, 1H), 8.18 (d, *J* = 8.4 Hz, 1H).

### Step 4

In a similar manner to Step 2 of Reference Example 2-5, 4-(4-*tert*-butyldimethylsilyloxy-3-methoxyphenyl)-7-[1-(*tert*-buto xycarbonyl)-5-(piperidinomethyl)indol-2-yl]isoindolinone (34.7 mg, 0.0509 mmol) was dissolved in methanol (1.4 mL), and the solution was treated with 10% hydrogen chloride-methanol solution (1.4 mL). The precipitated solid was collected by filtration and washed with methanol, followed by drying under reduced pressure to obtain Compound 304 (15.9 mg, yield 62%).
¹H-NMR (DMSO-*d*₆)δ(ppm): 1.66-1.77 (m, 6H), 2.86 (m, 2H), 3.31 (m, 2H), 3.83 (s, 3H), 4.31 (s, 2H), 4.62 (s, 2H), 6.90 (d, *J* = 8.4 Hz, 1H), 7.04 (d, *J* = 8.1 Hz, 1H), 7.15 (s, 1H), 7.31 (br s, 2H), 7.55 (d, *J* = 8.2 Hz, 1H), 7.67 (d, *J* = 7.2 Hz, 1H), 7.77 (s, 1H), 8.27 (d, *J* = 7.7 Hz, 1H), 9.28 (s, 1H), 9.41 (s, 1H), 10.05 (br s, 1H), 14.15 (s, 1H).

### Reference Example 2-32:

### 4-(4-Hydroxyphenyl)-7-[1H-5-(piperidinomethyl)indol-2-yl]isoindo linone hydrochloride (Compound 305)

### Step 1

In a similar manner to Step 1 of Reference Example 2-19, 7-amino-4-bromoisoindolinone (200 mg, 0.880 mmol) was dissolved in dimethoxyethane (14 mL), and the solution was treated with 4-(4,4,5,5-tetramethyl-1,3,2-dioxaboran-2-yl)phenol (387 mg, 1.76 mmol), [bis(diphenylphosphino)ferrocene]dichloropalladium (57.4 mg, 0. 0704 mmol), potassium carbonate (607 mg, 4.40 mmol) and water (0.32 mL), followed by purification by slurry using chloroform to obtain 7-amino-4-(4-hydroxyphenyl)isoindolinone (192 mg, yield 91%).
APCI-MS *m*/*z*: 241 [M+H]⁺; ¹H-NMR (DMSO-*d*₆)δ(ppm): 4.34 (s, 2H), 6.10 (s, 2H), 6.62 (d, *J* = 8.4 Hz, 1H), 6.78 (d, *J* = 8.4 Hz, 2H), 7.22 (d, *J* = 8.4 Hz, 1H), 7.26 (d, *J* = 8.6 Hz, 2H), 8.20 (s, 1H), 9.40 (s, 1H).

### Step 2

In a similar manner to Step 1 of Reference Example 2-31, 7-amino-4-(4-hydroxyphenyl)isoindolinone (182 mg, 0.760 mmol) was dissolved in acetonitrile (9.1 mL), and the solution was treated with *tert*-butyldimethylsilyl chloride (206 mg, 1.37 mmol) and DBU (0.205 mL, 1.37 mmol), followed by purification by flash column chromatography (chloroform) to obtain 7-amino-4-(4-*tert*-butyldimethylsilyloxyphenyl)isoindolinone (182 mg, yield 68%).
APCI-MS *m*/*z*: 355 [M+H]⁺; ¹H-NMR (CDCl₃)δ(ppm): 0.23 (s, 6H), 1.00 (s, 9H), 4.44 (s, 2H), 5.93 (br s, 1H), 6.66 (d, *J* = 8.4 Hz, 1H), 6.88 (d, *J* = 8.6 Hz, 2H), 7.24 (d, *J* = 8.8 Hz, 2H), 7.30 (d, *J* = 8.2 Hz, 1H).

### Step 3

In a similar manner to Step 4 of Reference Example 2-17, 7-amino-4-(4-*tert*-butyldimethylsilyloxyphenyl)isoindolinone (179 mg, 0.505 mmol) was dissolved in acetonitrile (12.5 mL), and the solution was treated with potassium iodide (133 mg, 0.808 mmol), copper iodide (152 mg, 0.808 mmol), iodine (203 mg, 0.808 mmol) and tert-butyl nitrate (0.127 mL, 1.06 mmol), followed by purification by flash column chromatography (chloroform) to obtain 7-iodo-4-(4-*tert*-butyldimethylsilyloxyphenyl)isoindolinone (109 mg, yield 47%).
APCI-MS *m*/*z*: 466 [M+H]⁺; ¹H-NMR (CDCl₃)δ(ppm): 0.24 (s, 6H), 1.01 (s, 9H), 4.39 (s, 2H), 6.39 (br s, 1H), 6.92 (d, *J* = 8.6 Hz, 2H), 7.21 (d, *J* = 8.1 Hz, 1H), 7.28 (d, *J* = 8.6 Hz, 2H), 7.98 (d, *J* = 8.1 Hz, 1H).

### Step 4

In a similar manner to Step 2 of Reference Example 2-1, 7-iodo-4-(4-*tert*-butyldimethylsilyloxyphenyl)isoindolinone (59.4 mg, 0.128 mmol) was dissolved in acetonitrile (4.8 mL), and the solution was treated with Compound BD (92.0 mg, 0.256 mmol), palladium acetate (2.3 mg, 0.0102 mmol), tri(o-tolyl)phosphine (6.2 mg, 0.020 mmol) and triethylamine (0.178 mL, 1.28 mmol), followed by purification by preparative thin-layer chromatography (chloroform/methanol=7/1) to obtain 4-(4-*tert*-butyldimethylsilyloxyphenyl)-7-[1-(*tert*-butoxycarbonyl )-5-(piperidinomethyl)indol-2-yl]isoindolinone (53.6 mg, yield 64%).
APCI-MS *m*/*z*: 652 [M+H]⁺; ¹H-NMR (CDCl₃)δ(ppm): 0.26 (s, 6H), 1.02 (s, 9H), 1.30 (s, 9H), 1.44 (br s, 2H), 1.62 (br s, 4H), 2.48 (br s, 4H), 3.66 (s, 2H), 4.47 (s, 2H), 6.67 (s, 1H), 6.94 (d, *J* = 8.6 Hz, 2H), 7.29 (dd, *J* = 1.6, 8.7 Hz, 1H), 7.34 (d, *J* = 8.6 Hz, 2H), 7.49 (d, *J =* 7.8 Hz, 1H), 7.52 (s, 1H), 7.55 (d, *J =* 7.6 Hz, 1H), 7.77 (br s, 1H), 8.19 (d, *J =* 8.6 Hz, 1H).

### Step 5

In a similar manner to Step 2 of Reference Example 2-5, 4-(4-*tert*-butyldimethylsilyloxyphenyl)-7-[1-(*tert*-butoxycarbonyl )-5-(piperidinomethyl)indol-2-yl]isoindolinone (49.2 mg, 0.0755 mmol) was dissolved in methanol (1.5mL), and the solution was treated with 10% hydrogen chloride-methanol solution (1.5 mL). The precipitated solid was collected by filtration and washed with methanol, followed by drying under reduced pressure to obtain Compound 305 (31.5 mg, yield 88%).
APCI-MS *m*/*z*: 438 [M+H]⁺; ¹H-NMR (DMSO-*d*₆)δ(ppm) : 1.77 (m, 6H), 2.86 (m, 2H), 3.30 (m, 2H), 4.31 (d, *J* = 4.6 Hz, 2H), 4.59 (s, 2H), 6.90 (d, *J* = 8.4 Hz, 2H), 7.29 (d, *J* = 7.9 Hz, 1H), 7.30 (s, 1H) 7.45 (d, *J* = 8.4 Hz, 2H), 7.55 (d, *J* = 8.4 Hz, 1H), 7.63 (d, *J* = 8.1 Hz, 1H), 7.76 (s, 1H), 8.26 (d, *J* = 8.3 Hz, 1H), 9.40 (s, 1H), 9.73 (s, 1H), 9.90 (br s, 1H), 14.15 (s, 1H).

### Reference Example 2-33:

### 4-[4-(Hydroxymethyl)phenyl]-7-[1H-5-(piperidinomethyl)indol-2-yl ]isoindolinone hydrochloride (Compound 308)

### Step 1

In a similar manner to Step 1 of Reference Example 2-19, 4-trifluoromethanesulfonyloxy-7-[1-(*tert*-butoxycarbonyl)-5-(pipe ridinomethyl)indol-2-yl]isoindolinone (80.0 mg, 0.135 mmol) was dissolved in dimethoxyethane (9.6 mL), and the solution was treated with 4-(hydroxymethyl)phenylborate (62.0 mg, 0.405 mmol), [bis(diphenylphosphino)ferrocene]dichloropalladium(22 mg, 0.027 mmol), potassium carbonate (93.0 mg, 0.675 mmol) and water (0.098 mL), followed by purification by preparative thin-layer chromatography (chloroform/methanol=6/1) to obtain 4-[4-(hydroxymethyl)phenyl]-7-[1-(*tert*-butoxycarbonyl)-5-(piperi dinomethyl)indol-2-yl]isoindolinone (47.7 mg, yield 64%). APCI-MS *m*/*z*: 552 [M+H]⁺; ¹H-NMR (CDCl₃)δ(ppm) : 1.27 (s, 9H), 1.44 (br s, 2H), 1.61 (br s, 4H), 2.44 (br s, 4H), 3.58 (s, 2H), 4.34 (br s, 2H), 4.77 (s, 2H), 6.53 (s, 1H), 7.24 (d, *J* = 9.6 Hz, 1H), 7.42 (m, 3H), 7.48-7.55 (m, 4H), 8.03 (br s, 1H), 8.14 (d, *J* = 8.6 Hz, 1H).

### Step 2

In a similar manner to Step 2 of Reference Example 2-5, 4-[4-(hydroxymethyl)phenyl]-7-[1-(*tert*-butoxycarbonyl)-5-(piperi dinomethyl)indol-2-yl]isoindolinone (46.0 mg, 0.0834 mmol) was dissolved in methanol (1.6 mL), and the solution was treated with 10% hydrogen chloride-methanol solution (2.3 mL). The precipitated solid was collected by filtration and washed with methanol, followed by drying under reduced pressure to obtain Compound 308 (30.2 mg, yield 74%).
APCI-MS *m*/*z*: 452 [M+H]⁺; ¹H-NMR (DMSO-*d*₆)δ(ppm) : 1.77 (m, 6H), 2.84 (m, 2H), 3.32 (m, 2H), 4.31 (d, *J* = 4.9 Hz, 2H), 4.57 (s, 2H), 4.61 (s, 2H), 7.31 (dd, *J* = 1.4, 8.4 Hz, 1H), 7.33 (s, 1H), 7.45 (d, *J* = 8.2 Hz, 2H), 7.57 (d, *J* = 8.2 Hz, 1H), 7.60 (d, *J* = 8.7 Hz, 2H), 7.68 (d, *J* = 8.2 Hz, 1H), 7.78 (s, 1H), 8.30 (d, *J* = 8.4 Hz, 1H), 9.44 (s, 1H), 10.01 (br s, 1H), 14.16 (s, 1H).

### Reference Example 2-34:

### 4-(4-Ureidophenyl)-7-[1H-5-(piperidinomethyl)indol-2-yl]isoindol inone hydrochloride (Compound 314)

### Step 1

4-(4-Aminophenyl)-7-[1-(*tert*-butoxycarbonyl)-5-(piperidinom ethyl)indol-2-yl]isoindolinone (39.2 mg, 0.0730 mmol) was dissolved in acetic acid (1.57 mL) and water (1.57 mL), and the solution was added with aqueous sodium cyanate solution (0.139 mol/L, 1.57 mL, 0.219 mmol), followed by stirring at 40 °C for 2.3 hours. The reaction mixture was added with saturated aqueous sodium hydrogencarbonate solution and extracted with ethyl acetate. The organic layer was washed with saturated brine and dried over anhydrous sodium sulfate. The solvent was evaporated under reduced pressure and the residue was purified by preparative thin-layer chromatography (chloroform/methanol/7 mol/L ammonia-methanol solution=10/0.5/0.5) to obtain 4-(4-ureidophenyl)-7-[1-(*tert*-butoxycarbonyl)-5-(piperidinomethy 1)indol-2-yl]isoindolinone (23.7 mg, yield 56%).
ESI-MS *m*/*z*: 580 [M+H]⁺; ¹H-NMR (CDCl₃)δ(ppm) : 1. 30 (s, 9H), 1.43 (br s, 2H), 1.56 (br s, 4H), 2.39 (br s, 4H), 3.53 (s, 2H), 4.25 (s, 2H), 4.95 (s, 2H), 6.53 (s, 1H), 7.26-7.46 (m, 10H), 8.14 (d, *J* = 8.4 Hz, 1H).

### Step 2

In a similar manner to Step 2 of Reference Example 2-5, 4-(4-ureidophenyl)-7-[1-(*tert*-butoxycarbonyl)-5-(piperidinomethy 1)indol-2-yl]isoindolinone (20.7 mg, 0.0357 mmol) was dissolved in methanol (1.0 mL), and the solution was treated with 10% hydrogen chloride-methanol solution (1.0 mL). The precipitated solid was collected by filtration and washed with methanol, followed by drying under reduced pressure to obtain Compound 314 (14.6 mg, yield 79%). ¹H-NMR (DMSO-d₆)δ(ppm) : 1.65-1.77 (m, 6H), 2.86 (m, 2H), 3.32 (m, 2H), 4.30 (d, *J* = 4.3 Hz, 2H), 4.61 (s, 2H), 7.30 (m, 2H), 7.48-7.56 (m, 5H), 7.66 (d, *J* = 8.3 Hz, 1H), 7.77 (s, 1H), 8.27 (d, *J* = 8.3 Hz, 1H), 8.95 (s, 1H), 9.42 (s, 1H), 10.00 (br s, 1H), 14.15 (s, 1H).

### Reference Example 2-35:

### 4-(4-Hydroxy-1-butynyl)-7-[1H-5-(piperidinomethyl)indol-2-yl]iso indolinone hydrochloride (Compound 320)

### Step 1

4-Trifluoromethanesulfonyloxy-7-[1-(*tert*-butoxycarbonyl)-5-(piperidinomethyl)indol-2-yl]isoindolinone (80.0 mg, 0.135 mmol) was dissolved in acetonitrile (4.8 mL), and the solution was added with tetrakis(triphenylphosphine)palladium (23.4 mg, 0.0203 mmol), copper iodide (10 mg, 0.054 mmol), tetrabutylammonium chloride (150 mg, 0.405 mmol), 3-butyn-1-ol (0.051 mL, 0.68 mmol) andtriethylamine (0.96 mL, 0.70 mmol), followed by stirring at room temperature for 40.7 hours under argon atmosphere. The reaction mixture was added with water and extracted with ethyl acetate. The organic layer was washed with saturated brine and dried over anhydrous sodium sulfate. The solvent was evaporated under reduced pressure, and the residue was purified by preparative thin-layer chromatography (chloroform/methanol=5/1) to obtain 4-(4-hydroxy-1-butynyl)-7-[1-(*tert*-butoxycarbonyl)-5-(piperidino methyl)indol-2-yl]isoindolinone (35.5 mg, yield 51%).
ESI-MS *m*/*z*: 514 [M+H]⁺; ¹H-NMR (CDCl₃+CD₃OD)δ(ppm) : 1.28 (s, 9H), 1.52 (br s, 2H), 1.75 (br s, 4H), 2.59-2.76 (m, 6H), 3.70-3.91 (m, 4H), 4.46 (s, 2H), 6.58 (s, 1H), 7.34 (d, *J* = 8.6 Hz, 1H), 7.42 (d, *J* = 7.7 Hz, 1H), 7.59 (s, 1H), 7.62 (d, *J* = 7.7 Hz, 1H), 8.23 (d, *J* = 8.6 Hz, 1H).

### Step 2

In a similar manner to Step 2 of Reference Example 2-5, 4-(4-hydroxy-1-butynyl)-7-[1-(*tert*-butoxycarbonyl)-5-(piperidino methyl) indol-2-yl] isoindolinone (34.0 mg, 0.0662 mmol) was dissolved in methanol (1.7 mL), and the solution was added with 10% hydrogen chloride-methanol solution (1.0 mL). The precipitated solid was collected by filtration and washed with methanol, followed by drying under reduced pressure to obtain Compound 320 (11.8 mg, yield 40%). ¹H-NMR (DMSO-*d*₆)δ(ppm) : 1.34 (m, 2H), 1.76 (m, 4H), 2.64 (t, *J* = 6.8 Hz, 2H), 2.85 (m, 2H), 3.33 (m, 2H), 3.62 (t, *J* = 6.8 Hz, 2H), 4.30 (d, *J* = 4.8 Hz, 2H), 4.48 (s, 2H), 7.31 (m, 2H), 7.55 (d, *J* = 8.4 Hz, 1H), 7.64 (d, *J* = 8.3 Hz, 1H), 7.76 (s, 1H), 8.18 (d, *J* = 8.3 Hz, 1H), 9.45 (s, 1H), 9.92 (br s, 1H), 14.03 (s, 1H).

### Reference Example 2-36:

### 4-(3-Hydroxypropyl)-7-[1H-5-(piperidinomethyl)indol-2-yl]isoindo linone hydrochloride (Compound 328)

### Step 1

4-(3-Hydroxy-1-propynyl)-7-[1-(*tert*-butoxycarbonyl)-5-(pipe ridinomethyl)indol-2-yl]isoindolinone (89.7 mg, 0.180 mmol) was dissolved in methanol (5.4 mL), and the solution was added with 10% Pd-C (16.8 mg), followed by stirring at room temperature for 2.7 hours under hydrogen atmosphere at normal pressure. The mixture was added with 10% Pd-C (8.9 mg), and further stirred for 2.5 hours. The reaction mixture was filtered using Celite. The solvent of filtrate was evaporated under reduced pressure to obtain 4-(3-hydroxypropyl)-7-[1-(*tert*-butoxycarbonyl)-5-(piperidinometh yl)indol-2-yl]isoindolinone (84.6 mg, yield 93%).
ESI-MS *m*/*z*: 504 [M+H]⁺; ¹H-NMR (CDCl₃)δ(ppm) : 1.25 (s, 9H), 1.44 (m, 2H), 1.61 (br s, 4H), 1.92 (m, 2H), 2.48 (br s, 4H), 2.79 (t, *J* = 7.8 Hz, 2H), 3.65 (m, 2H), 3.72 (t, *J* = 6.2 Hz, 2H), 4.35 (s, 2H), 6.51 (s, 1H), 7.33 (d, *J* = 8.6 Hz, 1H), 7.42 (m, 2H), 7.49 (s, 1H), 7.78 (s, 1H), 8.25 (d, *J* = 8.6 Hz, 1H).

### Step 2

In a similar manner to Step 2 of Reference Example 2-5, 4-(3-hydroxypropyl)-7-[1-(*tert*-butoxycarbonyl)-5-(piperidinometh yl)indol-2-yl]isoindolinone (84.6 mg, 0.168 mmol) was dissolved in ethanol (2.1 mL), and the solution was treated with 10% hydrogen chloride-methanol solution (3.4 mL). The precipitated solid was collected by filtration and washed with methanol, followed by drying under reduced pressure to obtain Compound 328 (15.6 mg, yield 21%). ¹H-NMR (DMSO-*d*₆)δ(ppm) : 1.66-1.82 (m, 6H), 2.68 (t, *J* = 7.6 Hz, 2H), 3.15 (m, 2H), 3.34 (br s, 4H), 3.44 (t, *J* = 6.2 Hz, 2H), 4.30 (m, 2H), 4.49 (s, 2H), 7.24 (s, 1H), 7.26 (d, *J* = 8.8 Hz, 1H), 7.50 (d, *J* = 7.8 Hz, 1H), 7.53 (d, *J* = 7.9 Hz, 1H), 7.74 (s, 1H), 8.14 (d, *J* = 8.1 Hz, 1H), 9.33 (s, 1H), 9.85 (br s, 1H), 14.06 (s, 1H).

### Reference Example 2-37:

### 4-Hydroxymethyl-7-[1H-5-(piperidinomethyl)indol-2-yl]isoindolino ne hydrochloride (Compound 331)

### Step 1

4-Trifluoromethanesulfonyloxy-7-[1-(*tert*-butoxycarbonyl)-5-(piperidinomethyl)indol-2-yl]isoindolinone (80.0 mg, 0.135 mmol) was dissolved in 1,4-dioxane (2.4 mL), and the solution was added with hydroxymethyltributyltin (87 mg, 0.27 mmol) synthesized in a similar manner to the method described in Synthetic Communications, 1994, vol.24, p.1117, and tetrakis(triphenylphosphine)palladium (12.5 mg, 0.0108 mmol), followed by stirring at 90 °C for 2.7 hours under argon atmosphere. The mixture was further added with tetrakis(triphenylphosphine)palladium (6.3 mg, 0.054 mmol) and stirred for 2.3 hours. Further, the mixture was added with hydroxymethyltributyltin (44 mg, 0.14 mmol), tetrakis(triphenylphosphine)palladium (6.3 mg, 0.0054 mmol) and 1,4-dioxane (1.2 mL), followed by stirring for 4 hours. The reaction mixture was added with 10% aqueous ammonium fluoride solution and extracted with ethyl acetate. The organic layer was washed with saturated brine and dried over sodium sulfate. The solvent was evaporated under reduced pressure. The residue was purified by preparative thin-layer chromatography (chloroform/methanol=10/1) to obtain 4-hydroxymethyl-7-[1-(*tert*-butoxycarbonyl)-5-(piperidinomethyl)i ndol-2-yl]isoindolinone (15.0 mg, yield 23%).
APCI-MS *m*/*z*: 476 [M+H]⁺; ¹H-NMR (CDCl₃)δ(ppm) : 1.23 (s, 9H), 1.48 (br s, 2H), 1.72 (br s, 4H), 2.67 (br s, 4H), 3.83 (s, 2H), 4.29 (s, 2H), 4.71 (s, 2H), 6.38 (s, 1H), 7.35 (m, 1H), 7.37 (d, *J* = 7.7 Hz, 1H), 7.55-7.58 (m, 3H), 8.20 (d, *J* = 8.6 Hz, 1H).

### Step 2

In a similar manner to Step 2 of Reference Example 2-5, 4-hydroxymethyl-7-[1-(*tert*-butoxycarbonyl)-5-(piperidinomethyl)i ndol-2-yl]isoindolinone (32.0 mg, 0.0673 mmol) was dissolved in methanol (1.28 mL), and the solution was treated with 10% hydrogen chloride-methanol solution (1.92 mL). The precipitated solid was collected by filtration and washed with methanol, followed by drying under reduced pressure to obtain Compound 331 (14.9 mg, yield 54%). ¹H-NMR (DMSO-*d*₆)δ(ppm) : 1.67-1.76 (m, 6H), 2.74-2.86 (m, 2H), 3.32 (m, 2H), 4.30 (m, 2H), 4.51 (s, 2H), 4.61 (s, 2H), 7.26 (s, 1H), 7.28 (d, *J* = 7.1 Hz, 1H), 7.54 (d, *J* = 8.1 Hz, 1H), 7.65 (d, *J* = 8.1 Hz, 1H), 7.75 (s, 1H), 8.20 (d, *J* = 8.3 Hz, 1H), 9.32 (s, 1H), 9.85 (br s, 1H), 14.08 (s, 1H).

### Reference Example 2-38:

### 4-Cyano-7-[1H-5-(piperidinomethyl)indol-2-yl]isoindolinone hydrochloride (Compound 332)

### Step 1

4-Trifluoromethanesulfonyloxy-7-[1-(*tert*-butoxycarbonyl)-5-(piperidinomethyl)indol-2-yl]isoindolinone (300 mg, 0.505 mmol) was dissolved in acetonitrile (15 mL), and the solution was added with zinc cyanide (178 mg, 1.52 mmol) and [bis(diphenylphosphino)ferrocene]dichloropalladium (33 mg, 0.0404 mmol), followed by stirrig at 90 °C for 17 hours under argon atmosphere. The reaction mixture was added with water and extracted with ethyl acetate. The organic layer was washed with saturated brine and dried over sodium sulfate. The solvent was evaporated under reduced pressure. The residue was purified by preparative thin-layer chromatography (chloroform/methanol=8/1) to obtain 4-cyano-7-[1-(*tert*-butoxycarbonyl)-5-(piperidinomethyl)indol-2-y 1] isoindolinone (198 mg, yield 83%).
APCI-MS *m*/*z*: 471 [M+H]⁺; ¹H-NMR (CDCl₃)δ(ppm) : 1.42 (s, 9H), 1.58 (br s, 6H), 2.42 (br s, 4H), 3.59 (s, 2H), 4.60 (s, 2H), 6.46 (s, 1H), 6.63 (s, 1H), 7.33 (d, *J* = 8.6 Hz, 1H), 7.52 (s, 1H), 7.60 (d, *J* = 7.9 Hz, 1H), 7.87 (d, *J* = 7.9 Hz, 1H), 8.11 (d, *J* = 8.7 Hz, 1H).

### Step 2

In a similar manner to Step 2 of Reference Example 2-5, 4-cyano-7-[1-(*tert*-butoxycarbonyl)-5-(piperidinomethyl)indol-2-y 1] isoindolinone (52.8 mg, 0.112 mmol) was dissolved in methanol (1.58 mL), and the solution was treated with 10% hydrogen chloride-methanol solution (3.16 mL). The precipitated solid was collected by filtration and washed with methanol, followed by drying under reduced pressure to obtain Compound 332 (27.4 mg, yield 60%).
APCI-MS *m*/*z*: 371 [M+H]⁺; ¹H-NMR (DMSO-*d*₆)δ(ppm) : 1.67-1.77 (m, 6H), 2.83-2.87 (m, 2H), 3.34 (m, 2H), 4.33 (m, 2H), 4.71 (s, 2H), 7.38 (d, *J* = 8.4 Hz, 1H), 7.54 (s, 1H), 7.60 (d, *J* = 8.4 Hz, 1H), 7.83 (s, 1H), 8.11 (d, *J* = 8.2 Hz, 1H), 8.39 (d, *J* = 8.2 Hz, 1H), 9.68 (s, 1H), 10.06 (s, 1H), 14.04 (s, 1H).

### Reference Example 2-39:

### 4-Methanesulfonyloxy-5-methoxy-7-[1H-5-(dimethylaminomethyl)indo l-2-yl]isoindolinone (Compound 362)

### Step 1

In a similar manner to Step 2 of Reference Example 2-1, 4-methanesulfonyloxy-5-methoxy-7-iodoisoindolinone (128 mg, 0.334 mmol) was dissolved in acetonitrile (5 mL), and the solution was treated with Compound BA (193 mg, 0.668 mmol), palladium acetate (6. 0 mg, 0.027 mmol), tri(o-tolyl)phosphine (16.3 mg, 0.0536 mmol) and triethylamine (0.455 mL, 3.34 mmol), followed by purification by slurry using chloroform and diisopropylether to obtain 4-methanesulfonyloxy-5-methoxy-7-[1-(*tert*-butoxycarbonyl)-5-form yl]indolylisoindolinone (127 mg, yield 76%).
ESI-MS *m*/*z*: 501 [M+H]⁺; ¹H-NMR (CDCl₃)δ(ppm) : 1.36 (s, 9H), 3.36 (s, 3H), 4.03 (s, 3H), 4.58 (s, 2H), 6.15 (s, 1H), 6.72 (s, 1H), 7.14 (s, 1H), 7.89 (dd, *J* = 1.3, 8.6 Hz, 1H), 8.10 (d, *J* = 1.3 Hz, 1H), 8.39 (d, *J* = 8.6 Hz, 1H), 10.07 (s, 1H).

### Step 2

In a similar manner to Step 2 of Reference Example 2-4, 4-methanesulfonyloxy-5-methoxy-7-[1-(*tert*-butoxycarbonyl)-5-form yl]indolylisoindolinone (80 mg, 0.160 mmol) was dissolved in acetonitrile (5 mL), and the solution was treated with dimethylamine hydrochloride (260 mg, 3.19 mmol), triethylamine (0.446 ml, 3.20 mmol), acetic acid (0.183 mL, 3.20 mmol) and sodium triacetoxyborohydride (158 mg, 0.744 mmol) to obtain 4-methanesulfonyloxy-5-methoxy-7-[1-(*tert*-butoxycarbonyl)-5-(dim ethylaminomethyl)indol-2-yl]isoindolinone (78.5 mg, yield 93%).
ESI-MS *m*/*z*: 530 [M+H]⁺; ¹H-NMR (CDCl₃)δ(ppm) : 1.18 (s, 9H), 2.15 (s, 6H), 3.47 (s, 2H), 3.49 (s, 3H), 3.98 (s, 3H), 4.40 (s, 2H), 6.70 (s, 1H), 7.27 (dd, *J* = 1.5, 8.5 Hz, 1H), 7.31 (s, 1H), 7.50 (d, *J* = 1.5 Hz, 1H), 8.09 (d, *J* = 8.5 Hz, 1H), 8.56 (s, 1H).

### Step 3

In a similar manner to Step 2 of Reference Example 2-5, 4-methanesulfonyloxy-5-methoxy-7-[1-(*tert*-butoxycarbonyl)-5-(dim ethylaminomethyl)indol-2-yl]isoindolinone (78.0 mg, 0.147 mmol) was dissolved in methanol (1.5 mL), and the solution was treated with 10% hydrogen chloride-methanol solution (1.5 mL). The reaction mixture was added with saturated aqueous sodium hydrogencarbonate solution and extracted with ethyl acetate. The organic layer was washed with saturated brine and dried over anhydrous sodium sulfate. The solvent was evaporated under reduced pressure and the residue was purified by slurry using diisopropylether to obtain Compound 362 (33.3 mg, yield 53%).
mp 232-234 °C; ¹H-NMR (DMSO-*d*₆)δ(ppm) : 2.16 (s, 6H), 3.45 (s, 2H), 3.53 (s, 3H), 4.08 (s, 3H), 4.52 (s, 2H), 7.11 (dd, *J* = 0.9, 8.6 Hz, 1H), 7.37 (d, *J* = 0.9 Hz, 1H), 7.41 (d, *J* = 8.6 Hz, 1H), 7.46 (s, 1H), 7.83 (s, 1H), 9.24 (s, 1H), 13.80 (s, 1H).

### Reference Example 2-40:

### 4-Methanesulfonyloxy-5-methoxy-7-[1H-5-(2-methoxyethylaminomethy 1)indol-2-yl]isoindolinone (Compound 374)

### Step 1

In a similar manner to Step 2 of Reference Example 2-4, 4-methanesulfonyloxy-5-methoxy-7-[1-(*tert*-butoxycarbonyl)-5-form ylindol-2-yl]isoindolinone (1.02 g, 2.66 mmol) was dissolved in acetonitrile (13.0 mL), and the solution was treated with 2-methoxyethylamine (3.47 mL, 39.9 mmol), acetic acid (2.28 mL, 13.3 mmol) and sodium triacetoxyborohydride (2.82 g, 39.9 mmol), followed by purification by flash column chromatography (chloroform/methanol=4/1) to obtain 4-methanesulfonyloxy-5-methoxy-7-[1-(*tert*-butoxycarbonyl)-5-(2-m ethoxyethylaminomethyl)indol-2-yl]isoindolinone (0.83 g, 56%).
ESI-MS *m*/*z*: 560 [M+H]⁺; ¹H-NMR (CDCl₃)δ(ppm) : 1.34 (s, 9H), 2.83 (t, *J* = 5.4 Hz, 2H), 3.32 (s, 3H), 3.34 (s, 3H), 3.54 (t, *J* = 5.4 Hz, 2H), 3.97 (s, 2H), 4.00 (s, 3H), 4.56 (s, 2H), 6.56 (s, 1H), 6.77 (br s, 1H), 7.10 (s, 1H), 7.23 (dd, *J* = 1.8, 6.9 Hz, 1H), 7.52 (d, *J* = 1.8 Hz, 1H), 8.20 (d, *J* = 6.9 Hz, 1H).

### Step 2

4-Methanesulfonyloxy-5-methoxy-7-[1-(*tert*-butoxycarbonyl)-5 -(2-methoxyethylaminomethyl)indol-2-yl]isoindolinone (1.58 g, 2.82 mmol) was dissolved in 10% hydrogen chloride-methanol solution (30.0 mL), and the solution was stirred under reflux for 1.5 hours. The solvent was evaporated under reduced pressure and the residue was added with water, followed by extracting with ethyl acetate/methanol (9/1). The organic layer was washed with saturated brine and dried over anhydrous sodium sulfate. The solvent was evaporated under reduced pressure and the residue was purified by slurry using diisopropylether and methanol to obtain Compound 374 (837 mg, yield 64%).
mp 188-190 °C; ¹H-NMR (DMSO-*d*₆)δ(ppm) : 2.67 (t, *J* = 5.9 Hz, 2H), 3.23 (s, 3H), 3.41 (t, *J* = 5.9 Hz, 2H), 3.53 (s, 3H), 3.77 (s, 2H), 4.08 (s, 3H), 4.52 (s, 2H), 7.13 (d, *J* = 8.4 Hz, 1H), 7.37 (s, 1H), 7.40 (d, *J* = 8.4 Hz, 1H), 7.50 (s, 1H), 7.83 (s, 1H), 9.23 (s, 1H), 14.1 (s, 1H).

### Reference Example 2-41:

### 4-Methanesulfonyloxy-5-methoxy-7-[1H-5-(pyrrol-1-yl)indol-2-yl]i soindolinone (Compound 404)

4-Methanesulfonyloxy-5-methoxy-7-[1*H*-5-aminoindol-2-yl]isoi ndolinone (0.0830 g, 0.196 mmol) was dissolved in methanol (2.0 mL), and the solution was added with a solution of sodium borohydride (0.148 g, 3.92 mmol), 2,5-dimethoxytetrahydrofuran (0.127 mL, 0.979 mmol) and 2 mol/L sulfuric acid (0.979 mL, 2.45 mmol) in THF (2.0 mL), followed by stirring at room temperature for 4 hours. The reaction mixture was added with water and the precipitated crystal was collected by filtration. The crude product was purified by slurry using DMSO and water to obtain Compound 404 (0.0151 g, yield 180).
¹H-NMR (DMSO-*d*₆)δ(ppm) : 3.53 (s, 3H), 4.09 (s, 3H), 4.53 (s, 2H), 6.24 (t, *J* = 2.4 Hz, 2H), 7.29 (t, *J* = 2.4 Hz, 2H), 7.34 (dd, *J* = 2.1, 8.7 Hz, 1H), 7.45 (s, 1H), 7.57 (d, *J* = 8.7 Hz, 1H), 7.67 (d, *J* = 2.1 Hz, 1H), 7.86 (s, 1H), 9.26 (s, 1H), 14.0 (s, 1H).

### Reference Example 2-42:

### 4-Chloro-5-ethyl-7-[1H-5-(piperidin-1-ylmethyl)indol-2-yl]isoind olinone hydrochloride (Compound 426)

### Step 1

4-Chloro-5-trifluoromethanesulfonyloxy-7-[1-(*tert*-butoxycar bonyl)-5-(piperidin-1-ylmethyl)indol-2-yl]isoindolinone (93.5 mg, 0.149 mmol) was dissolved in dimethoxyethane (4.7 mL), and the solution was added with 1.1 mol/L diethyl zinc-toluene solution (0.68 mL, 0.744 mmol) and [bis(diphenylphosphino)ferrocene]dichloropalladium (34.4 mg, 0.030 mmol), followed by stirring at 90 °C for 12 hours under argon atmosphere. The reaction mixture was added with water and filtered using Celite. The filtrate was extracted with ethyl acetate. The organic layer was washed with saturated brine, followed by drying over sodium sulfate. The solvent was evaporated under reduced pressure and the residue was purified by preparative thin-layer chromatography (chloroform/methanol=6/1) to obtain 4-chloro-5-ethyl-7-(1-(*tert*-butoxycarbonyl)-5-(piperidin-1-ylmet hyl)indol-2-yl]isoindolinone (39.0 mg, yield 52%).
ESI-MS *m*/*z*: 508 [M+H]⁺; ¹H-NMR (CDCl₃)δ(ppm) : 1.24-1.33 (m, 13H), 1.44 (br s, 1H), 1.61-1.65 (m, 4H), 2.53 (br s, 4H), 2.88 (q, *J* = 7.5 Hz, 2H), 3.72 (s, 2H), 4.38 (s, 2H), 6.55 (s, 1H), 7.26-7.68 (m, 4H), 8.15 (d, *J* = 8.4 Hz, 1H).

### Step 2

In a similar manner to Step 2 of Reference Example 2-5, 4-chloro-5-ethyl-7-[1-(*tert*-butoxycarbonyl)-5-(piperidin-1-ylmet hyl)indol-2-yl] isoindolinone (36.8 mg, 0. 072 mmol) was dissolved in methanol (0.5 mL), and the solution was treated with 10% hydrogen chloride methanol solution (0.5 mL). The reaction mixture was added with diisopropylether. The obtained solid was collected by filtration and washed with diisopropylether, followed by drying under reduced pressure to obtain Compound 426 (7.4 mg, yield 23%). ¹H-NMR (DMSO-*d*₆)δ(ppm) : 1.24-1.34 (m, 4H), 1.66-1.76 (m, 5H), 2.83-2.90 (m, 4H), 3.28 (br s, 2H), 4.38 (d, *J* = 4.5 Hz, 2H), 4.47 (s, 2H), 7.32 (dd, *J* = 1.2, 8.4 Hz, 1H), 7.37 (d, *J* = 1.2 Hz, 1H), 7.54 (d, *J* = 8.4 Hz, 1H), 7.78 (s, 1H), 8.12 (s, 1H), 9.46 (s, 1H), 10.11 (br s, 1H), 13.88 (s, 1H).

### Reference Example 2-43:

### 4-Chloro-5-(3-hydroxypropoxy)-7-[1H-5-(piperidin-1-ylmethyl)indo 1-2-yl]isoindolinone hydrochloride (Compound 428)

### Step 1

In a similar manner to Step 1 of Reference Example 2-18, 4-chloro-5-hydroxy-7-[1-(*tert*-butoxycarbonyl)-5-(piperidinomethy 1)indol-2-yl]isoindolinone (65.7 mg, 0.136 mmol) was dissolved in THF (3.5 mL); and the solution was treated with triphenylphosphine (178 mg, 0.680 mmol), 3-(*tert*-butyldimethylsiloxy)-1-propanol (0.145 mL, 0.680 mmol) and 40% DEAD-toluene solution (0.310 mL, 0.680 mmol), followed by purification by preparative thin-layer chromatography (chloroform/methanol=6/1) to obtain 4-chloro-5-[3-(*tert*-butyldimethylsiloxy)propoxy]-7-[1-(*tert*-buto xycarbonyl)-5-(piperidin-1-ylmethyl)indol-2-yl]isoindolinone (58.2 mg, yield 64%).
APCI-MS *m*/*z*: 668 [M+H]⁺; ¹H-NMR (CDCl₃)δ(ppm) : 0.04 (s, 6H), 0.87 (s, 9H), 1.26-1.43 (m, 11H), 1.58-1.61 (m, 4H), 2.03-2.11 (m, 2H), 2.44 (br s, 4H), 3.62 (s, 2H), 3.86 (t, *J* = 5.7 Hz, 2H), 4.24 (t, *J* = 6.0 Hz, 2H), 4.36 (s, 2H), 6.55 (s, 1H), 7.05 (s, 1H), 7.26-7.36 (m, 2H), 7.50 (d, *J* = 0.9 Hz, 1H), 8.16 (d, *J* = 8.7 Hz, 1H).

### Step 2

In a similar manner to Step 2 of Reference Example 2-5, 4-chloro-5-[3-(*tert*-butyldimethylsiloxy)propoxy]-7-[1-(*tert-*buto xycarbonyl)-5-(piperidin-1-ylmethyl)indol-2-yl]isoindolinone (58.0 mg, 0.087 mmol) was dissolved in methanol (0.5 mL), and the solution was treated with 10% hydrogen chloride-methanol solution (0.5 mL). The reaction mixture was added with diisopropylether. The obtained solid was collected by filtration and washed with diisopropylether, followed by drying under reduced pressure to obtain Compound 428 (22.6 mg, yield 53%).
ESI-MS *m*/*z*: 454 [M+H]⁺; ¹H-NMR (DMSO-*d*₆)δ(ppm): 1.27-1.40 (m, 1H), 1.66-1.76 (m, 5H), 1.91-1.99 (m, 2H), 2.77-2.89 (m, 2H), 3.28 (br s, 2H), 3.60-3.65 (m, 2H), 4.30 (d, *J* = 4.5 Hz, 2H), 4.39 (t, *J* = 6.0 Hz, 2H), 4.44 (s, 2H), 4.68 (br s, 1H), 7.32 (dd, *J* = 1.2, 8.4 Hz, 1H), 7.48 (d, *J* = 1.2 Hz, 1H), 7.54 (d, J = 8.4 Hz, 1H), 7.80 (s, 1H), 7.82 (s, 1H), 9.31 (s, 1H), 10.14 (br s, 1H), 14.01 (s, 1H).

### Reference Example 2-44:

### 4-Chloro-5-(2-hydroxyethoxy)-7-[1H-5-(piperidin-1-ylmethyl)indol -2-yl]isoindolinone hydrochloride (Compound 429)

### Step 1

4-Chloro-5-hydroxy-7-iodoisoindolinone (100 mg, 0.323 mmol) was dissolved in DMF (2.0 mL), and the solution was added with potassium carbonate (134 mg, 0.969 mmol), tetrabutylammonium iodide (11.9 mg, 0.032 mmol) and *tert*-butyl(2-bromoethoxy)dimethylsilane (0.083 mL, 0.388 mmol), followed by stirring at 50 °C for 7 hours under nitrogen atmosphere. The reaction mixture was added with water and extracted with ethyl acetate. The organic layer was washed with saturated brine and dried over sodium sulfate. The solvent was evaporated under reduced pressure and the residue was purified by flash column chromatography (chloroform/methanol=19/1) to obtain 4-chloro-5-[3-(*tert*-butyldimethylsiloxy)ethoxy]-7-iodoisoindolin one (57.7 mg, yield 38%).
ESI-MS *m*/*z*: 467 [M+H]⁺; ¹H-NMR (CDCl₃)δ(ppm) : 0.11 (s, 6H), 0.91 (s, 9H), 4.04 (t, *J* = 4.5 Hz, 2H), 4.21 (t, *J* = 4.5 Hz, 2H). 4.29 (s, 2H), 6.28 (br s, 1H), 7.50 (s, 1H).

### Step 2

In a similar manner to Step 2 of Reference Example 2-1, 4-chloro-5-[3-(*tert*-butyldimethylsiloxy)ethoxy]-7-iodoisoindolin one (57.7 mg, 0.123 mmol) was dissolved in acetonitrile (1.8 mL), and the solution was treated with Compound BD (88.4 mg, 0.246 mmol), palladium acetate (2.2 mg, 0.0098 mmol), tri(o-tolyl)phosphine (6.0 mg, 0.017 mmol) and triethylamine (0.171 mL, 1.23 mmol), followed by purification by flash column chromatography (chloroform/methanol=9/1) to obtain 4-chloro-5-[3-(*tert*-butyldimethylsiloxy)ethoxy]-7-[1-(*tert*-butox ycarbonyl)-5-(piperidin-1-ylmethyl)indol-2-yl]isoindolinone (79.9 mg, yield 99%).
APCI-MS *m*/*z*: 654 [M+H]⁺; ¹H-NMR (CDCl₃)δ(ppm) : 0.12 (s, 6H), 0.90 (s, 9H), 1.24-1.47 (m, 11H), 1.58-1.61 (m, 4H), 2.43 (br s, 4H), 3.61 (s, 2H), 4.06 (t, *J* = 5.1 Hz, 2H), 4.21 (t, *J* = 5.1 Hz, 2H), 4.37 (s, 2H), 6.56 (s, 1H), 7.07 (s, 1H), 7.10 (br s, 1H), 7.30 (dd, *J* = 1.5, 8.7 Hz, 1H), 7.50 (d, *J* = 1.5 Hz, 1H), 8.14 (d, *J* = 8.7 Hz, 1H).

### Step 3

In a similar manner to Step 2 of Reference Example 2-5, 4-chloro-5-[3-(*tert*-butyldimethylsiloxy)ethoxy]-7-[1-(*tert*-butox ycarbonyl)-5-(piperidin-1-ylmethyl)indol-2-yl]isoindolinone (58.0 mg, 0.087 mmol) was dissolved in methanol (0.5 mL), and the solution was treated with 10% hydrogen chloride-methanol solution (0.5 mL). The obtained solid was collected by filtration and washed with methanol, followed by drying under reduced pressure to obtain Compound 429 (34.8 mg, yield 61%).
¹H-NMR (DMSO-*d*₆)δ(ppm) : 1.27-1.37 (m, 1H), 1.66-1.76 (m, 5H), 2.77-2.89 (m, 2H), 3.28 (br s, 2H), 3.82 (t, *J* = 4.8 Hz, 2H), 4.31 (d, *J* = 4.5 Hz, 2H), 4.36 (t, *J* = 4.8 Hz, 2H), 4.45 (s, 2H), 5.02 (br s, 1H), 7.31 (d, *J* = 8.4 Hz, 1H), 7.49 (br s, 1H), 7.55 (d, *J* = 8.4 Hz, 1H), 7.79 (s, 1H), 7.83 (s, 1H), 9.31 (s, 1H), 10.04 (br s, 1H), 14.00 (s, 1H).

### Example 1:

### 5-Hydroxy-7-[1H-5-(piperidinomethyl)indol-2-yl]isoindolinone hydrochloride (Compound 468)

### Step 1

4-Hydroxybenzoic acid (2.00 g, 14.5 mmol) was suspended in dichloromethane (50 mL), and the suspension was added with diisopropylethylamine (10.1 mL, 57.9 mmol) and chloromethyl methylether (2.20 mL, 29.0 mmol) under ice-cooling. Then, the reaction mixture was warmed to room temperature, followed by stirring for 2.5 hours. The reaction mixture was added with chloromethyl methylether (1.10 mL, 14.5 mmol) under ice-cooling, followed by stirring at room temperature for 17 hours. The solvent was evaporated under reduced pressure, then the reaction mixture was added with methanol (50 mL) and 4 mol/L aqueous potassium hydroxide solution (50 mL) followed by stirring at room temperature for 3 hours. The solvent was evaporated under reduced pressure, and the residue was added with 4 mol/L hydrochloric acid and water, followed by extracting with ethyl acetate. The organic layer was washed with saturated brine and dried over anhydrous sodium sulfate. The solvent was evaporated under reduced pressure to obtain 4-methoxymethoxybenzoic acid (2.63 g, yield 100%).
ESI-MS *m*/*z*: 181 [M-H]⁻; ¹H-NMR (CDCl₃)δ(ppm) : 3.37 (s, 3H), 5.26 (s, 2H), 7.00-7.16 (m, 2H), 7.80-7.96 (m, 2H), 12.65 (br s, 1H).

### Step 2

4-Methoxymethoxybenzoic acid (2.63 g, 14.4 mmol) was dissolved in DMF (52 mL), and the solution was added with EDCI (5.54 g, 28.9 mmol) and HOBt monohydrate (2.21 g, 14.4 mmol), and cumylamine (4.20 mL, 29.1 mmol) was added thereto under ice-cooling, follwed by stirring at room temperature for 2 hours. The reaction mixture was added with water, and the precipitated solid was collected by filtration. The solid was washed with water and dried under reduced pressure to obtain 4-methoxymethoxy-*N*-(1-methyl-1-phenylethyl)benzamide (3.37 g, yield 78%).
ESI-MS *m*/*z*: 300 [M+H]⁺; ¹H-NMR (CDCl₃)δ(ppm) : 1.82 (s, 6H), 3.47 (s, 3H), 5.21 (s, 2H), 6.31 (s, 1H), 6.98 (d, *J* = 8.5 Hz, 1H), 7.20-7.53 (m, 5H), 7.72 (d, *J* = 8.5 Hz, 1H).

### Step 3

4-Methoxymethoxy-*N*-(1-methyl-1-phenylethyl)benzamide (3.37 g, 11.3 mmol) was dissolved in THF (130 mL), and the solution was added with TMEDA (5.40 mL, 35.8 mmol). To the solution, sec-buthyl lithium-hexane solution (1.01 mol/L, 36.0 mL, 36.4 mmol) was added dropwise at -78 °C for 20 minutes under argon atmosphere, followed by stirring at same temperature for 1.5 hours. Then, the solution was added with DMF (1.90 mL, 24.5 mmol) at -78 °C, and warmed to room temperature for 2 hours. The reaction mixture was added with water, and extracted with ethyl acetate. The organic layer was washed with saturated brine and dried over anhydrous sodium sulfate. The solvent was evaporated under reduced pressure and the residue was purified by slurry using diisopropylether to obtain 5-methoxymethoxy-3-hydroxy-2-(1-methyl-1-phenylethyl)isoindolino ne (3.15 g, yield 86%).
ESI-MS *m*/*z*: 328 [M+H]⁺; ¹H-NMR (CDCl₃)δ(ppm) : 1.92 (s, 3H), 1.96 (s, 3H), 2.41 (d, *J* = 9.3 Hz, 1H), 3.48 (s, 3H), 5.20 (d, *J* = 9.0 Hz, 1H), 5.22 (d, *J* = 9.0 Hz, 1H), 6.08 (d, *J* = 9.3 Hz, 1H), 7.09 (dd, *J* = 2.1, 8.2 Hz, 1H), 7.16-7.47 (m, 6H), 7.59 (d, *J* = 8.2 Hz, 1H).

### Step 4

5-Methoxymethoxy-3-hydroxy-2-(1-methyl-1-phenylethyl)isoind olinone (3.15 g, 9.62 mmol) was dissolved in THF (110 mL), and the solution was added with TMEDA (4.60 mL, 30.5 mmol). To the solution, sec-buthyl lithium-hexane solution (0.95 mol/L, 32.0 mL, 30.4 mmol) was added dropwise at -78 °C for 15 minutes under argon atmosphere, followed by stirring at same temperature for 1.5 hours. Then, the solution was added with a solution of iodine (2.93 g, 11.5 mmol) in THF (25 mL), followed by warming to room temperature for 2.5 hours. The reaction mixture was added with saturated aqueous sodium thiosulfate solution, and extracted with ethyl acetate. The organic layer was washed with saturated brine and dried over anhydrous sodium sulfate. The solvent was evaporated under reduced pressure, and the residue was purified by flush column chromatography (hexane/ethyl acetate=80/20, 60/40, 50/50) to obtain 5-methoxymethoxy-3-hydroxy-7-iodo-2-(1-methyl-1-phenylethyl)isoi ndolinone (1.23 g, yield 28%).
ESI-MS *m*/*z*: 454 [M+H]⁺; ¹H-NMR (CDCl₃)δ(ppm) : 1.94 (s, 3H), 1.98 (s, 3H), 2.17 (d, *J* = 10.2 Hz, 1H), 3.47 (s, 3H), 5.19 (d, *J* = 10.9 Hz, 1H), 5.21 (d, *J* = 10.9 Hz, 1H), 5.92 (d, J = 10.2 Hz, 1H), 7.15-7.49 (m, 6H), 7.57 (d, *J* = 2.0 Hz, 1H).

### Step 5

5-Methoxymethoxy-3-hydroxy-7-iodo-2-(1-methyl-1-phenylethyl )isoindolinone (1.00 g, 2.21 mmol) was dissolved in nitromethane (40 mL), and the solution was added with trifluoroacetic acid (0.511 mL, 6.63 mmol) and triethylsilane (1.06 mL, 6.63 mmol) followed by stirring at room temperature for 1 hour. The reaction mixture was added with water and saturated aqueous sodium hydrogencarbonate solution, and extraced with ethyl acetate. The organic layer was washed with saturated brine and dried over anhydrous sodium sulfate. The solvent was evaporated under reduced pressure to obtain 5-hydroxy-7-iodo-2-(1-methyl-1-phenylethyl)isoindolinone (1.16g).

### Step 6

5-Hydroxy-7-iodo-2-(1-methyl-1-phenylethyl)isoindolinone (1.16 g) was dissolved in 10% hydrogen chloride-methanol solution (34 mL), and the solution stirred at 70 °C for 2.5 hours. Then, the solution was further added with 10% hydrogen chloride-methanol solution (11 mL) followed by stirring for 30 minutes. The reaction mixture was added with water and extracted with ethyl acetate. The organic layer was washed with saturated brine and dried over anhydrous sodium sulfate. The solvent was evaporated under reduced pressure and the residue was purified by slurry using diisopripylether to obtain 5-hydroxy-7-iodoisoindolinone (569 mg, yield 94%, 2 steps). ESI-MS *m*/*z*: 276 [M+H]⁺; ¹H-NMR (CDCl₃)δ(ppm) : 4.24 (s, 2H), 6.87 (s, 1H), 7.39 (s, 1H).

### Step 7

5-Hydroxy-7-iodoisoindolinone (47.0 mg, 0.171 mmol) was dissolved in acetonitrile (2 mL), and the solution was added with Compound BD (122 mg, 0.342 mmol), palladium acetate (3.1 mg, 0.014 mmol), tri(o-tolyl)phosphine (8.3 mg, 0.027 mmol) and triethylamine (0.238 mL, 1.71 mmol), followed by stirring under reflux for 2 hours, under argon atmosphere. The reaction mixture was added with water and extracted with ethyl acetate. The organic layer was washed with saturated brine and dried over anhydrous sodium sulfate. The solvent was evaporated under reduced pressure and the residue was purified by preparative thin-layer chromatography (chloroform/methanol=9/1) to obtain 5-hydroxy-7-[1-(*tert*-butoxycarbonyl)-5-piperidinomethyl]indol-2-yl]isoindolinone (52.1 mg, yield 66%).
ESI-MS *m*/*z*: 462 [M+H]⁺; ¹H-NMR (DMSO-*d*₆)δ(ppm) : 1.27 (s, 9H), 1.38-1.71 (m, 6H), 2.08-2.62 (m, 4H), 3.49 (s, 2H), 4.22 (s, 2H), 5.81 (s, 1H), 6.20 (br s, 1H), 6.51 (d, *J* = 1.6 Hz, 1H), 6.66 (s, 1H), 7.21 (dd, *J* = 1.6, 8.7 Hz, 1H), 7.36 (s, 1H), 8.15 (d, *J* = 8.6 Hz, 1H).

### Step 8

5-Hydroxy-7-[1-(*tert*-butoxycarbonyl)-5-piperidinomethyl]ind ol-2-yl] isoindolinone (50.3 mg, 0.109 mmol) was dissolved in methanol (1.0 mL), and the solution was added with 10% hydrogen chloride-methanol solution (1.0 mL), followed by stirring at 60 °C for 6 hours. The reaction mixture was cooled to room temperature and added with diisopropylether. The precipitated solid was collected by filtration and washed with diisopropylether, then dried under reduced pressure to obtain Compound 468 (33.9 mg, yield 86%).
ESI-MS *m*/*z*: 362 [M+H]⁺; ¹H-NMR (DMSO-*d*₆)δ(ppm) : 1.34 (m, 1H), 1.54-1.88 (m, 5H), 2.75-2.90 (m, 2H), 3.20-3.40 (m, 2H), 4.30 (d, *J* = 4.3 Hz, 2H), 4.41 (s, 2H), 6.90 (s, 1H), 7.15 (s, 1H), 7.26 (d, *J* = 8.4 Hz, 1H), 7.53 (m, 1H), 7.74 (s, 1H), 8.96 (s, 1H), 9.69 (br s, 1H), 10.43 (s, 1H), 14.26 (s, 1H).

### Example 2:

### 5-Methanesulfonyloxy-7-{1H-5-[4-(2-hydroxyethyl)piperazin-1-ylme thyl)indol-2-yl}isoindolinone dihydrochloride (Compound 469)

### Step 1

In a similar manner to Step 7 of Example 1, 5-hydroxy-7-iodoisoindolinone (50.7 mg, 0.184 mmol) was dissolved in acetonitrile (2.5 mL), and the solution was treated with Compound BU (186 mg, 0.359 mmol), palladium acetate (3.2 mg, 0.014 mmol), tri(o-tolyl)phosphine (8.8 mg, 0.029 mmol) and triethylamine (0.251 mL, 1.80 mmol) followed by purification by slurry using hexane to obtain 5-hydroxy-7-(1-(*tert*-butoxycarbonyl)-5-{4-[2-(tert-butyldimethyl silyloxy)ethyl]piperazin-1-ylmethyl}indol-2-yl)isoindolinone (104 mg, yield 93%).
ESI-MS *m*/*z*: 621 [M+H]⁺; ¹H-NMR (CDCl₃)δ(ppm) : 0. 04 (s, 6H), 0.87 (s, 9H), 1.30 (s, 9H), 2.40-2.68 (m, 10H), 3.59 (br s, 2H), 3.75 (t, *J* = 6.3 Hz, 2H), 4.27 (s, 2H), 5.94 (s, 1H), 6.15 (s, 1H), 6.61 (s, 1H), 6.71 (s, 1H), 7.24 (m, 1H), 7.35 (s, 1H), 8.17 (d, *J* = 8.8 Hz, 1H).

### Step 2

5-Hydroxy-7-(1-(*tert*-butoxycarbonyl)-5-{4-[2-(tert-butyldim ethylsilyloxy)ethyl]piperazin-1-ylmethyl}indol-2-yl)isoindolinon e (50.6 mg, 0.0815 mmol) was dissolved in dichloromethane (2 mL), and the solution was added with triethylamine (0.045 mL, 0.80 mmol), then added with methanesulfonylchloride (0. 012 mL, 0.16 mmol) at 0°C, followed by stirring at room temperature for 1 hour. The reaction mixture was added with water and extracted with ethyl acetate. The organic layer was washed with saturated brine and dried over anhydrous sodium sulfate. The solvent was evaporated under reduced pressure and the residue was purified by slurry using hexane to obtain 5-methanesulfonyloxy-7-(1-(*tert*-butoxycarbonyl)-5-{4-[2-(tert-bu tyldimethylsilyloxy)ethyl]piperazin-1-ylmethyl}indol-2-yl)isoind olinone (38.2 mg, yield 68%).
ESI-MS *m*/*z*: 699 [M+H]⁺; ¹H-NMR (CDCl₃)δ(ppm) : 0. 05 (s, 6H), 0. 88 (s, 9H), 1.34 (s, 9H), 2.54 (t, *J* = 6.3 Hz, 2H), 2.40-2.66 (m, 8H), 3.24 (s, 3H), 3.59 (s, 2H), 3.76 (t, *J* = 6.3 Hz, 2H), 4.46 (s, 2H), 6.56 (br s, 1H), 6.59 (s, 1H), 7.32 (d, *J* = 8.9 Hz, 1H), 7.35 (d, *J* = 2.0 Hz, 1H), 7.43 (d, *J* = 2.0 Hz, 1H), 7.50 (s, 1H), 8.16 (d, *J* = 8.9 Hz, 1H).

### Step 3

In a similar manner to Step 8 of Example 1, 5-methanesulfonyloxy-7-(1-(*tert*-butoxycarbonyl)-5-{4-[2-(tert-bu tyldimethylsilyloxy)ethyl]piperazin-1-ylmethyl}indol-2-yl)isoind olinone (48.0 mg, 0.0650 mmol) was dissolved in methanol (2 mL), and the solution was treated with 10% hydrogen chloride-methanol solution (2 mL). The precipitated solid was collected by filtration and washed with diisopropylether, then dried under reduced pressure to obtain Compound 469 (14.0 mg, yield 50%).
ESI-MS *m*/*z*: 485 [M+H]⁺; ¹H-NMR (DMSO-*d*₆)δ(ppm) : 3.05-3.71 (m, 10H), 3.53 (s, 3H), 3.75 (s, 2H), 4.44 (br s, 2H), 4.56 (s, 2H), 7.37 (m, 1H), 7.44 (S, 1H), 7.55 (s, 1H), 7.59 (d, *J* = 8.1 Hz, 1H), 7.85 (s, 1H), 8.12 (s, 1H), 9.44 (s, 1H), 14.10 (s, 1H).

### Example 3:

### 5-Methoxy-7-{1H-5-[4-(2-hydroxyethyl)piperazin-1-ylmethyl)indol-2-yl}isoindolinone dihydrochloride (Compound 470)

### Step 1

5-Hydroxy-7-iodoisoindolinone (50.5 mg, 0.184 mmol) was dissolved in DMF (2 mL), and the solution was added with potassium carbonate (102 mg, 0.736 mmol) and iodomethane (0.012 mL, 0.19 mmol), followed by stirring at room temperature for 5 hours. The reaction mixture was added with iodomethane (0.012 mL, 0.19 mmol) and stirred for 1 hour, followed by adding with water. The precipitated solid was collected by filtration and washed with water, followed by drying under reduced pressure to obtain 5-methoxy-7-iodoisoindolinone (33.7 mg, yield 64%).
APCI-MS *m*/*z*: 290 [M+H]⁺; ¹H-NMR (CDCl₃)δ(ppm) : 3.87 (s, 3H), 4.30 (s, 2H), 6.33 (br s, 1H), 6.94 (d, *J* = 2.2 Hz, 1H), 7.46 (d, *J* = 2.2 Hz, 1H).

### Step 2

In a similar manner to Step 7 of Example 1, 5-methoxy-7-iodoisoindolinone (31.5 mg, 0.109 mmol) was suspended in acetonitrile (2 mL), and the suspension was treated with Compound BU (113 mg, 0.218 mmol), palladium acetate (2.0 mg, 0.0089 mmol), tri(o-tolyl)phosphine (6.0 mg, 0.020 mmol) and triethylamine (0.152 mL, 1.09 mmol), followed by purification using preparative thin-layer chromatography (chloroform/methanol=9/1) to obtain 5-methoxy-7-(1-(tert-butoxycarbonyl)-5-{4-[2-(tert-butyldimethyl silyloxy)ethyl]piperazin-1-ylmethyl}indol-2-yl)isoindolinone (52.2 mg, yield 75%).
ESI-MS *m*/*z*: 635 [M+H]⁺; ¹H-NMR (CDCl₃)δ(ppm) : 0. 05 (s, 6H), 0. 88 (s, 9H), 1.35 (s, 9H), 2.40-2.65 (m, 8H), 2.53 (t, *J* = 6.4 Hz, 2H), 3.58 (s, 2H), 3.75 (t, *J* = 6.4 Hz, 2H), 3.90 (s, 3H), 4.39 (s, 2H), 6.17 (br s, 1H), 6.54 (s, 1H), 6.93 (d, *J* = 1.8 Hz, 1H), 7.00 (d, *J* = 1.8 Hz, 1H), 7.28 (d, *J* = 8.6 Hz, 1H), 7.49 (s, 1H), 8.16 (d, *J* = 8.6 Hz, 1H).

### Step 3

In a similar manner to Step 8 of Example 1, 5-methoxy-7-(1-(tert-butoxycarbonyl)-5-{4-[2-(tert-butyldimethyl silyloxy)ethyl]piperazin-1-ylmethyl}indol-2-yl)isoindolinone (50.0 mg, 0.0788 mmol) was dissolved in methanol (3 mL), and the solution was treated with 10% hydrogen chloride-methanol solution (3 mL). The reaction mixture was added with diisopropylether. The obtained solid was collected by filtration and washed with diisopropylether, then dried under reduced pressure to obtain Compound 470 (35.8 mg, yield 92%).
ESI-MS *m*/*z*: 421 [M+H]⁺; ¹H-NMR (DMSO-*d*₆)δ(ppm) : 3.10-3.83 (m, 12H), 3.91 (s, 3H), 4.45 (br s, 4H), 7.12 (d, *J* = 2.2 Hz, 1H), 7.33 (d, *J* = 8.4 Hz, 1H), 7.35 (s, 1H), 7.54 (d, *J* = 8.4 Hz, 1H), 7.68 (d, *J* = 2.2 Hz, 1H), 7.80 (br s, 1H), 9.08 (s, 1H), 10.96 (br s, 1H), 11.65 (br s, 1H), 14.21 (s, 1H).

### Example 4:

### 5-Dimethylamino-7-[1H-5-(piperidinomethyl)indol-2-yl]isoindolino ne dihydrochloride (Compound 471)

### Step 1

In a similar manner to Step 2 of Example 1, 4-dimethylaminobenzoic acid (5.00 g, 30.3 mmol) was dissolved in DMF (100 mL), and the solution was treated with EDCI (11.6 g, 60.5 mmol), HOBt monohydrate (4.64 g, 30.3 mmol) and cumylamine (8.71 mL, 60.5 mmol) The reaction mixture was added with water. The precipitated solid was collected by filtration and washed by water, followed by drying under reduced pressure to obtain 4-dimethylamino-N-(1-methyl-1-phenylethyl)benzamide (8.02 g, yield 94%).
ESI-MS *m*/*z*: 283 [M+H]⁺; ¹H-NMR (CDCl₃)δ(ppm) : 1.81 (s, 6H), 3.01 (s, 6H), 6.26 (br s, 1H), 6.60-6.72 (m, 2H), 7.19-7.60 (m, 5H), 7.65-7.79 (m, 2H).

### Step 2

In a similar manner to Step 3 of Example 1, 4-dimethylamino-N-(1-methyl-1-phenylethyl)benzamide (8.02 g, 28.4 mmol) was dissolved in THF (307 mL), and the solution was treated with TMEDA (13.7 mL, 90.8 mmol), sec-butyl lithium haxane solution (1.00 mol/L, 91. 0 mL, 91. 0 mmol) and DMF (4. 60 mL, 59.4 mmol), followed by purification by slurry using chloroform to obtain 5-dimethylamino-3-hydroxy-2-(1-methyl-1-phenylethyl)isoindolinon e (4.49 g, yield 51%).
APCI-MS *m*/*z*: 311 [M+H]⁺; ¹H-NMR (CDCl₃)δ(ppm) : 1.91 (s, 3H), 1.96 (s, 3H), 2.25 (d, *J* = 10.9 Hz, 1H), 3.05 (s, 6H), 6.65 (d, *J* = 10.9 Hz, 1H), 6.69-6.80 (m, 2H), 7.16-7.45 (m, 5H), 7.52 (d, *J* = 8.4 Hz, 1H).

### Step 3

In a similar manner to Step 4 of Example 1, 5-dimethylamino-3-hydroxy-2-(1-methyl-1-phenylethyl)isoindolinon e (4.49 g, 14.5 mmol) was dissolved in THF (168 mL), and the solution was treated with TMEDA (7.00 mL, 46.4 mmol), sec-butyl lithium haxane solution (1.00 mol/L, 46.3 mL, 46.3 mmol) and iodine (5.51 g, 21.7 mmol), followed by purification by flush column chromatography (haxane/ethyl acetate=80/20, 70/30) to obtain 5-dimethylamino-3-hydroxy-7-iodo-2-(1-methyl-1-phenylethyl)isoin dolinone (3.57 g, yield 57%).
ESI-MS *m*/*z*: 437 [M+H]⁺; ¹H-NMR (CDCl₃)δ(ppm) : 1. 92 (s, 3H), 1.97 (s, 3H), 2.11 (d, *J* = 10.4 Hz, 1H), 3.03 (s, 6H), 5.86 (d, *J* = 10.4 Hz, 1H), 6.73 (d, *J* = 2.1 Hz, 1H), 7.12 (d, *J* = 2.1 Hz, 1H), 7.17-7.48 (m, 5H).

### Step 4

5-Dimethylamino-3-hydroxy-7-iodo-2-(1-methyl-1-phenylethyl) isoindolinone (3.57 g, 8.18 mmol) was dissolved in nitromethane (71 mL), and the solution was added with trifluoroacetic acid (12.6 mL, 163 mmol) and triethylsilane (2.60 mL, 16.1 mmol) followed by stirring at 50 °C for 3 hours. The reaction mixture was added with water and saturated aqueous sodium hydrogencarbonate solution and extraced with ethyl acetate. The organic layer was washed with saturated brine and dried over anhydrous sodium sulfate. The solvent was evaporated under reduced pressure and the residue was purified by slurry using hexane to obtain 5-dimethylamino-7-iodoisoindolinone (1.66 g, yield 67%).
APCI-MS *m*/*z*: 302 [M+H]⁺; ¹H-NMR (CDCl₃)δ(ppm) : 3.04 (s, 6H), 4.25 (s, 2H), 6.37 (br s, 1H), 6.63 (d, *J* = 2.1 Hz, 1H), 7.17 (d, *J* = 2.1 Hz, 1H).

### Step 5

In a similar manner to Step 7 of Example 1, 5-dimethylamino-7-iodoisoindolinone (40.0 mg, 0.132 mmol) was dissolved in acetonitrile (2 mL), and the solution was treated with Compound BD (95.0 mg, 0.265 mmol), palladium acetate (2.4 mg, 0.011 mmol), tri(o-tolyl)phosphine (6.4 mg, 0.021 mmol) and triethylamine (0.184 mL, 1.32 mmol), followed by purification by slurry using hexane to obtain 5-dimethylamino-7-[1-(tert-butoxycarbonyl)-5-(piperidinomethyl)i ndol-2-yl]isoindolinone (64.7 mg, yield 100%).
ESI-MS *m*/*z*: 489 [M+H]⁺; ¹H-NMR (DMSO-*d*₆)δ(ppm) : 1.35 (s, 9H), 1.43-1.70 (m, 6H), 2.30-2.50 (m, 4H), 3.07 (s, 6H), 3.58 (s, 2H), 4.35 (s, 2H), 8.92 (s, 1H), 6.55 (s, 1H), 6.64 (d, *J* = 2.1 Hz, 1H), 6.76 (d, *J* = 2.1 Hz, 1H), 7.27 (m, 1H), 7.46 (s, 1H), 8.15 (d, *J* = 8.3 Hz, 1H).

### Step 6

In a similar manner to Step 8 of Example 1, 5-dimethylamino-7-[1-(tert-butoxycarbonyl)-5-(piperidinomethyl)i ndol-2-yl]isoindolinone (62.0 mg, 0.127 mmol) was dissolved in methanol (2 mL), and the solution was treated with 10% hydrogen chloride-methanol solution (2 mL). The obtained solid was collected by filtration and washed with diisopropylether, then dried under reduced pressure to obtain Compound 471 (52.8 mg, yield 90%).
ESI-MS *m*/*z*: 389 [M+H]⁺; ¹H-NMR (DMSO-*d*₆)δ(ppm) : 1.21-1.88 (m, 6H), 2.80-2.94 (m, 2H), 3.10 (s, 6H), 3.20-3.40 (m, 2H), 4.32 (d, *J* = 4.9 Hz, 2H), 4.41 (s, 2H), 6.82 (d, *J* = 2.2 Hz, 1H), 7.25 (dd, *J* = 1.5, 8.3 Hz, 1H), 7.32 (s, 1H), 7.38 (d, *J* = 2.2 Hz, 1H), 7.54 (d, *J* = 8.3 Hz, 1H), 7.74 (s, 1H), 8.76 (s, 1H), 9.66 (br s, 1H), 14.37 (s, 1H).

### Example 5:

### 5-Dimethylamino-7-{1H-5-[4-(2-hydroxyetyl)piperazin-1-ylmethyl]i ndol-2-yl}isoindolinone trihydrochloride (Compound 472)

### Step 1

In a similar manner to Step 7 of Example 1, 5-dimethylamino-7-iodoisoindolinone (79.7 mg, 0.264 mmol) was dissolved in acetonitrile (4 mL), and the solution was treated with Compound BU (273 mg, 0.528 mmol), palladium acetate (4.8 mg, 0.021 mmol), tri(o-tolyl)phosphine (12.9 mg, 0.042 mmol) and triethylamine (0.368 mL, 2.64 mmol), followed·by purification by flush column chromatography (hexane/ethyl acetate=60/40, 50/50, 0/100, chloroform/methanol=9/1) to obtain 5-dimethylamino-7-(1-(tert-butoxycarbonyl)-5-{4-[2-(tert-butyldi methylsilyloxy)etyl]piperazin-1-ylmethyl}indol-2-yl)isoindolinon e (155 mg, yield 88%) .
ESI-MS *m*/*z*: 648 [M+H]⁺; ¹H-NMR (CDCl₃)δ(ppm) : 0.05 (s, 6H), 0.88 (s, 9H), 1.35 (s, 9H), 2.43-2.64 (m, 8H), 2.53 (t, *J* = 6.5 Hz, 2H), 3.07 (s, 6H), 3.58 (s, 2H), 3.75 (t, *J* = 6.5 Hz, 2H), 4.35 (s, 2H), 5.87 (br s, 1H), 6.55 (s, 1H), 6.64 (s, 1H), 6.76 (d, *J* = 2.0 Hz, 1H), 6.76 (d, *J* = 2.0 Hz, 1H), 7.27 (dd, *J* = 1.5, 8.6 Hz, 1H), 7.47 (d, *J* = 1.5 Hz, 1H), 8.15 (d, *J* = 8.6 Hz, 1H).

### Step 2

In a similar manner to Step 8 of Example 1, 5-dimethylamino-7-(1-(tert-butoxycarbonyl)-5-{4-[2-(tert-butyldi methylsilyloxy)etyl]piperazin-1-ylmethyl}indol-2-yl)isoindolinon e (152 mg, 0.228 mmol) was dissolved in methanol (6 mL), and the solution was treated with 10% hydrogen chloride-methanol solution (6 mL). The obtained solid was collected by filtration and washed with diisopropylether, then dried under reduced pressure to obtain Compound 472 (110 mg, yield 89%).
ESI-MS *m*/*z*: 434 [M+H]⁺; ¹H-NMR (DMSO-*d*₆)δ(ppm) : 3. 09 (s, 6H), 3. 15-4. 12 (m, 12H), 4.39 (s, 2H), 4.46 (br s, 2H), 6.82 (s, 1H), 7.31 (d, *J* = 8.4 Hz, 1H), 7.32 (d, *J* = 2.0 Hz, 1H), 7.39 (d, *J* = 2.0 Hz, 1H), 7.53 (d, *J* = 8.4 Hz, 1H), 7.83 (s, 1H), 8.77 (s, 1H), 11.00 (br s, 1H), 11.80 (br s, 1H), 14.37 (s, 1H).

### Example 6:

### 5-(Dimethylaminomethyl)-7-[1H-5-(piperidinomethyl)indol-2-yl]iso indolinone (Compound 473)

### Step 1

In a similar manner to Step 2 of Example 1, 4-carboxybenzaldehyde (5.00 g, 33.3 mmol) was dissolved in DMF (50 mL), and the solution was treated with EDCI (12.8 g, 66.6 mmol), HOBt monohydrate (5.10 g, 33.3 mmol) and cumylamine (9.58 mL, 66.6 mmol). The reaction mixture was added with water. The precipitated solid was collected by filtration and washed by water, followed by drying under reduced pressure to obtain 4-folmyl-N-(1-methyl-1-phenylethyl)benzamide (7.03 g, yield 79%).
ESI-MS *m*/*z*: 268 [M+H]⁺; ¹H-NMR (CDCl₃)δ(ppm) : 1.85 (s, 6H), 6.46 (br s, 1H), 7.26 (m, 1H), 7.32-7.40 (m, 2H), 7.42-7.50 (m, 2H), 7.88-7.98 (m, 4H), 10.07(s, 1H).

### Step 2

4-Folmyl-N-(1-methyl-1-phenylethyl)benzamide (7.03 g, 26.3 mmol) was dissolved in methanol (70 mL), and the solution was added with trimethyl orthoformate (5.75 mL, 52.6 mmol) and p-toluenesulfonic acid monohydrate (45 mg, 0.263 mmol) followed by stirring at room temperature for 1.5 hours. The reaction mixture was added with saturated aqueous sodium hydrogencarbonate solution and water, followed by cooling at 0 °C. The precipitated solid was collected by filtration and washed with water, followed by drying under reduced pressure to obtain 4-(dimethoxymethyl)-N-(1-methyl-1-phenylethyl)benzamide (7.97 g, yield 97%)
ESI-MS *m*/*z*: 314 [M+H]⁺; ¹H-NMR (CDCl₃)δ(ppm) : 1. 83 (s, 6H), 3.31 (s, 6H), 5.44 (s, 1H), 6.39 (br s, 1H), 7.25 (m, 1H), 7.32-7.40 (m, 2H), 7.43-7.55 (m, 4H), 7.74-7.79 (m, 2H).

### Step 3

In a similar manner to Step 3 of Example 1, 4-(dimethoxymethyl)-N-(1-methyl-1-phenylethyl)benzamide (7.97 g, 25. 0 mmol) was dissolved in THF (240 mL), and the solution was treated with TMEDA (12.1 mL, 80.2 mmol), sec-butyl lithium hexane solution (0.95 mol/L, 84.0 mL, 79.8 mmol) andDMF (4.25 mL, 54.9 mmol), followed by purification by slurry using diisopropylether and hexane to obtain 5-(dimethoxymethyl)-3-hydroxy-2-(1-methyl-1-phenylethyl)isoindol inone (7.87 g, yield 92%).
ESI-MS *m*/*z*: 342 [M+H]⁺; ¹H-NMR (CDCl₃)δ(ppm) : 1.93 (s, 3H), 1.98 (s, 3H), 2.52 (br s, 1H), 3.34 (s, 6H), 5.42 (s, 1H), 6.16 (br s, 1H), 7.23 (m, 1H), 7.28-7.37 (m, 2H), 7.39-7.46 (m, 2H), 7.35 (dd, *J* = 1.3, 7.9 Hz, 1H), 7.65 (d, *J* = 1.3 Hz, 1H), 7.77 (d, *J* = 7.9 Hz, 1H).

### Step 4

In a similar manner to Step 4 of Example 1, 5-(dimethoxymethyl)-3-hydroxy-2-(1-methyl-1-phenylethyl)isoindol inone (3.00 g, 8.79 mmol) was dissolved in THF (120 mL), and the solution was treated with TMEDA (4.20 mL, 27.8 mmol), sec-butyl lithium hexane solution (0.95 mol/L, 29.6 mL, 28.1 mmol) and iodine (3.35 g, 13.2 mmol), followed by purification by flush column chromatography (hexane/ethyl acetate=90/10, 70/30) to obtain 5-(dimethoxymethyl)-3-hydroxy-7-iodo-2-(1-methyl-1-phenylethyl)i soindolinone (2.75 g, yield 67%).
ESI-MS *m*/*z*: 468 [M+H]⁺; ¹H-NMR (CDCl₃)δ(ppm) : 1.96 (s, 3H), 1.99 (s, 3H), 2.23 (d, *J* = 10.1 Hz, 1H), 3.33 (s, 3H), 3.34 (s, 3H), 5.38 (s, 1H), 5.98 (d, *J* = 10.1 Hz, 1H), 7.22 (m, 1H), 7.30-7.37 (m, 2H), 7.41-7.47 (m, 2H), 7.61 (d, *J* = 0.6 Hz, 1H), 8.02 (d, *J* = 0.6 Hz, 1H).

### Step 5

5-(Dimethoxymethyl)-3-hydroxy-7-iodo-2-(1-methyl-1-phenylet hyl) isoindolinone (57.0 mg, 0. 122 mmol) was dissolved in acetonitrile (2 mL), and the solution was added with 1 mol/L hydrochloric acid (1 mL) followed by stirring at room temperature for 1 hour. The reaction mixture was added with water and extraced with ethyl acetate. The organic layer was washed with saturated brine and dried over anhydrous sodium sulfate. The solvent was evaporated under reduced pressure to obtain 5-formyl-3-hydroxy-7-iodo-2-(1-methyl-1-phenylethyl)isoindolinon e (62.7 mg).

### Step 6

5-Formyl-3-hydroxy-7-iodo-2-(1-methyl-1-phenylethyl)isoindo linone (62.7 mg) was dissolved in acetonitrile (5 mL), and the solution was added with dimethylamine hydrochloride (199 mg, 2.44 mmol), acetic acid (0.140 mL, 2.45 mmol) and triethylamine (0.340 mL, 2.44 mmol) followed by stirring at room temperature for 30 minutes. The mixture was added with sodium triacetoxyborohydride (78.0 mg, 218 mmol) followed by stirring at room temperature for 1 hour. The reaction mixture was added with water and sodium carbonate to adjust the pH to 9. Then, the extracted with ethyl acetate. The organic layer was washed with saturated brine and dried over anhydrous sodium sulfate. The solvent was evaporated under reduced pressure to obtain 5-(dimethylaminomethyl)-3-hydroxy-7-iodo-2-(1-methyl-1-phenyleth yl)isoindolinone (65.2 mg).

### Step 7

5-(Dimethylaminomethyl)-3-hydroxy-7-iodo-2-(1-methyl-1-phen ylethyl) isoindolinone (65.2 mg) was dissolved in nitromethane (1 mL), and the solution was added with trifluoroacetic acid (0.188 mL, 2.44 mmol) and triethylsilane (0.099 mL, 0.61 mmol) followed by stirring at room temperature for 12 hours. The reaction mixture was added with water and saturated aqueous sodium hydrogencarbonate solution, and extracted with ethyl acetate. The organic layer was washed with water and dried over anhydrous sodium sulfate. The solvent was evaporated under reduced pressure, and the residue was dissolved in ethyl acetate followed by adding with hexane. The obtained solid was collected by filtration and washed with hexane, followed by drying under reduced pressure to obtain 5-(dimethylaminomethyl)-7-iodoisoindolinone (28.9 mg, yield 75%, 3 steps).
ESI-MS *m*/*z*: 317 [M+H]⁺; ¹H-NMR (CDCl₃)δ(ppm) : 2.27 (s, 6H), 3.46 (s, 2H), 4.33 (s, 2H), 6.47 (br s, 1H), 7.44 (s, 1H), 7.88 (s, 1H).

### Step 8

In a similar manner to Step 7 of Example 1, 5-(dimethylaminomethyl)-7-iodoisoindolinone (27.5 mg, 0.0870 mmol) was dissolved in acetonitrile (3 mL), and the solution was treated with Compound BD (62.3 mg, 0.174 mmol), palladium acetate (1.6 mg, 0.0071 mmol), tri(o-tolyl)phosphine (4.2 mg, 0.014 mmol) and triethylamine (0.119 mL, 0.873 mmol), followed by purification by slurry using hexane to obtain 5-(dimethylaminomethyl)-7-[1-(tert-butoxycarbonyl)-5-(piperidino methyl}indol-2-yl)isoindolinone (51.1 mg).

### Step 9

5-(Dimethylaminomethyl)-7-[1-(tert-butoxycarbonyl)-5-(piper idinomethyl}indol-2-yl)isoindolinone (51.1 mg) was dissolved in methanol (1.5 mL), and the solution was added with 10% hydrogen chloride-methanol solution (1.5 mL) followed by stirring at 60 °C for 5 hours. The reaction mixture was added with water and saturated aqueous sodium hydrogencarbonate solution, and extracted with ethyl acetate. The organic layer was washed with saturated brine and dried over anhydrous sodium sulfate. The solvent was evaporated under reduced pressure, and the residue was purified by slurry using diisopropyl ether to obtain Compound 473 (21.4 mg, yield 61%, 2 steps)
ESI-MS *m*/*z*: 403 [M+H]⁺; ¹H-NMR (DMSO-*d*₆)δ(ppm) : 1.33-1.56 (m, 6H), 2.21 (s, 6H), 2.28-2.42 (m, 4H), 3.48 (br s, 2H), 3.55 (s, 2H), 4.49 (s, 2H), 7.08 (dd, *J* = 0.9, 8.4 Hz, 1H), 7.16 (t, *J* = 0.9 Hz, 1H), 7.39 (d, *J* = 8.4 Hz, 1H), 7.42 (s, 1H), 7.45 (s, 1H), 8.04 (s, 1H), 9.22 (s, 1H), 13.88 (s, 1H).

### Example 7:

### 5-(Methoxymethyl)-7-[1H-5-(piperidinomethyl)indol-2-yl]isoindoli none (Compound 474)

### Step 1

In a similar manner to Step 7 of Example 6, 5-(dimethoxymethyl)-3-hydroxy-7-iodo-2-(1-methyl-1-phenylethyl)i soindolinone (100 mg, 0.214 mmol) was dissolved in nitromethane (2 mL), and the solution was treated with trifluoroacetic acid (0.330 mL, 4.28 mmol) and triethylsilane (0.346 mL, 2.14 mmol) followed by purification by slurry using hexane to obtain 5-(methoxymethyl)-7-iodoisoindolinone (59.0 mg, yield 91%).
ESI-MS *m*/*z*: 303 [M+H]⁺; ¹H-NMR (CDCl₃)δ(ppm) : 3.44 (s, 3H), 4.35 (s, 2H), 4.50 (s, 2H), 6.86 (br s, 1H), 7.45 (d, *J* = 0.5 Hz, 1H), 7.88 (d, *J* = 0.5 Hz, 1H).

### Step 2

In a similar manner to Step 7 of Example 1, 5-(methoxymethyl)-7-iodoisoindolinone (50.6 mg, 0.167 mmol) was dissolved in acetonitrile (3 mL), and the solution was treated with Compound BD (120 mg, 0.334 mmol), palladium acetate (3.0 mg, 0.013 mmol), tri(o-tolyl)phosphine (8.1 mg, 0.027 mmol) and triethylamine (0.233 mL, 1.67 mmol), followed by purification by slurry using hexane to obtain 5-(methoxymethyl)-7-[1-(tert-butoxycarbonyl)-5-(piperidinomethyl )indol-2-yl]isoindolinone (72.6 mg, yield 89%).
ESI-MS *m*/*z*: 490 [M+H]⁺; ¹H-NMR (CDCl₃)δ(ppm) : 1.35 (s, 9H), 1.21-1.72 (m, 6H), 2.27-2.54 (m, 4H), 3.46 (s, 3H), 3.58 (s, 2H), 4.43 (s, 2H), 4.59 (s, 2H), 6.40 (br s, 1H), 6.56 (s, 1H), 7.14-7.62 (m, 4H), 8.15 (d, *J* = 8.4 Hz, 1H).

### Step 3

In a similar manner to Step 9 of Example 6, 5-(methoxymethyl)-7-[1-(tert-butoxycarbonyl)-5-(piperidinomethyl )indol-2-yl]isoindolinone (70.6 mg, 0.144 mmol) was dissolved in methanol (2.5 mL), and the solution was treated with 10% hydrogen chloride-methanol solution (2.5 mL), followed by purification by slurry using diisopropylether to obtain Compound 474 (30.2 mg, yield 54%).
ESI-MS *m*/*z:* 390 [M+H]⁺; ¹H-NMR (DMSO-*d*₆)δ(ppm) : 1.28-1.64 (m, 6H), 2.20-2.50 (m, 4H), 3.37 (s, 3H), 3.51 (br s, 2H), 4.49 (s, 2H), 4.58 (s, 2H), 7.09 (d, *J* = 8.2 Hz, 1H), 7.18 (s, 1H), 7.35-7.56 (m, 3H), 8.06 (s, 1H), 9.23 (s, 1H), 13.88 (s, 1H).

### Example 8:

### 5-[(2-Hydroxyethyl)aminomethyl)]-7-[1H-5-(piperidinomethyl)indol -2-yl]isoindolinone dihydrochloride (Compound 475)

### Step 1

In a similar manner to Step 5 of Example 6, 5-(dimethoxymethyl)-3-hydroxy-7-iodo-2-(1-methyl-1-phenylethyl)i soindolinone (100 mg, 0.214 mmol) was dissolved in acetonitrile (2 mL), and the solution was treated with 1 mol/L hydrochloric acid (1 mL) to obtain 5-formyl-3-hydroxy-7-iodo-2-(1-methyl-1-phenylethyl)isoindolinon e (113 mg).

### Step 2

In a similar manner to Step 6 of Example 6, 5-formyl-3-hydroxy-7-iodo-2-(1-methyl-1-phenylethyl)isoindolinon e (113 mg) was dissolved in acetonitrile (4 mL), and the solution was treated with ethanolamine (0.129 mL, 2.14 mmol), acetic acid (0.245 mL, 4.28 mmol) and sodium triacetoxyborohydride (136 mg, 0.642 mmol) to obtain 5-[(2-hydroxyethyl)aminomethyl]-3-hydroxy-7-iodo-2-(1-methyl-1-p henylethyl)isoindolinone (100 mg).

### Step 3

In a similar manner to Step 7 of Example 6, 5-[(2-hydroxyethyl)aminomethyl]-3-hydroxy-7-iodo-2-(1-methyl-1-p henylethyl)isoindolinone (100 mg) was dissolved in nitromethane (2 mL), and the solution was treated with trifluoroacetic acid (0.330 mL, 4.28 mmol) and triethylsilane (0.173 mL, 1.07 mmol) followed by purification by slurry using chloroform and diisopropylether to obtain 5-[(2-hydroxyethyl)aminomethyl]-7-iodoisoindolinone (57.3 mg, yield 81%, 3 steps).
ESI-MS *m*/*z*: 333 [M+H]⁺; ¹H-NMR (DMSO-*d*₆)δ(ppm) : 2.75 (t, *J* = 5.6 Hz, 2H), 3.50-3.60 (m, 2H), 3.97 (s, 2H), 4.28 (s, 2H), 4.82 (br s, 1H), 7.60 (s, 1H), 7.96 (s, 1H), 8.74 (br s, 1H).

### Step 4

In a similar manner to Step 7 of Example 1, 5-[(2-hydroxyethyl)aminomethyl]-7-iodoisoindolinone (49.4 mg, 0.149 mmol) was dissolved in acetonitrile (4 mL), and the solution was treated with Compound BD (107 mg, 0.299 mmol), palladium acetate (2.7 mg, 0.012 mmol), tri(o-tolyl)phosphine (7.3 mg, 0.024 mmol) and triethylamine (0.203mL, 1.49 mmol), followed by purification by flush column chromatography (chloroform/methanol=90/10, 80/20, 70/30, 50/50) to obtain 5-[(2-hydroxyethyl)aminomethyl]-7-[1-(tert-butoxycarbonyl)-5-(pi peridinomethyl}indol-2-yl)isoindolinone (53.1 mg, yield 69%).
ESI-MS *m*/*z*: 519 [M+H]⁺; ¹H-NMR (CDCl₃)δ(ppm) : 1.32 (s, 9H), 1.38-1.40 (m, 2H), 1.52-1.64 (m, 4H), 2.36-2.46 (m, 4H), 2.86 (t, *J* = 5.1 Hz, 2H), 3.58 (s, 2H), 3.70 (t, *J* = 5.1 Hz, 2H), 3.95 (s, 2H), 4.40 (s, 2H), 6.55 (s, 1H), 6.81 (brs, 1H), 7.28 (dd, *J* = 1.4, 8.5 Hz, 1H), 7.40 (s, 1H), 7.44 (s, 1H), 7.48 (d, *J* = 1.4 Hz, 1H), 8.15 (d, *J* = 8.5 Hz, 1H).

### Step 5

In a similar manner to Step 8 of Example 1, 5-[(2-hydroxyethyl)aminomethyl]-7-[1-(tert-butoxycarbonyl)-5-(pi peridinomethyl}indol-2-yl)isoindolinone (52.0 mg, 0.100 mmol) was dissolved in methanol (1 mL), and the solution was treated with 10% hydrogen chloride-methanol solution (1 mL). The obtained solid was collected by filtration and washed with diisopropylether, then dried under reduced pressure to obtain Compound 475 (31.0 mg, yield 63%).
ESI-MS *m*/*z*: 418 [M+H]⁺; ¹H-NMR (DMSO-*d*₆)δ(ppm) : 1.35 (m, 1H), 1.62-1.88 (m, 5H), 2.77-2.96 (m, 2H), 3.00-3.10 (m, 2H), 3.25-3.45 (m, 2H), 3.70-3.80 (m, 2H), 4.28-4.40 (m, 4H), 4.55 (s, 2H), 5.30 (br s, 1H), 7.32 (dd, *J* = 1.0, 8.3 Hz, 1H), 7.40 (d, *J* = 1.0 Hz, 1H), 7.57 (d, *J* = 8.3 Hz, 1H), 7.64 (s, 1H), 7.82 (s, 1H), 8.55 (s, 1H), 9.44 (s, 1H), 9.51 (br s, 2H), 9.99 (br s, 1H), 14.05 (s, 1H).

### Example 9:

### 5-(Piperidinomethyl)-7-[1H-5-(piperidinomethyl)indol-2-yl]isoind olinone dihydrochloride (Compound 476)

### Step 1

In a similar manner to Step 5 of Example 6, 5-(dimethoxymethyl)-3-hydroxy-7-iodo-2-(1-methyl-1-phenylethyl)i soindolinone (100 mg, 0.214 mmol) was dissolved in acetonitrile (2 mL), and the solution was treated with 1 mol/L hydrochloric acid (1 mL) to obtain 5-formyl-3-hydroxy-7-iodo-2-(1-methyl-1-phenylethyl)isoindolinon e (103 mg).

### Step 2

In a similar manner to Step 6 of Example 6, 5-formyl-3-hydroxy-7-iodo-2-(1-methyl-1-phenylethyl)isoindolinon e (103 mg) was dissolved in acetonitrile (2 mL), and the solution was treated with piperidine (0.340 mL, 4.28 mmol), acetic acid (0.245 mL, 4.28 mmol) and sodium triacetoxyborohydride (136 mg, 0.642 mmol) to obtain 5-(piperidinomethyl)-3-hydroxy-7-iodo-2-(1-methyl-1-phenylethyl) isoindolinone (154 mg)

### Step 3

In a similar manner to Step 7 of Example 6, 5-(piperidinomethyl)-3-hydroxy-7-iodo-2-(1-methyl-1-phenylethyl) isoindolinone (154 mg) was dissolved in nitromethane (2 mL), and the solution was treated with trifluoroacetic acid (0.330 mL, 4.28 mmol) and triethylsilane (0.173 mL, 1.07 mmol) followed by purification by flush column chromatography (hexane/ethyl acetate=95/5, 70/30, 50/50 and 0/100) to obtain 5-(piperidinomethyl)-7-iodo-2-(1-methyl-1-phenylethyl)isoindolin one (83.4 mg, yield 82%, 3 steps).
ESI-MS *m*/*z*: 475 [M+H]⁺; ¹H-NMR (CDCl₃)δ(ppm) : 1.40-1.50 (m, 2H), 1.52-1.62 (m, 4H), 1.94 (s, 6H), 2.32-2.41 (m, 4H), 3.45 (s, 2H), 4.21 (s, 2H), 7.28-7.39 (m, 5H), 7.84 (s, 1H).

### Step 4

In a similar manner to Step 6 of Example 1, 5-(piperidinomethyl)-7-iodo-2-(1-methyl-1-phenylethyl)isoindolin one (82.0 mg, 0.173 mmol) was treated with 10% hydrogen chloride-methanol solution (4 mL), followed by purification by slurry using hexane to obtain 5-(piperidinomethyl)-7-iodoisoindolinone (49.8 mg, yield 81%).
ESI-MS *m*/*z*: 357 [M+H]⁺; ¹H-NMR (CDCl₃)δ(ppm) : 1.34-1.44 (m, 2H), 1.45-1.55 (m, 4H), 2.29-2.37 (m, 4H), 3.47 (s, 2H), 4.26 (s, 2H), 7.50 (d, *J* = 0.3 Hz, 1H), 7.81 (d, *J* = 0.3 Hz, 1H), 8.66 (s, 1H).

### Step 5

In a similar manner to Step 7 of Example 1, 5-(piperidinomethyl)-7-iodoisoindolinone (45.0 mg, 0.126 mmol) was dissolved in acetonitrile (2 mL), and the solution was treated with Compound BD (90.3 mg, 0.252 mmol), palladium acetate (2.3 mg, 0.010 mmol), tri(o-tolyl)phosphine (6.1 mg, 0.020 mmol) and triethylamine (0.172 mL, 1.26 mmol), followed by purification by preparative thin-layer chromatography (chloroform/methanol/7 mol/L ammonia-methanol solution=24/1/1) to obtain 5-(piperidinomethyl)-7-[1-(tert-butoxycarbonyl)-5-(piperidinomet hyl)indol-2-yl]isoindolinone (84.7 mg).

### Step 6

In a similar manner to Step 8 of Example 1, 5-(piperidinomethyl)-7-[1-(tert-butoxycarbonyl)-5-(piperidinomet hyl)indol-2-yl]isoindolinone (84.7 mg) was dissolved in methanol (2 mL), and the solution was treated with 10% hydrogen chloride-methanol solution (2 mL). The obtained solid was collected by filtration and washed with diisopropylether, then dried under reduced pressure to obtain Compound 476 (59.6 mg, yield 92%, 2 steps).
ESI-MS *m*/*z:* 443 [M+H]⁺; ¹H-NMR (DMSO-*d*₆)δ(ppm) : 1.29-1.46 (m, 2H), 1.63-1.92 (m, 10H), 2.79-2.97 (m, 4H), 3.27-3.44 (m, 4H), 4.32 (d, *J* = 5.0 Hz, 2H), 4.42 (d, *J* = 4.8 Hz, 2H), 4.56 (s, 2H), 7.31 (dd, *J* = 1.1, 8.5 Hz, 1H), 7.45 (d, *J* = 1.1 Hz, 1H), 7.58 (d, *J* = 8.5 Hz, 1H), 7.66 (s, 1H), 7.81 (s, 1H), 8.67 (s, 1H), 9.47 (s, 1H), 9.84 (br s, 1H), 10.96 (br s, 1H), 14.04 (s, 1H).

### Example 10:

### 5-(Hydroxymethyl)-7-[1H-5-(piperidinomethyl)indol-2-yl]isoindoli none hydrochloride (Compound 477)

### Step 1

In a similar manner to Step 5 of Example 6, 5-(dimethoxymethyl)-3-hydroxy-7-iodo-2-(1-methyl-1-phenylethyl)i soindolinone (100 mg, 0.214 mmol) was dissolved in acetonitrile (2 mL), and the solution was treated with 1 mol/L hydrochloric acid (1 mL) to obtain 5-formyl-3-hydroxy-7-iodo-2-(1-methyl-1-phenylethyl)isoindolinon e (106 mg).

### Step 2

5-Formyl-3-hydroxy-7-iodo-2-(1-methyl-1-phenylethyl)isoindo linone (106 mg) was dissolved in THF (4 mL), and the solution was added with sodium borohydride (24.0 mg, 0.634 mmol) followed by stirring at room temperature for 30 minutes. The reaction mixture was added with water and extracted with ethyl acetate. The organic layer was washed with saturated brine and dried over anhydrous sodium sulfate. The solvent was evaporated under reduced pressure to obtain 5-hydroxymethyl-3-hydroxy-7-iodo-2-(1-methyl-1-phenylethyl)isoin dolinone (107 mg).

### Step 3

In a similar manner to Step 7 of Example 6, 5-hydroxymethyl-3-hydroxy-7-iodo-2-(1-methyl-1-phenylethyl)isoin dolinone (107 mg) was dissolved in nitromethane (2 mL), and the solution was treated with trifluoroacetic acid (0.082 mL, 1.1 mmol) and triethylsilane (0.069 mL, 0.43 mmol) followed by purification by slurry using hexane to obtain 5-hydroxymethyl-7-iodo-2-(1-methyl-1-phenylethyl)isoindolinone (133 mg).

### Step 4

In a similar manner to Step 6 of Example 1, 5-hydroxymethyl-7-iodo-2-(1-methyl-1-phenylethyl)isoindolinone (133 mg) was treated with 10% hydrogen chloride-methanol solution (2 mL), followed by purification by slurry using hexane to obtain 5-hydroxymethyl-7-iodoisoindolinone (35.2 mg, yield 57%, 4 steps). ESI-MS *m*/*z*: 289 [M+H]⁺; ¹H-NMR (DMSO-*d*₆)δ(ppm) : 4.25 (s, 2H), 4.56 (d, *J* = 5.6 Hz, 2H), 5.44 (t, *J* = 5.6 Hz, 1H), 7.51 (s, 1H), 7.83 (s, 1H), 8.66 (br s, 1H).

### Step 5

In a similar manner to Step 7 of Example 1, 5-hydroxymethyl-7-iodoisoindolinone (33.0 mg, 0.114 mmol) was dissolved in acetonitrile (2 mL), and the solution was treated with Compound BD (82.0 mg, 0.229 mmol), palladium acetate (2.0 mg, 0.0089 mmol), tri(o-tolyl)phosphine (5.5 mg, 0.018 mmol) and triethylamine (0.311 mL, 2.28 mmol), followed by purification by preparative thin-layer chromatography (chloroform/7 mol/L ammonia-methanol solution=24/1) to obtain 5-hydroxymethyl-7-[1-(tert-butoxycarbonyl)-5-(piperidinomethyl)i ndol-2-yl]isoindolinone (59.3 mg).

### Step 6

In a similar manner to Step 8 of Example 1, 5-hydroxymethyl-7-[1-(tert-butoxycarbonyl)-5-(piperidinomethyl)i ndol-2-yl]isoindolinone (59.3 mg) was dissolved in methanol (2 mL), and the solution was treated with 10% hydrogen chloride-methanol solution (2 mL). The obtained solid was collected by filtration and washed with diisopropylether, then dried under reduced pressure to obtain Compound 477 (10.1 mg, yield 22%, 2 steps).
ESI-MS *m*/*z*: 376 [M+H]⁺; ¹H-NMR (DMSO-*d*₆)δ(ppm) : 1.37 (m, 1H), 1.56-1.88 (m, 5H), 2.80-2.96 (m, 2H), 3.28-3.40 (m, 2H), 4.33 (d, *J* = 3.3 Hz, 2H), 4.51 (s, 2H), 4.68 (d, *J* = 5.9 Hz, 2H), 5.50 (t, *J* = 5.9 Hz, 1H), 7.26 (t, *J* = 8.6 Hz, 1H), 7.27 (s, 1H), 7.48 (s, 1H), 7.56 (d, *J* = 8.6 Hz, 1H), 7.75 (s, 1H), 8.12 (s, 1H), 9.27 (s, 1H), 9.48 (br s, 1H), 14. 16 (s, 1H).

### Example 11:

### 5-(3-Hydroxypropyloxy)-7-[1H-5-(piperidinomethyl)indol-2-yl]isoi ndolinone hydrochloride (Compound 478)

### Step 1

5-Hydroxy-7-iodoisoindolinone (100 mg, 0.364 mmol) was dissolved in N,N-dimethylformamide (3.0 mL), and the solution was Added with potassium carbonate (151 mg, 1.09 mmol), tetrabutylammonium iodide (13.3 mg, 0.036 mmol) and tert-butyl(2-bromopropyloxy)dimethylsilane (0.126 mL, 0.545 mmol), followed by stirring at 50 °C for 5 hours under nitrogen atmosphere. The reaction mixture was added with water and extracted with ethyl acetate. The organic layer was washed with saturated brine and dried over anhydrous sodium sulfate. The solvent was evaporated under reduced pressure, and the residue was purified by flush column chromatography (chloroform/methanol=19/1) to obtain 5-[3-(tert-butyldimethylsilyloxy)propyloxy]-7-iodoisoindolinone (123 mg, yield 75%).
ESI-MS *m*/*z*: 448 [M+H]⁺; ¹H-NMR (CDCl₃)δ(ppm) : 0.05 (s, 6H), 0. 89 (s, 9H), 1.95-2.03 (m, 2H), 3.80 (t, *J* = 6.2 Hz, 2H), 4.12 (t, *J* = 6.6 Hz, 2H), 4.29 (s, 2H), 6.32 (brs, 1H), 6.94 (d, *J* = 2.1 Hz, 1H), 7.46 (d, *J* = 2.1 Hz, 1H).

### Step 2

In a similar manner to Step 7 of Example 1, 5-[3-(tert-butyldimethylsilyloxy)propyloxy]-7-iodoisoindolinone (72.9 mg, 0.163 mmol) was dissolved in acetonitrile (2.2 mL), and the solution was treated with Compound BD (117 mg, 0.326 mmol), palladium acetate (2.9 mg, 0.013 mmol), tri(o-tolyl)phosphine (7.9 mg, 0.026 mmol) and triethylamine (0.227 mL, 1.63 mmol), followed by purification by flush column chromatography (chloroform/methanol=9/1) to obtain 5-[3-(tert-butyldimethylsilyloxy)propyloxy]-7-[1-(tert-butoxycar bonyl)-5-(piperidinomethyl)indol-2-yl]isoindolinone (102 mg, yield 98%).
APCI-MS *m*/*z*: 634 [M+H]⁺; ¹H-NMR (CDCl₃)δ(ppm) : 0.05 (s, 6H), 0.89 (s, 9H), 1.29 (s, 9H), 1.43-1.67 (m, 6H), 2.00-2.04 (m, 2H), 2.44 (brs, 4H), 3.62 (s, 2H), 3.82 (t, *J* = 6.0 Hz, 2H), 4.16 (t, *J* = 6.0 Hz, 2H), 4.36 (s, 2H), 6.54 (s, 1H), 6.93 (d, J = 2.1 Hz, 1H), 6.70 (d, *J* = 2.1 Hz, 1H), 7.26-7.58 (m, 3H), 8.20 (d, *J* = 8.4 Hz, 1H).

### Step 3

In a similar manner to Step 8 of Example 1, 5-[3-(tert-butyldimethylsilyloxy)propyloxy]-7-[1-(tert-butoxycar bonyl)-5-(piperidinomethyl)indol-2-yl]isoindolinone (102 mg, 0.160 mmol) was dissolved in methanol (1.0 mL), and the solution was treated with 10% hydrogen chloride-methanol solution (1.0 mL). The obtained solid was collected by filtration and washed with methanol, then dried under reduced pressure to obtain Compound 478 (48.9 mg, yield 67%). ESI-MS *m*/*z:* 420 [M+H]⁺; ¹H-NMR (DMSO-*d*₆)δ(ppm) : 1.27-1.39 (m, 1H), 1.65-1.76 (m, 5H), 1.88-1.96 (m, 2H), 2.82-2.85 (m, 2H), 3.29 (brs, 2H), 3.57-3.62 (m, 2H), 4.21 (t, *J* = 6.3 Hz, 2H), 4.30 (brs, 2H), 4.45 (s, 2H), 4.64 (t, J = 4.8 Hz, 1H), 7.12 (s, 1H), 7.29 (d, *J* = 8.1 Hz, 1H), 7.36 (s, 1H), 7.53 (d, *J* = 8.1 Hz, 1H), 7.67 (s, 1H), 7.75 (s, 1H), 9.08 (s, 1H), 9.97 (brs, 1H), 14.21 (s, 1H).

### Example 12:

### 5-(Aminomethyl)-7-[1H-5-(piperidinomethyl)indol-2-yl]isoindolino ne dihydrochloride (Compound 479)

### Step 1

5-(Dimethoxymethyl)-3-hydroxy-7-iodo-2-(1-methyl-1-phenylet hyl)isoindolinone (100 mg, 0.214 mmol) was dissolved in acetonitrile (4 mL), and the solution added with tert-butyl dicarbamate (75.0 mg, 0.640 mmol), trifluoroacetic acid (0.033 mL, 0.43 mmol) and triethylsilane (0.104 mL, 0.644 mmol) followed by stirring at room temperature for 6 hours. The reaction mixture was added with tert-butyl dicarbamate (75.0 mg, 0.640 mmol), trifluoroacetic acid (0.033 mL, 0.43 mmol) and triethylsilane (0.104 mL, 0.644 mmol) followed by stirring at room temperature for 12 hours. The reaction mixture was added with water and extracted with ethyl acetate. The organic layer was washed with saturated aqueous sodium hydrogencarbonate solution and saturated brine, and dried over anhydrous sodium sulfate. The solvent was evaporated under reduced pressure, and the residue was purified by preparative thin-layer chromatography (hexane/ethyl acetate=2/1) to obtain 5-[(tert-butoxycarbonyl)aminomethyl]-3-(tert-butoxycarbonyl)amin o-7-iodo-2-(1-methyl-1-phenylethyl)isoindolinone (87.5 mg, yield 66%).
ESI-MS *m*/*z*: 622 [M+H]⁺; ¹H-NMR (CDCl₃)δ(ppm) : 1.43 (s, 9H), 1.45 (s, 9H), 1.89 (s, 3H), 1.92 (s, 3H), 4.29 (d, *J* = 5.8 Hz, 2H), 4.84 (d, *J* = 10.0 Hz, 1H), 5.10 (t, *J* = 5.8 Hz, 1H), 6.36 (d, *J* = 10.0 Hz, 1H), 7.22 (m, 1H), 7.28-7.44 (m, 5H), 7.78 (s, 1H).

### Step 2

5-[(tert-Butoxycarbonyl)aminomethyl]-3-(tert-butoxycarbonyl )amino-7-iodo-2-(1-methyl-1-phenylethyl)isoindolinone (87.0 mg, 0.140 mmol) was dissolved in nitromethane (4 mL), and the solution was added with trifluoroacetic acid (0.216 mL, 2.80 mmol) and triethylsilane (0.113 mL, 0.700 mmol) followed by stirring at 60 °C for 5 hours. The reaction mixture was added with water, saturated aqueous sodium hydrogencarbonate solution and ethyl acetate, and the organic layer and the aqueous layer were separated. The aqueous layer was added with acetonitrile (10 mL) and di-tert-butyl dicarbonate (1.00 mL, 4.35 mmol), followed by stirring at room temperature for 12 hours. The reaction mixture was extracted with ethyl acetate. The organic layer was washed with saturated brine and dried over anhydrous sodium sulfate. The solvent was evaporated under reduced pressure, and the residue was purified by slurry using hexane to obtain 5-[(tert-butoxycarbonyl)aminomethyl]-7-iodoisoindolinone (20.0mg, yiels 37%).

### Step 3

In a similar manner to Step 7 of Example 1, 5-[(tert-butoxycarbonyl)aminomethyl]-7-iodoisoindolinone (19.0 mg, 0.114 mmol) was dissolved in acetonitrile (2 mL), and the solution was treated with Compound BD (35.0 mg, 0.0977 mmol), palladium acetate (1.0 mg, 0.0045 mmol), tri(o-tolyl)phosphine (2.4 mg, 0.0079 mmol) and triethylamine (0.070 mL, 0.51 mmol), followed by purification by preparative thin-layer chromatography (chloroform/methanol=10/1), and slurry using chloroform and hexane to obtain 5-[(tert-butoxycarbonyl)aminomethyl]-7-[1-(tert-butoxycarbonyl)-5-(piperidinomethyl)indol-2-yl]isoindolinone (19.4 mg, yield 69%).
ESI-MS *m*/*z*: 575 [M+H]⁺; ¹H-NMR (CDCl₃)δ(ppm) : 1.32-1.52 (m, 6H), 1.34 (s, 9H), 1.47 (s, 9H), 2.37-2.47 (m, 4H), 3.59 (s, 2H), 4.42 (s, 2H), 4.45 (d, *J* = 5.5 Hz, 2H), 4.99 (br s, 1H), 6.16 (br s, 1H), 6.54 (s, 1H), 7.28 (dd, *J* = 1.8, 8.4 Hz, 1H), 7.36 (s, 1H), 7.40 (s, 1H), 7.48 (s, 1H), 8.14 (s, 1H).

### Step 4

In a similar manner to Step 8 of Example 1, 5-[(tert-butoxycarbonyl)aminomethyl]-7-[1-(tert-butoxycarbonyl)-5- (piperidinomethyl) indol-2-yl] isoindolinone (19.4 mg, 0. 0331 mmol) was dissolved in methanol (1 mL), and the solution was treated with 10% hydrogen chloride-methanol solution (1 mL). Then, the reaction mixture was concentrated under reduced pressure, and the residue was added with methanol and ethyl acetate. The obtained solid was collected by filtration and washed with ethyl acetate, then dried under reduced pressure to obtain Compound 479 (13.0 mg, yield 88%). ESI-MS *m*/*z*: 376 [M+H]⁺; ¹H-NMR (DMSO-*d*₆)δ(ppm) : 1.20-1.83 (m, 6H), 2.75-3.06 (m, 2H), 3.20-3.48 (m, 2H), 4.21 (s, 2H), 4.30 (br s, 2H), 4.54 (s, 2H), 7.32 (d, *J* = 8.5 Hz, 1H), 7.39 (s, 1H), 7.56 (d, *J* = 8.5 Hz, 1H), 7.60 (s, 1H), 7.82 (s, 1H), 8.47 (s, 1H), 8.66 (br s, 3H), 9.43 (s, 1H), 10.08 (br s, 1H), 14.07 (s, 1H).

### Example 13:

### 5-(1H-Benzimidazol-2-yl)-7-[1H-indol-2-yl]isoindolinone hydrochloride (Compound 480)

### Step 1

In a similar manner to Step 5 of Example 6, 5-(dimethoxymethyl)-3-hydroxy-7-iodo-2-(1-methyl-1-phenylethyl)i soindolinone (200 mg, 0.428 mmol) was dissolved in acetonitrile (4 mL), and the solution was treated with 1 mol/L hydrochloric acid (2 mL) to obtain 5-formyl-3-hydroxy-7-iodo-2-(1-methyl-1-phenylethyl)isoindolinon e (204 mg).

### Step 2

5-Formyl-3-hydroxy-7-iodo-2-(1-methyl-1-phenylethyl)isoindo linone (204 mg) was dissolved in methanol (4 mL), and the solution was added with 1,2-phenylenediamine (46.3 mg, 0.428 mmol) followed by stirring at room temperature for 1 hour. Then, the mixture was added with benzofuroxan (58.3 mg, 0.428 mmol) in acetonitrile (1 mL), followed by stirring at 50 °C for 2 hours. The reaction mixture was added with 2 mol/L aqueous sodium hydroxide solution, and extraced with ethyl acetate. The organic layer was washed with saturate brine and dried over anhydrous sodium sulfate. The solvent was evaporated under reduced pressure, and the residue was added with methanol and diidopropylether. The obtained solid was collected by filtration and washed with diisopropylether, followed by drying under reduced pressure to obtain 5-(1H-benzimidazol-2-yl)-7-iodo-2-(1-methyl-1-phenylethyl)isoind olinone (178 mg, yield 82%, 2 steps).
ESI-MS *m*/*z*: 510 [M+H]⁺; ¹H-NMR (DMSO-*d*₆)δ(ppm) : 1.83 (s, 3H), 1.85 (s, 3H), 6.33 (d, *J* = 9.5 Hz, 1H), 6.74 (d, *J* = 9.5 Hz, 1H), 7.18 (m, 1H), 7.22-7.32 (m, 4H), 7.39-7.46 (m, 2H), 7.61-7.70 (m, 2H), 13.23 (br s, 1H).

### Step 3

In a similar manner to Step 7 of Example 6, 5-(1H-Benzimidazol-2-yl)-7-iodo-2-(1-methyl-1-phenylethyl)isoind olinone (175 mg, 0.344 mmol) was dissolved in nitromethane (5 mL), and the solution was treated with trifluoroacetic acid (0.265 mL, 3.44 mmol) and triethylsilane (0.556 mL, 0.344 mmol) followed by purification by slurry using hexane to obtain 5-(1H-Benzimidazol-2-yl)-7-iodoisoindolinone (133 mg, yield 79%).

### Step 4

In a similar manner to Step 7 of Example 1, 5-(1H-benzimidazol-2-yl)-7-iodoisoindolinone (30.0 mg, 0.0800 mmol) was dissolved in acetonitrile (1.5 mL), and the solution was treated with 1-(tert-butoxycarbonyl)indol-2-boronic acid (41.0 mg, 0.157 mmol), palladium acetate (1.4 mg, 0.0062 mmol), tri(o-tolyl)phosphine (3.9 mg, 0.012 mmol) and triethylamine (0.109 mL, 0.799 mmol), followed by purification by slurry using chloroform and hexane to obtain 5-(1H-benzimidazol-2-yl)-7-[1-(tert-butoxycarbonyl)indol-2-yl]is oindolinone (30.8 mg, yield 83%).
ESI-MS *m*/*z*: 465 [M+H]⁺.

### Step 5

In a similar manner to Step 8 of Example 1, 5-(1H-benzimidazol-2-yl)-7-[1-(tert-butoxycarbonyl)indol-2-yl]is oindolinone (28.0 mg, 0.0603 mmol) was dissolved in methanol (1.5 mL), and the solution was treated with 10% hydrogen chloride-methanol solution (1.5 mL). Then, the obtained solid was collected by filtration and washed with methanol, then dried under reduced pressure to obtain Compound 480 (17.9 mg, yield 74%).
ESI-MS *m*/*z*: 365 [M+H]⁺; ¹H-NMR (DMSO-*d*₆)δ(ppm) : 4. 67 (s, 2H), 7. 07 (dd, *J* = 7.0, 7.8 Hz, 1H), 7.18 (dd, *J* = 7.0, 8.2 Hz, 1H), 7.44-7.56 (m, 4H), 7.67 (d, *J* = 7.8 Hz, 1H), 7.79-7.88 (m, 2H), 8.32 (s, 1H), 9.05 (s, 1H), 9.57 (s, 1H), 13.91 (s, 1H).

### Example 14:

### 5-(1H-Benzimidazol-2-yl)-7-[1H-5-(piperidinomethyl)indol-2-yl]is oindolinone dihydrochloride (Compound 481)

### Step 1

In a similar manner to Step 7 of Example 1, 5-(1H-benzimidazol-2-yl)-7-iodoisoindolinone (30.0 mg, 0.0800 mmol) was dissolved in acetonitrile (1.5 mL), and the solution was treated with Compound BD (57.0 mg, 0.159 mmol), palladium acetate (1.4 mg, 0.0062 mmol), tri(o-tolyl)phosphine (3.9 mg, 0.012 mmol) and triethylamine (0.109 mL, 0.799 mmol), followed by purification by slurry using chloroform and hexane to obtain 5-(1H-benzimidazol-2-yl)-7-[1-(tert-butoxycarbonyl)-5-(piperidin omethyl)indol-2-yl]isoindolinone (23.1 mg, yield 51%) ESI-MS *m*/*z*: 562 [M+H]⁺.

### Step 2

In a similar manner to Step 8 of Example 1, 5-(1H-benzimidazol-2-yl)-7-[1-(tert-butoxycarbonyl)-5-(piperidin omethyl)indol-2-yl]isoindolinone (23.0 mg, 0.0409 mmol) was dissolved in methanol (1.5 mL), and the solution was treated with 10% hydrogen chloride-methanol solution (1.5 mL). Then, the obtained solid was collected by filtration and washed with methanol, then dried under reduced pressure to obtain Compound 481 (18.7 mg, yield 85%).
ESI-MS *m*/*z*: 462 [M+H]⁺; ¹H-NMR (DMSO-*d*₆)δ(ppm) : 1.38 (m, 1H), 1.60-1.88 (m, 5H), 2.80-2.99 (m, 2H), 3.30-3.45 (m, 2H), 4.35 (d, *J* = 5.0 Hz, 2H), 4.68 (s, 2H), 7.33 (d, *J* = 8.3 Hz, 1H), 7.42-7.50 (m, 2H), 7.61 (d, *J* = 8.3 Hz, 1H), 7.70 (s, 1H), 7.76-7.85 (m, 2H), 7.87 (s, 1H), 8.37 (s, 1H), 9.17 (s, 1H), 9.59 (s, 1H), 9.78 (br s, 1H), 14.11 (s, 1H).

### Example 15:

### 5-(Dimethylaminomethyl)-7-[1H-indol-2-yl]isoindolinone (Compound 482)

### Step 1

5-(Dimethylaminomethyl)-7-iodoisoindolinone (50.0 mg, 0.158 mmol) was dissolved in dimethoxyethane (5 mL), and the solution was added with 1-(tert-butoxycarbonyl)indol-2-ylboronic acid (83.0 mg, 0.318 mmol), [bis(diphenylphosphino)ferrocene]dichloropalladium (10.3 mg, 0.0126 mmol), potassium carbonate (109 mg, 0.789 mmol) and water (0.114 mL, 6.33 mmol),followed by stirring at 90 °C for 4.3 hours under argon atmosphere. The reaction mixture was added with water and extracted with ethyl acetate. The organic layer was washed with saturated brine and dried over anhydrous sodium sulfate. The solvent was evaporated under reduced pressure, and the residue was purified by preparative thin-layer chromatography (chloroform/methanol/7 mol/L ammonia-methanol solution=12/1/1) to obtain 5-(dimethylaminomethyl)-7-[1-(tert-butoxycarbonyl)indol-2-yl]iso indolinone (59.9 mg, yield 93%).
ESI-MS *m*/*z*: 406 [M+H]⁺; ¹H-NMR (CDCl₃)δ(ppm) : 1.26 (s, 9H), 2.29 (s, 6H), 3.54 (s, 2H), 4.39 (s, 2H), 6.59 (s, 1H), 7.22 (ddd, *J* = 0.9, 7.1, 7.7 Hz, 1H), 7.33 (ddd, *J* = 0.7, 7.1, 8.4 Hz, 1H), 7.40 (s, 1H), 7.43 (s, 1H), 7.55 (d, *J* = 7.7 Hz, 1H), 7.73 (br s, 1H), 8.28 (d, *J* = 8.4 Hz, 1H).

### Step 2

In a similar manner to Step 8 of Example 1, 5-(dimethylaminomethyl)-7-[1-(tert-butoxycarbonyl)indol-2-yl]iso indolinone (59.5 mg, 0.147 mmol) was dissolved in methanol (1 mL), and the solution was treated with 10% hydrogen chloride-methanol solution (1 mL), followed by adding diisopropylether thereto. The obtained solid was collected by filtration and washed with diisopropylether, and then dried under reduced pressure to obtain Compound 482 (41.9 mg, yield 83%).
ESI-MS *m*/*z*: 306 [M+H]⁺; ¹H-NMR (DMSO-*d*₆)δ(ppm) : 2.76 (br s, 6H), 4.40 (br s, 2H), 4.55 (s, 2H), 7.04 (ddd, *J* = 0.8, 7.0, 8.2 Hz, 1H), 7.15 (ddd, *J* = 1.0, 7.0, 8.2 Hz, 1H), 7.29 (s, 1H), 7.48 (dd, *J* = 1.0, 8.2 Hz, 1H), 7.59 (s, 1H), 7.61 (dd, *J* = 0.8, 8.2 Hz, 1H), 8.43 (s, 1H), 9.43 (s, 1H), 10.52 (br s, 1H), 13.87 (s, 1H).

### Example 16:

### 5-(Dimethylaminomethyl)-7-{1H-5-[4-(2-hydroxyethyl)piperazin-1-y lmethyl]indol-2-yl}isoindolinone trihydrochloride (Compound 483)

### Step 1

In a similar manner to Step 1 of Example 15, 5-(dimethylaminomethyl)-7-iodoisoindolinone (50.0 mg, 0.158 mmol) was dissolved in dimethoxyethane (5 mL), and the solution was treated with Compound BU (163 mg, 0.315 mmol), [bis(diphenylphosphino)ferrocene]dichloropalladium (10.3 mg, 0.0126 mmol), potassium carbonate (109 mg, 0.789 mmol) and water (0.114 mL, 6.33 mmol),followed by purification by preparative thin-layer chromatography (chloroform/methanol/7 mol/L ammonia-methanol solution=24/1/1) to obtain 5-(dimethylaminomethyl)-7-(1-(tert-butoxycarbonyl)-5-{4-[2-(tert -butyldimethylsilyloxy)ethyl]piperazin-1-ylmethyl}indol-2-yl)iso indolinone (103 mg, yield 98%).
ESI-MS *m*/*z:* 662 [M+H]⁺; ¹H-NMR (CDCl₃)δ(ppm) : 0.05 (s, 6H), 0. 88 (s, 9H), 1.26 (s, 9H), 2.29 (s, 6H), 2.43-2.63 (m, 8H), 2.53 (t, *J* = 6.6 Hz, 2H), 3.54 (s, 2H), 3.59 (s, 2H), 3.76 (t, *J* = 6.6 Hz, 2H), 4.39 (s, 2H), 6.55 (s, 1H), 7.29 (dd, *J* = 1.3, 8.5 Hz, 1H), 7.38 (s, 1H), 7.43 (s, 1H), 7.48 (d, *J* = 1.3 Hz, 1H), 7.79 (br s, 1H), 8.20 (d, *J* = 8.5 Hz, 1H).

### Step 2

In a similar manner to Step 8 of Example 1, 5-(dimethylaminomethyl)-7-(1-(tert-butoxycarbonyl)-5-{4-[2-(tert -butyldimethylsilyloxy)ethyl]piperazin-1-ylmethyl}indol-2-yl)iso indolinone (102 mg, 0.154 mmol) was dissolved in methanol (1.5 mL), and the solution was treated with 10% hydrogen chloride-methanol solution (1.5 mL), followed by adding diisopropylether thereto. The obtained solid was collected by filtration and washed with diisopropylether, then dried under reduced pressure to obtain Compound 483 (65.0 mg, yield 76%).
ESI-MS *m*/*z*: 448 [M+H]⁺; ¹H-NMR (DMSO-*d*₆)δ(ppm) : 2.76 (br d, *J* = 4.8 Hz, 6H), 3.10-3.86 (m, 12H), 4.40-4.60 (m, 2H), 4.45 (br d, *J* = 4.9 Hz, 2H), 4.56 (s, 2H), 7.37 (m, 1H), 7.43 (s, 1H), 7.58 (d, *J* = 8.4 Hz, 1H), 7.64 (s, 1H), 7.87 (s, 1H), 8.62 (s, 1H), 9.46 (s, 1H), 11.28 (br s, 1H), 14.03 (s, 1H).

### Example 17:

### 5-(2-Propyloxy)-7-[1H-5-(piperidinomethyl)indol-2-yl]isoindolino ne hydrochloride (Compound 484)

### Step 1

In a similar manner to Step 1 of Example 3, 5-hydroxy-7-iodoisoindolinone (500 mg, 0.182 mmol) was dissolved in DMF (5 mL), and the solution was treated with potassium carbonate (503 mg, 3.64 mmol) and isopropyl bromide (0.205 mL, 2.18 mmol), followed by purification by slurry using diisopropylether to obtain 5-(2-propyloxy)-7-iodoisoindolinone (448 mg, yield 78%).
ESI-MS *m*/*z*: 318 [M+H]⁺; ¹H-NMR (CDCl₃)δ(ppm): 1.37 (d, *J* = 6.0 Hz, 6H), 4.29 (s, 2H), 4.61 (sep, *J* = 6.0 Hz, 1H), 6.57 (br s, 1H), 6.91 (d, *J* = 2.0 Hz, 1H), 7.43 (d, *J* = 2.0 Hz, 1H).

### Step 2

In a similar manner to Step 1 of Example 15, 5-(2-propyloxy)-7-iodoisoindolinone (50.0 mg, 0.158 mmol) was dissolved in dimethoxyethane (5 mL), and the solution was treated with Compound BD (113 mg, 0.315 mmol), [bis(diphenylphosphino)ferrocene]dichloropalladium (10.3 mg, 0.0126 mmol), potassium carbonate (109 mg, 0.789 mmol) and water (0.114 mL, 6.33 mmol),followed by purification by preparative thin-layer chromatography (chloroform/methanol/7 mol/L ammonia-methanol solution=24/1/1) to obtain 5-(2-propyloxy)-7-[1-(tert-butoxycarbonyl)-5-(piperidinomethyl)i ndol-2-yl]isoindolinone (58.2 mg, yield 73%).
ESI-MS *m*/*z*: 504 [M+H]⁺; ¹H-NMR (CDCl₃)δ(ppm) : 1.32 (s, 9H), 1.39 (d, *J* = 6.0 Hz, 6H), 1.40-1.49 (m, 2H), 1.52-1.65 (m, 4H), 2.35-2.45 (m, 4H), 3.58 (s, 2H), 4.36 (s, 2H), 4.65 (sep, *J* = 6.0 Hz, 1H), 6.45 (br s, 1H), 6.54 (s, 1H), 6.89 (d, *J* = 2.1 Hz, 1H), 6.97 (d, *J* = 2.1 Hz, 1H), 7.27 (dd, *J* = 1.4, 8.6 Hz, 1H), 7.47 (d, *J* = 1.4 Hz, 1H), 8.17 (d, *J* = 8.6 Hz, 1H).

### Step 3

In a similar manner to Step 8 of Example 1, 5-(2-propyloxy)-7-[1-(tert-butoxycarbonyl)-5-(piperidinomethyl)i ndol-2-yl]isoindolinone (54.7 mg, 0.109 mmol) was dissolved in methanol (2 mL), and the solution was treated with 10% hydrogen chloride-methanol solution (2 mL), followed by condensing under reduced pressure and adding methanol and ethyl acetate thereto. The obtained solid was collected by filtration and washed with ethyl acetate, then dried under reduced pressure to obtain Compound 484 (43.9 mg, yield 91%).
ESI-MS *m*/*z*: 404 [M+H]⁺; ¹H-NMR (DMSO-*d*₆)δ(ppm) : 1.35 (d, *J* = 6.0 Hz, 6H), 1.37 (m, 1H), 1.58-1.88 (m, 5H), 2.78-2.95 (m, 2H), 3.25-3.42 (m, 2H), 4.32 (br d, *J* = 5.3 Hz, 2H), 4.46 (s, 2H), 4.86 (sep, *J* = 6.0 Hz, 1H), 7.12 (d, *J* = 1.9 Hz, 1H), 7.28 (dd, *J* = 1.2, 8.4 Hz, 1H), 7.36 (d, *J* = 1.2 Hz, 1H), 7.54 (d, *J* = 8.4 Hz, 1H), 7.64 (d, *J* = 1.9 Hz, 1H), 7.75 (s, 1H), 9.06 (s, 1H), 9.70 (br s, 1H), 14.22 (s, 1H).

### Example 18:

### 5-(2-Propyloxy)methyl-7-[1H-5-(piperidinomethyl)indol-2-yl]isoin dolinone hydrochloride (Compound 485)

### Step 1

In a similar manner to Step 5 of Example 6, 5-(dimethoxymethyl)-3-hydroxy-7-iodo-2-(1-methyl-1-phenylethyl)i soindolinone (100 mg, 0.214 mmol) was dissolved in acetonitrile (2 mL), and the solution was treated with 1 mol/L hydrochloric acid (1 mL) to obtain 5-formyl-3-hydroxy-7-iodo-2-(1-methyl-1-phenylethyl)isoindolinon e (103 mg).

### Step 2

5-Formyl-3-hydroxy-7-iodo-2-(1-methyl-1-phenylethyl)isoindo linone (113 mg) was dissolved in nitromethane (3 mL), and the solution was added with trifluoroacetic acid (0.330 mL, 4.28 mmol), isopropyl alcohol (0.330 mL, 4.31 mmol) and triethylsilane (0.173 mL, 1.07 mmol), followed by stirring at 50 °C for 3 hours. The reaction mixture was added with trifluoroacetic acid (0.330 mL, 4.28 mmol) and triethylsilane (0.173 mL, 1.07 mmol), followed by stirring at 50 °C for 5 hours. The reaction mixture was added with trifluoroacetic acid (0.330 mL, 4.28 mmol) and triethylsilane (0.173 mL, 1.07 mmol), followed by stirring at 50 °C for 3 hours. The reaction mixture was added with water, and extracted with ethyl acetate. The organic layer was washed with saturated aqueous sodium hydrogencarbonate solution and saturated brine, and dried over anhydrous sodium sulfate. The solvent was evaporated under reduced pressure, and the residue was purified by preparative thin-layer chromatography (ethyl acetate) to obtain 5- (2-propyloxy)methyl-7-iodoisoindolinone (43.0 mg, yield 61%, 2 steps).
ESI-MS *m*/*z*: 332 [M+H]⁺; ¹H-NMR (CDCl₃)δ(ppm) : 1.24 (d, *J* = 6.1 Hz, 6H), 3.71 (sep, *J* = 6.1 Hz, 1H), 4.36 (s, 2H), 4.55 (s, 2H), 7.47 (s, 1H), 7.88 (s, 1H).

### Step 3

In a similar manner to Step 1 of Example 15, 5-(2-propyloxy)methyl-7-iodoisoindolinone (43.0 mg, 0.130 mmol) was dissolved in dimethoxyethane (3 mL), and the solution was treated with Compound BD (93 mg, 0.260 mmol), [bis(diphenylphosphino)ferrocene]dichloropalladium (8.5 mg, 0.0104 mmol), potassium carbonate (90.0 mg, 0.651 mmol) and water (0.094 mL, 5.2 mmol),followed by purification by preparative thin-layer chromatography (chloroform/methanol/7 mol/L ammonia-methanol solution=25/1/1) to obtain 5-(2-propyloxy)methyl-7-[1-(tert-butoxycarbonyl)-5-(piperidinome thyl)indol-2-yl]isoindolinone (60.0 mg, yield 89%).
ESI-MS *m*/*z*: 518 [M+H]⁺; ¹H-NMR (CDCl₃)δ(ppm) : 1.25 (d, *J* = 6.0 Hz, 6H), 1.29 (s, 9H), 1.38-1.49 (m, 2H), 1.52-1.63 (m, 4H), 2.35-2.46 (m, 4H), 3.58 (s, 2H), 3.74 (sep, *J* = 6.0 Hz, 1H), 4.40 (s, 2H), 4.63 (s, 2H), 6.56 (s, 1H), 7.28 (dd, *J* = 1.3, 8.5 Hz, 1H), 7.40 (s, 1H), 7.46 (s, 1H), 7.48 (s, *J* = 1.3 Hz, 1H), 8.17 (d, *J* = 8.5 Hz, 1H).

### Step 4

In a similar manner to Step 8 of Example 1, 5-(2-propyloxy)methyl-7-[1-(tert-butoxycarbonyl)-5-(piperidinome thyl)indol-2-yl]isoindolinone (60.0 mg, 0.116 mmol) was dissolved in methanol (2.5 mL), and the solution was treated with 10% hydrogen chloride-methanol solution (2.5 mL), followed by purification by slurry using diisopropylether to obtain Compound 485 (33.6 mg, yield 64%).
ESI-MS *m*/*z*: 418 [M+H]⁺; ¹H-NMR (DMSO-*d*₆)δ(ppm) : 1.20 (d, *J* = 6.0 Hz, 6H), 1.37 (m, 1H), 1.60-1.87 (m, 5H), 2.76-2.97 (m, 2H), 3.27-3.41 (m, 2H), 3.73 (sep, *J* = 6.0 Hz, 1H), 4.31 (br s, 2H), 4.52 (s, 2H), 4.64 (s, 2H), 7.28 (s, 1H), 7.29 (d, *J* = 8.2 Hz, 1H), 7.48 (s, 1H), 7.56 (d, *J* = 8.2 Hz, 1H), 7.77 (s, 1H), 8.11 (s, 1H), 9.29 (s, 1H), 9.76 (br s, 1H), 14. 12 (s, 1H).

### Example 19:

### 5-Ethoxymethyl-7-[1H-5-(piperidinomethyl)indol-2-yl]isoindolinon e hydrochloride (Compound 486)

### Step 1

In a similar manner to Step 5 of Example 6, 5-(dimethoxymethyl)-3-hydroxy-7-iodo-2-(1-methyl-1-phenylethyl)i soindolinone (100 mg, 0.214 mmol) was dissolved in acetonitrile (2 mL), and the solution was treated with 1 mol/L hydrochloric acid (1 mL) to obtain 5-formyl-3-hydroxy-7-iodo-2-(1-methyl-1-phenylethyl)isoindolinon e (100 mg).

### Step 2

In a similar manner to Step 2 of Example 18, 5-formyl-3-hydroxy-7-iodo-2-(1-methyl-1-phenylethyl)isoindolinon e (100 mg) was dissolved in nitromethane (3 mL), and the solution was treated with trifluoroacetic acid (1.32 mL, 17.1 mmol), ethanol (0.250 mL, 4.29 mmol) and triethylsilane (0.692 mL, 4.28 mmol), followed by purification by preparative thin-layer chromatography (ethyl acetate) to obtain 5-ethoxymethyl-7-iodoisoindolinone (23.5 mg, yield 35%, 2 steps).
ESI-MS *m*/*z*: 315 [M+H]⁺; ¹H-NMR (CDCl₃)δ(ppm) : 1.27 (t, *J* = 7.0 Hz, 3H), 3.59 (q, *J* = 7.0 Hz, 2H), 4.37 (s, 2H), 4.54 (s, 2H), 7.46 (d, *J* = 1.1 Hz, 1H), 7.87 (d, J = 1.1 Hz, 1H), 8.02 (br s, 1H).

### Step 3

In a similar manner to Step 1 of Example 15, 5-ethoxymethyl-7-iodoisoindolinone (23.0 mg, 0.0725 mmol) was dissolved in dimethoxyethane (2 mL), and the solution was treated with Compound BD (52 mg, 0.145 mmol), [bis(diphenylphosphino)ferrocene]dichloropalladium (4.7 mg, 0.0058 mmol), potassium carbonate (50.0 mg, 0.362 mmol) and water (0.052 mL, 2.9 mmol),followed by purification by preparative thin-layer chromatography (chloroform/methanol/7 mol/L ammonia-methanol solution=25/1/1) to obtain 5-ethoxymethyl-7-[1-(tert-butoxycarbonyl)-5-(piperidinomethyl)in dol-2-yl]isoindolinone (34.2 mg, yield 94%).
ESI-MS *m*/*z*: 504 [M+H]⁺; ¹H-NMR (CDCl₃)δ(ppm) : 1.28 (t, *J* = 7.0 Hz, 3H), 1.29 (s, 9H), 1.38-1.48 (m, 2H), 1.52-1.63 (m, 4H), 2.35-2.46 (m, 4H), 3.58 (s, 2H), 3.61 (q, *J* = 7.0 Hz, 2H), 4.41 (s, 2H), 4.63 (s, 2H), 6.56 (s, 1H), 7.28 (dd, *J* = 1.4, 8.7 Hz, 1H), 7.40 (s, 1H), 7.45 (s, 1H), 7.48 (d, *J* = 1.4 Hz, 1H), 8.17 (d, *J* = 8.7 Hz, 1H).

### Step 4

In a similar manner to Step 8 of Example 1, 5-ethoxymethyl-7-[1-(tert-butoxycarbonyl)-5-(piperidinomethyl)in dol-2-yl]isoindolinone (34.0 mg, 0.0675 mmol) was dissolved in methanol (2.5 mL), and the solution was treated with 10% hydrogen chloride-methanol solution (2.5 mL), followed by condensing under reduced pressure and adding methanol and ethyl acetate thereto. The obtained solid was collected by filtration and washed with ethyl acetate, then dried under reduced pressure to obtain Compound 486 (19.2 mg, yield 65%).
ESI-MS *m*/*z*: 404 [M+H]⁺; ¹H-NMR (DMSO-*d*₆)δ(ppm) : 1.22 (t, *J* = 7.0 Hz, 3H), 1.37 (m, 1H), 1.57-1.87 (m, 5H), 2.77-2.97 (m, 2H), 3.27-3.40 (m, 2H), 3.58 (q, *J* = 7.0 Hz, 2H), 4.32 (br s, 2H), 4.52 (s, 2H), 4.64 (s, 2H), 7.28 (d, *J* = 8.4 Hz, 1H), 7.30 (s, 1H), 7.48 (s, 1H), 7.56 (d, *J* = 8.4 Hz, 1H), 7.76 (s, 1H), 8.12 (s, 1H), 9.30 (s, 1H), 9.68 (s, 1H), 14.12 (s, 1H).

### Example 20:

### 5-(Dimethylaminomethyl)-7-(1H-5-aminoindol-2-yl)isoindolinone hydrochloride (Compound 487)

### Step 1

In a similar manner to Step 1 of Example 15, 5-(dimethylaminomethyl)-7-iodoisoindolinone (50.0 mg, 0.158 mmol) was dissolved in dimethoxyethane (5 mL), and the solution was treated with Compound BP (119 mg, 0.316 mmol), [bis(diphenylphosphino)ferrocene]dichloropalladium (10.3 mg, 0.013 mmol), potassium carbonate (109 mg, 0.789 mmol) and water (0.114 mL, 6.33 mmol), followed by purification by preparative thin-layer chromatography (chloroform/methanol=9/1) to obtain 5-(dimethylaminomethyl)-7-[1-(tert-butoxycarbonyl)-5-(tert-butox ycarbonylamino)indol-2-yl]isoindolinone (69.0 mg, yield 84%).
ESI-MS *m*/*z*: 521 [M+H]⁺; ¹H-NMR (CDCl₃)δ(ppm) : 1.28 (s, 9H), 1.53 (s, 9H), 2.30 (s, 6H), 3.56 (s, 2H), 4.41 (s, 2H), 6.44-6.57 (m, 3H), 7.13 (dd, *J* = 2.1, 8.9 Hz, 1H), 7.39 (s, 1H), 7.45 (s, 1H), 7.69 (br s, 1H), 8.14 (d, *J* = 8.9 Hz, 1H).

### Step 2

In a similar manner to Step 8 of Example 1, 5-(dimethylaminomethyl)-7-[1-(tert-butoxycarbonyl)-5-(tert-butox ycarbonylamino)indol-2-yl]isoindolinone (62.0 mg, 0.119 mmol) was dissolved in methanol'(1.5 mL), and the solution was treated with 10% hydrogen chloride-methanol solution (1.5 mL). The obtained solid was collected by filtration and washed with methanol, then dried under reduced pressure to obtain Compound 487 (37.8 mg, yield 89%). ESI-MS m/z: 321 [M+H]⁺; ¹H-NMR (DMSO-d₆)δ(ppm) : 2.75 (s, 6H), 4.44 (s, 2H), 4.55 (s, 2H), 7.11 (dd, J = 2.0, 8.8 Hz, 1H), 7.41 (s, 1H), 7.58 (s, 1H), 7.61 (d, J = 8.8 Hz, 1H), 7.65 (s, 1H), 8.57 (s, 1H), 9.47 (s, 1H), 10.19 (br s, 3H), 14.10 (s, 1H).

### Example 21:

### 5-{[N-(Methyl)cyclohexylamino]methyl}-7-[1H-5-(piperidinomethyl) indol-2-yl)isoindolinone hydrochloride (Compound 488)

### Step 1

In a similar manner to Steps 6 and 7 of Example 6, the crude product obtained using 5-formyl-3-hydroxy-7-iodo-2-(1-methyl-1-phenylethyl)isoindolinon e (90.0 mg, 0.214 mmol), N-methylcyclohexylamine (557 µL, 4.27 mmol), acetic acid (244 µL, 4.27 mmol), sodium triacetoxyborohydride (181 mg, 0.856 mmol) and acetonitrile (2.3 mL) was treated with triethylsilane (170 µL, 1.07 mmol), trifluoroacetic acid (330 µL, 4.28 mmol) and nitromethane (2.8 mL) to obtain 5-{[N-(methyl)cyclohexylamino]methyl}-7-iodoisoindolinone (62.5 mg, yield 76%).
¹H-NMR (DMSO-*d*₆)δ(ppm) : 1.00-1.37 (m, 6H), 1.52-1.63 (m, 1H), 1.69-1.87 (m, 4H), 2.10 (s, 3H), 3.59 (s, 2H), 4.25 (s, 2H), 7.50 (s, 1H), 7.82 (s, 1H), 8.64 (s, 1H).

### Step 2

5-{[N-(Methyl)cyclohexylamino]methyl}-7-iodoisoindolinone (61.0 mg, 0.159 mmol) was dissolved in a mixed solvent of dimethoxyethane (2.4 mL) and water (0.11 mL), and the solution was added with Compound BD (114 mg, 0.318 mmol), potassium carbonate (110 mg, 0.795 mmol) and [bis(diphenylphosphino)ferrocene]dichloropalladium (13.0 mg, 0.0159 mmol), followed by stirring at 90 °C for 6.0 hours. The reaction mixture was added with water, and extracted with ethyl acetate. The organic layer was evaporated under reduced pressure, and the residue was purified by silica gel column chromatography (hexane/ethyl acetate=95/5-30/70) to obtain 5-{[N-(methyl)cyclohexylamino]methyl}-7-[1-(tert-butoxycarbonyl) -5- (piperidinomethyl) indol-2-yl] isoindolinone (34.7 mg, yield 38%). ESI-MS *m*/*z*: 571 [M+H]⁺

### Step 3

In a similar manner to Step 8 of Example 1, Compound 488 (26.0 mg, 91%) was obtained using 5-{[N-(methyl)cyclohexylamino]methyl}-7-[1-(tert-butoxycarbonyl) -5-(piperidinomethyl)indol-2-yl]isoindolinone (34.7 mg, 0.0608 mmol), 10% hydrogen chloride-methanol solution (0.75 mL) and methanol (0.75 mL).
ESI-MS *m*/*z*: 471 [M+H]⁺; ¹H-NMR (DMSO-*d*₆) δ: 1.11-1.65 (m, 14H), 1.70-1.93 (m, 4H), 2.17 (s, 3H), 2.27-2.38 (m, 3H), 3.45 (s, 2H), 3.70 (s, 2H), 4.48 (s, 2H), 7.07 (d, *J* = 8.3 Hz, 1H), 7.13 (s, 1H), 7.38 (d, *J* = 8.3 Hz, 1H), 7.42 (s, 1H), 7.44 (s, 1H), 8.04 (s, 1H), 9.18 (s, 1H), 13.87 (s, 1H).

### Example 22:

### 5-{[N-(Methyl)phenylamino]methyl}-7-[1H-5-(piperidinomethyl)indo 1-2-yl)isoindolinone (Compound 489)

### Step 1

In a similar manner to Step 6 and 7 of Example 6, the crude product obtained using 5-formyl-3-hydroxy-7-iodo-2-(1-methyl-1-phenylethyl)isoindolinon e (90.0 mg, 0.214 mmol), N-methylaniline (463 µL, 4.27 mmol), acetic acid (244 µL, 4.27 mmol), sodium triacetoxyborohydride (181 mg, 0.856 mmol) and acetonitrile (2.3 mL) was treated with triethylsilane (85.7 µL, 0.537 mmol), trifluoroacetic acid (165 µL, 2.14 mmol) and nitromethane (1.4 mL) to obtain 5-{[N-(methyl)phenylamino]methyl}-7-iodoisoindolinone (19.8 mg, yield 25%).
ESI-MS *m*/*z*: 379 [M+H]⁺; ¹H-NMR (DMSO-*d*₆) δ: 3.00 (s, 3H), 4.22 (s, 2H), 4.63 (s, 2H), 6.64 (t, *J* = 7.6 Hz, 1H), 6.71 (d, *J* = 8.7 Hz, 2H), 7.16 (t, *J* = 7.6 Hz, 2H), 7.38 (s, 1H), 7.75 (s, 1H), 8.65 (s, 1H).

### Step 2

In a similar manner to Step 2 of Example 21, 5-{[N-(methyl)phenylamino]methyl}-7-[1-(tert-butoxycarbonyl)-5-( piperidinomethyl)indol-2-yl]isoindolinone (22.4 mg, yield 83%) was obtained using 5-{[N-(methyl)phenylamino]methyl}-7-iodoisoindolinone (18.0 mg, 0.0476 mmol), Compound BD (34.1 mg, 0.0952 mmol), potassium carbonate (32.9 mg, 0.238 mmol), [bis(diphenylphosphino)ferrocene]dichloropalladium (3.89 mg, 0.00476 mmol) and dimethoxyethane (0.72 mL)/water (34 µL).

### Step 3

In a similar manner to Step 8 of Example 1, Compound 489 (17.3 mg) was quantitatively obtained using 5-{[N-(methyl)phenylamino]methyl}-7-[1-(tert-butoxycarbonyl)-5-( piperidinomethyl)indol-2-yl]isoindolinone (21.0 mg, 0.0372 mmol), 10% hydrogen chloride-methanol solution (1.5 mL) and methanol (1.5 mL).
ESI-MS *m*/*z*: 465 [M+H]⁺; ¹H-NMR (DMSO-*d*₆)δ(ppm) : 1.39 (br s, 2H), 1.49 (br s, 4H), 2.33 (br s, 4H), 3.10 (s, 3H), 3.47 (s, 2H), 4.45 (s, 2H), 4.72 (s, 2H), 6.63 (t, *J* = 7.2 Hz, 1H), 6.77 (d, *J* = 8.0 Hz, 2H), 7.03-7.10 (m, 2H), 7.17 (t, *J* = 8.0 Hz, 2H), 7.27 (s, 1H), 7.38 (d, *J* = 8.9 Hz, 1H), 7.43 (s, 1H), 8.02 (s, 1H), 9.20 (s, 1H), 13.86 (s, 1H).

### Example 23:

### 5-(Dimethylaminomethyl)-7-[1H-5-(2-propyloxy)indol-2-yl)isoindol inone hydrochloride (Compound 490)

### Step 1

In a similar manner to Step 1 of Example 15, 5-(dimethylaminomethyl)-7-iodoisoindolinone (50.0 mg, 0.158 mmol) was dissolved in dimethoxyethane (5 mL), and the solution was treated with Compound CJ (101 mg, 0.316 mmol), [bis(diphenylphosphino)ferrocene]dichloropalladium (10.3 mg, 0.013 mmol), potassium carbonate (109 mg, 0.789 mmol) and water (0.114 mL, 6.33 mmol), followed by purification by preparative thin-layer chromatography (chloroform/methanol=9/1) to obtain 5-(dimethylaminomethyl)-7-[1-(tert-butoxycarbonyl)-5-(2-propylox y)indol-2-yl]isoindolinone (55.1 mg, yield 75%). ESI-MS *m*/*z*: 464 [M+H]⁺; ¹H-NMR (CDCl₃)δ(ppm) : 1.27 (s, 9H), 1.35 (d, *J* = 6.1 Hz, 6H), 2.29 (s, 6H), 3.54 (s, 2H), 4.41 (s, 2H), 4.54 (m, 1H), 6.50 (s, 1H), 6.83 (br s, 1H), 6.93 (dd, *J* = 2.4, 8.9 Hz, 1H), 7.02 (d, *J* = 2.4 Hz, 1H), 7.39 (s, 1H), 7.44 (s, 1H), 8.14 (d, *J* = 8.9 Hz, 1H).

### Step 2

In a similar manner to Step 8 of Example 1, 5-(dimethylaminomethyl)-7-[1-(tert-butoxycarbonyl)-5-(2-propylox y)indol-2-yl]isoindolinone (52.5 mg, 0.113 mmol) was dissolved in methanol (1.5 mL), and the solution was treated with 10% hydrogen chloride-methanol solution (1.5 mL). The obtained solid was collected by filtration and washed with methanol, then dried under reduced pressure to obtain Compound 490 (36.8 mg, yield 81%). ESI-MS *m*/*z*: 364 [M+H]⁺; ¹H-NMR (DMSO-*d*₆)δ(ppm) : 1.27 (d, *J* = 5.9 Hz, 6H), 2.75 (s, 6H), 4.41 (s, 2H), 4.53 (s, 2H), 4.55 (m, 1H), 6.77 (dd, *J* = 2.1, 8.9 Hz, 1H), 7.06 (d, *J* = 2.1 Hz, 1H), 7.19 (s, 1H), 7.36 (d, *J* = 8.9 Hz, 1H), 7.58 (s, 1H), 8.42 (s, 1H), 9.38 (s, 1H), 10.83 (br s, 1H), 13.71 (s, 1H).

### Example 24:

### 5-(1-Hydroxy-2-methylpropyl)-7-[1H-5-(piperidinomethyl)indol-2-y l)isoindolinone hydrochloride (Compound 491)

### Step 1

In a similar manner to Step 5 of Example 6, 5-(dimethoxymethyl)-3-hydroxy-7-iodo-2-(1-methyl-1-phenylethyl)i soindolinone (100 mg, 0.214 mmol) was dissolved in acetonitrile (2 mL), and the solution was treated with 1 mol/L hydrochloric acid (1 mL) to obtain 5-formyl-3-hydroxy-7-iodo-2-(1-methyl-1-phenylethyl)isoindolinon e (117 mg).

### Step 2

5-Formyl-3-hydroxy-7-iodo-2-(1-methyl-1-phenylethyl)isoindo linone (117 mg) was dissolved in THF (4 mL), and the solution was added with isopropylmagnesium bromide-THF solution (2.0 mol/L, 0.27 mL, 0.54 mmol) at -78 °C, followed by stirring at -78 °C for 10 minutes, then at 0 °C for 30 minutes. The reaction mixture was cooled to -78 °C, and added with isopropylmagnesium bromide-THF solution (2.0 mol/L, 0.14 mL, 0.28 mmol) at -78 °C, followed by stirring at -78 °C for 10 minutes, then at 0 °C for 1 hour. The reaction mixture was added with water and 1 mol/L hydrochloric acid, and extracted with ethyl acetate. The organic layer was washed with saturated brine, and dried over anhydrous sodium sulfate. The solvent was evaporated under reduced pressure, and the residue was purified by preparative thin-layer chromatography (hexane/ethyl acetate=50/50) to obtain 5-(1-hydroxy-2-methylpropyl)-3-hydroxy-7-iodo-2-(1-methyl-1-phen ylethyl)isoindolinone (25.8 mg, yield 26%, 2 steps). ESI-MS *m*/*z*: 466 [M+H]⁺

### Step 3

In a similar manner to Step 7 of Example 6, 5-(1-hydroxy-2-methylpropyl)-3-hydroxy-7-iodo-2-(1-methyl-1-phen ylethyl)isoindolinone (140 mg, 0.301 mmol) was dissolved in nitromethane (5 mL), and the solution was treated with trifluoroacetic acid (0.464 mL, 6.02 mmol) and triethylsilane (0.243 mL, 1.50 mmol), followed by purification by slurry using hexane to obtain 5-(1-hydroxy-2-methylpropyl)-7-iodoisoindolinone (47.3 mg, yield 47%).
ESI-MS *m*/*z*: 332 [M+H]⁺; ¹H-NMR (CD₃OD)δ(ppm) : 0.81 (d, *J* = 6.8 Hz, 3H), 0.95 (d, *J* = 6.8 Hz, 3H), 1.92 (m, 1H), 4.35 (s, 2H), 4.39 (d, *J* = 6.6 Hz, 1H), 7.52 (s, 1H), 7.88 (s, 1H).

### Step 4

In a similar manner to Step 1 of Example 15, 5-(1-hydroxy-2-methylpropyl)-7-iodoisoindolinone (7.8 mg, 0.024 mmol) was dissolved in dimethoxyethane (1 mL), and the solution was treated with Compound BD (25.4 mg, 0.0709 mmol), [bis(diphenylphosphino)ferrocene]dichloropalladium (1.9 mg, 0.0023 mmol), potassium carbonate (16.0 mg, 0.116 mmol) and water (0.017 mL, 0.94 mmol), followed by purification by preparative thin-layer chromatography (chloroform/methanol/7 mol/L ammonia-methanol solution=25/1/1) to obtain 5-(1-hydroxy-2-methylpropyl)-7-[1-(tert-butoxycarbonyl)-5-(piper idinomethyl)indol-2-yl]isoindolinone (13.0 mg).
ESI-MS *m*/*z*: 518 [M+H]⁺; ¹H-NMR (CDCl₃)δ(ppm) : 0.88 (d, *J* = 6.8 Hz, 3H), 0.99 (d, *J* = 6.8 Hz, 3H), 1.27 (s, 9H), 1.29 (m, 1H), 1.38-1.48 (m, 2H), 1.52-1.64 (m, 4H), 2.36-2.47 (m, 4H), 3.58 (s, 2H), 4.38 (s, 2H), 4.53 (d, *J* = 6.3 Hz, 1H), 6.49 (s, 1H), 7.16 (s, 1H), 7.25-7.47 (m, 5H), 8.18 (d, *J* = 8.4 Hz, 1H).

### Step 5

In a similar manner to Step 8 of Example 1, 5-(1-hydroxy-2-methylpropyl)-7-[1-(tert-butoxycarbonyl)-5-(piper idinomethyl)indol-2-yl]isoindolinone (13.0 mg) was dissolved in methanol (0.5 mL), and the solution was treated with 10% hydrogen chloride-methanol solution (0.5 mL), followed by condensing under reduced pressure, and adding methanol and ethyl acetate thereto. The obtained solid was collected by filtration and washed with ethyl acetate, then dried under reduced pressure to obtain Compound 491 (6.5 mg, yield 61%, 2 steps).
ESI-MS *m*/*z*: 418 [M+H]⁺; ¹H-NMR (DMSO-*d*₆)δ(ppm) : 0.84 (d, *J* = 6.8 Hz, 3H), 0.89 (d, *J* = 6.6Hz, 3H), 1.36 (m, 1H), 1.62-1.86 (m, 6H), 2.78-2.97 (m, 2H), 3.36-3.49 (m, 2H), 4.25-4.38 (m ,2H), 4.46 (m, 1H), 4.51 (s, 2H), 5.38 (d, *J* = 4.8 Hz, 1H), 7.27 (m, 1H), 7.29 (s, 1H), 7.45 (s, 1H), 7.55 (d, *J* = 8.6 Hz, 1H), 7.75 (s, 1H), 8.08 (s, 1H), 9.24 (s, 1H), 9.69 (br s, 1H), 14.13 (s, 1H).

### Example 25:

### 5-(1-Oxo-2-methylpropyl)-7-[1H-5-(2-propyloxy)indol-2-yl)isoindo linone hydrochloride (Compound 492)

### Step 1

5-(1-Hydroxy-2-methylpropyl)-7-iodoisoindolinone (18.8 mg, 0. 0568 mmol) was dissolved in dichloromethane (1 mL), and the solution was added with pyridine (0.023 mL, 0.28 mmol), then with Dess-Martin periodinane (24.1 mg, 0.0568 mmol) at 0 °C, followed by stirring at 0 °C for 1 hour and at room temperature for 3 hours. The reaction mixture was added with Dess-Martinperiodinane (12.0 mg, 0.0283 mmol), followed by stirring at room temperature for 1 hour. The reaction mixture was added with water and saturated aqueous sodium hydrogencarbonate solution, and extracted with ethyl acetate. The organic layer was washed with saturated brine, and dried over anhydrous sodium sulfate. The solvent was evaporated under reduced pressure to obtain 5-(1-oxo-2-methylpropyl)-7-iodoisoindolinone (27.5 mg).

### Step 2

In a similar manner to Step 1 of Example 15, 5-(1-oxo-2-methylpropyl)-7-iodoisoindolinone (27.5 mg) was dissolved in dimethoxyethane (2 mL), and the solution was treated with Compound BD (41.0 mg, 0.114 mmol), [bis(diphenylphosphino)ferrocene]dichloropalladium (3.7 mg, 0.0045 mmol), potassium carbonate (39.0 mg, 0.282 mmol) and water (0.041 mL, 2.3 mmol), followed by purification by preparative thin-layer chromatography (chloroform/methanol/7 mol/L ammonia-methanol solution=25/1/1) to obtain 5-(1-oxo-2-methylpropyl)-7-[1-(tert-butoxycarbonyl)-5-(piperidin omethyl)indol-2-yl]isoindolinone (25.7 mg, yield 87%, 2 steps).
ESI-MS *m*/*z*: 516 [M+H]⁺; ¹H-NMR (CDCl₃)δ(ppm) : 1.26 (d, *J* = 6.8 Hz, 6H), 1.32 (s, 9H), 1.38-1.48 (m, 2H), 1.52-1.64 (m, 4H), 2.36-2.45 (m, 4H), 3.587 (s, 2H), 3.593 (sep, *J* = 6.8 Hz, 1H), 4.48 (s, 2H), 6.61 (s, 1H), 7.32 (d, *J* = 8.8 Hz, 1H), 7.51 (s, 1H), 8.01 (s, 1H), 8.02 (s, 1H), 8.17 (d, *J* = 8.8 Hz, 1H).

### Step 2

5-(1-Oxo-2-methylpropyl)-7-[1-(tert-butoxycarbonyl)-5-(pipe ridinomethyl)indol-2-yl]isoindolinone (25.7 mg, 0.498 mmol) was dissolved in 4 mol/L hydrochloric acid-ethyl acetate solution (2 mL), followed by stirring at 60 °C for 5 hours. The obtained solid was collected by filtration, and washed with ethyl acetate, followed by drying under reduced pressure to obtain Compound 492 (15.5 mg, yield 69%).
ESI-MS *m*/*z*: 416 [M+H]⁺; ¹H-NMR (DMSO-*d*₆)δ(ppm) : 1.17 (d, *J* = 6.8 Hz, 6H), 1.37 (m, 1H), 1.59-1.88 (m, 5H), 2.79-2.96 (m, 2H), 3.26-3.40 (m, 2H), 3.86 (sep, *J* = 6.8 Hz, 1H), 4.34 (d, *J* = 4.5 Hz, 2H), 4.59 (s, 2H), 7.30 (d, *J* = 8.1 Hz, 1H), 7.47 (s, 1H), 7.59 (d, *J* = 8.1 Hz, 1H), 7.78 (s, 1H), 8.04 (d, J = 0.8 Hz, 1H), 8.60 (d, *J* = 0.8 Hz, 1H), 9.53 (s, 1H), 9.62 (br s, 1H), 13.97 (s, 1H).

### Example 26:

### 5-Diethylaminomethyl-7-[1H-5-(piperidinomethyl)indol-2-yl)isoind olinone (Compound 493)

### Step 1

In a similar manner to Steps 6 and 7 of Example 6, the crude product obtained using 5-formyl-3-hydroxy-7-iodo-2-(1-methyl-1-phenylethyl)isoindolinon e (90.0 mg, 0.214 mmol), diethylamine (442 µL, 4.27 mmol), acetic acid (244 µL, 4.27 mmol), sodium triacetoxyborohydride (181 mg, 0.856 mmol) and acetonitrile (2.3 mL) was treated with triethylsilane (171 µL, 1.07 mmol), trifluoroacetic acid (330 µL, 4.28 mmol) and nitromethane (2.6 mL) to obtain 5-(diethylaminomethyl)-7-iodoisoindolinone (56.6 mg, yield 77%).
ESI-MS *m*/*z*: 345 [M+H]⁺; ¹H-NMR (DMSO-*d*₆) δ: 0.98 (t, *J* = 7.1 Hz, 6H), 3.21-3.39 (m, 4H), 3.58 (s, 2H), 4.25 (s, 2H), 7.52 (s, 1H), 7.84 (s, 1H), 8.65 (s, 1H).

### Step 2

In a similar manner to Step 2 of Example 21, 5-(diethylaminomethyl)-7-[1-(tert-butoxycarbonyl)-5-(piperidinom ethyl)indol-2-yl]isoindolinone (73.7 mg, yield 87%) was obtained using 5-(diethylaminomethyl)-7-iodoisoindolinone (55.0 mg, 0.160 mmol), Compound BD (115 mg, 0.320 mmol), potassium carbonate (111 mg, 0.800 mmol), [bis(diphenylphosphino)ferrocene]dichloropalladium (13.0 mg, 0.0160 mmol) and dimethoxyethane (0.72 mL)/water (34 µL).
ESI-MS *m*/*z*: 531 [M+H]⁺

### Step 3

In a similar manner to Step 8 of Example 1, Compound 493 (10.1 mg, yield 17%) was obtained using 5-(diethylaminomethyl)-7-[1-(tert-butoxycarbonyl)-5-(piperidinom ethyl)indol-2-yl]isoindolinone (73.0 mg, 0.138 mmol), 10% hydrogen chloride-methanol solution (1.5 mL) and methanol (1.5 mL).
ESI-MS *m*/*z*: 431 [M+H]⁺; ¹H-NMR (DMSO-*d*₆)δ(ppm) : 1.03 (t, *J* = 6.8 Hz, 6H), 1.40 (br s, 2H), 1.49 (br s, 4H), 2.34 (br s, 4H), 3.27-3.44 (m, 4H), 3.54 (s, 2H), 3.69 (s, 2H), 4.49 (s, 2H), 7.07 (d, *J* = 8.1 Hz, 1H), 7.14 (s, 1H), 7.38 (d, *J* = 8.1 Hz, 1H), 7.44 (s, 2H), 8.06 (s, 1H), 9.18 (s, 1H), 13.87 (s, 1H).

### Example 27:

### 5-{[N-Methyl(2-hydroxyethyl)amino]methyl}-7-[1H-5-(piperidinomet hyl)indol-2-yl)isoindolinone (Compound 494)

### Step 1

In a similar manner to Steps 6 and 7 of Example 6, the crude product obtained using 5-formyl-3-hydroxy-7-iodo-2-(1-methyl-1-phenylethyl)isoindolinon e (90.0 mg, 0.214 mmol), N-methylethanolamine (516 µL, 6.42 mmol), acetic acid (368 µL, 6.42 mmol), sodium triacetoxyborohydride (272 mg, 1.28 mmol) and acetonitrile (2.7 mL) was treated with triethylsilane (171 µL, 1.07 mmol), trifluoroacetic acid (330 µL, 4.28 mmol) and nitromethane (2.6 mL) to obtain 5-{[N-methyl(2-hydroxyethyl)amino]methyl}-7-iodoisoindolinone (74.1 mg, quantitative).
ESI-MS *m*/*z*: 347 [M+H]⁺

### Step 2

In a similar manner to Step 2 of Example 21, 5-{[N-methyl(2-hydroxyethyl)amino]methyl}-7-[1-(tert-butoxycarbo nyl)-5-(piperidinomethyl)indol-2-yl]isoindolinone (88.1 mg, yield 77%) was obtained using 5-{[N-methyl(2-hydroxyethyl)amino]methyl}-7-iodoisoindolinone (74.1 mg, 0.214 mmol), Compound BD (153 mg, 0.428 mmol), potassium carbonate (148 mg, 1.07 mmol), [bis(diphenylphosphino)ferrocene]dichloropalladium (17.5 mg, 0.0214 mmol) and dimethoxyethane (3.0 mL)/water (0.15 mL).
ESI-MS *m*/*z*: 533 [M+H]⁺

### Step 3

In a similar manner to Step 8 of Example 1, Compound 494 (11.6 mg, yield 16%) was obtained using 5-{[N-methyl(2-hydroxyethyl)amino]methyl}-7-[1-(tert-butoxycarbo nyl)-5-(piperidinomethyl)indol-2-yl]isoindolinone (87.0 mg, 0.163 mmol), 10% hydrogen chloride-methanol solution (1.7 mL) and methanol (1.7 mL).
ESI-MS *m*/*z*: 433 [M+H]⁺; ¹H-NMR (DMSO-*d*₆)δ(ppm) : 1.40 (br s, 2H), 1.50 (br s, 4H), 2.23 (s, 3H), 2.35 (br s, 4H), 3.28 (s, 2H), 3.39-3.49 (m, 2H), 3.51-3.62 (m, 2H), 3.67 (s, 2H), 4.45 (t, *J* = 4.9 Hz, 1H), 4.49 (s, 2H), 7.02-7.24 (m, 2H), 7.34-7.51 (m, 2H), 7.45 (s, 1H), 8.08 (s, 1H), 9.20 (s, 1H), 13.87 (s, 1H).

### Example 28:

### 5-{[N-(Methyl)ethylamino]methyl}-7-[1H-5-(piperidinomethyl)indol -2-yl)isoindolinone (Compound 495)

### Step 1

In a similar manner to Steps 6 and 7 of Example 6, the crude product obtained using 5-formyl-3-hydroxy-7-iodo-2-(1-methyl-1-phenylethyl)isoindolinon e (90.0 mg, 0.214 mmol), N-methylethylamine (367 µL, 4.27 mmol), acetic acid (244 µL, 4.27 mmol), sodium triacetoxyborohydride (181 mg, 0.856 mmol) and acetonitrile (2.3 mL) was treated with triethylsilane (170 µL, 1.07 mmol), trifluoroacetic acid (330 µL, 4.28 mmol) and nitromethane (2.5 mL) to obtain 5-{[N-(methyl)ethylamino]methyl}-7-iodoisoindolinone (49.3 mg, yield 70%).
ESI-MS *m*/*z*: 331 [M+H]⁺; ¹H-NMR (DMSO-*d*₆)δ(ppm) : 1. 03 (t, *J* = 7.1 Hz, 3H), 2.11 (s, 3H), 2.40 (q, *J* = 7.1 Hz, 2H), 3.50 (s, 2H), 4.26 (s, 2H), 7.50 (s, 1H), 7.82 (s, 1H), 8.66 (s, 1H).

### Step 2

In a similar manner to Step 2 of Example 21, 5-{[N-(methyl)ethylamino]methyl}-7-[1-(tert-butoxycarbonyl)-5-(p iperidinomethyl)indol-2-yl]isoindolinone (62.6 mg, yield 84%) was obtained using 5-{[N-(methyl)ethylamino]methyl}-7-iodoisoindolinone (48.0 mg, 0.145 mmol), Compound BD (104 mg, 0.290 mmol), potassium carbonate (100 mg, 0.725 mmol), [bis(diphenylphosphino)ferrocene]dichloropalladium (11.9 mg, 0.0145 mmol) and dimethoxyethane (1.9 mL)/water (0.10 mL).
¹H-NMR (DMSO-*d*₆) δ: 1.02-1.08 (m, 6H), 1.14 (s, 9H), 1.33-1.66 (m, 8H), 2.15 (s, 3H), 2.30-2.59 (m, 4H), 3.59 (s, 2H), 4.37 (s, 2H), 6.55-6.64 (m, 1H), 7.21-7.32 (m, 1H), 7.36 (s, 1H), 7.46-7.55 (m, 2H), 8.53 (s, 1H).

### Step 3

In a similar manner to Step 8 of Example 1, Compound 495 (36.5 mg, yield 74%) was obtained using 5-{[N-(methyl)ethylamino]methyl}-7-[1-(tert-butoxycarbonyl)-5-(p iperidinomethyl)indol-2-yl]isoindolinone (61.0 mg, 0.118 mmol), 10% hydrogen chloride-methanol solution (1.5 mL) and methanol (1.5 mL).
ESI-MS *m*/*z*: 417 [M+H]⁺; ¹H-NMR (DMSO-*d*₆)δ(ppm) : 1.07 (t, *J* = 7.0 Hz, 3H), 1.40 (br s, 2H), 1.49 (br s, 4H), 2.18 (s, 3H), 2.35 (br s, 4H), 3.28-3.43 (m, 2H), 3.48 (s, 2H), 3.61 (s, 2H), 4.49 (s, 2H), 7.07 (d, *J* = 8.6 Hz, 1H), 7.15 (s, 1H), 7.39 (d, *J* = 8.6 Hz, 1H), 7.42 (s, 1H), 7.45 (s, 1H), 8.04 (s, 1H), 9.20 (s, 1H), 13.87 (s, 1H).

### Example 29:

### 5-(Pyrrolidin-1-ylmethyl)-7-[1H-5-(piperidinomethyl)indol-2-yl)i soindolinone (Compound 496)

### Step 1

In a similar manner to Steps 6 and 7 of Example 6, the crude product obtained using 5-formyl-3-hydroxy-7-iodo-2-(1-methyl-1-phenylethyl)isoindolinon e (90.0 mg, 0.214 mmol), pyrrolidine (356 µL, 4.27 mmol), acetic acid (244 µL, 4.27 mmol), sodium triacetoxyborohydride (181 mg, 0.856 mmol) and acetonitrile (2.3 mL) was treated with triethylsilane (170 µL, 1. 07 mmol), trifluoroacetic acid (330 µL, 4.28 mmol) and nitromethane (2.6 mL) to obtain 5-(pyrrolidin-1-ylmethyl)-7-iodoisoindolinone (53.0 mg, yield 72%).
ESI-MS *m*/*z*: 343 [M+H]⁺; ¹H-NMR (DMSO-*d*₆)δ(ppm) : 1.62-1.78 (m, 4H), 2.36-2.52 (m, 4H), 3.63 (s, 2H), 4.25 (s, 2H), 7.52 (s, 1H), 7.82 (s, 1H), 8.66 (s, 1H).

### Step 2

In a similar manner to Step 2 of Example 21, 5-(pyrrolidin-1-ylmethyl)-7-[1-(tert-butoxycarbonyl)-5-(piperidi nomethyl)indol-2-yl]isoindolinone (62.5 mg, yield 78%) was obtained using 5-(pyrrolidin-1-ylmethyl)-7-iodoisoindolinone (52.0 mg, 0.152 mmol), Compound BD (109 mg, 0.304 mmol), potassium carbonate (105 mg, 0.760 mmol), [bis(diphenylphosphino)ferrocene]dichloropalladium (12.4 mg, 0.0152 mmol) and dimethoxyethane (2.1 mL)/water (0.11 mL).
ESI-MS *m*/*z*: 529 [M+H]⁺

### Step 3

In a similar manner to Step 8 of Example 1, Compound 496 (40.9 mg, yield 83%) was obtained using 5-(pyrrolidin-1-ylmethyl)-7-[1-(tert-butoxycarbonyl)-5-(piperidi nomethyl)indol-2-yl]isoindolinone (61.0 mg, 0.115 mmol), 10% hydrogen chloride-methanol solution (1.5 mL) and methanol (1.5 mL).
ESI-MS *m*/*z*: 429 [M+H]⁺; ¹H-NMR (DMSO-*d*₆)δ(ppm): 1.40 (br s, 2H), 1.49 (br s, 4H), 1.74 (br s, 4H), 2.34 (br s, 4H), 3.27-3.42 (m, 4H), 3.47 (s, 2H), 3.74 (s, 2H), 4.49 (s, 2H), 7.07 (d, J = 8.1 Hz, 1H), 7.16 (s, 1H), 7.38 (d, J = 8.1 Hz, 1H), 7.43 (s, 1H), 7.45 (s, 1H), 8.05 (s, 1H), 9.20 (s, 1H), 13.86 (s, 1H).

### Example 30:

### 5-(3H-Imidazo[4,5-b]pyridin-2-yl)-7-[1H-5-(piperidinomethyl)indo 1-2-yl)isoindolinone (Compound 497)

### Step 1

5-Formyl-3-hydroxy-7-iodo-2-(1-methyl-1-phenylethyl)isoindo linone (90.0 mg, 0.214 mmol) was dissolved in DMF (2.3 mL), and the solution was added with 2,3-diaminopyridine (27.0 mg, 0.256 mmol), followed by stirring at room temperature for 1.5 hours. The mixture was added with sodium disulfite (81.4 mg, 0.428 mmol) followed by stirring at 100 °C for 10 hours. The reaction mixture was added with saturated aqueous sodium hydrogencarbonate solution and saturated aqueous sodium thiosulfate solution, and extracted with ethyl acetate. The organic layer was concentrated under reduced pressure to obtain the crude prodduct. In a similar manner to Step 7 of Example 473, the crude product was treated with triethylsilane (188 µL, 1.18 mmol), trifluoroacetic acid (362 µL, 4.70 mmol) and nitromethane (3.0 mL) to obtain 5-(3H-imidazo[4,5-b]pyridin-2-yl)-7-iodoisoindolinone (39.8 mg, yield 45%).
ESI-MS *m*/*z*: 377 [M+H]⁺; ¹H-NMR (DMSO-*d*₆)δ(ppm): 4.41 (s, 2H), 7.30 (dd, *J* = 8.0, 4.7 Hz, 1H), 8.06 (s, 1H), 8.36-8.45 (m, 2H), 8.76 (s, 1H), 8.90 (s, 1H).

### Step 2

In a similar manner to Step 2 of Example 21, 5-(3H-imidazo[4,5-b]pyridin-2-yl)-7-[1-(tert-butoxycarbonyl)-5-( piperidinomethyl)indol-2-yl]isoindolinone (49.6 mg, yield 87%) was obtained using 5-(3H-imidazo[4,5-b]pyridin-2-yl)-7-iodoisoindolinone (38.0 mg, 0.101 mmol), Compound BD (72.4 mg, 0.202 mmol), potassium carbonate (69.8 mg, 0.505 mmol), [bis(diphenylphosphino)ferrocene]dichloropalladium (8.25 mg, 0.0101 mmol) and dimethoxyethane (1.5 mL)/water (72 µL).
ESI-MS *m*/*z*: 563 [M+H]⁺

### Step 3

In a similar manner to Step 8 of Example 1, Compound 497 (19.4 mg, yield 49%) was obtained using 5-(3H-imidazo[4,5-b]pyridin-2-yl)-7-[1-(tert-butoxycarbonyl)-5-piperidinomethyl)indol-2-yl]isoindolinone (48.0 mg, 0.0853 mmol), 10% hydrogen chloride-methanol solution (1.2 mL) and methanol (1.2 mL).
ESI-MS *m*/*z*: 463 [M+H]⁺; ¹H-NMR (DMSO-d₆)δ(ppm): 1.33-1.55 (m, 6H), 2.36 (s, 2H), 3.51 (s, 2H), 4.64 (s, 2H), 7.13 (d, *J* = 9.6 Hz, 1H), 7.27-7.36 (m, 2H), 7.40-7.46 (m, 2H), 7.52 (s, 1H), 8.14 (s, 1H), 8.33 (s, 1H), 8.41 (s, 1H), 9.00 (s, 1H), 9.45 (s, 1H), 13.91 (s, 1H).

### Example 31:

### 5-(1-Methoxyethyl)-7-[1H-5-(piperidinomethyl)indol-2-yl)isoindol inone hydrochloride (Compound 498)

### Step 1

In a similar manner to Step 2 of Example 1, 4-acetylbenzoic acid (2.00 g, 12.2 mmol) was dissolved in DMF (20 mL), and the solution was treated with EDCI (4.67 g, 24.4 mmol), HOBt monohydrate (1.87 g, 12.2 mmol) and cumylamine (3. 50 mL, 24.4 mmol). Then, the reaction mixture was added with water. The precipitated solid was collected by filtration and washed by water, followed by drying under reduced pressure to obtain 4-acetyl-N-(1-methyl-1-phenylethyl)benzamide (2.96 g, yield 86%).
ESI-MS *m*/*z*: 282 [M+H]⁺; ¹H-NMR (CDCl₃)δ(ppm): 1.84 (s, 6H), 2.63 (s, 3H), 6.43 (br s, 1H), 7.23-7.49 (m, 5H), 7.79-7.88 (m, 2H), 7.95-8.05 (m, 2H).

### Step 2

In a similar manner to Step 2 of Example 6, 4-acetyl-N-(1-methyl-1-phenylethyl)benzamide (2.96 g, 26.3 mmol) was dissolved in methanol (30 mL), and the solution was treated with trimethyl orthoformate (2.30 mL, 21.0 mmol) and p-toluenesulfonic acid monohydrate (20.0 mg, 0.105 mmol). Then, the reaction mixture was added with saturated aqueous sodium hydrogencarbonate solution and water. The precipitated solid was collected by filtration and washed with water, followed by drying under reduced pressure to obtain 4-(1,1-dimethoxyethyl)-N-(1-methyl-1-phenylethyl)benzamide (3.37g, yield 98%).
ESI-MS *m*/*z*: 328 [M+H]⁺; ¹H-NMR (CDCl₃)δ(ppm): 1.53 (s, 3H), 1.83 (s, 6H), 3.18 (s, 6H), 6.39 (br s, 1H), 7.24 (m, 1H), 7.29-7.60 (m, 6H), 7.71-7.78 (m, 2H).

### Step 3

In a similar manner to Step 3 of Example 1, 4-(1,1-dimethoxyethyl)-N-(1-methyl-1-phenylethyl)benzamide (3.30 g, 10.1 mmol) was dissolved in THF (95 mL), and the solution was treated with TMEDA (4.90 mL, 32.5 mmol), sec-buthyl lithium-hexane solution (1.00 mol/L, 32.3 mL, 32.3 mmol) and DMF (1.70 mL, 22.0 mmol), followed by purification by slurry using diisopropylether and hexane to obtain 5-(1,1-dimethoxyethyl)-3-hydroxy-2-(1-methyl-1-phenylethyl)isoin dolinone (3.18 g, yield 89%).
ESI-MS *m*/*z*: 356 [M+H]⁺; ¹H-NMR (CDCl₃)δ(ppm): 1.53 (s, 3H), 1.94 (s, 3H), 1.99 (s, 3H), 3.19 (s, 6H), 6.15 (s, 1H), 7.18-7.47 (m, 5H), 7.59-7.77 (m, 3H).

### Step 4

In a similar manner to Step 4 of Example 1, 5-(1,1-dimethoxyethyl)-3-hydroxy-2-(1-methyl-1-phenylethyl)isoin dolinone (3.10 g, 8.72 mmol) was dissolved in THF (120 mL), and the solution was treated with TMEDA (4.20 mL, 27.8 mmol), sec-buthyl lithium-hexane solution (1.00 mol/L, 28.0 mL, 28.0 mmol) and iodine (3.32 g, 13.1 mmol), followed by purification by flush column chromatography (hexane/ethyl acetate=90/10, 70/30) to obtain 5-(1,1-dimethoxyethyl)-3-hydroxy-7-iodo-2-(1-methyl-1-phenylethy l)isoindolinone (3.52 g, yield 84%).
ESI-MS *m*/*z*: 482 [M+H]⁺; ¹H-NMR (CDCl₃)δ(ppm): 1.52 (s, 3H), 1.96 (s, 3H), 1.99 (s, 3H), 2.18 (d, *J* = 10.2 Hz, 1H), 3.19 (s, 6H), 5.98 (d, *J* = 10.2 Hz, 1H), 7.21-7.49 (m, 5H), 7.66 (s, 1H), 8.08 (s, 1H).

### Step 5

In a similar manner to Step 7 of Example 6, 5-(1,1-dimethoxyethyl)-3-hydroxy-7-iodo-2-(1-methyl-1-phenylethy l)isoindolinone (500 mg, 1.04 mmol) was dissolved in nitromethane (9 mL), and the solution was added with trifluoroacetic acid (0.831 mL, 5.20 mmol) and triethylsilane (0.801 mL, 10.4 mmol), followed by purification by preparative thin-layer chromatography (ethyl acetate) to obtain 5-(1-methoxyethyl)-7-iodoisoindolinone (59 mg, yield 18%) and 5-acetyl-7-iodoisoindolinone (109 mg, yield 35%). 5-(1-methoxyethyl)-7-iodoisoindolinone
ESI-MS *m*/*z*: 318 [M+H]⁺; ¹H-NMR (CDCl₃)δ(ppm): 1.45 (d, *J* = 6.5 Hz, 3H), 3.27 (s, 3H), 4.34 (q, *J* = 6.5 Hz, 1H), 4.36 (s, 2H), 6.89 (m, 1H), 7.43 (s, 1H), 7.85 (s, 1H). 5-acetyl-7-iodoisoindolinone
ESI-MS *m*/*z*: 302 [M+H]⁺; ¹H-NMR (CDCl₃)δ(ppm): 2.66 (s, 3H), 4.43 (s, 2H), 6.96 (br s, 1H), 8.03 (s, 1H), 8.46 (s, 1H).

### Step 6

In a similar manner to Step 7 of Example 1, 5-(1-methoxyethyl)-7-iodoisoindolinone (50.0 mg, 0.158 mmol) was dissolved in acetonitrile (2.5 mL), and the solution was treated with Compound BD (113 mg, 0.315 mmol), palladium acetate (2.8 mg, 0.010 mmol), tri(o-tolyl)phosphine (7.7 mg, 0.030 mmol) and triethylamine (0.220 mL, 1.58 mmol), followed by purification by preparative thin-layer chromatography (chloroform/methanol=4/1) to obtain 5-(1-methoxyethyl)-7-[1-(tert-butoxycarbonyl)-5-(piperidinomethy l)indol-2-yl]isoindolinone (64.4 mg, yield 81%).
ESI-MS *m*/*z*: 504 [M+H]⁺; ¹H-NMR (CDCl₃)δ(ppm): 1.30 (s, 9H), 1.38-1.46 (m, 2H), 1.50 (d, *J* = 6.6 Hz, 3H), 1.51-1.80 (m, 4H), 2.30-2.54 (m, 4H), 3.31 (s, 3H), 3.62 (br s, 2H), 4.42 (m, 1H), 4.44 (s, 2H), 6.51 (br s, 1H), 6.57 (s, 1H), 7.29 (m, 1H), 7.39 (s, 1H), 7.43 (s, 1H), 7.50 (s, 1H), 8.19 (d, *J* = 8.7 Hz, 1H).

### Step 7

In a similar manner to Step 8 of Example 1, 5-(1-methoxyethyl)-7-[1-(tert-butoxycarbonyl)-5-(piperidinomethy l)indol-2-yl]isoindolinone (62.0 mg, 0.123 mmol) was dissolved in methanol (1.5 mL), and the solution was treated with 10% hydrogen chloride-methanol solution (1.5 mL), followed by condensing under reduced pressure, and adding methanol and ethyl acetate thereto. The obtained solid was collected by filtration and washed with ethyl acetate, then dried under reduced pressure to obtain Compound 498 (48.3 mg, yield 89%).
ESI-MS *m*/*z*: 404 [M+H]⁺; ¹H-NMR (DMSO-*d*₆)δ(ppm): 1.35 (m, 1H), 1.43 (d, *J* = 6.4 Hz, 3H),1.55-1.90 (m, 5H), 2.80-2.90 (m, 2H), 3.21 (s, 3H), 3.25-3.40 (m, 2H), 4.28-4.33 (m, 2H), 4.51 (s, 2H), 4.53 (m, 1H), 7.27 (d, *J* = 7.8 Hz, 1H), 7.33 (s, 1H), 7.46 (s, 1H), 7.55 (d, *J* = 7.8 Hz, 1H), 7.75 (s, 1H), 8.09 (s, 1H), 9.27 (s, 1H), 9.66 (br s, 1H), 14.09 (s, 1H).

### Example 32:

### 5-{[N-(Methyl)phenetylamino]methyl}-7-[1H-5-(piperidinomethyl)in dol-2-yl)isoindolinone (Compound 499)

### Step 1

In a similar manner to Steps 6 and 7 of Example 6, the crude product obtained using 5-formyl-3-hydroxy-7-iodo-2-(1-methyl-1-phenylethyl)isoindolinon e (90.0 mg, 0.214 mmol), N- (methyl)phenetylamine (621 µL, 4.27 mmol), acetic acid (244 µL, 4.27 mmol), sodium triacetoxyborohydride (181 mg, 0.856 mmol) and acetonitrile (2.3 mL) was treated with triethylsilane (171 µL, 1. 07 mmol), trifluoroacetic acid (330 µL, 4.28 mmol) and nitromethane (2.9 mL) to obtain 5-{[N-(methyl)phenetylamino]methyl}-7-iodoisoindolinone (61.2 mg, yield 70%).
ESI-MS *m*/*z*: 407 [M+H]⁺; ¹H-NMR (DMSO-*d*₆)δ(ppm): 2.21 (s, 3H), 2.59 (t, *J* = 7.5 Hz, 2H), 2.78 (t, *J* = 7.5 Hz, 2H), 3.58 (s, 2H), 4.22 (s, 2H), 7.13-7.32 (m, 5H), 7.40 (s, 1H), 7.78 (s, 1H), 8.65 (s, 1H).

### Step 2

In a similar manner to Step 2 of Example 21, 5-{[N-(methyl)phenetylamino]methyl}-7-[1-(tert-butoxycarbonyl)-5 -(piperidinomethyl)indol-2-yl]isoindolinone (87.7 mg, quantitative yield) was obtained using 5-{[N-(methyl)phenetylamino]methyl}-7-iodoisoindolinone (60.0 mg, 0.148 mmol), Compound BD (106 mg, 0.296 mmol), potassium carbonate (102 mg, 0.740 mmol), [bis(diphenylphosphino)ferrocene]dichloropalladium (12.1 mg, 0.0148 mmol) and dimethoxyethane (2.4 mL)/water (0.11 mL). ESI-MS *m*/*z*: 593 [M+H]⁺

### Step 3

In a similar manner to Step 8 of Example 1, Compound 499 (57.9 mg, yield 79%) was obtained using 5-{[N-(methyl)phenetylamino]methyl}-7-[1-(tert-butoxycarbonyl)-5 -(piperidinomethyl)indol-2-yl]isoindolinone (87.7 mg, 0.148 mmol), 10% hydrogen chloride-methanol solution (2.9 mL) and methanol (2.9 mL).
ESI-MS *m*/*z:* 493 [M+H]⁺; ¹H-NMR (DMSO-*d*₆)δ(ppm): 1.34-1.55 (m, 6H), 2.30-2.40 (m, 2H), 2.66 (t, *J* = 7.6 Hz, 2H), 2.83 (t, *J* = 7.6 Hz, 2H), 3.48 (s, 2H), 3.69 (s, 2H), 4.44 (s, 2H), 7.04-7.11 (m, 2H), 7.17-7.33 (m, 5H), 7.38 (s,1H), 7.45 (s, 1H), 8.00 (s, 1H), 9.19 (s, 1H), 13.86 (s, 1H).

### Example 33:

### (E)-5-[2-(Ethoxycarbonyl)vinyl]-7-[1H-5-(piperidinomethyl)indol-2-yl)isoindolinone (Compound 500)

### Step 1

Potassium tert-butoxide (36.0 mg, 0.321 mmol) and triethyl phosphonoacetate (84.9 µL, 0.428 mmol) was stirred in THF (2.3 mL) at -78 °C for 30 minutes. The mixture was added with 5-formyl-3-hydroxy-7-iodo-2-(1-methyl-1-phenylethyl)isoindolinon e (90.0 mg, 0.214 mmol) followed by stirring at 0 °C for 1.5 hours. The reaction mixture was added with water, and extracted with ethyl acetate. The organic layer was concentrated under reduced pressure. The residue was crystallized by adding a mixed solvent of hexane (1.2 mL) /ethyl acetate (0.3 mL) to obtain the crude product. In a similar manner to Step 7 of Example 473, the crude product was treated with triethylsilane (146 µL, 0.916 mmol), trifluoroacetic acid (282 µL, 3.66 mmol) and nitromethane (2.3 mL) to obtain (E)-5-[2-(ethoxycarbonyl)vinyl]-7-iodoisoindolinone (76.4 mg, quantitative yield).
¹H-NMR (DMSO-*d*₆)δ(ppm) : 1.27 (t, *J* = 7.2 Hz, 3H), 4.21 (q, *J* = 7.2 Hz, 2H), 4.29 (s, 2H), 6.80 (d, *J* = 16.0 Hz, 1H), 7.69 (d, *J* = 16.0 Hz, 1H), 7.97 (s, 1H), 8.25 (s, 1H), 8.82 (s, 1H).

### Step 2

In a similar manner to Step 2 of Example 21, (E)-5-[2-(ethoxycarbonyl)vinyl]-7-[1-(tert-butoxycarbonyl)-5-(pi peridinomethyl)indol-2-yl]isoindolinone (61.8 mg, yield 56%) was obtainedusing (E)-5-[2- (ethoxycarbonyl)vinyl] -7-iodoisoindolinone (72.0 mg, 0.202 mmol), Compound BD (145 mg, 0.404 mmol), potassium carbonate (140 mg, 1.01 mmol), [bis(diphenylphosphino)ferrocene]dichloropalladium (16.5 mg, 0.0202 mmol) and dimethoxyethane (2.9 mL)/water (0.15 mL).
ESI-MS *m*/*z*: 544 [M+H]⁺

### Step 3

In a similar manner to Step 8 of Example 1, Compound 500 (24.5 mg, quantitative yield) was obtained using (E)-5-[2-(ethoxycarbonyl)vinyl]-7-[1-(tert-butoxycarbonyl)-5-(pi peridinomethyl) indol-2-yl] isoindolinone (30. 0 mg, 0. 0552 mmol), 4. 0 mol/L hydrogen chloride-ethyl acetate solution (0.90 mL) and ethanol (0.90 mL).
ESI-MS m/z: 444 [M+H]⁺; ¹H-NMR (DMSO-*d*₆)δ(ppm): 1.30 (t, *J* = 6. 9 Hz, 3H), 1.49 (m, 6H), 2.34 (s, 2H), 4.24 (q, *J* = 6.9 Hz, 2H), 4.52 (s, 2H), 6.94 (d, *J* = 16.2 Hz, 1H), 7.08 (d, *J* = 8.1 Hz, 1H), 7.37-7.47 (m, 4H), 7.83 (d, *J* = 16.2 Hz, 4H), 7.84 (s, 1H), 8.50 (s, 1H), 9.37 (s, 1H), 13.81 (s, 1H).

### Example 34:

### 5-Acetyl-7-[1H-5-(piperidinomethyl)indol-2-yl)isoindolinone (Compound 501)

### Step 1

In a similar manner to Step 7 of Example 1, 5-acetyl-7-iodoisoindolinone (50.0 mg, 0.166 mmol) was dissolved in acetonitrile (2.5 mL), and the solution was treated with Compound BD (113 mg, 0.315 mmol), palladium acetate (2.8 mg, 0.010 mmol), tri(o-tolyl)phosphine (7.7 mg, 0.030 mmol) and triethylamine (0.231 mL, 1.66 mmol), followed by purification by preparative thin-layer chromatography (chloroform/methanol=4/1) to obtain 5-acetyl-7-[1-(tert-butoxycarbonyl)-5-(piperidinomethyl)indol-2-yl]isoindolinone (63.0 mg, yield 78%).
ESI-MS *m*/*z*: 488 [M+H]⁺; ¹H-NMR (CDCl₃)δ(ppm): 1.32-1.74 (m, 6H), 1.36 (s, 9H), 2.33-2.56 (m, 4H), 2.69 (s, 3H), 3.61 (s, 2H), 4.50 (s, 2H), 6.62 (s, 1H), 6.72 (s, 1H), 7.32 (d, *J* = 7.9 Hz, 1H), 7.52 (s, 1H), 8.04 (s, 1H), 8.05 (s, 1H), 8.15 (d, *J* = 7.9 Hz, 1H).

### Step 2

5-Acetyl-7-[1-(tert-butoxycarbonyl)-5-(piperidinomethyl)ind ol-2-yl] isoindolinone (60.5 mg, 0.124 mmol) was dissolved in 4 mol/L hydrogen chloride-ethyl acetate solution (5 mL) followed by stirring at 70 °C for 5 hours. The reaction mixture was concentrated under reduced pressure, and purified by preparative thin-layer chromatography (chloroform/methanol/7 mol/L ammonia-methanol solution=24/1/1) to obtain Compound 501 (10.4 mg, yield 22%). ESI-MS *m*/*z*: 388 [M+H]⁺; ¹H-NMR (DMSO-*d*₆)δ(ppm): 1.16-1.89 (m, 6H), 2.49 (s, 3H), 3.13-3.49 (m, 4H), 4.08-4.15 (m, 2H), 4.57 (s, 2H), 7.23 (br s, 1H), 7.40 (s, 1H), 7.50 (m, 1H), 7.67 (m, 1H), 8.01 (s, 1H), 8.59 (s, 1H), 9.52 (s, 1H), 13.86 (br s, 1H).
ESI-MS *m*/*z:* 388 [M+H]⁺; ¹H-NMR (DMSO-*d*₆)δ(ppm): 1.16-1.89 (m, 6H), 2.49 (s, 3H), 3.13-3.49 (m, 4H), 4.08-4.15 (m, 2H), 4.57 (s, 2H), 7.23 (br s, 1H), 7.40 (s, 1H), 7.50 (m, 1H), 7.67 (m, 1H), 8.01 (s, 1H), 8.59 (s, 1H), 9.52 (s, 1H), 13.86 (br s, 1H).

### Example 35:

### 5-tert-Butyl-7-[1H-5-(piperidinomethyl)indol-2-yl)isoindolinone (Compound 502)

### Step 1

In a similar manner to Step 2 of Example 1, 4-tert-butyl-N-(1-methyl-1-phenylethyl)benzamide (4.75 g, yield 72%) was obtained using 4-tert-butylbenzoic acid (4. 00 g, 22.4 mmol), EDCI (5.59 g, 29.2 mmol), HOBt monohydrate (686 mg, 4.48 mmol), cumylamine (4.20 mL, 29.2 mmol), and DMF (640 mL). ¹H-NMR (DMSO-*d*₆) δ: 1.27 (s, 9H), 1.66 (s, 6H), 7.16 (t, *J* = 9.7 Hz, 1H), 7.27 (t, *J* = 7.5 Hz, 2H), 7.36 (d, *J* = 7.5 Hz, 2H), 7.46 (d, *J* = 6.6 Hz, 2H), 7.77 (d, *J* = 6.6 Hz, 2H), 8.32 (s, 1H).

### Step 2

4-tert-Butyl-N-(1-methyl-1-phenylethyl)benzamide (500 mg, 1. 69 mmol) was dissolved in THF (5.0 mL), and n-butyllithium-n-hexane solution (2.6 mol/L, 1.95 mL, 5.08 mmol) was added dropwise thereto at -78 °C, followed by stirring at -78 °C for 30 minutes and at 0 °C for 30 minutes. The mixture was cooled to -78 °C again, and added with DMF (393 µL, 5.08 mmol), followed by stirring at -78 °C for 1.0 hour and at room temperature for 1.0 hour. The reaction mixture was added with saturated aqueous ammonium chloride solution, and extracted with ethyl acetate. The organic layer was concentrated under reduced pressure. The residue was crystallized by adding mixed solvent of hexane (2.0 mL)/ethyl acetate (0.23 mL) to obtain 5-tert-butyl-3-hydroxy-2-(1-methyl-1-phenylethyl)isoindolinone (468 mg, yield 86%).
¹H-NMR (DMSO-*d*₆) δ: 1.34 (s, 9H), 1.79 (s, 3H), 1.81 (s, 3H), 6.27 (d, *J* = 9.9 Hz, 1H), 6.52 (d, *J* = 9.9 Hz, 1H), 7.14 (t, *J* = 7.5 Hz, 1H), 7.24 (t, *J* = 7.5 Hz, 2H), 7.36-7.44 (m, 3H), 7.55 (d, *J* = 8.1, Hz, 1H), 7.60 (s, 1H).

### Step 3

5-tert-Butyl-3-hydroxy-2-(1-methyl-1-phenylethyl)isoindolin one (425 mg, 1.31 mmol) was dissolved in THF (11 mL), and n-butyllithium-n-hexane solution (2.6 mol/L, 1.52 mL, 3.94 mmol) was added dropwise thereto at -78 °C, followed by stirring at -78 °C for 30 minutes and at 0 °C for 30 minutes. The mixture was cooled to -78 °C again, and added with iodine (997 mg, 3.93 mmol), followed by stirring at -78 °C for 1.0 hour and at 0 °C for 2.5 hours. The reaction mixture was added with saturated aqueous sodium hydrogencarbonate solution and saturated aqueous sodium thiosulfate solution, and extracted with ethyl acetate. The organic layer was concentrated under reduced pressure. The residue was purified by silica gel column chromatography (hexane/ethyl acetate=100/0-85/15) to obtain 5-tert-butyl-3-hydroxy-7-iodo-2-(1-methyl-1-phenylethyl)isoindol inone (143 mg, yield 24%).
ESI-MS *m*/*z*: 450 [M+H]⁺; ¹H-NMR (DMSO-*d*₆)δ(ppm): 1.32 (s, 9H), 1.78 (s, 3H), 1.80 (s, 3H), 6.17 (d, *J* = 9.9 Hz, 1H), 6.53 (d, *J* = 9.9 Hz, 1H), 7.16 (t, *J* = 7.1 Hz, 1H), 7.26 (t, *J* = 7.1 Hz, 2H), 7.39 (d, *J* = 7.1 Hz, 2H), 7.63 (s, 1H), 7.86 (s, 1H).

### Step 4

In a similar manner to step 7 of Example 6, 5-*tert*-butyl-7-iodoisoindolinone (40.8 mg, yield 41%) was obtained using 5-*tert*-butyl-3-hydroxy-7-iodo-2-(1-methyl-1-phenylethyl)isoindol inone (143 mg, 0.318 mmol), triethylsilane (254 µL, 1.59 mmol), trifluoroacetyc acid (490 µL, 6.36 mmol) and nitromethane (3.6 mL). ESI-MS *m*/*z:* 316 [M+H]⁺; ¹H-NMR (DMSO-*d*₆)δ(ppm) : 1.32 (t, *J* = 2.5 Hz, 10H), 4.25 (s, 2H), 7.62 (s, 1H), 7.85 (s, 1H), 8.62 (s, 1H).

### Step 5

In a similar manner to Step 2 of Example 21, 5-*tert*-butyl-7-[1-(tert-butoxycarbonyl)-5-(piperidinomethyl)indo l-2-yl]isoindolinone (59.7 mg, yield 96%) was obtained using 5-*tert*-butyl-7-iodoisoindolinone (39.0 mg, 0.124 mmol), Compound BD (88.8 mg, 0.248 mmol), potassium carbonate (85.7 mg, 0.620 mmol), [bis(diphenylphosphino)ferrocene]dichloropalladium (10.1 mg, 0.0124 mmol) and dimethoxyethane (1.6 mL)/water (89 µL).
ESI-MS *m*/*z*: 502 [M+H]⁺

### Step 6

In a similar manner to Step 8 of Example 1, Compound 502 (47.8 mg, quantitative yield) was obtained using 5-*tert*-butyl-7-[1-(tert-butoxycarbonyl)-5-(piperidinomethyl)indo l-2-yl]isoindolinone (59.7 mg, 0.119 mmol), 10% hydrogen chloride-methanol solution (1.8 mL) and methanol (1.8 mL).
ESI-MS *m*/*z:* 402 [M+H]⁺; ¹H-NMR (DMSO-*d*₆)δ(ppm) : 1.32-1.55 (m, 6H), 1.40 (s, 9H), 2.33 (s, 4H), 3.48 (s, 2H), 4.48 (s, 2H), 7.07 (d, *J* = 7.3 Hz, 1H), 7.25 (s, 1H), 7.38 (d, *J* = 7.3 Hz, 1H), 7.45 (s, 1H), 7.52 (s, 1H), 8.10 (s, 1H), 9.17 (s, 1H), 13.83 (s, 1H).

### Example 36:

### 5-(4-Ethoxycarbonyl-5-propyl-3H-imidazol-2-yl)-7-[1H-5-(piperidi nomethyl)indol-2-yl)isoindolinone (Compound 503)

### Step 1

5-Formyl-3-hydroxy-7-iodo-2-(1-methyl-1-phenylethyl)isoindo linone (50.0 mg, 0.119 mmol) was dissolved in acetic acid (1.3 mL), and the solution was added with ammonium acetate (45.9 mg, 0.595 mmol) and ethyl 2,3-dioxohexanoate (38.7 mg, 0.225 mmol) followed by stirring at 65 °C for 9.5 hours. The reaction mixture was added with saturated aqueous sodium hydrogencarbonate solution, and extracted with ethyl acetate. The organic layer was concentrated under reduced pressure. The residue was purified by silica gel column chromatography (hexane/ethyl acetate=90/10-40/60) to obtain 5-(4-ethoxycarbonyl-5-propyl-3H-imidazol-2-yl)-3-hydroxy-7-iodo-2-(1-methyl-1-phenylethyl)isoindolinone (19.6 mg, yield 29%).
ESI-MS *m*/*z*: 574 [M+H]⁺; ¹H-NMR (DMSO-*d*₆)δ(ppm): 0.93 (t, *J* = 7.3 Hz, 3H), 1.26-1.34 (m, 3H), 1.62-1.72 (m, 2H), 1.81 (s, 3H), 1.83 (s, 3H), 3.24-3.31 (m, 2H), 4.18-4.33 (m, 2H), 6.28 (d, *J* = 9.9 Hz, 1H), 6.67 (d, *J* = 9.9 Hz, 1H), 7.18 (t, *J* = 7.5 Hz, 1H), 7.28 (t, *J* = 7.5 Hz, 2H), 7.40 (d, *J* = 7.5 Hz, 2H), 8.17 (s, 1H), 8.48 (s, 1H), 13.07 (s, 1H).

### Step 2

In a similar manner to step 7 of Example 6, 5-(4-ethoxycarbonyl-5-propyl-3H-imidazol-2-yl)-7-iodoisoindolino ne (7.50 mg, yield 52%) was obtained using 5-(4-ethoxycarbonyl-5-propyl-3H-imidazol-2-yl)-3-hydroxy-7-iodo-2-(1-methyl-1-phenylethyl)isoindolinone (19.0 mg, 0.0331 mmol), triethylsilane (26.5 µL, 0.166 mmol), trifluoroacetyc acid (51.0 µL, 0.662 mmol) and nitromethane (0.48 mL).
ESI-MS *m*/*z*: 440 [M+H]⁺; ¹H-NMR (DMSO-*d*₆)δ(ppm): 0.92 (t, *J* = 6.8 Hz, 3H), 1.30 (t, *J* = 7.2 Hz, 3H), 1.67 (t, *J* = 7.5 Hz, 2H), 2.92 (t, *J* = 7.5 Hz, 2H), 4.25 (q, *J* = 7.2 Hz, 2H), 4.34 (s, 2H), 8.15 (s, 1H), 8.47 (s, 1H), 8.78 (s, 1H), 13.00 (s, 1H).

### Step 3

In a similar manner to Step 2 of Example 21, 5-(4-ethoxycarbonyl-5-propyl-3H-imidazol-2-yl)-7-[1-(tert-butoxy carbonyl)-5-(piperidinomethyl)indol-2-yl]isoindolinone (7.30 mg, yield 68%) was obtained using 5-(4-ethoxycarbonyl-5-propyl-3H-imidazol-2-yl)-7-iodoisoindolino ne (7.50 mg, 0.0171 mmol), Compound BD (12.3 mg, 0.0342 mmol), potassium carbonate (11.8 mg, 0.0855 mmol), [bis(diphenylphosphino)ferrocene]dichloropalladium (1.40 mg, 0.00171 mmol) and dimethoxyethane (0.30 mL)/water (12 µL). ESI-MS *m*/*z*: 627 [M+H]⁺

### Step 4

In a similar manner to Step 8 of Example 1, Compound 503 (1.50 mg, yield 24%) was obtained using 5-(4-ethoxycarbonyl-5-propyl-3H-imidazol-2-yl)-7-[1-(tert-butoxy carbonyl)-5-(piperidinomethyl)indol-2-yl]isoindolinone (7.30 mg, 0.117 mmol), 10% hydrogen chloride-methanol solution (0.37 mL) and methanol (0.37 mL).
ESI-MS *m*/*z*: 526 [M+H]⁺; ¹H-NMR (DMSO-*d*₆)δ(ppm): 0.96 (t, *J* = 7.1 Hz, 3H), 1.29-1.56 (m, 11H), 2.35 (s, 4H), 2.98 (t, *J* = 8.4 Hz, 2H), 3.50 (s, 2H), 4.27 (q, *J* = 7.1 Hz, 2H), 4.57 (s, 2H), 7.11 (d, *J* = 6.6 Hz, 1H), 7.31 (s, 1H), 7.42 (d, *J* = 6.6 Hz, 1H), 7.50 (s, 1H), 8.06 (s, 1H), 8.64 (s, 1H), 9.32 (s, 1H), 13.08 (s, 1H), 13.83 (s, 1H).

### Example 37:

### 5-(Dimethylaminomethyl)-7-[1H-5-(diethylaminomethyl)indol-2-yl)i soindolinone (Compound 504)

### Step 1

In a similar manner to Step 1 of Example 15, 5-(dimethylaminomethyl)-7-iodoisoindolinone (133 mg, 0.422 mmol) was dissolved in dimethoxyethane (13 mL), and the solution was treated with Compound CE (292 mg, 0.844 mmol), [bis(diphenylphosphino)ferrocene]dichloropalladium (28.0 mg, 0.0343 mmol), potassium carbonate (292 mg, 2.11 mmol) and water (0.304 mL, 16.9 mmol), followed by purification by preparative thin-layer chromatography (chloroform/methanol/7 mol/L ammonia-methanol solution=600/100/1) to obtain 5-(dimethylaminomethyl)-7-[1-(tert-butoxycarbonyl)-5-(diethylami nomethyl)indol-2-yl]isoindolinone (172 mg, yield 83%).
ESI-MS *m*/*z*: 491 [M+H]⁺; ¹H-NMR (CDCl₃)δ(ppm): 1.08 (t, *J* = 7.0 Hz, 6H), 1.30 (s, 9H), 2.29 (s, 6H), 2.57 (q, *J* = 7.0 Hz, 4H), 3.55 (s, 2H), 3.69 (br s, 2H), 4.42 (s, 2H), 6.43 (s, 1H), 6.56 (s, 1H), 7.30 (d, *J* = 9.2 Hz, 1H), 7.40 (s, 1H), 7.45 (s, 1H), 7.50 (s, 1H), 8.18 (d, *J* = 9.2 Hz, 1H).

### Step 2

In a similar manner to Step 8 of Example 1, 5-(dimethylaminomethyl)-7-[1-(tert-butoxycarbonyl)-5-(diethylami nomethyl) indol-2-yl] isoindolinone (170 mg, 0.346 mmol) was dissolved in methanol (3 mL), and the solution was treated with 10% hydrogen chloride-methanol solution (3 mL), followed by condensing under reduced pressure. The residue was added with methanol and ethyl acetate. The obtained solid was collected by filtration and washed with ethyl acetate, followed by drying under reduced pressure to obtain Compound 504 (70.6 mg, yield 26%).
ESI-MS *m*/*z*: 391 [M+H]⁺; ¹H-NMR (DMSO-*d*₆)δ(ppm): 1.00 (t, *J* = 6.9 Hz, 6H), 2.21 (s, 6H), 2.35-2.68 (m, 4H), 3.54 (s, 2H), 3.57-3.64 (m, 2H), 4.47 (s, 2H), 7.10 (d, *J* = 8.5 Hz, 1H), 7.15 (s, 1H), 7.39 (d, *J* = 8.5 Hz, 1H), 7.40 (s, 1H), 7.48 (br s, 1H), 8.02 (s, 1H), 9.20 (s, 1H), 13.86 (br s, 1H).

### Example 38:

### 5-Fluoro-7-[1H-5-(piperidinomethyl)indol-2-yl)isoindolinone hydrochloride (Compound 505)

### Step 1

In a similar manner to Step 2 of Example 1, 4-fluorobenzoic acid (5.00 g, 35.7 mmol) was dissolved in DMF (40 mL), and the solution was treated with EDCI (13.7 g, 71.5 mmol), HOBt monohydrate (5.47 g, 35.7 mmol) and cumylamine (10.3 mL, 71.6 mmol), followed by adding water. The precipitated solid was collected by filtration and washed with water, followed by drying under reduced pressure to obtain 4-fluoro-N-(1-methyl-1-phenylethyl)benzamide (8.06 g, yield 88%). ESI-MS *m*/*z*: 258 [M+H]⁺; ¹H-NMR (CDCl₃)δ(ppm): 1. 82 (s, 6H), 6.33 (br s, 1H), 7.05-7.13 (m, 2H), 7.26 (m, 1H), 7.32-7.39 (m, 2H), 7.42-7.48 (m, 2H), 7.73-7.80 (m, 2H).

### Step 2

4-Fluoro-N-(1-methyl-1-phenylethyl)benzamide (8.00 g, 31.1 mmol) was dissolved in THF (200 mL), and the solution was added with sec-butyllithium-hexane solution (1.00 mol/L, 78.0 mL, 78.0 mmol) by drops at -78 °C for 30 minutes under argon atmosphere, followed by stirring at the same temperature for 1 hour. Then, the mixture was added with DMF (6.00 mL, 77.5 mmol) and warmed from -78 °C to room temperature over 2 hours. The reaction mixture was added with water, and extracted with ethyl acetate. The organic layer was washed with saturated brine and dried over anhydrous sodium sulfate. The solvent was evaporated under reduced pressure and the residue was purified by flush column chromatography (chloroform/methanol=9/1) and then by slurry using diisopropylether to obtain 5-fluoro-3-hydroxy-2-(1-methyl-phenylethyl)isoindolinone (2.44 g, yield 28%).
ESI-MS *m*/*z*: 286 [M+H]⁺; ¹H-NMR (CDCl₃)δ(ppm): 1.79 (s, 3H), 1.81 (s, 3H), 6.30 (br s, 1H), 6.68 (br s, 1H), 7.15 (m, 1H), 7.22-7.32 (m, 3H), 7.33-7.40 (m, 2H), 7.43 (dd, *J* = 2.1, 8.4 Hz, 1H), 7.54 (dd, *J* = 5.0, 8.3 Hz, 1H).

### Step 3

5-Fluoro-3-hydroxy-2-(1-methyl-phenylethyl)isoindolinone (2.00 g, 7.01 mmol) was dissolved in THF (57 mL), and the solution was added with sec-butyllithium-hexane solution (1.00 mol/L, 17.5 mL, 17.5 mmol) by drops at -78 °C for 15 minutes under argon atmosphere, followed by stirring at the same temperature for 1.5 hours. Then, the mixture was added with a solution of iodine (1.78 mg, 7.01 mmol) in THF (5 mL) and warmed from -78 °C to room temperature over 2.5 hours. The reaction mixture was added with saturated aqueous sodium thiosulfate solution, and extracted with ethyl acetate. The organic layer was washed with saturated brine and dried over anhydrous sodium sulfate. The solvent was evaporated under reduced pressure and the residue was purified by flush column chromatography (hexane/ethyl acetate=95/5, 80/20) to obtain 5-fluoro-3-hydroxy-7-iodo-2-(1-methyl-phenylethyl)isoindolinone (1.35 g, yield 47%).
ESI-MS *m*/*z*: 412 [M+H]⁺; ¹H-NMR (CDCl₃)δ(ppm): 1.95 (s, 3H), 1.99 (s, 3H), 2.24 (d, *J* = 10.1 Hz, 1H), 5.93 (d, *J* = 10.1 Hz, 1H), 7.19-7.50 (m, 6H), 7.65 (dd, *J* = 2.1, 8.4 Hz, 1H).

### Step 4

In a similar manner to step 7 of Example 6, 5-fluoro-3-hydroxy-7-iodo-2-(1-methyl-phenylethyl)isoindolinone (1.35 g, 3.29 mmol) was dissolved in nitromethane (30 mL), and the solution was treated with trifluoroacetic acid (5.07 mL, 65.8 mmol) and triethylsilane (2.66 mL, 16.5 mmol), followed by purification by slurry using chloroform to obtain 5-fluoro-7-iodoisoindolinone (606 mg, yield 66%).
ESI-MS *m*/*z*: 277 [M+H]⁺; ¹H-NMR (CDCl₃)δ(ppm): 4.35 (s, 2H), 6.78 (br s, 1H), 7.18 (d, *J* = 7.4 Hz, 1H), 7.67 (d, *J* = 8.6 Hz, 1H).

### Step 5

In a similar manner to Step 1 of Example 15, 5-fluoro-7-iodoisoindolinone (300 mg, 1.08 mmol) was dissolved in dimethoxyethane (30 mL), and the solution was treated with Compound BD (776 mg, 2.17 mmol), [bis(diphenylphosphino)ferrocene]dichloropalladium (71.0 mg, 0.0869 mmol), potassium carbonate (746 mg, 5.40 mmol) and water (0.778 mL, 43.2 mmol), followed by purification by flush column chromatography (chloroform/methanol=9/1) to obtain 5-fluoro-7-[1-(tert-butoxycarbonyl)-5-(piperidinomethyl)indol-2-yl]isoindolinone (283 mg, yield 57%).
ESI-MS *m*/*z:* 464 [M+H]⁺; ¹H-NMR (CDCl₃)δ(ppm): 1.21-1.50 (m, 6H), 1.36 (s, 9H), 2.30-2.50 (m, 4H), 3.58 (s, 2H), 4.42 (s, 2H), 6.50 (br s, 1H), 6.57 (s, 1H), 7.09-7.22 (m, 2H), 7.31 (d, *J* = 8.7 Hz, 1H), 7.49 (s, 1H), 8.15 (d, *J* = 8.7 Hz, 1H).

### Step 6

In a similar manner to Step 8 of Example 1, 5-fluoro-7-[1-(tert-butoxycarbonyl)-5-(piperidinomethyl)indol-2-yl]isoindolinone (45.6 mg, 0.0984 mmol) was dissolved in methanol (1.5 mL), and the solution was treated with 10% hydrogen chloride-methanol solution (1.5 mL). The obtained solid was collected by filtration and washed with methanol, followed by drying under reduced pressure to obtain Compound 505 (25.7 mg, yield 66%). ESI-MS *m*/*z*: 364 [M+H]⁺; ¹H-NMR (DMSO-*d*₆)δ(ppm) : 1.34 (m, 1H), 1.68-1.89 (m, 5H), 2.70-2.90 (m, 2H), 2.75-2.98 (m, 2H), 4.26-4.36 (m, 2H), 4.51 (s, 2H), 7.31 (d, *J* = 8.3 Hz, 1H), 7.41 (dd, *J* = 2.1, 7.7 Hz, 1H), 7.43 (s, 1H), 7.56 (d, *J* = 8.3 Hz, 1H), 7.77 (s, 1H), 8.06 (dd, *J* = 2.1, 11.2 Hz, 1H), 9.35 (s, 1H), 9.83 (br s, 1H), 14.13 (s, 1H).

### Example 39:

### 5-({N-Methyl[2-(3,4-dimethoxyphenyl)ethyl]amino}methyl)-7-[1H-5-(piperidinomethyl)indol-2-yl)isoindolinone (Compound 506)

### Step 1

In a similar manner to Steps 6 and 7 of Example 6, the crude product obtained using 5-formyl-3-hydroxy-7-iodo-2-(1-methyl-1-phenylethyl)isoindolinon e (90.0 mg, 0.214 mmol), 2-(3,4-dimethoxyphenyl)-N-methylethylamine (787 µL, 4.27 mmol), acetic acid (244 µL, 4.27 mmol), sodium triacetoxyborohydride (181 mg, 0.856 mmol) and acetonitrile (2.3 mL) was treated with triethylsilane (146 µL, 0. 916 mmol), trifluoroacetic acid (282 µL, 3.66 mmol) and nitromethane (2.8 mL) to obtain 5-({N-methyl[2-(3,4-dimethoxyphenyl)ethyl]amino}methyl)-7-iodois oindolinone (85.3 mg, yield 87%).
ESI-MS *m*/*z*: 467 [M+H]⁺; ¹H-NMR (DMSO-*d*₆)δ(ppm): 2.20 (s, 3H), 2.53-2.62 (m, 2H), 2.70 (t, *J* = 7.0 Hz, 2H), 3.58 (s, 2H), 3.71 (s, 3H), 3.71 (s, 3H), 4.21 (s, 2H), 6.70 (d, *J* = 8.1 Hz, 1H), 6.80 (s, 1H), 6.84 (d, *J* = 8.1 Hz, 1H), 7.40 (s, 1H), 7.79 (s, 1H), 8.66 (s, 1H).

### Step 2

In a similar manner to Step 2 of Example 21, 5-({N-methyl[2-(3,4-dimethoxyphenyl)ethyl]amino}methyl)-7-[1-(te rt-butoxycarbonyl)-5-(piperidinomethyl)indol-2-yl]isoindolinone (94.2 mg, yield 75%) was obtained using 5-({N-methyl[2-(3,4-dimethoxyphenyl)ethyl]amino}methyl)-7-iodois oindolinone (90.0 mg, 0.193 mmol), Compound BD (138 mg, 0.386 mmol), potassium carbonate (133 mg, 0.965 mmol), [bis(diphenylphosphino)ferrocene]dichloropalladium (15.8 mg, 0.0193 mmol) and dimethoxyethane (3.6 mL)/water (0.14 mL). ESI-MS *m*/*z*: 653 [M+H]⁺

### Step 3

In a similar manner to Step 8 of Example 1, Compound 506 (70.1 mg, yield 88%) was obtained using 5-({N-methyl[2-(3,4-dimethoxyphenyl)ethyl]amino}methyl)-7-[1-(te rt-butoxycarbonyl)-5-(piperidinomethyl)indol-2-yl]isoindolinone (94.2 mg, 0.144 mmol), 10% hydrogen chloride-methanol solution (2.4 mL) and methanol (2.4 mL).
ESI-MS *m*/*z*: 553 [M+H]⁺; ¹H-NMR (DMSO-*d*₆)δ(ppm): 1.30-1.58 (m, 6H), 2.26 (s, 3H), 2.34 (s, 4H), 2.60-2.68 (m, 2H), 2.71-2.80 (m, 2H), 3.47 (s, 2H), 3.67 (s, 3H), 3.70 (s, 3H), 4.44 (s, 2H), 6.73 (d, *J* = 9.9 Hz, 1H), 6.79-6.89 (m, 2H), 7.03-7.11 (m, 2H), 7.30 (s, 1H), 7.38 (d, *J* = 8.1 Hz, 1H), 7.45 (s, 1H), 8.00 (s, 1H), 9.19 (s, 1H), 13.86 (s, 1H).

### Example 40:

### 5-Chloro-7-[1H-5-(piperidinomethyl)indol-2-yl)isoindolinone (Compound 507)

### Step 1

In a similar manner to Step 2 of Example 1, 4-chloro-N-(1-methyl-1-phenylethyl)benzamide (5.32 g, yield 76%) was obtained using 4-chlorobenzoic acid (4.00 g, 25.5 mmol), EDCI (6.36 g, 33.2 mmol), HOBt monohydrate (781 mg, 5.10 mmol), cumylamine (4.77 mL, 33.2 mmol) and DMF (64 mL).
ESI-MS *m*/*z:* 275 [M+H]⁺

### Step 2

In a similar manner to Step 2 of Example 35, 5-chloro-3-hydroxy-2-(1-methyl-1-phenylethyl)isoindolinone (1.66 g, yield 90%) was obtained using 4-chloro-N-(1-methyl-1-phenylethyl)benzamide (1.68 g, 6.14 mmol), 1.5 mol/L n-butyllithium-n-hexane solution (12.1 mL, 18.4 mmol), DMF (1.42 mL, 18.4 mmol)and THF (17 mL).
ESI-MS *m*/*z*: 302 [M+H]⁺; ¹H-NMR (DMSO-*d*₆)δ(ppm): 1.79 (s, 3H), 1.81 (s, 3H), 6.32 (d, *J* = 9.7 Hz, 1H), 6.69 (d, *J* = 9.7 Hz, 1H), 7.15 (t, *J* = 8.3 Hz, 1H), 7.25 (t, *J* = 7.3 Hz, 2H), 7.37 (d, *J* = 7.3 Hz, 2H), 7.48-7.60 (m, 2H), 7.66 (d, *J* = 1.7 Hz, 1H).

### Step 3

In a similar manner to Step 3 of Example 35, 5-chloro-3-hydroxy-7-iodo-2-(1-methyl-1-phenylethyl)isoindolinon e (1.27 g, yield 60%) was obtained using 5-chloro-3-hydroxy-2-(1-methyl-1-phenylethyl)isoindolinone (1.50 g, 4.97 mmol), 1.5 mol/L n-butyllithium-n-hexane solution (19.6 mL, 29.8 mmol), iodine (10.1 g, 39.8 mmol)and THF (38 mL).
¹H-NMR (DMSO-*d*₆)δ(ppm): 1.78 (s, 3H), 1.80 (s, 3H), 6.20 (d, *J* = 9. 7 Hz, 1H), 6.67 (d, *J* = 9.7 Hz, 1H), 7.17 (t, *J* = 7.4 Hz, 1H), 7.27 (t, *J* = 7.4 Hz, 2H), 7.38 (d, *J* = 7.3 Hz, 2H), 7.70 (s, 1H), 8.00 (s, 1H).

### Step 4

In a similar manner to step 7 of Example 6, 5-chloro-7-iodoisoindolinone (323 mg, yield 94%) was obtained using 5-chloro-3-hydroxy-7-iodo-2-(1-methyl-1-phenylethyl)isoindolinon e (500 mg, 1.17 mmol), triethylsilane (934 µL, 5.85 mmol), trifluoroacetic acid (1.81 mL, 23.4 mmol) and nitromethane (13 mL). ESI-MS *m*/*z*: 292 [M-H]⁻; ¹H-NMR (DMSO-*d*₆)δ(ppm): 4.27 (s, 2H), 7.73 (s, 1H), 7.97 (s, 1H), 8.81 (s, 1H).

### Step 5

In a similar manner to Step 2 of Example 21, 5-chloro-7-[1-(tert-butoxycarbonyl)-5-(piperidinomethyl)indol-2-yl]isoindolinone (230 mg, yield 70%) was obtained using 5-chloro-7-iodoisoindolinone (200 mg, 0.681 mmol), Compound BD (488 mg, 1.36 mmol), potassium carbonate (471 mg, 3.41 mmol), [bis(diphenylphosphino)ferrocene]dichloropalladium (55.6 mg, 0.0681 mmol) and dimethoxyethane (8.0 mL)/water (0.49 mL).
¹H-NMR (DMSO-*d*₆)δ(ppm): 1.22 (s, 9H), 1.31-1.56 (m, 6H), 2.26-2.42 (m, 4H), 3.51 (s, 2H), 4.40 (s, 2H), 6.69 (s, 1H), 7.28 (d, *J* = 8.2 Hz, 1H), 7.50 (s, 1H), 7.52 (s, 1H), 7.72 (s, 1H), 8.08 (d, *J* = 8.6 Hz, 1H), 8.69 (s, 1H).

### Step 6

In a similar manner to Step 8 of Example 1, Compound 507 (33.3 mg, yield 84%) was obtained using 5-chloro-7-[1-(tert-butoxycarbonyl)-5-(piperidinomethyl)indol-2-yl]isoindolinone (50.0 mg, 0.104 mmol), 10% hydrogen chloride-methanol solution (1.3 mL) and methanol (1.3 mL).
ESI-MS *m*/*z*: 380 [M+H]⁺; ¹H-NMR (DMSO-*d*₆)δ(ppm): 1.33-1.56 (m, 6H), 2.31 (s, 4H), 3.47 (s, 2H), 4.50 (s, 2H), 7.10 (d, *J* = 8.2 Hz, 1H), 7.33 (s, 1H), 7.40 (d, *J* = 8.2 Hz, 1H), 7.45 (s, 1H), 7.58 (s, 1H), 8.19 (s, 1H), 9.35 (s, 1H), 13.81 (s, 1H).

### Example 41:

### 5-{[N-Methyl(2-hydroxy-2-phenylethyl)amino]methyl}-7-[1H-5-(pipe ridinomethyl)indol-2-yl)isoindolinone (Compound 508)

### Step 1

In a similar manner to Steps 6 and 7 of Example 6, the crude product obtained using 5-formyl-3-hydroxy-7-iodo-2-(1-methyl-1-phenylethyl)isoindolinon e (80.0 mg, 0.190 mmol), α-(methylaminomethyl)benzyl alcohol (574 mg, 3.80 mmol), acetic acid (218 µL, 3.80 mmol), sodium triacetoxyborohydride (161 mg, 0.760 mmol) and acetonitrile (2.0 mL) was treated with triethylsilane (36.4 µL, 0.228 mmol), trifluoroacetic acid (147 µL, 1.90 mmol) and nitromethane (2.7 mL) to obtain 5-{[N-methyl(2-hydroxy-2-phenylethyl)amino]methyl}-7-iodoisoindo linone (46.9 mg, yield 58%).
ESI-MS *m*/*z*: 423 [M+H]⁺; ¹H-NMR (DMSO-*d*₆)δ(ppm) : 2.22 (s, 3H), 2.44-2.64 (m, 2H), 3.52-3.68 (m, 2H), 4.21 (s, 2H), 5.12 (d, *J* = 4.0 Hz, 1H), 7.20-7.34 (m, 6H), 7.37 (s, 1H), 7.78 (s, 1H), 8.65 (s, 1H).

### Step 2

In a similar manner to Step 2 of Example 21, 5-{[(2-hydroxy-2-phenylethyl)methylamino]methyl}-7-[1-(tert-buto xycarbonyl)-5-(piperidinomethyl)indol-2-yl]isoindolinone (51.0 mg, yield 78) was obtained using 5-{[N-methyl(2-hydroxy-2-phenylethyl)amino]methyl}-7-iodoisoindo linone (45.0 mg, 0.107 mmol), Compound BD (76.7 mg, 0.214 mmol), potassium carbonate (73.9 mg, 0.535 mmol), [bis(diphenylphosphino)ferrocene]dichloropalladium (8.70 mg, 0.0107 mmol) and dimethoxyethane (1.8 mL)/water (77 µL).
ESI-MS *m*/*z*: 610 [M+H]⁺

### Step 3

In a similar manner to Step 8 of Example 1, Compound 508 (26.0 mg, yield 61%) was obtained using 5-{[(2-hydroxy-2-phenylethyl)methylamino]methyl}-7-[1-(tert-buto xycarbonyl)-5-(piperidinomethyl)indol-2-yl]isoindolinone (51.0mg, 0.0838 mmol), 10% hydrogen chloride-methanol solution (1.3 mL) and methanol (1.3 mL).
ESI-MS *m*/*z:* 509 [M+H]⁺; ¹H-NMR (DMSO-*d*₆)δ(ppm): 1.45 (m, 6H), 2.29 (s, 3H), 2.34 (s, 4H), 3.48 (s, 2H), 3.71 (s, 2H), 4.44 (s, 2H), 4.80 (m, 1H), 5.16 (d, *J* = 3.8 Hz, 1H), 7.05-7.13 (m, 2H), 7.24-7.40 (m, 8H), 7.45 (s, 1H), 8.02 (s, 1H), 9.19 (s, 1H), 13.86 (s, 1H).

### Example 42:

### 5-(2-Propylamino)-7-[1H-5-(piperidinomethyl)indol-2-yl)isoindoli none dihydrochloride (Compound 509)

### Step 1

5-Fluoro-7-iodoisoindolinone (68.7 mg, 0.248 mmol) was dissolved in DMSO (2 mL), and the solution was added with calcium carbonate (124 mg, 1.24 mmol) and isopropylamine (1.06 mL, 12.4 mmol), followed by stirring at 100 °C for 4 hours under nitrogen atmosphere. The reaction mixture was added with isopropylamine (1.06 mL, 12.4 mmol) followed by stirring at 100 °C for 27 hours. The reaction mixture was added with isopropylamine (1.06 mL, 12.4 mmol) followed by stirring at 100 °C for 97 hours. The reaction mixture was added with water, and extracted with ethyl acetate after removing the solid by filtration. The organic layer was washed with saturated brine and dried over anhydrous sodium sulfate. The solvent was evaporated under reduced pressure, and the residue was purified by preparative thin-layer chromatography (ethyl acetate) to obtain 5-(2-propylamino)-7-iodoisoindolinone (18.2 mg, yield 23%).
ESI-MS *m*/*z*: 317 [M+H]⁺; ¹H-NMR (CDCl₃)δ(ppm): 1.23 (s, 3H), 1.25 (s, 3H), 3.68 (m, 1H), 3.95 (d, *J* = 6.4 Hz, 1H), 4.23 (s, 2H), 6.52 (s, 1H), 6.86 (br s, 1H), 7.06 (s, 1H).

### Step 2

In a similar manner to Step 1 of Example 15, 5-(2-propylamino)-7-iodoisoindolinone (18.1 mg, 0.0573 mmol) was dissolved in dimethoxyethane (2 mL), and the solution was treated with Compound BD (41.0 mg, 0.115 mmol), [bis(diphenylphosphino)ferrocene]dichloropalladium (4.0 mg, 0.0049 mmol), potassium carbonate (39.0 mg, 0.285 mmol) and water (0.041 mL, 2.3 mmol), followed by purification by preparative thin-layer chromatography (chloroform/methanol=4/1) to obtain 5-(2-propylamino)-7-[1-(tert-butoxycarbonyl)-5-(piperidinomethyl )indol-2-yl]isoindolinone (29.5 mg).
ESI-MS *m*/*z*: 503 [M+H]⁺; ¹H-NMR (CDCl₃)δ(ppm): 1.19-1.50 (m, 6H), 1.25 (s, 3H), 1.28 (s, 3H), 1.35 (s, 9H), 2.29-2.53 (m, 4H), 3.59 (br s, 2H), 3.71 (m, 1H), 3.94 (d, *J* = 7.8 Hz, 1H), 4.32 (s, 2H), 5.92 (s, 1H), 6.46-6.56 (m, 2H), 7.24 (s, 1H), 7.25 (d, *J* = 8.4 Hz, 1H), 7.47 (s, 1H), 8.16 (d, *J* = 8.4 Hz, 1H).

### Step 3

In a similar manner to Step 8 of Example 1, 5-(2-propylamino)-7-[1-(tert-butoxycarbonyl)-5-(piperidinomethyl ) indol-2-yl] isoindolinone (29.5 mg) was dissolved in methanol (1 mL), and the solution was treated with 10% hydrogen chloride-methanol solution (1 mL). The obtained solid was collected by filtration and washed with methanol, followed by drying under reduced pressure to obtain Compound 509 (10.0 mg, yield 46%, 2 steps).
ESI-MS *m*/*z*: 403 [M+H]⁺; ¹H-NMR (DMSO-*d*₆)δ(ppm): 1.18 (s, 3H), 1.21 (s, 3H), 1.34 (m, 1H), 1.60-1.88 (m, 5H), 2.75-2.85 (m, 2H), 3.32 (d, *J* = 10.9 Hz, 2H), 3.75 (m, 1H), 4.29 (d, *J* = 4.8 Hz, 2H), 4.36 (s, 2H), 6.64 (s, 1H), 7.05 (s, 1H), 7.26 (d, *J* = 8.4 Hz, 1H), 7.35 (s, 1H), 7.51 (d, *J* = 8.4 Hz, 1H), 7.74 (s, 1H), 8.67 (s, 1H), 9.85 (br s, 1H), 14.38 (s, 1H).

### Example 43:

### 5-Phenyl-7-[1H-5-(piperidinomethyl)indol-2-yl)isoindolinone (Compound 510)

### Step 1

5-Chloro-7-[1-(tert-butoxycarbonyl)-5-(piperidinomethyl)ind ol-2-yl]isoindolinone (50.0 mg, 0.104 mmol) was dissolved in 1,4-dioxane (2.0 mL), and the solution was added with phenylboronic acid (25.4 mg, 0.208 mmol), cesium fluoride (55.3 mg, 0.364 mmol), 2-dicyclohexylphosphino-2',4',6'-triisopropylbiphenyl (14.9 mg, 0.0312 mmol) and tris(dibenzylideneacetone)dipalladium(0) (9.52 mg, 0.0104 mmol), followed by stirring at 126 °C for 10 minutes under microwave irradiation. The reaction mixture was added with water and extracted with ethyl acetate. The organic layer was concentrated under reduced pressure. The residue was purified by silica gel column chromatography (chloroform/methanol=95/5-80/20) to obtain crude 5-phenyl-7-[1-(tert-butoxycarbonyl)-5-(piperidinomethyl)indol-2-yl]isoindolinone.
ESI-MS *m*/*z*: 522 [M+H]⁺

### Step 2

In a similar manner to Step 8 of Example 1, Compound 510 (43.8 mg, quantitative yield, 2 steps) was obtained using crude 5-phenyl-7-[1-(tert-butoxycarbonyl)-5-(piperidinomethyl)indol-2-yl]isoindolinone, 10% hydrogen chloride-methanol solution (0.69 mL) and methanol (0.69 mL).
ESI-MS *m*/*z*: 422 [M+H]⁺; ¹H-NMR (DMSO-*d*₆)δ(ppm): 1.45 (m, 6H), 2.34 (s, 4H), 3.48 (s, 2H), 4.57 (s, 2H), 7.09 (d, *J* = 9.4 Hz, 1H), 6.39 (s, 1H), 7.41-7.60 (m, 5H), 7.78 (s, 1H), 7.87 (d, *J* = 7.4 Hz, 2H), 8.37 (s, 1H), 9.28 (s, 1H), 13.85 (s, 1H).

### Example 44:

### 5-(4-Hydroxyphenyl)-7-[1H-5-(piperidinomethyl)indol-2-yl)isoindo linone (Compound 511)

### Step 1

In a similar manner to Step 1 of Example 43, crude 5-(4-hydroxyphenyl)-7-[1-(tert-butoxycarbonyl)-5-(piperidinometh yl)indol-2-yl]isoindolinone was obtained using 5-Chloro-7-[1-(tert-butoxycarbonyl)-5-(piperidinomethyl)indol-2-yl]isoindolinone (50.0 mg, 0.104 mmol), (4-hydroxyphenyl)boronic acid (28.7 mg, 0.208 mmol), cesium fluoride (55.3 mg, 0.364 mmol), 2-dicyclohexylphosphino-2',4',6'-triisopropylbiphenyl (14.9 mg, 0.0312 mmol), tris(dibenzylideneacetone)dipalladium(0) (9.52 mg, 0.0104 mmol) and 1,4-dioxane (1.5 mL).
ESI-MS *m*/*z*: 538 [M+H]⁺

### Step 2

In a similar manner to Step 8 of Example 1, Compound 511 (10.1 mg, yield 23%, 2 steps) was obtained using crude 5-(4-hydroxyphenyl)-7-[1-(tert-butoxycarbonyl)-5-(piperidinometh yl)indol-2-yl]isoindolinone, 10% hydrogen chloride-methanol solution (0.53 mL) and methanol (0.53 mL).
ESI-MS *m*/*z*: 438 [M+H]⁺; ¹H-NMR (DMSO-*d*₆)δ(ppm): 1.31-1.59 (m, 6H), 2.35 (s, 4H), 3.48 (s, 2H), 4.54 (s, 2H), 6.93 (d, *J* = 8.4 Hz, 2H), 7.09 (d, *J* = 8.6 Hz, 1H), 7.33-7.50 (m, 3H), 7.65-7.73 (m, 3H), 8.29 (s, 1H), 9.20 (s, 1H), 9.76 (s, 1H), 13.87 (s, 1H).

### Example 45:

### 5-(Dimethylaminomethyl)-7-[1H-5-(morpholinomethyl)indol-2-yl)iso indolinone dihydrochloride (Compound 512)

### Step 1

In a similar manner to Step 7 of Example 1, 5-(dimethylaminomethyl)-7-iodoisoindolinone (200 mg, 0.633 mmol) was dissolved in acetonitrile (8 mL), and the solution was treated with Compound BA (366 mg, 1.27 mmol), palladium acetate (11.4 mg, 0.0508 mmol), tri(o-tolyl)phosphine (30.8 mg, 0.101 mmol) and triethylamine (0.882 mL, 6.33 mmol), followed by purification by flush column chromatography (chloroform/methanol=98/2, 95/5, 93/7) to obtain 5-(dimethylaminomethyl)-7-[1-(tert-butoxycarbonyl)-5-formylindol -2-yl]isoindolinone (250 mg, yield 91%).
ESI-MS m/z*:* 434 [M+H]⁺; ¹H-NMR (CDCl₃)δ(ppm): 1.30 (s, 9H), 2.31 (s, 6H), 3.57 (s, 2H), 4.45 (s, 2H), 6.38 (br s, 1H), 6.71 (s, 1H), 7.44 (s, 1H), 7.49 (s, 1H), 7.87 (dd, *J* = 1.4, 8.7 Hz, 1H), 8.08 (d, *J* = 1.4 Hz, 1H), 8.40 (d, *J* = 8.7 Hz, 1H), 10.07 (s, 1H).

### Step 2

5-(Dimethylaminomethyl)-7-[1-(tert-butoxycarbonyl)-5-formyl indol-2-yl]isoindolinone (120 mg, 0.277 mmol) was dissolved in acetonitrile (7 mL), and the solution was added with morpholine (0.097 mL, 1.11 mmol) and acetic acid (0.317 mL, 5.54 mmol), followed by stirring at room temperature for 1 hour. The mixture was added with sodium triacetoxyborohydride (176 mg, 0.831 mmol), followed by stirring at room temperature for 2 hours. The reaction mixture was added with water and sodium carbonate, and extracted with ethyl acetate. The organic layer was washed with saturated brine and dried over anhydrous sodium sulfate. The solvent was evaporated under reduced pressure. The residue was purified by preparative thin-layer chromatography (chloroform/methanol=4/1) to obtain 5-(dimethylaminomethyl)-7-[1-(tert-butoxycarbonyl)-5-(morpholino methyl)indol-2-yl]isoindolinone (103 mg, yield 73%).
ESI-MS *m*/*z*: 505 [M+H]⁺; ¹H-NMR (CDCl₃)δ(ppm): 1.30 (s, 9H), 2.30 (s, 6H), 2.40-2.58 (m, 4H), 3.56 (s, 2H), 3.59 (s, 2H), 3.66-3.81 (m, 4H), 4.77 (s, 2H), 6.39 (br s, 1H), 6.55 (s, 1H), 7.27 (s, 1H), 7.28 (m, 1H), 7.40 (s, 1H), 7.47 (d, *J* = 7.4 Hz, 1H), 8.19 (d, *J* = 8.6 Hz, 1H).

### Step 3

In a similar manner to Step 8 of Example 1, 5-(dimethylaminomethyl)-7-[1-(tert-butoxycarbonyl)-5-(morpholino methyl)indol-2-yl]isoindolinone (101 mg, 0.199 mmol) was dissolved in methanol (2.5 mL), and the solution was treated with 10% hydrogen chloride-methanol solution (2.5 mL), followed by condensing under reduced pressure and adding methanol and ethyl acetate thereto. The obtained solid was collected by filtration and washed with ethyl acetate, followed by drying under reduced pressure to obtain Compound 512 (65.4 mg, yield 69%).
ESI-MS *m*/*z*: 391 [M+H]⁺; ¹H-NMR (DMSO-*d*₆)δ(ppm): 1.00 (t, *J* = 6.9 Hz, 6H), 2.21 (s, 6H), 2.35-2.68 (m, 4H), 3.54 (s, 2H), 3.57-3.64 (m, 2H), 4.47 (s, 2H), 7.10 (d, *J* = 8.5 Hz, 1H), 7.15 (s, 1H), 7.39 (d, *J* = 8.5 Hz, 1H), 7.40 (s, 1H), 7.48 (br s, 1H), 8.02 (s, 1H), 9.20 (s, 1H), 13.86 (br s, 1H).

### Example 46:

### 5-(Dimethylaminomethyl)-7-{1H-5-[(4,4-difluoropiperidino)methyl] indol-2-yl)isoindolinone dihydrochloride (Compound 513)

### Step 1

In a similar manner to Step 2 of Example 45, 5-(dimethylaminomethyl)-7-[1-(tert-butoxycarbonyl)-5-formylindol -2-yl]isoindolinone (120 mg, 0.277 mmol) was dissolved in acetonitrile (7 mL), and the solution was added with 4,4-difluoropiperidine hydrochloride (44.0 mg, 0.279 mmol), acetic acid (0.317 mL, 5.54 mmol) and sodium triacetoxyborohydride (176 mg, 0.831 mmol), followed by purification by preparative thin-layer chromatography (chloroform/methanol=6/1) to obtain 5-(dimethylaminomethyl)-7-{1-(tert-butoxycarbonyl)-5-[(4,4-diflu oropiperidino)methyl]indol-2-yl}isoindolinone (84.2 mg, yield 56%).
ESI-MS *m*/*z*: 539 [M+H]⁺; ¹H-NMR (CDCl₃)δ(ppm): 1.30 (s, 9H), 1.90-2.09 (m, 4H), 2.30 (s, 6H), 2.51-2.64 (m, 4H), 3.56 (s, 2H), 3.63 (s, 2H), 4.42 (s, 2H), 6.40 (br s, 1H), 6.56 (s, 1H), 7.28 (m, 1H), 7.40 (s, 1H), 7.42-7.52 (m, 2H), 8.19 (d, *J* = 8.6 Hz, 1H).

### Step 2

In a similar manner to Step 8 of Example 1, 5-(dimethylaminomethyl)-7-{1-(tert-butoxycarbonyl)-5-[(4,4-diflu oropiperidino)methyl]indol-2-yl}isoindolinone (82.2 mg, 0.153 mmol) was dissolved in methanol (2 mL), and the solution was treated with 10% hydrogen chloride-methanol solution (2 mL), followed by condensing under reduced pressure and adding methanol and ethyl acetate thereto. The obtained solid was collected by filtration and washed with ethyl acetate, followed by drying under reduced pressure to obtain Compound 513 (33.5 mg, yield 43%).
ESI-MS *m*/*z*: 439 [M+H]⁺; ¹H-NMR (DMSO-*d*₆)δ(ppm): 2.20-2.60 (m, 4H), 2.76 (d, *J* = 4.2 Hz, 6H), 3.08-3.18 (m, 2H), 3.42-3.50 (m, 2H), 4.43 (s, 2H), 4.44 (s, 2H), 4.55 (s, 2H), 7.35 (d, *J* = 8.5 Hz, 1H), 7.41 (s, 1H), 7.57 (d, *J* = 8.5 Hz, 1H), 7.62 (s, 1H), 7.85 (s, 1H), 8.58 (s, 1H), 9.44 (s, 1H), 11.08 (br s, 2H), 14.02 (s, 1H).

### Example 47:

### 5-(Pyridin-3-yl)-7-[1H-5-(piperidinomethyl)indol-2-yl]isoindolin one (Compound 514)

### Step 1

In a similar manner to Step 1 of Example 43, crude 5-(pyridin-3-yl)-7-[1-(tert-butoxycarbonyl)-5-(piperidinomethyl) indol-2-yl]isoindolinone was obtained using 5-chloro-7-[1-(tert-butoxycarbonyl)-5-(piperidinomethyl)indol-2-yl]isoindolinone (50. 0 mg, 0.104 mmol), 3-pyridineboronic acid (25. 6 mg, 0.208 mmol), cesium fluoride (55.3 mg, 0.364 mmol), 2-dicyclohexylphosphino-2',4',6'-triisopropylbiphenyl (14.9 mg, 0.0312 mmol), tris(dibenzylideneacetone)dipalladium(0) (9.52 mg, 0.0104 mmol) and 1,4-dioxane (1.5 mL).
ESI-MS *m*/*z*: 523 [M+H]⁺

### Step 2

In a similar manner to Step 8 of Example 1, Compound 514 (5.30 mg, yield 12%, 2 steps) was obtained using crude 5-(pyridin-3-yl)-7-[1-(tert-butoxycarbonyl)-5-(piperidinomethyl) indol-2-yl]isoindolinone, 10% hydrogen chloride-methanol solution (1.0 mL) and methanol (1.0 mL).
ESI-MS *m*/*z:* 423 [M+H]⁺; ¹H-NMR (DMSO-*d*₆)δ(ppm): 1.33-1.56 (m, 6H), 2.34 (s, 4H), 3.49 (s, 2H), 4.58 (s, 2H), 7.10 (d, *J* = 8.1 Hz, 1H), 7.38-7.49 (m, 3H), 7.54 (m, 1H), 7.86 (s, 1H), 8.29 (d, *J* = 8.1 Hz, 1H), 8.45 (s, 1H), 8.67 (d, *J* = 4.5 Hz, 1H), 9.09 (s, 1H), 9.33 (s, 1H), 13.84 (s, 1H).

### Example 48:

### 5-(3-Hydroxyphenyl)-7-[1H-5-(piperidinomethyl)indol-2-yl]isoindo linone (Compound 515)

### Step 1

In a similar manner to Step 1 of Example 43, crude 5-(3-hydroxyphenyl)-7-[1-(tert-butoxycarbonyl)-5-(piperidinometh yl)indol-2-yl]isoindolinone was obtained using 5-chloro-7-[1-(tert-butoxycarbonyl)-5-(piperidinomethyl)indol-2-yl]isoindolinone (50.0 mg, 0.104 mmol), 3-hydroxyphenylboronic acid (28.7 mg, 0.208 mmol), cesium fluoride (55.3 mg, 0.364 mmol), 2-dicyclohexylphosphino-2',4',6'-triisopropylbiphenyl (14.9 mg, 0.0312 mmol), tris(dibenzylideneacetone)dipalladium(0) (9.52 mg, 0.0104 mmol) and 1,4-dioxane (1.5 mL).
¹H-NMR (DMSO-*d*₆)δ(ppm): 1.16 (s, 9H), 1.31-1.60 (m, 6H), 2.27-2.41 (m, 4H), 3.52 (s, 2H), 4.44 (s, 2H), 6.66-6.76 (m, 1H), 6.83 (d, *J* = 8.1 Hz, 1H), 7.13 (s, 1H), 7.19 (d, *J* = 7.7 Hz, 1H), 7.29 (d, *J* = 7.7 Hz, 1H), 7.45-7.55 (m, 1H), 7.63 (s, 1H), 7.81 (s, 1H), 8.03-8.16 (m, 1H), 8.58 (s, 1H), 9.59 (s, 1H).

### Step 2

In a similar manner to Step 8 of Example 1, Compound 515 (6.40 mg, yield 14%, 2 steps) was obtained using crude 5-(3-hydroxyphenyl)-7-[1-(tert-butoxycarbonyl)-5-(piperidinometh yl)indol-2-yl]isoindolinone, 10% hydrogen chloride-methanol solution (1.0 mL) and methanol (1.0 mL).
ESI-MS *m*/*z*: 438 [M+H]⁺; ¹H-NMR (DMSO-*d*₆)δ(ppm): 1.32-1.57 (m, 6H), 2.35 (s, 4H), 3.48 (s, 2H), 4.55 (s, 2H), 6.87 (d, *J* = 8.6 Hz, 1H), 7.09 (d, *J* = 9.7 Hz, 1H), 7.19 (s, 1H), 7.22-7.43 (m, 4H), 7.45 (s, 1H), 7.69 (s, 1H), 8.29 (s, 1H), 9.26 (s, 1H), 9.64 (s, 1H), 13.84 (s, 1H).

### Example 49:

### 5-(1H-Pyrrol-2-yl)-7-[1H-5-(piperidinomethyl)indol-2-yl]isoindol inone (Compound 516)

### Step 1

In a similar manner to Step 1 of Example 43, crude 5-[(1-tert-butoxycarbonyl)pyrrol-2-yl]-7-[1-(tert-butoxycarbonyl )-5-(piperidinomethyl)indol-2-yl]isoindolinone was obtained using 5-chloro-7-[1-(tert-butoxycarbonyl)-5-(piperidinomethyl)indol-2-yl]isoindolinone (50.0 mg, 0.104 mmol), (1-tert-butoxycarbonyl)pyrrole-2-boronic acid (43.9 mg, 0.208 mmol), cesium fluoride (55.3 mg, 0.364 mmol), 2-dicyclohexylphosphino-2',4',6'-triisopropylbiphenyl (14.9 mg, 0.0312 mmol), tris(dibenzylideneacetone)dipalladium(0) (9.52 mg, 0.0104 mmol) and 1,4-dioxane (1.5 mL).
ESI-MS *m*/*z*: 611 [M+H]⁺

### Step 2

In a similar manner to Step 8 of Example 1, Compound 516 (10.0 mg, yield 23%, 2 steps) was obtained using crude 5-[(1-tert-butoxycarbonyl)pyrrol-2-yl]-7-[1-(tert-butoxycarbonyl )-5-(piperidinomethyl)indol-2-yl]isoindolinone, 10% hydrogen chloride-methanol solution (1.0 mL) and methanol (1.0 mL).
ESI-MS *m*/*z*: 411 [M+H]⁺; ¹H-NMR (DMSO-*d*₆)δ(ppm): 1.32-1.60 (m, 6H), 2.35 (s, 4H), 3.49 (s, 2H), 4.51 (s, 2H), 6.22 (s, 1H), 6.83 (s, 1H), 7.02 (s, 1H), 7.08 (d, *J* = 8.4 Hz, 1H), 7.35-7.50 (m, 3H), 7.70 (s, 1H), 8.40 (s, 1H), 9.13 (s, 1H), 11.67 (s, 1H), 13.95 (s, 1H).

### Example 50:

### 5-(3-Aminophenyl)-7-[1H-5-(piperidinomethyl)indol-2-yl]isoindoli none (Compound 517)

### Step 1

In a similar manner to Step 1 of Example 43, crude 5-(3-acetylaminophenyl)-7-[1-(tert-butoxycarbonyl)-5-(piperidino methyl)indol-2-yl]isoindolinone was obtained using 5-chloro-7-[1-(tert-butoxycarbonyl)-5-(piperidinomethyl)indol-2-yl]isoindolinone (50.0 mg, 0.104 mmol), 3-acetoamidophenylboronic acid (37.2 mg, 0.208 mmol), cesium fluoride (55.3 mg, 0.364 mmol), 2-dicyclohexylphosphino-2',4',6'-triisopropylbiphenyl (14.9 mg, 0.0312 mmol), tris(dibenzylideneacetone)dipalladium(0) (9.52 mg, 0.0104 mmol) and 1,4-dioxane (1.5 mL).
ESI-MS *m*/*z*: 579 [M+H]⁺

### Step 2

In a similar manner to Step 8 of Example 1, Compound 517 (10.0 mg, yield 22%, 2 steps) was obtained using crude 5-(3-acetylaminophenyl)-7-[1-(tert-butoxycarbonyl)-5-(piperidino methyl)indol-2-yl]isoindolinone, 10% hydrogen chloride-methanol solution (1.0 mL) and methanol (1.0 mL).
ESI-MS *m*/*z*: 437 [M+H]⁺; ¹H-NMR (DMSO-*d*₆)δ(ppm): 1.30-1.59 (m, 6H), 2.35 (s, 4H), 3.49 (s, 2H), 4.55 (s, 2H), 5.24 (s, 2H), 6.66 (d, *J* = 7.6 Hz, 1H), 6.96 (d, *J* = 7.6 Hz, 1H), 7.02 (s, 1H), 7.10 (d, *J* = 8.4 Hz, 1H), 7.18 (t, *J* = 7.6 Hz, 1H), 7.29 (s, 1H), 7.41 (d, *J* = 8.4 Hz, 1H), 7.46 (s, 1H), 7.65 (s, 1H), 8.26 (s, 1H), 9.24 (s, 1H), 13.87 (s, 1H).

### Example 51:

### 5-({[N-Methyl[2-(pyridin-2-yl)ethyl]amino}methyl)-7-[1H-5-(piper idinomethyl)indol-2-yl]isoindolinone (Compound 518)

### Step 1

In a similar manner to Steps 6 and 7 of Example 6, the crude product obtained using 5-formyl-3-hydroxy-7-iodo-2-(1-methyl-1-phenylethyl)isoindolinon e (80.0 mg, 0.190 mmol), 2-(2-methylaminoethyl)pyridine (526 µL, 3.80 mmol), acetic acid (218 µL, 3.80 mmol), sodium triacetoxyborohydride (161 mg, 0.760 mmol) and acetonitrile (2.0 mL) was treated with triethylsilane (152 µL, 0.950 mmol), trifluoroacetic acid (294 µL, 3.80 mmol) and nitromethane (2.6 mL) to obtain 5-({[N-methyl[2-(pyridin-2-yl)ethyl]amino}methyl)-7-iodoisoindol inone (61.1 mg, yield 79%).
ESI-MS *m*/*z*: 408 [M+H]⁺; ¹H-NMR (DMSO-*d*₆)δ(ppm): 2.20 (s, 3H), 2.73 (t, *J* = 7.1 Hz, 2H), 2.92 (t, *J* = 7.1 Hz, 2H), 3.57 (s, 2H), 4.22 (s, 2H), 7.20 (t, *J* = 7.3Hz, 1H), 7.27 (d, *J* = 7.3 Hz, 1H), 7.38 (s, 1H), 7.69 (t, *J* = 7.3 Hz, 1H), 7.74 (s, 1H), 8.46 (d, *J* = 7.3 Hz, 1H), 8.66 (s, 1H).

### Step 2

In a similar manner to Step 2 of Example 21, 5-({[N-methyl[2-(pyridin-2-yl)ethyl]amino}methyl)-7-[1-(tert-but oxycarbonyl)-5-(piperidinomethyl)indol-2-yl]isoindolinone (62.3 mg, yield 71%) was obtained using 5-({[N-methyl[2-(pyridin-2-yl)ethyl]amino}methyl)-7-iodoisoindol inone (60.0 mg, 0.147 mmol), Compound BD (105 mg, 0.294 mmol), potassium carbonate (102 mg, 0.735 mmol), [bis(diphenylphosphino)ferrocene]dichloropalladium (12.0 mg, 0.0147 mmol) and dimethoxyethane (2.4 mL)/water (0.11 mL).
ESI-MS *m*/*z*: 594 [M+H]⁺

### Step 3

In a similar manner to Step 8 of Example 1, Compound 518 (32.7 mg, yield 63%) was obtained using 5-({[N-methyl[2-(pyridin-2-yl)ethyl]amino}methyl)-7-[1-(tert-but oxycarbonyl)-5-(piperidinomethyl)indol-2-yl]isoindolinone (62.3 mg, 0.105 mmol), 10% hydrogen chloride-methanol solution (1.6 mL) and methanol (1.6 mL).
ESI-MS *m*/*z:* 494 [M+H]⁺; ¹H-NMR (DMSO-*d*₆)δ(ppm) : 1.32-1.57 (m, 6H), 2.26 (s, 3H), 2.35 (s, 4H), 2.77 (t, *J* = 8.6 Hz, 2H), 2.98 (t, *J* = 8.6 Hz, 2H), 3.48 (s, 2H), 3.68 (s, 2H), 4.45 (s, 2H), 7.06 (s, 1H), 7.08 (d, *J* = 6.8 Hz, 1H), 7.20 (t, *J* = 6.8 Hz, 1H), 7.29 (s, 1H), 7.31 (d, *J* = 8.2 Hz, 1H), 7.38 (d, *J* = 8.2 Hz, 1H), 7.45 (s, 1H), 7.70 (t, *J* = 6.8 Hz, 1H), 7.98 (s, 1H), 8.46 (d, *J* = 2.5 Hz, 1H), 9.20 (s, 1H), 13.86 (s, 1H).

### Example 52:

### 5-(Thiophen-2-yl)-7-[1H-5-(piperidinomethyl)indol-2-yl]isoindoli none (Compound 519)

### Step 1

In a similar manner to Step 1 of Example 43, crude 5-(thiophen-2-yl)-7-[1-(tert-butoxycarbonyl)-5-(piperidinomethyl )indol-2-yl]isoindolinone was obtained using 5-chloro-7-[1-(tert-butoxycarbonyl)-5-(piperidinomethyl)indol-2-yl]isoindolinone (60.0 mg, 0.125 mmol) , 3-thiopheneboronic acid (32.0 mg, 0.250 mmol), cesium fluoride (66.5 mg, 0.438 mmol), 2-dicyclohexylphosphino-2',4',6'-triisopropylbiphenyl (17.9 mg, 0.0375 mmol), tris(dibenzylideneacetone)dipalladium(0) (11.4 mg, 0.0125 mmol) and 1,4-dioxane (1.8 mL).
ESI-MS *m*/*z*: 528 [M+H]⁺

### Step 2

In a similar manner to Step 8 of Example 1, Compound 519 (3.60 mg, yield 7%, 2 steps) was obtained using crude 5-(thiophen-2-yl)-7-[1-(tert-butoxycarbonyl)-5-(piperidinomethyl )indol-2-yl]isoindolinone, 10% hydrogen chloride-methanol solution (1.0 mL) and methanol (1.0 mL).
ESI-MS *m*/*z*: 428 [M+H]⁺; ¹H-NMR (CDCl₃)δ(ppm) : 0.95 (m, 10H), 4.12 (s, 2H), 4.58 (s, 2H), 6.37 (s, 1H), 7.18 (d, *J* = 4.9, Hz, 1H), 7.21-7.37 (m, 3H), 7.43 (d, *J* = 5.0 Hz, 1H), 7.50-7.55 (m, 3H), 8.32 (s, 1H).

### Example 53:

### 5-(Phenetylaminomethyl)-7-[1H-5-(piperidinomethyl)indol-2-yl]iso indolinone (Compound 520)

### Step 1

5-Fomyl-3-hydroxy-7-iodo-2-(1-methyl-1-phenylethyl)isoindol inone (100 mg, 0.237 mmol) obtained in Step 5 of Example 6 was dissolved in nitromethane (2.5 mL), and the solution was added with triethylsilane (114 µL, 0.712 mmo) and trifluoroacetic acid (59.4 µL, 0.0712 mmol), followed by stirring at room temperature for 4.0 hours. The reaction mixture was added with saturated aqueous sodium hydrogencarbonate solution, and extracted with ethyl acetate. The organic layer was concentrated under reduced pressure. The residue was crystallized by addition of diisopropylether to obtain 5-fomyl-7-iodo-2-(1-methyl-1-phenylethyl)isoindolinone (83.7 mg, yield 87%).
¹H-NMR (DMSO-*d*₆)δ(ppm) : 1.79 (s, 6H), 4.76 (s, 2H), 7.15-7.25 (m, 1H), 7.27-7.32 (m, 4H), 8.11 (s, 1H), 8.39 (s, 1H), 10.07 (s, 1H).

### Step 2

5-Fomyl-7-iodo-2-(1-methyl-1-phenylethyl)isoindolinone (43.0 mg, 0.106 mmol) was dissolved in dichloromethane (1.1 mL) , and the solution was added with trifluoroacetic acid (327 µL, 4.24 mmol, followed by stirring at room temperature for 2.0 hours. The reaction mixture was added with saturated aqueous sodium hydrogencarbonate solution, and extracted with ethyl acetate. The organic layer was concentrated under reduced pressure. The residue was crystallized by addition of diisopropylether to obtain 5-fomyl-7-iodoisoindolinone (21.2 mg, yield 70%).
ESI-MS *m*/*z*: 288 [M+H]⁺; ¹H-NMR (DMSO-*d*₆)δ(ppm) : 4.38 (s, 2H), 8.08 (s, 1H), 8.39 (s, 1H), 9.02 (s, 1H), 10.07 (d, *J* = 0.8 Hz, 1H).

### Step 3

In a similar manner to Step 2 of Example 21, 5-formyl-7-[1-(tert-butoxycarbonyl)-5-(piperidinomethyl)indol-2-yl]isoindolinone (431 mg, yield 52%) was obtained using 5-fomyl-7-iodoisoindolinone (500 mg, 1.74 mmol), Compound BD (1.25 g, 3.48 mmol), potassium carbonate (1.20 g, 8.70 mmol), [bis(diphenylphosphino)ferrocene]dichloropalladium (142 mg, 0.174 mmol) and dimethoxyethane (20 mL)/water (1.3 mL). ESI-MS *m*/*z*: 474 [M+H]⁺

### Step 4

In a similar manner to Step 6 of Example 6, crude 5-(phenetylaminomethyl)-7-[1-(tert-butoxycarbonyl)-5-(piperidino methyl)indol-2-yl]isoindolinone was obtained using 5-formyl-7-[1-(tert-butoxycarbonyl)-5-(piperidinomethyl)indol-2-yl] isoindolinone (50. 0 mg, 0.106 mmol) , β-phenetylamine (265 µL, 2.11 mmol), acetic acid (121 µL, 2.11 mmol), sodium triacetoxyborohydride (89.9 mg, 0.424 mmol) and acetonitrile (1.3 mL).
ESI-MS *m*/*z*: 579 [M+H]⁺

### Step 5

In a similar manner to Step 8 of Example 1, Compound 520 (23.5 mg, yield 47%) was obtained using crude 5-(phenetylaminomethyl)-7-[1-(tert-butoxycarbonyl)-5-(piperidino methyl)indol-2-yl]isoindolinone, 10% hydrogen chloride-methanol solution (1.0 mL) and methanol (1.0 mL).
ESI-MS *m*/*z*: 479 [M+H]⁺; ¹H-NMR (DMSO-*d*₆)δ(ppm) : 1.31-1.56 (m, 6H), 2.34 (s, 4H), 2.77 (s, 3H), 3.19-3.43 (m, 4H), 3.48 (s, 2H), 3.88 (s, 2H), 4.46 (s, 2H), 7.13-7.28 (m, 10H), 7.38 (d, *J* = 8.8 Hz, 1H), 7.40 (s, 1H), 7.45 (s, 1H), 8.08 (s, 1H), 9.20 (s, 1H).

### Example 54:

### 5-(2-Methylphenyl)-7-[1H-5-(piperidinomethyl)indol-2-yl]isoindol inone (Compound 521)

### Step 1

In a similar manner to Step 1 of Example 43, crude 5-(2-methylphenyl)-7-[1-(tert-butoxycarbonyl)-5-(piperidinomethy l)indol-2-yl]isoindolinone was obtained using 5-chloro-7-[1-(tert-butoxycarbonyl)-5-(piperidinomethyl)indol-2-yl]isoindolinone (60.0 mg, 0.125 mmol), o-tolylboronic acid (34.0 mg, 0.250 mmol), cesium fluoride (66.5 mg, 0.438 mmol), 2-dicyclohexylphosphino-2',4',6'-triisopropylbiphenyl (17.9 mg, 0.0375 mmol), tris(dibenzylideneacetone)dipalladium(0) (11.4 mg, 0.0125 mmol) and 1,4-dioxane (1.8 mL).
ESI-MS *m*/*z*: 536 [M+H]⁺

### Step 2

In a similar manner to Step 8 of Example 1, Compound 521 (20.5 mg, yield 38%, 2 steps) was obtained using crude 5-(2-methylphenyl)-7-[1-(tert-butoxycarbonyl)-5-(piperidinomethy l) indol-2-yl] isoindolinone, 10% hydrogen chloride-methanol solution (1.0 mL) and methanol (1.0 mL).
ESI-MS *m*/*z*: 436 [M+H]⁺; ¹H-NMR (DMSO-*d*₆)δ(ppm) : 1.31-1.57 (m, 6H), 2.32 (m, 7H), 3.47 (s, 2H), 4.55 (s, 2H), 7.08 (d, *J* = 9.3 Hz, 1H), 7.26 (s, 1H), 7.33-7.45 (m, 7H), 8.06 (s, 1H), 9.29 (s, 1H), 13.88 (s, 1H).

### Example 55:

### 5-(tert-Butylaminomethyl)-7-[1H-5-(piperidinomethyl)indol-2-yl]i soindolinone (Compound 522)

### Step 1

In a similar manner to Step 6 of Example 6, crude 5-(tert-butylaminomethyl)-7-[1-(tert-butoxycarbonyl)-5-(piperidi nomethyl)indol-2-yl]isoindolinone was obtained using 5-formyl-7-[1-(tert-butoxycarbonyl)-5-(piperidinomethyl)indol-2-yl]isoindolinone (40.0 mg, 0.0845 mmol), tert-butylamine (178 µL, 1.69 mmol), acetic acid (96.7 µL, 1.69 mmol), sodium triacetoxyborohydride (71.6 mg, 0.338 mmol) and acetonitrile (1.0 mL).
ESI-MS *m*/*z*: 531 [M+H]⁺

### Step 2

In a similar manner to Step 8 of Example 1, Compound 522 (4.80 mg, yield 13%, 2 steps) was obtained using crude 5-(tert-butylaminomethyl)-7-[1-(tert-butoxycarbonyl)-5-(piperidi nomethyl)indol-2-yl]isoindolinone, 10% hydrogen chloride-methanol solution (1.0 mL) and methanol (1.0 mL).
ESI-MS *m*/*z*: 431 [M+H]⁺; ¹H-NMR (DMSO-*d*₆)δ(ppm) : 1.14 (s, 9H) , 1.32-1.56 (m, 6H), 2.35 (s, 4H), 3.47 (s, 2H), 3.83 (s, 2H), 4.48 (s, 2H), 7.07 (d, *J* = 8.6 Hz, 1H), 7.17 (s, 1H), 7.38 (d, *J* = 8.6 Hz, 1H), 7.45 (s, 1H), 7.48 (s, 1H), 8.11 (s, 1H), 9.18 (s, 1H), 13.89 (s, 1H).

### Example 56:

### 5-(1-Hydroxy-2,2-dimethylpropyl)-7-[1H-5-(piperidinomethyl)indol -2-yl]isoindolinone hydrochloride (Compound 523)

### Step 1

5-Formyl-7-[1-(tert-butoxycarbonyl)-5-(piperidinomethyl)ind ol-2-yl]isoindolinone (30.0 mg, 0.0633 mmol) was dissolved in THF (1 mL), and tert-butyllithium-pentane solution (1.50 mol/L, 0.20 mL, 0.30 mmol) was added dropwise thereto at -78 °C, followed by stirring at same temperature for 20 minutes. The reaction mixture was added with water, and extracted with ethyl acetate. The organic layer was washed with saturated brine and dried over anhydrous sodium sulfate. The solvent was evaporated under reduced pressure, and the residue was purified by preparative thin-layer chromatography (chloroform/methanol=9/1) to obtain 5-(1-hydroxy-2,2-dimethylpropyl)-7-[1-(tert-butoxycarbonyl)-5-(p iperidinomethyl)indol-2-yl]isoindolinone (13.7 g, yield 41%).
ESI-MS *m*/*z*: 532 [M+H]⁺; ¹H-NMR (CDCl₃)δ(ppm) : 0. 96 (s, 9H) , 1.30 (s, 9H), 1.37-1.48 (m, 2H), 1.52-1.66 (m, 4H), 2.34-2.48 (m, 4H), 3.59 (s, 2H), 4.40 (s, 2H), 4.52 (s, 1H), 6.50 (s, 1H), 7.12 (br s, 1H), 7.28 (m, 1H), 7.32-7.52 (m, 3H), 8.20 (d, *J* = 8.6 Hz, 1H).

### Step 2

In a similar manner to Step 8 of Example 1, 5-(1-hydroxy-2,2-dimethylpropyl)-7-[1-(tert-butoxycarbonyl)-5-(p iperidinomethyl)indol-2-yl]isoindolinone (13.0 mg, 0.0245 mmol) was dissolved in methanol (0.5 mL), and the solution was treated with 10% hydrogen chloride-methanol solution (0.5 mL). The obtained solid was collected by filtration, washed with methanol, and then dried under reduced pressure to obtain Compound 523 (9.0 mg, yield 78%).
ESI-MS *m*/*z*: 432 [M+H]⁺; ¹H-NMR (DMSO-*d*₆)δ(ppm) : 0.90 (s, 9H) , 1.35 (m, 1H), 1.57-1.87 (m, 5H), 2.78-2.96 (m, 2H), 3.28-3.40 (m, 2H), 4.33 (br d, *J* = 4.8 Hz, 2H), 4.44 (d, *J* = 4.5 Hz, 1H), 4.51 (s, 2H) , 5.46 (d, *J* = 4.5 Hz, 1H), 7.23-7.29 (m, 2H), 7.44 (s, 1H), 7.56 (d, *J* = 8.4 Hz, 1H), 7.74 (d, *J* = 0.7 Hz, 1H), 8.06 (s, 1H), 9.25 (s, 1H), 9.47 (br s, 1H), 14.13 (br s, 1H).

### Example 57:

### 5-{[(2-Methoxyethyl)methylamino]methyl}-7-[1H-5-(piperidinomethy l)indol-2-yl]isoindolinone (Compound 524)

### Step 1

In a similar manner to Step 6 of Example 6, crude 5-{[(2-methoxyethyl)methylamino]methyl}-7-[1-(tert-butoxycarbony l)-5-(piperidinomethyl)indol-2-yl]isoindolinone was obtained using 5-formyl-7-[1-(tert-butoxycarbonyl)-5-(piperidinomethyl)indol-2-yl]isoindolinone (40.0 mg, 0.0845 mmol), N- (2-methoxyethyl)methylamine (181 µL, 1.69 mmol) , acetic acid (96.7 µL, 1.69 mmol), sodium triacetoxyborohydride (71.6 mg, 0.338 mmol) and acetonitrile (1.0 mL).
¹H-NMR (DMSO-*d*₆)δ(ppm) : 1.14 (s, 9H), 1.34-1.58 (m, 6H) , 2.20 (s, 3H), 2.27-2.42 (m, 4H), 2.53-2.61 (m, 2H), 3.23 (s, 3H), 3.48 (m, 2H), 3.65 (s, 2H), 4.37 (s, 2H), 6.54-6.62 (m, 1H), 7.21-7.30 (m, 1H), 7.37 (s, 1H), 7.46-7.55 (m, 2H), 8.53 (s, 1H).

### Step 2

In a similar manner to Step 8 of Example 1, Compound 524 (2.10 mg, yield 6%, 2 steps) was obtained using crude 5-{[(2-methoxyethyl)methylamino]methyl}-7-[1-(tert-butoxycarbony l)-5-(piperidinomethyl)indol-2-yl]isoindolinone, 10% hydrogen chloride-methanol solution (1.0 mL) and methanol (1.0 mL).
ESI-MS *m*/*z:* 447 [M+H]⁺; ¹H-NMR (DMSO-*d*₆)δ(ppm) : 1.33-1.63 (m, 6H), 2.23 (s, 4H), 2.60 (t, *J* = 6.0 Hz, 2H), 3.24 (s, 3H), 3.30 (s, 3H), 3.35 (s, 2H), 3.49 (t, *J* = 6.0 Hz, 2H), 3.69 (s, 2H), 4.47 (s, 2H), 7.15-7.26 (m, 2H), 7.36-7.50 (m, 2H), 7.55 (s, 1H), 8.08 (s, 1H), 8.96 (s, 1H), 12.92 (s, 1H).

### Example 58:

### 5-(Isopropylaminomethyl)-7-[1H-5-(piperidinomethyl)indol-2-yl]is oindolinone (Compound 525)

### Step 1

In a similar manner to Step 6 of Example 6, crude 5-(isopropylaminomethyl)-7-[1-(tert-butoxycarbonyl)-5-(piperidin omethyl)indol-2-yl]isoindolinone was obtained using 5-formyl-7-[1-(tert-butoxycarbonyl)-5-(piperidinomethyl)indol-2-yl]isoindolinone (50.0 mg, 0.106 mmol), isopropylamine (180 µL, 2.11 mmol), acetic acid (121 µL, 2.11 mmol), sodium triacetoxyborohydride (89.9 mg, 0.424 mmol) and acetonitrile (1.3 mL).
ESI-MS *m*/*z*: 517 [M+H]⁺

### Step 2

In a similar manner to Step 8 of Example 1, Compound 525 (4.10 mg, yield 9%, 2 steps) was obtained using crude 5-(isopropylaminomethyl)-7-[1-(tert-butoxycarbonyl)-5-(piperidin omethyl)indol-2-yl]isoindolinone, 10% hydrogen chloride-methanol solution (1.0 mL) and methanol (1.0 mL).
ESI-MS *m*/*z*: 417 [M+H]⁺; ¹H-NMR (DMSO-*d*₆)δ(ppm) : 1.05 (d, *J* = 6.8 Hz, 6H), 1.32-1.59 (m, 6H), 2.34 (s, 2H), 2.76 (s, 1H), 3.48 (s, 2H), 3.85 (s, 2H), 4.48 (s, 2H), 7.09 (s, 1H), 7.18 (s, 1H), 7.38 (d, *J* = 8.1 Hz, 1H), 7.46 (m, 2H), 8.11 (s, 1H), 8.60 (s, 1H), 9.18 (s, 1H) 13.87 (s, 1H).

### Example 59:

### 5-[(Isopropylmethylamino)methyl]-7-[1H-5-(piperidinomethyl)indol -2-yl]isoindolinone (Compound 526)

### Step 1

In a similar manner to Step 6 of Example 6, crude 5-[(isopropylmethylamino)methyl]-7-[1-(tert-butoxycarbonyl)-5-(p iperidinomethyl)indol-2-yl]isoindolinone was obtained using 5-formyl-7-[1-(tert-butoxycarbonyl)-5-(piperidinomethyl)indol-2-yl]isoindolinone (50.0 mg, 0. 106 mmol), N-isopropylmethylamine (180 µL, 2.11 mmol), acetic acid (121 µL, 2.11 mmol), sodium triacetoxyborohydride (89.9 mg, 0.424 mmol) and acetonitrile (1.3 mL).
ESI-MS *m*/*z*: 531 [M+H]⁺

### Step 2

In a similar manner to Step 8 of Example 1, Compound 526 (10.0 mg, yield 22%, 2 steps) was obtained using crude 5-[(isopropylmethylamino)methyl]-7-[1-(tert-butoxycarbonyl)-5-(p iperidinomethyl)indol-2-yl]isoindolinone, 10% hydrogen chloride-methanol solution (1.0 mL) and methanol (1.0 mL).
ESI-MS *m*/*z*: 431 [M+H]⁺; ¹H-NMR (DMSO-*d*₆)δ(ppm) : 1.05 (d, *J* = 6.9 Hz, 6H), 1.32-1.57 (m, 6H), 2.12 (s, 3H), 2.34 (s, 4H), 3.49 (s, 2H), 3.64 (s, 2H), 4.49 (s, 2H), 6.55 (s, 1H), 7.07 (d, *J* = 8.1 Hz, 1H), 7.14 (s, 1H), 7.38 (d, *J* = 8.1 Hz, 1H), 7.44 (s, 1H), 7.45 (s, 1H), 8.05 (s, 1H), 9.19 (s, 1H), 13.88 (s, 1H).

### Example 60:

### 5-(2-Fluorophenyl)-7-[1H-5-(piperidinomethyl)indol-2-yl]isoindol inone (Compound 527)

### Step 1

In a similar manner to Step 1 of Example 43, crude 5-(2-fluorophenyl)-7-[1-(tert-butoxycarbonyl)-5-(piperidinomethy l)indol-2-yl]isoindolinone was obtained using 5-chloro-7-[1-(tert-butoxycarbonyl)-5-(piperidinomethyl)indol-2-yl]isoindolinone (153 mg, 0.319 mmol), 2-fluorophenylboronic acid (89.3 mg, 0.638 mmol), cesium fluoride (170 mg, 1.12 mmol), 2-dicyclohexylphosphino-2',4',6'-triisopropylbiphenyl (45.6 mg, 0.00957 mmol), tris(dibenzylideneacetone)dipalladium(0) (29.2 mg, 0.0319 mmol) and 1,4-dioxane (2.5. mL).
ESI-MS *m*/*z*: 540 [M+H]⁺

### Step 2

In a similar manner to Step 8 of Example 1, Compound 527 (40.0 mg, yield 29%, 2 steps) was obtained using crude 5-(2-fluorophenyl)-7-[1-(tert-butoxycarbonyl)-5-(piperidinomethy l)indol-2-yl]isoindolinone, 10% hydrogen chloride-methanol solution (1.0 mL) and methanol (1.0 mL).
¹H-NMR (DMSO-*d*₆) δ: 1.33-1.56 (m, 6H), 2.29-2.40 (m, 4H), 3.46 (s, 2H), 4.57 (s, 2H), 7.09 (d, *J* = 9.0 Hz, 1H), 7.28 (s, 1H), 7.37-7.44 (m, 6H), 7.53 (d, *J* = 6.6 Hz, 1H), 7.65 (s, 1H), 7.69 (d, *J* = 6.0 Hz, 1H), 8.26 (s, 1H), 9.35 (s, 1H), 13.84 (s, 1H).

### Example 61:

### 5-(Thiophen-3-yl)-7-[1H-5-(piperidinomethyl)indol-2-yl]isoindoli none (Compound 528)

### Step 1

In a similar manner to Step 1 of Example 43, crude 5-(thiophen-3-yl)-7-[1-(tert-butoxycarbonyl)-5-(piperidinomethyl )indol-2-yl]isoindolinone was obtained using 5-chloro-7-[1-(tert-butoxycarbonyl)-5-(piperidinomethyl)indol-2-yl] isoindolinone (60. 0 mg, 0.125 mmol), 3-thiopheneboronic acid (32.0 mg, 0.250 mmol), cesium fluoride (66.5 mg, 0.438 mmol), 2-dicyclohexylphosphino-2',4',6'-triisopropylbiphenyl (17.9 mg, 0.0375 mmol), tris(dibenzylideneacetone)dipalladium(0) (11.4 mg, 0.0125 mmol) and 1,4-dioxane (1.8 mL).
ESI-MS *m*/*z*: 528 [M+H]⁺

### Step 2

In a similar manner to Step 8 of Example 1, Compound 528 (23.9 mg, yield 50%, 2 steps) was obtained using crude 5-(thiophen-3-yl)-7-[1-(tert-butoxycarbonyl)-5-(piperidinomethyl )indol-2-yl]isoindolinone, 10% hydrogen chloride-methanol solution (1.0 mL) and methanol (1.0 mL).
ESI-MS *m*/*z*: 428 [M+H]⁺; ¹H-NMR (DMSO-*d*₆)δ(ppm) : 1.33-1.57 (m, 6H), 2.29-2.43 (m, 4H), 3.49 (s, 2H), 4.55 (s, 2H), 7.09 (d, *J* = 8.3 Hz, 1H), 7.40 (d, *J* = 8.3 Hz, 1H), 7.44 (s, 1H), 7.46 (s, 1H), 7.71-7.77 (m, 1H), 7.81-7.84 (m, 2H), 8.25 (s, 1H), 8.45 (s, 1H), 9.25 (s, 1H), 13.88 (s, 1H).

### Example 62:

### 5-(Dimethylaminocarbonyl)-7-[1H-5-(piperidinomethyl)indol-2-yl]i soindolinone (Compound 529)

### Step 1

5-Formyl-7-[1-(tert-butoxycarbonyl)-5-(piperidinomethyl)ind ol-2-yl]isoindolinone (100 mg, 0.211 mmol) was dissolved in a mixed solvent of tert-butanol (2.5 mL)/water (1.0 mL), and the solution was added with 2-methyl-2-butene (447 µL, 4.22 mmol), sodium dihydrogen phosphate (75.9 mg, 0.633 mmol) and sodium chlorite (72.6 mg, 0.802 mmol), followed by stirring at room temperature for 3.5 hours. The reaction mixture was added with water and ethyl acetate, and the organic layer was removed. The aqueous layer was added with saturated aqueous sodium hydrogencarbonate solution, and extracted with THF. The organic layer was concentrated under reduced pressure. The residue was crystallized by addition of ethyl acetate (1.5 mL) to obtain 5-carboxy-7-[1-(tert-butoxycarbonyl)-5-(piperidinomethyl)indol-2 -yl]isoindolinone (49.7 mg, yield 48%).
ESI-MS *m*/*z*: 490 [M+H]⁺; ¹H-NMR (DMSO-*d*₆)δ(ppm) : 1.14 (s, 9H) , 1.32-1.58 (m, 6H), 2.28-2.42 (m, 4H), 3.52 (s, 2H), 4.39 (s, 2H), 6.60 (s, 1H), 7.26 (d, *J* = 9.9 Hz, 1H), 7.50 (s, 1H), 7.91 (s, 1H), 8.04 (s, 1H), 8.10 (d, *J* = 9.9 Hz, 1H), 8.59 (s, 1H).

### Step 2

In a similar manner to Step 2 of Example 1, 5-(dimethylaminocarbonyl)-7-[1-(tert-butoxycarbonyl)-5-(piperidi nomethyl) indol-2-yl] isoindolinone (8.30 mg, yield 47%) was obtained using 5-carboxy-7-[1-(tert-butoxycarbonyl)-5-(piperidinomethyl)indol-2 -yl] isoindolinone (16. 7 mg, 0.0341 mmol), EDCI (16.3 mg, 0.0853 mmol), HOBT monohydrate (2.61 mg, 0.0171 mmol), dimethylamine-THF solution (2.0 mol/L, 34.1 µL, 0.0682 mmol) and THF (1.0 mL).
ESI-MS *m*/*z*: 517 [M+H]⁺; ¹H-NMR (DMSO-*d*₆)δ(ppm) : 1.17 (s, 9H) , 1.32-1.59 (m, 6H), 2.28-2.43 (m, 4H), 2.96 (s, 3H), 3.02 (s, 3H), 3.51 (s, 2H), 4.43 (s, 2H), 6.66 (s, 1H), 7.28 (d, *J* = 9.2 Hz, 1H), 7.43 (s, 1H), 7.50 (s, 1H), 7.63 (s, 1H), 8.10 (d, *J* = 9.2 Hz, 1H), 8.71 (s, 1H).

### Step 3

In a similar manner to Step 8 of Example 1, Compound 529 (6.69 mg, quantitative yield) was obtained using 5-(dimethylaminocarbonyl)-7-[1-(tert-butoxycarbonyl)-5-(piperidi nomethyl)indol-2-yl]isoindolinone (8.30 mg, 0.0161 mmol), 10% hydrogen chloride-methanol solution (1.0 mL) and methanol (1.0 mL) .
ESI-MS *m*/*z*: 417 [M+H]⁺; ¹H-NMR (DMSO-*d*₆)δ(ppm) : 1.37-1.69 (m, 6H), 2.94-3.0 (m, *J* = 12.4 Hz, 10H), 3.42 (s, 2H), 4.53 (s, 2H), 7.26 (d, *J* = 7.9 Hz, 1H), 7.43-7.59 (m, 2H), 7.48 (d, *J* = 7.9 Hz, 1H), 7.68 (s, 1H), 8.01 (s, 1H), 9.09 (s, 1H), 12.79 (s, 1H).

### Example 63:

### 5-(Dimethylaminomethyl)-7-{1H-5-[(3-hydroxypiperidino)methyl]ind ol-2-yl}isoindolinone dihydrochloride (Compound 530)

### Step 1

In a similar manner to Step 2 of Example 45, 5-(dimethylaminomethyl)-7-[1-(tert-butoxycarbonyl)-5-formylindol -2-yl]isoindolinone (100 mg, 0.231 mmol) was dissolved in acetonitrile (7 mL), and the solution was treated with 3-hydroxypiperidine hydrochloride (318 mg, 2.31 mmol), acetic acid (0.264 mL, 4.62 mmol) and sodium triacetoxyborohydride (147 mg, 0.692 mmol), followed by purification by preparative thin-layer chromatography (chloroform/methanol/7 mol/L ammonia-methanol solution=12/1/1) to obtain 5-(dimethylaminomethyl)-7-{1-(tert-butoxycarbonyl)-5-[(3-hydroxy piperidino)methyl]indol-2-yl}isoindolinone (88.1 mg, yield 74%).
ESI-MS *m*/*z*: 519 [M+H]⁺; ¹H-NMR (CDCl₃)δ(ppm) : 1.29 (s, 9H), 1.43-1.98 (m, 4H), 2.30 (s, 6H), 2.41-2.66 (m, 4H), 3.55 (s, 2H), 3.63 (d, *J* = 3.0 Hz, 2H), 3.84 (m, 1H), 4.42 (s, 2H), 6.55 (s, 1H), 6.70 (br s, 1H), 7.25 (d, *J* = 8.4 Hz, 1H), 7.40 (s, 1H), 7.40-7.55 (m, 2H), 8.19 (d, *J* = 8.4 Hz, 1H).

### Step 2

In a similar manner to Step 8 of Example 1, 5-(dimethylaminomethyl)-7-{1-(tert-butoxycarbonyl)-5-[(3-hydroxy piperidino)methyl]indol-2-yl}isoindolinone (88.1 mg, 0.170 mmol) was dissolved in methanol (2.5 mL), and the solution was added with 10% hydrogen chloride-methanol solution (2.5 mL) , followed by adding with diisopropylether. The obtained solid was collected by filtration and washed with diisopropylether, followed by drying under reduced pressure to obtain Compound 530 (65.0 mg, yield 78%).
ESI-MS *m*/*z*: 419 [M+H]⁺; ¹H-NMR (DMSO-*d*₆)δ(ppm) : 1.14-2.29 (m, 4H), 2.75 (s, 6H), 2.84-3.55 (m, 4H), 4.01 (m, 1H), 4.15-4.48 (m, 2H), 4.43 (br s, 2H), 4.55 (s, 2H), 5.38 (m, 1H), 7.31 (d, *J* = 8.5 Hz, 1H), 7.44 (s, 1H), 7.57 (d, *J* = 8.5 Hz, 1H), 7.63 (s, 1H), 7.82 (s, 1H), 8.62 (s, 1H), 9.45 (s, 1H), 10.45 (br s, 1H), 11.24 (br s, 1H), 14.02 (s, 1H).

### Example 64:

### 5-(Dimethylaminomethyl)-7-{1H-5-[(4-hydroxypiperidino)methyl]ind ol-2-yl}isoindolinone dihydrochloride (Compound 531)

### Step 1

In a similar manner to Step 2 of Example 45, 5-(dimethylaminomethyl)-7-[1-(tert-butoxycarbonyl)-5-formylindol -2-yl]isoindolinone (100 mg, 0.231 mmol) was dissolved in acetonitrile (7 mL), and the solution was treated with 4-hydroxypiperidine (234 mg, 2.31 mmol) , acetic acid (0.264 mL, 4.62 mmol) and sodium triacetoxyborohydride (147 mg, 0.692 mmol), followed by purification by preparative thin-layer chromatography (chloroform/methanol/7 mol/L ammonia-methanol solution=12/1/1) to obtain 5-(dimethylaminomethyl)-7-{1-(tert-butoxycarbonyl)-5-[(4-hydroxy piperidino)methyl]indol-2-yl}isoindolinone (56.7 mg, yield 47%).
ESI-MS *m*/*z*: 519 [M+H]⁺; ¹H-NMR (CDCl₃)δ(ppm) : 1.30 (s, 9H), 1.48-1.70 (m, 2H), 1.95-2.04 (m, 2H), 2.08-2.24 (m, 2H), 2.29 (s, 6H), 2.84-2.92 (m, 2H), 3.55 (s, 2H), 3.60 (s, 2H), 3.71 (m, 1H), 4.42 (s, 2H), 6.39 (br s, 1H), 6.55 (s, 1H), 7.27 (m, 1H), 7.40 (s, 1H), 7.45 (s, 1H), 7.46 (s, 1H), 8.18 (d, *J* = 8.4 Hz, 1H).

### Step 2

In a similar manner to Step 8 of Example 1, 5-(dimethylaminomethyl)-7-{1-(tert-butoxycarbonyl)-5-[(4-hydroxy piperidino)methyl]indol-2-yl}isoindolinone (54.4 mg, 0.105 mmol) was dissolved in methanol (1.5 mL) , and the solution was treated with 10% hydrogen chloride-methanol solution (1.5 mL) , followed by adding with diisopropylether. The obtained solid was collected by filtration and washed with diisopropylether, followed by drying under reduced pressure to obtain Compound 531 (34.6 mg, yield 67%).
ESI-MS *m*/*z:* 419 [M+H]⁺; ¹H-NMR (DMSO-*d*₆)δ(ppm) : 1.58-1.74 (m, 2H), 1.75-1.85 (m, 2H), 2.75 (s, 6H), 2.80-3.45 (m, 4H), 3.91 (m, 1H), 4.26-4.36 (m, 2H), 4.43 (d, *J* = 4.9 Hz, 2H), 4.54 (s, 2H), 5.96 (m, 1H), 7.32 (d, *J* = 8.4 Hz, 1H), 7.42 (s, 1H), 7.56 (d, *J* = 8.4 Hz, 1H), 7.62 (s, 1H), 7.82 (s, 1H), 8.59 (s, 1H), 9.45 (s, 1H), 10.04 (br s, 1H), 11.18 (br s, 1H), 14.02 (s, 1H).

### Example 65:

### 5-(Dimethylaminomethyl)-7-[1H-5-(piperazin-1-ylcarbonyl)indol-2-yl]isoindolinone hydrochloride (Compound 532)

### Step 1

In a similar manner to Step 7 of Example 1, 5-(dimethylaminomethyl)-7-iodoisoindolinone (50.0 mg, 0.158 mmol) was dissolved in acetonitrile (5 mL), and the solution was treated with Compound BB (100 mg, 0.211 mmol), palladium acetate (2.5 mg, 0.011 mmol), tri(o-tolyl)phosphine (6.8 mg, 0.022 mmol) and triethylamine (0.194 mL, 1.39 mmol), followed by purification by preparative thin-layer chromatography (chloroform/methanol/7 mol/L ammonia-methanol solution=12/1/1) to obtain 5-(dimethylaminomethyl)-7-{1-(tert-butoxycarbonyl)-5-[4-(tert-bu toxycarbonyl)piperazin-1-ylcarbonyl]indol-2-yl}isoindolinone (107 mg).

### Step 2

In a similar manner to Step 8 of Example 1, 5-(dimethylaminomethyl)-7-{1-(tert-butoxycarbonyl)-5-[4-(tert-bu toxycarbonyl)piperazin-1-ylcarbonyl]indol-2-yl}isoindolinone (107 mg) was dissolved in methanol (3 mL), and the solution was treated with 10% hydrogen chloride-methanol solution (3 mL), followed by purification by preparative thin-layer chromatography (chloroform/methanol/7 mol/L ammonia-methanol solution=25/1/1) and slurry using metanol and 4 mol/L hydrochloric acid-ethyl acetate solution, to obtain Compound 532 (16.3 mg, yield 24%, 2 steps) and Compound 533 (14.3 mg, yield 26%, 2 steps).
ESI-MS *m*/*z*: 418 [M+H]⁺; ¹H-NMR (DMSO-*d*₆)δ(ppm) : 2.77 (s, 6H), 3.12-3.24 (m, 4H), 3.71-3.84 (m, 4H), 4.44 (br s, 2H), 4.56 (s, 2H), 7.26 (dd, *J* = 1.3, 8.6 Hz, 1H), 7.41 (s, 1H), 7.57 (d, *J* = 8.6 Hz, 1H), 7.65 (s, 1H), 7.76 (s, 1H), 8.54 (s, 1H), 9.25 (br s, 2H), 9.48 (s, 1H), 11.01 (br s, 1H), 14.10 (s, 1H).

### Example 66:

### 5-(Dimethylaminomethyl)-[1H-5-(methoxycarbonyl)indol-2-yl]isoind olinone hydrochloride (Compound 533)

Compound 533 (14.3 mg, yield 26%, 2 steps) was obtained in Steps 1 and 2 of Example 65.
ESI-MS *m*/*z*: 364 [M+H]⁺; ¹H-NMR (DMSO-*d*₆)δ(ppm) : 2.60 (br s, 6H) , 3.86 (s, 3H), 4.13 (br s, 2H), 4.55 (s, 2H), 7.39 (d, *J* = 1.4 Hz, 1H), 7.58 (s, 1H), 7.59 (d, *J* = 8.4 Hz, 1H), 7.77 (dd, *J* = 1.4, 8.4 Hz, 1H), 8.31 (s, 2H), 9.44 (s, 1H), 10.30 (br s, 1H), 14.28 (s, 1H).

### Example 67:

### 5-(Pyridin-4-yl)-7-[1H-5-(piperidinomethyl)indol-2-yl]isoindolin one (Compound 534)

### Step 1

In a similar manner to Step 1 of Example 43, crude 5-(pyridin-4-yl)-7-[1-(tert-butoxycarbonyl)-5-(piperidinomethyl) indol-2-yl]isoindolinone was obtained using 5-chloro-7-[1-(tert-butoxycarbonyl)-5-(piperidinomethyl)indol-2-yl] isoindolinone (60.0 mg, 0.125 mmol), 4-pyridineboronic acid (30. 7 mg, 0.250 mmol), cesium fluoride (66.5 mg, 0.438 mmol), 2-dicyclohexylphosphino-2',4',6'-triisopropylbiphenyl (17.9 mg, 0.0375 mmol), tris(dibenzylideneacetone)dipalladium(0) (11.4 mg, 0.0125 mmol) and 1,4-dioxane (1.8 mL).
ESI-MS *m*/*z*: 523 [M+H]⁺

### Step 2

In a similar manner to Step 8 of Example 1, Compound 534 (5.00 mg, yield 10%, 2 steps) was obtained using crude 5-(pyridin-4-yl)-7-[1-(tert-butoxycarbonyl)-5-(piperidinomethyl) indol-2-yl]isoindolinone, 10% hydrogen chloride-methanol solution (1.0 mL) and methanol (1.0 mL).
ESI-MS *m*/*z*: 423 [M+H]⁺; ¹H-NMR (DMSO-*d*₆)δ(ppm) : 1.37-1.68 (m, 6H), 2.46-2.73 (m, 4H), 3.47 (s, 2H), 4.58 (s, 2H), 7.18 (d, *J* = 7.7 Hz, 1H), 7.49 (d, *J* = 7.7 Hz, 1H), 7.51 (s, 1H), 7.58 (s, 1H), 7.89-7.93 (m, 3H), 8.50 (s, 1H), 8.74 (d, *J* = 6.1 Hz, 2H), 9.38 (s, 1H), 13.91 (s, 1H).

### Example 68:

### 5-({N-Methyl[2-(1H-indol-3-yl)ethyl]amino}methyl)-7-[1H-5-(piper idinomethyl)indol-2-yl]isoindolinone (Compound 535)

### Step 1

5-Formyl-7-[1-(tert-butoxycarbonyl)-5-(piperidinomethyl)ind ol-2-yl] isoindolinone (60. 0 mg, 0.127 mmol) was dissolved in methanol (2.0 mL), and the solution was added with *N*ω-methyltryptamine (55.2 mg, 0.317 mmol) and molecular sieves 4A (300 mg), followed by stirring at room temperature for 12 hours. Molecular sieves was removed by filtration, and the filtrate was added with sodium borohydride (41.8 mg, 1.02 mmol) at 0 °C, followed by stirring at room temperature for 4.5 hours. The reaction mixture was added with water and extracted with ethyl acetate. The organic layer was concentrated under reduced pressure. The residue was purified by silica gel column chromatography (chloroform/methanol=97/3-80/20) to obtain crude 5-({N-methyl[2-(1*H*-indol-3-yl)ethyl]amino}methyl)-7-[1-(*tert*-but oxycarbonyl)-5-(piperidinomethyl)indol-2-yl]isoindolinone.
ESI-MS *m*/*z*: 632 [M+H]⁺

### Step 2

In a similar manner to Step 8 of Example 1, Compound 535 (13.4 mg, yield 20%, 2 steps) was obtained using crude 5-({N-methyl[2-(1*H*-indol-3-yl)ethyl]amino}methyl)-7-[1-(*tert*-but oxycarbonyl)-5-(piperidinomethyl)indol-2-yl]isoindolinone, 10% hydrogen chloride-methanol solution (1.0 mL) and methanol (1.0 mL).
ESI-MS *m*/*z*: 532 [M+H]⁺; ¹H-NMR (DMSO-*d*₆)δ(ppm) : 1.33-1.60 (m, 6H), 2.33 (s, 3H), 2.33-2.49 (m, 4H), 2.71 (t, *J* = 7.3 Hz, 2H), 2.94 (t, *J* = 7.3 Hz, 2H), 3.73 (s, 2H), 4.45 (s, 2H), 6.89 (t, *J* = 7.5 Hz, 1H), 7.03 (t, *J* = 7.5 Hz, 1H), 7.05-7.18 (m, 3H), 7.30-7.58 (m, 7H), 8.05 (s, 1H), 9.19 (s, 1H), 10.78 (s, 1H), 13.89 (s, 1H).

### Example 69:

### 5-(Dimethylaminomethyl)-7-[1H-5-(ethylaminomethyl)indol-2-yl]iso indolinone dihydrochloride (Compound 536)

### Step 1

In a similar manner to Step 2 of Example 45, 5-(dimethylaminomethyl)-7-[1-(tert-butoxycarbonyl)-5-formylindol -2-yl]isoindolinone (80.0 mg, 0.185 mmol) was dissolved in acetonitrile (4.8 mL) , and the solution was treated with 70% aqueous ethylamine solution (0.238 mL, 3.70 mmol), acetic acid (0.212 mL, 3.70 mmol) and sodium triacetoxyborohydride (118 mg, 0.555 mmol), followed by purification by preparative thin-layer chromatography (chloroform/methanol/7 mol/L ammonia-methanol solution=12/1/1) to obtain 5-(dimethylaminomethyl)-7-[1-(tert-butoxycarbonyl)-5-(ethylamino methyl)indol-2-yl]isoindolinone (79.5 mg, yield 93%).
ESI-MS *m*/*z:* 463 [M+H]⁺; ¹H-NMR (CDCl₃)δ(ppm) : 1.14 (t, *J* = 7_{.}2 Hz, 3H), 1.29 (s, 9H), 2.29 (s, 6H), 2.70 (q, *J* = 7.2 Hz, 2H), 3.55 (s, 2H), 3.73 (m, 1H), 3.88 (s, 2H), 4.42 (s, 2H), 6.50 (br s, 1H), 6.55 (s, 1H), 7.27 (dd, *J* = 1.6, 8.6 Hz, 1H), 7.40 (s, 1H), 7.44 (s, 1H), 7.48 (s, 1H), 8.20 (d, *J* = 8.6 Hz, 1H).

### Step 2

In a similar manner to Step 8 of Example 1, 5-(dimethylaminomethyl)-7-[1-(tert-butoxycarbonyl)-5-(ethylamino methyl)indol-2-yl]isoindolinone (77.0 mg, 0.166 mmol) was dissolved in methanol (2 mL), and the solution was added with 10% hydrogen chloride-methanol solution (2 mL). The reaction mixture was condensed under reduced pressure, and added with methanol and ethyl acetate. The obtained solid was collected by filtration and washed with ethyl acetate, followed by drying under reduced pressure to obtain Compound 536 (60.0 mg, yield 83%).
ESI-MS *m*/*z*: 363 [M+H]⁺; ¹H-NMR (DMSO-*d*₆)δ(ppm) : 1.22 (t, *J* = 7.1 Hz, 3H), 2.76 (d, *J* = 5.1 Hz, 6H), 2.96 (q, *J* = 7.1 Hz, 2H), 4.12-4.20 (m, 2H), 4.43 (d, *J* = 5.1 Hz, 2H), 4.54 (s, 2H), 7.29 (d, *J* = 8.4 Hz, 1H), 7.40 (s, 1H), 7.55 (d, *J* = 8.4 Hz, 1H), 7.62 (s, 1H), 7.77 (s, 1H), 8.59 (s, 1H), 8.95 (br s, 2H), 9.45 (s, 1H), 11.18 (br s, 1H), 14.01 (s, 1H).

### Example 70:

### 5-(Dimethylaminomethyl)-7-[1H-5-(pyrrolidin-1-ylmethyl)indol-2-y l]isoindolinone dihydrochloride (Compound 537)

### Step 1

In a similar manner to Step 2 of Example 45, 5-(dimethylaminomethyl)-7-[1-(tert-butoxycarbonyl)-5-formylindol -2-yl]isoindolinone (80.0 mg, 0.185 mmol) was dissolved in acetonitrile (4.8 mL), and the solution was treated with pyrrolidine (0.153 mL, 1.84 mmol), acetic acid (0.105 mL, 1.84 mmol) and sodium triacetoxyborohydride (119 mg, 0.561 mmol), followed by purification by preparative thin-layer chromatography (chloroform/methanol/7 mol/L ammonia-methanol solution=12/1/1) to obtain 5-(dimethylaminomethyl)-7-[1-(tert-butoxycarbonyl)-5-(pyrrolidin -1-ylmethyl)indol-2-yl]isoindolinone (29.9 mg, yield 67%).
ESI-MS *m*/*z*: 489 [M+H]⁺; ¹H-NMR (CDCl₃)δ(ppm) : 1.29 (s, 9H), 1.71-1.88 (m, 4H) , 2.29 (s, 6H), 2.47-2.62 (m, 4H), 3.55 (s, 2H), 3.71 (s, 2H), 4.41 (s, 2H), 6.55 (s, 2H), 7.29 (dd, *J* = 1.7, 8.6 Hz, 1H), 7.39 (s, 1H), 7.44 (s, 1H), 7.49 (s, 1H), 8.18 (d, *J* = 8.6 Hz, 1H).

### Step 2

In a similar manner to Step 8 of Example 1, 5-(dimethylaminomethyl)-7-[1-(tert-butoxycarbonyl)-5-(pyrrolidin -1-ylmethyl)indol-2-yl]isoindolinone (27.5 mg, 0.0563 mmol) was dissolved in methanol (1 mL), and the solution was added with 10% hydrogen chloride-methanol solution (1 mL). The obtained solid was collected by filtration and washed with methanol, followed by drying under reduced pressure to obtain Compound 537 (15.2 mg, yield 59%).
ESI-MS *m*/*z*: 389 [M+H]⁺; ¹H-NMR (DMSO-*d*₆)δ(ppm) : 1.75-2.11 (m, 4H), 2.75 (s, 6H), 3.00-3.22 (m ,4H), 4.38 (d, *J* = 5.6 Hz, 2H), 4.43 (s, 2H), 4.55 (s, 2H), 7.33 (d, *J* = 8.3 Hz, 1H), 7.41 (s, 1H), 7.56 (d, *J* = 8.3 Hz, 1H), 7.62 (s, 1H), 7.82 (s, 1H), 8.59 (s, 1H), 9.45 (s, 1H), 10.46 (br s, 1H), 11.14 (br s, 1H), 14.01 (s, 1H).

### Example 71:

### 5-(Dimethylaminomethyl)-7-[1H-5-(dimethylaminomethyl)indol-2-yl] isoindolinone dihydrochloride (Compound 538)

### Step 1

In a similar manner to Step 2 of Example 45, 5-(dimethylaminomethyl)-7-[1-(tert-butoxycarbonyl)-5-formylindol -2-yl]isoindolinone (60.0 mg, 0.138 mmol) was dissolved in acetonitrile (3.5 mL), and the solution was treated with 2.0 mol/L dimethylamine-THF solution (1.40 mL, 2.80 mmol), acetic acid (0.158 mL, 2.76 mmol) and sodium triacetoxyborohydride (118 mg, 0.555 mmol) , followed by purification by preparative thin-layer chromatography (chloroform/methanol=4/1) to obtain 5-(dimethylaminomethyl)-7-[1-(tert-butoxycarbonyl)-5-(dimethylam inomethyl)indol-2-yl]isoindolinone (51.4 mg, yield 81%).
ESI-MS *m*/*z*: 463 [M+H]⁺; ¹H-NMR (CDCl₃)δ(ppm) : 1.29 (s, 9H), 2.29 (s, 12H), 3.55 (s, 2H), 3.58 (s, 2H), 4.42 (s, 2H), 6.56 (s, 1H), 6.67 (br s, 1H), 7.27 (dd, *J* = 1.8, 8.4 Hz, 1H), 7.40 (s, 1H), 7.45 (s, 1H), 7.48 (s, 1H), 8.20 (d, *J* = 8.4 Hz, 1H).

### Step 2

In a similar manner to Step 8 of Example 1, 5-(dimethylaminomethyl)-7-[1-(tert-butoxycarbonyl)-5-(dimethylam inomethyl)indol-2-yl]isoindolinone (49.0 mg, 0.106 mmol) was dissolved in methanol (1.5 mL), and the solution was added with 10% hydrogen chloride-methanol solution (1.5 mL). The reaction mixture was added with diisopropylether. The obtained solid was collected by filtration and washed with diisopropylether, followed by drying under reduced pressure to obtain Compound 538 (40.8 mg, yield 88%).
ESI-MS *m*/*z*: 363 [M+H]⁺; ¹H-NMR (DMSO-*d*₆)δ(ppm) : 2.70 (d, *J* = 4.5 Hz, 6H), 2.75 (d, *J* = 3.5 Hz, 6H), 4.31 (d, *J* = 4.6 Hz, 2H), 4.43 (br s, 2H), 4.54 (s, 2H), 7.29 (dd, *J* = 1.5, 8.4 Hz, 1H), 7.42 (s, 1H), 7.57 (d, *J* = 8.4 Hz, 1H), 7.63 (s, 1H), 7.79 (s, 1H), 8.61 (s, 1H), 9.45 (s, 1H), 10.30 (br s, 1H), 11.27 (br s, 1H), 14.02 (s, 1H).

### Example 72:

### 5-(Dimethylaminomethyl)-[1H-5-(piperidinocarbonyl)indol-2-yl]iso indolinone hydrochloride (Compound 539)

### Step 1

In a similar manner to Step 7 of Example 1, 5-(dimethylaminomethyl)-7-iodoisoindolinone (50.0 mg, 0.158 mmol) was dissolved in acetonitrile (3 mL), and the solution was treated with Compound CI (118 mg, 0.316 mmol), palladium acetate (3.0 mg, 0.013 mmol), tri(o-tolyl)phosphine (8.0 mg, 0.025 mmol) and triethylamine (0.220 mL, 1.58 mmol), followed by purification by preparative thin-layer chromatography (chloroform/methanol=9/1) to obtain 5-(dimethylaminomethyl)-7-[1-(tert-butoxycarbonyl)-5-(piperidino carbonyl)indol-2-yl]isoindolinone (67.9 mg, yield 83%).
ESI-MS *m*/*z*: 517 [M+H]⁺; ¹H-NMR (CDCl₃)δ(ppm) : 1.30 (s, 9H), 1.43-1.83 (m, 6H), 2.30 (s, 6H), 3.33-3.88 (m, 4H), 3.56 (s, 2H), 4.43 (s, 2H), 6.46 (br s, 1H), 6.60 (s, 1H), 7.35 (dd, *J* = 1.2, 8.6 Hz, 1H), 7.42 (s, 1H), 7.47 (s, 1H), 7.61 (s, 1H), 8.20 (d, *J* = 8.6 Hz, 1H).

### Step 2

In a similar manner to Step 2 of Example 34, 5-(dimethylaminomethyl)-7-[1-(tert-butoxycarbonyl)-5-(piperidino carbonyl)indol-2-yl]isoindolinone (65.4 mg, 0.127 mmol) was treated with 4 mol/L hydrochloric acid-ethyl acetate solution (3 mL). The obtained solid was collected by filtration, and washed with ethyl acetate, followed by drying under reduced pressure to obtain Compound 539 (29.7 mg, yield 52%).
ESI-MS *m*/*z*: 417 [M+H]⁺; ¹H-NMR (DMSO-*d*₆)δ(ppm) : 1.41-1.73 (m, 6H), 2.76 (d, *J* = 4.5 Hz, 6H), 3.30-3.66 (m, 4H), 4.42 (d, *J* = 4.8 Hz, 2H), 4.54 (s, 2H), 7.14 (dd, *J* = 1.3, 8.5 Hz, 1H), 7.34 (s, 1H), 7.51 (d, *J* = 8.5 Hz, 1H), 7.61 (s, 1H), 7.62 (s, 1H), 8.47 (s, 1H), 9.45 (s, 1H), 10.77 (br s, 1H), 14.02 (s, 1H).

### Example 73:

### 5-(2,6-Dimethylphenyl)-7-[1H-5-(piperidinomethyl)indol-2-yl]isoi ndolinone (Compound 540)

### Step 1

In a similar manner to Step 1 of Example 43, crude 5-(2,6-dimethylphenyl)-7-[1-(tert-butoxycarbonyl)-5-(piperidinom ethyl)indol-2-yl]isoindolinone was obtained using 5-chloro-7-[1-(tert-butoxycarbonyl)-5-(piperidinomethyl)indol-2-yl]isoindolinone (60.0 mg, 0.125 mmol), 2,6-dimethylphenylboronic acid (37.5 mg, 0.250 mmol), cesium fluoride (66.5 mg, 0.438 mmol), 2-dicyclohexylphosphino-2',4',6'-triisopropylbiphenyl (17.9 mg, 0.0375 mmol), tris(dibenzylideneacetone)dipalladium(0) (11.4 mg, 0.0125 mmol) and 1,4-dioxane (1.8 mL).
ESI-MS *m*/*z*: 550 [M+H]⁺

### Step 2

In a similar manner to Step 8 of Example 1, Compound 540 (8.40 mg, yield 15%, 2 steps) was obtained using crude 5-(2,6-dimethylphenyl)-7-[1-(tert-butoxycarbonyl)-5-(piperidinom ethyl)indol-2-yl]isoindolinone, and 10% hydrogen chloride-methanol solution (2.0 mL).
ESI-MS *m*/*z*: 450 [M+H]⁺; ¹H-NMR (DMSO-*d*₆)δ(ppm) : 1.31-1.59 (m, 6H), 2.05 (s, 6H), 2.24-2.40 (m, 4H), 3.30 (s, 2H), 4.55 (s, 2H), 6.55 (s, 1H), 7.07 (d, *J* = 9.0 Hz, 1H), 7.14-7.31 (m, 3H), 7.36-7.43 (m, 2H), 7.90 (s, 1H), 8.61 (s, 1H), 9.29 (s, 1H), 13.91 (s, 1H).

### Example 74:

### 5-{[N-Methyl(3-furan-2-ylpropyl)amino]methyl}-7-[1H-5-(piperidin omethyl)indol-2-yl]isoindolinone (Compound 541)

### Step 1

In a similar manner to Step 1 of Example 68, crude 5-{[N-methyl(3-furan-2-ylpropyl)amino]methyl}-7-[1-(tert-butoxyc arbonyl)-5-(piperidinomethyl)indol-2-yl]isoindolinone was obtained using 5-formyl-7-[1-(tert-butoxycarbonyl)-5-(piperidinomethyl)indol-2-yl]isoindolinone (60.0 mg, 0.127 mmol), (3-furan-2-ylpropyl)methylamine (51.4 mg, 0.370 mmol), molecular sieves 4A (350 mg), sodium borohydride (24.3 mg, 0.592 mmol) and methanol (1.8 mL)
ESI-MS *m*/*z*: 597 [M+H]⁺

### Step 2

In a similar manner to Step 8 of Example 1, Compound 541 (7.60 mg, yield 10%, 2 steps) was obtained using crude 5-{[N-methyl(3-furan-2-ylpropyl)amino]methyl}-7-[1-(tert-butoxyc arbonyl)-5-(piperidinomethyl)indol-2-yl]isoindolinone, 10% hydrogen chloride-methanol solution (1.0 mL) and methanol (1.0 mL).
ESI-MS *m*/*z*: 497 [M+H]⁺; ¹H-NMR (DMSO-*d*₆)δ(ppm) : 1.82 (t, *J* = 7.7 Hz, 2H), 2.19 (s, 3H), 2.65 (t, *J* = 7.7 Hz, 2H), 3.55-3.68 (m, 4H), 4.50 (s, 2H), 6.06 (s, 1H), 6.30 (s, 1H), 7.09-7.30 (m, 2H), 7.39-7.59 (m, 4H), 8.08 (s, 1H), 9.24 (s, 1H), 13.48 (s, 1H).

### Example 75:

### 5-(2-Propyloxy)-7-(1H-indol-2-yl)isoindolinone (Compound 542)

### Step 1

In a similar manner to Step 7 of Example 1, 5-(2-propyloxy)-7-iodoisoindolinone (50.0 mg, 0.158 mmol) was dissolved in acetonitrile (3 mL), and the solution was treated with 1- (tert-butoxycarbonyl)indole-2-boronic acid (82. 0 mg, 0.315 mmol) , palladium acetate (3.0 mg, 0.013 mmol), tri(o-tolyl)phosphine (8.0 mg, 0.025 mmol) and triethylamine (0.220 mL, 1.58 mmol), followed by purification by preparative thin-layer chromatography (chloroform/methanol=20/1) to obtain 5-(2-propyloxy)-7-[1-(tert-butoxycarbonyl)indol-2-yl]isoindolino ne (59.3 mg, yield 92%).
ESI-MS *m*/*z*: 407 [M+H]⁺; ¹H-NMR (CDCl₃)δ(ppm) : 1.33 (s, 9H) , 1.39 (d, *J* = 6.0 Hz, 6H), 4.37 (s, 2H), 4.67 (m, 1H), 6.43 (s, 1H), 6.58 (s, 1H), 6.90 (d, *J* = 2.2 Hz, 1H), 6.99 (d, *J* = 2.2 Hz, 1H), 7.22 (m, 1H), 7.32 (m, 1H), 7.55 (d, *J* = 7.5 Hz, 1H), 8.25 (d, *J* = 7.9 Hz, 1H).

### Step 2

In a similar manner to Step 8 of Example 1, 5-(2-propyloxy)-7-[1-(tert-butoxycarbonyl)indol-2-yl]isoindolino ne (57.0 mg, 0.140 mmol) was dissolved in methanol (1.5 mL), and the solution was treated with 10% hydrogen chloride-methanol solution (1.5 mL). The obtained solid was collected by filtration and washed with methanol, followed by drying under reduced pressure to obtain Compound 542 (27.4 mg, yield 64%).
ESI-MS *m*/*z:* 307 [M+H]⁺; ¹H-NMR (DMSO-*d*₆)δ(ppm) : 1.33 (d, *J* = 6.0 Hz, 6H), 4.44 (s, 2H), 4.85 (m, 1H), 6.98-7.18 (m, 3H), 7.26 (s, 1H), 7.44 (d, *J* = 8.1 Hz, 1H), 7.52-7.67 (m, 2H), 9.01 (s, 1H), 14.01 (s, 1H).

### Example 76:

### 5-(Dimethylaminomethyl)-(1H-5-{[N-(ethyl)acetylamino]methyl}indo 1-2-yl)isoindolinone (Compound 543)

Compound 536 (25.0 mg, 0.0574 mmol) was dissolved in THF (1 mL), and the solution was added with acetic anhydride (0.110 mL, 1.14 mmol) and pyridine (1.00 mL, 12.4 mmol), followed by stirring at room temperature for 24 hours. The reaction mixture was added with water and sodium carbonate, and extracted with ethyl acetate. The organic layer was washed with saturated brine and dried over anhydrous sodium sulfate. The solvent was evaporated under reduced pressure, and the residue was purified by preparative thin-layer chromatography (chloroform/methanol/7 mol/L ammonia-methanol solution/12/1/1) to obtain Compound 543 (9.5 mg, yield 41%).
ESI-MS *m*/*z*: 405 [M+H]⁺; ¹H-NMR (CDCl₃)δ(ppm) : 1.13 (t, *J* = 7.1 Hz, 3H), 2.19 (s, 3H), 2.31 (s, 6H), 3.47 (q, *J* = 7.1 Hz, 2H), 3.56 (s, 2H), 4.51 (s, 2H), 4.61 (s, 2H), 6.56 (s, 1H), 6.95-7.16 (m, 2H), 7.22-7.57 (m, 3H), 8.02 (s, 1H), 13.52 (s, 1H).

### Example 77:

### 5-(Dimethylaminomethyl)-[1H-5-(2-oxopyrrolidin-1-ylmethyl)indol-2-yl)isoindolinone (Compound 544)

In a similar manner to Step 2 of Example 45, 5-(dimethylaminomethyl)-7-[1-(tert-butoxycarbonyl)-5-formyl]indo lylisoindolinone (40.0 mg, 0. 0923 mmol) was dissolved in acetonitrile (5 mL), and the solution was treated with methyl 4-aminobutylate hydrochloride (142 mg, 0.924 mmol), triethylamine (0.129 mL, 0.926 mmol), acetic acid (0.106 mL, 1.85 mmol) and sodium triacetoxyborohydride (80.0 mg, 0.373 mmol) , followed by purification by preparative thin-layer chromatography (chloroform/methanol=5/1) to obtain 5-(dimethylaminomethyl)-7-{1-(tert-butoxycarbonyl)-5-[3-(methoxy carbonyl)propylaminomethyl]indol-2-yl}isoindolinone (21.7 mg, yield 44%).
ESI-MS *m*/*z*: 535 [M+H]⁺; ¹H-NMR (CDCl₃)δ(ppm) : 1.26 (s, 9H), 1.86 (tt, *J* = 7.3, 7.4 Hz, 2H), 2.29 (s, 6H), 2.33 (t, *J* = 7.4 Hz, 2H), 2.69 (t, *J* = 7.3 Hz, 2H), 3.55 (s, 2H), 3.62 (s, 3H), 3.96 (s, 2H), 4.43 (s, 2H), 6.55 (s, 1H), 7.27 (dd, *J* = 1.7, 8.6 Hz, 1H), 7.40 (s, 1H), 7.46 (br s, 2H), 7.60 (br s, 1H), 8.25 (d, *J* = 8.6 Hz, 1H).

### Step 2

In a similar manner to Step 8 of Example 1, 5-(dimethylaminomethyl)-7-{1-(tert-butoxycarbonyl)-5-[3-(methoxy carbonyl)propylaminomethyl]indol-2-yl}isoindolinone (21.5 mg, 0.0402 mmol) was dissolved in methanol (2 mL), and the solution was treated with 10% hydrogen chloride-methanol solution (2 mL). The reaction mixture was condensed under reduced pressure, and the residue was added with acetonitrile (3 mL), followed by stirring at 80 °C for 10 hours. The reaction mixture was condensed under reduced pressure, and the residue was purified by preparative thin-layer chromatography (chloroform/methanol=6/1) and by slurry using hexane and ethyl acetate to obtain Compound 544 (9.0 mg, yield 56%).
ESI-MS *m*/*z*: 403 [M+H]⁺; ¹H-NMR (DMSO-*d*₆)δ(ppm) : 1.90-2.02 (m, 2H), 2.32 (s, 6H), 2.46 (t, *J* = 8.2 Hz, 2H), 3.28 (t, *J* = 7.0 Hz, 2H), 3.56 (s, 2H), 4.48 (s, 2H), 4.54 (s, 2H), 6.91 (s, 1H), 7.05-7.12 (m, 2H), 7.32 (s, 1H), 7.45 (d, *J* = 8.3 Hz, 1H), 7.49 (s, 1H), 8.00 (s, 1H), 13.48 (s, 1H).

### Example 78:

### 5-(1-Hydroxy-2,2-dimethylproyl)-(1H-indol-2-yl)isoindolinone (Compound 545)

### Step 1

In a similar manner to Step 7 of Example 1, 5-formyl-7-iodoisoindolinone (100 mg, 0.348 mmol) was dissolved in acetonitrile (5.8 mL), and the solution was treated with 1-(tert-butoxycarbonyl)indole-2-boronic acid (182.0 mg, 0.696 mmol), palladium acetate (6.3 mg, 0.028 mmol), tri(o-tolyl)phosphine (17.0 mg, 0.0559 mmol) and triethylamine (0.485 mL, 3.48 mmol), followed by purification by preparative thin-layer chromatography (chloroform/methanol=20/1) to obtain 5-formyl-7-[1-(tert-butoxycarbonyl)indol-2-yl]isoindolinone (55.5 mg, yield 42%).
ESI-MS *m*/*z*: 377 [M+H]⁺; ¹H-NMR (CDCl₃)δ(ppm) : 1.31 (s, 9H), 4.46 (s, 2H), 6.66 (s, 1H), 7.23 (m, 1H), 7.37 (dd, *J* = 1.3, 7.5 Hz, 1H), 7.59 (d, *J* = 7.5 Hz, 1H), 7.91 (s, 1H), 7.92-8.04 (m, 2H), 8.26 (d, *J* = 8.4 Hz, 1H), 10.15 (s, 1H).

### Step 2

In a similar manner to Step 1 of Example 56, 5-formyl-7-[1-(tert-butoxycarbonyl)indol-2-yl]isoindolinone (55.5 mg, 0.147 mmol) was dissolved in THF (2 mL), and the solution was treated with tert-butyllithium-pentane solution (1.46 mol/L, 0.40 mL, 0.58 mmol), followed by purification by preparative thin-layer chromatography (chloroform/methanol=20/1) to obtain 5-(1-hydroxy-2,2-dimethylpropyl)-7-[1-(tert-butoxycarbonyl)indol -2-yl]isoindolinone (9.6 g, yield 15%).
ESI-MS *m*/*z*: 435 [M+H]⁺; ¹H-NMR (CDCl₃)δ(ppm) : 0.98 (s, 9H), 1. 31 (s, 9H), 4.44 (s, 2H), 4.54 (s, 1H), 6.36 (br s, 1H), 6.56 (s, 1H), 7.18-7.37 (m, 3H), 7.39 (s, 1H), 7.45 (s, 1H), 7.55 (d, *J* = 7.6 Hz, 1H), 8.27 (d, *J* = 8.3 Hz, 1H).

### Step 3

In a similar manner to Step 8 of Example 1, 5-(1-hydroxy-2,2-dimethylpropyl)-7-[1-(tert-butoxycarbonyl)indol -2-yl]isoindolinone (9.6 mg, 0.022 mmol) was dissolved in methanol (1 mL), and the solution was treated with 10% hydrogen chloride-methanol solution (1 mL), followed by purification by preparative thin-layer chromatography (chloroform/methanol=20/1) to obtain Compound 545 (5.4 mg, yield 78%).
ESI-MS *m*/*z*: 335 [M+H]⁺; ¹H-NMR (DMSO-*d*₆)δ(ppm) : 0.99 (s, 9H), 1.24 (m, 1H), 2.19 (br s, 1H), 4.46 (s, 2H), 4.53 (s, 1H), 6.37 (s, 1H), 7.08 (s, 1H), 7.20 (dd, *J* = 7.6, 8.1 Hz, 1H), 7.51 (d, *J* = 8.1 Hz, 1H), 7.63 (d, *J* = 7.6 Hz, 1H), 7.99 (s, 1H), 13.35 (br s, 1H).

### Example 79:

### 5-Bromo-7-[1H-5-(piperidinomethyl)indol-2-yl]isoindolinone (Compound 546)

### Step 1

4-Bromo-2-methylbenzoic acid (3.0 g, 14.0 mmol) was dissolved in DMF (30 mL), and the solution was added with N-iodosuccinimide (3.77 g, 16.7 mmol) and palladium acetate (314 mg, 1.4 mmol), followed by stirring at 100 °C for 3 days. The reaction mixture was added with water and extracted with ethyl acetate. The organic layer was condensed under reduced pressure. The residue was purified by silica gel column chromatography (chloroform/methanol=100/0-80/20) to obtain 4-bromo-2-iodo-6-methylbenzoic acid (2.87 g, yield 60%)
ESI-MS *m*/*z*: 341 [M-H]⁻; ¹H-NMR (DMSO-*d*₆)δ(ppm) : 2.26 (s, 3H), 7.56 (s, 1H), 7.90 (s, 1H).

### Step 2

4-Bromo-2-iodo-6-methylbenzoic acid (2.87 g, 8.42 mmol) was dissolved in dichloromethane (43 mL) , and the solution was added with thionyl chloride (1.02 mL, 14.0 mmol) followed by stirring at room temperature for 2.0 hours. The reaction mixture was condensed under reduced pressure. The residue was added into a mixture of methanol (43 mL) and triethylamine (2.35 mL) at 0 °C, followed by stirring at room temperature for 4.0 hours. The reaction mixture was added with water and extracted with ethyl acetate. The organic layer was condensed under reduced pressure. The residue was purified by silica gel column chromatography (hexane/ethyl acetate=97/3-10/90) to obtain methyl 4-bromo-2-iodo-6-methylbenzoate (1.31 g, yield 44%).
¹H-NMR (DMSO-*d*₆)δ(ppm) : 2.25 (s, 3H), 3.87 (s, 3H), 7.60 (s, 1H), 7.94 (s, 1H).

### Step 3

Methyl 4-bromo-2-iodo-6-methylbenzoate (1.31 g, 3.69 mmol) was dissolved in carbon tetrachloride (20 mL), and the solution was added with *N*-bromosuccinimide (788 mg, 4.43 mmol) and benzoyl peroxide (89.4 mg, 0.369 mmol) , followed by stirring at 80 °C for 2 days. The reaction mixture was added with 2 mol/L aqueous sodium hydroxide solution and extracted with ethyl acetate. The organic layer was condensed under reduced pressure. The residue was purified by silica gel column chromatography (hexane/ethyl acetate=99/1-85/15) to obtain methyl 4-bromo-2-bromoethyl-6-iodobenzoate (1.53 g, yield 96%). ¹H-NMR (DMSO-*d*₆)δ(ppm) : 2.25 (s, 2H), 3.91 (s, 3H) , 7.87 (s, 1H), 8.13 (s, 1H).

### Step 4

Methyl 4-bromo-2-bromoethyl-6-iodobenzoate (1. 56 g, 3.40 mmol) was dissolved in THF (39 mL), and the solution was added with 28% aqueous ammonia (16 mL), followed by stirring at room temperature for 7.5 hours. The reaction mixture was added with water and extracted with ethyl acetate. The organic layer was condensed under reduced pressure. The residue was crystallized by addition of a mixed solvent of hexane (2.5 mL)/ethyl acetate (2.5 mL) to obtain 5-bromo-7-iodoisoindolinone (554 mg, yield 48%).
ESI-MS *m*/*z*: 339 [M+H]⁺; ¹H-NMR (DMSO-*d*₆)δ(ppm) : 4.27 (s, 2H), 7.87 (s, 1H), 8.09 (s, 1H), 8.80 (s, 1H).

### Step 5

5-Bromo-7-iodoisoindolinone (554 mg, 1.64 mmol) was dissolved in a mixed solvent of DMA (11 mL)/water (1.2 mL), and the solution was added with Compound BD (705 mg, 1.97 mmol), triethylamine (1.14 mL, 8.20 mmol) and [bis(diphenylphosphino)ferrocene]dichloropalladium (134 mg, 0.164 mmol), followed by stirring at room temperature for 12 hours. The reaction mixture was added with water and extracted with ethyl acetate. The organic layer was condensed under reduced pressure. The residue was purified by silica gel column chromatography (chloroform/methanol=99/1-85/15) to obtain 5-Bromo-7-[1-(tert-butoxycarbonyl)-5-(piperidinomethyl)indol-2-y l]isoindolinone (177 mg, yield 21%).
ESI-MS *m*/*z*: 526 [M+H]⁺

### Step 6

In a similar manner to Step 8 of Example 1, Compound 546 (10.0 mg, yield 25%) was obtained using 5-bromo-7-[1-(tert-butoxycarbonyl)-5-(piperidinomethyl)indol-2-y l]isoindolinone (50.0 mg, 0.0953 mmol), 10% hydrogen chloride-methanol solution (1.0 mL) and methanol (1.0 mL). ESI-MS *m*/*z*: 426 [M+H]⁺; ¹H-NMR (DMSO-*d*₆)δ(ppm) : 1.32-1.61 (m, 6H), 2.28-2.43 (m, 4H), 3.37 (s, 2H), 4.50 (s, 2H), 6.55 (s, 1H), 7.05-7.20 (m, 1H), 7.30-7.58 (m ,2H), 7.73 (s, 1H), 8.33 (s, 1H), 9.35 (s, 1H), 13.16 (s, 1H).

### Example 80:

### 5-(2-Propyloxy)-7-{1H-5-[4-(2-hydroxyethyl)piperazin-1-ylmethyl] indol-2-yl}isoindolinone dihydrochloride (Compound 547)

### Step 1

In a similar manner to Step 7 of Example 1, 5-(2-propyloxy)-7-iodoisoindolinone (200 mg, 0.631 mmol) was dissolved in acetonitrile (10 mL) , and the solution was treated with Compound BA (365 mg, 1.26 mmol), palladium acetate (11.3 mg, 0.0503 mmol), tri(o-tolyl)phosphine (30.7mg, 0.101 mmol) and triethylamine (0.880 mL, 6.31 mmol), followed by purification by flush column chromatography (chloroform/methanol=95/5) to obtain 5-(2-propyloxy)-7-[1-(tert-butoxycarbonyl)-5-formylindol-2-yl]is oindolinone (268 mg, yield 98%).
ESI-MS *m*/*z*: 435 [M+H]⁺; ¹H-NMR (CDCl₃)δ(ppm) : 1.33 (s, 9H), 1.40 (d, *J* = 6.2 Hz, 6H), 4.39 (s, 2H), 4.68 (m, 1H), 6.34 (br s, 1H), 6.69 (s, 1H), 6.94 (s, 1H), 7.00 (s, 1H), 7.87 (dd, *J* = 1.6, 8.5 Hz, 1H), 8.09 (d, *J* = 1.6 Hz, 1H), 8.39 (d, *J* = 8.5 Hz, 1H), 10.08 (s, 1H).

### Step 2

In a similar manner to Step 2 of Example 45, 5-(2-propyloxy)-7-[1-(tert-butoxycarbonyl)-5-formylindol-2-yl]is oindolinone (132 mg, 0.304 mmol) was dissolved in acetonitrile (6 mL), and the solution was treated with 1-piperazineethanol (0.373 mL, 3.04 mmol), acetic acid (0.348 mL, 6.08 mmol) and sodium triacetoxyborohydride (193 mg, 0.912 mmol), followed by purification by preparative thin-layer chromatography (chloroform/methanol=9/1) to obtain 5-(2-propyloxy)-7-{1-(tert-butoxycarbonyl)-5-[4-(2-hydroxyethyl) piperazin-1-ylmethyl]indol-2-yl}isoindolinone (135 mg, yield 81%).
ESI-MS *m*/*z*: 549 [M+H]⁺; ¹H-NMR (CDCl₃)δ(ppm) : 1.33 (s, 9H) , 1.39 (d, *J* = 5.9 Hz, 6H) , 2.41-2.65 (m, 10H), 3.52-3.68 (m, 2H), 3.61 (s, 2H), 4.38 (s, 2H), 4.66 (m, 1H), 6.24 (br s, 1H), 6.90 (d, *J* = 2.0 Hz, 1H), 6.98 (d, *J* = 2.0 Hz, 1H), 7.28 (dd, *J* = 1.5, 8.6 Hz, 1H), 7.47 (s, 1H), 8.18 (d, *J* = 8.6 Hz, 1H).

### Step 3

In a similar manner to Step 8 of Example 1, 5-(2-propyloxy)-7-{1-(tert-butoxycarbonyl)-5-[4-(2-hydroxyethyl) piperazin-1-ylmethyl]indol-2-yl}isoindolinone (133 mg, 0.241 mmol) was dissolved in methanol (2.5 mL), and the solution was treated with 10% hydrogen chloride-methanol solution (2.5 mL). The reaction mixture was condensed under reduced pressure, and the residue was added with methanol and ethyl acetate. The obtained solid was collected by filtration and washed with ethyl acetate, followed by drying under reduced pressure to obtain Compound 547 (104 mg, yield 83%). 4.31-4.60 (m, 4H), 4.85 (m, 1H), 7.11 (d, *J* = 1.9 Hz, 1H), 7.32 (m, 1H), 7.35 (s, 1H), 7.54 (d, *J* = 8.4 Hz, 1H), 7.63 (d, *J* = 1.9 Hz, 1H), 7.77 (br s, 1H), 9.05 (s, 1H), 14.21 (br s, 1H).

### Example 81:

### 5-(2-Propyloxy)-7-[1H-5-(diethylaminomethyl)indol-2-yl]isoindoli none hydrochloride (Compound 548)

### Step 1

In a similar manner to Step 2 of Example 45, 5-(2-propyloxy)-7-[1-(tert-butoxycarbonyl)-5-formylindol-2-yl]is oindolinone (132 mg, 0.304 mmol) was dissolved in acetonitrile (6 mL), and the solution was treated with diethylamine (0.314 mL, 3.04 mmol), acetic acid (0.348 mL, 6.08 mmol) and sodium triacetoxyborohydride (518 mg, 2.44 mmol) , followed by purification by preparative thin-layer chromatography (chloroform/methanol=9/1) to obtain 5-(2-propyloxy)-7-[1-(tert-butoxycarbonyl)-5-(diethylaminomethyl )indol-2-yl]isoindolinone (85.2 mg, yield 57%).
ESI-MS *m*/*z*: 492 [M+H]⁺; ¹H-NMR (CDCl₃)δ(ppm) : 1.10 (t, *J* = 7.1 Hz, 6H), 1.33 (s, 9H), 1.39 (d, J= 5.9 Hz, 6H), 2.48-2.70 (m, 4H), 3.66-3.76 (m, 2H), 4.37 (s, 2H), 4.66 (m, 1H), 6.36 (br s, 1H), 6.54 (s, 1H), 6.90 (br s, 1H), 6.98 (d, *J* = 1.7 Hz, 1H), 7.31 (dd, *J* = 1.7, 8.7 Hz, 1H), 7.52 (br s, 1H), 8.17 (d, *J* = 8.7 Hz, 1H).

### Step 2

In a similar manner to Step 8 of Example 1, 5-(2-propyloxy)-7-[1-(tert-butoxycarbonyl)-5-(diethylaminomethyl )indol-2-yl]isoindolinone (82.9 mg, 0.169 mmol) was dissolved in methanol (2 mL), and the solution was treated with 10% hydrogen chloride-methanol solution (2 mL). The reaction mixture was condensed under reduced pressure, and the residue was added with methanol and ethyl acetate. The obtained solid was collected by filtration and washed with ethyl acetate, followed by drying under reduced pressure to obtain Compound 548 (61.3 mg, yield 85%). ESI-MS *m*/*z*: 392 [M+H]⁺; ¹H-NMR (DMSO-*d*₆)δ(ppm) : 1.25 (t, *J* = 7.2 Hz, 6H), 1.33 (d, *J* = 5.8 Hz, 6H), 2.90-3.20 (m, 4H), 4.33 (d, *J* = 4.6 Hz, 2H), 4.45 (s, 2H), 4.85 (m, 1H), 7.11 (s, 1H), 7.29 (d, *J* = 8.4 Hz, 1H), 7.35 (s, 1H), 7.54 (d, *J* = 8.4 Hz, 1H), 7.62 (s, 1H), 7.76 (s, 1H), 9.06 (s, 1H), 9.78 (br s, 1H), 14.21 (s, 1H).

### Example 82:

### 5-[(1-Hydroxy-2-propyl)amino]-7-[1H-5-(piperidinomethyl)indol-2-yl]isoindolinone dihydrochloride (Compound 549)

### Step 1

In a similar manner to Step 2 of Example 1, 4-aminobenzoic acid (5.00 g, 36.5 mmol) was dissolved in DMF (100 mL), and the solution was treated with EDCI (10.5 g, 54.7 mmol), HOBt monohydrate (2.79 g, 18.2 mmol) and cumylamine (10.5 mL, 72.9 mmol), followed by purification by slurry using diisopropylether to obtain 4-amino-N-(1-methyl-1-phenylethyl)benzamide (8.87 g, yield 96%). ESI-MS *m*/*z*: 255 [M+H]⁺

### Step 2

4-Amino-*N*-(1-methyl-1-phenylethyl)benzamide (8.87 g, 34.9 mmol) was dissolved in THF (355 mL), and the solution was added with di-tert-butyl dicarbonate (11.4 g, 52.4 mmol) and 4 mol/L aqueous sodium hydroxide solution (26.2 mL, 105 mmol), followed by stirring at 60 °C for 19 hours. The reaction mixture was added with water and extracted with ethyl acetate. The organic layer was washed with saturated brine and dried over sodium sulfate. The solvent was evaporated under reduced pressure, and the residue was purified by flush column chromatography (chloroform/methanol=100/0, 99/1, 98/2) to obtain 4-(*tert*-butoxycarbonylamino)-*N*-(1-methyl-1-phenylethyl)benzamide (2.57 g, yield 21%).
ESI-MS *m*/*z*: 355 [M+H]⁺

### Step 3

4-(*tert*-Butoxycarbonylamino)-*N*-(1-methyl-1-phenylethyl)benz amide (1.00 g, 2.82 mmol) was dissolved in THF (50 mL), and the solution was added with TMEDA (1.70 mL, 11.3 mmol), then, sec-butyllithium-hexane solution (1.00 mol/L, 11.3 mL, 11.3 mmol) was added dropwise thereto at -78 °C for 10 minutes under nitrogen atmosphere, followed by stirring at same temperature for 10 minutes, then at -30 °C for 1 hour. To the mixture, DMF (0.880 mL, 11.4 mmol) was added at -78 °C, followed by heating from -78 °C to room temperature for 1 hour. The reaction mixture was added with water and extracted with ethyl acetate. The organic layer was washed with saturated brine and dried over sodium sulfate. The solvent was evaporated under reduced pressure, and the residue was purified by flush column chromatography (hexane/ethyl acetate=90/10, 80/20, 70/30) to obtain 5-(*tert*-butoxycarbonylamino)-3-hydroxy-2-(1-methyl-1-phenylethyl )isoindolinone (1.03 g, yield 95%).
ESI-MS *m*/*z*: 383 [M+H]⁺; ¹H-NMR (CDCl₃)δ(ppm) : 1.53 (s, 9H), 1.90 (s, 3H), 1.95 (s, 3H), 2.84 (d, *J* = 10.4 Hz, 1H), 6.06 (d, *J* = 10.4 Hz, 1H), 6.76 (s, 1H), 7.18-7.23 (m, 2H), 7.26-7.34 (m, 2H), 7.37-7.44 (m, 2H), 7.53 (d, *J* = 8.3 Hz, 1H), 7.76 (d, *J* = 1.3 Hz, 1H).

### Step 4

5-(*tert*-Butoxycarbonylamino)-3-hydroxy-2-(1-methyl-1-phenyl ethyl) isoindolinone (1.03 g, 2.69 mmol) was dissolved in THF (20 mL), and then, sec-buthyllithium-hexane solution (1.00 mol/L, 13.5 mL, 13.5 mmol) was added dropwise thereto at -78 °C for 15 minutes under nitrogen atmosphere, followed by stirring at same temperature for 10 minutes, then at -30 °C for 1.5 hours. To the mixture, a solution of iodine (1. 02 g, 4.01 mmol) in THF (10 mL) solution was added-78 °C, followed by heating from -78 °C to room temperature for 1 hour. The reaction mixture was added with saturated aqueous sodium thiosulfate solution, and extracted with ethyl acetate. The organic layer was washed with saturated brine and dried over anhydrous sodium sulfate. The solvent was evaporated under reduced pressure, and the residue was purified by flush column chramatography (hexane/ethyl acetate=90/10, 80/20, 75/25) to obtain 5-(*tert*-butoxycarbonylamino)-3-hydroxy-7-iodo-2-(1-methyl-1-phen ylethyl)isoindolinone (767 mg, yield 56%).
ESI-MS *m*/*z*: 509 [M+H]⁺; ¹H-NMR (CDCl₃)δ(ppm) : 1.52 (s, 9H), 1.93 (s, 3H), 1.97 (s, 3H), 2.27 (d, *J* = 10.1 Hz, 1H), 5.91 (d, *J* = 10.1 Hz, 1H), 6.66 (s, 1H), 7.23 (m, 1H), 7.29-7.37 (m, 2H), 7.40-7.46 (m, 2H), 7.74 (d, *J* = 1.8 Hz, 1H), 7.77 (d, *J* = 1.8 Hz, 1H).

### Step 5

In a similar manner to Step 7 of Example 6, 5-(*tert*-butoxycarbonylamino)-3-hydroxy-7-iodo-2-(1-methyl-1-phen ylethyl)isoindolinone (760 mg, 1.50 mmol) was dissolved in nitromethane (15 mL), and the solution was treated with trifluoroacetic acid (1.16 mL, 15.1 mmol) and triethylsilane (1.21 mL, 7.49 mmol) followed by purification by slurry using methanol and diisopropylether to obtain 5-amino-7-iodoisoindolinone (257 mg, yield 63%).
ESI-MS *m*/*z*: 274 [M+H]⁺; ¹H-NMR (DMSO-*d₆*)δ(ppm) : 4.07 (s, 2H), 5.88 (s, 2H), 6.61 (d, *J* = 1.8 Hz, 1H), 7.06 (d, *J* = 1.8 Hz, 1H), 8.05 (br s, 1H).

### Step 6

5-Amino-7-iodoisoindolinone (50.0 mg, 0.182 mmol) was dissolved in acetonitrile (4 mL), and the solution was added with hydroxyacetone (0.0062 mL, 0.906 mmol) and acetic acid (0.208 mL, 3.63 mmol), followed by stirring at 50 °C for 1 hour. The mixture was added with sodium triacetoxyborohydride (116 mg, 0.547 mmol) at room temperature, followed by stirring at room temperature for 1 hour. The mixture was added with hydroxyacetone (0.0062 mL, 0.906 mmol) and sodium triacetoxyborohydride (116 mg, 0.547 mmol), followed by stirring at room temperature for 2 hour. The reaction mixture was added with sodium triacetoxyborohydride (116 mg, 0.547 mmol), followed by stirring at room temperature for 1 hour. The reaction mixture was added with water and sodium carbonate to adjust the pH to 9, and extracted with ethyl acetate. The organic layer was washed with saturated brine and dried over anhydrous sodium sulfate. The solvent was evaporated under reduced pressure, and the residue was purified by slurry using thanol and diisopropylether to obtain 5-[(1-hydroxy-2-propyl)amino]-7-iodoisoindolinone (34.4 mg, yield 57%).
ESI-MS *m*/*z*: 333 [M+H]⁺; ¹H-NMR (DMSO-*d₆*)δ(ppm) : 1.11 (d, *J* = 6.2 Hz, 3H), 3.26 (m, 1H), 3.38-3.51 (m, 2H), 4.09 (s, 2H), 4.74 (t, *J* = 5.3 Hz, 1H), 6.19 (d, *J* = 7.5 Hz, 1H), 6.65 (br s, 1H), 7.09 (d, *J* = 1.7 Hz, 1H), 8.05 (br s, 1H).

### Step 7

In a similar manner to Step 7 of Example 1, 5-[(1-hydroxy-2-propyl)amino]-7-iodoisoindolinone (30.0 mg, 0.0903 mmol) was dissolved in acetonitrile (2 mL), and the solution was treated with Compound BD (65.0 mg, 0.181 mmol), palladium acetate (1.6 mg, 0.0071 mmol), tri(o-tolyl)phosphine (4.4 mg, 0.015 mmol) and triethylamine (0.126 mL, 0.904 mmol), followed by purification by preparative thin-layer chromatography (chloroform/methanol/7 mol/L ammonia-methanol solution=25/1/1) to obtain 5-[(1-hydroxy-2-propyl)amino]-7-[1-(tert-butoxycarbonyl)-5-(pipe ridinomethyl)indol-2-yl]isoindolinone (35.3 mg, yield 75%).
ESI-MS *m*/*z*: 519 [M+H]⁺; ¹H-NMR (CDCl₃)δ(ppm) : 1.21 (d, *J* = 6.2 Hz, 3H), 1.53 (s, 9H), 1.39-1.48 (m, 2H), 1.53-1.65 (m, 4H), 2.37-2.48 (m, 4H), 3.56 (m, 1H), 3.59 (s, 2H), 3.62-3.76 (m, 2H), 4.20-4.35 (m, 3H), 6.35-6.50 (m, 2H), 6.56 (s, 1H), 6.61 (s, 1H), 7.25 (m, 1H), 7.44 (s, 1H), 8.15 (d, *J* = 8.6 Hz, 1H).

### Step 8

In a similar manner to Step 8 of Example 1, 5-[(1-hydroxy-2-propyl)amino]-7-[1-(tert-butoxycarbonyl)-5-(pipe ridinomethyl)indol-2-yl]isoindolinone (34.0 mg, 0.0656 mmol) was dissolved in methanol (2 mL), and the solution was treated with 10% hydrogen chloride-methanol solution (2 mL). The obtained solid was collected by filtration and washed with methanol, followed by drying under reduced pressure to obtain Compound 549 (17.1 mg, yield 53%).
ESI-MS *m*/*z*: 419 [M+H]⁺; ¹H-NMR (DMSO-*d₆*)δ(ppm) : 1.18 (d, *J* = 6.4 Hz, 3H), 1.36 (m, 1H), 1.57-1.74 (m, 3H), 1.74-1.86 (m, 2H), 2.79-2.94 (m, 2H), 3.50-3.79 (m, 5H), 4.32 (d, *J* = 4.8 Hz, 2H), 4.36 (s, 2H), 6.66 (s, 1H), 7.07 (s, 1H), 7.24 (dd, *J* = 1.5, 8.5 Hz, 1H), 7.37 (d, *J* = 1.5 Hz, 1H), 7.53 (d, *J* = 8.5 Hz, 1H), 7.73 (s, 1H), 8.67 (s, 1H), 9.48 (br s, 1H), 14.42 (s, 1H).

### Example 83:

### 5-(4-Hydroxy-2-methylphenyl)-7-[1H-5-(piperidinomethyl)indol-2-y l]isoindolinone (Compound 550)

### Step 1

5-Bromo-7-[1-(tert-butoxycarbonyl)-5-(piperidinomethyl)indo 1-2-yl] isoindolinone (60.0 mg, 0.114 mmol) was dissolved in a mixed solvent of DMA (1.8 mL)/water (82 µL), and the solution was added with 4-hydroxy-2-methylphenylboronic acid (26.0 mg, 0.171 mmol), triethylamine (79.4 µL, 0.570 mmol) and [bis(diphenylphosphino)ferrocene]dichloropalladium (9.34 mg, 0.0114 mmol), followed by stirring at 100 °C for 4.5 hours. The reaction mixture was added with water and extracted with ethyl acetate The organic layer was condensed under reduced pressure and the residue was purified by silica gel column chromatography (chloroform/methanol=99/1-80/20) to obtain crude 5-(4-hydroxy-2-methylphenyl)-7-[1-(tert-butoxycarbonyl)-5-(piper idinomethyl)indol-2-yl]isoindolinone.
ESI-MS *m*/*z*: 552 [M+H]⁺

### Step 2

In a similar manner to Step 8 of Example 1, Compound 550 (12.2 mg, yield 24%, 2 steps) was obtained using crude 5-(4-hydroxy-2-methylphenyl)-7-[1-(tert-butoxycarbonyl)-5-(piper idinomethyl)indol-2-yl]isoindolinone, 10% hydrogen chloride-methanol solution (1.0 mL) and methanol (1.0 mL).
ESI-MS *m*/*z:* 452 [M+H]⁺; ¹H-NMR (DMSO-*d₆*)δ(ppm) : 1.32-1.56 (m, 6H), 2.26 (s, 3H), 2.28-2.41 (m, 4H), 3.47 (s, 2H), 4.53 (s, 2H), 6.72 (d, *J* = 7.5 Hz, 1H), 6.76 (s, 1H), 7.07 (d, *J* = 7.5 Hz, 1H), 7.15 (d, *J* = 8.2 Hz, 1H), 7.23 (s, 1H), 7.36-7.66 (m, 3H), 8.00 (s, 1H), 9.24 (s, 1H), 9.52 (s, 1H), 13.88 (s, 1H).

### Example 84:

### 5-(2-Methoxyphenyl)-7-[1H-5-(piperidinomethyl)indol-2-yl]isoindo linone (Compound 551)

### Step 1

In a similar manner to Step 1 of Example 83, crude 5-(2-methoxyphenyl)-7-[1-(tert-butoxycarbonyl)-5-(piperidinometh yl)indol-2-yl]isoindolinone was obtained using 5-bromo-7-[1-(tert-butoxycarbonyl)-5-(piperidinomethyl)indol-2-y l]isoindolinone (62.8 mg, 0.120 mmol), 2-methoxyphenylboronic acid (27.4 mg, 0.180 mmol), triethylamine (83.6 µL, 0.600 mmol), [bis(diphenylphosphino)ferrocene]dichloropalladium (9.78 mg, 0.0120 mmol) and DMA (1.9 mL)/water (86 µL).
ESI-MS *m*/*z:* 552 [M+H]⁺

### Step 2

In a similar manner to Step 8 of Example 1, Compound 551 (10.9 mg, yield 20%, 2 steps) was obtained using crude 5-(2-methoxyphenyl)-7-[1-(tert-butoxycarbonyl)-5-(piperidinometh yl)indol-2-yl]isoindolinone, 10% hydrogen chloride-methanol solution (1.5 mL) and methanol (1.5 mL).
ESI-MS *m*/*z*: 452 [M+H]⁺; ¹H-NMR (DMSO-*d*₆)δ(ppm) : 1.33-1.57 (m, 6H), 2.29-2.41 (m, 4H), 3.47 (s, 2H), 3.82 (s, 3H), 4.54 (s, 2H), 7.06-7.13 (m, 2H), 7.18 (s, 1H), 7.20 (d, *J* = 7.5 Hz, 1H), 7.40 (d, *J* = 8.7 Hz, 1H), 7.40-7.47 (m, 3H), 7.56 (s, 1H), 8.18 (s, 1H), 9.26 (s, 1H), 13.84 (s, 1H).

### Example 85:

### 5-(Dimethylaminomethyl)-7-[1H-5-(piperidino)indol-2-yl]isoindoli none dihydrochloride (Compound 552)

### Step 1

In a similar manner to Step 7 of Example 1, 5-(dimethylaminomethyl)-7-iodoisoindolinone (50.0 mg, 0.158 mmol) was dissolved in acetonitrile (3 mL), and the solution was treated with Compound CF (109 mg, 0.316 mmol), palladium acetate (2.8 mg, 0.013 mmol), tri(o-tolyl)phosphine (7.7 mg, 0.025 mmol) and triethylamine (0.220 mL, 1.58 mmol), followed by purification by preparative thin-layer chromatography (chloroform/methanol=9/1) to obtain 5-(dimethylaminomethyl)-7-[1-(tert-butoxycarbonyl)-5-(piperidino )indol-2-yl]isoindolinone (72.4 mg, yield 94%).
ESI-MS *m*/*z*: 489 [M+H]⁺; ¹H-NMR (CDCl₃)δ(ppm) : 1.28 (s, 9H), 1.50-1.65 (m, 2H), 1.70-1.85 (m, 4H), 2.29 (s, 6H), 3.06-3.18 (m, 4H), 3.55 (s, 2H), 4.41 (s, 2H), 6.49 (s, 1H), 6.54 (br s, 1H), 7.02 (m, 1H), 7.07 (s, 1H), 7.38 (s, 1H), 7.43 (s, 1H), 8.10 (d, *J* = 8.9 Hz, 1H).

### Step 2

In a similar manner to Step 8 of Example 1, 5-(dimethylaminomethyl)-7-[1-(tert-butoxycarbonyl)-5-(piperidino )indol-2-yl]isoindolinone (69.6 mg, 0.142 mmol) was dissolved in methanol (2 mL), and the solution was treated with 10% hydrogen chloride-methanol solution (2 mL), followed by adding with diisopropylether. The obtained solid was collected by filtration and washed with diisopropylether, followed by drying under reduced pressure to obtain Compound 552 (13.3 mg, yield 20%).
ESI-MS *m*/*z*: 389 [M+H]⁺; ¹H-NMR (DMSO-*d*₆)δ(ppm) : 2.15-2.60 (m, 4H), 2.60-2.89 (m, 2H), 3.32 (s, 3H), 3.34 (s, 3H), 3.95-4.35 (m, 4H), 5.02 (d, *J* = 4.8 Hz, 2H), 5.13 (s, 2H), 8.06 (s, 1H), 8.10-8.34 (m, 3H), 8.71 (br s, 1H), 9.19 (s, 1H), 10.06 (s, 1H), 11.79 (br s, 1H), 12.66 (br s, 1H), 14.78 (s, 1H).

### Example 86:

### 5-(1-Hydroxy-2-propyloxy)-7-[1H-5-(piperidinomethyl)indol-2-yl]i soindolinone (Compound 553)

### Step 1

In a similar manner to Step 1 of Example 3, 5-hydroxy-7-iodoisoindolinone (500 mg, 0.182 mmol) was dissolved in DMF (8 mL), and the solution was treated with potassium carbonate (503 mg, 3.64 mmol) and methyl 2-bromopropionate (0.324 mL, 2.91 mmol), followed by purification by slurry using hexane to obtain 5-(1-carbomethoxyethyl)-7-iodoisoindolinone (507 mg, yield 77%).
ESI-MS *m*/*z*: 361 [M+H]⁺; ¹H-NMR (CDCl₃)δ(ppm) : 1.52 (d, *J* = 6.8 Hz, 3H), 3.69 (s, 3H), 4.21 (s, 2H), 5.16 (q, *J* = 6.8 Hz, 1H), 7.11 (d, *J* = 2.0 Hz, 1H), 7.40 (d, *J* = 2.0 Hz, 1H), 8.52 (br s, 1H).

### Step 2

5-(1-Carbomethoxyethyl)-7-iodoisoindolinone (200 mg, 0.554 mmol) was dissolved in THF (4 mL), and the solution was added with water (1 mL) and sodium borohydride (84.0 mg, 2.22 mmol), followed by stirring at room temperature for 3 hours. The reaction mixture was added with water, and extracted with ethyl acetate. The organic layer was washed with saturated brine and dried over anhydrous sodium sulfate. The solvent was evaporated under reduced pressure, and the residue was purified by slurry using chloroform to obtain 5-(1-hydroxy-2-propyloxy)-7-iodoisoindolinone (93.3 mg, yield 51%).
ESI-MS *m*/*z*: 334 [M+H]⁺; ¹H-NMR (DMSO-*d*₆)δ(ppm) : 1.21 (d, *J* = 6.1 Hz, 3H), 3.44-3.58 (m, 2H), 4.20 (s, 2H), 4.53 (tq, *J* = 5.5, 6.1 Hz, 1H), 4.90 (t, *J* = 5.7 Hz, 1H), 7.18 (d, *J* = 2.1 Hz, 1H), 7.40 (d, *J* = 2. 1 Hz, 1H), 8.47 (br s, 1H).

### Step 3

In a similar manner to Step 7 of Example 1, 5-(1-hydroxy-2-propyloxy)-7- iodoisoindolinone (60.0 mg, 0.180 mmol) was dissolved in acetonitrile (4 mL), and the solution was treated with Compound BD (129 mg, 0.360 mmol), palladium acetate (3.2 mg, 0.014 mmol), tri(o-tolyl)phosphine (8.8 mg, 0.029 mmol) and triethylamine (0.250 mL, 1.79 mmol), followed by purification by preparative thin-layer chromatography (chloroform/methanol/7 mol/L ammonia-methanol=15/1/1) to obtain 5-(1-hydroxy-2-propyloxy)-7-[1-(tert-butoxycarbonyl)-5-(piperidi nomethyl)indol-2-yl]isoindolinone (91.8 mg, yield 98%).
ESI-MS *m*/*z*: 520 [M+H]⁺; ¹H-NMR (CDCl₃)δ(ppm) : 1.34 (d, *J* = 6.4 Hz, 3H), 1.36 (s, 9H), 1.38-1.48 (m, 2H), 1.50-1.65 (m, 4H), 2.36-2.45 (m, 4H), 3.56 (s, 2H), 3.75-3.83 (m, 2H), 4.38 (s, 2H), 4.62 (m, 1H), 6.14 (br s, 1H), 6.55 (s, 1H), 6.96 (d, *J* = 2.1 Hz, 1H), 7.03 (d, *J* = 2.1 Hz, 1H), 7.28 (m, 1H), 7.48 (s, 1H), 8.15 (d, *J* = 8.6 Hz, 1H).

### Step 4

In a similar manner to Step 8 of Example 1, 5-(1-hydroxy-2-propyloxy)-7-[1-(tert-butoxycarbonyl)-5-(piperidi nomethyl)indol-2-yl]isoindolinone (90.0 mg, 0.173 mmol) was dissolved in methanol (2 mL), and the solution was treated with 10% hydrogen chloride-methanol solution (2 mL) , followed by purification by preparative thin-layer chromatography (chloroform/methanol/7 mol/L ammonia-methanol=15/1/1) to obtain Compound 553 (44.2 mg, yield 61%).
ESI-MS *m*/*z:* 420 [M+H]⁺; ¹H-NMR (DMSO-*d*₆)δ(ppm) : 1.28 (d, *J* = 6.0 Hz, 3H), 1.33-1.44 (m, 2H), 1.45-1.55 (m, 4H), 2.29-2.38 (m, 4H), 3.47 (s, 2H), 3.50-3.66 (m, 2H), 4.44 (s, 2H), 4.67 (m, 1H), 4.94 (t, *J* = 5.9 Hz, 1H), 7.07 (dd, *J* = 1.4, 8.3 Hz, 1H), 7.10 (d, *J* = 3.0 Hz, 1H), 7.20 (d, *J* = 1.4 Hz, 1H), 7.37 (d, *J* = 8.3 Hz, 1H), 7.43 (s, 1H), 7.60 (d, *J* = 2.0 Hz, 1H), 9.01 (s, 1H), 13.96 (s, 1H).

### Example 87:

### 5-(Dimethylaminomethyl)-7-{1H-5-[(3-pentylamino)methyl]indol-2-y l}isoindolinone dihydrochloride (Compound 554)

### Step 1

In a similar manner to Step 2 of Example 45, 5-(dimethylaminomethyl)-7-[1-(tert-butoxycarbonyl)-5-formylindol -2-yl]isoindolinone (50.0 mg, 0.115 mmol) was dissolved in acetonitrile (3 mL) , and the solution was treated with 3-pentylamine (0.134 mL, 1.15 mmol), acetic acid (0.132 mL, 2.30 mmol) and sodium triacetoxyborohydride (73.1mg, 0.345 mmol), followed by purification by preparative thin-layer chromatography (chloroform/methanol=6/1) to obtain 5-(dimethylaminomethyl)-7-{1*H*-5-[(3-pentylamino)methyl]indol-2-y l}isoindolinone (44.6 mg, yield 77%).
ESI-MS *m*/*z*: 505 [M+H]⁺; ¹H-NMR (CDCl₃)δ(ppm) : 0.88 (t, *J* = 7.3 Hz, 6H), 1.29 (s, 9H), 1.40-1.60 (m, 4H), 2.28 (s, 6H), 2.46 (m, 1H), 3.54 (s, 2H), 3.86 (s, 2H), 4.41 (s, 2H), 6.54 (s, 1H), 6.65 (br s, 1H), 7.27 (m, 1H), 7.39 (s, 1H), 7.44 (s, 1H), 7.47 (s, 1H), 8.19 (d, *J* = 8.8 Hz, 1H).

### Step 2

In a similar manner to Step 8 of Example 1, 5-(dimethylaminomethyl)-7-{1*H*-5-[(3-pentylamino)methyl]indol-2-y l}isoindolinone (42.0 mg, 0.0832 mmol) was dissolved in methanol (1.5 mL) , and the solution was treated with 10% hydrogen chloride-methanol solution (1.5 mL), followed by adding with diisopropylether. The obtained solid was collected by filtration and dried under reduced pressure to obtain Compound 554 (22.7 mg, yield 57%).
ESI-MS *m*/*z*: 405 [M+H]⁺; ¹H-NMR (DMSO-*d*₆)δ(ppm) : 0.89 (t, *J* = 7.4 Hz, 6H), 1.56-1.83 (m, 4H), 2.75 (d, *J* = 4.3 Hz, 6H), 2.92 (m, 1H), 4.21 (br s, 2H), 4.43 (d, *J* = 5.1 Hz, 2H), 4.54 (s, 2H), 7.34 (dd, *J* = 1.4, 8.5 Hz, 1H), 7.42 (s, 1H), 7.56 (d, *J* = 8.5 Hz, 1H), 7.62 (s, 1H), 7.82 (s, 1H), 8.61 (s, 1H), 8.85 (br s, 2H), 9.45 (s, 1H), 11.28 (br s, 1H), 14.00 (s, 1H).

### Example 88:

### 5-(1-Hydroxy-2-methylpropyl)-7-[1H-5-(diethylaminomethyl)indol-2 -yl]isoindolinone hydrochloride (Compound 555)

### Step 1

In a similar manner to Step 7 of Example 1, 5-(1-hydroxy-2-methylpropyl)-7-iodoisoindolinone (60.0 mg, 0.181 mmol) was dissolved in acetonitrile (4 mL), and the solution was treated with Compound CE (125 mg, 0.361 mmol), palladium acetate (3.3 mg, 0.015 mmol), tri(o-tolyl)phosphine (8.8 mg, 0.029 mmol) and triethylamine (0.252 mL, 1.81 mmol), followed by purification by preparative thin-layer chromatography (chloroform/methanol=10/1) to obtain 5-(1-hydroxy-2-methylpropyl)-7-[1-(tert-butoxycarbonyl)-5-(dieth ylaminomethyl)indol-2-yl]isoindolinone (59.8 mg, yield 65%).
ESI-MS *m*/*z*: 506 [M+H]⁺; ¹H-NMR (CDCl₃)δ(ppm) : 0.91 (d, *J* = 6.8 Hz, 3H), 1.01 (d, *J* = 6.8 Hz, 3H), 1.11 (t, *J* = 7.1 Hz, 6H), 1.31 (s, 9H), 2.03 (m, 1H), 2.61 (d, *J* = 7.1 Hz, 4H), 3.73 (br s, 2H), 4.44 (s, 2H), 4.56 (d, *J* = 6.6 Hz, 1H), 6.30 (br s, 1H), 6.54 (s, 1H), 7.25 (s, 1H), 7.31 (dd, *J* = 1.5, 8.5 Hz, 1H), 7.39 (d, *J* = 0.8 Hz, 1H), 7.46 (s, 1H), 7.53 (d, *J* = 0.8 Hz, 1H), 8.18 (d, *J* = 8.5 Hz, 1H).

### Step 2

In a similar manner to Step 8 of Example 1, 5-(1-hydroxy-2-methylpropyl)-7-[1-(tert-butoxycarbonyl)-5-(dieth ylaminomethyl)indol-2-yl]isoindolinone (56.0 mg, 0.111 mmol) was dissolved in methanol (2 mL), and the solution was treated with 10% hydrogen chloride-methanol solution (2 mL) , followed by purification by preparative thin-layer chromatography (chloroform/methanol/7 mol/L ammonia-methanol solution=25/1/1). The residue was dissolved in 10% hydrogen chloride-methanol solution, followed by condensing under reduced pressure, and purification by slurry using ethyl acetate to obtain Compound 555 (28.7 mg, yield 54%).
ESI-MS *m*/*z*: 406 [M+H]⁺; ¹H-NMR (DMSO-*d*₆)δ(ppm) : 0.84 (d, *J* = 6.8 Hz, 3H), 0.89 (d, *J* = 6.6 Hz, 3H), 1.26 (t, *J* = 7.1 Hz, 6H), 1.93 (m, 1H), 3.08 (dq, *J* = 4.6, 7.1 Hz, 4H), 4.36 (d, *J* = 5.1 Hz, 2H), 4.46 (t, *J* = 5.1 Hz, 1H), 4.51 (s, 2H), 5.39 (d, *J* = 4.6 Hz, 1H), 7.29 (dd, *J* = 1.7, 8.6 Hz, 1H), 7.30 (s, 1H), 7.46 (s, 1H), 7.57 (d, *J* = 8.6 Hz, 1H), 7.77 (s, 1H), 8.09 (s, 1H), 9.26 (s, 1H), 9.56 (br s, 1H), 14.15 (s, 1H).

### Example 89:

### 5-(Dimethylaminomethyl)-7-[1H-5-(diethylamino)indol-2-yl]isoindo linone dihydrochloride (Compound 556)

### Step 1

In a similar manner to Step 7 of Example 1, 5-(dimethylaminomethyl)-7-iodoisoindolinone (50.0 mg, 0.158 mmol) was dissolved in acetonitrile (3 mL), and the solution was treated with Compound CH (105 mg, 0.316 mmol), palladium acetate (2.8 mg, 0.013 mmol), tri(o-tolyl)phosphine (7.7 mg, 0.025 mmol) and triethylamine (0.220 mL, 1.58 mmol), followed by purification by preparative thin-layer chromatography (chloroform/methanol=9/1) to obtain 5-(dimethylaminomethyl)-7-[1-(tert-butoxycarbonyl)-5-(diethylami no)indol-2-yl]isoindolinone (55.4 mg, yield 74%).
ESI-MS *m*/*z*: 477 [M+H]⁺; ¹H-NMR (CDCl₃)δ(ppm) : 1.14 (t, *J* = 7.0 Hz, 6H), 1.23 (s, 9H), 2.29 (s, 6H), 3.35 (q, *J* = 7.0 Hz, 4H), 3.55 (s, 2H), 4.41 (s, 2H), 6.40-6.52 (m, 2H), 6.82-6.95 (m, 2H), 7.38 (s, 1H), 7.43 (s, 1H), 8.07 (d, *J* = 9.9 Hz, 1H).

### Step 2

In a similar manner to Step 8 of Example 1, 5-(dimethylaminomethyl)-7-[1-(tert-butoxycarbonyl)-5-(diethylami no)indol-2-yl]isoindolinone (53.0 mg, 0.111 mmol) was dissolved in methanol (2 mL), and the solution was treated with 10% hydrogen chloride-methanol solution (2 mL), followed by adding with diisopropylether. The obtained solid was collected by filtration and washed with diisopropylether, followed by drying under reduced pressure to obtain Compound 556 (28.6 mg, yield 57%).
ESI-MS *m*/*z*: 377 [M+H]⁺; ¹H-NMR (DMSO-*d*₆)δ(ppm) : 1.24 (t, *J* = 7.0 Hz, 6H), 2.96 (s, 6H), 3.61-3.90 (m, 4H), 4.66 (br s, 2H), 4.76 (s, 2H), 7.60-7.76 (m, 2H), 7.86 (s, 1H), 7.93 (d, *J* = 8.9 Hz, 1H), 8.23 (s, 1H), 8.84 (s, 1H), 9.69 (s, 1H), 11.50 (br s, 1H), 12.40 (br s, 1H), 14.40 (s, 1H).

### Example 90:

### 5-(Dimethylaminomethyl)-7-[1H-5-(cyclohexylamino)indol-2-yl]isoi ndolinone hydrochloride (Compound 557)

### Step 1

In a similar manner to Step 7 of Example 1, 5-(dimethylaminomethyl)-7-iodoisoindolinone (50.0 mg, 0.158 mmol) was dissolved in acetonitrile (3 mL), and the solution was treated with Compound CG (144 mg, 0.314 mmol), palladium acetate (2.8 mg, 0.013 mmol), tri(o-tolyl)phosphine (7.7 mg, 0.025 mmol) and triethylamine (0.220 mL, 1.58 mmol), followed by purification by preparative thin-layer chromatography (chloroform/methanol=9/1) to obtain 5-(dimethylaminomethyl)-7-[1-(tert-butoxycarbonyl)-5-(cyclohexyl amino)indol-2-yl]isoindolinone (62.6 mg, yield 66%).
ESI-MS *m*/*z:* 603 [M+H]⁺; ¹H-NMR (CDCl₃)δ(ppm) : 1.04-1.66 (m, 6H) , 1.31 (s, 9H), 1.36 (s, 9H), 1.65-1.78 (m ,2H), 1.83-1.95 (m, 2H), 2.30 (s, 6H), 3.56 (s, 2H), 4.15 (br s, 1H), 4.44 (s, 2H), 6.41 (br s, 1H), 6.57 (s, 1H), 7.00 (d, *J* = 8.9 Hz, 1H), 7.21 (s, 1H), 7.41 (s, 1H), 7.46 (s, 1H), 8.17 (d, *J* = 8.9 Hz, 1H).

### Step 2

In a similar manner to Step 8 of Example 1, 5-(dimethylaminomethyl)-7-[1-(tert-butoxycarbonyl)-5-(cyclohexyl amino) indol-2-yl] isoindolinone (60.0 mg, 0.0995 mmol) was dissolved in methanol (2 mL), and the solution was treated with 10% hydrogen chloride-methanol solution (2 mL), followed by condensing under reduced pressure. The residue was added with methanol and ethyl acetate. The obtained solid was collected by filtration and washed with ethyl acetate, followed by drying under reduced pressure to obtain Compound 557 (42.0 mg, yield 89%).
ESI-MS *m*/*z*: 377 [M+H]⁺; ¹H-NMR (DMSO-*d*₆)δ(ppm) : 0.95-1.69 (m, 6H), 1.65-1.80 (m, 2H), 1.87-1.98 (m, 2H), 2.76 (s, 3H), 2.77 (s, 3H), 3.40 (m, 1H), 4.45 (br s, 2H), 4.56 (s, 2H), 7.26 (d, *J* = 7.8 Hz, 1H), 7.45 (s, 1H), 7.66 (s, 1H), 7.67 (d, *J* = 7.8 Hz, 1H), 7.75 (s, 1H), 8.59 (s, 1H), 9.49 (s, 1H), 10.87 (br s, 2H), 11.15 (br s, 1H), 14.16 (s, 1H).

### Example 91:

### 5-(2-Methoxypyridin-3-yl)-7-[1H-5-(piperidinomethyl)indol-2-yl]i soindolinone (Compound 558)

### Step 1

In a similar manner to Step 1 of Example 43, crude 5-(2-fluoropyridin-3-yl)-7-[1-(tert-butoxycarbonyl)-5-(piperidin omethyl)indol-2-yl]isoindolinone was obtained using 5-chloro-7-[1-(tert-butoxycarbonyl)-5-(piperidinomethyl)indol-2-yl]isoindolinone (50.0 mg, 0.104 mmol), 2-fluoro-3-pyridineboronic acid (29.3 mg, 0.208 mmol), cesium fluoride (55.3 mg, 0.364 mmol), 2-dicyclohexylphosphino-2',4',6'-triisopropylbiphenyl (14.9 mg, 0.0312 mmol), tris(dibenzylideneacetone)dipalladium(0) (9.52 mg, 0.0104 mmol) and 1,4-dioxane (2.0 mL).
ESI-MS *m*/*z*: 541 [M+H]⁺

### Step 2

In a similar manner to Step 8 of Example 1, Compound 558 (1.00 mg, yield 2%, 2 steps) was obtained using crude 5-(2-fluoropyridin-3-yl)-7-[1-(tert-butoxycarbonyl)-5-(piperidin omethyl)indol-2-yl]isoindolinone, 10% hydrogen chloride-methanol solution (1.0 mL) and methanol (1.0 mL). ESI-MS *m*/*z*: 453 [M+H]⁺; ¹H-NMR (DMSO-*d*₆)δ(ppm) : 1.31-1.64 (m, 6H), 2.27-2.45 (m, 4H), 3.51 (s, 2H), 3.93 (s, 3H), 4.55 (s, 2H), 7.08-7.29 (m, 2H), 7.39-7.59 (m, 1H), 7.67 (s, 1H), 7.93 (d, *J* = 8.6 Hz, 1H), 8.02-8.20 (m, 2H), 8.27 (d, *J* = 8.6 Hz, 1H), 8.29 (s, 1H), 9.31 (s, 1H), 13.31 (s, 1H).

### Example 92:

### 5-(Dimethylaminomethyl)-7-(1H-5-{[N-(ethyl)propylamino]methyl}in dol-2-yl)isoindolinone dihydrochloride (Compound 559)

### Step 1

In a similar manner to Step 2 of Example 45, 5-(dimethylaminomethyl)-7-[1-(tert-butoxycarbonyl)-5-formylindol -2-yl]isoindolinone (50.0 mg, 0.115 mmol) was dissolved in acetonitrile (3 mL), and the solution was treated with N-ethylpropylamine (0.139 mL, 1.15 mmol), acetic acid (0.132 mL, 2.30 mmol) and sodium triacetoxyborohydride (195 mg, 0.920 mmol) , followed by purification by preparative thin-layer chromatography (chloroform/methanol=6/1) to obtain 5-(dimethylaminomethyl)-7-(1*H*-5-{[N-(ethyl)propylamino]methyl}in dol-2-yl)isoindolinone (34.6 mg, yield 60%).
ESI-MS *m*/*z*: 505 [M+H]⁺; ¹H-NMR (CDCl₃)δ(ppm) : 0.89 (t, *J* = 7.3 Hz, 3H), 1.05-1.15 (m, 3H), 1.30 (s, 9H), 1.55-1.65 (m, 2H), 2.30 (s, 6H), 2.43-2.58 (m, 4H), 3.55 (s, 2H), 3.74 (br s, 2H), 4.42 (s, 2H) , 6.46 (s, 1H), 6.57 (s, 1H), 7.31 (dd, *J* = 1.3, 8.6 Hz, 1H), 7.40 (s, 1H), 7.45 (s, 1H), 7.53 (br s, 1H), 8.19 (d, *J* = 8.6 Hz, 1H).

### Step 2

In a similar manner to Step 8 of Example 1, 5-(dimethylaminomethyl)-7-(1H-5-{[N-(ethyl)propylamino]methyl}in dol-2-yl)isoindolinone (32.0 mg, 0.0634 mmol) was dissolved in methanol (1 mL), and the solution was treated with 10% hydrogen chloride-methanol solution (1 mL), followed by condensing under reduced pressure. The residue was added with methanol and ethyl acetate. The obtained solid was collected by filtration and washed with ethyl acetate, followed by drying under reduced pressure to obtain Compound 559 (11.4 mg, yield 38%).
ESI-MS *m*/*z*: 405 [M+H]⁺; ¹H-NMR (DMSO-*d*₆)δ(ppm) : 0.88 (t, *J* = 7.3 Hz, 3H), 1.29 (t, *J* = 7.3 Hz, 3H), 1.65-1.77 (m, 2H), 2.77 (d, *J* = 4.6 Hz, 6H), 2.89-3.00 (m, 2H), 3.03-3.10 (m, 2H), 4.37 (d, *J* = 5.0 Hz, 2H), 4.46 (d, *J* = 5.3 Hz, 2H), 4.57 (s, 2H), 7.36 (d, *J* = 8.4 Hz, 1H), 7.46 (s, 1H), 7.60 (d, *J* = 8.4 Hz, 1H), 7.65 (s, 1H), 7.86 (s, 1H), 8.64 (s, 1H), 9.47 (s, 1H), 10.08 (br s, 1H), 11.34 (br s, 1H), 14.04 (s, 1H).

### Example 93:

### 5-[1-(Dimethylamino)ethyl]-7-[1H-5-(piperidinomethyl)indol-2-yl] isoindolinone dihydrochloride (Compound 560)

### Step 1

5-Acetyl-7-iodoisoindolinone (274 mg, 0.910 mmol) was dissolved in acetonitrile (15 mL), and the solution was added with 2.0 mol/L dimethylamine-THF solution (9.1 mL, 18 mmol), acetic acid (1.04 mL, 18.2 mmol) and molecular sieves 4A (2.74 g), followed by stirring at room temperature for 30 minutes. The mixture was added with sodium triacetoxyborohydride (579 mg, 2.73 mmol), followed by stirring at room temperature for 17 hours. The reaction mixture was added with acetic acid (1.04 mL, 18.2 mmol) and sodium triacetoxyborohydride (579 mg, 2.73 mmol), followed by stirring at room temperature for 18 hours. The reaction mixture was added with acetic acid (1.04 mL, 18.2 mmol) and sodium triacetoxyborohydride (579 mg, 2.73 mmol), followed by stirring at room temperature for 17 hours. The reaction mixture was added with water and sodium carbonate, and extracted with ethyl acetate. The organic layer was washed with saturated brine and dried over anhydrous sodium sulfate. The solvent was evaporated under reduced pressure. The residue was purified by preparative thin-layer chromatography (chloroform/methanol=9/1) to obtain 5-[1-(dimethylamino)ethyl]-7-iodoisoindolinone (85.6 mg, yield 29%).
ESI-MS *m*/*z:* 331 [M+H]⁺; ¹H-NMR (DMSO-*d*₆)δ(ppm) : 1.22 (d, *J* = 6.8 Hz, 3H), 2.06 (s, 6H), 3.29 (m, 1H), 4.30 (s, 2H), 7.46 (s, 1H), 7.77 (s, 1H), 6.61 (s, 1H).

### Step 2

In a similar manner to Step 7 of Example 1, 5-[1-(dimethylamino)ethyl)]-7-iodoisoindolinone (39.6 mg, 0.120 mmol) was dissolved in acetonitrile (2.5 mL), and the solution was treated with Compound BD (86.0 mg, 0.240 mmol), palladium acetate (2.2 mg, 0.0098 mmol), tri(o-tolyl)phosphine (5.8 mg, 0.019 mmol) and triethylamine (0.167 mL, 1.20 mmol), followed by purification by preparative thin-layer chromatography (chloroform/methanol=6/1) to obtain 5-[1-(dimethylamino)ethyl)]-7-[1-(tert-butoxycarbonyl)-5-(piperi dinomethyl)indol-2-yl]isoindolinone (55.3 mg, yield 89%).
ESI-MS *m*/*z*: 517 [M+H]⁺; ¹H-NMR (CDCl₃)δ(ppm) : 1.27 (s, 9H) , 1.42 (d, *J* = 6.6 Hz, 3H), 1.48-1.80 (m, 6H), 2.25 (s, 6H), 2.30-2.50 (m, 4H), 3.37 (q, *J* = 6.6 Hz, 1H), 3.59 (s, 2H), 4.42 (s, 2H), 6.55 (s, 1H), 6.73 (br s, 1H), 7.29 (dd, *J* = 1.7, 8.6 Hz, 1H), 7.39 (s, 1H), 7.43 (s, 1H), 7.48 (s, 1H), 8.19 (d, *J* = 8.6 Hz, 1H).

### Step 3

In a similar manner to Step 8 of Example 1, 5-[1-(dimethylamino)ethyl)]-7-[1-(tert-butoxycarbonyl)-5-(piperi dinomethyl)indol-2-yl]isoindolinone (52.5 mg, 0.102 mmol) was dissolved in methanol (2 mL), and the solution was treated with 10% hydrogen chloride-methanol solution (2 mL), followed by condensing under reduced pressure. The residue was added with methanol and ethyl acetate. The obtained solid was collected by filtration and washed with ethyl acetate, followed by drying under reduced pressure to obtain Compound 560 (41.9 mg, yield 84%).
ESI-MS *m*/*z*: 417 [M+H]⁺; ¹H-NMR (DMSO-*d*_{d})δ(ppm) : 1.34 (m, 1H), 1.57-1.90 (m, 5H), 1.74 (d, *J* = 6.8 Hz, 3H), 2.59 (d, *J* = 4.6 Hz, 3H), 2.75-2.86 (m, 2H), 2.82 (d, *J* = 4.6 Hz, 3H), 3.30-3.40 (m, 2H), 4.31 (d, *J* = 4.8 Hz, 2H), 4.54 (s, 2H), 4.62 (m, 1H), 7.30 (d, *J* = 8.4 Hz, 1H), 7.51 (s, 1H), 7.56 (d, *J* = 8.4 Hz, 1H), 7.66 (s, 1H), 7.80 (s, 1H), 8.61 (s, 1H), 9.44 (s, 1H), 9.97 (br s, 1H), 11.40 (br s, 1H), 14.02 (s, 1H).

### Example 94:

### 5-[1-(Dimethylamino)ethyl]-7-[1H-5-(diethylaminomethyl)indol-2-y l]isoindolinone dihydrochloride (Compound 561)

### Step 1

In a similar manner to Step 7 of Example 1, 5-[1-(dimethylamino)ethyl]-7-iodoisoindolinone (44.2 mg, 0.134 mmol) was dissolved in acetonitrile (2.5 mL), and the solution was treated with Compound CE (92.7 mg, 0.268 mmol), palladium acetate (2.4 mg, 0.0011 mmol), tri(o-tolyl)phosphine (6.5 mg, 0.021 mmol) and triethylamine (0.187 mL, 1.34 mmol), followed by purification by preparative thin-layer chromatography (chloroform/methanol=6/1) to obtain 5-[1-(dimethylamino)ethyl]-7-[1-(tert-butoxycarbonyl)-5-(diethyl aminomethyl)indol-2-yl]isoindolinone (52.7 mg, yield 78%).
ESI-MS *m*/*z:* 505 [M+H]⁺; ¹H-NMR (CDCl₃)δ(ppm) : 1.07 (t, *J* = 7.0 Hz, 6H), 1.27 (s, 9H), 1.42 (d, *J* = 6.6 Hz, 3H), 2.25 (s, 6H), 2.56 (q, *J* = 7.0 Hz, 4H), 3.37 (q, *J* = 6.6 Hz, 1H), 3.68 (s, 2H), 4.41 (s, 2H), 6.55 (s, 1H), 6.72 (br s, 1H), 7.30 (dd, *J* = 1.7, 8.5 Hz, 1H), 7.39 (s, 1H), 7.42 (s, 1H), 7.49 (s, 1H), 8.19 (d, *J* = 8.5 Hz, 1H).

### Step 2

In a similar manner to Step 8 of Example 1, 5-[1-(dimethylamino)ethyl]-7-[1-(tert-butoxycarbonyl)-5-(diethyl aminomethyl)indol-2-yl]isoindolinone (50.0 mg, 0.0991 mmol) was dissolved in methanol (2 mL), and the solution was treated with 10% hydrogen chloride-methanol solution (2 mL), followed by condensing under reduced pressure. The residue was added with methanol and ethyl acetate. The obtained solid was collected by filtration and washed with ethyl acetate, followed by drying under reduced pressure to obtain Compound 561 (37.9 mg, yield 80%).
ESI-MS *m*/*z*: 405 [M+H]⁺; ¹H-NMR (DMSO-*d*₆)δ(ppm) : 1.25 (t, *J* = 7.0 Hz, 6H), 1.73 (d, *J* = 6.8 Hz, 3H), 2.59 (d, *J* = 4.7 Hz, 3H), 2.81 (d, *J* = 4.7 Hz, 3H), 2.90-3.20 (m, 4H), 4.34 (d, *J* = 4.9 Hz, 2H), 4.54 (s, 2H), 4.62 (m, 1H), 7.34 (dd, *J* = 1.5, 8.4 Hz, 1H), 7.52 (s, 1H), 7.57 (d, *J* = 8.4 Hz, 1H), 7.67 (s, 1H), 7.83 (s, 1H), 8.63 (s, 1H), 9.45 (s, 1H), 9.99 (br s, 1H), 11.47 (br s, 1H), 14.03 (s, 1H).

### Example 95:

### 4-Hydroxy-5-(dimethylaminomethyl)-7-[1H-5-(piperidinomethyl)indo l-2-yl]isoindolinone dihydrochloride (Compound 562)

### Step 1

4-Formyl-3-hydroxybenzoic acid (2.20 g, 13.2 mmol) was suspended in dichloromethane (40 mL), and the suspension was added with diisopropylethylamine (9.20 mL, 52.8 mmol) and chloromethyl methyl ether (2.01 mL, 26.5 mmol) under ice-cooling. The reaction mixture was heated to room temperature and stirred for 1 hour. The solvent was evaporated under reduced pressure. The residue was dissoved in methanol (40 mL), and the solution was added with 2 mol/L aqueous potassium hydroxide solution (40 mL), followed by stirring at room temperature for 1 hour. Methanol was evaporated under reduced pressure, and the residue was added with 4 mol/L hydrochloric acid and water. The precipitated solid was collected by filtration, and the solid was washed with water, followed by drying under reduced pressure to obtain 4-formyl-3-methoxymethoxybenzoic acid (2.21 g, yield 80%).
ESI-MS *m*/*z*: 211 [M+H]⁺; ¹H-NMR (DMSO-*d*₆)δ(ppm) : 3.46 (s, 3H) , 5.47 (s, 2H), 7.67 (dd, *J* = 1.3, 7.9 Hz, 1H), 7.807 (d, *J* = 1.3 Hz, 1H), 7.812 (d, *J* = 7.9 Hz, 1H), 10.44 (s, 1H), 13.47 (s, 1H).

### Step 2

4-Formyl-3-methoxymethoxybenzoic acid (2.57 g, 12.2 mmol) was dissolved in DMF (50 mL) , and the solution was added with EDCI (4.68 g, 24.4 mmol), HOBT monohydrate (1.65 g, 12.2 mmol) and cumylamine (4.03 mL, 24.4 mmol), followed by stirring at room temperature for 4 hours. The reaction mixture was added with water (10 mL) and 1 mol/L hydrochloric acid (10 mL) , followed by stirring at room temperature for 1 hour. The reaction mixture was added with water and extracted with ethyl acetate. The organic layer was washed with water, saturated aqueous sodium hydrogencarbonate solution and saturated brine, and dried over anhydrous sodium sulfate. The solvent was evaporated under reduced pressure, and the residue was purified by flush column chromatography (hexane/ethyl acetate=95/5-80/20-70/30) to obtain 4-formyl-3-methoxymethoxy-*N*-(1-methyl-1-phenylethyl)benzamide (3.12 g, yield 78%).
ESI-MS *m*/*z*: 328 [M+H]⁺; ¹H-NMR (CDCl₃)δ(ppm) : 1.84 (s, 6H), 3.52 (s, 3H), 5.34 (s, 2H), 6.44 (br s, 1H), 7.27 (m, 1H), 7.32-7.41 (m, 4H), 7.42-7.50 (m, 2H), 7.68 (d, *J* = 1.5 Hz, 1H), 7.88 (d, *J* = 7.9 Hz, 1H).

### Step 3

4-Formyl-3-methoxymethoxy-*N*-(1-methyl-1-phenylethyl)benzami de (3.12 g, 9.53 mmol) was dissolved in methanol (30 mL), and the solution was added with trimethyl orthoformate (2.09 mL, 19.1 mmol) and p-toluenesulfonic acid monohydrate (16.4 mg, 0.0952 mmol), followed by stirring at room temperature for 1 hour. The reaction mixture was added with saturated aqueous sodium hydrogencarbonate solution and water, and extracted with ethyl acetate. The organic layer was washed with saturated brine, and dried over anhydrous sodium sulfate. The solvent was evaporated under reduced pressure, to obtain 3-methoxymethoxy-4-dimethoxymethyl-*N*-(1-methyl-1-phenylethyl)ben zamide (3.61 g, yield 100%).
ESI-MS *m*/*z*: 374 [M+H]⁺; ¹H-NMR (CDCl₃)δ(ppm) : 1.82 (s, 6H), 3.35 (s, 6H), 3.48 (s, 3H), 5.25 (s, 2H) , 5.69 (s, 1H), 6.40 (brs, 1H), 7.23-7.41 (m, 4H), 7.43-7.50 (m, 2H), 7.55-7.62 (m, 2H).

### Step 4

In a similar manner to Step 3 of Example 1, 3-methoxymethoxy-4-dimethoxymethyl-*N*-(1-methyl-1-phenylethyl)ben zamide (3.61 g, 9.67 mmol) was dissolved in THF (100 mL), and the solution was treated with TMEDA (4.70 mL, 31.0 mmol), sec-butyl lithium-haxane solution (1. 03 mol/L, 30.0 mL, 30.9 mmol) and DMF (1.65 mL, 21.3 mmol) , followed by purification by slurry using chloroform and hexane to obtain 4-methoxymethoxy-5-dimethoxymethyl-3-hydroxy-2-(1-methyl-1-pheny lethyl)isoindolinone (2.95 g, yield 85%).
ESI-MS *m*/*z:* 402 [M+H]⁺; ¹H-NMR (DMSO-*d*₆)δ(ppm) : 1.80 (s, 3H), 1.82 (s, 3H), 3.30 (s, 3H), 3.32 (s, 3H), 3.53 (s, 3H), 5.25 (d, *J* = 5.9 Hz, 1H), 5.52 (d, *J* = 5.9 Hz, 1H), 5.66 (s, 1H), 6.37 (d, *J* = 10.7 Hz, 1H), 6.66 (d, *J* = 10.7 Hz, 1H), 7.15 (m, 1H), 7.22-7.29 (m, 3H), 7.34-7.40 (m, 2H), 7.57 (d, *J* = 7.7 Hz, 1H).

### Step 5

In a similar manner to Step 4 of Example 1, 4-methoxymethoxy-5-dimethoxymethyl-3-hydroxy-2-(1-methyl-1-pheny lethyl)isoindolinone (3.34 g, 8.31 mmol) was dissolved in THF (100 mL), and the solution was treated with TMEDA (4.00 mL, 27.0 mmol), sec-butyl lithium-hexane solution (1.03 mol/L, 25.8 mL, 26.6 mmol) and iodine (3.16 g, 12.5 mmol), followed by purification by slurry using methanol and diisopropylether to obtain 4-methoxymethoxy-5-dimethoxymethyl-3-hydroxy-7-iodo-2-(1-methyl-1-phenylethyl)isoindolinone (2.24 g, yield 51%).
ESI-MS *m*/*z*: 528 [M+H]⁺; ¹H-NMR (CDCl₃)δ(ppm) : 1.94 (s, 3H), 1.98 (s, 3H), 3.31 (s, 3H), 3.38 (s, 3H), 3.58 (s, 3H), 3.87 (d, *J* = 5.3 Hz, 1H), 5.15 (d, *J* = 15.6 Hz, 1H), 5.18 (d, *J* = 15.6 Hz, 1H), 5.58 (s, 1H), 6.14 (d, *J*= 5.3 Hz, 1H), 7.23 (m, 1H), 7.28-7.36 (m, 2H) , 7.43-7.48 (m, 2H), 8.09 (s, 1H).

### Step 6

In a similar manner to Step 5 of Example 6, 4-methoxymethoxy-5-dimethoxymethyl-3-hydroxy-7-iodo-2-(1-methyl-1-phenylethyl)isoindolinone (400 mg, 0.759 mmol) was dissolved in acetonitrile (8 mL), and the solution was treated with 1 mol/L hydrochloric acid (4 mL) to obtain 4-hydroxy-5-formyl-3-hydroxy-7-iodo-2-(1-methyl-1-phenylethyl)is oindolinone (340 mg).

### Step 7

In a similar manner to Step 6 of Example 6, 4-hydroxy-5-formyl-3-hydroxy-7-iodo-2-(1-methyl-1-phenylethyl)is oindolinone (340 mg) was dissolved in acetonitrile (10 mL), and the solution was treated with 2.0 mol/L dimethylamine-THF solution (7.60 mL, 15.2 mmol) and acetic acid (0.870 mL, 15.2 mmol) to obtain 4-hydroxy-5-(dimethylaminomethyl)-3-hydroxy-7-iodo-2-(1-methyl-1 -phenylethyl)isoindolinone (370 mg).

### Step 8

In a similar manner to Step 7 of Example 6, 4-hydroxy-5-(dimethylaminomethyl)-3-hydroxy-7-iodo-2-(1-methyl-1 -phenylethyl)isoindolinone (370 mg) was dissolved in nitromethane (15 mL), and the solution was treated with trifluoroacetic acid (1.17 mL, 15.2 mmol) and triethylsilane (0.613 mL, 3.80 mmol), followed by purification by slurry using methanol and diisopropylether to obtain 4-hydroxy-5-(dimethylaminomethyl)-7-iodoisoindolinone (184 mg, yield 73%, 3 steps).
ESI-MS *m*/*z*: 333 [M+H]⁺; ¹H-NMR (CDCl₃)δ(ppm) : 2.33 (s, 6H), 3.79 (s, 2H), 4.14 (s, 2H), 7.59 (s, 1H), 8.66 (br s, 1H).

### Step 9

In a similar manner to Step 7 of Example 1, 4-hydroxy-5-(dimethylaminomethyl)-7-iodoisoindolinone (180 mg, 0.542 mmol) was dissolved in acetonitrile (10 mL), and the solution was treated with Compound BD (388 mg, 1.08 mmol), palladium acetate (9.7 mg, 0.0043 mmol), tri(o-tolyl)phosphine (26.4 mg, 0.0867 mmol) and triethylamine (0.755 mL, 5.42 mmol), followed by purification by preparative thin-layer chromatography (chloroform/methanol=4/1) to obtain 4-hydroxy-5-(dimethylaminomethyl)-7-[1-(tert-butoxycarbonyl)-5-( piperidinomethyl)indol-2-yl]isoindolinone (236 mg, yield 84%).
ESI-MS *m*/*z*: 519 [M+H]⁺; ¹H-NMR (CDCl₃)δ(ppm) : 1.34 (s, 9H), 1.38-1.46 (m, 2H), 1.50-1.69 (m, 4H), 2.30-2.54 (m, 4H), 2.40 (s, 6H), 3.59 (s, 2H), 3.79 (s, 2H), 4.39 (s, 2H), 6.29 (br s, 1H), 6.47 (s, 1H), 7.06 (s, 1H), 7.25 (m, 1H), 7.46 (s, 1H), 8.15 (d, *J* = 8.4 Hz, 1H).

### Step 10

In a similar manner to Step 8 of Example 1, 4-hydroxy-5-(dimethylaminomethyl)-7-[1-(tert-butoxycarbonyl)-5-( piperidinomethyl)indol-2-yl]isoindolinone (100 mg, 0.193 mmol) was dissolved in methanol (2.5 mL), and the solution was treated with 10% hydrogen chloride-methanol solution (2.5 mL), followed by adding with diisopropylether. The obtained solid was collected by filtration and washed with diisopropylether, followed by drying under reduced pressure to obtain Compound 562 (78.8 mg, yield 83%). ESI-MS *m*/*z*: 419 [M+H]⁺; ¹H-NMR (DMSO-*d*₆)δ(ppm) : 1.35 (m, 1H), 1.60-1.86 (m, 5H), 2.78 (s, 6H), 2.76-2.86 (m, 2H), 3.25-3.35 (m, 2H), 4.29 (d, *J* = 4.6 Hz, 2H), 4.43 (s, 2H), 4.47 (s, 2H), 7.20 (s, 1H), 7.25 (dd, *J* = 1.5, 8.3 Hz, 1H), 7.51 (d, *J* = 8.3 Hz, 1H), 7.75 (s, 1H), 8.46 (s, 1H), 9.40 (s, 1H), 9.96 (br s, 1H), 10.62 (br s, 2H), 13.77 (s, 1H).

### Example 96:

### 4-(2-Propyloxy)-5-(dimethylaminomethyl)-7-[1H-5-(piperidinomethy l)indol-2-yl]isoindolinone (Compound 563)

In a similar manner to Step 1 of Example 3, Compound 562 (29.1 g, 0.0592 mmol) was dissolved in DMF (1 mL), and the solution was treated with potassium carbonate (82.0 mg, 0.593 mmol) and isopropylbromide (0.056 mL, 0.60 mmol), followed by adding with water. The precipitated solid was collected by filtration and washed with water, followed by drying under reduced pressure to obtain Compound 563 (12.0 mg, yield 44%).
ESI-MS *m*/*z*: 461 [M+H]⁺; ¹H-NMR (DMSO-*d*₆)δ(ppm) : 1.19-1.57 (m, 6H), 1.27 (d, *J* = 6.1 Hz, 6H), 2.22 (s, 6H), 2.24-2.45 (m, 4H), 3.45 (s, 2H), 3.53 (s, 2H), 4.48 (s, 2H), 4.49 (m, 1H), 6.98 (s, 1H), 7.04 (dd, *J* = 1.5, 8.3 Hz, 1H), 7.35 (d, *J* = 8.3 Hz, 1H), 7.42 (s, 1H), 8.12 (s, 1H), 9.27 (s, 1H), 13.68 (s, 1H).

### Example 97:

### 4-Methanesulfonyloxy-5-(dimethylaminomethyl)-7-[1H-5-(piperidino methyl)indol-2-yl]isoindolinone dihydrochloride (Compound 564)

### Step 1

In a similar manner to Step 2 of Example 2, 4-hydroxy-5-(dimethylaminomethyl)-7-[1-(tert-butoxycarbonyl)-5-( piperidinomethyl)indol-2-yl]isoindolinone (27.0 mg, 0.0521 mmol) was dissolved in dichloromethane (2 mL) , and the solution was treated with triethylamine (0.029 mL, 0.21 mmol) and methanesulfonyl chloride (0.0080 mL, 0.10 mmol), followed by preparative thin-layer chromatography (chloroform/methanol=4/1) to obtain 4-methanesulfonyloxy-5-(dimethylaminomethyl)-7-[1-(tert-butoxyca rbonyl)-5-(piperidinomethyl)indol-2-yl]isoindolinone (22.5 mg, yield 73%).
ESI-MS *m*/*z:* 597 [M+H]⁺; ¹H-NMR (CDCl₃)δ(ppm) : 1.30 (s, 9H), 1.38-1.81 (m, 6H), 2.29 (s, 6H), 2.33-2.57 (m, 4H), 3.45 (s, 3H), 3.61 (s, 2H), 3.65 (br s, 2H), 4.61 (s, 2H), 6.54 (br s, 1H), 6.57 (s, 1H), 7.30 (d, *J* = 8.9 Hz, 1H), 7.51 (s, 1H), 7.57 (s, 1H), 8.21 (d, *J* = 8.9 Hz, 1H).

### Step 2

In a similar manner to Step 8 of Example 1, 4-methanesulfonyloxy-5-(dimethylaminomethyl)-7-[1-(tert-butoxyca rbonyl)-5-(piperidinomethyl)indol-2-yl]isoindolinone (22.5 mg, 0.0377 mmol) was dissolved in methanol (1 mL), and the solution was treated with 10% hydrogen chloride-methanol solution (1 mL). The obtained solid was collected by filtration and washed with methanol, followed by drying under reduced pressure to obtain Compound 564 (9.4 mg, yield 44%).
ESI-MS *m*/*z*: 497 [M+H]⁺; ¹H-NMR (DMSO-*d*₆)δ(ppm) : 1. 31 (m, 1H) , 1.58-1.90 (m, 5H), 2.49 (s, 3H), 2.50 (s, 3H), 2.75-2.85 (m, 2H), 3.24-3.33 (m, 2H), 3.80 (s, 3H), 4.26-4.36 (m, 2H), 4.46-4.55 (m, 2H), 4.63 (s, 2H), 7.33 (d, *J* = 8.1 Hz, 1H), 7.58 (s, 1H), 7.59 (d, *J* = 8.3 Hz, 1H), 7.82 (s, 1H), 8.91 (s, 1H), 9.62 (s, 1H), 9.91 (br s, 1H), 11.38 (br s, 1H), 13.80 (s, 1H).

### Example 98:

### 4-Trifluoromethanesulfonyloxy-5-(dimethylaminomethyl)-7-[1H-5-(p iperidinomethyl)indol-2-yl]isoindolinone dihydrochloride (Compound 565)

### Step 1

4-Hydroxy-5-(dimethylaminomethyl)-7-[1-(tert-butoxycarbonyl )-5-(piperidinomethyl)indol-2-yl]isoindolinone (100 mg, 0.193 mmol) was suspended in dichloromethane (4 mL) , and the suspension was added with triethylamine (0.439mL, 3.15 mmol) and trifluoromethanesulfonyl chloride (0.126 mL, 1.18 mmol) under ice-cooling, followed by stirring for 10 hours. The reaction mixture was added with water, and extracted with ethyl acetate. The organic layer was washed with saturated brine, and dried over anhydrous sodium sulfate. The solvent was evaporated under reduced pressure to obtain 4-trifluoromethanesulfonyloxy-5-(dimethylaminomethyl)-7-[1-(tert -butoxycarbonyl)-5-(piperidinomethyl)indol-2-yl]isoindolinone (46.7 mg, yield 37%).
ESI-MS *m*/*z*: 651 [M+H]⁺; ¹H-NMR (CDCl₃)δ(ppm) : 1.32 (s, 9H), 1.43-1.50 (m, 2H), 1.57-1.80 (m, 4H), 2.29 (s, 6H), 2.41-2.72 (m, 4H), 3.64 (s, 2H), 3.75-3.83 (m, 2H), 4.56 (s, 2H), 6.60 (s, 1H), 6.73 (br s, 1H), 7.32 (d, *J* = 8.5 Hz, 1H), 7.57 (br s, 1H), 7.72 (s, 1H), 8.21 (d, *J* = 8.5 Hz, 1H).

### Step 2

In a similar manner to Step 8 of Example 1, 4-trifluoromethanesulfonyloxy-5-(dimethylaminomethyl)-7-[1-(tert -butoxycarbonyl)-5-(piperidinomethyl)indol-2-yl]isoindolinone (16.9 mg, 0.260 mmol) was dissolved in methanol (1 mL), and the solution was treated with 10% hydrogen chloride-methanol solution (1 mL). The reaction mixture was added with diisopropylether and purified by slurry to obtain Compound 565 (8.8 mg, yield 54%).
ESI-MS *m*/*z*: 551 [M+H]⁺; ¹H-NMR (DMSO-*d*₆)δ(ppm) : 1.31 (m, 1H), 1.53-1.89 (m, 5H), 2.75-2.85 (m, 2H), 2.81 (s, 6H), 3.20-3.40 (m, 2H), 4.32 (d, *J* = 4.6 Hz, 2H), 4.54 (br s, 2H), 4.62 (s, 2H), 7.34 (dd, *J* = 1.3, 8.5 Hz, 1H), 7.60 (d, *J* = 8.5 Hz, 1H), 7.71 (s, 1H), 7.84 (s, 1H), 9.04 (s, 1H), 9.74 (s, 1H), 9.90 (br s, 1H), 11.70 (br s, 1H), 13.76 (s, 1H).

### Example 99:

### 5-(1H-Benzimidazol-2-yl)-7-(1H-pyrrol-2-yl)isoindolinone hydrochloride (Compound 566)

### Step 1

In a similar manner to Step 1 of Example 15, 5-(1H-benzimidazol-2-yl)-7-iodoisoindolinone (19.0 mg, 0.0506 mmol) was dissolved in dimethoxyethane (2 mL), and the solution was treated with 1-(tert-butoxycarbonyl)pyrrole-2-boronic acid (22.0 mg, 0.104 mmol), [bis(diphenylphosphino)ferrocene]dichloropalladium (3.3 mg, 0.040 mmol), potassium carbonate (35.0 mg, 0.253 mmol) and water (0.036 mL, 2.0 mmol),followed by purification by preparative thin-layer chromatography (chloroform/methanol=9/1) to obtain 5-(1H-benzimidazol-2-yl)-7-[1-(tert-butoxycarbonyl)pyrrol-2-yl]i soindolinone (15.0 mg, yield 71%).
ESI-MS *m*/*z*: 415 [M+H]⁺; ¹H-NMR (CDCl₃)δ(ppm) : 1.34 (s, 9H), 4.40 (s, 2H), 6.27 (dd, *J* = 3.1, 3.3 Hz, 1H), 6.31 (dd, *J* = 1.8, 3.3 Hz, 1H), 7.43 (dd, *J* = 1.8, 3.1 Hz, 1H), 7.52 (m, 1H), 7.76 (m, 1H), 8.01 (s, 1H), 8.15 (s, 1H).

### Step 2

In a similar manner to Step 8 of Example 1, 5-(1H-benzimidazol-2-yl)-7-[1-(tert-butoxycarbonyl)pyrrol-2-yl]i soindolinone (15.0 mg, 0.0362 mmol) was dissolved in methanol (1 mL), and the solution was treated with 10% hydrogen chloride-methanol solution (1 mL). The obtained solid was collected by filtration, and washed with methanol, followed by drying under reduced pressure to obtain Compound 566 (7.6 mg, yield 60%).
ESI-MS *m*/*z:* 315 [M+H]⁺; ¹H-NMR (DMSO-*d*₆)δ(ppm) : 4.61 (s, 2H), 6.27 (dd, *J* = 2.4, 6.0 Hz, 1H), 7.09 (m, 1H), 7.15 (m, 1H), 7.44-7.52 (m, 2H), 7.77-7.86 (m, 2H), 7 (q, *J* = 3.1 Hz, 2H), 8.09 (d, *J* = 1.1 Hz, 1H), 8.74 (d, *J* = 1.1 Hz, 1H), 9.41 (s, 1H), 13.79 (s, 1H).

### Example 100:

### 5-(Methoxymethyl)-7-(1H-5-formylpyrrol-2-yl)isoindolinone (Compound 567)

In a similar manner to Step 1 of Example 15, 5-(methoxymethyl)-7-iodoisoindolinone (50.0 mg, 0.165 mmol) was dissolved in dimethoxyethane (5 mL), and the solution was treated with Compound CK (45.7 mg, 0.330 mmol), [bis(diphenylphosphino)ferrocene]dichloropalladium (10.8 mg, 0.0132 mmol), potassium carbonate (114 mg, 0.825 mmol) and water (0.119 mL, 6.61 mmol), followed by purification by preparative thin-layer chromatography (chloroform/methanol=20/1) to obtain Compound 567 (18.5 mg, yield 41%).
ESI-MS *m*/*z*: 271 [M+H]⁺; ¹H-NMR (DMSO-*d*₆)δ(ppm) : 3.35 (s, 3H), 4.50 (s, 2H), 4.56 (s, 2H), 7.08 (s, 1H), 7.09 (s, 1H), 7.46 (s, 1H), 7.97 (s, 1H), 9.28 (br s, 1H), 9.51 (s, 1H), 14.83 (br s, 1H).

### Example 101:

### 5-(Dimethylaminomethyl)-7-(1H-pyrrol-2-yl)isoindolinone hydrochloride (Compound 568)

### Step 1

In a similar manner to Step 1 of Example 15, 5-(dimethylaminomethyl)-7-iodoisoindolinone (50.0 mg, 0.158 mmol) was dissolved in dimethoxyethane (5 mL), and the solution was treated with 1-(tert-butoxycarbonyl)pyrrole-2-boronic acid (67.0 mg, 0.318 mmol), [bis(diphenylphosphino)ferrocene]dichloropalladium (10.3 mg, 0.0126 mmol), potassium carbonate (109 mg, 0.789 mmol) and water (0.114 mL, 6.33 mmol), followed by purification by preparative thin-layer chromatography (chloroform/methanol/7 mol/L ammonia-methanol solution=24/1/1) to obtain 5-(dimethylaminomethyl)-7-[1-(tert-butoxycarbonyl)pyrrol-2-yl]is oindolinone (50.2 mg, yield 89%).
ESI-MS *m*/*z*: 356 [M+H]⁺; ¹H-NMR (CDCl₃)δ(ppm) : 1.26 (s, 9H), 2.28 (s, 6H), 3.52 (s, 2H), 4.38 (s, 2H), 6.24 (dd, *J* = 1.8, 3.3 Hz, 1H), 6.26 (dd, *J* = 3.2, 3.3 Hz, 1H), 7.31 (s, 1H), 7.38 (s, 1H), 7.45 (dd, *J* = 1.8, 3.2 Hz, 1H), 8.15 (br s, 1H).

### Step 2

In a similar manner to Step 8 of Example 1, 5-(dimethylaminomethyl)-7-[1-(tert-butoxycarbonyl)pyrrol-2-yl]is oindolinone was dissolved (1 mL), and the solution was treated with 10% hydrogen chloride-methanol solution (1 mL), followed by adding with diisopropylether. The obtained solid was collected by filtration, and washed with diisopropylether, followed by drying under reduced pressure to obtain Compound 568 (30.7 mg, yield 75%).
ESI-MS *m*/*z:* 256 [M+H]⁺; ¹H-NMR (DMSO-*d*₆)δ(ppm) : 2.73 (s, 6H), 4.34 (br s, 2H), 4.48 (s, 2H), 6.20 (m, 1H), 6.93 (m, 1H), 7.04 (m, 1H), 7.39 (s, 1H), 8.13 (s, 1H), 9.26 (s, 1H), 10.60 (br s, 1H), 13.74 (s, 1H).

### Example 102:

### 5-(Dimethylaminomethyl)-7-(1H-5-formylpyrrol-2-yl)isoindolinone (Compound 569)

In a similar manner to Step 1 of Example 15, 5-(dimethylaminomethyl)-7-iodoisoindolinone (72.8 mg, 0.230 mmol) was dissolved in dimethoxyethane (5 mL), and the solution was treated with Compound CK (95.5 mg, 0.690 mmol), [bis(diphenylphosphino)ferrocene]dichloropalladium (15.0 mg, 0.0184 mmol), potassium carbonate (159 mg, 1.15 mmol) and water (0.166 mL, 9.22 mmol), followed by purification by preparative thin-layer chromatography (chloroform/methanol=5/1) to obtain Compound 569 (37.9 mg, yield 58%).
ESI-MS *m*/*z*: 284 [M+H]⁺; ¹H-NMR (DMSO-*d*₆)δ(ppm) : 2.20 (s, 6H), 3.53 (s, 2H), 4.50 (s, 2H), 7.06-7.12 (m, 2H), 7.46 (s, 1H), 7.95 (s, 1H), 9.28 (s, 1H), 9.52 (s, 1H), 14.85 (s, 1H).

### Example 103:

### 5-(Dimethylaminomethyl)-7-[1H-5-(dimethylaminomethyl)pyrrol-2-yl ]isoindolinone (Compound 570)

### Step 1

In a similar manner to Step 2 of Example 45, Compound 569 (15.0 mg, 0.0529 mmol) was dissolved in acetonitrile (1 mL) , and the solution was treated with dimethylamine hydrochloride (85.0 mg, 1.04 mmol), acetic acid (0. 060 mL, 1.1 mmol) , triethylamine (0.147 mL, 1.05 mmol) , and sodium triacetoxyborohydride (34 mg, 0.160 mmol), followed by purification by preparative thin-layer chromatography (chloroform/methanol/7 mol/L ammonia-methanol solution=12/1/1) to obtain Compound 570 (11.5 mg, yield 70%).
ESI-MS *m*/*z*: 313 [M+H]⁺; ¹H-NMR (DMSO-*d*₆)δ(ppm) : 2.16 (s, 6H), 2.18 (s, 6H), 3.39 (s, 2H), 3.46 (s, 2H), 4.41 (s, 2H), 6.00 (dd, *J* = 2.8, 3.2 Hz, 1H), 6.72 (dd, *J* = 2.6, 3.2 Hz, 1H), 7.20 (s, 1H), 7.71 (s, 1H), 8.92 (s, 1H), 13.70 (s, 1H).

### Example 104:

### 5-(2-Propyloxy)-7-(1H-pyrrol-2-yl)isoindolinone (Compound 571)

### Step 1

In a similar manner to Step 7 of Example 1, 5-(2-propyloxy)-7-iodoisoindolinone (50.0 mg, 0.158 mmol) was dissolved in acetonitrile (3 mL) , and the solution was treated with 1- (tert-butoxycarbonyl)pyrrole-2-boronic acid (67.0 mg, 0.316 mmol), palladium acetate (3.0 mg, 0.013 mmol), tri(o-tolyl)phosphine (8.0 mg, 0.025 mmol) and triethylamine (0.220 mL, 1.58 mmol), followed by purification by preparative thin-layer chromatography (chloroform/methanol=9/1) to obtain 5-(2-propyloxy)-7-[1-(tert-butoxycarbonyl)pyrrol-2-yl]isoindolin one (55.9 mg, yield 99%).
ESI-MS *m*/*z*: 357 [M+H]⁺; ¹H-NMR (CDCl₃)δ(ppm) : 1.34 (s, 9H), 1.38 (d, *J* = 6.0 Hz, 6H), 4.27 (s, 2H), 4.64 (m, 1H), 6.18-6.26 (m, 2H), 6.31 (br s, 1H), 6.84 (d, *J* = 2.2 Hz, 1H) , 6.91 (d, *J* = 2.2 Hz, 1H), 7.43 (m, 1H).

### Step 2

In a similar manner to Step 8 of Example 1, 5-(2-propyloxy)-7-[1-(tert-butoxycarbonyl)pyrrol-2-yl]isoindolin one (53.2 mg, 0.149 mmol) was dissolved in methanol (1.5 mL), and the solution was treated with 10% hydrogen chloride-methanol solution (1.5 mL), followed by purification by preparative thin-layer chromatography (chloroform/methanol/7 mol/L ammonia-methanol solution=24/1/1) to obtain Compound 571 (10.5 mg, yield 28%). ESI-MS *m*/*z*: 257 [M+H]⁺; ¹H-NMR (CDCl₃)δ(ppm) : 1.39 (d, *J* = 5.9 Hz, 6H), 4.43 (s, 2H), 4.65 (m, 1H), 6.20-6.40 (m, 2H), 6.67 (s, 1H), 6.78 (s, 1H), 6.97 (s, 1H), 7.32 (s, 1H), 13.51 (br s, 1H).

### Example 105:

### 5-Amino-7-[1H-5-(piperidinomethyl)indol-2-yl)isoindolinone dihydrochroride (Compound 572)

### Step 1

In a similar manner to Step 2 of Example 1, 4-tert-butoxycarbonylaminobenzoic acid (10.1 g, 42.5 mmol) was dissolved in DMF (100 mL), and the solution was treated with EDCI (16.3 g, 85.0 mmol), HOBt monohydrate (6.51 g, 42.5 mmol) and cumylamine (12.2 mL, 85.0 mmol). The reaction mixture was added with water. The precipitated solid was collected by filtration and washed by water, followed by drying under reduced pressure to obtain 4-tert-butoxycarbonylamino-N-(1-methyl-1-phenylethyl)benzamide (13.8 g, yield 92%).
ESI-MS *m*/*z*: 355 [M+H]⁺

### Step 2

In a similar manner to Step 3 of Example 1, 4-tert-butoxycarbonylamino-N-(1-methyl-1-phenylethyl)benzamide (1.00 g, 2.82 mmol) was dissolved in THF (50 mL), and the solution was treated with TMEDA (1.70 mL, 11.3 mmol), sec-butyl lithium-hexane solution (1.00 mol/L, 11.3 mL, 11.3 mmol) and DMF (0.880 mL, 11.4 mmol), followed by purification by diflush column chromatography (hexane/ethyl acetate=95/5, 90/10, 80/20) to obtain 5-tert-butoxycarbonylamino-3-hydroxy-2-(1-methyl-1-phenylethyl)i soindolinone (1.03 g, yield 95%).
ESI-MS *m*/*z*: 383 [M+H]⁺; ¹H-NMR (CDCl₃)δ(ppm): 1.53 (s, 9H), 1.90 (s, 3H), 1.95 (s, 3H), 2.84 (d, *J* = 10.4 Hz, 1H), 6.06 (d, *J* = 10.4 Hz, 1H), 6.76 (s, 1H), 7.18-7.23 (m, 2H), 7.26-7.34 (m, 2H), 7.37-7.44 (m, 2H), 7.53 (d, *J* = 8.3 Hz, 1H), 7.76 (d, *J* = 1.3 Hz, 1H).

### Step 3

In a similar manner to Step 4 of Example 1, 5-tert-butoxycarbonylamino-3-hydroxy-2-(1-methyl-1-phenylethyl)i soindolinone (1.03 g, 2.69 mmol) was dissolved in THF (20 mL), and the solution was treated with TMEDA (2.03 mL, 13.5 mmol), sec-butyl lithium-hexane solution (1.00 mol/L, 13.5 mL, 13.5 mmol) and iodine (1.02 g, 4.01 mmol), followed by purification by flush column chromatography (hexane/ethyl acetate=95/5, 90/10, 85/15, 80/20, 75/25) to obtain 5-tert-butoxycarbonylamino-3-hydroxy-7-iodo-2-(1-methyl-1-phenyl ethyl)isoindolinone (767 mg, yield 56%).
ESI-MS *m*/*z*: 509 [M+H]⁺; ¹H-NMR (CDCl₃)δ(ppm): 1.52 (s, 9H), 1.93 (s, 3H), 1.97 (s, 3H), 2.27 (d, *J* = 10.1 Hz, 1H), 5.91 (d, *J* = 10.1 Hz, 1H), 6.66 (s, 1H), 7.23 (m, 1H), 7.29-7.37 (m, 2H), 7.40-7.46 (m, 2H), 7.74 (d, *J* = 1.8 Hz, 1H), 7.77 (d, *J* = 1.8 Hz, 1H).

### Step 4

In a similar manner to Step 7 of Example 6, 5-tert-butoxycarbonylamino-3-hydroxy-7-iodo-2-(1-methyl-1-phenyl ethyl) isoindolinone (760 mg, 1.50 mmol) was dissolved in nitromethane (15 mL), and the solution was treated with trifluoroacetic acid (1.16 mL, 15.1 mmol) and triethylsilane (1.21 mL, 7.49 mmol) followed by purification by slurry using diisopropylether to obtain 5-amino-7-iodoisoindolinone (257 mg, yield 63%).
ESI-MS *m*/*z*: 274 [M+H]⁺; ¹H-NMR (DMSO-d*₆*)δ(ppm): 4.07 (s, 2H), 5.88 (s, 2H), 6.61 (d, *J* = 1.8 Hz, 1H), 7.06 (d, *J* = 1.8 Hz, 1H), 8.05 (br s, 1H).

### Step 5

In a similar manner to Step 5 of Example 79, 5-amino-7-iodoisoindolinone (50.0 mg, 0.182 mmol) was treated with Compound BD (131 mg, 0.365 mmol), triethylamine (0.127 mL, 0.910 mmol), [bis(diphenylphosphino)ferrocene]dichloropalladium (12 mg, 0.015 mmol) and DMA (1 mL)/water (0.13 mL), followed by purification by preparative thin-layer chromatography (chloroform/methanol=6/1) to obtain 5-amino-7-[1-(*tert*-butoxycarbonyl)-5-(piperidinomethyl)indol-2-y 1]soindolinone (66.0 mg, yield 79%).
ESI-MS *m*/*z*: 461 [M+H]⁺; ¹H-NMR (CDCl₃)δ(ppm): 1.34 (s, 9H), 1.42-1.82 (m, 6H), 2.33-2.52 (m, 4H), 3.60 (s, 2H), 4.06 (s, 2H), 4.31 (s, 2H), 6.16 (br s, 1H), 6.51 (s, 1H), 6.65 (s, 1H), 6.72 (s, 1H), 7.26 (dd, *J* = 1.5, 8.4 Hz, 1H), 7.47 (s, 1H), 8.16 (d, *J* = 8.4 Hz, 1H).

### Step 6

In a similar manner to Step 8 of Example 1, Compound 572 (38.4 mg, yield 64%) was obtained using 5-amino-7-[1-(*tert*-butoxycarbonyl)-5-(piperidinomethyl)indol-2-y l]isoindolinone (64.0 mg, 0.139 mmol), 10% hydrogen chloride-methanol solution (2 mL) and methanol (2 mL).
ESI-MS *m*/*z*: 361 [M+H]⁺; ¹H-NMR (DMSO-*d₆*)δ(ppm): 1.35 (m, 1H), 1.56-1.87 (m, 5H), 2.75-2.90 (m, 2H), 3.28-3.36 (m, 2H), 4.24-4.34 (m, 2H), 4.34 (s, 2H), 6.65 (s, 1H), 6.97 (s, 1H), 7.26 (d, *J* = 8.3 Hz, 1H), 7.36 (s, 1H), 7.51 (d, *J* = 8.3 Hz, 1H), 7.74 (s, 1H), 8.70 (s, 1H), 9.81 (br s, 1H), 14.38 (s, 1H).

Compounds 573-577 were synthesized in a similar manner to Example 105 (Examples 106-110).

### Example 106:

### 5-Methanesulfonylamino-7-[1H-5-(piperidinomethyl)indol-2-yl)isoi ndolinone (Compound 573)

ESI-MS *m*/*z*: 439 [M+H]⁺; ¹H-NMR (DMSO-*d*₆)δ(ppm): 1.30-1.60 (m, 6H), 2.25-2.50 (m, 4H), 3.15 (s, 3H), 3.56 (s, 2H), 4.48 (s, 2H), 6.94 (s, 1H), 7.10 (d, *J* = 8.4 Hz, 1H), 7.31 (s, 1H), 7.41 (d, *J* = 8.4 Hz, 1H), 7.50 (s, 1H), 7.85 (s, 1H), 9.13 (s, 1H), 10.23 (br s, 1H), 13.92 (s, 1H).

### Example 107:

### 5-Bis(methanesulfonyl)amino-7-[1H-5-(piperidinomethyl)indol-2-yl )isoindolinone hydrochloride (Compound 574)

ESI-MS *m*/*z*: 517 [M+H]⁺; ¹H-NMR (DMSO-*d*₆)δ(ppm): 1.35 (m, 1H), 1.55-1.90 (m, 5H), 2.79-2.90 (m, 2H), 3.28-3.36 (m, 2H), 3.66 (s, 6H), 4.33 (d, *J* = 4.4 Hz, 2H), 4.57 (s, 2H), 7.31 (d, *J* = 8.4 Hz, 1H), 7.56 (s, 1H), 7.59 (d, *J* = 8.4 Hz, 1H), 7.73 (s, 1H), 8.25 (s, 1H), 9.51 (s, 1H), 9.65 (m, 1H), 13.99 (s, 1H).

### Example 108:

### 5-Acetylamino-7-[1H-5-(piperidinomethyl)indol-2-yl)isoindolinone (Compound 575)

ESI-MS *m*/*z*: 403 [M+H]⁺; ¹H-NMR (DMSO-*d*₆)δ(ppm): 1.30-1.60 (m, 6H), 2. 12 (s, 3H), 2.22-2.45 (m, 4H), 3.47 (br s, 2H), 4.47 (s, 2H), 6.92 (s, 1H), 7.07 (d, *J* = 8.7 Hz, 1H), 7.38 (d, *J* = 8.7 Hz, 1H), 7.48 (s, 1H), 7.87 (s, 1H), 8.17 (s, 1H), 9.10 (s, 1H), 10.34 (s, 1H), 13.94 (s, 1H).

### Example 109:

### 5-(2-Propylamino)-7-{1H-5-[4-(2-hydroxyethyl)piperazin-1-ylmethy 1]indol-2-yl}isoindolinone trihydrochloride (Compound 576)

ESI-MS *m*/*z*: 448 [M+H]⁺; ¹H-NMR (DMSO-*d*₆)δ(ppm): 1.20 (d, *J* = 6.3 Hz, 6H), 3.08-4.30 (m, 13H), 4.36 (s, 2H), 4.43 (br s, 2H), 6.65 (s, 1H), 7.06 (s, 1H), 7.32 (d, *J* = 8.4 Hz, 1H), 7.36 (s, 1H), 7.52 (d, *J* = 8.4 Hz, 1H), 7.81 (s, 1H), 8.18 (s, 1H), 11.04 (br s, 1H), 11.75 (br s, 2H), 14.42 (s, 1H).

### Example 110:

### 5-(N-Methyl-2-propylamino)-7-{1H-5-[4-(2-hydroxyethyl)piperazin-1-ylmethyl]indol-2-yl}isoindolinone dihydrochloride (Compound 577)

ESI-MS *m*/*z*: 462 [M+H]⁺; ¹H-NMR (DMSO-*d*₆)δ(ppm): 1.20 (d, *J* = 6.4 Hz, 6H), 2.89 (s, 3H), 3.16-3.30 (m, 2H), 3.30-3.90 (m, 10H), 4.36 (sep, *J* = 6.4 Hz, 1H), 4.40 (s, 2H), 4.47 (brs, 2H), 6.93 (brs, 1H), 7.29-7.36 (m, 2H), 7.51 (br s, 1H), 7.54 (d, *J* = 8.4 Hz, 2H), 7.84 (s, 1H), 8.76 (br s, 1H), 11.04 (br s, 1H), 11.82 (br s, 1H), 14.35 (s, 1H).

### Example 111:

### 5-Vinyl-7-[1H-5-(piperidinomethyl)indol-2-yl)isoindolinone hydrochloride (Compound 578)

### Step 1

5-Bromo-7-[1-(*tert*-butoxycarbonyl)-5-(piperidinomethyl)indo 1-2-yl]isoindolinone (120 mg, 0.229 mmol) was dissolved in DMF (1.2 mL), and the solution was added with 2,4,6-trivinylcyclotriboroxane-pyridine complex (110 mg, 0.458 mmol), triethylamine (0.159 mL, 1.14 mmol), water (0.166 mL) and [bis(diphenylphosphino)ferrocene]dichloropalladium (18.7 mg, 0.0229 mmol), followed by stirring at 80 °C for 2 hours under argon atmosphere. The reaction mixture was diluted with water and ethyl acetate, and the insoluble material in the reaction mixture was filtered off using Celite. The filtrate was extracted with ethyl acetate. The organic layer was washed with saturated brine and dried over anhydrous sodium sulfate. The solvent was evaporated under reduced pressure, and the residue was purified by flush column chromatography (chloroform/methanol=99/1, 80/20) to obtain 5-vinyl-7-[1-(*tert*-butoxycarbonyl)-5-(piperidinomethyl)indol-2-y 1]isoindolinone (102 mg, yield 95%).
ESI-MS *m*/*z*: 472 [M+H]⁺; ¹H-NMR (DMSO-*d*₆)δ(ppm): 1.31 (s, 9H), 1.41-1.49 (m, 2H), 1.58-1.70 (m, 4H), 2.45-2.56 (m, 4H), 3.69 (s, 2H), 4.43 (s, 2H), 5.41 (d, *J* = 10.7 Hz, 1H), 5.89 (d, *J* = 17.6 Hz , 1H), 6.58 (s, 1H), 6.82 (dd, *J* = 10.7, 17.6 Hz, 1H), 6.87 (m, 1H), 7.30 (dd, *J* = 2.0, 8.2 Hz, 1H), 7.45-7.55 (m, 3H), 8.19 (d, *J* = 8.4 Hz, 1H).

### Step 2

5-Vinyl-7-[1-(*tert*-butoxycarbonyl)-5-(piperidinomethyl)indo 1-2-yl] isoindolinone (125 mg, 0.265 mmol) was added with 10% hydrogen chloride-methanol solution (2.5 mL), followed by stirring at 80 °C for 4 hours. The reaction mixture was condensed under reduced pressure. The residue was added with ethyl acetate and diethyl ether, and purified by slurry to obtain Compound 578 (70'.7 mg, yield 65%). ESI-MS *m*/*z*: 372 [M+H]⁺; ¹H-NMR (DMSO-*d*₆)δ(ppm): 1.28-1.45 (m, 1H), 1.61-1.86 (m, 6H), 2.77-2.94 (m, 2H), 3.23-3.35 (m, 2H), 4.31 (s, 1H), 4.33 (s, 1H), 4.52 (s, 1H), 5.51 (d, *J* = 11.4 Hz, 1H), 6.16 (d, *J* = 17.6 Hz, 1H), 6.93 (dd, *J* = 11.4, 17.6 Hz, 1H), 7.31 (dd, *J* = 1.5, 8.4 Hz, 1H), 7.42 (d, *J* = 1.5 Hz, 1H), 7.56 (d, *J* = 8.4 Hz, 1H), 7.66 (s, 1H), 7.79 (s, 1H), 8.26 (s, 1H), 9.30 (s, 1H), 9.88 (br s, 1H), 14.08 (s, 1H).

Compounds 579-582 were synthesized in a similar manner to Example 111 (Examples 112-115).

### Example 112:

### 5-(cis-1-Propenyl)-7-[1H-5-(piperidinomethyl)indol-2-yl)isoindol inone hydrochloride (Compound 579)

ESI-MS *m*/*z*: 386 [M+H]⁺; ¹H-NMR (DMSO-*d*₆)δ(ppm): 1.28-1.45 (m, 1H), 1.62-1.86 (m, 5H), 1. 97 (dd, *J* = 1.6, 7.2 Hz, 3H), 2.77-2.94 (m, 2H), 3.45-3.66 (m, 2H), 4.31 (s, 1H), 4.33 (s, 1H), 4.53 (s, 2H), 5.94-6.07 (m, 1H), 6.62 (dd, *J* = 1.6, 11.7 Hz, 1H), 7.30 (dd, *J* = 1.5, 8.7 Hz, 1H), 7.31 (s, 1H), 7.48 (s, 1H), 7.57 (d, *J* = 8.7 Hz, 1H), 7.77 (s, 1H), 8.08 (s, 1H), 9.29 (s, 1H), 9.86 (br s, 1H), 14.11 (s, 1H).

### Example 113:

### 5-(1,2-Dimethylpropenyl)-7-[1H-5-(piperidinomethyl)indol-2-yl)is oindolinone (Compound 580)

ESI-MS *m*/*z*: 414 [M+H]⁺; ¹H-NMR (DMSO-*d*₆)δ(ppm): 1.33-1.56 (m, 6H), 2.35 (s, 3H), 2.50 (s, 6H), 3.49 (s, 2H), 3.55-3.66 (m, 4H), 4.49 (s, 2H), 7.11 (d, *J* = 8.2 Hz, 1H), 7.32 (s, 1H), 7.40 (d, *J* = 8.2 Hz, 1H), 7.45 (s, 1H), 7.71 (s, 1H), 8.31 (s, 1H), 9.33 (s, 1H), 13.79 (s, 1H).

### Example 114:

### 5-(trans-1-Propenyl)-7-[1H-5-(piperidinomethyl)indol-2-yl)isoind olinone hydrochloride (Compound 581)

ESI-MS *m*/*z*: 386 [M+H]⁺; ¹H-NMR (DMSO-*d*₆)δ(ppm): 1.28-1.45 (m, 1H), 1.62-1.89 (m, 5H), 1.94 (d, J = 5.0 Hz, 3H), 2.78-2.94 (m, 2H), 3.45-3.66 (m, 2H), 4.31 (s, 1H), 4.33 (s, 1H), 4.49 (s, 2H), 6.59-6.68 (m, 2H), 7.30 (dd, *J* = 1.7, 8.4 Hz, 1H), 7.40 (d, *J* = 1.7 Hz, 1H), 7.54 (s, 1H), 7.55 (d, *J* = 8.4 Hz, 1H), 7.78 (s, 1H), 8.17 (s, 1H), 9.24 (s, 1H), 9.86 (br s, 1H), 14.12 (s, 1H).

### Example 115:

### 5-(trans-3,3-Dimethyl-1-butenyl)-7-[1H-5-(piperidinomethyl)indol -2-yl)isoindolinone hydrochloride (Compound 582)

ESI-MS *m*/*z*: 428 [M+H]⁺; ¹H-NMR (DMSO-*d*₆)δ(ppm): 1.16 (s, 9H), 1.28-1.45 (m, 1H), 1.62-1.89 (m, 5H), 2.78-2.94 (m, 2H), 3.45-3.66 (m, 2H), 4.32 (s, 1H), 4.33 (s, 1H), 4.49 (s, 2H), 6.51 (d, *J* = 16.3 Hz, 1H), 6.69 (d, *J* = 16.3 Hz, 1H), 7.30 (d, *J* = 8.6 Hz, 1H), 7.42 (s, 1H), 7.56 (d, *J* = 8.6 Hz, 1H), 7.61 (s, 1H), 7.77 (s, 1H), 8.20 (s, 1H), 9.24 (s, 1H), 9.77 (br s, 1H), 14.13 (s, 1H).

### Example 116:

### 5-(1,2-Dihydroxy-3,3-dimethylbutyl)-7-[1H-5-(piperidinomethyl)in dol-2-yl)isoindolinone hydrochloride (Compound 583)

### Step 1

5-(trans-3,3-Dimethyl-1-butenyl)-7-[1-(tert-butoxycarbonyl) -5-(piperidinomethyl)indol-2-yl]isoindolinone (136 mg, 0.258 mmol) was dissolved in THF (2.7 mL) and water (0.27 mL), and the solution was added with aqueous N-methylmorpholine N-oxide solution (50 wt. %, 0.181 mL, 0.773 mmol) and aqueous Osmium tetroxide (VIII) solution (2.5 wt. %, 0.131 mL, 0.129 mmol), followed by stirring at room temperature for 4 hours. The reaction mixture was added with saturated aqueous sodium thiosulfate solution, followed by stirring at room temperature for 12 hours. The reaction mixture was extracted with ethyl acetate. The organic layer was washed with saturated brine and dried over anhydrous sodium sulfate. The solvent was evaporated under reduced pressure, and the residue was purified by flush column chromatography (chloroform/methanol=95/5, 80/20) to obtain 5-(1,2-dihydroxy-3,3-dimethylbutyl)-7-[1-(tert-butoxycarbonyl)-5 (piperidinomethyl)indol-2-yl]isoindolinone (55.7 mg, yield 38%).
ESI-MS *m*/*z*: 562 [M+H]⁺; ¹H-NMR (CDCl₃)δ(ppm): 1.01 (s, 9H), 1.23-1.35 (m, 2H), 1.29 (s, 9H), 1.37-1.48 (m, 2H), 1.54-1.65 (m, 4H), 2.39-2.52 (m, 4H), 3.38 (s, 1H), 3.63 (s, 2H), 4.28 (s, 2H), 4.93 (s, 1H), 6.49 (s, 1H), 6.98 (s, 1H), 7.26 (d, *J* = 8.4 Hz, 1H), 7.35 (s, 1H), 7.46 (s, 2H), 7.14 (d, *J* = 8.4 Hz, 1H).

### Step 2

5-(1,2-Dihydroxy-3,3-dimethylbutyl)-7-[1-(tert-butoxycarbon yl)-5-(piperidinomethyl)indol-2-yl]isoindolinone (58.5 mg, 0.104 mmol) was added with 10% hydrogen chloride-methanol solution (1.2 mL), followed by stirring at 80 °C for 2 hours. The reaction mixture was condensed under reduced pressure. The residue was added with diisopropylether and ethyl acetate, and purified by slurry to obtain Compound 583 (51.0 mg, yield 98%).
ESI-MS *m*/*z*: 462 [M+H]⁺; ¹H-NMR (DMSO-d₆)δ(ppm): 1.00 (s, 9H), 1.28-1.45 (m, 1H), 1.62-1.89 (m, 5H), 2.78-2.95 (m, 2H), 3.24-3.35 (m, 2H), 4.32 (s, 2H), 4.44-4.54 (m, 3H), 4.90 (s, 1H), 5.22 (s, 1H), 7.30 (d, *J* = 8.4 Hz, 1H), 7.31 (s, 1H), 7.49 (s, 1H), 7.55 (d, *J* = 8.4 Hz, 1H), 7.79 (s, 1H), 8.17 (s, 1H), 9.24 (s, 1H), 9.89 (br s, 1H), 14.12 (s, 1H).

### Example 117:

### 5-Isobutyl-7-[1H-5-(piperidinomethyl)indol-2-yl)isoindolinone (Compound 584)

### Step 1

To a solution of 5-bromo-7-[1-(tert-butoxycarbonyl)-5-(piperidinomethyl)indol-2-y 1]isoindolinone (200 mg, 0.381 mmol) in 1,2-dimethoxyethane/water (5.0/0.27 mL), [bis(diphenylphosphino)ferrocene]dichloropalladium (134 mg, 0.164 mmol), isobutylboronic acid (311 mg, 3.05 mmol) and cesium carbonate (1.99 g, 6.10 mmol) was added, followed by stirring at 85 °C for 30 minutes under microwave irradiation. The reaction mixture was added with water, and extracted with ethyl acetate. The organic layer was condensed and the residue was purified by silica gel column chromatography (chloroform/methanol=99/1, 80/20) to obtain 5-isobutyl-7-[1-(tert-butoxycarbonyl)-5-(piperidinomethyl)indol-2-yl]isoindolinone (51.9 mg, yield 27%).
ESI-MS *m*/*z*: 502 [M+H]⁺

### Step 2

To a solution of 5-isobutyl-7-[1-(tert-butoxycarbonyl)-5-(piperidinomethyl)indol-2-yl]isoindolinone (51.9 mg, 0.103 mmol) in dichloromethane (1.3 mL), trifluoroacetic acid (260 µL) was added, followed by stirring at room temperature for 4.0 hours under nitrogen atmosphere. The reaction mixture was added into saturated aqueous sodium hydrogencarbonate solution, and extracted with ethyl acetate. The organic layer was condensed, and the residue was added with diisopropylether. The precipitated solid was collected by filtration to obtain Compound 584 (10.0 mg, yield 24%).
ESI-MS *m*/*z:* 402 [M+H]⁺; ¹H-NMR (DMSO-*d₆*)δ(ppm): 0.93 (d, *J* = 6.1 Hz, 3H), 1.04 (d, *J* = 6.1 Hz, 3H), 1.24 (m, 1H), 1.29-1.84 (m, 6H), 2.24-2.44 (m, 4H), 2.63 (d, *J* = 7.1 Hz, 2H), 3.51 (s, 2H), 4.48 (s, 2H), 7.05-7.34 (m, 3H), 7.31 (s, 1H), 7.51 (d, *J* = 7.8 Hz, 1H), 7.98 (s, 1H), 9.19 (s, 1H).

Compounds 585-595 were synthesized in a similar manner to Example 117 (Examples 118-128).

### Example 118:

### 5-Butyl-7-[1H-5-(piperidinomethyl)indol-2-yl)isoindolinone (Compound 585)

ESI-MS *m*/*z*: 402 [M+H]⁺; ¹H-NMR (DMSO-*d₆*)δ(ppm): 0.94 (t, *J* = 7. 3 Hz, 3H), 1.37 (m, 2H), 1.50-1.91 (m, 6H), 1.65 (m, 2H), 2.75 (m, 2H), 2.78-2.91 (m, 4H), 3.34 (s, 2H), 4.48 (s, 2H), 7.23 (m, 1H), 7.35 (s, 1H), 7.40-7.61 (m, 2H), 7.72 (m, 1H), 8.03 (s, 1H), 9.21 (s, 1H), 14.14 (s, 1H).

### Example 119:

### 5-(2-Phenetyl)-7-[1H-5-(piperidinomethyl)indol-2-yl)isoindolinon e (Compound 586)

ESI-MS *m*/*z*: 450 [M+H]⁺; ¹H-NMR (CDCl₃)δ(ppm): 1.35-1.71 (m, 6H), 2.44 (br s, 4H), 2.96-3.12 (m, 4H), 3.63 (s, 2H), 4.47 (s, 2H), 6.24 (s, 1H), 6.99 (s, 1H), 7.03-7.37 (m, 7H), 7.44 (d, J = 8.3 Hz, 1H), 7.54 (s, 1H), 7.87 (s, 1H), 13.38 (s, 1H).

### Example 120:

### 5-Isobutyl-7-[1H-5-(diethylaminomethyl)indol-2-yl)isoindolinone (Compound 587)

ESI-MS *m*/*z*: 390 [M+H]⁺; ¹H-NMR (CDCl₃)δ(ppm): 0.96 (s, 3H), 0.97 (s, 3H), 1.08 (t, *J* = 7.1 Hz, 6H), 1.24 (s, 2H), 1.98 (m, 1H), 2.59 (q, *J* = 7.1 Hz, 4H), 3.69 (s, 2H), 4.49 (s, 2H), 6.26 (s, 1H), 7.08 (d, *J* = 9.7 Hz, 2H), 7.18 (d, *J* = 8.3 Hz, 1H), 7.45 (d, *J* = 8.3 Hz, 1H), 7.54 (s, 1H), 7.87 (s, 1H), 13.37 (s, 1H).

### Example 121:

### 5-Methyl-7-[1H-5-(piperidinomethyl)indol-2-yl)isoindolinone (Compound 588)

ESI-MS *m*/*z:* 360 [M+H]⁺; ¹H-NMR (CDCl₃)δ(ppm): 1.37-1.73 (m, 6H), 1.56 (s, 3H), 2.32-2.67 (br s, 4H), 3.64 (br s, 2H), 4.48 (s, 2H), 6.24 (s, 1H), 7.06 (s, 1H), 7.13 (s, 1H), 7.17 (d, J = 8.4 Hz, 1H), 7.45 (d, *J* = 8.4 Hz, 1H), 7.56 (s, 1H), 7.91 (s, 1H), 13.42 (s, 1H).

### Example 122:

### 5-Cyclohexyl-7-[1H-5-(piperidinomethyl)indol-2-yl)isoindolinone (Compound 589)

ESI-MS *m*/*z*: 428 [M+H]⁺; ¹H-NMR (CDCl₃)δ(ppm): 1.20-1.75 (m, 7H), 1.93 (br s, 4H), 2.27-2.77 (m, 6H), 3.58 (s, 2H), 3.59 (br s, 4H), 4.49 (s, 2H), 6.66 (s, 1H), 7.07 (s, 1H), 7.16-7.32 (m, 2H), 7.44 (d, *J* = 8.1 Hz, 1H), 7.53 (s, 1H), 7.93 (s, 1H), 13.37 (s, 1H).

### Example 123:

### 5-(3-Hydroxy-1-propynyl)-7-[1H-5-(piperidinomethyl)indol-2-yl)is oindolinone (Compound 590)

ESI-MS *m*/*z*: 400 [M+H]⁺; ¹H-NMR (DMSO-d₆)δ(ppm): 1.33-1.58 (m, 6H), 2.29-2.40 (m, 4H), 3.48 (s, 2H), 4.38 (d, *J* = 5.8 Hz, 2H), 4.49 (s, 2H), 5.49 (t, *J* = 5.8 Hz, 1H), 7.09 (d, *J* = 8.3 Hz, 1H), 7.27 (s, 1H), 7.40 (d, *J* = 8.3 Hz, 1H), 7.44 (s, 1H), 7.51 (s, 1H), 8.14 (s, 1H), 9.37 (s, 1H), 13.81 (s, 1H).

### Example 124:

### 5-(4-Methylthiophen-3-yl)-7-[1H-5-(piperidinomethyl)indol-2-yl)i soindolinone (Compound 591)

ESI-MS *m*/*z*: 442 [M+H]⁺; ¹H-NMR (DMSO-*d₆*)δ(ppm): 1.27-1.76 (m, 6H), 2.23-2.46 (m, 4H), 2.41 (s, 3H), 3.31 (s, 2H), 4.55 (s, 2H), 7.14 (m, 1H), 7.33 (m, 1H), 7.38 (d, *J* = 3.2 Hz, 1H), 7.45 (m, 1H), 7.55 (s, 1H), 7.73 (s, 1H), 7.74 (d, *J* = 3.2 Hz, 1H), 8.16 (s, 1H), 9.29 (s, 1H), 13.91 (s, 1H).

### Example 125:

### 5-(2-Fluoropyridin-3-yl)-7-[1H-5-(piperidinomethyl)indol-2-yl)is oindolinone (Compound 592)

ESI-MS *m*/*z:* 441 [M+H]⁺; ¹H-NMR (CDCl₃)δ(ppm): 1.31-1.62 (m, 6H), 2.33 (br s, 4H), 3.48 (s, 2H), 4.58 (s, 2H), 7.10 (m, 1H), 7.33 (s, 1H), 7.38-7.50 (m, 2H), 7.58 (m, 1H), 7.72 (s, 1H), 8.28-8.35 (m, 3H), 9.38 (s, 1H), 13.81 (s, 1H).

### Example 126:

### 5-(2-Fluoropyridin-3-yl)-7-[1H-5-(diethylaminomethyl)indol-2-yl) isoindolinone (Compound 593)

ESI-MS *m*/*z*: 429 [M-H]⁻; ¹H-NMR (CDCl₃)δ(ppm): 1.10 (t, *J* = 6.6 Hz, 6H), 2.58 (q, *J* = 6.6 Hz, 4H), 3.70 (s, 2H), 4.60 (s, 2H), 6.43 (s, 1H), 7.11 (s, 1H), 7.22 (d, *J* = 8.4 Hz, 1H), 7.38 (t, *J* = 9.1 Hz, 1H), 7.47 (d, *J* = 8.4 Hz, 1H), 7.52 (s, 1H), 7.56 (s, 1H), 7.97-8.04 (m, 1H), 8.24 (s, 1H), 8.31 (m, 1H), 13.35 (s, 1H).

### Example 127:

### 5-(2-Fluoropyridin-3-yl)-7-{1H-5-[4-(2-hydroxyethyl)piperazin-1-ylmethyl]indo1-2-yl}isoindolinone (Compound 594)

ESI-MS *m*/*z*: 486 [M+H]⁺; ¹H-NMR (DMSO-*d₆*)δ(ppm): 2.23-2.57 (m, 8H), 3.43-3.55 (m, 6H), 4.36 (br s, 1H), 4.58 (s, 2H), 7.10 (d, *J* = 9.7 Hz, 1H), 7.33 (s, 1H), 7.42 (d, *J* = 9.7 Hz, 1H), 7.46 (s, 1H), 7.57 (m, 1H), 7.72 (s, 1H), 8.31 (m, 1H), 8.34 (s, 2H), 9.38 (s, 1H), 13.84 (s, 1H).

### Example 128:

### 5,7-Bis[1H-5-(piperidinomethyl)indol-2-yl]isoindolinone (Compound 595)

ESI-MS *m*/*z*: 558 [M+H]⁺; ¹H-NMR (CDCl₃)δ(ppm): 1.26-1.46 (m, 2H), 1.61-1.89 (m, 10H), 2.77-2.97 (m, 4H), 4.35 (br s, 4H), 4.61 (s, 2H), 7.27-7.46 (m, 3H), 7.56 (d, *J* = 9.0 Hz, 1H), 7.60 (d, *J* = 9.0 Hz, 1H), 7.65 (s, 1H), 7.81 (s, 1H), 7.86 (s, 1H), 8.05 (s, 1H), 8.72 (s, 1H), 9.38 (s, 1H), 12.23 (s, 1H), 14.19 (s, 1H).

### Example 129:

### 5-Isopropyl-7-[1H-5-(piperidinomethyl)indol-2-yl)isoindolinone (Compound 596)

### Step 1

To a solution of 5-isopropenyl-7-[1-(tert-butoxycarbonyl)-5-(piperidinomethyl)ind ol-2-yl]isoindolinone (95.0 mg, 0.196 mmol) in ethanol (3.0 mL), 10% palladium on carbon (30. 0 mg) was added, followed by stirring at room temperature for 4.5 hours under hydrogen atmosphere. The reaction mixture was filtered, and the filtrate was condensed to obtain 5-isopropyl-7-[1-(tert-butoxycarbonyl)-5-(piperidinomethyl)indol -2-yl]isoindolinone (89.0 mg, yield 93%).
H-NMR (DMSO-*d₆*)δ(ppm): 1.13 (s, 9H), 1.26 (s, 3H), 1.29 (s, 3H), 1.33-1.57 (m, 6H), 2.29-2.43 (m, 4H), 3.05 (m, 1H), 3.51 (s, 2H), 4.35 (s, 2H), 6.59 (s, 1H), 7.25 (d, *J* = 8.4 Hz, 1H), 7.31 (s, 1H), 7.47 (s, 1H), 7.48 (d, *J* = 6.8 Hz, 1H), 8.08 (d, *J* = 8.4 Hz, 1H), 8.47 (s, 1H).

### Step 2

To a solution of 5-isopropyl-7-[1-(tert-butoxycarbonyl)-5-(piperidinomethyl)indol -2-yl]isoindolinone (89.0 mg, 0.183 mmol) in dichloromethane (2.2 mL), trifluoroacetic acid (445 µL) was added, followed by stirring at room temperature for 4.0 hours under nitrogen atmosphere. The reaction mixture was added to a saturated aqueous sodium hydrogencarbonate solution, and extracted with ethyl acetate. The organic layer was condensed, and the residue was added with isopropylether. The precipitated solid was collected by filtration to obtain Compound 596 (65.8 mg, yield 93%).
ESI-MS *m*/*z:* 388 [M+H]⁺; ¹H-NMR (DMSO- *d₆*)δ(ppm): 1.26 (s, 3H), 1.29 (s, 3H), 1.34-1.71 (m, 6H), 2.24-2.44 (m, 4H), 3.09 (m, 1H), 3.51 (s, 2H), 4.48 (s, 2H), 7.13 (s, 1H), 7.26 (s, 1H), 7.35-7.58 (m, 3H), 8.02 (s, 1H), 9.17 (s, 1H), 13.91 (s, 1H).

Compounds 597 and 598 were synthesized in a similar manner to Example 129 (Examples 130 and 131).

### Example 130:

### 5-(3,3-Dimethylbutyl)-7-[1H-5-(piperidinomethyl)indol-2-yl)isoin dolinone (Compound 597)

ESI-MS *m*/*z*: 430 [M+H]⁺; ¹H-NMR (DMSO-*d₆*)δ(ppm): 1.02 (s, 9H), 1.31-1.60 (m, 8H), 2.34 (br s, 4H), 2.65-2.78 (m, 2H), 3.47 (s, 2H), 4.46 (s, 2H), 7.07 (d, *J* = 8.1 Hz, 1H), 7.22 (s, 1H), 7.32 (s, 1H), 7.38 (d, *J* = 8.1 Hz, 1H), 7.44 (s, 1H), 8.00 (s, 1H), 9.14 (s, 1H), 13.86 (s, 1H).

### Example 131:

### 5-Propyl-7-[1H-5-(piperidinomethyl)indol-2-yl)isoindolinone (Compound 598)

ESI-MS m/z: 388 [M+H]⁺; ¹H-NMR (CDCl₃)δ(ppm): 0.95 (t, *J* = 7.3 Hz, 3H), 1.04 (d, *J* = 7.3 Hz, 2H), 1.29-1.61 (m, 6H), 2:36 (br s, 4H), 2.75 (d, *J* = 7.3 Hz, 2H), 3.49 (s, 2H), 4.51 (s, 2H), 7.08 (d, *J* = 7.9 Hz, 1H), 7.19 (s, 1H), 7.28-7.52 (m, 2H), 7.65 (d, *J* = 7.8 Hz, 1H), 8.16 (d, *J* = 7.8 Hz, 1H), 9.27 (s, 1H), 13.87 (s, 1H).

### Example 132:

### 5-(1-Hydroxy-2-methyl-2-propenyl)-7-[1H-5-(piperidinomethyl)indo 1-2-yl)isoindolinone (Compound 599)

### Step 1

In a similar manner to Step 5 of Example 6, 5-(dimethoxymethyl)-3-hydroxy-7-iodo-2-(1-methyl-1-phenylethyl)i soindolinone (3.01 g, 6.44 mmol) was dissolved in acetonitrile (28 mL), and the solution was treated with 1 mol/L hydrochloric acid (4 mL) to obtain 5-formyl-3-hydroxy-7-iodo-2-(1-methyl-1-phenylethyl)isoindolinon e (3.10 g).

### Step 2

In a similar manner to Step 2 of Example 24, 5-formyl-3-hydroxy-7-iodo-2-(1-methyl-1-phenylethyl)isoindolinon e (3.10 g) was dissolved in THF (100 mL), and the solution was treated with isopropenylmagnesiumbromide-THF solution (0.50 mol/L, 0.39 mL, 20 mmol), followed by purification by flush column chromatography (hexane/ethyl acetate=90/10, 70/30) to obtain 5-(1-hydroxy-2-methyl-2-propenyl)-3-hydroxy-7-iodo-2-(1-methyl-1 -phenylethyl)isoindolinone (2.10 g, yield 70%, 2 steps).
ESI-MS *m*/*z:* 464 [M+H]⁺

### Step 3

In a similar manner to Step 5 of Example 1, 5-(1-hydroxy-2-methyl-2-propenyl)-3-hydroxy-7-iodo-2-(1-methyl-1 -phenylethyl)isoindolinone (2.10 g, 4.54 mmol) was dissolved in nitromethane (35 mL), and the solution was treated with trifluoroacetic acid (1.05 mL, 13.5 mmol) and triethylsilane (2.20 mL, 13.5 mmol) to obtain 5-(1-hydroxy-2-methyl-2-propenyl)-7-iodo-2-(1-methyl-1-phenyleth yl)isoindolinone (2.34 g).

### Step 4

5-(1-Hydroxy-2-methyl-2-propenyl)-7-iodo-2-(1-methyl-1-phen ylethyl) isoindolinone (2.34 g) was dissolved in dichloromethane (20 mL), and the solution was added with trifluoroacetic acid (3.50 mL, 454 mmol) followed by stirring at room temperature for 4 hours. The reaction mixture was added with saturated aqueous sodium hydrogencarbonate solution, and extracted with ethyl acetate. The organic layer was washed with saturated brine and dried over anhydrous sodium sulfate. The solvent was evaporated under reduced pressure. The residue was purified by flush column chromatography (hexane/ethyl acetate=70/30, 50/50) to obtain 5-(1-hydroxy-2-methyl-2-propenyl)-7-iodoisoindolinone (969 mg, yield 65%, 2 steps).
ESI-MS *m*/*z:* 330 [M+H]⁺; ¹H-NMR (DMSO-*d*₆)δ(ppm): 1.49 (s, 3H), 4.25 (s, 2H), 4.86 (s, 1H), 5.08 (s, 1H), 5.14 (s, 1H), 5.74 (d, *J* = 2.4 Hz, 1H), 7.50 (s, 1H), 7.81 (s, 1H), 8.67 (s, 1H).

### Step 5

In a similar manner to Step 7 of Example 1, 5-(1-hydroxy-2-methyl-2-propenyl)-7-iodoisoindolinone (67.1 mg, 0.204 mmol) was dissolved in acetonitrile (4 mL), and the solution was treated with Compound BD (146 mg, 0.408 mmol), palladium acetate (3.7 mg, 0.020 mmol), tri (o-tolyl)phosphine (9.9 mg, 0.030 mmol) and triethylamine (0.284 mL, 2.04 mmol), followed by purification by preparative thin-layer chromatography (chloroform/methanol=9/1) to obtain 5-(1-hydroxy-2-methyl-2-propenyl)-7-[1-(tert-butoxycarbonyl)-5-( piperidinomethyl)indol-2-yl]isoindolinone (112 mg, quantitative yield).
ESI-MS *m*/*z*: 516 [M+H]⁺; ¹H-NMR (CDCl₃)δ(ppm): 1.28 (s, 9H), 1.44 (m, 1H), 1.47-1.80 (m, 5H), 1.65 (s, 3H), 2.30-2.50 (m, 4H), 3.59 (s, 2H), 4.41 (s, 2H), 5.00 (s, 1H), 5.24 (s, 1H), 5.28 (s, 1H), 6.52 (s, 1H), 6.65 (br s, 1H), 7.27 (dd, *J* = 1.5, 8.6 Hz, 1H), 7.43 (s, 1H), 7.48 (s, 1H), 7.52 (s, 1H), 8.18 (d, *J* = 8.6 Hz, 1H).

### Step 6

In a similar manner to Step 8 of Example 1, 5-(1-hydroxy-2-methyl-2-propenyl)-7-[1-(tert-butoxycarbonyl)-5-( piperidinomethyl)indol-2-yl]isoindolinone (110 mg, 0.213 mmol) was treated with 10% hydrogen chloride-methanol solution (2 mL) and methanol (2 mL), followed by purification by preparative thin-layer chromatography (chloroform/methanol=4/1) to obtain Compound 599 (23.4 mg, yield 26%).
ESI-MS *m*/*z:* 416 [M+H]⁺; ¹H-NMR (DMSO-*d*₆)δ(ppm): 1.31-1.61 (m, 6H), 1.57 (s, 3H), 2.20-2.50 (m, 4H), 3.45-3.55 (m, 2H), 4.49 (s, 2H), 4.90 (s, 1H), 5.19 (d, *J* = 4.1 Hz, 1H), 5.21 (s, 1H), 5.76 (d, *J* = 4.0 Hz, 1H), 7.11 (s, 1H), 7.37 (s, 1H), 7.40 (d, *J* = 8.4 Hz, 1H), 7.42 (s, 1H), 7.46 (s, 1H), 8.08 (s, 1H), 9.21 (s, 1H), 13.88 (s, 1H).

Compounds 600 and 601 were synthesized in a similar manner to Example 132 (Examples 133 and 134).

### Example 133:

### 5-(1-Hydroxy-2-methylpropyl)-7-{1H-5-[4-(2-hydroxyethyl)piperazi n-1-ylmethyl]indol-2-yl}isoindolinone dihydrocholoride (Compound 600)

ESI-MS *m*/*z:* 463 [M+H]⁺; ¹H-NMR (DMSO-*d*₆)δ(ppm): 0.84 (d, *J* = 6. 8 Hz, 3H), 0.89 (d, *J* = 6.8 Hz, 3H), 1.92 (m, 1H), 2.90-3.89 (m, 12H), 4.40 (m, 1H), 4.45 (d, *J* = 5.6 Hz, 1H), 4.50 (s, 2H), 5.31 (m, 1H), 7.28 (s, 1H), 7.30 (m, 1H), 7.44 (s, 1H), 7.55 (d, *J* = 7.9 Hz, 1H), 7.79 (br s, 1H), 8.08 (s, 1H), 9.24 (s, 1H), 10.90 (br s, 1H), 11.50 (br s, 1H), 14.12 (br s, 1H).

### Example 134:

### 5-(1-Hydroxy-2-methylpropyl)-7-[1H-5-(ethylaminomethyl)indol-2-y l}isoindolinone (Compound 601)

ESI-MS *m*/*z*: 378 [M+H]⁺; ¹H-NMR (DMSO-*d*₆)δ(ppm): 0.83 (d, *J* = 6.7 Hz, 3H), 0.88 (d, *J* = 6.7 Hz, 3H), 1.21 (d, *J* = 7.3 Hz, 3H), 1.90 (m, 1H), 2.85-2.97 (m, 2H), 4.10-4.21 (m, 2H), 4.44 (d, *J* = 5.8 Hz, 1H), 4.49 (s, 2H), 5.37 (br s, 1H), 7.25 (d, *J* = 8.3 Hz, 1H), 7.26 (s, 1H), 7.43 (s, 1H), 7.52 (d, *J* = 8.3 Hz, 1H), 7.72 (s, 1H), 8.07 (s, 1H), 8.79-9.00 (m, 2H), 9.24 (s, 1H), 14.10 (s, 1H).

### Example 135:

### 5-(3-Hydroxypropyl)-7-[1H-5-(piperidinomethyl)indol-2-yl)isoindo linone (Compound 602)

### Step 1

To a solution of triethyl phosphonoacetate (126 µL, 0.633 mmol) in THF (2.5 mL), potassium tert-butoxide (47.4 mg, 0.422 mmol) was added, followed by stirring at 0 °C for 1.0 hour under nitrogen atmosphere. The reaction mixture was added with 5-formyl-7-[1-(*tert*-butoxycarbonyl)-5-(piperidinomethyl)indol-2-yl]isoindolinone (100 mg, 0.211 mmol), followed by additional stirring for 1.0 hour. The reaction mixture was added with water, and extracted with ethyl acetate. The organic layer was condensed, and the residue was purified by silica gel column chromatography (chloroform/methanol=99/1, 80/20) to obtain (*E*)-5-(ethoxycarbonylethenyl)-7-[1-(*tert*-butoxycarbonyl)-5-(pipe ridinomethyl)indol-2-yl]isoindolinone (85.4 mg, yield 74%).
ESI-MS *m*/*z*: 544 [M+H]⁺

### Step 2

To a solution of (*E*)-5-(ethoxycarbonylethenyl)-7-[1-(*tert*-butoxycarbonyl)-5-(pipe ridinomethyl)indol-2-yl]isoindolinone (85.4 mg, 0.157 mmol) in dichloromethane (2.1 mL), diisobutylaluminum hydride-toluene solution (1.01 mol/L, 544 µL, 0.550 mmol) was added dropwise at -78 °C under nitrogen atmosphere, followed by stirring at -78 °C for 30 minutes and 0 °C for 30 minutes. The reaction mixture was added with water, and extracted with ethyl acetate. The organic layer was condensed, and the residue was added with diisopropyl ether. The precipitated solid was collected by filtration to obtain (*E*)-5-(3-hydroxypropenyl)-7-[1-(*tert*-butoxycarbonyl)-5-(piperidi nomethyl)indol-2-yl]isoindolinone (40.8 mg, yield 52%).
ESI-MS *m*/*z*: 502 [M+H]⁺

### Step 3

To a solution of (*E*)-5-(3-hydroxypropenyl)-7-[1-(*tert*-butoxycarbonyl)-5-(piperidi nomethyl)indol-2-yl]isoindolinone (49.1 mg, 0.0979 mmol) in methanol (1.6 mL), 10% palladium on carbon (15.7 mg) was added, followed by stirring at room temperature for 4.5 hours under hydrogen atmosphere. The reaction mixture was filtered, and the filtrate was condensed to obtain 5-(3-hydroxypropyl)-7-[1-(*tert*-butoxycarbonyl)-5-(piperidinometh yl)indol-2-yl]isoindolinone (43.6 mg, yield 88%). ESI-MS *m*/*z:* 504 [M+H]⁺

### Step 4

To a solution of 5-(3-hydroxypropyl)-7-[1-(*tert*-butoxycarbonyl)-5-(piperidinometh yl)indol-2-yl]isoindolinone (43.6 mg, 0.0866 mmol) in dichloromethane (1.1 mL), trifluoroacetic acid (218 µL) was added, followed by stirring at room temperaure for 4.0 hours under nitrogen atmosphere. The reaction mixture was added with saturated aqueous sodium hydrogencarbonte solution, and extracted with ethyl acetate. The organic layer was condensed, and the residue was added with diisopropyl ether. The precipitated solid was collected by filtration to obtain Compound 602 (3.0 mg, yield 9%).
ESI-MS *m*/*z*: 404 [M+H]⁺; ¹H-NMR (DMSO-*d₆*)δ(ppm): 1.31-1.51 (m, 6H), 1.81 (t, *J* = 7.5 Hz, 2H), 2.34 (br s, 4H), 2.78 (t, *J* = 7.5 Hz, 2H), 3.47 (t, *J* = 7.5 Hz, 2H), 3.49 (s, 2H), 4.47 (s, 2H), 4.54 (br s, 1H), 7.07 (d, *J* = 8.4 Hz, 1H), 7.19 (s, 1H), 7.32 (s, 1H), 7.38 (d, *J* = 8.4 Hz, 1H), 7.44 (s, 1H), 7.99 (s, 1H), 9.15 (s, 1H), 13.87 (s, 1H).

### Example 136:

### 4-Chloro-5-(2-propyloxy)-7-{1H-5-[4-(2-hydroxyethyl)piperazin-1-ylmethyl]indol-2-yl}isoindolinone dihydrochloride (Compound 603)

### Step 1

In a similar manner to Step 1 of Example 3, 4-chloro-5-hydroxy-7-iodoisoindolinone (200 mg, 0.646 mmol) was dissolved in DMF (2.5 mL), and the solution was treated with potassium carbonate (179 mg, 1.29 mmol) and isopropyl bromide (0.121 mL, 1.29 mmol), followed by adding with water. The precipitated solid was collected by filtration to obtain 4-chloro-5-(2-propyloxy)-7-iodoisoindolinone (198 mg, yield 87%).
ESI-MS *m*/*z*: 352 [M+H]⁺; ¹H-NMR (CDCl₃)δ(ppm): 1.42 (d, *J* = 6.1 Hz, 3H), 1.43 (d, *J* = 6.1 Hz, 3H), 4.29 (s, 2H), 4.68 (m, 1H), 6.51 (br s, 1H), 7.43 (s, 1H),.

### Step 2

In a similar manner to Step 7 of Example 1, 4-chloro-5-(2-propyloxy)-7-iodoisoindolinone (50 mg, 0.142 mmol) was dissolved in acetonitrile (3 mL), and the solution was treated with Compound BA (82.1 mg, 0.284 mmol), palladium acetate (2.6 mg, 0.012 mmol), tri(o-tolyl)phosphine (6.9 mg, 0.023 mmol) and triethylamine (0.198 mL, 1.42 mmol), followed by purification by flush column chromatography (chloroform/methanol=95/5) to obtain 4-chloro-5-(2-propyloxy)-7-[1-(tert-butoxycarbonyl)-5-formylindo l-2-yl]isoindolinone (52.2 mg, yield 78%).
ESI-MS *m*/*z*: 469 [M+H]⁺; ¹H-NMR (CDCl₃)δ(ppm): 1.37 (s, 9H), 1.45 (d, *J* = 6.1 Hz, 6H), 4.39 (s, 2H), 4.73 (m, 1H), 6.49 (br s, 1H), 6.69 (s, 1H), 7.07 (s, 1H), 7.88 (dd, *J* = 1.6, 8.7 Hz, 1H), 8.10 (d, *J* = 1.6 Hz, 1H), 8.36 (d, *J* = 8.7 Hz, 1H), 10.07 (s, 1H).

### Step 3

In a similar manner to Step 2 of Example 45, 4-chloro-5-(2-propyloxy)-7-[1-(tert-butoxycarbonyl)-5-formylindo l-2-yl]isoindolinone (50.0 mg, 0.107 mmol) was dissolved in acetonitrile (3 mL), and the solution was treated with 1-piperazineethanol (0.131 mL, 1.07 mmol), acetic acid (0.123 mL, 2.14 mmol) and sodium triacetoxyborohydride (68 mg, 0.32 mmol), followed by purification by preparative thin-layer chromatography (chloroform/methanol=9/1) to obtain 4-chloro-5-(2-propyloxy)-7-{1-(tert-butoxycarbonyl)-5-[4-(2-hydr oxyethyl)piperazin-1-ylmethyl]indol-2-yl}isoindolinone (52.4 mg, yield 84%).
ESI-MS *m*/*z*: 583 [M+H]⁺; ¹H-NMR (CDCl₃)δ(ppm): 1.38 (s, 9H), 1.44 (d, *J* = 5.9 Hz, 6H), 2.40-2.70 (m , 10H), 3.55-3.65 (m, 2H), 3.61 (s, 2H), 4.38 (s, 2H), 4.71 (m, 1H), 6.29 (br s, 1H), 6.55 (s, 1H), 7.04 (s, 1H), 7.29 (dd, *J* = 1.7, 8.8 Hz, 1H), 7.48 (s, 1H), 8.15 (d, *J* = 8.8 Hz, 1H).

### Step 4

In a similar manner to Step 8 of Example 1, 4-chloro-5-(2-propyloxy)-7-{1-(tert-butoxycarbonyl)-5-[4-(2-hydr oxyethyl)piperazin-1-ylmethyl]indol-2-yl}isoindolinone (50.0 mg, 0.0857 mmol) was treated with 10% hydrogen chloride-methanol solution (2 mL) and methanol (2 mL). The precipitated solid was collected by filtration to obtain Compound 603 (40.6 mg, yield 85%).
ESI-MS *m*/*z*: 483 [M+H]⁺; ¹H-NMR (DMSO-*d*₆)δ(ppm): 1.38 (d, *J* = 5. 9 Hz, 6H), 3.05-3.84 (m, 12H), 4.46 (s, 4H), 5.13 (m, 1H), 7.35 (d, *J* = 8.4 Hz, 1H), 7.51 (s, 1H), 7.57 (d, *J* = 8.3 Hz, 1H), 7.85 (s, 2H), 9.31 (s, 1H), 10.90 (m, 1H), 11.25 (m, 1H), 14.03 (s, 1H).

Compounds 604 and 605 were synthesized in a similar manner to Example 136 (Examples 137 and 138).

### Example 137:

### 4-Chloro-5-isopropoxy-7-[1H-5-(piperidinomethyl)indol-2-yl]isoin dolinone hydrochloride (Compound 604)

ESI-MS *m*/*z*: 438 [M+H]⁺; ¹H-NMR (DMSO-*d*₆)δ(ppm): 1.38 (d, *J* = 6.2 Hz, 6H), 1.62-1.84 (m, 6H), 2.76-2.93 (m, 2H), 3.27-3.41 (m, 2H), 4.32 (s, 1H), 4.33 (s, 1H), 4.45 (s, 2H), 5.08-5.17 (m, 1H), 7.32 (d, *J* = 8.8 Hz, 1H), 7.50 (s, 1H), 7.57 (d, *J* = 8.8 Hz, 1H), 7.80 (s, 1H), 7.84 (s, 1H), 8.31 (s, 1H), 9.96 (br s, 1H), 14.02 (s, 1H).

### Example 138:

### 4-Chloro-5-isopropoxy-7-[1H-5-(diethylaminomethyl)indol-2-yl]iso indolinone hydrochloride (Compound 605)

ESI-MS *m*/*z*: 427 [M+H]⁺; ¹H-NMR (DMSO-*d*₆)δ(ppm): 1.26 (t, *J* = 7.3 Hz, 6H), 1.38 (d, *J* = 6.1 Hz, 6H), 3.03-3.10 (m, 4H), 4.35 (s, 1H), 4.37 (s, 1H), 4.45 (s, 2H), 5.08-5.17 (m, 1H), 7.37 (d, *J* = 8.6 Hz, 1H), 7.49 (s, 1H), 7.57 (d, *J* = 8.6 Hz, 1H), 7.81 (s, 1H), 7.84 (s, 1H), 8.31 (s, 1H), 9.92 (br s, 1H), 14.02 (s, 1H).

### Example 139:

### 4-Chloro-5-amino-7-[1H-5-(piperidinomethyl)indol-2-yl]isoindolin one hydrochloride (Compound 606)

### Step 1

5-Amino-7-iodoisoindolinone (400 mg, 1.46 mmol) was dissolved in 2-propanol (12 mL), and the solution was added with NCS (220 mg, 1.65 mmol), followed by stirring at room temperature for 40 hours. The reaction mixture was added with water, and extracted with ethyl acetate. The organic layer was washed with saturated brine and dried over anhydrous sodium sulfate. The solvent was evaporated under reduced pressure, and the residue was added with methanol. The precipitated solid was filtered off. The filtrate was condensed under reduced pressure, and the residue was added with methanol. The precipitated solid was collected by filtration and washed with methanol, followed by drying under reduced pressure to obtain 4-chloro-5-amino-7-iodoisoindolinone (142 mg, yield 32%).
ESIMS *m*/*z:* 309 [M+H]⁺ ; ¹H-NMR (DMSO-D₆) δ: 4.12 (s, 2H), 6.15 (br s, 2H), 7.28 (s, 1H), 8.33 (br s, 1H).

### Step 2

In a similar manner to Step 5 of Example 79, 4-chloro-5-amino-7-iodoisoindolinone (60.0 mg, 0.194 mmol) was treated with Compound BD (139 mg, 0.388 mmol), triethylamine (0.135 mL, 0.969 mmol), [bis(diphenylphosphino)ferrocene]dichloropalladium (16.0 mg, 0.0196 mmol) and DMA (1 mL)/water (0.140 mL), followed by purification by preparative thin-layer chromatography (chloroform/methanol=5/1) to obtain 4-chloro-5-amino-7-[1-(*tert*-butoxycarbonyl)-5-(piperidinomethyl) indol-2-yl]isoindolinone (74.6 mg, yield 78%).
ESI-MS *m*/*z*: 495 [M+H]⁺; ¹H-NMR (CDCl₃)δ(ppm): 1.37-1.67 (m, 6H), 1.41 (s, 9H), 2.35-2.47 (m, 4H), 3.58 (s, 2H), 4.34 (s, 2H), 4.45 (br s, 2H), 6.00 (br s, 1H), 6.52 (s, 1H), 6.85 (s, 1H), 7.27 (dd, *J* = 1.2, 8.4 Hz, 1H), 7.47 (d, *J* = 1.2 Hz, 1H), 8.11 (d, *J* = 8.4 Hz, 1H).

### Step 3

In a similar manner to Step 8 of Example 1, 4-chloro-5-amino-7-[1-(*tert*-butoxycarbonyl)-5-(piperidinomethyl) indol-2-yl]isoindolinone (30.0 mg, 0.0606 mmol) was treated with 10% hydrogen chloride-methanol solution (2 mL) and methanol (2 mL). The precipitated solid was collected by filtration to obtain Compound 606 (15.8 mg, yield 61%).
ESI-MS *m*/*z*: 395 [M+H]⁺; ¹H-NMR (DMSO-*d*₆)δ(ppm): 1.37 (m, 1H), 1.60-1.87 (m, 5H), 2.78-2.94 (m, 2H), 3.27-3.40 (m, 2H), 4.31 (d, *J* = 4.8 Hz, 2H), 4.37 (s, 2H), 6.98 (s, 1H), 7.28 (dd, *J* = 1.0, 8.5 Hz, 1H), 7.54 (d, *J* = 8.5 Hz, 1H), 7.57 (s, 1H), 7.76 (d, *J* = 1.0 Hz, 1H), 8.96 (s, 1H), 9.73 (br s, 1H), 14.17 (s, 1H).

Compounds 607 to 612 were synthesized in a similar manner to Example 139 (Examples 140 to 145).

### Example 140:

### 4-Chloro-5-acetylamino-7-[1H-5-(piperidinomethyl)indol-2-yl]isoi ndolinone (Compound 607)

ESI-MS *m*/*z*: 437 [M+H]⁺; ¹H-NMR (DMSO-*d*₆)δ(ppm): 1.30-1.68 (m, 6H), 2.20 (s, 3H), 2.30-2.64 (m, 4H), 4.50 (s, 2H), 7.04-7.22 (m, 2H), 7.39-7.63 (m, 2H), 8.57 (s, 1H), 9.43 (s, 1H), 9.86 (s, 1H), 13.76 (br s, 1H).

### Example 141:

### 4-Chloro-5-(2-propylamino)-7-[1H-5-(piperidinomethyl)indol-2-yl] isoindolinone hydrochloride (Compound 608)

ESI-MS *m*/*z*: 437 [M+H]⁺; ¹H-NMR (DMSO-*d*₆)δ(ppm): 1.28 (d, *J* = 6.8 Hz, 6H), 1.26 (m, 1H), 1.60-1.87 (m, 5H), 2.78-2.96 (m, 2H), 3.26-3.42 (m, 2H), 4.10 (m, 1H), 4.32 (d, *J* = 5.0 Hz, 2H), 4.38 (s, 2H), 5.55 (d, *J* = 8.1 Hz, 1H), 7.27 (dd, *J* = 1.2, 8.3 Hz, 1H), 7.36 (d, *J* = 1.6 Hz, 1H), 7.40 (s, 1H), 7.54 (d, *J* = 8.3 Hz, 1H), 7.75 (s, 1H), 8.96 (s, 1H), 9.68 (br s, 1H), 14.15 (s, 1H).

### Example 142:

### 4-Chloro-5-dimethylamino-7-{1H-5-[4-(2-hydroxyethyl)piperazin-1-ylmethyl]indol-2-yl]isoindolinone dihydrochloride (Compound 609)

ESI-MS *m*/*z*: 468 [M+H]⁺; ¹H-NMR (DMSO-*d*₆)δ(ppm): 2.96 (s, 6H), 3.11-4.31 (m, 12H), 4.44 (s, 2H), 4.49 (br s, 2H), 7.36 (dd, *J* = 1.7, 8.4 Hz, 1H), 7.39 (d, *J* = 1.7 Hz, 1H), 7.57 (d, *J* = 8.4 Hz, 1H), 7.75 (s, 1H), 7.86 (s, 1H), 9.25 (s, 1H), 11.09 (br s, 1H), 11. 91 (br s, 1H), 13.98 (s, 1H).

### Example 143:

### 4-Chloro-5-dimethylaminomethyl-7-[1H-5-(diethylaminomethyl)indol -2-yl]isoindolinone hydrochloride (Compound 610)

Melting point 224-226 °C; ESI-MS m/z: 425 [M+H]⁺; ¹H-NMR (DMSO-*d*₆)δ(ppm): 1.27 (t, *J* = 7.2 Hz, 6H), 2.84 (s, 6H), 3.08 (q, *J* = 7.2 Hz, 4H), 4.31-4.41 (m, 2H), 4.53 (s, 2H), 4.62 (s, 2H), 7.38 (d, *J* = 7.8 Hz, 1H), 7.59 (d, *J* = 7.8 Hz, 1H), 7.60 (s, 1H), 7.86 (s, 1H), 8.91 (s, 1H), 9.68 (s, 1H), 10.1 (br s, 1H), 11.5 (br s, 1H), 13.78 (s, 1H).

### Example 144:

### 4-Chloro-5-dimethylaminomethyl-7-{1H-5-[4-(2-hydroxyethyl)pipera zin-1-ylmethyl]indol-2-yl}isoindolinone hydrochloride (Compound 611)

ESI-MS *m*/*z:* 482 [M+H]⁺; ¹H-NMR(DMSO-*d*₆)δ(ppm): 2.89 (s, 6H), 3.21-3.61 (m, 8H), 3.78-3.81 (m, 2H), 4.38-4.62 (m, 6H), 6.63 (s, 1H), 7.52-7.98 (m, 4H), 8.76 (s, 1H), 9.26 (s, 1H), 11.15-11.34 (br s, 2H), 13.79 (s,1H).

### Example 145:

### 4-Chloro-5-(N-methyl-2-propylamino)-7-{1H-5-[4-(2-hydroxyethyl)p iperazin-1-ylmethyl]indol-2-yl}isoindolinone dihydrochloride (Compound 612)

ESI-MS *m*/*z:* 496 [M+H]⁺; ¹H-NMR (DMSO-*d*₆)δ(ppm) : 1.17 (d, *J* = 6.6 Hz, 6H), 2.81 (s, 3H), 3.17-3.30 (m, 2H), 3.30-3.90 (m, 10H), 3.84 (sep, *J* = 6.6 Hz, 1H), 4.44 (s, 2H), 4.47 (br s, 2H), 7.37 (dd, *J* = 1.2, 8.6 Hz, 1H), 7.40 (d, *J* = 1.1 Hz, 1H), 7.57 (d, *J* = 8.6 Hz, 1H), 7.77 (s, 1H), 7.87 (s, 1H), 9.27 (s, 1H), 11.20 (br s, 1H), 11.90 (br s, 1H), 13.99 (s, 1H).

### Example 146:

### 4-Chloro-5-(1-hydroxy-2-methylpropyl)-7-[1H-5-(diethylaminomethy 1)indol-2-yl]isoindolinone hydrochloride (Compound 613)

### Step 1

3-Chloro-4-formyl-*N*-(1-methyl-1-phenylethyl)benzamide (0.59 g, 1.76 mmol) was dissolved in THF (12 mL), and isopropenylmagnesium bromide-THF solution (0.5 mol/L, 11.7 mL, 5.87 mmol) was added dropwise thereto at -78 °C for 10 minutes under argon atmosphere, then heated to -40 °C for 1.5 hours. The reaction mixture was added with acetic acid (0.336 mL) and water, and extracted with ethyl acetate. The organic layer was washed with saturated brine and dried over anhydrous sodium sulfate. The solvent was evaporated under reduced pressure, and the residue was purified by flush column chromatography (hexane/ethyl acetate=95/5, 60/40) to obtain 3-chloro-4-(1-hydroxy-2-methylallyl)-*N*-(1-methyl-1-phenylethyl)b enzamide (0.596 g, yield 88%).
ESI-MS *m*/*z:* 344 [M+H]⁺; ¹H-NMR (CDCl₃)δ(ppm): 1.67 (s, 3H), 1.82 (s, 6H), 4.98-5.21 (m, 2H), 5.56 (s, 2H), 6.37 (s, 1H), 7.22-7.51 (m, 6H), 7.60-7.77 (m, 2H).

### Step 2

3-Chloro-4-(1-hydroxy-2-methylallyl)-*N*-(1-methyl-1-phenylet hyl)benzamide (1.02 g, 2.97 mmol) was dissolved in ethanol (10 mL), and the solution was added with palladium-active chacoal ethylenediamine complex (Pd 3.5-6.5%, 100 mg), followed by stirring at room temperature for 1 hour under hydrogen atmosphere. The reaction mixture was filtered to obtain 3-chloro-4-(1-hydroxy-2-methylpropyl)-*N*-(1-methyl-1-phenylethyl) benzamide (0.99 g, yield 96%).
ESI-MS *m*/*z*: 346 [M+H]⁺; ¹H-NMR (CDCl₃)δ(ppm) : 0.85-0.95 (m, 6H), 1.85 (s, 6H), 2.14-2.18 (m, 1H), 4.85-4.95 (m, 1H), 6.48 (s, 1H), 7.58-7.75 (m, 8H).

### Step 3

3-Chloro-4-(1-hydroxy-2-methylpropyl)-*N*-(1-methyl-1-phenyle thyl)benzamide (1.30 g, 3.78 mmol) was dissolved in acetonitrile (26 mL), and the solution was added with imidazole (0.566 g, 8.32 mmol) and tert-butyldimethylsilyl chloride (1.14 g, 7.56 mmol), followed by stirring at room temperature for 12 hours. The reaction mixture was added with water, and extracted with ethyl acetate. The organic layer was washed with saturated brine, and dried over anhydrous sodium sulfate. The solvent was evaporated under reduced pressure, and the residue was purified by flush column chromatography (hexane/ethyl acetate=99/1, 90/10) to obtain 3-chloro-4-[1-(tert-butyldimethylsilyloxy)-2-methylpropyl]-*N*-(1-methyl-1-phenylethyl)benzamide (1.25 g, yield 72%).
ESI-MS *m*/*z*: 461 [M+H]⁺; ¹H-NMR (CDCl₃)δ(ppm) : 0.23 (s, 3H), 0.28 (s, 3H), 1.09 (d, *J* = 6.7 Hz, 3H), 1.12 (s, 9H), 1.30 (d, *J* = 6.9 Hz, 3H), 2.06 (s, 6H), 2.08-2.17 (m, 1H), 5.10 (d, *J* = 5.2 Hz, 1H), 6.57 (s, 1H), 7.44-7.87 (m, 7H), 7.94 (d, *J* = 1.8 Hz, 1H).

### Step 4

3-Chloro-4-[1-(*tert*-butyldimethylsilyloxy)-2-methylpropyl]-*N*-(1-methyl-1-phenylethyl)benzamide (1.25 g, 2.72 mmol) was dissolved in THF (25 mL), and the solution was added with TMEDA (1.31 mL, 8.69 mmol), then sec-butyllithium-hexane solution (1.00 mol/L, 8.69 mL, 8.69 mmol) was added dropwise at -78 °C for 20 minutes under argon atmosphere, followed by stirring at same temperature for 1 hour. The mixture was added with DMF (0.464 mL, 5. 98 mmol) , and heated from -78 °C to room temperature for 1 hour. The reaction mixture was added with water and acetic acid (1.50mL), and extracted with ethyl acetate. The organic layer was washed with saturated brine, and dried over anhydrous sodium sulfate. The solvent was evaporated under reduced pressure, and the residue was purified by flush column chromatography (hexane/ethyl acetate=95/5, 75/25) to obtain 4-chloro-5-[1-(tert-butyldimethylsilyloxy)-2-methylpropyl]-3-hyd roxy-2-(1-methyl-1-phenylethyl)isoindolinone (1.16 g, yield 87%). ESI-MS *m*/*z:* 488 [M+H]⁺; ¹H-NMR (CDCl₃)δ(ppm) : 0.23 (s, 3H), 0.29 (s, 3H), 1.08 (d, *J* = 6.6 Hz, 3H), 1.12 (s, 9H), 1.30 (d, *J* = 7.0 Hz, 3H), 2.04-2.15 (m, 1H), 2.18 (s, 3H), 2.21 (s, 3H), 3.97 (m, 1H), 5.14-5.21 (m, 1H), 6.48 (d, *J* = 8.6 Hz, 1H), 7.45-7.91 (m, 7H).

### Step 5

4-Chloro-5-[1-(*tert*-butyldimethylsilyloxy)-2-methylpropyl]-3-hydroxy-2-(1-methyl-1-phenylethyl)isoindolinone (1.16 g, 2.38 mmol) was dissolved in THF (46 mL), and the solution was added with TMEDA (1.26 mL, 8.32 mmol). Then, sec-butyllithium-hexane solution (1.00 mol/L, 8.32 mL, 8.32 mmol) was added dropwise thereto at -78 °C for 10 minutes under argon atmosphere, followed by stirring at same temperature for 1.5 hours. The mixture was slowly added with iodine (0.724 mg, 2.85 mmol) in THF (10 mL), and heated from -78 °C to room temperature for 1 hour. The reaction mixture was added with aqueous sodium thiosulfate solution and acetic acid (1.43 mL) , and extracted with ethyl acetate. The organic layer was washed with saturated brine and dried over anhydrous sodium sulfate. The solvent was evaporated under reduced pressure, and the residue was purified by flush column chromatography (hexane/ethyl acetate=98/2, 80/20) to obtain 4-chloro-5-[1-(tert-butyldimethylsilyloxy)-2-methylpropyl]-7-iod o-3-hydroxy-2-(1-methyl-1-phenylethyl)isoindolinone (1.10 g, yield 75%).
ESI-MS *m*/*z*: 614 [M+H]⁺; ¹H-NMR (CDCl₃)δ(ppm) : -0.19 (s, 3H), 0.07 (s, 3H), 0.82-0.91 (m, 12H), 1.91-2.05 (m, 9H), 2.37 (dd, *J* = 2.0, 7.9 Hz, 1H), 4.82-4.89 (m, 1H), 3.57 (s, 1H), 6.08 (s, 1H), 7.20-7.46 (m, 5H), 8.02 (s, 1H).

### Step 6

4-Chloro-5-[1-(*tert*-butyldimethylsilyloxy)-2-methylpropyl]-7-iodo-3-hydroxy-2-(1-methyl-1-phenylethyl)isoindolinone (902 mg, 1.47 mmol) was dissolved in nitromethane (18 mL), and the solution was added with triethylsilane (1. 17 mL, 7.34 mmol) and trifluoroacetic acid (2.26 mL, 29.4 mmol), followed by stirring at room temperature for 1 hour. The reaction mixture was added with water, and extracted with ethyl acetate. The organic layer was washed with saturated brine and dried over anhydrous sodium sulfate. The solvent was evaporated under reduced pressure to obtain crude product.

The crude product obtained above was dissolved in 10% hydrogen chloride-methanol solution (18 mL), and the solution was stirred at 80 °C for 3 hours. The reaction mixture was condensed under reduced pressure, and the residue was purified by slurry using diisopripylether and chloroform to obtain 4-chloro-5-(1-hydroxy-2-methylpropyl)-7-iodoisoindolinone (499 mg, yield 93%).
ESI-MS *m*/*z*: 366 [M+H]⁺; ¹H-NMR (DMSO-*d*₆)δ(ppm) : 0.82 (d, *J* = 7.1 Hz, 3H), 0.90 (d, *J* = 6.5 Hz, 3H), 1.81-1.95 (m, 1H), 4.27 (s, 2H), 4.71 (d, *J* = 5.1 Hz, 1H), 5.56 (d, *J* = 4.4 Hz, 1H), 7.99 (s, 1H), 8.89 (s, 1H).

### Step 7

4-Chloro-5-(1-hydroxy-2-methylpropyl)-7-iodoisoindolinone (78.0 mg, 0.213 mmol) was dissolved in DMF (0.78 mL) and water (0.156 mL), and the solution was added with 1-(tert-butoxycarbonyl)-5-(diethylaminomethyl)indol-2-boronic acid (89.0 mg, 0.256 mmol), triethylamine (0.149 mL, 1.07 mmol) and [bis(diphenylphosphino)ferrocene]dichloropalladium (17.4 mg, 0.0213 mmol), followed by stirring at 80 °C for 2 hours under argon atmosphere. The reaction mixture was diluted with water and ethyl acetate, and filtered by celite to remove insoluble materials. The filtrate was extracted with ethyl acetate. The organic layer was washed with saturated brine and dried over anhydrous sodium sulfate. The solvent was evaporated under reduced pressure, and the residue was purified by flush column chromatography (chloroform/methanol/ammonia=95/5/1, 80/20/1) to obtain 4-chloro-5-(1-hydroxy-2-methylpropyl)-7-[1-(*tert*-butoxycarbonyl) -5-(diethylaminomethyl)indol-2-yl]isoindolinone (90.0 mg, yield 78%).
ESI-MS *m*/*z*: 540 [M+H]⁺; ¹H-NMR (CDCl₃)δ(ppm) : 0.99 (d, *J* = 4.4 Hz, 3H), 1.01 (d, *J* = 4.6 Hz, 3H), 1.06 (d, *J* = 6.9 Hz, 3H), 1.09 (d, *J* = 6.9 Hz, 3H), 1.36 (s, 9H), 2.03-2.18 (m, 1H), 2.61 (q, *J* = 6.9 Hz, 4H), 3.69 (s, 1H), 4.39 (s, 1H), 5.03 (dd, *J* = 5.6 Hz, 1H), 6.56 (s, 1H), 6.72 (s, 1H), 7.31 (d, *J* = 8.6 Hz, 3H), 7.50 (s, 1H), 7.67 (s, 1H), 8.13 (d, *J* = 8.6 Hz, 1H).

### Step 8

4-Chloro-5-(1-hydroxy-2-methylpropyl)-7-[1-(*tert*-butoxycarb onyl)-5-(diethylaminomethyl)indol-2-yl]isoindolinone (90.2 mg, 0.167 mmol) was added with 10% hydrogen chloride-methanol solution (1.8 mL), followed by stirring at 80 °C for 3 hours. The reaction mixture was condensed under reduced pressure, and the residue was recrystallized from diisopropylether (3.6 mL) and ethanol (1.8 mL) to obtain Compound 613 (70.7 mg, yield 89%).
ESI-MS *m*/*z*: 440 [M+H]⁺; ¹H-NMR (DMSO-*d*₆)δ(ppm) : 0.91 (d, *J* = 7.2 Hz, 3H), 0.94 (d, *J* = 7.2 Hz, 3H), 1.24 (d, *J* = 6.6 Hz, 3H), 1.27 (d, *J* = 6.6 Hz, 3H), 1.96-2.06 (m, 1H), 2.98-3.17 (m, 4H), 4.36 (d, *J* = 5.1 Hz, 2H), 4.51 (s, 2H), 4.80 (dd, *J* = 5.3 Hz, 1H), 5.61 (d, *J* = 5.3 Hz, 1H), 7.26 (d, *J* = 1.5 Hz, 1H), 7.33 (dd, *J* = 1.6 & 8.5 Hz, 1H), 7.59 (d, *J* = 8.5 Hz, 1H), 7.80 (s, 1H), 8.27 (s, 1H), 9.49 (s, 1H), 9.73 (br s, 1H), 13.88 (s, 1H).

Compounds 614 and 615 were synthesized in a similar manner to Example 146 (Examples 147 and 148).

### Example 147:

### 4-Chloro-5-(1-hydroxy-2-methylpropyl)-7-[1H-5-(piperidinomethyl) indol-2-yl]isoindolinone hydrochloride (Compound 614)

ESI-MS *m*/*z*: 452 [M+H]⁺; ¹H-NMR (DMSO-*d*₆)δ(ppm) : 0.91 (d, *J* = 7.2 Hz, 3H), 0.94 (d, *J* = 7.2 Hz, 3H), 1.62-1.86 (m, 6H), 1.94-2.08 (m, 1H), 2.77-2.95 (m, 2H), 3.27-3.35 (m, 2H), 4.32 (d, *J* = 4.6 Hz, 2H) , 4.50 (s, 2H), 4.80 (dd, *J* = 5.1 Hz, 1H), 5.61 (d, *J* = 5.1 Hz, 1H), 7.27 (d, *J* = 1.5 Hz, 1H), 7.33 (dd, *J* = 1.5, 8.5 Hz, 1H), 7.57 (d, *J* = 8.5 Hz, 1H), 7.79 (s, 1H), 8.27 (s, 1H), 9.50 (s, 1H), 9.96 (br s, 1H), 13.88 (s, 1H).

### Example 148:

### 4-Chloro-5-(1-hydroxy-2-methylpropyl)-7-{1H-5-[4-(2-hydroxyethyl )piperazin-l-ylmethyl]indol-2-yl}isoindolinone hydrochloride (Compound 615)

ESI-MS *m*/*z*: 497 [M+H]⁺; ¹H-NMR (DMSO-*d*₆)δ(ppm) : 0.91 (d , *J* = 6.6 Hz, 3H), 0.94 (d , *J* = 6.6 Hz, 3H), 1.95-2.07 (m, 1H), 3.11-3.82 (m, 16H), 4.50 (s, 2H), 4.80 (d, *J* = 5.6 Hz, 1H), 7.26 (s, 1H), 7.38 (d, *J* = 8.1 Hz, 1H), 7.57 (d, *J* = 8.1 Hz, 1H), 7.85 (s, 1H), 8.28 (s, 1H), 9.49 (s, 1H), 13.79 (s, 1H).

### Example 149:

### 5-Dimethylaminomethyl-7-(1H-4-hydroxyindol-2-yl)isoindolinone hydrochloride (Compound 616)

### Step 1

4-Hydroxyindole (5.10 g, 38.3 mmol) was dissolved in acetonitrile (51 mL), and the solution was added with di-*tert*-butyl dicarbonate (26.4 mL, 115 mmol) and DMAP (430 mg, 3.83 mmol) , followed by stirring at room temperature for 1 hour. The reaction mixture was condensed under reduced pressure. The residue was dissolved in methanol (51 mL) , and the solution was added with potassium carbonate (26.5 g, 192 mmol) , followed by stirring at room temperature for 3 hours. The reaction mixture was added with acetic acid (21.9 mL), and extracted with ethyl acetate. The organic layer was washed with saturated brine and dried over anhydrous sodium sulfate. The solvent was evaporated under reduced pressure, and the residue was purified by flush column chromatography (hexane/ethyl acetate=90:10, 60/40) to obtain 1-(*tert*-butoxycarbonyl)-4-hydroxyindole (8.77 g, yield 98%).
ESI-MS *m*/*z*: 234 [M+H]⁺; ¹H-NMR (CDCl₃)δ(ppm): 1.66 (s , 9H), 4.68 (s, 1H), 6.45 (dd, *J* = 0.8, 3.8 Hz, 1H), 6.83 (dd, *J* = 2.6, 8.9 Hz, 1H), 6.97 (d, *J* = 0.8, 2.6Hz, 1H), 7.56 (d, *J* = 3.8 Hz, 1H), 7.99 (d, *J* = 8.9 Hz, 1H).

### Step 2

To a solution of 1-(*tert*-butoxycarbonyl)-4-hydroxyindole (443 mg, 1.90 mmol) in THF (4.5 mL), triisopropyl borate (0.657 mL, 2.85 mmol) was added, then 2 mol/L lithiumdiisopropylamide-THF solution (2.85 mL, 5.70 mmol) was slowly added under ice-cooling, followed by stirring at same temperature for 1 hour. The reaction mixture was added with saturated aqueous ammonium chloride solution (15 mL) , and extracted with THF. The organic layer was washed with saturated brine, dried over anhydrous sodium sulfate, and added with *N,N*-dimethyl acetoamide (3.0 mL), followed by evaporation of THF under reduced pressure.

To the solution obtained above, 5-(dimethylaminomethyl)-7-iodoisoindolinone (300 mg, 0.949 mmol), triethylamine (0.661 mL, 4.74 mmol), water (0.684 mL) and [bis(diphenylphosphino)ferrocene]dichloropalladium (62.0 mg, 0.0759 mmol) were added, followed by stirring at 60 °C for 2 hours under argon atmosphere. The reaction mixture was diluted with water and ethyl acetate, and filtered using Celite to remove insoluble materials. The filtrate was extracted with ethyl acetate, and the organic layer was washed with saturated brine and dried over anhydrous sodium sulfate. The solvent was evaporated under reduced pressure, and the residue was purified by flush column chromatography (chloroform/methanol=99/1, 90/10) to obtain 5-(dimethylaminomethyl)-7-[1-(*tert*-butoxycarbonyl)-4-hydroxyindo 1-2-yl]isoindolinone (331 mg, yield 83%).
ESI-MS *m*/*z*: 422 [M+H]⁺; ¹H-NMR (CDCl₃)δ(ppm) : 1.27 (s, 9H), 2.33 (s, 6H), 3.55 (s, 2H), 4.25 (s, 2H), 6.47 (s, 1H), 6.52 (d, *J* = 8.1 Hz, 1H), 6.98 (s, 1H), 7.06 (dd, *J* = 8.1 Hz, 1H), 7.38 (s, 2H), 7.72 (d, *J* = 8.1 Hz, 1H).

### Step 3

5-(Dimethylaminomethyl)-7-[1-(*tert*-butoxycarbonyl)-4-hydrox yindol-2-yl]isoindolinone (92.0 mg, 0.218 mmol) was added with 10% hydrogen chloride-methanol solution (4.0 mL) and stirred at 60 °C for 3 hours. The reaction mixture was added with diisopropylether and purified by slurry to obtain Compound 616 (68.9 mg, yield 88%) ESI-MS *m*/*z*: 322 [M+H]⁺; ¹H-NMR (DMSO-*d*₆)δ(ppm) : 2.77 (s, 3H), 2.78 (s, 3H), 4.43 (s, 2H), 4.53 (s, 2H), 6.40 (dd, *J* = 1. 9 & 6.4Hz, 2H), 6.91 (s, 1H), 7.32 (s, 1H), 7.59 (s, 1H), 8.42 (s, 1H), 9.40 (s, 1H), 9.65 (s, 1H), 10.8 (br s, 1H), 13.7 (s, 1H).

### Example 150:

### 5-Dimethylaminomethyl-7-[1H-4-(3-hydroxypropyloxy)indol-2-yl]iso indolinone hydrochloride (Compound 617)

Compound 617 was synthesized in a similar manner to Example 149. ESI-MS *m*/*z*: 380 [M+H]⁺; ¹H-NMR (DMSO-*d*₆)δ(ppm) : 1.91-2.03 (m, 2H), 2.78 (s, 6H), 3.66 (t, *J* = 5.9 Hz, 2H), 4.18 (t, *J* = 5.9 Hz, 2H), 4.43 (s, 2H), 4.54 (s, 2H), 4.61 (s, 1H), 6.52 (dd, *J* = 3.7, 4.7 Hz, 1H), 7.04 (d, *J* = 4.7 Hz, 1H), 7.06 (d, *J* = 3.7 Hz, 1H), 7.30 (s, 1H), 7.61 (s, 1H), 8.45 (s, 1H), 9.43 (s, 1H), 10.7 (br s, 1H), 13.89 (s, 1H).

### Example 151:

### 4-Chloro-5-isopropyloxy-7-[1H-4-hydroxy-5-(piperidinomethyl)indo 1-2-yl)isoindolinone hydrochloride (Compound 618)

### Step 1

1-(*tert*-Butoxycarbonyl)-4-hydroxyindole (4.20 g, 18.0 mmol) was dissolved in ethanol (42 mL), and the solution was added with 37% aqueous formaldehyde solution (5.51 mL, 54.0 mmol) and piperidine (5.31 mL, 54.0 mmol). Then, the mixture was added with acetic acid (6.20 mL, 108 mmol) while keeping the reaction temperature below-7 °C, followed by stirring for 7 hours until the reaction temperature rises to 20 °C. The reaction mixture was added with sodium hydrogencarbonate (4.50 g, 54.0 mmol) and water, followed by stirring at room temperature for 30 minutes. The reaction mixture was extracted with ethyl acetate. The organic layer was washed with saturated brine and dried over anhydrous sodium sulfate. The solvent was evaporated under reduced pressure, and the residue was purified by flush column chromatography (chloroform/methanol= 99/1, 80/20) to obtain 1-(*tert*-butoxycarbonyl)-4-hydroxy-5-(piperidinomethyl)indole (4.24 g, yield 69%).
ESI-MS *m*/*z*: 331 [M+H]⁺; ¹H-NMR (CDCl₃)δ(ppm): 1.30 (s, 9H), 1.46-1.69 (m, 6H), 2.44-2.50 (m, 4H), 3.76 (d, *J* = 0.8 Hz, 2H), 4.44 (s, 1H), 6.70 (dd, *J* = 0.8, 3.8 Hz, 1H), 6.89 (d, *J* = 8.4 Hz, 1H), 7.47 (d, *J* = 3.8 Hz, 1H), 7.54 (d, *J* = 8.4 Hz, 1H).

### Step 2

To a solution of 1-(*tert*-butoxycarbonyl)-4-hydroxy-5-(piperidinomethyl)indole (218 mg, 0.660 mmol) in THF (2.1 mL), triisopropyl borate (0.228 mL, 0.990 mmol) was added. Next, to a solution of diisopropylamine (0.324 mL, 2.31 mmol) in THF (2.1mL), *n*-butyllithium-hexane solution (2.66 mol/L, 0.868mL, 2.31mmol) was added under ice-cooling, followed by stirring at same temperature for 10 minutes. To the reaction mixture, lithium diisopropylamide-THF solution was slowly added, followed by stirring for 1.5 hours under ice-cooling. The reaction mixture was added with saturated aqueous ammonium chloride solution (5 mL), and extracted with THF. The organic layer was washed with saturated brine, dried over anhydrous sodium sulfate, and added with N,N-dimethylacetoamide (3.0 mL), followed by evaporation of THF under reduced pressure.

To the solution obtained in the above step, 4-chloro-5-(isopropyloxy)-7-iodoisoindolinone (145 mg, 0.413 mmol), triethylamine (0.287 mL, 2.06 mmol), water (0.297 mL) and [bis(diphenylphosphino)ferrocene]dichloropalladium (34.0 mg, 0.0413 mmol) were added, followed by stirring at 60 °C for 2 hours under argon atmosphere. The reaction mixture was diluted with water and ethyl acetate, and filtered by Celite to remove insoluble materials. The filtrate was extracted with ethyl acetate. The organic layer was washed with saturated brine and dried over anhydrous sodium sulfate. The solvent was evaporated under reduced pressure, and the residue was purified by flush column chromatography (chloroform/methanol=99/1, 80/20) to obtain 4-chloro-5-(isopropyloxy)-7-[1-(*tert*-butoxycarbonyl)-4-hydroxy-5 -(piperidinomethyl)indol-2-yl]isoindolinone (264 mg).
ESI-MS *m*/*z*: 555 [M+H]⁺; ¹H-NMR (CDCl₃)δ(ppm) : 1.36 (s, 9H), 1.43 (d, *J* = 6.3 Hz, 6H), 1.58-1.70 (m, 6H), 2.35-2.67 (m, 4H), 3.77 (s, 2H), 4.36 (s, 2H), 4.66-4.75 (m, 1H), 6.72 (s, 1H), 6.71-6.77 (m, 1H), 6.91 (d, *J* = 8.8 Hz, 1H), 7.09 (s, 1H), 7.25-7.28 (m, 1H), 7.63 (d, *J* = 8.8 Hz, 1H).

### Step 3

4-Chloro-5-(isopropyloxy)-7-[1-(*tert*-butoxycarbonyl)-4-hydr oxy-5-(piperidinomethyl)indol-2-yl]isoindolinone (166 mg, 0.300 mmol) was added with 10% hydrogen chloride-methanol solution (5.0 mL), followed by stirring at 80 °C for 4 hours. The precipitated solid was collected by filtration to obtain Compound 618 (67.7 mg, yield 46%, 2 steps).
ESI-MS *m*/*z*: 455 [M+H]⁺; ¹H-NMR (DMSO-*d*₆)δ(ppm): 1.41 (d, *J* = 6.3 Hz, 6H), 1.62-1.84 (m, 6H), 2.84-2.92 (m, 2H), 3.27-3.41 (m, 2H), 4.29 (s, 2H), 4.45 (s, 2H), 4.93-5.02 (m, 1H), 7.04 (d, *J* = 8.3 Hz, 1H), 7.16 (d, *J* = 8.3 Hz, 1H), 7.55 (s, 1H), 7.66 (s, 1H), 9.29 (s, 1H), 9.39 (br s, 1H), 9.97 (s, 1H), 13.86 (s, 1H).

Compounds 619-621 were synthesized in a similar manner to Example 151 (Examples 152-154)

### Example 152:

### 4-chloro-5-isopropyloxy-7-[1H-4-(methanesulfonyloxy)-5-(piperidi nomethyl)indol-2-yl)isoindolinone hydrochloride (Compound 619)

ESI-MS *m*/*z:* 533 [M+H]⁺; ¹H-NMR (DMSO-*d*₆)δ(ppm) : 1.39 (d, *J* = 5. 9 Hz, 6H), 1.61-1.82 (m, 6H), 2.87-3.01 (m, 2H), 3.28-3.37 (m, 2H), 3.71 (s, 3H), 4.41-4.50 (m, 4H), 5.06-5.15 (m, 1H), 7.52 (d, *J* = 8.4 Hz, 1H), 7.55 (s, 1H), 7.65 (d, *J* = 8.4 Hz, 1H), 7.82 (s, 1H), 9.37 (s, 1H), 9.82 (br s, 1H), 14.30 (s, 1H).

### Example 153:

### 4-Chloro-5-isopropyloxy-7-[1H-4-(3-hydroxypropyloxy)-5-(piperidi nomethyl)indol-2-yl)isoindolinone hydrochloride (Compound 620)

ESI-MS *m*/*z*: 513 [M+H]⁺; ¹H-NMR (DMSO-*d*₆)δ(ppm) : 1.38 (d, *J* = 5.9 Hz, 6H), 1.41-1.46 (m, 2H), 1.61-1.86 (m, 4H), 1.96-2.09 (m, 2H), 2.81-2.97 (m, 2H) , 3.31-3.45 (m, 2H), 3.72 (t, *J* = 6.1 Hz, 2H) , 4.30 (s, 1H), 4.32 (s, 2H), 4.46 (s, 2H), 4.50 (d, *J* = 5.9 Hz, 2H), 5.09-5.17 (m, 1H), 7.27 (s, 2H), 7.63 (d, *J* = 1.7 Hz, 1H), 7.86 (s, 1H), 9.32 (s, 1H), 9.44 (br s, 1H), 14.10 (s, 1H).

### Example 154:

### 4-Chloro-5-isopropyloxy-7-{1H-4-(methanesulfonyloxy)-5-[4-(2-hyd roxyethyl)piperazin-1-ylmethyl]indol-2-yllisoindolinone (Compound 621)

ESI-MS *m*/*z:* 578 [M+H]⁺; ¹H-NMR (DMSO-*d*₆)δ(ppm) : 1.17 (d, *J* = 5.6 Hz, 6H), 2.09-2.23 (m, 8H), 3.15-3.30 (m, 6H), 3.34-3.44 (m, 4H), 4.16 (s, 2H), 4.24 (s, 2H), 4.83-4.91 (m, 1H), 7.06 (d, *J* = 8.2 Hz, 1H), 7.21 (s, 1H), 7.25 (d, *J* = 8.2 Hz, 1H), 7.57 (s, 1H), 9.10 (s, 1H), 13.86 (s, 1H).

### Example 155:

### 4-(4-Hydroxy-1-butynyl)-7-[1H-5-(diethylaminomethyl)indol-2-yl]i soindolinone (Compound 622)

### Step 1

4-Trifluorometanesulfonyloxy-7-[1-(*tert*-butoxycarbonyl)-5-( diethylaminomethyl)indol-2-yl]isoindolinone (240 mg, 0.413 mmol) was dissolved in acetonitrile (2.9 mL), and the solution was added with tetrakis(triphenylphosphine)palladium (47.7 mg, 0.0413 mmol), cuprous iodide (23.6 mg, 0.124 mmol), tetrabutylammonium iodide (458 mg, 1.24 mmol), 3-butyn-l-ol (156 µL, 2.07 mmol), and triethylamine (2.88 mL, 20.7 mmol), followed by stirring at room temperature for 40.7 hours under argon atmosphere. The reaction mixture was added with water and extracted with ethyl acetate. The organic layer was washed with saturated brine and dried over anhydrous sodium sulfate. The solvent was evaporated under reduced pressure, and the residue was purified by preparative thin-layer chromatography (chloroform/methanol=5/1) to obtain 4-(4-hydroxy-1-butynyl)-7-[1-(*tert*-butoxycarbonyl)-5-(diethylami nomethyl)indol-2-yl]isoindolinone (207 mg, yield 100%).
ESI-MS *m*/*z*: 502 [M+H]⁺; ¹H-NMR (DMSO-*d*₆)δ(ppm) : 0.94 (t, *J* = 7.2 Hz, 6H), 1.16 (s, 9H), 2.64 (t, *J* = 6.8 Hz, 2H), 3.30 (s, 2H), 3.64 (t, *J* = 6.8 Hz, 2H), 4.10 (q, *J* = 7.2 Hz, 4H), 4.37 (s, 2H), 4.97 (t, *J* = 6.8 Hz, 1H), 6.65 (s, 1H), 7.32 (d, *J* = 7.5 Hz, 1H), 7.44 (d, *J* = 7.9 Hz, 1H), 7.56 (br s, 1H), 7.64 (d, *J* = 7.9 Hz, 1H), 8.10 (d, *J* = 7.5 Hz, 1H), 8.73 (s, 1H).

### Step 2

To a solution of 4-(4-hydroxy-1-butynyl)-7-[1-(*tert*-butoxycarbonyl)-5-(diethylami nomethyl)indol-2-yl]isoindolinone (34.0 mg, 0.0662 mmol) in ethyl acetate (1.0 mL), hydrogen chloride-ethyl acetate solution (4.0mol/L, 1.0 mL) was added, followed by stirring at 60 °C for 4.0 hours under nitrogen atmosphere. The reaction mixture was added with saturated aqueous sodium hydrogencarbonate solution, and extracted with ethyl acetate. The organic layer was condensed, and the residue was purified by flush chromatography (chloroform/methanol=99/1, 80/20). The obtained product was added with isopropylether, and the precipitated solid was collected by filtration to obtain Compound 622 (26.5 mg, yield 16%).
ESI-MS *m*/*z*: 402 [M+H]⁺; ¹H-NMR (DMSO-*d*₆)δ(ppm) : 1.02 (br s, 6H), 2.64 (t, *J* = 6.6 Hz, 2H), 3.31 (s, 2H), 3.63 (t, *J* = 6.6 Hz, 2H), 3.73 (m, 4H), 4.48 (s, 2H), 4.97 (t, *J* = 6.6 Hz, 1H), 7.14 (d, *J* = 8.4 Hz, 1H), 7.24 (s, 1H), 7.37-7.57 (m, 2H), 7.63 (d, *J* = 8.1 Hz, 1H), 8.15 (d, *J* = 8.1 Hz, 1H), 9.42 (s, 1H), 13.83 (s, 1H).

Compounds 623-628 were synthesized in a similar manner to Example 155 (Examples 156-161).

### Example 156:

### 4-(4-Hydroxy-1-butynyl)-7-{1H-5-[4-(2-hydroxyethyl)piperazin-1-y lmethyl]indol-2-yl}isoindolinone (Compound 623)

ESI-MS *m*/*z*: 459 [M+H]⁺; ¹H-NMR (DMSO-*d*₆)δ(ppm) : 2.32-2.56 (m, 4H), 2.65 (t, *J* = 6.8 Hz, 2H), 2.78-3.82 (m, 4H), 3.34 (s, 2H), 3.45 (br s, 2H), 3.62 (t, *J* = 7.9 Hz, 2H), 3.73 (br s, 2H), 4.48 (s, 2H), 4.99 (t, *J* = 6.8 Hz, 1H), 7.11 (d, *J* = 7.9 Hz, 1H), 7.24 (s, 1H), 7.43 (d, *J* = 7.9 Hz, 1H), 7.49 (s, 1H), 7.63 (d, *J* = 7.5 Hz, 1H), 8.15 (d, *J* = 7.5 Hz, 1H), 9.44 (s, 1H), 13.86 (s, 1H).

### Example 157:

### 4-(4-Hydroxy-1-butynyl)-7-{1H-5-[(2-methoxyethylamino)methyl]ind ol-2-yl}isoindolinone (Compound 624)

ESI-MS *m*/*z*: 404 [M+H]⁺; ¹H-NMR (DMSO-*d*₆)δ(ppm) : 2.65 (t, *J* = 6.7 Hz, 2H), 3.08 (m, 1H), 3.16 (m, 1H), 3.31 (s, 3H), 3.55-3.69 (m, 4H), 4.02 (t, *J* = 6.7 Hz, 2H), 4.21 (s, 2H), 4.49 (s, 2H), 7.27 (d, *J* = 8.2 Hz, 1H), 7.34 (s, 1H), 7.55 (d, *J* = 8.4 Hz, 1H), 7.65 (d, *J* = 8.2 Hz, 1H), 7.74 (s, 1H), 8.20 (d, *J* = 8.4 Hz, 1H), 9.47 (s, 1H), 14.03 (s, 1H).

### Example 158:

### 4-(4-Hydroxy-1-pentynyl)-7-[1H-5-(piperidinomethyl)indol-2-yl]is oindolinone (Compound 625)

ESI-MS *m*/*z*: 428 [M+H]⁺; ¹H-NMR (DMSO-d₆)δ(ppm) : 1.23 (d, *J* = Hz, 3H), 1.33-1.54 (m, 6H), 2.28-2.39 (m, 4H), 2.53-2.62 (m, 2H), 3.47 (s, 2H), 3.85-3.95 (m, 1H), 4.48 (s, 2H), 4.92 (d, *J* = 4.6 Hz, 1H), 7.09 (d, *J* = 8.6 Hz, 1H), 7.21 (s, 1H), 7.39 (d, *J* = 7.8 Hz, 1H), 7.44 (s, 1H), 7.64 (d, *J* = 7.8 Hz, 1H), 8.14 (d, *J* = 8.6 Hz, 1H), 9.36 (s, 1H), 13.82 (s, 1H).

### Example 159:

### 4-[2-(Thiophen-3-yl)ethynyl]-7-[1H-5-(piperidinomethyl)indol-2-y 1]isoindolinone (Compound 626)

ESI-MS *m*/*z*: 452 [M+H]⁺; ¹H-NMR (DMSO-d₆)δ(ppm): 1.33-1.62 (m, 6H), 2.27-2.46 (m, 4H), 3.46 (s, 2H), 4.60 (s, 2H), 7.13 (d, *J* = 8.2 Hz, 1H), 7.29 (s, 1H), 7.34 (dd, *J* = 0.8, 5.0 Hz, 1H), 7.40-7.56 (m, 2H) , 7.70 (dd, *J* = 2.8, 5.1 Hz, 1H), 7.75 (d, *J* = 8.4 Hz, 1H), 7.99 (dd, *J* = 0.8, 3.0 Hz, 1H), 8.21 (d, *J* = 8.2 Hz, 1H), 9.49 (s, 1H), 13.89 (s, 1H).

### Example 160:

### 4-(trans-5-Hydroxy-3-penten-1-ynyl)-7-[1H-5-(piperidinomethyl)in dol-2-yl]isoindolinone (Compound 627)

ESI-MS *m*/*z*: 426 [M+H]⁺; ¹H-NMR (DMSO-*d*₆)δ(ppm) : 1.35-1.53 (m, 6H), 2.29-2.38 (m, 4H), 3.47 (s, 2H), 4.07-4.16 (m, 2H), 4.51 (s, 1H), 5.05 (t, *J* = 6.1 Hz, 1H), 6.03 (d, *J* = 15.6 Hz, 1H), 6.46 (dt, *J* = 4.2, 15.6 Hz, 1H), 7.09 (d, *J* = 8.4 Hz, 1H), 7.24 (s, 1H), 7.42 (d, *J* = 8.4 Hz, 1H), 7.45 (s, 1H), 7.67 (d, *J* = 8.2 Hz, 1H), 8.16 (d, *J* = 8.2 Hz, 1H), 9.43 (s, 1H), 13.91 (s, 1H).

### Example 161:

### 4-[2-(3-Methyl-3H-imidazol-4-yl)ethynyl-7-[1H-5-(piperidinomethy l)indol-2-yl]isoindolinone (Compound 628)

ESI-MS *m*/*z:* 450 [M+H]⁺; ¹H-NMR (DMSO-*d*₆)δ(ppm) : 1.27-1.45 (m, 1H), 1.52-1.89 (m, 5H), 2.76-2.99 (m, 2H), 3.28-3.44 (m, 2H), 3.81 (s, 3H), 4.35 (s, 2H), 4.65 (s, 2H), 7.26 (dd, *J* = 1.5, 8.1 Hz, 1H), 7.43 (d, *J* = 1.5 Hz, 1H), 7.60 (d, *J* = 8.1 Hz, 1H), 7.75 (s, 1H), 7.83 (d, *J* = 8.2 Hz, 1H), 8.29 (d, *J* = 8.2 Hz, 1H), 7.67 (d, *J* = 8.2 Hz, 1H), 8.16 (d, *J* = 8.2 Hz, 1H), 9.57 (s, 1H), 14.11 (s, 1H).

### Example 162:

### 4-[3-(3-Fluorophenylcarbonylamino)phenyl]-7-phenylisoindolinone (Compound 629)

### Step 1

4-Bromo-7-aminoisoindolinone (3.02 g, 13.3 mmol), 3-(*tert*-butoxycarbonylamino)phenylboronic acid (3.78 g, 15.9 mmol), Cesium carbonate (13.0 g, 39.9 mmol), and [(diphenylphosphino)ferrocene]dichloropalladium (543 mg, 0.665 mmol) were suspended in 1,4-dioxane (45 mL) and water (15 mL), and the suspension was stirred at 75 °C overnight. The reaction mixture was added with water and extracted with ethyl acetate. The organic layer was washed with saturated brine and dried over magnesium sulfate. The solvent was evaporated under reduced pressure, and the residue was purified by flush column chromatography (chloroform/ethyl acetate=65/35) to obtain 4-(3-*tert*-butoxycarbonylaminophenyl)-7-aminoisoindolinone (3.43 g, yield 76%).
ESI-MS *m*/*z*: 340 [M+H]⁺; ¹H-NMR (DMSO-*d*₆)δ(ppm) : 1.48 (s, 9H), 4.38 (s, 2H), 6.23 (s, 2H), 6.67 (d, *J* = 8.4 Hz, 1H), 7.07 (d, *J* = 7.7 Hz, 1H), 7.24-7.32 (m, 2H), 7.41 (d, *J* = 8.4 Hz, 1H), 7.54 (s, 1H), 8.28 (s, 1H), 9.35 (s, 1H).

### Step 2

4-(3-*tert*-butoxycarbonylaminophenyl)-7-aminoisoindolinone (2.20 g, 6.48 mmol) was dissolved in acetonitrile (30 mL), and the suspension was added with Cesium iodide (2.02 g, 7.77 mmol), iodine (1.97 g, 7.76 mmol) and cuprous iodide (1.48 g, 7.77 mmol), followed by adding with 11% hydroiodic acid (30 mL) under ice-cooling. Then, isoamyl nitrite (3.48 mL, 25.9 mmol) was added dropwise thereto for 5 minites under ice-cooling, followed by stirring at room temperature for 30 minutes. The reaction mixture was further added with isoamyl nitrite (0.870 mL, 6.48 mmol) followed by stirring for additional 30 minutes. The reaction mixture was added with 10% aqueous sodium thiosulfate solution and extracted with ethyl acetate. The organic layer was washed with saturated aqueous sodium hydrogencarbonate solution and dried over magnesium sulfate. The solvent was evaporated under reduced pressure and purified by slurry using methanol to obtain 4-(3-*tert*-butoxycarbonylaminophenyl)-7-iodoisoindolinone (1.88 g, yield 65%).
ESI-MS *m*/*z*: 451 [M+H]⁺; ¹H-NMR (DMSO-*d*₆)δ(ppm) : 1.48 (s, 9H), 4.36 (s, 2H), 7.19 (d, *J* = 7.9 Hz, 1H), 7.32 (d, *J* = 8.2 Hz, 1H), 7.37. (t, *J* = 7.9 Hz, 1H), 7.52 (d, *J* = 8.2 Hz, 1H), 7.64 (s, 1H), 8.00 (d, *J* = 7.9 Hz, 1H), 8.86 (s, 1H), 9.46 (s, 1H).

### Step 3

4-(3-*tert*-butoxycarbonylaminophenyl)-7-iodoisoindolinone (624 mg, 1.39 mmol) was dissolved in dichloromethane (2.4 mL), and the solution was added with trifluoroacetic acid (7.2 mL), followed by stirring at room temperature for 4 hours. The solvent was evaporated under reduced pressure and the residue was dissolved in ethyl acetate, followed by adding with 1 mol/L aqueous sodium hydroxide solution to adjust the pH to 6. The reaction mixture was extracted with ethyl acetate and chloroform. The organic layer was washed with saturated brine and dried over magnesium sulfate. The solvent was evaporated under reduced pressure, and the residue was purified by flush column chromatography (chloroform/methanol=90/10) to obtain 4- (3-aminophenyl) -7-iodoisoindolinone (430 mg, yield 88%) . ESI-MS *m*/*z:* 351 [M+H]⁺; ¹H-NMR (DMSO-*d*₆)δ(ppm) : 4.35 (s, 2H), 5.21 (s, 2H), 6.61 (dd, *J* = 7.9, 1.6 Hz, 1H), 6.69 (d, *J* = 7.9 Hz, 1H), 6.74 (s, 1H), 7.11 (t, *J* = 7.9 Hz, 1H), 7.27 (d, *J* = 7.9 Hz, 1H), 7.96 (d, *J* = 7.9 Hz, 1H), 8.82 (s, 1H).

### Step 4

4-(3-Aminophenyl)-7-iodoisoindolinone (578 mg, 1.65 mmol), phenylboronic acid (242 mg, 1.98 mmol), Cesium carbonate (1.20 g, 3.68 mmol) and [bis(diphenylphosphino)ferrocene]dichloropalladium (100 mg, 0.122 mmol) were suspended in 1,4-dioxane (9 mL) and water (3 mL), and the suspension was stirred at at 80 °C for 3 hours. The reaction mixture was filtered by Celite, and the solvent was evaporarted under reduced pressure. The residue was purified by flush column chromatography (chloroform/methanol=90/10) to obtain 4-(3-aminophenyl)-7-phenylisoindolinone (429 mg, yield 87%).
ESI-MS *m*/*z*: 301 [M+H]⁺; ¹H-NMR (DMSO-*d*₆)δ(ppm) : 4.42 (s, 2H), 5.20 (s, 2H), 6.61 (dd, *J* = 7.7, 1.5 Hz, 1H), 6.74 (d, *J* = 7.7 Hz, 1H), 6.79 (s, 1H), 7.14 (t, *J* = 7.7 Hz, 1H), 7.32-7.46 (m, 4H), 7.52 (dd, *J* = 7.6, 1.6 Hz, 2H), 7.58 (d, *J* = 7.9 Hz, 1H), 8.57 (s, 1H).

### Step 5

4-(3-Aminophenyl)-7-phenylisoindolinone (94.0 mg,0.313 mmol) was dissolved in DMF (3 mL), and the solution was added with HOBt monohydrate (63.2 mg, 0.468 mmol), 3-fluorobenzoic acid (88.1 mg, 0.629 mmol) and EDCI (90.3 mg, 0.471 mmol), followed by stirring at 50 °C overnight. The reaction mixture was added with saturated aqueous sodium hydrogencarbonate solution, and the precipitated solid was collected by filtration and washed with water, followed by drying under reduced pressure to obtain Compound 629 (116 mg, yield 88%). ESI-MS *m*/*z*: 423 [M+H]⁺; ¹H-NMR (DMSO-*d*₆)δ(ppm) : 4.51 (s, 2H), 7.33-7.72 (m, 11H), 7.76-7.89 (m, 3H), 8.05 (s, 1H), 8.65 (s, 1H), 10.44 (s, 1H).

Compounds 630-636 were synthesized in a similar manner to Example 162 (Examples 163-169).

### Example 163:

### 4-[3-(3-Trifluoromethylphenylcarbonylamino)phenyl]-7-(2,5-dimeth ylphenyl)isoindolinone (Compound 630)

ESI-MS *m*/*z*: 501 [M+H]⁺; ¹H-NMR (DMSO-*d*₆)δ(ppm) : 2.03 (s, 3H), 2.30 (s, 3H), 4.53 (d, *J* = 6.3 Hz, 2H), 6.95 (s, 1H), 7.04-7.16 (m, 2H), 7.29 (d, *J* = 7.9 Hz, 1H), 7.44 (d, *J* = 7.9 Hz, 1H), 7.53 (t, *J* = 7.9 Hz, 1H), 7.68 (d, *J* = 7.6 Hz, 1H), 7.77-7.88 (m, 2H), 8.00 (d, *J* = 7.9 Hz, 1H), 8.08 (s, 1H), 8.26-8.35 (m, 2H), 8.59 (s, 1H), 10.59 (s, 1H).

### Example 164:

### 4-[3-(3-Trifluoromethylphenylcarbonylamino)phenyl]-7-(4-tert-but ylphenyl)isoindolinone (Compound 631)

ESI-MS *m*/*z*: 529 [M+H]⁺; ¹H-NMR (DMSO-*d*₆)δ(ppm) : 1. 35 (s, 9H) , 4.51 (s, 2H), 7.39-7.57 (m, 7H), 7.68 (d, *J* = 7.9 Hz, 1H), 7.78-7.88 (m, 2H), 8.00 (d, *J* = 7.6 Hz, 1H), 8.05 (s, 1H), 8.27-8.34 (m, 2H), 8.66 (s, 1H), 10.61 (s, 1H).

### Example 165:

### 4-{3-[3-(3-Methylphenyl)ureido]phenyl}-7-phenylisoindolinone (Compound 632)

ESI-MS *m*/*z*: 434 [M+H]⁺; ¹H-NMR (DMSO-*d*₆) δ (ppm) : 2.28 (s, 3H), 4.48 (s, 2H), 6.80 (d, *J* = 7.2 Hz, 1H), 7. 16 (t, *J* = 7.7 Hz, 1H), 7.21-7.28 (m, 2H), 7.32 (s, 1H), 7.36-7.49 (m, 6H), 7.54 (dd, *J* = 7.6, 1.6 Hz, 2H), 7.67 (d, *J* = 7.9 Hz, 1H), 7.80 (s, 1H), 8.62 (s, 1H), 8.67 (s, 1H), 8.80 (s, 1H).

### Example 166:

### 4-[3-(3-Trifluoromethylphenylcarbonylamino)phenyl]-7-(thiophen-2 -yl)isoindolinone (Compound 633)

ESI-MS *m*/*z*: 479 [M+H]⁺; ¹H-NMR (DMSO-*d*₆)δ(ppm) : 4.51 (s, 2H) , 7.16 (dd, *J* = 4.9, 3.6 Hz, 1H), 7.41 (d, *J* = 7.9 Hz, 1H), 7.53 (t, *J* = 7. 9 Hz, 1H), 7.61-7.71 (m, 3H), 7.76-7.89 (m, 3H), 7.96-8.05 (m, 2H), 8.26-8.34 (m, 2H), 8.74 (s, 1H), 10.61 (s, 1H).

### Example 167:

### 4-[3-(3-Trifluoromethylphenylcarbonylamino)phenyl]-7-(3-cyanophe nyl)isoindolinone (Compound 634)

ESI-MS *m*/*z*: 498 [M+H]⁺; ¹H-NMR (DMSO-*d*₆)δ(ppm) : 4.54 (s, 2H), 7.44 (d, *J* = 7.6 Hz, 1H), 7.50-7.62 (m, 2H), 7.66 (d, *J* = 7.6 Hz, 1H), 7.75 (d, *J* = 7.9 Hz, 1H), 7.78-7.92 (m, 4H), 7.97 (d, *J* = 8.9 Hz, 1H), 8.01 (s, 1H), 8.07 (s, 1H), 8.27-8.34 (m, 2H), 8.76 (s, 1H), 10.62 (s, 1H).

### Example 168:

### 4-[3-(3-Trifluoromethylphenylcarbonylamino)phenyl]-7-(benzo[1,3] dioxol-4-yl)isoindolinone (Compound 635)

ESI-MS *m*/*z:* 517 [M+H]⁺; ¹H-NMR (DMSO-*d*₆)δ(ppm) : 4.50 (s, 2H), 6.07 (s, 2H), 6.94-7.04 (m, 2H), 7.11 (d, *J* = 1.3 Hz, 1H), 7.38-7.57 (m, 3H), 7.66 (d, *J* = 7.6 Hz, 1H), 7.77-7.88 (m, 2H), 8.00 (d, *J* = 7.9 Hz, 1H), 8.04 (s, 1H), 8.26-8.34 (m, 2H), 8.65 (s, 1H), 10.60 (s, 1H).

### Example 169:

### 4-[3-(3-Trifluoromethylphenylcarbonylamino)phenyl]-7-(4-phenoxyp henyl)isoindolinone (Compound 635)

ESI-MS *m*/*z:* 565 [M+H]⁺; ¹H-NMR (DMSO-*d*₆)δ(ppm) : 4.51 (s, 2H), 7.03 (d, *J* = 8.9 Hz, 2H), 7.11 (d, *J* = 7.9 Hz, 2H), 7.19 (t, *J* = 7.4 Hz, 1H), 7.39-7.61 (m, 7H), 7.69 (d, *J* = 7.9 Hz, 1H), 7.80-7.88 (m, 1H), 7.80 (d, *J* = 7.9 Hz, 1H), 7.98 (t, *J* = 7.9 Hz, 1H), 8.05 (s, 1H), 8.26-8.34 (m, 2H), 8.67 (s, 1H), 10.60 (s, 1H).

### Industrial Applicability

The present invention provides a JAK inhibitor comprising, as an active ingredient, a nitrogen-containing heterocyclic compound or a pharmaceutically acceptable salt thereof, or the like.

## Claims

1. A Janus kinase (JAK) inhibitor comprising, as an active ingredient, a nitrogen-containing heterocyclic compound represented by formula (I) : 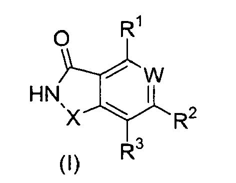
<wherein W represents a nitrogen atom or -CH-;
X represents -C (=O) - or -CHR⁴- (wherein R⁴ represents a hydrogen atom, hydroxy, substituted or unsubstituted lower alkyl, substituted or unsubstituted cycloalkyl, or substituted or unsubstituted lower alkoxy);
R¹ represents substituted or unsubstituted aryl, 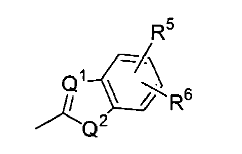
[wherein Q¹ represents a nitrogen atom or -CR⁸- {wherein R⁸ represents a hydrogen atom, halogen, nitro, hydroxy, cyano, carboxy, substituted or unsubstituted lower alkyl, substituted or unsubstituted cycloalkyl, substituted or unsubstituted aralkyl, substituted or unsubstituted lower alkenyl, substituted or unsubstituted lower alkynyl, substituted or unsubstituted aryl, a substituted or unsubstituted heterocyclic group, substituted or unsubstituted lower alkanoyl, substituted or unsubstituted aroyl, substituted or unsubstituted heteroaroyl, substituted or unsubstituted lower alkoxy, substituted or unsubstituted lower alkoxycarbonyl, -CONR⁹R¹⁰ (wherein R⁹ and R¹⁰ may be the same or different and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, substituted or unsubstituted lower alkanoyl, substituted or unsubstituted aroyl, or substituted or unsubstituted heteroaroyl, or R⁹ and R¹⁰ are combined together with the adjacent nitrogen atom thereto to form a substituted or unsubstituted heterocyclic group) or -NR¹¹R¹² [wherein R¹¹ and R¹² may be the same or different and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, substituted or unsubstituted lower alkanoyl, substituted or unsubstituted aroyl, substituted or unsubstituted heteroaroyl, substituted or unsubstituted lower alkylsulfonyl, substituted or unsubstituted arylsulfonyl, or -CONR¹³R¹⁴ (wherein R¹³ and R¹⁴ are the same as R⁹ and R¹⁰ defined above, respectively)]};
Q² represents an oxygen atom, a sulfur atom, or -NR¹⁵- [wherein R¹⁵ represents a hydrogen atom, substituted or unsubstituted lower alkyl, substituted or unsubstituted cycloalkyl, substituted or unsubstituted aralkyl, substituted or unsubstituted lower alkenyl, substituted or unsubstituted aryl, a substituted or unsubstituted heterocyclic group, substituted or unsubstituted lower alkanoyl, substituted or unsubstituted aroyl, substituted or unsubstituted heteroaroyl, substituted or unsubstituted lower alkoxycarbonyl, substituted or unsubstituted lower alkylsulfonyl, substituted or unsubstituted arylsulfonyl, or -CONR¹⁶R¹⁷ (wherein R¹⁶ and R¹⁷ are the same as R⁹ and R¹⁰ defined above, respectively)]; and
R⁵ and R⁶ may be the same or different and each represents a hydrogen atom, halogen, nitro, hydroxy, cyano, carboxy, substituted or unsubstituted lower alkyl, substituted or unsubstituted cycloalkyl, substituted or unsubstituted aralkyl, substituted or unsubstituted lower alkenyl, substituted or unsubstituted lower alkynyl, substituted or unsubstituted aryl, a substituted or unsubstituted heterocyclic group, substituted or unsubstituted lower alkanoyl, substituted or unsubstituted lower alkoxycarbonyl, substituted or unsubstituted aroyl, substituted or unsubstituted heteroaroyl, -S(O)m¹R¹⁸ [wherein m¹ repres'ents an integer of 0 to 2, R¹⁸ represents a hydrogen atom, hydroxy, substituted or unsubstituted lower alkoxy, substituted or unsubstituted lower alkyl, substituted or unsubstituted cycloalkyl, substituted or unsubstituted aralkyl, substituted or unsubstituted lower alkenyl, substituted or unsubstituted aryl, a substituted or unsubstituted heterocyclic group, or -NR¹⁹R²⁰ (wherein R¹⁹ and R²⁰ are the same as R⁹ and R¹⁰ defined above, respectively)], -OR²¹ [wherein R²¹ represents substituted or unsubstituted lower alkyl, substituted or unsubstituted cycloalkyl, substituted or unsubstituted aralkyl, substituted or unsubstituted lower alkenyl, substituted or unsubstituted aryl, a substituted or unsubstituted heterocyclic group, substituted or unsubstituted lower alkanoyl, substituted or unsubstituted aroyl, substituted or unsubstituted heteroaroyl, -CONR²²R²³ (wherein R²² and R²³ are the same as R⁹ and R¹⁰ defined above, respectively), or -S(O)m²R²⁴ (wherein m² is the same as m¹ defined above, and R²⁴ is the same as R¹⁸ defined above)], -CONR²⁵R²⁶ (wherein R²⁵ and R²⁶ are the same as R⁹ and R¹⁰ defined above, respectively), or -NR²⁷R²⁸ (wherein R²⁷ and R²⁸ are the same as R¹¹ and R¹² defined above, respectively)], or 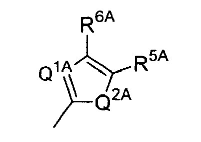
(wherein Q^{1A}, Q^{2A}, R^{5A} and R^{6A} are the same as Q¹, Q², R⁵ and R⁶ defined above, respectively); and
R² and R³ may be the same or different and each represents a hydrogen atom, halogen, nitro, hydroxy, cyano, carboxy, substituted or unsubstituted lower alkyl, substituted or unsubstituted cycloalkyl, substituted or unsubstituted aralkyl, substituted or unsubstituted lower alkenyl, substituted or unsubstituted lower alkynyl, substituted or unsubstituted aryl, a substituted or unsubstituted heterocyclic group, substituted or unsubstituted lower alkanoyl, substituted or unsubstituted lower alkoxycarbonyl, substituted or unsubstituted aroyl, substituted or unsubstituted heteroaroyl, -S(O)m³R²⁹ (wherein m³ is the same as m¹ defined above, and R²⁹ is the same as R¹⁸ defined above) , -OR³⁰ (wherein R³⁰ is the same as R²¹ defined above), -CONR³¹R³² (wherein R³¹ and R³² are the same as R⁹ and R¹⁰ defined above, respectively), or -NR³³R³⁴ (wherein R³³ and R³⁴ are the same as R¹¹ and R¹² defined above, respectively) > or a pharmaceutically acceptable salt thereof.

2. The Janus kinase (JAK) inhibitor according to Claim 1, wherein R¹ is 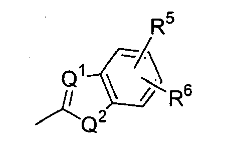 (wherein Q¹, Q², R⁵ and R⁶ are the same as those defined above, respectively).

3. The Janus kinase (JAK) inhibitor according to Claim 2, wherein Q¹ is -CH-.

4. The Janus kinase (JAK) inhibitor according to Claim 2 or 3, wherein Q² is -NR¹⁵- (wherein R¹⁵ is the same as that defined above).

5. The Janus kinase (JAK) inhibitor according to Claim 2 or 3, wherein Q² is -NH-.

6. The Janus kinase (JAK) inhibitor according to any one of Claims 2 to 5, wherein R⁶ is a hydrogen atom, and R⁵ is substituted or unsubstituted lower alkyl or -CONR²⁵R²⁶ (wherein R²⁵ and R²⁶ are the same as those defined above, respectively).

7. The Janus kinase (JAK) inhibitor according to any one of Claims 2 to 5, wherein R⁶ is a hydrogen atom, and R⁵ is -S(O)m¹R¹⁸ (wherein m¹ and R¹⁸ are the same as those defined above, respectively), -OR²¹ (wherein R²¹ is the same as that defined above), or -NR²⁷R²⁸ (wherein R²⁷ and R²⁸ are the same as those defined above, respectively).

8. The Janus kinase (JAK) inhibitor according to any one of Claims 2 to 5, wherein R⁶ is a hydrogen atom, and R⁵ is heteroalicyclic-lower alkyl or heteroalicyclic-lower alkyl substituted with hydroxy-lower alkyl.

9. The Janus kinase (JAK) inhibitor according to any one of Claims 2 to 5, wherein R⁶ is a hydrogen atom, and R⁵ is [mono or di(lower alkyl)amino]lower alkyl or (hydroxy-lower alkylamino)lower alkyl.

10. The Janus kinase (JAK) inhibitor according to any one of Claims 1 to 9, wherein R² is a hydrogen atom, and R³ is halogen, hydroxy, substituted or unsubstituted aryl, a substituted or unsubstituted heterocyclic group, or -OR³⁰ (wherein R³⁰ is the same as that defined above).

11. The Janus kinase (JAK) inhibitor according to any one of Claims 1 to 9, wherein R² is halogen, hydroxy, unsubstituted lower alkyl, unsubstituted lower alkenyl, unsubstituted lower alkynyl, substituted or unsubstituted aryl, a substituted or unsubstituted aromatic heterocyclic group, or -OR^{30a} (wherein R^{30a} is the same as R³⁰ defined above), and
R³ is halogen, hydroxy, substituted or unsubstituted aryl, a substituted or unsubstituted heterocyclic group, or -OR^{30b} (wherein R^{30b} is the same as R³⁰ defined above).

12. The Janus kinase (JAK) inhibitor according to any one of Claims 1 to 9, wherein R² is substituted or unsubstituted lower alkoxy, and R³ is lower alkylsulfonyloxy.

13. The Janus kinase (JAK) inhibitor according to any one of Claims 1 to 12, wherein X is -(C=O)-.

14. The Janus kinase (JAK) inhibitor according to any one of Claims 1 to 12, wherein X is -CHR⁴- (wherein R⁴ is the same as that defined above).

15. The Janus kinase (JAK) inhibitor according to any one of Claims 1 to 14, wherein W is a nitrogen atom.

16. The Janus kinase (JAK) inhibitor according to any one of Claims 1 to 14, wherein W is -CH-.

17. A therapeutic agent for a disease associated with Janus kinase (JAK) comprising, as an active ingredient, the nitrogen-containing heterocyclic compound or the pharmaceutically acceptable salt thereof described in any one of Claims 1 to 16.

18. A therapeutic agent for a myeloproliferative disorder comprising, as an active ingredient, the nitrogen-containing heterocyclic compound or the pharmaceutically acceptable salt thereof described in any one of Claims 1 to 16.

19. A therapeutic agent for a immunological disorder comprising, as an active ingredient, the nitrogen-containing heterocyclic compound or the pharmaceutically acceptable salt thereof described in any one of Claims 1 to 16.

20. A nitrogen-containing heterocyclic compound represented by formula (II): 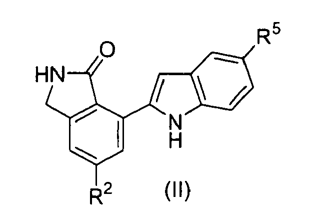 (wherein R² and R⁵ are the same as those defined above, respectively) or a pharmaceutically acceptable salt thereof.

21. The nitrogen-containing heterocyclic compound or the pharmaceutically acceptable salt thereof according to Claim 20, wherein R² is halogen, hydroxy, substituted or unsubstituted lower alkoxy, substituted or unsubstituted lower alkylsulfonyl, or mono- or di-(substituted or unsubstituted lower alkyl)amino.

22. The nitrogen-containing heterocyclic compound or the pharmaceutically acceptable salt thereof according to Claim 20, wherein R² is hydroxy-lower alkyl, lower alkoxy-lower alkyl, [mono- or di- ( lower alkyl) amino] lower alkyl or substituted or unsubstituted lower alkanoyl.

23. The nitrogen-containing heterocyclic compound or the pharmaceutically acceptable salt thereof according to Claim 20, wherein R² is substituted or unsubstituted aryl or a substituted or unsubstituted heterocyclic group.

24. The nitrogen-containing heterocyclic compound or the pharmaceutically acceptable salt thereof according to Claim 20, wherein R² is substituted or unsubstituted lower alkenyl or substituted or unsubstituted lower alkynyl.

25. The nitrogen-containing heterocyclic compound or the pharmaceutically acceptable salt thereof according to any one of Claims 20 to 24, wherein R⁵ is [mono- or di- (lower alkyl)amino]lower alkyl, heteroalicyclic-lower alkyl, or heteroalicyclic-lower alkyl substituted with hydroxy-lower alkyl.

26. A nitrogen-containing heterocyclic compound represented by formula (III) : 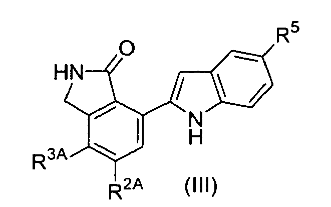 [wherein R⁵ is the same as that defined above, R^{2A} represents substituted or unsubstituted lower alkyl, -OR³⁰
(wherein R³⁰ is the same as that defined above), or -NR³³R³⁴ (wherein R³³ and R³⁴ are the same as those defined above, respectively), and R^{3A} represents halogen, hydroxy, substituted or unsubstituted lower alkoxy, substituted or unsubstituted lower alkylsulfonyl, or -OS(O)₂R^{30A} (wherein R^{30A} represents amino, substituted or unsubstituted lower alkyl, substituted or unsubstituted aryl, or a substituted or unsubstituted aromatic heterocyclic group)] or a pharmaceutically acceptable salt thereof.

27. The nitrogen-containing heterocyclic compound or the pharmaceutically acceptable salt thereof according to Claim 26, wherein R⁵ is substituted or unsubstituted heteroalicyclic-lower alkyl or [mono- or di-(substituted or unsubstituted lower alkyl)amino]lower alkyl.

28. A nitrogen-containing heterocyclic compound represented by formula (IV): 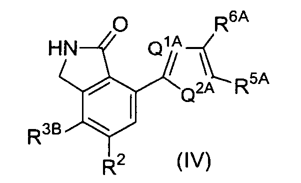 (wherein R², R^{5A}, R^{6A}, Q^{1A} and Q^{2A} are the same as those defined above, respectively, and R^{3B} represents a hydrogen atom, hydroxy, or halogen) or a pharmaceutically acceptable salt thereof.

29. The nitrogen-containing heterocyclic compound or the pharmaceutically acceptable salt thereof according to Claim 28, wherein Q^{1A} is -CH-, and
Q^{2A} is -NR^{15A}- (wherein R^{15A} represents a hydrogen atom or substituted or unsubstituted lower alkyl).

30. The nitrogen-containing heterocyclic compound or the pharmaceutically acceptable salt thereof according to Claim 29, wherein R^{15A} is a hydrogen atom.

31. The nitrogen-containing heterocyclic compound or the pharmaceutically acceptable salt thereof according to any one of Claims 28 to 30, wherein R^{5A} is a hydrogen atom, substituted or unsubstituted lower alkyl, or substituted or unsubstituted lower alkanoyl.

32. The nitrogen-containing heterocyclic compound or the pharmaceutically acceptable salt thereof according to any one of Claims 28 to 31, wherein R^{6A} is a hydrogen atom.

33. The nitrogen-containing heterocyclic compound or the pharmaceutically acceptable salt thereof according to any one of Claims 28 to 32, wherein R^{3B} is a hydrogen atom.

34. A nitrogen-containing heterocyclic compound represented by formula (V): 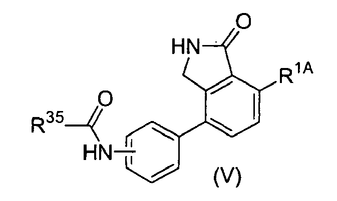 [wherein R^{1A} represents substituted or unsubstituted aryl or substituted or unsubstituted thienyl, and
R³⁵ represents aryl, aryl substituted with 1 to 3 subsituent(s) selected from Substituent Group A described below (Substituent Group A: halogen, lower alkyl, halogen-lower alkyl), or NHR^{35a} (wherein R^{35a} represents aryl or aryl substituted with 1 to 3 subsituent(s) selected from Substituent Group A described above)] or a pharmaceutically acceptable salt thereof.

35. A nitrogen-containing heterocyclic compound represented by formula (VI): 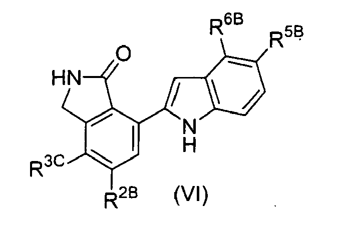 {wherein R^{2B} represents substituted or unsubstituted lower alkoxy, substituted or unsubstituted heteroalicyclic-lower alkyl, or [mono- or di-(substituted or unsubstituted lower alkyl)amino]lower alkyl, R^{3C} represents a hydrogen atom or halogen,
R^{5B} represents a hydrogen atom, substituted or unsubstituted heteroalicyclic-lower alkyl, or [mono- or di-(substituted or unsubstituted lower alkyl)amino]lower alkyl,
R^{6B} represents hydroxy, substituted or unsubstituted lower alkoxy, or substituted or unsubstituted lower alkylsulfonyloxy} or a pharmaceutically acceptable salt thereof.

36. The nitrogen-containing heterocyclic compound or the pharmaceutically acceptable salt thereof according to Claim 35, wherein R^{2B} is [di(lower alkyl)amino]lower alkyl, and R^{3c} and R^{5B} are hydrogen atoms.

37. The nitrogen-containing heterocyclic compound or the pharmaceutically acceptable salt thereof according to Claim 35, wherein R^{2B} is lower alkoxy,
R^{3C} is halogen, and
R^{5B} is substituted or unsubstituted heteroalicyclic-lower alkyl.

38. A pharmaceutical composition comprising, as an active ingredient, the nitrogen-containing heterocyclic compound or the pharmaceutically acceptable salt thereof described in any one of Claims 20 to 37.

39. A protein kinase inhibitor comprising, as an active ingredient, the nitrogen-containing heterocyclic compound or the pharmaceutically acceptable salt thereof described in any one of Claims 20 to 37.

40. A Janus kinase (JAK) inhibitor comprising, as an active ingredient, the nitrogen-containing heterocyclic compound or the pharmaceutically acceptable salt thereof described in any one of Claims 20 to 37.

41. A therapeutic agent for a disease associated with Janus kinase (JAK) comprising, as an active ingredient, the nitrogen-containing heterocyclic compound or the pharmaceutically acceptable salt thereof described in any one of Claims 20 to 37.

42. An antitumor agent comprising, as an active ingredient, the nitrogen-containing heterocyclic compound or the pharmaceutically acceptable salt thereof described in any one of Claims 20 to 37.

43. A therapeutic agent for a hematopoietic tumor comprising, as an active ingredient, the nitrogen-containing heterocyclic compound or the pharmaceutically acceptable salt thereof described in any one of Claims 20 to 37.

44. A therapeutic agent for a myeloproliferative disorder comprising, as an active ingredient, the nitrogen-containing heterocyclic compound or the pharmaceutically acceptable salt thereof described in any one of Claims 20 to 37.

45. A therapeutic agent for an immunological disorder comprising, as an active ingredient, the nitrogen-containing heterocyclic compound or the pharmaceutically acceptable salt thereof described in any one of Claims 20 to 37.

46. A method for inhibiting Janus kinase (JAK) comprising a step of administering an effective amount of the nitrogen-containing heterocyclic compound or the pharmaceutically acceptable salt thereof described in any one of Claims 1 to 16.

47. A method for treating a disease associated with Janus kinase (JAK) comprising a step of administering an effective amount of the nitrogen-containing heterocyclic compound or the pharmaceutically acceptable salt thereof described in any one of Claims 1 to 16.

48. A method for treating a myeloproliferative disorder comprising a step of administering an effective amount of the nitrogen-containing heterocyclic compound or the pharmaceutically acceptable salt thereof described in any one of Claims 1 to 16.

49. A method for treating an immunological disorder comprising a step of administering an effective amount of the nitrogen-containing heterocyclic compound or the pharmaceutically acceptable salt thereof described in any one of Claims 1 to 16.

50. A method for inhibiting a protein kinase comprising a step of administering an effective amount of the nitrogen-containing heterocyclic compound or the pharmaceutically acceptable salt thereof described in any one of Claims 20 to 37.

51. A method for inhibiting Janus kinase (JAK) comprising a step of administering an effective amount of the nitrogen-containing heterocyclic compound or the pharmaceutically acceptable salt thereof described in any one of Claims 20 to 37.

52. A method for treating a disease associated with Janus kinase (JAK) comprising a step of administering an effective amount of the nitrogen-containing heterocyclic compound or the pharmaceutically acceptable salt thereof described in any one of Claims 20 to 37.

53. A method for treating a tumor comprising a step of administering an effective amount of the nitrogen-containing heterocyclic compound or the pharmaceutically acceptable salt thereof described in any one of Claims 20 to 37.

54. A method for treating a hematopoietic tumor comprising a step of administering an effective amount of the nitrogen-containing heterocyclic compound or the pharmaceutically acceptable salt thereof described in any one of Claims 20 to 37.

55. A method for treating a myeloproliferative disorder comprising a step of administering an effective amount of the nitrogen-containing heterocyclic compound or the pharmaceutically acceptable salt thereof described in any one of Claims 20 to 37.

56. A method for treating an immunological disorder comprising a step of administering an effective amount of the nitrogen-containing heterocyclic compound or the pharmaceutically acceptable salt thereof described in any one of Claims 20 to 37.

57. Use of the nitrogen-containing heterocyclic compound or the pharmaceutically acceptable salt thereof described in any one of Claims 1 to 16 for the manufacture of a Janus kinase (JAK) inhibitor.

58. Use of the nitrogen-containing heterocyclic compound or the pharmaceutically acceptable salt thereof described in any one of Claims 1 to 16 for the manufacture of a therapeutic agent for a disease associated with Janus kinase (JAK).

59. Use of the nitrogen-containing heterocyclic compound or the pharmaceutically acceptable salt thereof described in any one of Claims 1 to 16 for the manufacture of a therapeutic agent for a myeloproliferative disorder.

60. Use of the nitrogen-containing heterocyclic compound or the pharmaceutically acceptable salt thereof described in any one of Claims 1 to 16 for the manufacture of a therapeutic agent for an immunological disorder.

61. Use of the nitrogen-containing heterocyclic compound or the pharmaceutically acceptable salt thereof described in any one of Claims 20 to 37 for the manufacture of a protein kinase inhibitor.

62. Use of the nitrogen-containing heterocyclic compound or the pharmaceutically acceptable salt thereof described in any one of Claims 20 to 37 for the manufacture of a Janus kinase (JAK) inhibitor.

63. Use of the nitrogen-containing heterocyclic compound or the pharmaceutically acceptable salt thereof described in any one of Claims 20 to 37 for the manufacture of a therapeutic agent for a disease associated with Janus kinase (JAK).

64. Use of the nitrogen-containing heterocyclic compound or the pharmaceutically acceptable salt thereof described in any one of Claims 20 to 37 for the manufacture of an antitumor agent.

65. Use of the nitrogen-containing heterocyclic compound or the pharmaceutically acceptable salt thereof described in any one of Claims 20 to 37 for the manufacture of a therapeutic agent for a hematopoietic tumor.

66. Use of the nitrogen-containing heterocyclic compound or the pharmaceutically acceptable salt thereof described in any one of Claims 20 to 37 for the manufacture of a therapeutic agent for a myeloproliferative disorder.

67. Use of the nitrogen-containing heterocyclic compound or the pharmaceutically acceptable salt thereof described in any one of Claims 20 to 37 for the manufacture of a therapeutic agent for an immunological disorder.
